# EUROPÄISCHE PATENTSCHRIFT

(11) **EP 2 782 565 B1**
(45) Veröffentlichungstag und Bekanntmachung des Hinweises auf die Patenterteilung: **06.11.2019**
(21) Anmeldenummer: 12788573.9
(22) Anmeldetag: 23.11.2012
(51) Int. Cl.: A61K 31/166, A61K 31/18, A61K 31/341, A61K 31/381, A61K 31/425, A61K 31/4402, A61K 31/4406, A61K 31/443, A61K 31/4436, A61K 31/4439, A61P 33/00

(54) **VERWENDUNG VON ARYL- UND HETARYLCARBOXAMIDEN ALS ENDOPARASITIZIDE**
USE OF ARYL AND HETARYL CARBOXAMIDES AS ENDOPARASITICIDES
ARYLCARBOXAMIDES ET HÉTARYLCARBOXAMIDES UTILISÉES EN TANT QU'ENDOARASITICIDES

(30) Priorität: 25.11.2011 EP 11190735
(43) Veröffentlichungstag der Anmeldung: 01.10.2014
(73) Patentinhaber: Bayer Intellectual Property GmbH, 40789 Monheim (DE)
(72) Erfinder: SCHWARZ, Hans-Georg, 46282 Dorsten (DE); TRAUTWEIN, Axel, 40593 Düsseldorf (DE); WILlMS, Lothar, 65719 Hofheim (DE); HINK, Maike, 71706 Markgröningen (DE); LÜMMEN, Peter, 65510 Idstein (DE); GÖRGENS, Ulrich, 40882 Ratingen (DE); COQUERON, Pierre-Yves, F-69009 Lyon (FR); HARDER, Achim, 51109 Köln (DE); WELZ, Claudia, 40597 Düsseldorf (DE); GREUL, Jörg, 51381 Leverkusen (DE); TRAUTWEIN, Axel, 40593 Düsseldorf (DE); COQUERON, Pierre-Yves, 69009 Lyon (FR)
(74) Vertreter: BIP Patents
(86) Internationale Anmeldenummer: PCT/EP2012/073431
(87) Internationale Veröffentlichungsnummer: WO 2013/076230

(56) Entgegenhaltungen:
- EP-A1- 1 256 569
- EP-A1- 1 792 901
- EP-A1- 1 997 800
- EP-A1- 2 132 987
- EP-A1- 2 682 115
- WO-A1-2007/060166
- WO-A1-2008/101976
- WO-A2-03/080577
- WO-A2-2006/108791
- WO-A2-2007/062308
- US-A1- 2006 276 515

## Beschreibung

Die vorliegende Anmeldung betrifft Aryl- und Hetarylcarboxamide zur Verwendung als Arzneimittel zur Bekämpfung von Endoparasiten in Tieren oder Menschen.

Im Bereich der Tiermedizin stellt das Auftreten von Resistenzen gegen alle kommerziell verfügbaren Anthelmintika ein zunehmendes Problem dar, demzufolge Endoparasitizide mit neuen molekularen Wirkmechanismen benötigt werden. Solche Verbindungen sollten exzellente Effektivität gegen ein breites Helminthen- bzw. Nematodenspektrum zeigen und zugleich bei den behandelten Tieren keine toxischen Effekte hervorrufen. Endoparasitizide Mittel sind Arzneimittel, die zur Bekämpfung von Endoparasiten in Menschen und Tieren angewendet werden.

Bestimmte Phenacylbenzamide werden in der WO-A 2001/060783 für die orale Anwendung als Anthelmintika für die Veterinärmedizin beansprucht.

Isothiazolcarboxamide sind aus der WO-A 1999/24413, Heterocyclylethylcarboxamid-Derivate aus der WO-A 2006/108791, Heterocyclylethylbenzamid-Derivate aus der WO-A 2006/108792, N-(1-Methyl-2-phenylethyl)-benzamide aus der WO-A 2007/060162, N-(1-Methyl-2-phenylethyl)-carboxamide aus der WO-A 2007060164, N-Phenethylcarboxamid-Derivative aus der WO-A 2007/060166, N-(3-Phenylpropyl)-carboxamide aus der WO-A 2008/101976, Pyrazolcarboxamide aus der WO-A 2008/148570 und WO-A 2010/063700, Pyrazinylcarboxamide aus der WO-A 2011/128989 sowie verschiedene 2-Pyridylethylcarboxamid-Derivate aus der WO-A 2004/016088, WO-A 2004/074280, WO-A 2005/014545, WO-A 2005/058828, WO-A 2005/058833 und WO-A 2005/085238 als agrochemische Fungizide bekannt. Ferner sind N-2-(Hetero)arylethyl-carboxamid-Derivate in WO-A 2007/108483 als Fungizide und Nematizide beschrieben. Die Verwendung von 2-Pyridylethylcarboxamid-Derivaten wird in der WO-A 2008/126922 explizit zur Anwendung gegen Nematoden im Pflanzenanbau beansprucht.

EP-A 1 997 800 offenbart N-2-(Hetero)arylethylcarboxamid-Derivate, die zur Bekämpfung von Pflanzenkrankheiten geeignet sind. WO-A 2003/080577 offenbart Amidoacetonitril-Verbindungen, die zur Bekämpfung von Endo- und Ektoparasiten, insbesondere Helminthen in Warmblütern geeignet sind. EP-A 1 256 569 offenbart Phenacylamine-Verbindungen, die als Pestizide geeignet sind. EP-A 2 132 987 offenbart N-2-(Pyridyl)-Ethyl-Carboxamide, die nicht an der Ethylene-Gruppe substituiert sind und die zur Bekämpfung von Nematoden geeignet sind. EP-A 1 792 901 offenbart N-(1-alkyl-2-Phenylethyl)-Carboxamide, die als Fungizide für der Landwirtschaft geeignet sind. US-A 2006/0276515 offenbart bestimmte Heteroarylverbindungen, die zur Behandlung von Bluthochdruck eingesetzt werden können. EP-A 2 682 115 offenbart ebenfalls bestimmte substituierte Phenylcarboxamide, die zur Bekämpfung von Endoparasiten bei Tieren geeignet sind.

Die Anwendung dieser vorbekannten (Het)arylethyl- bzw. (Het)arylpropyl-Carboxamide als Endoparasitizide im Bereich der Veterinärmedizin ist bislang nicht beschrieben.

Es wurde nun gefunden, dass Verbindungen der Formel (I), 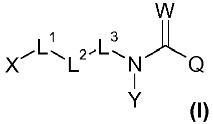 in welcher
- X: für ein ein- oder mehrfach durch M¹ substituiertes Phenyl, Thienyl oder Furanyl steht;
- Q: für ein ein- oder mehrfach durch M¹ substituiertes Phenyl, Pyridyl, Thienyl, Furanyl oder Isothiazolyl steht;
- Y: für Wasserstoff oder für gegebenenfalls ein- oder mehrfach durch M² substituiertes (C₁-C₁₀)-Alkyl, (C₂-C₁₀)-Alkenyl, (C₂-C₁₀)-Alkinyl, (C₁-C₁₀)-Halogenalkyl, (C₂-C₁₀)-Halogenalkenyl, (C₂-C₁₀)-Halogenalkinyl, (C₁-C₁₀)-Alkoxy, (C₂-C₁₀)-Alkenyloxy, (C₃-C₁₀)-Alkinyloxy, (C₃-C₁₄)-Cycloalkyl-(Ci-Cio)-alkyl oder für eine gegebenenfalls ein- oder mehrfach durch M² substituierte 3- bis 14-gliedrige cyclische Gruppe steht;
- W: für Sauerstoff oder Schwefel steht;
- L¹: für -C(R¹¹,R¹²)-, Sauerstoff, Schwefel, oder -N(R¹)- steht;
- L²: für -C(R²¹,R²²)- steht;
- L³: für -C(R³¹,R³²)- oder Direktbindung steht;
- M¹: für Wasserstoff, Halogen, Cyano, Nitro, OH, (C₁-C₁₀)-Alkyl, (C₁-C₁₀)-Halogenalkyl, (C₁-C₁₀)-Alkoxy, (C₁-C₁₀)-Halogenalkoxy, (C₁-C₁₀)-Alkylthio, (C₁-C₁₀)-Halogenalkylthio, (C₁-C₁₀)-Alkylsulfonyl, (C₁-C₁₀)-Halogenalkylsulfonyl, (C₁-C₁₀)-Alkylsulfanyl, (C₁-C₁₀)-Halogenalkylsulfanyl, (3- bis 14-gliedrige cyclische Gruppe)-O- steht;
- R¹: für Wasserstoff oder gegebenenfalls ein- oder mehrfach durch M² substituiertes (C₁-C₁₀)-Alkyl, (C₂-C₁₀)-Alkenyl, (C₂-C₁₀)-Alkinyl, (C₁-C₁₀)-Halogenalkyl, (C₂-C₁₀)-Halogenalkenyl, (C₂-C₁₀)-Halogenalkinyl, (C₃-C₁₀)-Cycloalkyl-(C₁-C₁₀)-alkyl oder für eine gegebenenfalls ein- oder mehrfach durch M² substituierte 3- bis 14-gliedrige cyclische Gruppe steht;
- R¹¹, R¹²: für jeweils unabhängig voneinander Wasserstoff, Halogen oder gegebenenfalls ein- oder mehrfach durch M² substituiertes (C₁-C₁₀)-Alkyl, (C₂-C₁₀)-Alkenyl, (C₂-C₁₀)-Alkinyl, (C₁-C₁₀)-Halogenalkyl, (C₂-C₁₀)-Halogenalkenyl, (C₂-C₁₀)-Halogenalkinyl, (C₁-C₁₀)-Alkoxy, (C₂-C₁₀)-Alkenyloxy, (C₃-C₁₀)-Alkinyloxy, (C₃-C₁₄)-Cycloalkyl-(C₁-C₁₀)-alkyl oder für eine gegebenenfalls ein- oder mehrfach durch M² substituierte 3- bis 14-gliedrige cyclische Gruppe steht;
- R¹¹, R¹²: zusammen für eine jeweils gegebenenfalls ein- oder mehrfach durch M² substituierte spiroverknüpfte 3- bis 14-gliedrige carbo- oder 3- bis 10-gliedrige heterocyclische Gruppe stehen;
- R²¹, R²²: für jeweils unabhängig voneinander Wasserstoff, Halogen oder gegebenenfalls ein- oder mehrfach durch M² substituiertes (C₁-C₁₀)-Alkyl, (C₂-C₁₀)-Alkenyl, (C₂-C₁₀)-Alkinyl, (C₁-C₁₀)-Halogenalkyl, (C₂-C₁₀)-Halogenalkenyl, (C₂-C₁₀)-Halogenalkinyl, (C₁-C₁₀)-Alkoxy, (C₁-C₁₀)-Halogenalkoxy, (C₂-C₁₀)-Alkenyloxy, (C₃-C₁₀)-Alkinyloxy, (C₃-C₁₄)-Cycloalkyl-(C₁-C₁₀)-alkyl oder für eine gegebenenfalls ein- oder mehrfach durch M² substituierte 3- bis 14-gliedrige cyclische Gruppe steht;
- R²¹, R²²: zusammen für eine jeweils gegebenenfalls ein- oder mehrfach durch M² substituierte spiroverknüpfte 3- bis 14-gliedrige carbo- oder 3- bis 10-gliedrige heterocyclische Gruppe stehen;
- R³¹, R³²: für jeweils unabhängig voneinander Wasserstoff oder gegebenenfalls ein- oder mehrfach durch M² substituiertes (C₁-C₁₀)-Alkyl, (C₂-C₁₀)-Alkenyl, (C₂-C₁₀)-Alkinyl, (C₁-C₁₀)-Halogenalkyl, (C₂-C₁₀)-Halogenalkenyl, (C₂-C₁₀)-Halogenalkinyl, (C₃-C₁₄)-Cycloalkyl-(C₁-C₁₀)-alkyl oder für eine gegebenenfalls ein- oder mehrfach durch M² substituierte 3- bis 14-gliedrige cyclische Gruppe steht;
- R³¹, R³²: zusammen für eine jeweils gegebenenfalls ein- oder mehrfach durch M² substituierte spiroverknüpfte 3- bis 14-gliedrige carbo- oder 3- bis 10-gliedrige heterocyclische Gruppe stehen;
- M²: für jeweils unabhängig voneinander für Halogen, Formyl, Cyano, Nitro, (C₁-C₁₀)-Alkyl, (C₁-C₁₀)-Halogenalkyl, (C₂-C₁₀)-Alkenyl, (C₂-C₁₀)-Halogenalkenyl, (C₂-C₁₀)-Alkinyl, (C₂-C₁₀)-Halogenalkinyl, (C₁-C₁₀)-Alkoxy, (C₁-C₁₀)-Halogenalkoxy, (C₂-C₁₀)-Alkenyloxy, (C₂-C₁₀)-Halogenalkenoxy, (C₃-C₁₀)-Alkinyloxy, (C₃-C₁₀)-Halogenalkinoxy, (C₁-C₁₀)-Alkylthio, (C₁-C₁₀)-Halogenalkylthio, (C₂-C₁₀)-Alkenylthio, (C₂-C₁₀)-Halogenalkenylthio, (C₃-C₁₀)-Alkinylthio, (C₃-C₁₀)-Halogenalkinylthio, (C₁-C₁₀)-Alkylsulfonyl, (C₁-C₁₀)-Halogenalkylsulfonyl, (C₂-C₁₀)-Alkenylsulfonyl, (C₂-C₁₀)-Halogenalkenylsulfonyl, (C₃-C₁₀)-Alkinylsulfonyl, (C₃-C₁₀)-Halogenalkinylsulfonyl, (C₁-C₁₀)-Alkylsulfanyl, (C₁-C₁₀)-Halogenalkylsulfanyl, (C₂-C₁₀)-Alkenylsulfanyl, (C₂-C₁₀)-Halogenalkenylsulfanyl, (C₃-C₁₀)-Alkinylsulfanyl, (C₃-C₁₀)-Halogenalkinylsulfanyl, (C₁-C₁₀)-Alkylcarbonyl, (C₁-C₁₀)-Halogenalkylcarbonyl, (C₂-C₁₀)-Alkenylcarbonyl, (C₂-C₁₀)-Halogenalkenylcarbonyl, (C₂-C₁₀)-Alkinylcarbonyl, (C₂-C₁₀)-Halogenalkinylcarbonyl, (C₁-C₁₀)-Alkoxycarbonyl, (C₁-C₁₀)-Halogenalkoxycarbonyl, (C₂-C₁₀)-Alkenoxycarbonyl, (C₂-C₁₀)-Halogenalkenoxycarbonyl, (C₃-C₁₀)-Alkinoxycarbonyl, (C₃-C₁₀)-Halogenalkinoxycarbonyl, (C₁-C₁₀)-Alkylcarbonyloxy, (C₁-C₁₀)-Halogenalkylcarbonyloxy, (C₂-C₁₀)-Alkenylcarbonyloxy, (C₂-C₁₀)-Halogenalkenylcarbonyloxy, (C₂-C₁₀)-Alkinylcarbonyloxy, (C₂-C₁₀)-Halogenalkinylcarbonyloxy, 3 bis 14-gliedrige cyclische Gruppe steht;
sowie Salze, N-Oxide und tautomere Formen der Verbindungen der Formel (I);
zur Verwendung als Arzneimittel zur Bekämpfung von Endoparasiten bei Tieren oder Menschen verwendet werden.

Die Verbindungen der Formel (I) können gegebenenfalls in Abhängigkeit von der Art der Substituenten als geometrische und/oder als optisch aktive Isomere oder entsprechende Isomerengemische in unterschiedlicher Zusammensetzung vorliegen. Die Verbindungen der Formel (I) betreffen sowohl die reinen Isomere als auch die Isomerengemische.

Die Verbindungen können auch als Metallkomplexe vorliegen.

### Definitionen

Der Begriff Tiere umfasst nicht den Menschen.

Der Begriff "ein- oder mehrfach" bedeutet bevorzugt ein- bis sechsfach, besonders bevorzugt ein- bis vierfach, ganz besonders bevorzugt ein- bis dreifach und insbesonders ganz bevorzugt ein- oder zweifach.

Der Fachmann ist sich bewusst, dass die Ausdrücke "ein", "eine" oder "eines" wie in dieser Anmeldung genutzt je nach Situation "ein/eine/eines (1)", "ein/eine/eines (1) oder mehr" oder "mindestens ein/eine/eines (1)" bedeuten kann.

Für alle bisher beschriebenen Ringsysteme gilt, dass benachbarte Atome nicht -O-O- oder -O-S- sein dürfen.

Strukturen mit einer variablen Anzahl an möglichen Kohlenstoffatomen (C-Atomen) werden zur Vereinfachung in der vorliegenden Anmeldung als C₁-C₁₀-Strukturen bezeichnet (C₁-C₁₀). Beispiel: eine Alkylgruppe aus 1 bis 10 C-Atomen entspricht (C₁-C₁₀)-Alkyl. Ringstrukturen aus C-Atomen und Heteroatomen werden als "3 bis 14-gliedrige" Strukturen bezeichnet.

Steht ein Sammelbegriff für einen Substituenten, z. B. (C₁-C₁₀)-Alkyl, am Ende eines zusammengesetzten Substituenten wie z.B. (C₃-C₁₄)-Cycloalkyl-(C₁-C₁₀)-alkyl, so kann der am Ende stehende Bestandteil des zusammengesetzten Substituenten, z.B. das (C₁-C₁₀)-Alkyl, ein- bzw. mehrfach, gleich oder verschieden und unabhängig voneinander mit dem am Anfang stehenden Substituenten, z. B. (C₃-C₁₄)-Cycloalkyl, substituiert sein.

Die Definition für Sammelbegriffe solange nicht anders definiert gilt auch für diese Sammelbegriffe in zusammengesetzten Substituenten. Beispiel: Die Definition für (C₁-C₁₀)-Alkyl gilt auch für (C₁-C₁₀)-Alkyl als Bestandteil eines zusammengesetzten Substituenten wie z.B. (C₃-C₁₄)-Cycloalkyl-(C₁-C₁₀)-alkyl.

Dem Fachmann ist klar, dass in dieser Anmeldung genannte Beispiele nicht als beschränkend anzusehen sind sondern lediglich einige Ausführungsformen näher beschreiben.

Bei den in den vorstehenden Formeln angegebenen Definitionen der Symbole wurden Sammelbegriffe verwendet, die allgemein repräsentativ für die folgenden Substituenten stehen:

### Sammelbegriffe

**Halogen,** soweit nicht anders definiert: Elemente der 7. Hauptgruppe, bevorzugt ist Fluor, Chlor, Brom bzw. Iod.

**(C₁-C₁₀)-Alkyl,** soweit nicht an anderer Stelle anders definiert: gesättigte, geradkettige oder verzweigte Kohlenwasserstoffreste bevorzugt mit (C₁-C₆)-, besonders bevorzugt (C₁-C₄)- Kohlenstoffatomen. Beispiele: Methyl, Ethyl, iso-Propyl, n-Propyl, 1-Methylethyl, Butyl, tert. Butyl, etc.

**(C₂-C₁₀)-Alkenyl,** soweit nicht an anderer Stelle anders definiert: ungesättigte, geradkettige oder verzweigte Kohlenwasserstoffreste mit einer Doppelbindung. Bevorzugt ist (C₂-C₆)- bzw. (C₂-C₄)-Alkenyl. Beispiele: Ethenyl, 1-Propenyl, 3-Butenyl, etc.

**(C₂-C₁₀)-Alkinyl,** soweit nicht an anderer Stelle anders definiert: ungesättigte, geradkettige oder verzweigte Kohlenwasserstoffreste mit einer Dreifachbindung. Bevorzugt ist (C₂-C₆)- bzw. (C₂-C₄)-Alkinyl. Beispiele: Ethinyl, 1-Propinyl, etc.

**(C₁-C₁₀)-Alkoxy** (Alkylrest-O-), soweit nicht an anderer Stelle anders definiert: ein Alkylrest, der über ein Sauerstoffatom (-O-) an das Gerüst gebunden ist. Bevorzugt ist (C₁-C₆)- bzw. (C₁-C₄)-Alkoxy. Beispiele: Methoxy, Ethoxy, Propoxy, 1-Methylethoxy, etc.

Analog sind **(C₂-C₁₀)-Alkenoxy** und **(C₃-C₁₀)-Alkinoxy,** soweit nicht an anderer Stelle anders definiert Alkenylreste bzw. Alkinylreste, die über -O- an das Gerüst gebunden sind. Bevorzugt ist (C₂-C₆)- bzw. (C₂-C₄)-Alkenoxy. Bevorzugt (C₃-C₆)- bzw. (C₃-C₄)-Alkinoxy.

**(C₁-C₁₀)-Alkylcarbonyl** (Alkylrest-C(=O)-), soweit nicht an anderer Stelle anders definiert: bevorzugt ist (C₁-C₆)- bzw. (C₁-C₄)-Alkylcarbonyl. Die Anzahl der C-Atome bezieht sich dabei auf den Alkylrest in der Alkylcarbonylgruppe.

Analog sind **(C₂-C₁₀)-Alkenylcarbonyl** und **(C₂-C₁₀)-Alkinylcarbonyl,** soweit nicht an anderer Stelle anders definiert: Alkenylreste bzw. Alkinylreste, die über -C(=O)- an das Gerüst gebunden sind. Bevorzugt ist (C₂-C₆)- bzw. (C₂-C₄)-Alkenylcarbonyl. Bevorzugt ist (C₂-C₆)- bzw. (C₂-C₄)-Alkinylcarbonyl.

**(C₁-C₁₀)-Alkoxycarbonyl** (Alkylrest-O-C(=O)-), soweit nicht an anderer Stelle anders definiert: bevorzugt ist (C₁-C₆)- bzw. (C₁-C₄)-Alkoxycarbonyl. Die Anzahl der C-Atome bezieht sich dabei auf den Alkylrest in der Alkoxycarbonylgruppe.

Analog sind **(C₂-C₁₀)-Alkenoxycarbonyl** und **(C₃-C₁₀)-Alkinoxycarbonyl,** soweit nicht an anderer Stelle anders definiert: Alkenylreste bzw. Alkinylreste, die über -O-C(=O)- an das Gerüst gebunden sind. Bevorzugt ist (C₂-C₆)- bzw. (C₂-C₄)-Alkenoxycarbonyl. Bevorzugt ist (C₃-C₆)- bzw. (C₃-C₄)-Alkinoxycarbonyl.

**(C₁-C₁₀)-Alkylcarbonyloxy** (Alkylrest-C(=O)-O-), soweit nicht an anderer Stelle anders definiert: ein Alkylrest, der über eine Carbonyloxygruppe (-C(=O)-O-) mit dem Sauerstoff an das Gerüst gebunden ist. Bevorzugt ist (C₁-C₆)- bzw. (C₁-C₄)-Alkylcarbonyloxy.

Analog sind **(C₂-C₁₀)-Alkenylcarbonyloxy** und **(C₂-C₁₀)-Alkinylcarbonyloxy,** soweit nicht an anderer Stelle anders definiert: Alkenylreste bzw. Alkinylreste, die über (-C(=O)-O-) an das Gerüst gebunden sind. Bevorzugt ist (C₂-C₆)- bzw. (C₂-C₄)-Alkenylcarbonyloxy. Bevorzugt ist (C₂-C₆)- bzw. (C₂-C₄)-Alkinylcarbonyloxy.

**(C₁-C₁₀)-Alkylthio,** soweit nicht an anderer Stelle anders definiert: Alkylrest, der über -S- an das Gerüst gebunden ist. Bevorzugt ist (C₁-C₆)- bzw. (C₁-C₄)-Alkylthio.

Analog sind **(C₂-C₁₀)-Alkenylthio** und **(C₃-C₁₀)-Alkinylthio,** soweit nicht an anderer Stelle anders definiert: Alkenylreste bzw. Alkinylreste, die über -S- an das Gerüst gebunden sind. Bevorzugt ist (C₂-C₆)- bzw. (C₂-C₄)-Alkenylthio. Bevorzugt ist (C₃-C₆)- bzw. (C₃-C₄)-Alkinylthio.

**(C₁-C₁₀)-Alkylsulfinyl,** soweit nicht an anderer Stelle anders definiert: Alkylrest, der über -S(=O)- an das Gerüst gebunden ist. Bevorzugt ist (C₁-C₆)- bzw. (C₁-C₄)-Alkylsulfinyl.

Analog sind **(C₂-C₁₀)-Alkenylsulfinyl** und **(C₃-C₁₀)-Alkinylsulfinyl,** soweit nicht an anderer Stelle anders definiert: Alkenylreste bzw. Alkinylreste, die über -S(=O)- an das Gerüst gebunden sind. Bevorzugt ist (C₂-C₆)- bzw. (C₂-C₄)-Alkenylsulfinyl. Bevorzugt ist (C₃-C₆)- bzw. (C₃-C₄)-Alkinylsulfinyl.

**(C₁-C₁₀)-Alkylsulfonyl,** soweit nicht an anderer Stelle anders definiert: Alkylrest, der über -S(=O)₂- an das Gerüst gebunden ist. Bevorzugt ist (C₁-C₆)- bzw. (C₁-C₄)-Alkylsulfonyl.

Analog sind **(C₂-C₁₀)-Alkenylsulfonyl** und **(C₃-C₁₀)-Alkinylsulfonyl,** soweit nicht an anderer Stelle anders definiert: Alkenylreste bzw. Alkinylreste, die über -S(=O)₂- an das Gerüst gebunden sind. Bevorzugt ist (C₂-C₆)- bzw. (C₂-C₄)-Alkenylsulfonyl. Bevorzugt ist (C₃-C₆)- bzw. (C₃-C₄)-Alkinylsulfonyl.

(C₁-C₁₀)-Halogenalkyl, (C₂-C₁₀)-Halogenalkenyl, (C₂-C₁₀)-Halogenalkinyl, (C₁-C₁₀)-Halogenalkoxy, (C₂-C₁₀)-Halogenalkenoxy, (C₃-C₁₀)-Halogenalkinoxy, (C₁-C₁₀)-Halogenalkylcarbonyl, (C₂-C₁₀)-Halogenalkenylcarbonyl, (C₂-C₁₀)-Halogenalkinylcarbonyl, (C₁-C₁₀)-Halogenalkoxycarbonyl, (C₂-C₁₀)-Halogenalkenoxycarbonyl, (C₃-C₁₀)-Halogenalkinoxycarbonyl, (C₂-C₁₀)-Halogenalkylcarbonyloxy, (C₂-C₁₀)-Halogenalkenylcarbonyloxy, (C₂-C₁₀)-Halogenalkinylcarbonyloxy, (C₁-C₁₀)-Halogenalkylthio, (C₂-C₁₀)-Halogenalkenylthio, (C₃-C₁₀)-Halogenalkinylthio, (C₁-C₁₀)-Halogenalkylsulfinyl, (C₂-C₁₀)-Halogenalkenylsulfinyl, (C₃-C₁₀)-Halogenalkinylsulfinyl, (C₁-C₁₀)-Halogenalkylsulfonyl, (C₂-C₁₀)-Halogenalkenylsulfonyl, (C₃-C₁₀)-Halogenalkinylsulfonyl sind jeweils soweit nicht anders definiert analog zu (C₁-C₁₀)-Alkyl, (C₂-C₁₀)-Alkenyl, (C₂-C₁₀)-Alkinyl, (C₁-C₁₀)-Alkoxy, (C₂-C₁₀)-Alkenoxy, (C₃-C₁₀)-Alkinoxy, (C₁-C₁₀)-Alkylcarbonyl, (C₂-C₁₀)-Alkenylcarbonyl, (C₂-C₁₀)-Alkinylcarbonyl, (C₁-C₁₀)-Alkoxycarbonyl, (C₂-C₁₀)-Alkenoxycarbonyl, (C₃-C₁₀)-Alkinoxycarbonyl, (C₁-C₁₀)-Alkylcarbonyloxy, (C₂-C₁₀)-Alkenylcarbonyloxyl, (C₂-C₁₀)-Alkinylcarbonyloxy, (C₁-C₁₀)-Alkylthio, (C₂-C₁₀)-Alkenylthio, (C₃-C₁₀)-Alkinylthio, (C₁-C₁₀)-Alkylsulfinyl, (C₂-C₁₀)-Alkenylsulfinyl, (C₃-C₁₀)-Alkinylsulfinyl, (C₁-Cio)-Alkylsulfonyl, (C₃-C₁₀)-Alkenylsulfonyl, (C₃-C₁₀)-Alkinylsulfonyl definiert, wobei mindestens ein Wasserstoffatom durch ein Halogenatom wie vorstehend genannt ersetzt ist. In einer Ausführungsform sind alle Wasserstoffatome durch Halogen ersetzt. Beispiele für halogenierte Strukturen sind z. B. Chlormethyl, Trichlormethyl, Fluormethyl, Chlordifluormethyl, Dichlorfluormethyl, Trifluormethyl, 2,2-Difluorethyl, Difluormethyl, Difluormethoxy, Trifluormethoxy, Difluormethylthio, Trifluormethylthio.

### Cyclische Gruppen

**3 bis 14-gliedrige cyclische Gruppe,** soweit nicht an anderer Stelle anders definiert: (C₃-C₁₄)-carbocyclische Gruppe, 3 bis 10-gliedrige heterocyclische Gruppe, halogenierte (C₃-C₁₄)-carbocyclische Gruppe, halogenierte 3 bis 10-gliedrige heterocyclische Gruppe.

**(C₃-C₁₄)-carbocyclische Gruppe,** soweit nicht an anderer Stelle anders definiert: (C₃-C₁₄)-Cycloalkyl, (C₃-C₁₄)-Cycloalkenyl, (C₆-C₁₄)-Aryl, halogeniertes (C₃-C₁₄)-Cycloalkyl, halogeniertes (C₃-C₁₄)-Cycloalkenyl, halogeniertes (C₆-C₁₄)-Aryl.

**(C₃-C₁₄)-Cycloalkyl,** soweit nicht an anderer Stelle anders definiert: mono-, bi- oder tricyclische, gesättigte Kohlenwasserstoffgruppen, bevorzugt mit (C₃-C₁₄)-, (C₃-C₈)- bzw. (C₃-C₆)-Ringatomen. Ein Cycloalkyl kann auch eine spirocyclische Gruppe sein. Beispiele: Cyclopropyl, -butyl, -pentyl, -hexyl,-heptyl, Bicyclo[2.2.1]heptyl oder Adamantyl. Bevorzugt steht "Cycloalkyl" für monocyclische Gruppen aus 3, 4, 5, 6 oder 7 Ringatomen.

Analog ist **(C₃-C₁₄)-Cycloalkenyl,** soweit nicht an anderer Stelle anders definiert: eine mono-, bi- oder tricyclische jedoch partiell ungesättigte Kohlenwasserstoffgruppe mit mindestens einer Doppelbindung, bevorzugt mit (C₃-C₈)- bzw. (C₃-C₆)-Ringatomen. Beispiele: Cyclopropenyl, Cyclobutenyl, Cyclopentenyl und Cyclohexenyl.

**(C₆-C₁₄)-Aryl,** soweit nicht an anderer Stelle anders definiert: mono- bi- oder tricyclische Ringsystemgruppe wobei mindestens ein Cyclus aromatisch ist, bevorzugt mit (C₆-C₈)- bzw. (C₆)-Ringatomen. Bevorzugt ist Aryl eine aromatische C₆-monocyclische Ringsystemgruppe; eine bicyclische (C₈-C₁₄)-Ringsystemgruppe; oder eine tricyclische (C₁₀-C₁₄)-Ringsystemgruppe. Beispiele: Phenyl, Naphthyl, Anthryl, Phenanthryl, Tetrahydronaphthyl, Indenyl, Indanyl, Fluorenyl.

Halogenierte (C₃-C₁₄)-carbocyclische Gruppe, halogeniertes (C₃-C₁₄)-Cycloalkyl, halogeniertes (C₃-C₁₄)-Cycloalkenyl, halogeniertes (C₆-C₁₄)-Aryl sind jeweils soweit nicht anders definiert analog zu (C₃-C₁₄)-carbocyclische Gruppe, (C₃-C₁₄)-Cycloalkyl, (C₃-C₁₄)-Cycloalkenyl, (C₆-C₁₄)-Aryl definiert, wobei mindestens ein Wasserstoffatom durch ein Halogenatom wie vorstehend genannt ersetzt ist. In einer Ausführungsform sind alle Wasserstoffatome durch Halogen ersetzt. Beispiele für halogenierte Strukturen sind 3-Chlorphenyl, 2-Bromcyclopentyl.

**Heteroatom:** zum Beispiel N, O, S, P, B, Si.

**3 bis 10-gliedrige heterocyclische Gruppe,** soweit nicht an anderer Stelle anders definiert: 3 bis 9-gliedrige Heterocyclylgruppe oder 5 bis 10-gliedrige Heteroarylgruppe, halogenierte 3 bis 9-gliedrige Heterocyclylgruppe oder halogenierte 5 bis 10-gliedrige Heteroarylgruppe.

**3 bis 9-gliedriges Heterocyclyl,** soweit nicht an anderer Stelle anders definiert: 3 bis 9-gliedrige gesättigte oder partiell ungesättigte mono-, bi- oder tricyclische Ringsystemgruppe, aus C-Atomen und mindestens einem Heteroatom, vorzugsweise ausgewählt aus N, O und/oder S. Bevorzugt ist das Ringsystem ein 3 bis 6-gliedriges Ringsystem. Bevorzugt enthält das Ringsystem 1, 2, 3 oder 4 Heteroatome, besonders bevorzugt 1 oder 2 Heteroatome. Ebenfalls bevorzugt ist ein monocyclisches Ringsystem. In einer weiteren bevorzugten Ausführungsform ist ein monocyclisches Ringsystem ein partiell ungesättigtes monocyclisches Ringsystem mit einer Doppelbindung. Ein Heterocyclyl kann ein spirocyclisches System sein. Beispiele: Piperazinyl, Dihydropyridyl, Morpholinyl, etc.. Diese Definition gilt auch für Heterocyclyl als Bestandteil eines zusammengesetzten Substituenten wie z.B. 3 bis 9-gliedriges Heterocyclyl(C₁-C₁₀)-alkyl, sofern an anderer Stelle nicht definiert.

**5 bis 10-gliedriges Heteroaryl,** soweit nicht an anderer Stelle anders definiert: mono- bi- oder tricyclische 5 bis 10-gliedrige heterocyclische Gruppe aus C-Atomen und mindestens einem Heteroatom, wobei mindestens ein Cyclus aromatisch ist, vorzugsweise ausgewählt aus N, O und/oder S. Bevorzugt ist das Ringsystem ein ein 5 bis 6-gliedriges Ringsystem. In einer Ausführungsform ist Heteroaryl ein aromatisches monocyclisches Ringsystem aus 5 oder 6 Ringatomen. Bevorzugt ist Heteroaryl als ein aromatisches monocyclisches Ringsystem, enthaltend 1 bis 4 Heteroatome aus der Gruppe O, N, oder S. Weiterhin kann Heteroaryl ein bicyclisches Ringsystem darstellen, das aus 8 bis 14 Ringatomen besteht oder ein tricyclisches Ringsystem, das aus 13 bis 14 Ringatomen besteht. Beispiele: Furyl, Thienyl, Pyrazolyl, Imidazolyl, Triazolyl, Thiazolyl, Indolyl, Benzimidazolyl, Indazolyl, Benzofuranyl, Benzothiophenyl, Benzothiazolyl, Benzoxazolyl, Chinolinyl, Isochinolinyl. Diese Definition gilt auch für Heteroaryl als Bestandteil eines zusammengesetzten Substituenten wie z.B. 5 bis 10-gliedriges Heteroaryl(C₅-C₁₀)-alkyl, sofern an anderer Stelle nicht definiert. Im folgenden sind 5- und 6-gliedrige Heteroarylgruppen näher beschrieben:

**5-gliedriges Heteroaryl,** soweit nicht an anderer Stelle anders definiert: Heteroarylgruppe enthaltend ein bis drei bzw. ein bis vier N-, O- und/oder S-Atom(e) als Ringatome. Beispiele: Furanyl, Thienyl, Oxazolyl, Thiazolyl. In einer Ausführungsform enthält eine 5-gliedrige Heteroarylgruppe neben C-Atomen ein bis vier N-Atome bzw. ein bis drei N-Atome als Ringglieder. Beispiele: Pyrrolyl, Pyrazolyl, Triazolyl, Imidazolyl. In einer weiteren Ausführungsform enthält ein 5-gliedriges Heteroaryl ein bis drei N-Atome oder ein N-Atom und ein O- oder S-Atom. Beispiele: Thiazolyl, Oxazolyl, Oxadiazolyl.

**6-gliedriges Heteroaryl,** soweit nicht an anderer Stelle anders definiert: Heteroarylgruppe enthaltend ein bis drei bzw. ein bis vier N-Atom(e) als Ringatome. In einer Ausführungsform enthält eine 6-gliedrige Heteroarylgruppe ein bis drei N-Atome. Beispiele: Pyridyl, Pyridazinyl, Pyrimidinyl, Pyrazinyl, Triazinyl, Tetrazinyl.

Halogenierte 3 bis 9-gliedrige Heterocyclylgruppe oder halogenierte 5 bis 10-gliedrige Heteroarylgruppe jeweils soweit nicht anders definiert sind analog wie 3 bis 9-gliedrige Heterocyclylgruppe oder 5 bis 10-gliedrige Heteroarylgruppe definiert, wobei mindestens ein Wasserstoffatom durch ein Halogenatom wie vorstehend genannt ersetzt ist. In einer Ausführungsform sind alle Wasserstoffatome durch Halogen ersetzt. Beispiel für halogenierte heterocyclische Strukturen: 3-Chlortetrahydrothiopyran-2-yl, 4-Chlorpyridin-2-yl.

Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

### Ausführungsformen der Verbindungen

Dem Fachmann ist offenbar, dass alle Ausführungsformen alleine oder in Kombination vorliegen können.

Die Verbindungen der Formel (I) können gegebenenfalls in Abhängigkeit der Art der Substituenten als Salze, Tautomere, geometrische und/oder optisch aktive Isomere oder entsprechende Isomerengemische in unterschiedlicher Zusammensetzung vorliegen.

Die Verbindungen können gegebenenfalls in verschiedenen polymorphen Formen oder als Mischung verschiedener polymorpher Formen vorliegen. Sowohl die reinen Polymorphe als auch die Polymorphgemische können verwendet werden.

Im Folgenden werden Ausführungsformen der Verbindungen der Formel (I) näher beschrieben:
- X: steht besonders bevorzugt für ein ein- oder mehrfach durch M¹ substituiertes Phenyl, 2-Thienyl, 3-Thienyl, 2-Furanyl oder 3-Furanyl;
- X: steht besonders bevorzugt für ein ein- bis dreifach durch M¹ substituiertes Phenyl oder 2-Thienyl, 3-Thienyl, 2-Furanyl, 3-Furanyl;
- X: steht besonders bevorzugt für ein einfach durch M¹ substituiertes Phenyl;
- X: steht besonders bevorzugt für ein einfach durch M¹ substituiertes Thienyl;
- X: steht besonders bevorzugt für ein einfach durch M¹ substituiertes Furanyl;
- X: steht besonders bevorzugt für ein zweifach durch M¹ substituiertes Phenyl;
- X: steht besonders bevorzugt für ein zweifach durch M¹ substituiertes Thienyl;
- X: steht besonders bevorzugt für ein zweifach durch M¹ substituiertes Furanyl;
- Q: steht für ein ein- oder mehrfach durch M¹ substituiertes Pyridyl, Thienyl, Furanyl oder Isothiazolyl;
- Q: steht bevorzugt für folgende Strukturelemente, wobei n für jedes Q jeweils wie unten angegeben definiert ist: 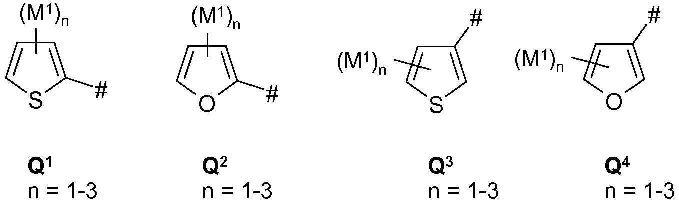 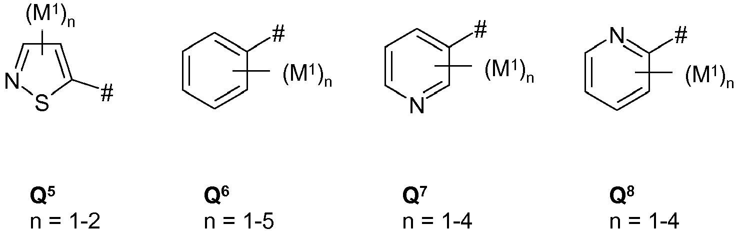
- Q: steht bevorzugt für folgende Strukturelemente, wobei n für jedes Q jeweils wie unten angegeben definiert ist: 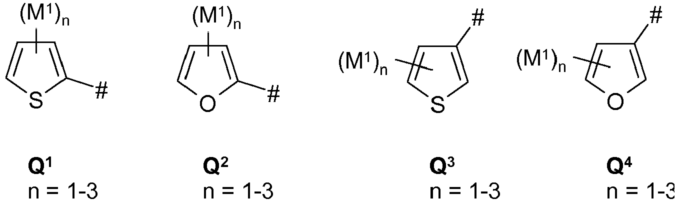 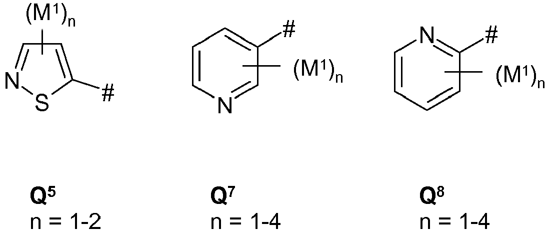
- Q: steht besonders bevorzugt für ein- oder mehrfach durch M¹ substituiertes 2-Thienyl, 3- Thienyl, 2-Furanyl, 5-Isothiazolyl, Phenyl, 3-Pyridyl oder 2-Pyridyl;
- Q: steht besonders bevorzugt für ein- oder zweifach durch M¹ substituiertes 2-Thienyl, 3- Thienyl, 2-Furanyl, 5-Isothiazolyl, Phenyl, 3-Pyridyl oder 2-Pyridyl;
- Q: steht ganz besonders bevorzugt für 2-Thienyl, 3-Fluor-2-thienyl, 3-Chlor-2-thienyl, 3,4-Dichlor-2-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 3-Thienyl, 2-Fluor-3-thienyl, 2-Chlor-3-thienyl, 2-Brom-3-thienyl, 2-Iod-3-thienyl, 2-Cyano-3-thienyl, 2-Methyl-3-thienyl, 2-(Trifluormethyl)-3-thienyl, 2-Methoxy-3-thienyl, 2-Ethoxy-3-thienyl, 2-Furanyl, 3-Fluor-2-furanyl, 3-Chlor-2-furanyl, 3-Brom-2-furanyl, 3-Iod-2-furanyl, 3-Cyano-2-furanyl, 3-Methyl-2-furanyl, 3-(Trifluormethyl)-2-furanyl, 3-Methoxy-2-furanyl, 3-Ethoxy-2-furanyl, 3-Furanyl, 2-Chlor-3-furanyl, 2-Brom-3-furanyl, 2-Iod-3-furanyl, 2-Cyano-3-furanyl, 2-Methyl-3-furanyl, 2-(Trifluormethyl)-3-furanyl, 2-Methoxy-3-furanyl, 2-Ethoxy-3-furanyl, 4-Chlor-5-isothiazolyl, 3,4-Dichlor-5-isothiazolyl, 2-Fluor-phenyl, 2,6-Difluor-phenyl, 2-Chor-phenyl, 2,6-Dichlor-phenyl, 2-Brom-phenyl, 2-Iod-phenyl, 2-(Difluormethyl)-phenyl, 2-(Trifluormethyl)-phenyl, 2-Chlor-3-pyridyl, 3-Chlor-2-pyridyl oder 2-(Trifluormethyl)-3-pyridyl;
- Q: steht insbesonders ganz besonders bevorzugt für 3-Chlor-2-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 2-Chlor-3-thienyl, 2-Brom-3-thienyl, 2-Iod-3-thienyl, 2-Methyl-3-thienyl, 3-Brom-2-furanyl, 3-Methyl-2-furanyl, 2-Chlor-3-furanyl, 2-Brom-3-furanyl, 2-Iod-3-furanyl, 2-Methyl-3-furanyl, 3,4-Dichlor-5-isothiazolyl, 2-Fluor-phenyl, 2,6-Difluor-phenyl, 2-Chor-phenyl, 2,6-Dichlor-phenyl, 2-Brom-phenyl, 2-Iod-phenyl, 2-(Difluormethyl)-phenyl, 2-(Trifluormethyl)-phenyl, 2-Chlor-3-pyridyl, 3-Chlor-2-pyridyl oder 2-(Trifluormethyl)-3 -pyridyl;
- Q: steht besonders bevorzugt für ein- oder mehrfach durch M¹ substituiertes 2-Thienyl, 3- Thienyl, 2-Furanyl, 5-Isothiazolyl, 3-Pyridyl oder 2-Pyridyl;
- Q: steht besonders bevorzugt für ein- oder zweifach durch M¹ substituiertes 2-Thienyl, 3- Thienyl, 2-Furanyl, 5-Isothiazolyl, 3-Pyridyl oder 2-Pyridyl;
- Q: steht ganz besonders bevorzugt für 2-Thienyl, 3-Fluor-2-thienyl, 3-Chlor-2-thienyl, 3,4-Dichlor-2-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 3-Thienyl, 2-Fluor-3-thienyl, 2-Chlor-3-thienyl, 2-Brom-3-thienyl, 2-Iod-3-thienyl, 2-Cyano-3-thienyl, 2-Methyl-3-thienyl, 2-(Trifluormethyl)-3-thienyl, 2-Methoxy-3-thienyl, 2-Ethoxy-3-thienyl, 2-Furanyl, 3-Fluor-2-furanyl, 3-Chlor-2-furanyl, 3-Brom-2-furanyl, 3-Iod-2-furanyl, 3-Cyano-2-furanyl, 3-Methyl-2-furanyl, 3-(Trifluormethyl)-2-furanyl, 3-Methoxy-2-furanyl, 3-Ethoxy-2-furanyl, 3-Furanyl, 2-Chlor-3-furanyl, 2-Brom-3-furanyl, 2-Iod-3-furanyl, 2-Cyano-3-furanyl, 2-Methyl-3-furanyl, 2-(Trifluormethyl)-3-furanyl, 2-Methoxy-3-furanyl, 2-Ethoxy-3-furanyl, 4-Chlor-5-isothiazolyl, 3,4-Dichlor-5-isothiazolyl, 2-Chlor-3-pyridyl, 3-Chlor-2-pyridyl oder 2-(Trifluormethyl)-3 -pyridyl;
- Q: steht insbesonders ganz besonders bevorzugt für 3-Chlor-2-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 2-Chlor-3-thienyl, 2-Brom-3-thienyl, 2-Iod-3-thienyl, 2-Methyl-3-thienyl, 3-Brom-2-furanyl, 3-Methyl-2-furanyl, 2-Chlor-3-furanyl, 2-Brom-3-furanyl, 2-Iod-3-furanyl, 2-Methyl-3-furanyl, 3,4-Dichlor-5-isothiazolyl, 2-Chlor-3-pyridyl, 3-Chlor-2-pyridyl oder 2-(Trifluormethyl)-3-pyridyl;
- Y: steht bevorzugt für Wasserstoff oder für gegebenenfalls ein- oder mehrfach durch M² substituiertes (C₁-C₁₀)-Alkyl, (C₂-C₁₀)-Alkenyl, (C₂-C₁₀)-Alkinyl, (C₁-C₁₀)-Halogenalkyl, (C₂-C₁₀)-Halogenalkenyl, (C₂-C₁₀)-Halogenalkinyl, (C₁-C₁₀)-Alkoxy, (C₂-C₁₀)-Alkenyloxy, (C₃-C₁₀)-Alkinyloxy, (C₃-C₁₄)-Cycloalkyl-(C₁-C₁₀)-alkyl oder für eine gegebenenfalls ein- oder mehrfach durch M² substituierte 3- bis 14-gliedrige cyclische Gruppe;
- Y: steht bevorzugt für Wasserstoff oder für gegebenenfalls ein- oder mehrfach durch M² substituiertes (C₁-C₄)-Alkyl, (C₂-C₄)-Alkenyl, (C₃-C₄)-Alkinyl, (C₁-C₄)-Halogenalkyl, (C₂-C₄)-Halogenalkenyl, (C₃-C₄)-Halogenalkinyl, (C₁-C₄)-Alkoxy, (C₂-C₄)-Alkenyloxy, (C₃-C₄)-Alkinyloxy, (C₃-C₆)-Cycloalkyl-(C₁-C₄)-alkyl oder für eine gegebenenfalls ein- oder mehrfach durch M² substituierte C₃- bis C₆-gliedrige carbocyclische Gruppe;
- Y: steht besonders bevorzugt für Wasserstoff, Methyl, Ethyl, n-Propyl, iso-Propyl, n-Butyl, sec-Butyl, iso-Butyl, tert.-Butyl, Cyanomethyl, 2,2-Difluor-ethyl, 2,2,2-Trifluor-ethyl, Allyl, Butenyl, Propargyl, Butinyl, 3,3-Dichlor-prop-2-enyl, Methoxy, Ethoxy, Cyclopropylmethyl, Cyclopropyl, Cyclobutyl, Cyclopentyl oder Cyclohexyl;
- Y: steht ganz besonders bevorzugt für Wasserstoff, Methyl, Ethyl, n-Propyl, iso-Propyl, n-Butyl, sec-Butyl, iso-Butyl, tert.-Butyl, Methoxy, Ethoxy, Cyclopropylmethyl, Cyclopropyl, Cyclobutyl;
- Y: steht ganz besonders bevorzugt für Wasserstoff, Cyclopropyl;
- W: steht bevorzugt für Sauerstoff;
- W: steht bevorzugt für Schwefel;
- L¹: steht bevorzugt für -C(R¹¹,R¹²)-, Sauerstoff oder -N(R¹)-;
- L¹: steht ganz besonders bevorzugt für -C(R¹¹,R¹²)- oder -N(R¹)-;
- L¹: steht ganz besonders bevorzugt für -C(R¹¹,R¹²)-;
- L¹: steht ganz besonders bevorzugt für Sauerstoff;
- L¹: steht ganz besonders bevorzugt für -N(R¹)-;
- L³: steht bevorzugt für eine Direktbindung;
- L³: steht bevorzugt für -C(R³¹,R³²)-;
- M¹: steht bevorzugt für Wasserstoff, Halogen, Cyano, Nitro, OH, (C₁-C₆)-Alkyl, (C₁-C₆)-Halogenalkyl, (C₁-C₆)-Alkoxy, (C₁-C₆)-Halogenalkoxy, (C₁-C₆)-Alkylthio, (C₁-C₆)-Halogenalkylthio, (C₁-C₆)-Alkylsulfonyl, (C₁-C₆)-Halogenalkylsulfonyl, (C₁-C₆)-Alkylsulfanyl, (C₁-C₆)-Halogenalkylsulfanyl, (C₃-C₁₄)-Cycloalkyl-O-, (C₃-C₁₄)-Cycloalkenyl-O-, (C₆-C₁₄)-Aryl-O-, halogeniertes (C₃-C₁₄)-Cycloalkyl-O-, halogeniertes (C₃-C₁₄)-Cycloalkenyl-O-, halogeniertes (C₆-C₁₄)-Aryl)-O- ;
- M¹: steht bevorzugt für Wasserstoff, Halogen, Cyano, Nitro, OH, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Halogenalkylthio, (C₁-C₄)-Alkylsulfonyl, (C₁-C₄)-Halogenalkylsulfonyl, (C₁-C₄)-Alkylsulfanyl, (C₁-C₆)-Halogenalkylsulfanyl, (C₃-C₁₄)-Cycloalkyl-O-, (C₃-C₁₄)-Cycloalkenyl-O-, (C₆-C₁₄)-Aryl-O-, halogeniertes (C₃-C₁₄)-Cycloalkyl-O-, halogeniertes (C₃-C₁₄)-Cycloalkenyl-O-, halogeniertes (C₆-C₁₄)-Aryl)-O- ;
- M¹: steht ganz besonders bevorzugt für Wasserstoff, Halogen, Cyano, Nitro, OH, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Halogenalkylthio, (C₁-C₄)-Alkylsulfonyl, (C₁-C₄)-Halogenalkylsulfonyl, (C₁-C₄)-Alkylsulfanyl, (C₁-C₆)-Halogenalkylsulfanyl, (C₆-C₁₄)-Aryl-O-, halogeniertes (C₆-C₁₄)-Aryl)-O-;
- M¹: steht ganz besonders bevorzugt für Wasserstoff, Fluor, Brom, Chlor, Iod, Cyano, Nitro, OH, Methyl, Ethyl, iso-Propyl, tert.-Butyl, Trifluormethyl, Difluormethyl, Methoxy, Ethoxy, iso-Propoxy, Trifluormethoxy, Difluormethoxy, Methylthio, Trifluormethylthio, Difluormethylthio, 2,2,2-Trifluorethylthio, Methylsulfonyl, Ethylsulfonyl, Trifluormethylsulfonyl, 2,2,2-Trifluorethylsulfonyl, Methylsulfanyl, Ethylsulfanyl, Trifluormethylsulfanyl, 2,2,2-Trifluorethylsulfanyl oder Phenoxy;
- M¹: steht insbesonders ganz besonders bevorzugt für Wasserstoff, Fluor, Brom, Chlor, Iod, Cyano, Methyl, Trifluormethyl, Difluormethyl, Methoxy, Ethoxy, iso-Propoxy oder Phenoxy;
- R¹: steht bevorzugt für Wasserstoff oder gegebenenfalls ein- oder mehrfach durch M² substituiertes (C₁-C₆)-Alkyl, (C₂-C₆)-Alkenyl, (C₂-C₆)-Alkinyl, (C₁-C₆)-Halogenalkyl, (C₂-C₆)-Halogenalkenyl, (C₂-C₆)-Halogenalkinyl, (C₃-C₆)-Cycloalkyl-(C₁-C₆)-alkyl oder für eine gegebenenfalls ein- oder mehrfach durch M² substituierte (C₃-C₁₄)- carbocyclische Gruppe;
- R¹: steht besonders bevorzugt für Wasserstoff oder gegebenenfalls ein- oder mehrfach durch M² substituiertes (C₁-C₄)-Alkyl, (C₂-C₄)-Alkenyl, (C₂-C₄)-Alkinyl, (C₁-C₄)-Halogenalkyl, (C₂-C₄)-Halogenalkenyl, (C₂-C₄)-Halogenalkinyl, (C₃-C₄)-Cycloalkyl-(C₁-C₁₀)-alkyl oder für eine gegebenenfalls ein- oder mehrfach durch M² substituierte (C₃-C₈)-Cycloalkyl oder halogeniertes (C₃-C₈)-Cycloalkyl;
- R¹: steht besonders bevorzugt für Wasserstoff oder Methyl, Ethyl, n-Propyl, iso-Propyl, Allyl, Propargyl, Cyclopropylmethyl oder Cyclopropyl;
- R¹: steht ganz besonders bevorzugt für Wasserstoff oder (C₁-C₄)-Alkyl;
- R¹: steht besonders bevorzugt für Wasserstoff oder Methyl, Ethyl, n-Propyl, iso-Propyl;
- R¹¹, R¹²: steht bevorzugt für jeweils unabhängig voneinander Wasserstoff, Fluor oder gegebenenfalls ein- oder mehrfach durch M² substituiertes (C₁-C₁₀)-Alkyl, (C₁-C₁₀)-Halogenalkyl, (C₁-C₁₀)-Alkoxy, (C₃-C₁₄)-Cycloalkyl-(C₁-C₁₀)-alkyl oder für eine gegebenenfalls ein- oder mehrfach durch M² substituierte (C₃-C₁₄)- carbocyclische Gruppe;
- R¹¹, R¹²: steht besonders bevorzugt für jeweils unabhängig voneinander Wasserstoff, Fluor oder gegebenenfalls ein- oder mehrfach durch M² substituiertes (C₁-C₆)-Alkyl, (C₁-C₆)-Halogenalkyl, (C₁-C₆)-Alkoxy, (C₃-C₆)-Cycloalkyl-(C₁-C₆)-alkyl, (C₃-C₈)-Cycloalkyl oder halogeniertes (C₃-C₈)-Cycloalkyl;
- R¹¹, R¹²: steht ganz besonders bevorzugt für jeweils unabhängig voneinander Wasserstoff, Fluor oder (C₁-C₆)-Alkyl, (C₁-C₆)-Halogenalkyl, (C₁-C₆)-Alkoxy, (C₃-C₆)-Cycloalkyl-(C₁-C₆)-alkyl, (C₃-C₆)-Cycloalkyl;
- R¹¹, R¹²: steht ganz besonders bevorzugt für jeweils unabhängig voneinander Wasserstoff, Fluor, Methyl, Ethyl, n-Propyl, iso-Propyl, Trifluormethyl, Allyl, Propargyl, Methoxy, Ethoxy, Allyloxy, Propargyloxy, Cyclopropylmethyl, Cyclopropyl;
- R¹¹, R¹²: steht ganz besonders bevorzugt für jeweils unabhängig voneinander Wasserstoff, Fluor, Methyl, Ethyl, n-Propyl, iso-Propyl, Allyl, Propargyl, Methoxy, Ethoxy, Allyloxy, Propargyloxy, Cyclopropylmethyl, Cyclopropyl;
- R¹¹, R¹²: steht ganz besonders bevorzugt für jeweils unabhängig voneinander Wasserstoff, Fluor, Methyl, Ethyl, n-Propyl, iso-Propyl, Allyl, Propargyl, Methoxy, Ethoxy, Allyloxy, Propargyloxy, Cyclopropylmethyl, Cyclopropyl;R¹¹, R¹² steht ganz besonders bevorzugt für jeweils unabhängig voneinander Wasserstoff, Fluor, Methyl, Ethyl, n-Propyl, iso-Propyl, Methoxy, Ethoxy oder
- R¹¹, R¹²: steht ganz besonders bevorzugt für C(R¹¹, R¹²) als spiro-C(CH₂-CH₂);
- R¹¹, R¹²: steht insbesonders ganz besonders bevorzugt für jeweils unabhängig voneinander Wasserstoff, (C₁-C₆)-Alkoxy oder (C₁-C₆)-Alkyl;
- R¹¹, R¹²: steht insbesonders ganz besonders bevorzugt für jeweils unabhängig voneinander Wasserstoff, Halogen, (C₁-C₆)-Halogenalkyl, (C₁-C₆)-Alkoxy oder (C₁-C₆)-Alkyl;R¹¹, R¹² steht insbesonders ganz besonders bevorzugt für jeweils unabhängig voneinander Wasserstoff, Methoxy, Methyl, Ethyl;
- R¹¹, R¹²: steht insbesonders ganz besonders bevorzugt für jeweils unabhängig voneinander Wasserstoff, Methoxy, Methyl, Ethyl, Trifluormethyl, Fluor;
- R²¹, R²²: steht bevorzugt für jeweils unabhängig voneinander Wasserstoff, Fluor oder gegebenenfalls ein- oder mehrfach durch M² substituiertes (C₁-C₆)-Alkyl, (C₂-C₆)-Alkenyl, (C₂-C₆)-Alkinyl, (C₁-C₆)-Halogenalkyl, (C₂-C₆)-Halogenalkenyl, (C₂-C₆)-Halogenalkinyl, (C₁-C₆)-Alkoxy, (C₁-C₆)-Halogenalkoxy, (C₂-C₆)-Alkenyloxy, (C₃-C₆)-Alkinyloxy, (C₃-C₆)-Cycloalkyl-(C₁-C₆)-alkyl oder für eine gegebenenfalls ein- oder mehrfach durch M² substituierte (C₃-C₁₄)- carbocyclische Gruppe;
- R²¹, R²²: steht bevorzugt für jeweils unabhängig voneinander Wasserstoff, Fluor oder gegebenenfalls ein- oder mehrfach durch M² substituiertes (C₁-C₄)-Alkyl, (C₂-C₄)-Alkenyl, (C₂-C₄)-Alkinyl, (C₁-C₄)-Halogenalkyl, (C₂-C₄)-Halogenalkenyl, (C₂-C₄)-Halogenalkinyl, (C₁-C₆)-Alkoxy, (C₁-C₄)-Halogenalkoxy, (C₂-C₄)-Alkenyloxy, (C₃-C₄)-Alkinyloxy, (C₃-C₄)-Cycloalkyl-(C₁-C₄)-alkyl, (C₃-C₈)-Cycloalkyl oder halogeniertes (C₃-C₈)-Cycloalkyl;
- R²¹, R²²: steht besonders bevorzugt für jeweils unabhängig voneinander Wasserstoff, Fluor oder gegebenenfalls ein- oder mehrfach durch M² substituiertes (C₁-C₄)-Alkyl, (C₂-C₄)-Alkenyl, (C₂-C₄)-Alkinyl, (C₁-C₄)-Halogenalkyl, (C₁-C₆)-Alkoxy, (C₂-C₄)-Alkenyloxy, (C₃-C₄)-Alkinyloxy, (C₃-C₄)-Cycloalkyl-(C₁-C₄)-alkyl, (C₃-C₆)-Cycloalkyl;
- R²¹, R²²: steht bevorzugt für jeweils unabhängig voneinander Wasserstoff, Fluor, Methyl, Ethyl, n-Propyl, iso-Propyl, Allyl, Propargyl, Methoxy, Ethoxy, Allyloxy, Propargyloxy, Cyclopropylmethyl, Cyclopropyl ;
- R²¹, R²²: steht ganz besonders bevorzugt für jeweils unabhängig voneinander Wasserstoff, Fluor oder (C₁-C₄)-Alkyl, (C₁-C₄)-Halogenalkyl;
- R²¹, R²²: steht insbesonders ganz besonders bevorzugt für jeweils unabhängig voneinander Wasserstoff, Fluor oder Methyl, Ethyl;
- R²¹, R²²: steht ganz besonders bevorzugt für C(R²¹, R²²) als spiro-C(CH₂-CH₂);
- R³¹, R³²: steht bevorzugt für jeweils unabhängig voneinander Wasserstoff oder gegebenenfalls ein- oder mehrfach durch M² substituiertes (C₁-C₆)-Alkyl, (C₂-C₆)-Alkenyl, (C₂-C₆)-Alkinyl, (C₁-C₆)-Halogenalkyl, (C₂-C₆)-Halogenalkenyl, (C₂-C₆)-Halogenalkinyl, (C₁-C₆)-Alkoxy, (C₁-C₆)-Halogenalkoxy, (C₂-C₆)-Alkenyloxy, (C₃-C₆)-Alkinyloxy, (C₃-C₆)-Cycloalkyl-(C₁-C₆)-alkyl oder für eine gegebenenfalls ein- oder mehrfach durch M² substituierte (C₃-C₁₄)- carbocyclische Gruppe;
- R³¹, R³²: steht bevorzugt für jeweils unabhängig voneinander Wasserstoff oder gegebenenfalls ein- oder mehrfach durch M² substituiertes (C₁-C₄)-Alkyl, (C₂-C₄)-Alkenyl, (C₂-C₄)-Alkinyl, (C₁-C₄)-Halogenalkyl, (C₂-C₄)-Halogenalkenyl, (C₂-C₄)-Halogenalkinyl, (C₁-C₆)-Alkoxy, (C₁-C₄)-Halogenalkoxy, (C₂-C₄)-Alkenyloxy, (C₃-C₄)-Alkinyloxy, (C₃-C₄)-Cycloalkyl-(C₁-C₄)-alkyl oder für eine gegebenenfalls ein- oder mehrfach durch M² substituierte (C₃-C₈)-Cycloalkyl oder halogeniertes (C₃-C₈)-Cycloalkyl;
- R³¹, R³²: steht besonders bevorzugt für jeweils unabhängig voneinander Wasserstoff oder gegebenenfalls ein- oder mehrfach durch M² substituiertes (C₁-C₄)-Alkyl, (C₁-C₄)-Halogenalkyl, (C₂-C₄)-Halogenalkenyl, (C₂-C₄)-Alkenyl, (C₂-C₄)-Alkinyl, (C₁-C₆)-Alkoxy, (C₂-C₄)-Alkenyloxy, (C₃-C₄)-Alkinyloxy, (C₃-C₄)-Cycloalkyl-(C₁-C₄)-alkyl, (C₃-C₆)-Cycloalkyl;
- R³¹, R³²: steht besonders bevorzugt für jeweils unabhängig voneinander Wasserstoff, Methyl, Ethyl, n-Propyl, iso-Propyl, tert-Butyl, Allyl, Propargyl, Methoxy, Ethoxy, Allyloxy, Propargyloxy, Cyclopropylmethyl, Cyclopropyl;
- R³¹, R³²: steht ganz besonders bevorzugt für jeweils unabhängig voneinander Wasserstoff oder (C₁-C₄)-Alkyl;
- R³¹, R³²: steht insbesonders ganz besonders bevorzugt für jeweils unabhängig voneinander Wasserstoff, Methyl, Ethyl, n-Propyl, iso-Propyl oder tert-Butyl;
- R³¹, R³²: steht ganz besonders bevorzugt für C(R³¹, R³²) als spiro-C(CH₂-CH₂);
- M²: steht bevorzugt jeweils unabhängig voneinander für Halogen, Formyl, Cyano, Nitro, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₁-C₆)-Alkoxy, (C₁-C₆)-Halogenalkoxy, (C_{uG}-C_{oG})-Alkenyloxy, (C₂-C₆)-Halogenalkenoxy, (C₃-C₆)-Alkinyloxy, (C₃-C₆)-Halogenalkinoxy, (C₁-C₆)-Alkylthio, (C₁-C₆)-Halogenalkylthio, (C₂-C₆)-Alkenylthio, (C₂-C₆)-Halogenalkenylthio, (C₃-C₆)-Alkinylthio, (C₃-C₆)-Halogenalkinylthio, (C₁-C₆)-Alkylsulfonyl, (C₁-C₆)-Halogenalkylsulfonyl, (C₂-C₆)-Alkenylsulfonyl, (C₂-C₆)-Halogenalkenylsulfonyl, (C₃-C₆)-Alkinylsulfonyl, (C₃-C₆)-Halogenalkinylsulfonyl, (C₁-C₆)-Alkylsulfanyl, (C₁-C₆)-Halogenalkylsulfanyl, (C₂-C₆)-Alkenylsulfanyl, (C₂-C₆)-Halogenalkenylsulfanyl, (C₃-C₆)-Alkinylsulfanyl, (C₃-C₆)-Halogenalkinylsulfanyl, Formyl, (C₁-C₆)-Alkylcarbonyl, (C₁-C₆)-Halogenalkylcarbonyl, (C₂-C₆)-Al-kenylcarbonyl, (C₂-C₆)-Halogenalkenylcarbonyl, (C₂-C₆)-Alkinylcarbonyl, (C₂-C₆)-Halogenalkinylcarbonyl, (C₁-C₆)-Alkoxycarbonyl, (C₁-C₆)-Halogenalkoxycarbonyl, (C₂-C₆)-Alkenoxycarbonyl, (C₂-C₆)-Halogenalkenoxycarbonyl, (C₃-C₆)-Alkinoxycarbonyl, (C₃-C₆)-Halogen-alkinoxycarbonyl, (C₁-C₆)-Alkylcarbonyloxy, (C₁-C₆)-Halogenalkylcarbonyloxy, (C₂-C₆)-Alkenylcarbonyloxy, (C₂-C₆)-Halogenalkenylcarbonyloxy, (C₂-C₆)-Alkinylcarbonyloxy, (C₂-C₆)-Halogenalkinylcarbonyloxy oder (C₃-C₁₄)-Cycloalkyl.
- M²: steht besonders bevorzugt jeweils unabhängig voneinander für Halogen, Formyl, Cyano, Nitro, (C₁-C₄)-Alkyl, (C₁-C₄)-Halogenalkyl, (C₂-C₄)-Alkenyl, (C₂-C₄)-Halogenalkenyl, (C₂-C₄)-Alkinyl, (C₂-C₄)-Halogenalkinyl, (C₁-C₄)-Alkoxy, (C₁-C₄)-Halogenalkoxy, (C₂-C₄)-Alkenyloxy, (C₂-C₄)-Halogenalkenoxy, (C₃-C₄)-Alkinyloxy, (C₃-C₄)-Halogenalkinoxy, (C₁-C₄)-Alkylthio, (C₁-C₄)-Halogenalkylthio, (C₂-C₄)-Alkenylthio, (C₂-C₄)-Halogenalkenylthio, (C₃-C₄)-Alkinylthio, (C₃-C₄)-Halogenalkinylthio, (C₁-C₄)-Alkylsulfonyl, (C₁-C₄)-Halogenalkylsulfonyl, (C₂-C₄)-Alkenylsulfonyl, (C₂-C₄)-Halogenalkenylsulfonyl, (C₃-C₄)-Alkinylsulfonyl, (C₃-C₄)-Halogenalkinylsulfonyl, (C₁-C₄)-Alkylsulfanyl, (C₁-C₄)-Halogenalkylsulfanyl, (C₂-C₄)-Alkenylsulfanyl, (C₂-C₄)-Halogenalkenylsulfanyl, (C₃-C₄)-Alkinylsulfanyl, (C₃-C₄)-Halogenalkinylsulfanyl, (C₁-C₄)-Alkylcarbonyl, (C₁-C₄)-Halogenalkylcarbonyl, (C₂-C₄)-Alkenylcarbonyl, (C₂-C₄)-Halogenalkenylcarbonyl, (C₂-C₄)-Alkinylcarbonyl, (C₂-C₄)-Halogenalkinylcarbonyl, (C₁-C₄)-Alkoxycarbonyl, (C₁-C₄)-Halogenalkoxycarbonyl, (C₂-C₄)-Alkenoxycarbonyl, (C₂-C₄)-Halogenalkenoxycarbonyl, (C₃-C₄)-Alkinoxycarbonyl, (C₃-C₄)-Halogenalkinoxycarbonyl, (C₁-C₄)-Alkylcarbonyloxy, (C₁-C₄)-Halogenalkylcarbonyloxy, (C₂-C₄)-Alkenylcarbonyloxy, (C₂-C₄)-Halo-genalkenylcarbonyloxy, (C₂-C₄)-Alkinylcarbonyloxy, (C₂-C₄)-Halogenalkinylcarbonyloxy oder (C₃-C₆)-Cycloalkyl.
- M²: steht ganz besonders bevorzugt jeweils unabhängig voneinander für Chlor, Fluor, Formyl, Cyano, Nitro, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Halogenalkylthio, (C₁-C₄)-Alkylsulfonyl, (C₁-C₄)-Halogenalkylsulfonyl, (C₁-C₄)-Alkylsulfanyl, (C₁-C₄)-Halogenalkylsulfanyl, (C₁-C₄)-Alkylcarbonyl, (C₁-C₄)-Halogenalkylcarbonyl oder (C₃-C₆)-Cycloalkyl.
- M²: steht ganz besonders bevorzugt jeweils unabhängig voneinander für Fluor, Brom, Chlor, Iod, Cyano, Nitro, Methyl, Ethyl, iso-Propyl, tert.-Butyl, Trifluormethyl, Difluormethyl, Methoxy, Ethoxy, iso-Propoxy, Trifluormethoxy, Difluormethoxy, Methylthio, Trifluormethylthio, Difluormethylthio, 2,2,2-Trifluorethylthio, Methylsulfonyl, Ethylsulfonyl, Trifluormethylsulfonyl, 2,2,2-Trifluorethylsulfonyl, Methylsulfanyl, Ethylsulfanyl, Trifluormethylsulfanyl, 2,2,2-Trifluorethylsulfanyl, Cyclopropyl, Cyclobutyl oder Cyclopentyl.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Die genannten Definitionen können untereinander in beliebiger Weise kombiniert werden. Außerdem können auch einzelne Definitionen entfallen.

Bevorzugt, besonders bevorzugt oder ganz besonders bevorzugt sind Verbindungen der Formel (I), welche jeweils die unter bevorzugt, besonders bevorzugt, ganz besonders bevorzugt oder insbesonders ganz besonders bevorzugt genannten Substituenten tragen.

In einer Ausführungsform sind Verbindungen der Formel (I) Verbindungen 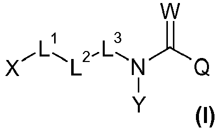 in welcher L¹, L², L³, Q, W und Y wie oben definiert sind und
- X: für Phenyl steht, bevorzugt ein- oder mehrfach substituiert durch M¹ ausgewählt aus Wasserstoff, Halogen, Cyano, Nitro, OH, (C₁-C₄)-Alkyl, (C₁-C₃)-Halogenalkyl, (C₁-C₄)-Alkoxy, (C₁-C₃)-Halogenalkoxy, (C₁-C₄)-Alkylthio, (C₁-C₃)-Halogenalkylthio, (C₁-C₄)-Alkylsulfonyl, (C₁-C₃)-Halogenalkylsulfonyl, (C₁-C₄)-Alkylsulfanyl, (C₁-C₃)-Halogenalkylsulfanyl oder Phenoxy;
- X: für Phenyl steht, besonders bevorzugt ein- oder mehrfach substituiert durch M¹ ausgewählt aus Wasserstoff, Fluor, Brom, Chlor, Iod, Cyano, Nitro, OH, Methyl, Ethyl, iso-Propyl, tert.-Butyl, Trifluormethyl, Difluormethyl, Methoxy, Ethoxy, iso-Propoxy, Trifluormethoxy, Difluormethoxy, Methylthio, Trifluormethylthio, Difluormethylthio, 2,2,2-Trifluorethylthio, Methylsulfonyl, Ethylsulfonyl, Trifluormethylsulfonyl, 2,2,2-Trifluorethylsulfonyl, Methylsulfanyl, Ethylsulfanyl, Trifluormethylsulfanyl, 2,2,2-Trifluorethylsulfanyl oder Phenoxy;
- X: für 2-Thienyl steht, bevorzugt ein- oder mehrfach substituiert durch M¹ ausgewählt aus Wasserstoff, Halogen, Cyano, Nitro, OH, gegebenenfalls substituiertes (C₁-C₄)-Alkyl, (C₁-C₃)-Halogenalkyl, (C₁-C₄)-Alkoxy oder (C₁-C₃)-Halogenalkoxy;
- X: für 2-Thienyl steht, besonders bevorzugt ein- oder mehrfach substituiert durch M¹ ausgewählt aus Wasserstoff, Fluor, Chlor, Brom, Iod, Methyl oder Trifluormethyl;
- X: für 3-Thienyl steht, bevorzugt ein- oder mehrfach substituiert durch M¹ ausgewählt aus Wasserstoff, Halogen, Cyano, Nitro, OH, gegebenenfalls substituiertes (C₁-C₄)-Alkyl, (C₁-C₃)-Halogenalkyl, (C₁-C₄)-Alkoxy oder (C₁-C₃)-Halogenalkoxy;
- X: für 3-Thienyl steht, besonders bevorzugt ein- oder mehrfach substituiert durch M¹ ausgewählt aus Wasserstoff, Fluor, Chlor, Brom, Iod, Methyl oder Trifluormethyl;
- X: für 2-Furanyl steht, bevorzugt ein- oder mehrfach substituiert durch M¹ ausgewählt aus Wasserstoff, Halogen, Cyano, Nitro, OH, gegebenenfalls substituiertes (C₁-C₄)-Alkyl, (C₁-C₃)-Halogenalkyl, (C₁-C₄)-Alkoxy oder (C₁-C₃)-Halogenalkoxy;
- X: für 2-Furanyl steht, besonders bevorzugt ein- oder mehrfach substituiert durch M¹ ausgewählt aus Wasserstoff, Fluor, Chlor, Brom, Iod, Methyl oder Trifluormethyl.
- X: für 3-Furanyl steht, bevorzugt ein- oder mehrfach substituiert durch M¹ ausgewählt aus Wasserstoff, Halogen, Cyano, Nitro, OH, gegebenenfalls substituiertes (C₁-C₄)-Alkyl, (C₁-C₃)-Halogenalkyl, (C₁-C₄)-Alkoxy oder (C₁-C₃)-Halogenalkoxy;
- X: für 3-Furanyl steht, besonders bevorzugt ein- oder mehrfach substituiert durch M¹ ausgewählt aus Wasserstoff, Fluor, Chlor, Brom, Iod, Methyl oder Trifluormethyl;

In einer weiteren Ausführungsform sind Verbindungen der Formel (I) Verbindungen 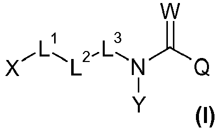 in welcher L¹, L², L³, W, X und Y wie oben definiert sind und
- Q: bevorzugt für folgende Strukturelemente steht, die ein- oder mehrfach durch M¹ ausgewählt aus Wasserstoff, Halogen, Cyano, gegebenenfalls substituiertes (C₁-C₄)-Alkyl, (C₁-C₃)-Halogenalkyl oder (C₁-C₄)-Alkoxy substituiert sein können: 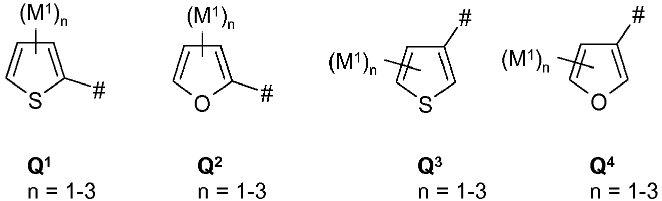 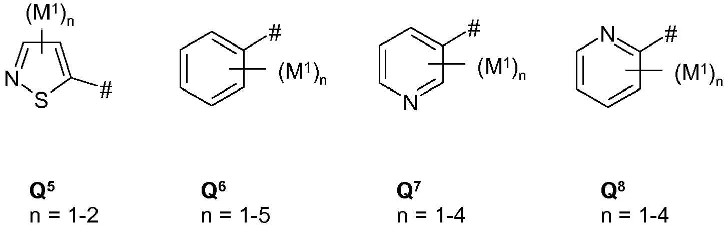
- Q: besonders bevorzugt für 2-Thienyl, 3-Fluor-2-thienyl, 3-Chlor-2-thienyl, 3,4-Dichlor-2-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 3-Thienyl, 2-Fluor-3-thienyl, 2-Chlor-3-thienyl, 2-Brom-3-thienyl, 2-Iod-3-thienyl, 2-Cyano-3-thienyl, 2-Methyl-3-thienyl, 2-(Trifluormethyl)-3-thienyl, 2-Methoxy-3-thienyl, 2-Ethoxy-3-thienyl, 2-Furanyl, 3-Fluor-2-furanyl, 3-Chlor-2-furanyl, 3-Brom-2-furanyl, 3-Iod-2-furanyl, 3-Cyano-2-furanyl, 3-Methyl-2-furanyl, 3-(Trifluormethyl)-2-furanyl, 3-Methoxy-2-furanyl, 3-Ethoxy-2-furanyl, 3-Furanyl, 2-Chlor-3-furanyl, 2-Brom-3-furanyl, 2-Iod-3-furanyl, 2-Cyano-3-furanyl, 2-Methyl-3-furanyl, 2-(Trifluormethyl)-3-furanyl, 2-Methoxy-3-furanyl, 2-Ethoxy-3-furanyl, 4-Chlor-5-isothiazolyl, 3,4-Dichlor-5-isothiazolyl, 2-Fluor-phenyl, 2,6-Difluor-phenyl, 2-Chorphenyl, 2,6-Dichlor-phenyl, 2-Brom-phenyl, 2-Iod-phenyl, 2-(Difluormethyl)-phenyl, 2-(Trifluormethyl)-phenyl, 2-Chlor-3-pyridyl, 3-Chlor-2-pyridyl oder 2-(Trifluormethyl)-3-pyridyl steht;
- Q: bevorzugt für folgende Strukturelemente steht, die ein- oder mehrfach durch M¹ ausgewählt aus Wasserstoff, Halogen, Cyano, gegebenenfalls substituiertes (C₁-C₄)-Alkyl, (C₁-C₃)-Halogenalkyl oder (C₁-C₄)-Alkoxy substituiert sein können: 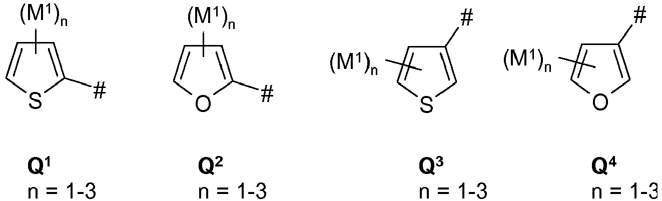 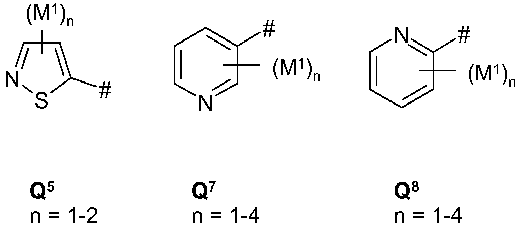
- Q: besonders bevorzugt für 2-Thienyl, 3-Fluor-2-thienyl, 3-Chlor-2-thienyl, 3,4-Dichlor-2-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 3-Thienyl, 2-Fluor-3-thienyl, 2-Chlor-3-thienyl, 2-Brom-3-thienyl, 2-Iod-3-thienyl, 2-Cyano-3-thienyl, 2-Methyl-3-thienyl, 2-(Trifluormethyl)-3-thienyl, 2-Methoxy-3-thienyl, 2-Ethoxy-3-thienyl, 2-Furanyl, 3-Fluor-2-furanyl, 3-Chlor-2-furanyl, 3-Brom-2-furanyl, 3-Iod-2-furanyl, 3-Cyano-2-furanyl, 3-Methyl-2-furanyl, 3-(Trifluormethyl)-2-furanyl, 3-Methoxy-2-furanyl, 3-Ethoxy-2-furanyl, 3-Furanyl, 2-Chlor-3-furanyl, 2-Brom-3-furanyl, 2-Iod-3-furanyl, 2-Cyano-3-furanyl, 2-Methyl-3-furanyl, 2-(Trifluormethyl)-3-furanyl, 2-Methoxy-3-furanyl, 2-Ethoxy-3-furanyl, 4-Chlor-5-isothiazolyl, 3,4-Dichlor-5-isothiazolyl, 2-Chlor-3-pyridyl, 3-Chlor-2-pyridyl oder 2-(Trifluormethyl)-3-pyridyl steht;
sowie Salze, N-Oxide und tautomere Formen der Verbindungen der Formel (I).

In einer weiteren Ausführungsform sind Verbindungen der Formel (I) Verbindungen 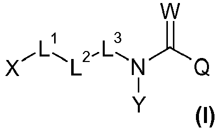 in welcher L¹, L², L³, Q, W und X wie oben definiert sind und
- Y: bevorzugt für Wasserstoff oder für gegebenenfalls ein- oder mehrfach durch M² substituiertes (C₁-C₄)-Alkyl, (C₂-C₄)-Alkenyl, (C₃-C₄)-Alkinyl, (C₁-C₄)-Halogenalkyl, (C₂-C₄)-Halogenalkenyl, (C₃-C₄)-Halogenalkinyl, (C₁-C₄)-Alkoxy, (C₂-C₄)-Alkenyloxy, (C₃-C₄)-Alkinyloxy, (C₃-C₆)-Cycloalkyl-(C₁-C₄)-alkyl oder für eine gegebenenfalls substituierte C₃- bis C₆-gliedrige carbocyclische Gruppe steht;
- Y: besonders bevorzugt für Wasserstoff, Methyl, Ethyl, n-Propyl, iso-Propyl, n-Butyl, sec-Butyl, iso-Butyl, tert.-Butyl, Cyanomethyl, 2,2-Difluor-ethyl, 2,2,2-Trifluor-ethyl, Allyl, Butenyl, Propargyl, Butinyl, 3,3-Dichlor-prop-2-enyl, Methoxy, Ethoxy, Cyclopropylmethyl, Cyclopropyl, Cyclobutyl, Cyclopentyl oder Cyclohexyl steht;
sowie Salze, N-Oxide und tautomere Formen der Verbindungen der Formel (I).

In einer weiteren Ausführungsform sind Verbindungen der Formel (I) Verbindungen 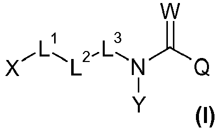 in welcher L¹, L², L³, Q, X und Y wie oben definiert sind und
- W: bevorzugt für Sauerstoff steht;
sowie Salze, N-Oxide und tautomere Formen der Verbindungen der Formel (I).

In einer weiteren Ausführungsform sind Verbindungen der Formel (I) Verbindungen 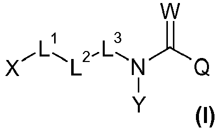 in welcher L², L³, Q, W, X und Y wie oben definiert sind und
- L¹: bevorzugt für C(R¹¹, R¹²) steht, wobei R¹¹ und R¹² jeweils unabhängig voneinander für Wasserstoff, Fluor oder gegebenenfalls ein- oder mehrfach durch M² substituiertes (C₁-C₄)-Alkyl, (C₃-C₄)-Alkenyl, (C₃-C₄)-Alkinyl, (C₁-C₄)-Halogenalkyl, (C₃-C₄)-Halogenalkenyl, (C₃-C₄)-Halogenalkinyl, (C₁-C₄)-Alkoxy, (C₃-C₄)-Alkenyloxy, (C₃-C₄)-Alkinyloxy, (C₃-C₆)-Cycloalkyl-(C₁-C₄)-alkyl oder für eine gegebenenfalls substituierte C₃- bis C₆-gliedrige carbocyclische Gruppe steht, oder wobei R¹¹ und R¹² zusammen für eine gegebenenfalls substituierte spiroverknüpfte 3 bis 6-gliedrige cyclische Gruppe stehen;
- L¹: bevorzugt für Sauerstoff oder Schwefel steht;
- L¹: bevorzugt für -N(R¹) steht, wobei R¹ für Wasserstoff oder gegebenenfalls ein- oder mehrfach durch M² substituiertes (C₁-C₄)-Alkyl, (C₃-C₄)-Alkenyl, (C₃-C₄)-Alkinyl, (C₁-C₄)-Halogenalkyl, (C₃-C₄)-Halogenalkenyl, (C₃-C₄)-Halogenalkinyl, (C₃-C₆)-Cycloalkyl-(C₁-C₄)-alkyl oder für eine gegebenenfalls substituierte C₃- bis C₆-gliedrige carbocyclische Gruppe steht;
- L¹: besonders bevorzugt für C(R¹¹, R¹²) steht, wobei R¹¹ und R¹² jeweils unabhängig voneinander für Wasserstoff, Fluor, Methyl, Ethyl, n-Propyl, iso-Propyl, Allyl, Propargyl, Methoxy, Ethoxy, Allyloxy, Propargyloxy, Cyclopropylmethyl, Cyclopropyl oder wobei C(R¹¹, R¹²) für spiro-C(CH₂-CH₂) steht;
- L¹: besonders bevorzugt für Sauerstoff steht;
sowie Salze, N-Oxide und tautomere Formen der Verbindungen der Formel (I).

In einer weiteren Ausführungsform sind Verbindungen der Formel (I) Verbindungen 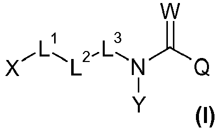 in welcher L², L³, W, X und Y wie oben definiert sind und
- Q: bevorzugt für ein ein- oder mehrfach durch M¹ substituiertes Phenyl, Pyridyl, Thienyl, Furanyl oder Isothiazolyl steht;
- Q: bevorzugt für ein ein- oder mehrfach durch M¹ substituiertes Pyridyl, Thienyl, Furanyl oder Isothiazolyl steht;
- Q: besonders bevorzugt für ein ein- oder mehrfach durch M¹ substituiertes Phenyl steht;
- L¹: bevorzugt für Sauerstoff oder Schwefel steht;
- L¹: bevorzugt für -N(R¹) steht, wobei R¹ für Wasserstoff oder gegebenenfalls ein- oder mehrfach durch M² substituiertes (C₁-C₄)-Alkyl, (C₃-C₄)-Alkenyl, (C₃-C₄)-Alkinyl, (C₁-C₄)-Halogenalkyl, (C₃-C₄)-Halogenalkenyl, (C₃-C₄)-Halogenalkinyl, (C₃-C₆)-Cycloalkyl-(C₁-C₄)-alkyl oder für eine gegebenenfalls substituierte C₃- bis C₆-gliedrige carbocyclische Gruppe steht;
- L¹: besonders bevorzugt für Schwefel steht;L¹ besonders bevorzugt für -N(R¹) steht, wobei R¹ für Wasserstoff, Methyl, Ethyl, n-Propyl, iso-Propyl, Allyl, Propargyl, Cyclopropylmethyl oder Cyclopropyl steht;
- L¹: bevorzugt für -C(R¹¹,R¹²)- steht, wobei R¹¹, R¹² jeweils unabhängig voneinander für Halogen, (C₁-C₆)-Halogenalkyl oder (C₁-C₆)-Alkoxy steht;
- L¹: besonders bevorzugt für -C(R¹¹,R¹²)- steht, wobei R¹¹,R¹² unabhängig voneinander für Fluor, Trifluormethyl, Methoxy steht;
sowie Salze, N-Oxide und tautomere Formen der Verbindungen der Formel (I).

In einer weiteren Ausführungsform sind Verbindungen der Formel (I) Verbindungen 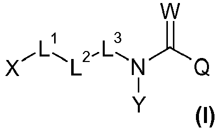 in welcher L¹, L³, Q, W, X und Y wie oben definiert sind und
- L²: bevorzugt für C(R²¹, R²²) steht, wobei R²¹ und R²² jeweils unabhängig voneinander für Wasserstoff, Fluor oder gegebenenfalls ein- oder mehrfach durch M² substituiertes (C₁-C₄)-Alkyl, (C₃-C₄)-Alkenyl, (C₃-C₄)-Alkinyl, (C₁-C₄)-Halogenalkyl, (C₃-C₄)-Halogenalkenyl, (C₃-C₄)-Halogenalkinyl, (C₁-C₄)-Alkoxy, (C₃-C₄)-Alkenyloxy, (C₃-C₄)-Alkinyloxy, (C₃-C₆)-Cycloalkyl-(C₁-C₄)-alkyl oder für eine gegebenenfalls substituierte C₃- bis C₆-gliedrige carbocyclische Gruppe steht, oder wobei R²¹ und R²² zusammen für eine gegebenenfalls substituierte spiroverknüpfte 3 bis 6-gliedrige cyclische Gruppe stehen;
- L²: besonders bevorzugt für C(R²¹, R²²) steht, wobei R²¹ und R²² jeweils unabhängig voneinander für Wasserstoff, Fluor, Methyl, Ethyl, n-Propyl, iso-Propyl, Allyl, Propargyl, Methoxy, Ethoxy, Allyloxy, Propargyloxy, Cyclopropylmethyl, Cyclopropyl oder wobei C(R²¹, R²²) für spiro-C(CH₂-CH₂) steht;
sowie Salze, N-Oxide und tautomere Formen der Verbindungen der Formel (I).

In einer weiteren Ausführungsform sind Verbindungen der Formel (I) Verbindungen 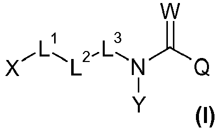 in welcher L¹, L², Q, W, X und Y wie oben definiert sind und
- L³: bevorzugt für C(R³¹, R³²) steht, wobei R³¹ und R³² jeweils unabhängig voneinander für Wasserstoff oder gegebenenfalls ein- oder mehrfach durch M² substituiertes (C₁-C₄)-Alkyl, (C₃-C₄)-Alkenyl, (C₃-C₄)-Alkinyl, (C₁-C₄)-Halogenalkyl, (C₃-C₄)-Halogenalkenyl, (C₃-C₄)-Halogenalkinyl, (C₁-C₄)-Alkoxy, (C₃-C₄)-Alkenyloxy, (C₃-C₄)-Alkinyloxy, (C₃-C₆)-Cycloalkyl-(C₁-C₄)-alkyl oder für eine gegebenenfalls substituierte C₃- bis C₆-gliedrige carbocyclische Gruppe steht, oder wobei R³¹ und R³² zusammen für eine gegebenenfalls substituierte spiroverknüpfte 3 bis 6-gliedrige cyclische Gruppe stehen;
- L³: besonders bevorzugt für C(R³¹, R³²) steht, wobei R³¹ und R³² jeweils unabhängig voneinander für Wasserstoff, Methyl, Ethyl, n-Propyl, iso-Propyl, Allyl, Propargyl, Methoxy, Ethoxy, Allyloxy, Propargyloxy, Cyclopropylmethyl, Cyclopropyl oder wobei C(R³¹, R³²) für spiro-C(CH₂-CH₂) steht;
- L³: bevorzugt und besonders bevorzugt für eine Direktbindung zwischen L² und N-Y steht;
sowie Salze, N-Oxide und tautomere Formen der Verbindungen der Formel (I).

In einer weiteren Ausführungsform sind Verbindungen der Formel (I) Verbindungen 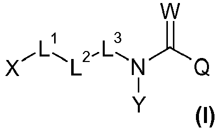 in welcher L¹, L², W, X und Y wie oben definiert sind und
- Q: bevorzugt für ein ein- oder mehrfach durch M¹ substituiertes Phenyl, Pyridyl, Thienyl, Furanyl oder Isothiazolyl steht;
- Q: besonders bevorzugt für ein ein- oder mehrfach durch M¹ substituiertes Phenyl steht;
- Q: ganz besonders bevorzugt für ein ein- oder mehrfach durch M¹ substituiertes Phenyl steht, wobei das Phenyl nicht mit Trifluormethyl in 2-Position substituiert sein kann;
- L³: bevorzugt für C(R³¹, R³²) steht, wobei R³¹ und R³² jeweils unabhängig voneinander für Wasserstoff oder gegebenenfalls ein- oder mehrfach durch M² substituiertes (C₁-C₄)-Alkyl, (C₃-C₄)-Alkenyl, (C₃-C₄)-Alkinyl, (C₁-C₄)-Halogenalkyl, (C₃-C₄)-Halogenalkenyl, (C₃-C₄)-Halogenalkinyl, (C₁-C₄)-Alkoxy, (C₃-C₄)-Alkenyloxy, (C₃-C₄)-Alkinyloxy, (C₃-C₆)-Cycloalkyl-(C₁-C₄)-alkyl oder für eine gegebenenfalls substituierte C₃- bis C₆-gliedrige carbocyclische Gruppe steht, oder wobei R³¹ und R³² zusammen für eine gegebenenfalls substituierte spiroverknüpfte 3 bis 6-gliedrige cyclische Gruppe stehen;
- L³: besonders bevorzugt für C(R³¹, R³²) steht, wobei R³¹ und R³² jeweils unabhängig voneinander für Wasserstoff, Methyl, Ethyl, n-Propyl, iso-Propyl, Allyl, Propargyl, Methoxy, Ethoxy, Allyloxy, Propargyloxy, Cyclopropylmethyl, Cyclopropyl steht;
- L³: besonders bevorzugt für C(R³¹, R³²) steht, wobei R³¹ und R³² jeweils unabhängig voneinander für, Methyl, Ethyl, n-Propyl, iso-Propyl, Allyl, Propargyl, Methoxy, Ethoxy, Allyloxy, Propargyloxy, Cyclopropylmethyl, Cyclopropyl steht;
sowie Salze, N-Oxide und tautomere Formen der Verbindungen der Formel (I).

In einer ganz besonders bevorzugten weiteren Ausführungsform sind Verbindungen der Formel (I) Verbindungen 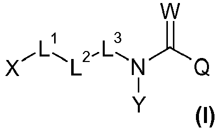 in welcher
- X: für ein- oder mehrfach durch M¹ substituiertes Phenyl steht, wobei M¹ ausgewählt ist aus Wasserstoff, Fluor, Brom, Chlor, Iod, Cyano, Nitro, OH, Methyl, Ethyl, iso-Propyl, tert.-Butyl, Trifluormethyl, Difluormethyl, Methoxy, Ethoxy, iso-Propoxy, Trifluormethoxy, Difluormethoxy, Methylthio, Trifluormethylthio, Difluormethylthio, 2,2,2-Trifluorethylthio, Methylsulfonyl, Ethylsulfonyl, Trifluormethylsulfonyl, 2,2,2-Trifluorethylsulfonyl, Methylsulfanyl, Ethylsulfanyl, Trifluormethylsulfanyl, 2,2,2-Trifluorethylsulfanyl oder Phenoxy ;
- X: für 2-Thienyl steht, ein- oder mehrfach substituiert durch M¹ ausgewählt aus Wasserstoff, Fluor, Chlor, Brom, Iod, Methyl oder Trifluormethyl;
- X: für 3-Thienyl steht, ein- oder mehrfach substituiert durch M¹ ausgewählt aus Wasserstoff, Fluor, Chlor, Brom, Iod, Methyl oder Trifluormethyl;
- X: für 2-Furanyl steht, ein- oder mehrfach substituiert durch M¹ ausgewählt aus Wasserstoff, Fluor, Chlor, Brom, Iod, Methyl oder Trifluormethyl;
- X: für 3-Furanyl steht, ein- oder mehrfach substituiert durch M¹ ausgewählt aus Wasserstoff, Fluor, Chlor, Brom, Iod, Methyl oder Trifluormethyl;
- Q: für 2-Thienyl, 3-Fluor-2-thienyl, 3-Chlor-2-thienyl, 3,4-Dichlor-2-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 3-Thienyl, 2-Fluor-3-thienyl, 2-Chlor-3-thienyl, 2-Brom-3-thienyl, 2-Iod-3-thienyl, 2-Cyano-3-thienyl, 2-Methyl-3-thienyl, 2-(Trifluormethyl)-3-thienyl, 2-Methoxy-3-thienyl, 2-Ethoxy-3-thienyl, 2-Furanyl, 3-Fluor-2-furanyl, 3-Chlor-2-furanyl, 3-Brom-2-furanyl, 3-Iod-2-furanyl, 3-Cyano-2-furanyl, 3-Methyl-2-furanyl, 3-(Trifluormethyl)-2-furanyl, 3-Methoxy-2-furanyl, 3-Ethoxy-2-furanyl, 3-Furanyl, 2-Chlor-3-furanyl, 2-Brom-3-furanyl, 2-Iod-3-furanyl, 2-Cyano-3-furanyl, 2-Methyl-3-furanyl, 2-(Trifluormethyl)-3-furanyl, 2-Methoxy-3-furanyl, 2-Ethoxy-3-furanyl, 4-Chlor-5-isothiazolyl, 3,4-Dichlor-5-isothiazolyl, 2-Fluor-phenyl, 2,6-Difluor-phenyl, 2-Chor-phenyl, 2,6-Dichlor-phenyl, 2-Brom-phenyl, 2-Iod-phenyl, 2-(Difluormethyl)-phenyl, 2-(Trifluormethyl)-phenyl, 2-Chlor-3-pyridyl, 3-Chlor-2-pyridyl oder 2-(Trifluormethyl)-3-pyridyl steht;
- Y: für Wasserstoff, Methyl, Ethyl, n-Propyl, iso-Propyl, n-Butyl, sec-Butyl, iso-Butyl, tert.-Butyl, Cyanomethyl, 2,2-Difluor-ethyl, 2,2,2-Trifluor-ethyl, Allyl, Butenyl, Propargyl, Butinyl, 3,3-Dichlor-prop-2-enyl, Methoxy, Ethoxy, Cyclopropylmethyl, Cyclopropyl, Cyclobutyl, Cyclopentyl oder Cyclohexyl steht;
- W: für Sauerstoff steht;
- L¹: für -N(R¹) steht, wobei R¹ für Wasserstoff, Methyl, Ethyl, n-Propyl, iso-Propyl, Allyl, Propargyl, Cyclopropylmethyl oder Cyclopropyl steht;
- L²: für C(R²¹, R²²) steht, wobei R²¹ und R²² jeweils unabhängig voneinander für Wasserstoff, Fluor, Methyl, Ethyl, n-Propyl, iso-Propyl, Allyl, Propargyl, Methoxy, Ethoxy, Allyloxy, Propargyloxy, Cyclopropylmethyl, Cyclopropyl oder wobei C(R²¹, R²²) für spiro-C(CH₂-CH₂) steht;
- L³: für eine Direktbindung zwischen L² und N-Y steht.

In einer ganz besonders bevorzugten weiteren Ausführungsform sind Verbindungen der Formel (I-b) Verbindungen 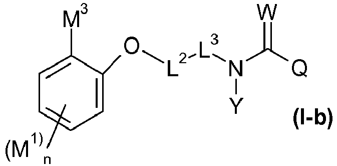 in welcher L², L³, Q, W und Y wie oben definiert sind und
- M¹ und M³: unabhängig voneinander für Halogen, Cyano, Nitro, OH, (C₁-C₁₀)-Alkyl, (C₁-C₁₀)-Halogenalkyl, (C₁-C₁₀)-Alkoxy, (C₁-C₁₀)-Halogenalkoxy, (C₁-C₁₀)-Alkylthio, (C₁-C₁₀)-Halogenalkylthio, (C₁-C₁₀)-Alkylsulfonyl, (C₁-C₁₀)-Halogenalkylsulfonyl, (C₁-C₁₀)-Alkylsulfanyl, (C₁-C₁₀)-Halogenalkylsulfanyl, (3- bis 14-gliedrige cyclische Gruppe)-O-

### Darstellungsverfahren A

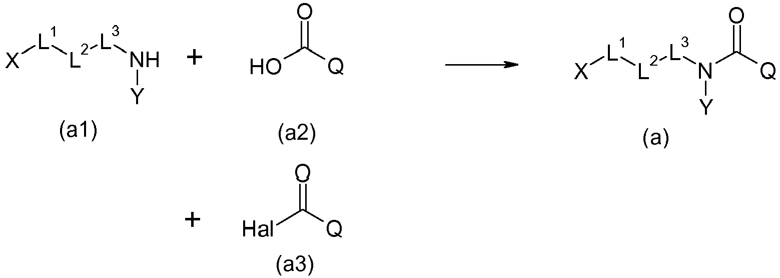

Die Reste X, Y, L¹, L², L³, und Q haben die oben beschriebenen Bedeutungen. W steht in diesem Fall für Sauerstoff.

Die Verbindungen der allgemeinen Formel (a) können ausgehend von Aminen gemäß Formel (a1) und Carbonsäuren gemäß Formel (a2) bzw. deren Halogenide gemäß Formel (a3) nach hinlänglich bekannten Verfahren dargestellt werden, beispielsweise wie in WO-A 2007/060166 beschrieben. Die Amine gemäß Formel (a1) und Carbonsäuren gemäß Formel (a2) sowie deren Halogenide gemäß Formel (a3) sind kommerziell verfügbar.

### Darstellungsverfahren A-1

Die Verbindungen der Formel (I-a) 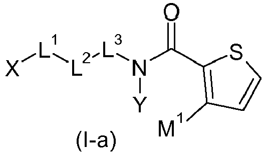 in welcher M¹ für Chlor, Brom oder Jod steht und L¹, L², L³, X und Y wie oben definiert sind, werden hergestellt, indem Amine der Formel (a1) 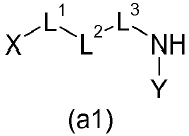 wobei X, Y, L¹, L², L³ die oben beschriebenen Bedeutungen haben,
mit Carbonsäuren der Formel (a2-1) 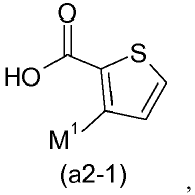 wobei M¹ für Chlor, Brom oder Jod steht, oder deren Säurehalogenide der Formel (a3-1) 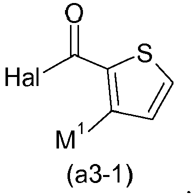 wobei M¹ für Chlor, Brom oder Jod und Hal für Fluor, Chlor oder Brom steht,
zu den Verbindungen der Formel (I-a) umgesetzt werden.

Die Amine gemäß Formel (a1) und Carbonsäuren gemäß Formel (a2-1) sowie deren Halogenide gemäß Formel (a3-1) sind kommerziell verfügbar. Alternativ lassen sich die Halogenide gemäß Formel (a3-1) nach hinlänglich bekannten Methoden aus den Carbonsäuren gemäß Formel (a2-1) mit entsprechenden Halogenierungsreagentien herstellen, beispielsweise mit Phosphorylchlorid, Phosphorylbromid, Thionylchlorid, Oxalylchlorid oder Phosgen.

Die Verbindungen der allgemeinen Formel (I-a) werden beim Einsatz von Carbonsäurehalogeniden der allgemeinen Struktur (a3-1) vorteilhafterweise in Gegenwart eines Reaktionshilfsmittels durchgeführt. Als solche kommen alle üblichen anorganischen oder organischen Basen in Frage. Hierzu gehören beispielsweise Erdalkali- oder Alkalimetallhydride, -hydroxide, -amide, alkoholate, -acetate, -carbonate oder -hydrogencarbonate, wie beispielsweise Natriumhydrid, Natriumamid, Natriummethylat, Natriumethylat, Kalium-t.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, oder Ammoniumcarbonat sowie tertiäre Amine, wie beispielsweise Trimethylamin, Triethylamin, Diisopropylethylamin, Tributylamin, N,N-Dimethylanilin, Pyridin, N-Methyl-piperidin, 4-(N,N-Dimethylamino)-pyridin, Diazabicyclooctan (DABCO), Diazabicyclononen (DBN) oder Diazabicycloundecen (DBU), insbesondere Triethylamin.

Die Verbindungen der allgemeinen Formel (I-a) werden beim Einsatz von Carbonsäuren der allgemeinen Struktur (a2-1) vorteilhafterweise in Gegenwart eines Kondensationsmittels durchgeführt. Die Carbonsäuren sind kommerziell verfügbar. Als Kondensationsmittel sind vor allem wasserentziehende Chemikalien geeignet. Hierzu gehören vorzugsweise Säureanhydride und Säurehalogenide, wie beispielsweise Acetanhydrid, Propionsäureanhydrid, Phosphor-(V)-oxid, Phosphorylchlorid, Phosphorylbromid, Phosportrichlorid, Phosphortribromid, Thionylchlorid, Oxalylchlorid, Phosgen, Diphosgen, Ameisensäuremethylester, Ameisensäureethylester, sowie Carbodiimide, wie beispielsweise N,N'-Dicyclohexylcarbodiimid (DCC) oder 1-Ethyl-3-(3-dimethylaminopropyl)carbodiimid Hydrochlorid (EDC-HCl). Weitere bekannte Kondensationsreagenzien sind Triphenylphosphin/Tetrachlormethan, 4-(4,6-Dimethoxy[1.3.5]triazin-2-yl)-4-methyl-morpholiniumchlorid-Hydrat oder Hydroxybenzotriazol (HOBt). Insbesondere die Kombination von 1-Ethyl-3-(3-dimethylaminopropyl)carbodiimid Hydrochlorid (EDC-HCl) und Hydroxybenzotriazol (HOBt) sei hier aufgeführt.

Die Verbindungen der allgemeinen Formel (I-a) werden gegebenenfalls unter Einsatz eines oder mehrerer Verdünnungsmittel hergestellt. Als Verdünnungsmittel kommen vor allem inerte organische Lösungsmittel in Betracht. Hierzu gehören insbesondere aliphatische, alicylische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan, Tetrahydrofuran, Dioxan, Acetonitril oder Dimethylformamid.

Die Reaktionstemperaturen können bei der Durchführung des Verfahrens A-1 in einem größeren Bereich variiert werden. Im Allgemeinen arbeitet man bei Temperaturen zwischen 0 °C und 150 °C, vorzugsweise zwischen 10 °C und 120 °C.

Das Verfahren A-1 wird im Allgemeinen unter Normaldruck durchgeführt. Es ist aber auch möglich, das Verfahren A-1 unter erhöhtem oder verminderten Druck - im Allgemeinen zwischen 0,1 bar und 10 bar - durchzuführen.

### Darstellungsverfahren B

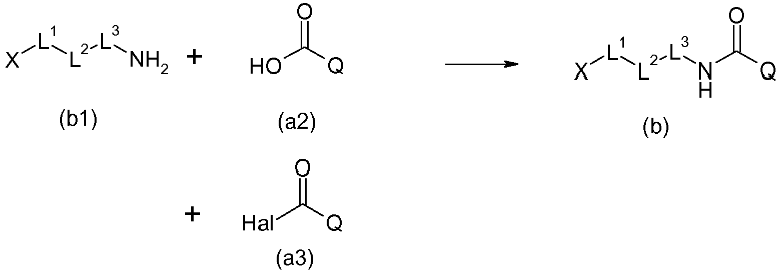

Die Reste X, L¹, L², L³, und Q haben die oben beschriebenen Bedeutungen. W steht in diesem Fall für Sauerstoff, Y steht für Wasserstoff.

Die Verbindungen der allgemeinen Struktur (I) bzw. deren Ausführungsform (b) können ausgehend von Aminen gemäß Formel (b1) und Carbonsäuren gemäß Formel (a2) bzw. deren Halogenide gemäß Formel (a3) nach hinlänglich bekannten Verfahren dargestellt werden, beispielsweise wie in EP2007060166 beschrieben. Die Amine gemäß Formel (b1) und Carbonsäuren gemäß Formel (a2) bzw. deren Halogenide gemäß Formel (a3) sind kommerziell verfügbar.

### Darstellungsverfahren C

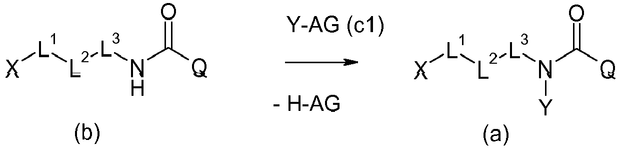

Die Reste X, Y, L¹, L², L³, und Q haben die oben beschriebenen Bedeutungen. W steht in diesem Fall für Sauerstoff, AG für Abgangsgruppe, beispielsweise Halogene oder Alkyl- bzw. Arylsulfonate.

Die Verbindungen der allgemeinen Formel (I) bzw. deren Ausführungsform (a) können ausgehend von Aminen gemäß Formel (b) und Alkylierungsmitteln gemäß Formel (c1) nach hinlänglich bekannten Verfahren dargestellt werden, beispielsweise wie in EP2007060166 beschrieben.

### Darstellungsverfahren D

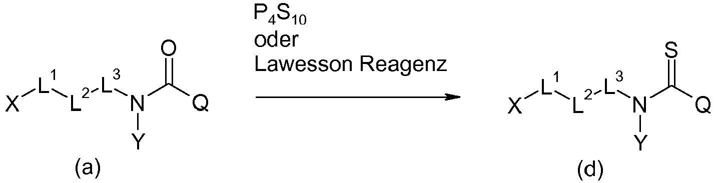

Die Reste X, Y, L¹, L², L³ und Q haben die oben beschriebenen Bedeutungen. W = Sauerstoff wird direkt zu W = Schwefel transformiert.

Die Verbindungen der allgemeinen Formel (I) bzw. deren Ausführungsform (d) können ausgehend von Verbindungen der Formel (a) und geeigneten Schwefelungsreagenzien, z.B. Tetraphosphordecasulfid ("Phosphorpentasulfid") oder 2,4-Bis-[4-methoxyphenyl]-2,4-dithiono-1,2,3,4-dithiadiphosphetan ("Lawesson Reagenz"), nach hinlänglich bekannten Verfahren hergestellt werden. Verfahrensbeispiele sind u.a. bekannt aus Houben-Weyl, Methoden der Organischen Chemie, E5, 1255 (Thieme Verlag, Stuttgart, 1985).

### Anthelmintische Anwendungsgebiete

Die offenbarten Mittel eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie können gegen alle oder einzelne Entwicklungsstadien der Schädlinge sowie gegen resistente und normal sensible Endoparasiten-Isolate eingesetzt werden. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so dass durch den Einsatz der Wirkstoffe eine wirtschaftlichere, einfachere und gesündere Tierhaltung möglich ist. Zu den pathogenen Endoparasiten zählen Helminthen wie Platyhelmintha (insbesondere Monogenea, Cestoden und Trematoden), Nematoden, Pentastoma und Acanthocephala. Als Beispiele seien genannt:
**Monogenea:** z.B.: Gyrodactylus spp., Dactylogyrus spp., Polystoma spp..
**Cestoden:** Aus der Ordnung der Pseudophyllidea z.B.: Diphyllobothrium spp., Spirometra spp., Schistocephalus spp., Ligula spp., Bothridium spp., Diphlogonoporus spp..
   Aus der Ordnung der Cyclophyllida z.B.: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..
**Trematoden:** Aus der Klasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..
**Nematoden:** Aus der Ordnung der Trichinellida z.B.: Trichuris spp., Capillaria spp., Trichomosoides spp., Trichinella spp..
   Aus der Ordnung der Tylenchida z.B.: Micronema spp., Strongyloides spp..
   Aus der Ordnung der Rhabditina z.B.: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp.
   Aus der Ordnung der Spirurida z.B.: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp.
**Acantocephala:** Aus der Ordnung der Oligacanthorhynchida z.B: Macracanthorhynchus spp., Prosthenorchis spp.; aus der Ordnung der Polymorphida z.B.: Filicollis spp.; aus der Ordnung der Moniliformida z.B.: Moniliformis spp.,
   Aus der Ordnung der Echinorhynchida z.B. Acanthocephalus spp., Echinorhynchus spp., Leptorhynchoides spp.
**Pentastoma:** Aus der Ordnung der Porocephalida z.B. Linguatula spp.

Gemäß einer bevorzugten Ausführungsform werden die Verbindungen der Formel (I) zur Bekämpfung von Nematoden eingesetzt. Als Nematoden seien besonders bevorzugt genannt: Trichinellida, Tylenchida, Rhabditina oder die folgenden aus der Ordnung der Spirurida: Gnathostoma spp., Physaloptera spp., Thelazia spp., Gongylonema spp., Habronema spp., Parabronema spp., Draschia spp., Dracunculus spp ..

Gegenstand einer weiteren besonders bevorzugten Ausführungsform ist die Verwendung zur Bekämpfung von Strongylida, insbesondere Haemonchus spp. (z. B. Haemonchus contortus), Trichostrongylus spp. (z. B. Trichostrongylus colubriformis), Cooperia spp., und Ostertagia spp oder Teladorsagia spp..

Gegenstand einer weiteren besonders bevorzugten Ausführungsform ist die Verwendung zur Bekämpfung von Ascaridida wie z. B. Parascaris spp.

Tiere können Fische, Reptilien, Vögel oder insbesondere Säugetiere sein.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Strauße, Fische wie Forellen, Lachse, Karpfen, Barsche, Hechte, Aale.

Zu Labor- und Versuchstieren gehören Mäuse, Ratten, Meerschweinchen, Goldhamster, Hunde und Katzen.

Zu den Hobbytieren gehören Hunde und Katzen.

Erfindungsgemäß bevorzugt ist die Anwendung bei Tieren, aber grundsätzlich kommt auch die Anwendung am Menschen in Frage. Vorzugsweise werden beim Menschen Ascaris spp., Ancylostoma spp, Necator spp., Trichuris spp., Strongyloides spp. und Enterobius spp. bekämpft.

Unter den Säugetieren werden gemäß einer Ausführungsform für die erfindungsgemäße Anwendung die Pflanzenfresser (Herbivoren) bevorzugt, also Tiere, die sich hauptsächlich von Pflanzen ernähren. Besonders bevorzugt ist die Behandlung von Wiederkäuern (wie z. B. Schafe, Ziegen, Rinder).

Als nicht wiederkäuende Pflanzenfresser, die Säugetiere sind, seien als bevorzugtes Beispiel die Pferde genannt. Dort können die obengenannten Kombinationen bevorzugt z. B. zur Bekämpfung von Strongylida oder insbesondere von Spulwürmern (Ascaridia), wie z. B. Parascaris equorum, eingesetzt werden.

Bei den Wiederkäuern können bevorzugt Strongylida, insbesondere Haemonchus spp., Trichostrongylus spp., Cooperia spp. und Ostertagia spp. bekämpft werden.

Besonders bevorzugt werden erfindungsgemäß Schafe behandelt.

Ebenfalls besonders bevorzugt werden erfindungsgemäß Rinder behandelt.

Die Anwendung der Wirkstoffe erfolgt im Veterinärsektor und bei der Tierhaltung in an sich bekannter Weise direkt oder in Form von geeigneten Zubereitungen. Die Anwendung kann sowohl prophylaktisch als auch therapeutisch erfolgen.

Die Verbindungen der allgemeinen Formel (I) oder (I-a) können alleine oder in Kombination mit anderen Wirkstoffen, z. B. mit anderen Parasitiziden, eingesetzt werden.

### Herstellungsbeispiele:

### Beispiel 14-11

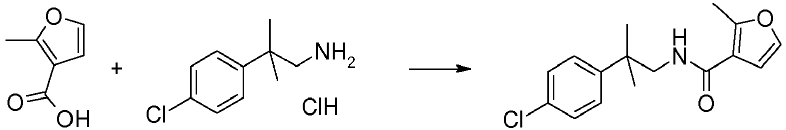

94,6 mg (0,75 mmol) 2-Methylfuran-3-carbonsäure, 198 mg (0,90 mmol) 2-(4-Chlorphenyl)-2-methylpropan-1-amin, 50,6 mg (0,37 mmol) 1-Hydroxybenzotriazole (HOBt), 46 mg (0,37 mmol) DMAP, 172,5 mg (0,90 mmol) EDC-Hydrochlorid und 116 mg (0,90 mmol) Diisopropylethylamin werden in 10 ml Dichlormethan gelöst und bei Raumtemperatur für 16 h gerührt. Nach beendeter Reaktion wird mit 10 ml Wasser versetzt und die organische Phase abgetrennt, die wässrige Phase mit 5 ml Dichlormethan nachextrahiert. Die Dichlormethanphasen werden über eine Natriumsulfat/Kieselgelkartusch filtriert, das Lösungsmittel wird abgedampft und der Rückstand mittels präparativer HPLC getrennt.
Ausbeute: 139,3 mg (63,6 % d. Th.), farbloser Feststoff.
¹H-NMR (d6-DMSO): δ [ppm], 7,73 (t, 1H, NH), 7,48 (d, 1H), 7,42 (d, 2H), 7,35 (d, 2H), 6,80 (d, 1H), 3,37 (d, 2H), 2,43 (s, 3H), 1,26 (s, 6H).

### Beispiel 20-238

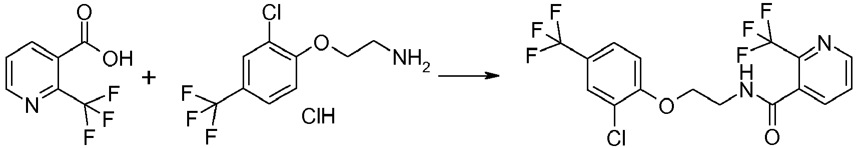

114 mg (0,6 mmol) 2-(2-Chlor-4-(trifluormethyl)phenoxy)-1-ethylamin, 163 mg (0,72 mmol) 2-(Trifluormethyl)-nicotinsäurechlorid, 40,5 mg (0,3 mmol) 1-Hydroxybenzotriazole (HOBt), 36,6 mg (0,3 mmol) DMAP, 115 mg (0,6 mmol) EDC-Hydrochlorid und 77,5 mg (0,6 mmol) Diisopropylethylamin werden in 10 ml Dichlormethan gelöst und bei Raumtemperatur für 16 h gerührt. Nach beendeter Reaktion wird mit 10 ml Wasser versetzt und die organische Phase abgetrennt, die wässrige Phase mit 5 ml Dichlormethan nachextrahiert. Die Dichlormethanphasen werden über eine Natriumsulfat/Kieselgelkartusche filtriert, das Lösungsmittel wird abgedampft und der Rückstand mittels präparativer HPLC getrennt.
Ausbeute: 146 mg (59,9 % d. Th.), farbloser Feststoff.
¹H-NMR (d6-DMSO): δ [ppm], 8,95 (t, 1H, NH), 8,80 - 8,79 (d, 1H), 7,99 - 7,97 (d, 1H), 7,84 (s, 1H), 7,80 - 7,77 (dd, 1H), 7,72 - 7,69 (d, 1H), 7,40 - 7,38 (d, 1H), 4,30 (t, 2H), 3,70 - 3,67 (quart, 2H).
Nachfolgende Tabellenbeispiele sind auf gleiche Weise herstellbar.

### ¹H-NMR Daten

Die Bestimmung der ¹H-NMR-Daten erfolgte mit einem Bruker Avance 400 ausgestattet mit einem Durchflussprobenkopf (60 µl Volumen), mit Tetramethylsilan als Referenz (0.0) und den Lösungsmitteln CD₃CN, CDCl₃, D₆-DMSO.

Die NMR-Daten ausgewählter Beispiele werden entweder in klassischer Form (δ-Werte, Multiplettaufspaltung, Anzahl der H-Atome) oder als NMR-Peak-Listen aufgeführt.

NMR-Peak-Listenverfahren: Wenn die ¹H-NMR-Daten ausgewählter Beispiele in Form von ¹H-NMR-Peaklisten notiert werden, wird zu jedem Signalpeak erst der δ-Wert in ppm und dann die Signalintensität durch ein Leerzeichen getrennt aufgeführt. Die δ-Wert - Signalintensitäts-Zahlenpaare von verschiedenen Signalpeaks werden durch Semikolons voneinander getrennt aufgelistet. Die Peakliste eines Beispieles hat daher die Form: δ₁(Intensität₁);δ₂ (Intensität₂);........;δᵢ(Intensitätᵢ);......; δₙ(Intensitätₙ). Das Lösungsmittel, in welchem das NMR-Spektrum aufgenommen wurde, wird in eckigen Klammern vor der NMR-Peakliste bzw. der klassischen NMR-Interpretationsliste aufgeführt.

**Tabelle 1**

| | | | | | | |
|---|---|---|---|---|---|---|
| Verbindungen der Formel I-1 | | | | | | |
| 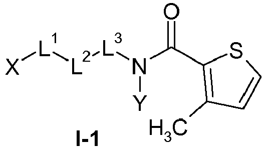 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 1-1 | 3-methyl-2-thienyl | CH₂ | CH₂ | - | H | WO-A 2006/108791 |
| 1-2 | 2,4-dichlor-phenyl | CH₂ | CH₂ | CH₂ | H | WO-A 2008/101976 |
| 1-3 | 4-chlor-phenyl | CH₂ | CH₂ | - | H | CAS: 1043286-26-5 |
| 1-4 | 4-chlor-phenyl | CH(OCH3) | CH(CH3) | - | H | |
| | | | | | | [DMSO], Spektrometer: 399.95MHz |
| 1-5 | 2,4-dichlor-phenyl | CH(OCH3) | CH(CH3) | - | H | 7.7469 (1.12); 7.7252 (1.15); 7.7057 (0.94); 7.6841 (0.94); 7.6179 (1.95); 7.6129 (2.08); 7.5900 (2.33); 7.5853 (2.53); 7.5290 (3.02); 7.5166 (3.14); 7.4844 (0.67); 7.4796 (0.55); 7.4634 (2.97); 7.4586 (3.00); 7.4477 (3.91); 7.4430 (2.06); 7.4377 (1.80); 7.4267 (1.08); 7.4179 (2.89); 7.3969 (1.08); 6.9234 (2.11); 6.9114 (4.42); 6.8992 (2.53); 4.7378 (1.61); 4.7244 (1.71); 4.6260 (2.08); 4.6102 (2.30); 4.3387 (0.65); 4.3221 (1.13); 4.3180 (1.07); 4.3049 (0.95); 4.3009 (1.21); 4.2840 (0.75); 3.3237 (7.87); 3.1838 (16.00); 3.1784 (13.52); 2.5115 (4.24); 2.5072 (8.58); 2.5027 (11.54); 2.4982 (8.53); 2.4939 (4.20); 2.3293 (10.23); 2.2726 (13.26); 1.3965 (4.48); 1.1408 (5.91); 1.1234 (7.31); 1.1033 (4.64); 0.0080 (0.42);-0.0002 (11.36); -0.0084 (0.44) |
| | | | | | | [DMSO], Spektrometer: 399.95MHz |
| 1-6 | 4-chlor-phenyl | CH(CH3) | CH2 | - | H | 7.9424 (0.59); 7.9286 (1.15); 7.9146 (0.61); 7.5253 (2.91); 7.5129 (3.03); 7.3730 (0.33); 7.3668 (3.29); 7.3619 (1.23); 7.3506 (1.51); 7.3455 (6.10); 7.3396 (0.97); 7.2968 (0.78); 7.2911 (5.46); 7.2862 (1.62); 7.2744 (1.17); 7.2699 (3.14); 6.9182 (3.00); 6.9058 (2.92); 3.9077 (13.77); 3.5200 (0.43); 3.3909 (365.99); 3.3564 (4.91); 3.3378 (3.29); 3.3241 (2.05); 3.3086 (0.84); 3.3055 (0.88); 3.2918 (0.59); 3.1730 (0.70); 3.0889 (0.58); 3.0712 (1.07); 3.0532 (1.03); 3.0349 (0.52); 2.6786 (0.40); 2.5486 (0.35); 2.5318 (1.35); 2.5184 (23.02); 2.5140 (45.87); 2.5094 (60.62); 2.5049 (45.57); 2.5005 (23.03); 2.3362 (0.40); 2.2661 (16.00); 1.2158 (6.84); 1.1983 (6.73) |
| | | | | | | [DMSO], Spektrometer: 399.95MHz |
| 1-7 | 2,4-dichlor-phenyl | CH(CH3) | CH2 | - | H | 8.0105 (0.50); 7.9962 (0.98); 7.9821 (0.49); 7.5641 (2.66); 7.5588 (2.84); 7.5264 (2.39); 7.5140 (2.48); 7.4725 (1.36); 7.4514 (3.22); 7.4255 (1.99); 7.4202 (1.82); 7.4045 (0.81); 7.3991 (0.80); 6.9178 (2.43); 6.9054 (2.33); 3.9098 (16.00); 3.5797 (0.40); 3.5615 (0.77); 3.5439 (0.88); 3.5266 (0.58); 3.4821 (0.34); 3.4668 (0.44); 3.4493 (1.23); 3.4350 (1.79); 3.4225 (1.79); 3.4184 (2.08); 3.4030 (2.01); 3.3667 (243.69); 3.1736 (0.54); 2.6785 (0.37); 2.5487 (0.60); 2.5317 (1.37); 2.5183 (22.50); 2.5139 (44.62); 2.5093 (58.74); 2.5048 (44.01); 2.5003 (22.23); 2.3361 (0.37); 2.2755 (13.20); 1.2055 (5.51); 1.1882 (5.42) |
| | | | | | | Verbindung Nr. 1-8, Solvent: [DMSO], Spektrometer: 399.95MHz |
| 1-8 | 4-chlor-phenyl | CH2 | CH(CH3) | - | H | 7,8223 (1,2); 7,8015 (1,24); 7,5241 (3,11); 7,5117 (3,23); 7,3417 (3,49); 7,3369 (1,32); 7,3255 (1,69); 7,3205 (6,55); 7,3148 (1,04); 7,268 (5,53); 7,2469 (3,16); 6,9166 (3,19); 6,9042 (3,11); 4,1649 (0,58); 4,1488 (0,86); 4,1325 (0,65); 3,9091 (16); 3,3749 (344,32); 3,2939 (0,38); 3,1799 (0,46); 3,1673 (0,47); 2,8526 (0,66); 2,8318 (0,66); 2,819 (1,42); 2,7983 (1,41); 2,7712 (1,42); 2,756 (1,48); 2,7375 (0,68); 2,7223 (0,62); 2,6831 (0,33); 2,6785 (0,44); 2,6742 (0,32); 2,5486 (0,34); 2,5318 (1,47); 2,5184 (25,95); 2,514 (50,99); 2,5094 (66,77); 2,5049 (49,84); 2,5004 (24,96); 2,3407 (0,33); 2,3362 (0,44); 2,3315 (0,32); 2,2708 (15,79); 1,1618 (7,33); 1,1453 (7,28) |
| 1-9 | 2,4-dichlor-phenyl | CH2 | CH(CH3) | - | H | |
| | | | | | | [DMSO], Spektrometer: 399.95MHz |
| 1-10 | 4-chlor-phenyl | C(CH3)2 | CH2 | - | H | 7.7190 (0.39); 7.7039 (0.75); 7.6887 (0.39); 7.5281 (2.00); 7.5157 (2.07); 7.4368 (2.17); 7.4316 (0.84); 7.4202 (1.10); 7.4149 (4.11); 7.4085 (0.64); 7.3725 (0.60); 7.3662 (4.25); 7.3608 (1.10); 7.3494 (0.82); 7.3443 (2.21); 6.9111 (2.05); 6.8987 (1.99); 3.9041 (11.04); 3.4232 (3.25); 3.4074 (3.61); 3.3650 (195.16); 3.1685 (0.56); 2.5265 (0.87); 2.5130 (17.60); 2.5087 (34.78); 2.5041 (45.86); 2.4996 (34.77); 2.4953 (18.07); 2.2374 (10.99); 1.2850 (16.00) |
| | | | | | | [DMSO], Spektrometer: 399.95MHz |
| 1-11 | 2,4-dichlor-phenyl | C(CH3)2 | CH2 | - | H | 7.7467 (0.44); 7.7315 (0.87); 7.7159 (0.43); 7.5311 (2.32); 7.5252 (2.49); 7.5174 (2.15); 7.5050 (2.15); 7.4758 (1.49); 7.4541 (2.25); 7.3769 (1.47); 7.3711 (1.40); 7.3554 (0.99); 7.3496 (0.94); 6.8988 (2.14); 6.8864 (2.07); 3.9046 (10.59); 3.7588 (2.77); 3.7430 (2.74); 3.3880 (1.35); 3.3535 (185.47); 3.1680 (0.59); 2.6726 (0.33); 2.5428 (0.63); 2.5257 (1.15); 2.5122 (20.13); 2.5080 (39.32); 2.5035 (51.69); 2.4990 (39.41); 2.4950 (20.61); 2.3302 (0.33); 2.3111 (0.33); 2.2063 (11.23); 1.4394 (16.00); -0.0002 (4.70) |
| | | | | | | [DMSO], Spektrometer: 399.95MHz |
| 1-12 | 2-chlor-phenyl | CH2 | CH2 | - | H | 8.0508 (0.61); 8.0368 (1.15); 8.0228 (0.60); 7.5422 (2.97); 7.5298 (3.08); 7.4403 (1.40); 7.4361 (1.12); 7.4223 (1.81); 7.4174 (1.79); 7.3536 (0.96); 7.3480 (1.18); 7.3353 (1.44); 7.3303 (1.95); 7.3045 (0.60); 7.3002 (0.80); 7.2862 (1.91); 7.2819 (1.78); 7.2732 (1.80); 7.2672 (2.05); 7.2546 (1.53); 7.2493 (1.28); 7.2362 (0.47); 7.2311 (0.37); 6.9380 (3.04); 6.9255 (2.93); 3.9044 (12.08); 3.4851 (1.15); 3.4681 (2.37); 3.4523 (2.36); 3.4349 (1.53); 3.3627 (281.12); 3.1686 (0.45); 2.9729 (2.05); 2.9547 (3.44); 2.9371 (1.82); 2.6731 (0.39); 2.5260 (1.29); 2.5125 (23.55); 2.5084 (45.80); 2.5040 (60.06); 2.4995 (45.39); 2.4954 (23.36); 2.3422 (16.00); -0.0002 (0.79) |
| | | | | | | [DMSO], Spektrometer: 399.95MHz |
| 1-13 | 3,4-dichlor-phenyl | CH2 | CH2 | - | H | 8.0102 (0.60); 7.9967 (1.14); 7.9831 (0.59); 7.5613 (3.22); 7.5468 (3.04); 7.5407 (3.90); 7.5345 (3.17); 7.5192 (2.81); 7.5143 (2.91); 7.3199 (0.42); 7.3151 (0.40); 7.2519 (1.61); 7.2468 (1.59); 7.2313 (1.42); 7.2263 (1.39); 6.9403 (3.14); 6.9279 (3.02); 3.9089 (11.33); 3.4723 (1.28); 3.4551 (2.84); 3.4405 (3.05); 3.4235 (2.16); 3.3770 (275.74); 3.1735 (0.54); 2.8541 (1.81); 2.8369 (3.59); 2.8196 (1.60); 2.6786 (0.34); 2.5318 (1.11); 2.5184 (19.55); 2.5140 (39.18); 2.5094 (51.97); 2.5049 (39.10); 2.5004 (19.77); 2.3362 (0.43); 2.3311 (0.44); 2.3198 (16.00) |
| | | | | | | Verbindung Nr. 1-14, Solvent: [DMSO], Spektrometer: 399.95MHz |
| 1-14 | 3,5-dichlor-phenyl | CH2 | CH2 | - | H | 8,0159 (0,55); 8,0022 (1,11); 7,9887 (0,61); 7,5485 (2,74); 7,536 (2,91); 7,4478 (1,39); 7,4431 (2,85); 7,4383 (1,7); 7,3187 (6,2); 7,314 (6,04); 6,9405 (2,88); 6,9281 (2,85); 3,9076 (13,02); 3,5201 (0,46); 3,4809 (1,61); 3,464 (3,39); 3,4494 (4,07); 3,4321 (4,33); 3,3912 (389,41); 3,1791 (0,47); 3,1663 (0,46); 2,8652 (1,72); 2,8483 (3,49); 2,8313 (1,59); 2,6831 (0,34); 2,6785 (0,45); 2,6742 (0,35); 2,5317 (1,51); 2,5184 (24,67); 2,514 (49,01); 2,5094 (64,75); 2,5048 (48,68); 2,5005 (24,91); 2,3407 (0,36); 2,3362 (0,47); 2,3316 (0,39); 2,3145 (16) |
| 1-15 | 3-chlor-phenyl | CH2 | CH2 | - | H | CAS: 1325308-79-9 |
| 1-16 | 2- fluor-phenyl | CH2 | CH2 | - | H | CAS: 1240811-68-0 |
| | | | | | | [DMSO], Spektrometer: 399.95MHz |
| 1-17 | 2,6-difluor-phenyl | CH2 | CH2 | - | H | 7.7467 (0.44); 7.7315 (0.87); 7.7159 (0.43); 7.5311 (2.32); 7.5252 (2.49); 7.5174 (2.15); 7.5050 (2.15); 7.4758 (1.49); 7.4541 (2.25); 7.3769 (1.47); 7.3711 (1.40); 7.3554 (0.99); 7.3496 (0.94); 6.8988 (2.14); 6.8864 (2.07); 3.9046 (10.59); 3.7588 (2.77); 3.7430 (2.74); 3.3880 (1.35); 3.3535 (185.47); 3.1680 (0.59); 2.6726 (0.33); 2.5428 (0.63); 2.5257 (1.15); 2.5122 (20.13); 2.5080 (39.32); 2.5035 (51.69); 2.4990 (39.41); 2.4950 (20.61); 2.3302 (0.33); 2.3111 (0.33); 2.2063 (11.23); 1.4394 (16.00); -0.0002 (4.70) |
| | | | | | | [DMSO], Spektrometer: 399.95MHz |
| 1-18 | 2,6-dichlor-phenyl | CH2 | CH2 | - | H | 8.1088 (0.60); 8.0946 (1.16); 8.0802 (0.61); 7.5393 (2.88); 7.5269 (3.00); 7.4613 (4.88); 7.4411 (6.73); 7.2999 (2.24); 7.2807 (2.27); 7.2789 (2.11); 7.2596 (1.45); 6.9373 (2.97); 6.9249 (2.89); 3.9046 (15.92); 3.4731 (0.91); 3.4564 (2.21); 3.4389 (2.16); 3.4231 (1.15); 3.3464 (188.35); 3.1666 (2.10); 3.1485 (3.24); 3.1314 (1.60); 2.6723 (0.43); 2.5425 (0.50); 2.5254 (1.38); 2.5121 (25.86); 2.5077 (51.09); 2.5032 (67.50); 2.4986 (51.02); 2.4943 (26.32); 2.3579 (16.00); 2.3346 (0.41); 2.3299 (0.49); 2.3255 (0.35); -0.0002 (7.67); -0.0085 (0.33) |
| | | | | | | Verbindung Nr. 1-19, Solvent: [DMSO], Spektrometer: 399.95MHz |
| 1-19 | 3-(trifluormethyl)-phenyl | CH2 | CH2 | - | H | 8,0151 (0,61); 8,0015 (1,13); 7,9878 (0,59); 7,5868 (2,73); 7,5694 (1,27); 7,5643 (2,16); 7,5567 (3,19); 7,5501 (3,21); 7,5399 (3,31); 7,5275 (3,32); 6,9301 (2,98); 6,9177 (2,88); 3,9045 (14,6); 3,5015 (1,06); 3,4841 (2,54); 3,4697 (2,57); 3,4524 (1,31); 3,428 (0,4); 3,4116 (0,71); 3,3597 (304,29); 3,3124 (0,59); 3,3005 (0,39); 3,1684 (0,53); 2,9509 (1,85); 2,9335 (3,64); 2,9159 (1,69); 2,6731 (0,42); 2,6685 (0,32); 2,5263 (1,39); 2,5128 (25,41); 2,5085 (50,76); 2,5039 (67,15); 2,4994 (50,71); 2,495 (25,94); 2,3353 (0,33); 2,3306 (0,45); 2,3262 (0,36); 2,3127 (0,68); 2,2986 (16); -0,0002 (2,03) |
| 1-20 | 4-(trifluormethyl)-phenyl | CH2 | CH2 | - | H | CAS: 1096213-61-4 |
| 1-21 | 2-methyl-phenyl | CH2 | CH2 | - | H | CAS: 1328661-91-1 |
| | | | | | | [DMSO], Spektrometer: 399.95MHz |
| 1-22 | 2,4,6-trimethylphenyl | CH2 | CH2 | - | H | 8.1607 (0.39); 8.1464 (0.82); 8.1319 (0.43); 7.5696 (1.94); 7.5572 (2.04); 6.9699 (1.97); 6.9575 (1.94); 6.8089 (4.27); 3.9096 (10.74); 3.3940 (2.46); 3.3686 (187.69); 3.2424 (0.58); 3.2277 (0.94); 3.2229 (0.80); 3.2141 (0.92); 3.2071 (0.78); 3.2008 (1.00); 3.1868 (0.67); 3.1739 (0.53); 2.8005 (1.09); 2.7871 (0.85); 2.7796 (1.09); 2.7744 (0.91); 2.7597 (0.94); 2.5488 (2.24); 2.5317 (0.96); 2.5183 (16.56); 2.5139 (33.04); 2.5094 (43.72); 2.5048 (33.10); 2.5005 (17.04); 2.4189 (10.37); 2.3362 (0.36); 2.3078 (16.00); 2.1865 (7.38) |
| 1-23 | 3,4-bismethoxyphenyl | CH2 | CH2 | - | H | CAS: 1023537-11-2 |
| 1-24 | phenyl | CH2 | CH2 | - | H | CAS: 1090485-43-0 |
| 1-25 | 4-chlor-phenyl | C(CH2-CH2) | CH2 | - | H | |
| 1-26 | 2,4-dichlor-phenyl | C(CH2-CH2) | CH2 | - | H | |
| 1-27 | 4-chlor-phenyl | CH2 | C(CH2-CH2) | - | H | |
| 1-28 | 2,4-dichlor-phenyl | CH2 | C(CH2-CH2) | - | H | |
| 1-29 | 4-chlor-phenyl | O | CH2 | CH2 | H | |
| | | | | | | Verbindung Nr. 1-30, Solvent: [DMSO], Spektrometer: 399.95MHz |
| 1-30 | 2,4-dichlor-phenyl | O | CH2 | CH2 | H | 8,0722 (0,7); 8,0589 (1,33); 8,0455 (0,71); 7,5707 (4,23); 7,5637 (3,95); 7,5605 (3,47); 7,3834 (1,58); 7,377 (1,49); 7,3613 (2,18); 7,3548 (2,12); 7,2495 (3,61); 7,2272 (2,64); 6,9582 (3,13); 6,9457 (3,03); 4,2136 (2,09); 4,1987 (4,6); 4,1838 (2,29); 3,624 (1,18); 3,6095 (3,26); 3,5951 (3,15); 3,5804 (1,07); 3,3333 (10,63); 2,5436 (10,77); 2,5086 (6,89); 2,5042 (9,01); 2,4998 (6,82); 2,4061 (16); -0,0002 (0,4) |
| 1-31 | 4-chlor-phenyl | CH2 | CH2 | CH2 | H | |
| 1-32 | 4-chlor-phenyl | NCH3 | CH2 | CH2 | H | |
| 1-33 | 2,4-dichlor-phenyl | NCH3 | CH2 | CH2 | H | |
| 1-34 | 4-chlor-phenyl | CH(OCH3) | CH2 | - | H | |
| 1-35 | 2,4-dichlor-phenyl | CH(OCH3) | CH2 | - | H | |
| 1-36 | 2-thienyl | CH2 | CH2 | - | H | CAS: 1182416-85-8 |
| 1-37 | 3-thienyl | CH2 | CH2 | - | H | |
| 1-38 | 2-furyl | CH2 | CH2 | - | H | |
| 1-39 | 3-furyl | CH2 | CH2 | - | H | |
| 1-40 | phenyl | CH2 | CH2 | CH(CH3) | H | |
| 1-41 | phenyl | CH2 | CH2 | CH2 | H | |
| 1-42 | 2-Cl-phenyl | CH2 | CH2 | CH(CH3) | H | |
| 1-43 | 4-t-butyl-phenyl | CH2 | CH2 | CH2 | H | |
| 1-44 | 4-methyl-phenyl | CH2 | CH2 | CH2 | H | |
| 1-45 | phenyl | CH2 | CH2 | CH(CH2CH3) | H | |
| 1-46 | 2-methoxy-phenyl | CH2 | CH2 | CH2 | H | |
| 1-47 | 2-methyl-phenyl | CH2 | CH2 | CH2 | H | |
| 1-48 | 3-methyl-phenyl | CH2 | CH2 | CH2 | H | |
| 1-49 | 3-chlor-phenyl | CH2 | CH2 | CH2 | H | |
| | | | | | | Verbindung Nr. 1-50, Solvent: [DMSO], Spektrometer: 399.95MHz |
| 1-50 | 2,6-difluor-phenyl | CH2 | CH2 | CH2 | H | 8,0032 (0,64); 7,9895 (1,2); 7,976 (0,67); 7,5479 (2,74); 7,5355 (2,88); 7,3477 (0,33); 7,3308 (0,77); 7,3272 (0,73); 7,31 (1,47); 7,2922 (0,79); 7,2892 (0,97); 7,2723 (0,43); 7,0861 (1,99); 7,0662 (3,26); 7,0463 (1,7); 6,9476 (2,95); 6,9352 (2,89); 3,332 (25,1); 3,2604 (1,01); 3,2433 (2,22); 3,2273 (2,24); 3,2103 (1,07); 2,6829 (1,42); 2,6639 (2,36); 2,6444 (1,55); 2,5427 (1,85); 2,5077 (12,73); 2,5032 (17,02); 2,4988 (13,19); 2,4284 (0,38); 2,392 (16); 1,7932 (0,54); 1,7742 (1,43); 1,7556 (1,99); 1,737 (1,35); 1,7185 (0,48) |
| 1-51 | 4-chlor-phenyl | CH2 | CH2 | CH2 | H | |
| 1-52 | 2,6-dichlor-phenyl | CH2 | CH2 | CH(CH3) | H | |
| 1-53 | 3,5-dichlor-phenyl | CH2 | CH2 | CH(CH3) | H | |
| 1-54 | 2,6-dimethyl-phenyl | CH2 | CH2 | CH2 | H | |
| 1-55 | 2,5-dichlor-phenyl | CH2 | CH2 | CH2 | H | |
| 1-56 | 4-i-propoxy-phenyl | CH2 | CH2 | CH2 | H | |
| 1-57 | 3-trifluormethyl-phenyl | CH2 | CH2 | CH(CH3) | H | |
| 1-58 | 4-methyl-phenyl | CH2 | CH2 | CH(CH3) | H | |
| 1-59 | 2-methyl-phenyl | CH2 | CH2 | CH(CH3) | H | |
| 1-60 | 3,4-dichlor-phenyl | CH2 | CH2 | CH(CH3) | H | |
| 1-61 | 3,5-dichlor-phenyl | CH2 | CH2 | CH2 | H | |
| 1-62 | 2,6-dimethyl-phenyl | CH2 | CH2 | CH(CH3) | H | |
| 1-63 | 4-trifluormethyl-phenyl | CH2 | CH2 | CH(CH3) | H | |
| 1-64 | 2,5-dichlor-phenyl | CH2 | CH2 | CH(CH3) | H | |
| 1-65 | 4-phenoxy-phenyl | CH2 | CH2 | CH2 | H | |
| 1-66 | 3-chlor-phenyl | CH2 | CH2 | CH(CH3) | H | |
| 1-67 | 4-phenoxy-phenyl | CH2 | CH2 | CH(CH3) | H | |
| 1-68 | 2,4-dichlor-phenyl | CH2 | CH2 | CH(CH3) | H | |
| 1-69 | 2-difluormethoxy-phenyl | CH2 | CH2 | CH(CH3) | H | |
| 1-70 | 4-methoxy-phenyl | CH2 | CH2 | CH2 | H | |
| 1-71 | 4-chlor-phenyl | CH2 | CH2 | CH(CH3) | H | |
| 1-72 | 4-chlor-phenyl | CH2 | CH2 | CH(i-propyl) | H | |
| 1-73 | 4-fluor-phenyl | CH2 | CH2 | CH2 | H | |
| 1-74 | 4-chlor-phenyl | CH2 | CH2 | CH(n-propyl) | H | |
| 1-75 | 4-chlor-phenyl | CH2 | CH2 | CH(t-butyl) | H | |
| 1-76 | 2-chlor-phenyl | CH2 | CH2 | CH2 | H | |
| | | | | | | Verbindung Nr. 1-77, Solvent: [DMSO], Spektrometer: 399.95MHz |
| 1-77 | 2-thienyl | CH2 | CH2 | CH2 | H | 8,0057 (0,68); 7,9926 (1,22); 7,9794 (0,73); 7,5501 (2,74); 7,5377 (2,9); 7,3195 (1,89); 7,3171 (1,8); 7,3068 (2,11); 7,3044 (1,92); 6,9512 (4,34); 6,9387 (4,46); 6,9303 (2,09); 6,8872 (2,22); 6,879 (1,86); 3,3316 (21,49); 3,2792 (1,19); 3,2623 (2,71); 3,2467 (2,76); 3,2299 (1,28); 2,857 (2,03); 2,8379 (3,54); 2,8189 (2,24); 2,5412 (3,08); 2,5059 (10,76); 2,5018 (14,05); 2,4977 (11,1); 2,3973 (16); 1,8846 (0,64); 1,8663 (1,92); 1,8478 (2,61); 1,8296 (1,87); 1,8113 (0,59) |
| 1-78 | 2,4-dichlor-phenyl | CH2 | CH2 | - | H | WO-A 2007/060166 |
| | | | | | | Verbindung Nr. 1-79, Solvent: [DMSO], Spektrometer: 601.6MHz |
| 1-79 | 4-chlor-2-thienyl | CH2 | CH2 | - | H | 8,0675 (0,49); 8,0583 (0,9); 8,0492 (0,47); 7,5538 (2,72); 7,5455 (2,82); 7,3741 (4,25); 7,3715 (4,3); 6,9466 (2,8); 6,9383 (2,73); 6,9062 (2,59); 6,9049 (2,22); 6,9037 (2,52); 3,4607 (1,01); 3,4491 (2,45); 3,4396 (2,5); 3,4282 (1,11); 3,3253 (249); 3,3101 (0,48); 3,0195 (1,62); 3,0086 (3,14); 3,0077 (3,17); 2,9969 (1,46); 2,653 (0,44); 2,6161 (0,36); 2,613 (0,49); 2,61 (0,35); 2,5474 (1,32); 2,5406 (144,26); 2,5223 (1,16); 2,5192 (1,5); 2,5161 (1,7); 2,5074 (27,92); 2,5044 (58,31); 2,5013 (79,87); 2,4982 (57,12); 2,4952 (26,25); 2,4245 (0,44); 2,3885 (0,37); 2,3854 (0,5); 2,3824 (0,37); 2,3611 (16); 2,0734 (0,47); -0,0002 (5,98) |
| | | | | | | Verbindung Nr. 1-80, Solvent: [DMSO], Spektrometer: 399.95MHz |
| 1-80 | 4-chlor-phenyl | N(CH3) | CH2 | CH2 | H | 8,0071 (0,64); 7,9933 (1,25); 7,9795 (0,66); 7,5515 (2,84); 7,5391 (2,93); 7,1827 (0,43); 7,1738 (4,27); 7,1685 (1,41); 7,1563 (1,57); 7,1511 (4,79); 7,1422 (0,51); 6,9457 (2,99); 6,9333 (2,88); 6,7718 (0,52); 6,763 (4,37); 6,7578 (1,48); 6,7403 (3,93); 6,7315 (0,44); 3,4764 (1,14); 3,4596 (2,71); 3,4424 (2,18); 3,3706 (1,25); 3,3551 (2,37); 3,3294 (60,06); 2,915 (15,97); 2,5413 (14,63); 2,5063 (24,52); 2,5019 (31,42); 2,4974 (23,16); 2,3739 (16); -0,0002 (4,01) |

**Tabelle 2**

| | | | | | | |
|---|---|---|---|---|---|---|
| Verbindungen der Formel I-2 | | | | | | |
| 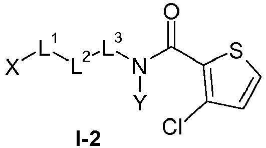 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 2-1 | 3-methyl-2-thienyl | CH2 | CH2 | - | H | |
| | | | | | | Verbindung Nr. 2-2, Solvent: [DMSO], Spektrometer: 399.95MHz |
| 2-2 | 2,4-dichlor-phenyl | CH2 | CH2 | CH2 | H | 8,2026 (1,69); 8,1889 (3,11); 8,175 (1,67); 7,8265 (15,43); 7,8133 (16); 7,8078 (0,37); 7,7959 (0,51); 7,7828 (0,47); 7,5677 (10,13); 7,5624 (10,39); 7,426 (5,49); 7,4053 (12,99); 7,3899 (0,44); 7,3803 (9,6); 7,375 (8,72); 7,3597 (3,95); 7,3544 (3,92); 7,1753 (0,46); 7,1623 (0,47); 7,1448 (15,95); 7,1316 (15,49); 3,5143 (0,38); 3,3561 (0,39); 3,3331 (88,56); 3,3198 (3,62); 3,3029 (7,61); 3,2879 (7,63); 3,2708 (3,31); 2,7545 (5,62); 2,7357 (6,9); 2,7158 (6,03); 2,6737 (0,32); 2,544 (14,87); 2,5272 (0,83); 2,5224 (1,34); 2,5138 (17,13); 2,5092 (34,25); 2,5046 (45,37); 2,5 (32,77); 2,4954 (15,38); 2,4798 (0,32); 1,8378 (1,65); 1,8198 (4,34); 1,8009 (5,53); 1,7822 (4,18); 1,7644 (1,46); -0,0002 (5,49) |
| 2-3 | 4-chlor-phenyl | CH2 | CH2 | - | H | [DMSO], Spektrometer: 399.95MHz 8.1377 (0.63); 8.1240 (1.15); 8.1101 (0.62); 7.8208 (4.47); 7.8077 (4.66); 7.3703 (0.45); 7.3642 (3.96); 7.3594 (1.50); 7.3482 (1.84); 7.3431 (6.97); 7.3374 (1.07); 7.2906 (0.99); 7.2846 (5.75); 7.2636 (3.41); 7.1343 (4.73); 7.1211 (4.56); 3.9045 (16.00); 3.5002 (1.31); 3.4829 (2.58); 3.4676 (2.57); 3.4498 (1.53); 3.3619 (349.85); 2.8484 (2.20); 2.8302 (3.85); 2.8125 (1.98); 2.6776 (0.36); 2.6732 (0.49); 2.6688 (0.37); 2.5428 (0.32); 2.5264 (1.67); 2.5130 (29.09); 2.5086 (57.80); 2.5041 (76.53); 2.4995 (57.82); 2.4951 (29.49); 2.3352 (0.34); 2.3308 (0.47); 2.3263 (0.34); -0.0002 (1.25) |
| 2-4 | 2,4-dichlor-phenyl | CH2 | CH2 | - | H | [DMSO], Spektrometer: 399.95MHz 8.2030 (0.50); 8.1889 (0.94); 8.1751 (0.49); 7.8178 (3.44); 7.8046 (3.60); 7.5878 (2.92); 7.3744 (6.79); 7.3714 (7.04); 7.1316 (3.64); 7.1185 (3.50); 3.9048 (16.00); 3.5280 (0.84); 3.5108 (2.14); 3.4957 (2.20); 3.4786 (0.89); 3.3931 (0.50); 3.3501 (207.99); 2.9699 (1.74); 2.9525 (3.42); 2.9352 (1.57); 2.6726 (0.42); 2.5258 (1.38); 2.5210 (2.13); 2.5124 (24.91); 2.5080 (49.70); 2.5035 (65.74); 2.4989 (49.43); 2.4945 (25.13); 2.3303 (0.40); -0.0002 (7.89) |
| 2-5 | 4-chlor-phenyl | CH(OCH3) | CH(CH3) | - | H | Verbindung Nr. 2-5, Solvent: [DMSO], Spektrometer: 399.95MHz 7,8969 (0,63); 7,8755 (0,65); 7,8395 (1,08); 7,8263 (1,16); 7,8054 (1,55); 7,7922 (1,61); 7,7428 (0,45); 7,7222 (0,45); 7,4341 (2,31); 7,4133 (3,65); 7,3501 (4,24); 7,329 (2,67); 7,1597 (1,12); 7,1465 (1,1); 7,1166 (1,63); 7,1034 (1,57); 4,3685 (0,72); 4,3558 (0,82); 4,314 (1,05); 4,2984 (1,23); 4,162 (0,32); 4,1446 (0,41); 4,1279 (0,52); 4,1231 (0,42); 4,1112 (0,37); 4,1065 (0,49); 3,3228 (9,49); 3,2061 (13,22); 2,5066 (21,23); 2,5023 (27,41); 2,498 (20,75); 1,3974 (16); 1,137 (3,55); 1,1202 (3,54); 1,0942 (2,48); 1,0773 (2,42); -0,0002 (4,23) |
| 2-6 | 2,4-dichlor-phenyl | CH(OCH3) | CH(CH3) | - | H | |
| 2-7 | 4-chlor-phenyl | CH(CH3) | CH2 | - | H | [DMSO], Spektrometer: 399.95MHz 8.0473 (0.68); 8.0332 (1.28); 8.0193 (0.67); 7.8099 (4.58); 7.7967 (4.74); 7.3752 (3.64); 7.3703 (1.40); 7.3590 (1.82); 7.3539 (7.44); 7.3483 (1.19); 7.3161 (1.22); 7.3107 (6.66); 7.3059 (1.87); 7.2941 (1.36); 7.2894 (3.39); 7.1195 (4.85); 7.1063 (4.69); 3.9091 (16.00); 3.5090 (0.43); 3.4617 (0.56); 3.4429 (0.79); 3.4235 (3.81); 3.4050 (6.69); 3.3749 (417.79); 3.1730 (0.36); 3.0954 (0.67); 3.0774 (1.33); 3.0595 (1.26); 3.0415 (0.62); 2.6831 (0.38); 2.6787 (0.51); 2.6743 (0.39); 2.5319 (1.66); 2.5184 (30.26); 2.5141 (60.39); 2.5096 (80.29); 2.5050 (60.84); 2.5007 (31.19); 2.3409 (0.35); 2.3363 (0.49); 2.3319 (0.36); 1.2350 (8.14); 1.2175 (7.98) |
| 2-8 | 2,4-dichlor-phenyl | CH(CH3) | CH2 | - | H | [DMSO], Spektrometer: 399.95MHz 7.7974 (0.68); 7.7842 (0.70); 7.5640 (0.54); 7.5587 (0.58); 7.4626 (0.64); 7.4268 (0.40); 7.4214 (0.37); 7.1100 (0.71); 7.0969 (0.68); 3.9045 (2.32); 3.5640 (0.34); 3.5473 (0.38); 3.5315 (0.46); 3.5182 (0.47); 3.5010 (0.76); 3.4291 (90.98); 2.5185 (4.75); 2.5141 (9.59); 2.5096 (12.81); 2.5050 (9.84); 2.5008 (5.14); 2.0747 (16.00); 1.2220 (1.09); 1.2053 (1.08) |
| 2-9 | 4-chlor-phenyl | CH2 | CH(CH3) | - | H | [DMSO], Spektrometer: 399.95MHz 7.9630 (1.22); 7.9424 (1.27); 7.7924 (4.06); 7.7793 (4.26); 7.3425 (3.49); 7.3378 (1.39); 7.3214 (6.93); 7.2752 (5.94); 7.2542 (3.24); 7.1119 (4.30); 7.0988 (4.20); 4.1713 (0.69); 4.1548 (0.99); 4.1385 (0.75); 4.1185 (0.38); 3.9043 (16.00); 3.4470 (0.40); 3.4286 (0.51); 3.3617 (361.09); 3.3140 (0.82); 3.1746 (0.46); 3.1619 (0.45); 2.8637 (0.63); 2.8435 (0.60); 2.8301 (1.62); 2.8098 (1.65); 2.7957 (1.67); 2.7803 (1.68); 2.7619 (0.65); 2.7468 (0.57); 2.6776 (0.40); 2.6731 (0.53); 2.6688 (0.41); 2.5262 (1.75); 2.5126 (30.67); 2.5085 (59.88); 2.5040 (78.76); 2.4995 (59.69); 2.4953 (30.88); 2.3350 (0.34); 2.3308 (0.48); 2.3263 (0.35); 1.1737 (7.79); 1.1572 (7.72) |
| 2-10 | 2,4-dichlor-phenyl | CH2 | CH(CH3) | - | H | |
| 2-11 | 4-chlor-phenyl | C(CH3)2 | CH2 | - | H | [DMSO], Spektrometer: 399.95MHz 7.8225 (2.75); 7.8093 (2.84); 7.7385 (0.36); 7.7237 (0.69); 7.7085 (0.36); 7.4631 (2.23); 7.4580 (0.84); 7.4467 (1.16); 7.4413 (3.99); 7.4348 (0.60); 7.3929 (0.56); 7.3863 (4.17); 7.3811 (1.11); 7.3696 (0.83); 7.3645 (2.30); 7.1202 (2.88); 7.1070 (2.78); 3.9093 (12.17); 3.4869 (2.95); 3.4713 (2.90); 3.3711 (239.20); 2.6784 (0.32); 2.5317 (1.02); 2.5183 (19.59); 2.5139 (38.92); 2.5093 (51.34); 2.5048 (38.69); 2.5004 (19.81); 2.3361 (0.32); 1.3129 (16.00) |
| 2-12 | 2,4-dichlor-phenyl | C(CH3)2 | CH2 | - | H | [DMSO], Spektrometer: 399.95MHz 7.8018 (3.13); 7.7887 (3.37); 7.7824 (0.47); 7.7666 (0.75); 7.7601 (0.43); 7.7512 (0.38); 7.5497 (2.38); 7.5439 (2.59); 7.4931 (1.53); 7.4714 (2.29); 7.3916 (1.64); 7.3857 (1.53); 7.3700 (1.12); 7.3641 (1.06); 7.0969 (3.23); 7.0837 (3.14); 3.9046 (14.38); 3.8154 (2.85); 3.7997 (2.83); 3.3628 (255.22); 2.6733 (0.37); 2.5266 (1.14); 2.5132 (21.80); 2.5088 (43.66); 2.5042 (57.88); 2.4997 (43.56); 2.4952 (22.23); 2.3310 (0.35); 1.4615 (16.00) |
| 2-13 | 2-chlor-phenyl | CH2 | CH2 | - | H | |
| 2-14 | 3,4-dichlor-phenyl | CH2 | CH2 | - | H | [DMSO], Spektrometer: 399.95MHz 8.1577 (0.61); 8.1436 (1.18); 8.1300 (0.67); 7.8199 (4.18); 7.8067 (4.38); 7.5614 (3.47); 7.5408 (4.24); 7.5347 (3.30); 7.5299 (3.39); 7.2628 (1.76); 7.2577 (1.77); 7.2422 (1.58); 7.2372 (1.57); 7.1321 (4.41); 7.1189 (4.29); 3.9046 (16.00); 3.5177 (1.16); 3.5006 (2.96); 3.4857 (3.01); 3.4688 (1.33); 3.4388 (0.42); 3.3608 (347.14); 3.1740 (0.40); 3.1622 (0.37); 2.8642 (2.11); 2.8470 (4.26); 2.8297 (1.93); 2.6779 (0.41); 2.6732 (0.53); 2.6688 (0.41); 2.5433 (0.40); 2.5264 (1.84); 2.5128 (31.80); 2.5086 (61.69); 2.5041 (80.66); 2.4996 (60.63); 2.4954 (31.03); 2.3351 (0.36); 2.3309 (0.51); 2.3264 (0.37); -0.0002 (5.83) |
| 2-15 | 3,5-dichlor-phenyl | CH2 | CH2 | - | H | |
| 2-16 | 3-chlor-phenyl | CH2 | CH2 | - | H | [DMSO], Spektrometer: 399.95MHz 8.1445 (0.64); 8.1314 (1.17); 8.1183 (0.66); 7.8222 (4.54); 7.8090 (4.73); 7.3499 (1.14); 7.3308 (5.66); 7.3115 (2.92); 7.2829 (2.38); 7.2782 (1.74); 7.2661 (0.88); 7.2627 (1.07); 7.2582 (0.70); 7.2282 (2.15); 7.2098 (1.50); 7.1341 (4.89); 7.1210 (4.79); 3.9043 (16.00); 3.5172 (1.29); 3.4998 (2.92); 3.4849 (3.21); 3.4675 (1.70); 3.4498 (0.41); 3.4387 (0.56); 3.3658 (387.37); 3.3078 (0.78); 3.2857 (0.39); 3.1739 (0.33); 2.8676 (2.33); 2.8499 (4.45); 2.8322 (2.20); 2.6779 (0.38); 2.6734 (0.54); 2.6690 (0.39); 2.5266 (1.74); 2.5132 (30.49); 2.5088 (60.36); 2.5043 (79.79); 2.4998 (60.13); 2.4954 (30.62); 2.3356 (0.37); 2.3311 (0.51); 2.3266 (0.38); - 0.0002 (0.33) |
| 2-17 | 2-fluor-phenyl | CH2 | CH2 | - | H | [DMSO], Spektrometer: 399.95MHz 8.1933 (0.69); 8.1795 (1.28); 8.1656 (0.73); 7.8226 (4.66); 7.8095 (4.87); 7.3443 (0.89); 7.3294 (1.55); 7.3251 (1.90); 7.3063 (1.47); 7.2930 (0.53); 7.2881 (1.10); 7.2736 (1.12); 7.2679 (1.39); 7.2636 (0.79); 7.2542 (0.78); 7.2498 (0.65); 7.1803 (1.50); 7.1596 (3.11); 7.1432 (2.78); 7.1405 (2.70); 7.1363 (6.30); 7.1231 (5.81); 3.9084 (16.00); 3.5173 (1.70); 3.5002 (3.08); 3.4843 (3.12); 3.4669 (1.92); 3.3799 (457.67); 3.2922 (0.60); 3.1793 (0.73); 3.1668 (0.69); 2.9003 (2.23); 2.8823 (4.00); 2.8645 (2.04); 2.6830 (0.41); 2.6785 (0.55); 2.6740 (0.41); 2.5485 (0.41); 2.5315 (1.86); 2.5181 (31.81); 2.5139 (62.45); 2.5094 (82.23); 2.5049 (62.52); 2.3405 (0.38); 2.3361 (0.52); 2.3315 (0.39) |
| 2-18 | 2,6-difluor-phenyl | CH2 | CH2 | - | H | [DMSO], Spektrometer: 399.95MHz 7.8107 (0.99); 7.7975 (1.05); 7.3236 (0.37); 7.1264 (1.05); 7.1132 (1.03); 7.0797 (0.51); 7.0599 (0.82); 7.0399 (0.45); 3.9037 (3.15); 3.4886 (0.91); 3.4718 (1.79); 3.4204 (120.43); 2.9156 (0.41); 2.8983 (0.78); 2.8811 (0.38); 2.5302 (0.44); 2.5170 (6.82); 2.5126 (13.44); 2.5081 (17.68); 2.5035 (13.27); 2.4992 (6.71); 2.0740 (16.00) |
| 2-19 | 2,6-dichlor-phenyl | CH2 | CH2 | - | H | [DMSO], Spektrometer: 399.95MHz 8.2795 (0.51); 8.2654 (0.95); 8.2512 (0.49); 7.8182 (3.62); 7.8050 (3.75); 7.4669 (4.20); 7.4468 (5.88); 7.3079 (2.06); 7.2888 (2.09); 7.2868 (1.90); 7.2677 (1.33); 7.1346 (3.78); 7.1214 (3.63); 3.9095 (16.00); 3.5349 (0.88); 3.5183 (2.14); 3.5022 (2.18); 3.4853 (1.14); 3.3672 (282.29); 3.1867 (1.81); 3.1690 (3.28); 3.1517 (1.47); 2.6784 (0.42); 2.5317 (1.42); 2.5183 (25.31); 2.5139 (50.14); 2.5093 (65.95); 2.5048 (49.33); 2.5004 (24.84); 2.3361 (0.42) |
| 2-20 | 3-(trifluormethyl)-phenyl | CH2 | CH2 | - | H | [DMSO], Spektrometer: 399.95MHz 8.1552 (0.57); 8.1420 (1.01); 8.1284 (0.54); 7.8190 (3.83); 7.8058 (3.95); 7.6056 (2.31); 7.5919 (0.86); 7.5829 (0.97); 7.5682 (4.08); 7.5548 (2.45); 7.5421 (0.96); 7.5310 (0.48); 7.5185 (0.32); 7.1293 (4.02); 7.1161 (3.87); 3.9045 (16.00); 3.5536 (1.08); 3.5363 (2.64); 3.5216 (2.73); 3.5044 (1.32); 3.3692 (384.74); 3.1744 (0.37); 3.1628 (0.33); 2.9655 (1.88); 2.9481 (3.71); 2.9307 (1.71); 2.6783 (0.36); 2.6740 (0.49); 2.6693 (0.36); 2.5271 (1.74); 2.5137 (30.05); 2.5093 (59.74); 2.5047 (79.00); 2.5002 (59.85); 2.4958 (30.81); 2.3359 (0.37); 2.3314 (0.52); 2.3270 (0.39) |
| 2-21 | 4-(trifluormethyl)-phenyl | CH2 | CH2 | - | H | [DMSO], Spektrometer: 399.95MHz 8.1817 (0.49); 8.1676 (0.91); 8.1538 (0.48); 7.8205 (3.55); 7.8074 (3.67); 7.6734 (2.50); 7.6532 (3.05); 7.4918 (2.75); 7.4718 (2.26); 7.1329 (3.74); 7.1197 (3.60); 3.9045 (16.00); 3.5502 (0.98); 3.5329 (2.08); 3.5177 (2.07); 3.5002 (1.15); 3.4504 (0.42); 3.3701 (329.49); 3.3107 (0.59); 3.1687 (0.49); 2.9563 (1.43); 2.9385 (2.63); 2.9208 (1.28); 2.6740 (0.41); 2.5440 (0.34); 2.5272 (1.46); 2.5138 (24.76); 2.5094 (49.66); 2.5048 (65.83); 2.5003 (49.54); 2.4958 (25.02); 2.3316 (0.42) |
| 2-22 | 2-methyl-phenyl | CH2 | CH2 | - | H | [DMSO], Spektrometer: 399.95MHz 8.2055 (0.56); 8.1914 (1.03); 8.1774 (0.57); 7.8320 (4.41); 7.8188 (4.61); 7.1771 (0.88); 7.1689 (1.69); 7.1554 (3.06); 7.1467 (6.97); 7.1392 (1.32); 7.1335 (5.44); 7.1284 (3.20); 7.1252 (3.44); 7.1159 (2.95); 7.1098 (1.36); 7.1039 (1.71); 3.9042 (14.10); 3.4495 (1.45); 3.4344 (2.40); 3.4312 (2.13); 3.4261 (1.89); 3.4160 (2.67); 3.4114 (3.29); 3.3961 (4.40); 3.3681 (316.33); 3.3082 (0.66); 3.2853 (0.33); 3.1749 (0.37); 3.1622 (0.37); 2.8482 (2.20); 2.8286 (2.58); 2.8100 (1.99); 2.6780 (0.33); 2.6736 (0.46); 2.6690 (0.34); 2.5268 (1.44); 2.5134 (25.01); 2.5090 (49.94); 2.5044 (65.99); 2.4998 (49.40); 2.4954 (24.78); 2.3244 (16.00); -0.0002 (5.37) |
| 2-23 | 2,4,6-trimethyl-phenyl | CH2 | CH2 | - | H | [DMSO], Spektrometer: 399.95MHz 8.3362 (0.37); 8.3218 (0.76); 8.3073 (0.39); 7.8493 (2.28); 7.8361 (2.39); 7.1654 (2.37); 7.1522 (2.31); 6.8107 (4.16); 3.9088 (8.26); 3.3796 (236.40); 3.2968 (0.78); 3.2819 (1.10); 3.2684 (1.01); 3.2614 (0.85); 3.2551 (1.02); 3.2410 (0.74); 3.1736 (0.50); 2.8176 (1.08); 2.8040 (0.84); 2.7966 (1.09); 2.7767 (0.93); 2.5316 (1.03); 2.5180 (16.89); 2.5140 (33.01); 2.5095 (43.47); 2.5050 (32.99); 2.5007 (17.03); 2.3364 (0.34); 2.3086 (16.00); 2.1862 (7.13) |
| 2-24 | 3,4-bismethoxy-phenyl | CH2 | CH2 | - | H | [DMSO], Spektrometer: 399.95MHz 8.0798 (0.48); 8.0658 (0.95); 8.0519 (0.52); 7.8244 (3.04); 7.8112 (3.20); 7.1400 (3.23); 7.1268 (3.15); 6.8786 (2.01); 6.8582 (2.84); 6.8405 (2.24); 6.8359 (2.53); 6.7659 (1.43); 6.7612 (1.32); 6.7456 (1.05); 6.7408 (0.98); 3.9041 (10.94); 3.7302 (15.17); 3.7118 (16.00); 3.4897 (1.07); 3.4726 (1.98); 3.4550 (2.16); 3.4389 (1.82); 3.3794 (338.51); 3.1752 (0.59); 3.1627 (0.56); 2.7848 (1.52); 2.7663 (2.52); 2.7485 (1.40); 2.6744 (0.40); 2.5275 (1.29); 2.5140 (22.66); 2.5097 (44.97); 2.5052 (59.64); 2.5007 (45.41); 2.4964 (23.63); 2.3318 (0.37) |
| 2-25 | phenyl | CH2 | CH2 | - | H | [DMSO], Spektrometer: 399.95MHz 7.8131 (0.94); 7.7999 (0.98); 7.2995 (0.82); 7.2815 (0.86); 7.2536 (1.09); 7.2366 (0.51); 7.2041 (0.44); 7.1292 (1.00); 7.1160 (0.98); 3.8956 (2.67); 3.5033 (0.61); 3.4857 (1.00); 3.4706 (1.25); 3.4669 (1.37); 3.4512 (1.93); 3.4109 (103.28); 2.8478 (0.54); 2.8288 (0.80); 2.8109 (0.49); 2.5087 (5.66); 2.5044 (11.35); 2.4998 (15.07); 2.4953 (11.41); 2.4910 (5.85); 2.0658 (16.00) |
| 2-26 | 4-chlor-phenyl | C(CH2-CH2) | CH2 | - | H | |
| 2-27 | 2,4-dichlor-phenyl | C(CH2-CH2) | CH2 | - | H | |
| 2-28 | 4-chlor-phenyl | CH2 | C(CH2-CH2) | - | H | |
| 2-29 | 2,4-dichlor-phenyl | CH2 | C(CH2-CH2) | - | H | |
| 2-30 | 4-chlor-phenyl | O | CH2 | CH2 | H | |
| | | | | | | Verbindung Nr. 2-31, Solvent: [DMSO], Spektrometer: 399.95MHz |
| 2-31 | 2,4-dichlor-phenyl | O | CH2 | CH2 | H | 8,2015 (2,25); 8,1882 (4,21); 8,1747 (2,31); 7,8594 (12,24); 7,8462 (12,76); 7,8257 (0,4); 7,8126 (0,38); 7,5737 (10,75); 7,5672 (11,64); 7,3865 (5,52); 7,38 (5,32); 7,3643 (7,63); 7,3579 (7,46); 7,2503 (12,26); 7,2281 (9,04); 7,193 (0,39); 7,18 (0,4); 7,1629 (12,99); 7,1498 (12,64); 4,2325 (7,21); 4,2179 (16); 4,2033 (8,11); 3,6922 (4); 3,6779 (11,3); 3,6635 (11,02); 3,649 (3,76); 3,3342 (54,47); 2,5446 (1,72); 2,5275 (0,63); 2,514 (13,13); 2,5098 (26,04); 2,5054 (34,67); 2,5009 (26,43); 2,4969 (13,89); -0,0002 (1,22) |
| 2-32 | 4-chlor-phenyl | CH2 | CH2 | CH2 | H | |
| 2-33 | 4-chlor-phenyl | NCH3 | CH2 | CH2 | H | |
| 2-34 | 2,4-dichlor-phenyl | NCH3 | CH2 | CH2 | H | |
| 2-35 | 4-chlor-phenyl | CH(OCH3) | CH2 | - | H | |
| 2-36 | 2,4-dichlor-phenyl | CH(OCH3) | CH2 | - | H | |
| | | | | | | Verbindung Nr. 2-37, Solvent: [DMSO], Spektrometer: 399.95MHz |
| 2-37 | 2-thienyl | CH2 | CH2 | - | H | 8,1958 (2,47); 8,1831 (4,27); 8,1699 (2,52); 7,8355 (11,56); 7,8223 (12,07); 7,802 (0,38); 7,7886 (0,35); 7,357 (7,35); 7,3549 (7,31); 7,3443 (8,02); 7,3422 (7,7); 7,1756 (0,39); 7,1625 (0,44); 7,1454 (12,37); 7,1322 (12,09); 6,974 (5,53); 6,9653 (8,64); 6,9618 (5,98); 6,9529 (8,04); 6,9286 (9,27); 6,9216 (6,48); 3,528 (4,5); 3,5104 (10,49); 3,4953 (10,88); 3,4777 (5,35); 3,3282 (51,56); 3,0758 (9,06); 3,0578 (16); 3,0399 (7,9); 2,6707 (0,4); 2,5413 (3,35); 2,5407 (3,34); 2,5056 (48,75); 2,5015 (63,37); 2,4974 (49,66); 2,3282 (0,42); -0,0002 (1,08) |
| 2-38 | 3-thienyl | CH2 | CH2 | - | H | |
| 2-39 | 2-furyl | CH2 | CH2 | - | H | |
| 2-40 | 3-furyl | CH2 | CH2 | - | H | |
| 2-41 | phenyl | CH2 | CH2 | CH(CH3) | H | Verbindung Nr. 2-41, Solvent: [DMSO], Spektrometer: 399.95MHz¬7,9928 (2,22); 7,9721 (2,24); 7,8193 (7,33); 7,8061 (7,61); 7,2931 (2,88); 7,2744 (7,63); 7,256 (6,79); 7,2108 (8,88); 7,1928 (5,41); 7,1907 (5,41); 7,1679 (3,85); 7,1465 (8,46); 7,1334 (7,49); 3,9791 (0,57); 3,9589 (1,26); 3,9434 (1,66); 3,928 (1,31); 3,9073 (0,6); 3,3467 (186,19); 2,7002 (0,45); 2,6853 (0,67); 2,6766 (0,76); 2,6662 (1,84); 2,6512 (1,83); 2,642 (3,39); 2,6255 (3,16); 2,6186 (2,03); 2,6014 (1,79); 2,59 (0,61); 2,5843 (0,7); 2,5671 (0,65); 2,5426 (34,53); 2,5257 (0,87); 2,5078 (29,8); 2,5033 (39,32); 2,4989 (29,92); 2,0742 (0,39); 1,8911 (0,44); 1,8759 (0,54); 1,8687 (0,78); 1,8565 (1,22); 1,8426 (1,11); 1,8344 (1,66); 1,8206 (1,37); 1,8132 (1,08); 1,7978 (0,88); 1,7922 (0,95); 1,7757 (1,36); 1,7677 (1,03); 1,7612 (1,35); 1,7538 (1,35); 1,7372 (1,15); 1,7278 (0,59); 1,72 (0,61); 1,7037 (0,39); 1,179 (16); 1,1625 (15,89); 0,0079 (0,51); -0,0002 (12,6); -0,0081 (0,51) |
| | | | | | | Verbindung Nr. 2-42, Solvent: [DMSO], Spektrometer: 399.95MHz |
| 2-42 | phenyl | CH2 | CH2 | CH2 | H | 8,1619 (2,22); 8,1492 (3,77); 8,1364 (2,21); 7,8225 (12,02); 7,8093 (12,51); 7,7925 (0,43); 7,7793 (0,36); 7,3039 (4,87); 7,2852 (13,51); 7,2669 (12,29); 7,2291 (16); 7,2116 (8,86); 7,1958 (4,5); 7,1778 (6,66); 7,1601 (2,64); 7,1434 (12,73); 7,1302 (12,26); 3,33 (81,78); 3,2909 (4,21); 3,2739 (9,58); 3,2585 (9,62); 3,2414 (4,44); 2,6714 (0,55); 2,6501 (7,43); 2,6312 (11,09); 2,6116 (7,94); 2,5417 (52,11); 2,5067 (44,47); 2,5024 (57,75); 2,4981 (43,9); 2,3293 (0,35); 2,0751 (0,66); 1,8583 (2,34); 1,8398 (6,48); 1,8211 (8,65); 1,8026 (6,12); 1,7845 (2,02); 0,0079 (0,66); -0,0002 (16,13); -0,0083 (0,63) |
| 2-43 | 2-Cl-phenyl | CH2 | CH2 | CH(CH3) | H | |
| 2-44 | 4-t-butyl-phenyl | CH2 | CH2 | CH2 | H | |
| 2-45 | 4-methyl-phenyl | CH2 | CH2 | CH2 | H | |
| 2-46 | phenyl | CH2 | CH2 | CH(CH2CH3) | H | |
| 2-47 | 2-methoxy-phenyl | CH2 | CH2 | CH2 | H | |
| 2-48 | 2-methyl-phenyl | CH2 | CH2 | CH2 | H | |
| 2-49 | 3 -methyl-phenyl | CH2 | CH2 | CH2 | H | |
| 2-50 | 3-chlor-phenyl | CH2 | CH2 | CH2 | H | |
| 2-51 | 2,6-difluor-phenyl | CH2 | CH2 | CH2 | H | |
| 2-52 | 4-chlor-phenyl | CH2 | CH2 | CH2 | H | |
| 2-53 | 2,6-dichlor-phenyl | CH2 | CH2 | CH(CH3) | H | |
| 2-54 | 3,5-dichlor-phenyl | CH2 | CH2 | CH(CH3) | H | Verbindung Nr. 2-54, Solvent: [DMSO], Spektrometer: 399.95MHz¬79656 (2,6); 7,9449 (2,62); 7,8261 (7,86); 7,8129 (8,13); 7,4001 (3,5); 7,3956 (6,47); 7,3909 (3,83); 7,2997 (13,96); 7,295 (12,97); 7,1477 (8,13); 7,1345 (7,85); 3,9691 (0,58); 3,9485 (1,26); 3,934 (1,62); 3,9182 (1,33); 3,8974 (0,61); 3,3344 (29,05); 2,7154 (0,49); 2,7002 (0,6); 2,6924 (0,58); 2,6807 (1,87); 2,6595 (3,09); 2,6431 (3,84); 2,6217 (1,81); 2,6051 (0,63); 2,5872 (0,47); 2,5454 (21,6); 2,5145 (7,6); 2,5105 (14,83); 2,506 (19,57); 2,5015 (14,69); 2,4975 (7,45); 2,0793 (0,86); 1,8865 (0,37); 1,8709 (0,49); 1,8645 (0,77); 1,8518 (1,18); 1,8373 (1,16); 1,8304 (1,93); 1,8152 (1,77); 1,8089 (2); 1,7926 (2,06); 1,7856 (1,23); 1,7776 (1,53); 1,7725 (1,48); 1,7552 (1,24); 1,7441 (0,53); 1,7383 (0,52); 1,7349 (0,49); 1,721 (0,32); 1,1772 (16); 1,1607 (15,86); -0,0002 (6,07) |
| | 2,6-dimethyl-phenyl | | | | | Verbindung Nr. 2-55, Solvent: [DMSO], Spektrometer: 399.95MHz |
| 2-55 | | CH2 | CH2 | CH2 | H | 8,3181 (2,09); 8,2061 (3,02); 8,1928 (5,42); 8,1793 (3); 7,8283 (14,21); 7,8152 (14,72); 7,7982 (0,55); 7,785 (0,44); 7,5008 (13,04); 7,4945 (13,66); 7,4607 (11,99); 7,4393 (16); 7,4263 (0,41); 7,3226 (8,63); 7,3162 (8,14); 7,3012 (6,51); 7,2948 (6,08); 7,177 (0,45); 7,164 (0,52); 7,1465 (14,9); 7,1334 (14,36); 3,3364 (121,82); 3,3094 (12,94); 3,2937 (12,47); 3,2771 (5,11); 2,7645 (8,71); 2,7458 (12,16); 2,7259 (9,31); 2,6742 (0,47); 2,5447 (42,98); 2,5094 (42,43); 2,5053 (54,42); 2,5012 (42,03); 2,3321 (0,36); 2,0782 (1,56); 1,8532 (2,58); 1,8355 (7,45); 1,8163 (9,57); 1,7978 (7,17); 1,7802 (2,28); -0,0002 (8,32) |
| 2-56 | 2,5-dichlor-phenyl | CH2 | CH2 | CH2 | H | |
| 2-57 | 4-i-propoxy-phenyl | CH2 | CH2 | CH2 | H | |
| | | | | | | Verbindung Nr. 2-58, Solvent: [DMSO], Spektrometer: 399.95MHz |
| 2-58 | 3-trifluormethyl-phenyl | CH2 | CH2 | CH(CH3) | H | 8,3199 (0,91); 8,0068 (2,56); 7,9862 (2,54); 7,8258 (7,05); 7,8126 (7,3); 7,6239 (0,33); 7,5712 (5,61); 7,5578 (1,06); 7,5285 (15,03); 7,5018 (0,46); 7,4925 (0,48); 7,1496 (7,32); 7,1365 (7,06); 3,9918 (0,56); 3,9715 (1,26); 3,9561 (1,64); 3,941 (1,32); 3,9204 (0,61); 3,3349 (49,97); 3,3111 (0,7); 2,8065 (0,46); 2,7918 (0,64); 2,7825 (0,64); 2,772 (1,75); 2,7575 (1,88); 2,7481 (3,46); 2,7316 (3,2); 2,7249 (2,16); 2,7074 (1,8); 2,6961 (0,63); 2,6903 (0,69); 2,6734 (0,69); 2,5447 (25,9); 2,5097 (21,29); 2,5053 (27,97); 2,501 (21,36); 2,0779 (0,48); 1,9173 (0,38); 1,9022 (0,5); 1,8946 (0,74); 1,8825 (1,2); 1,8689 (1,17); 1,8605 (1,74); 1,8464 (1,52); 1,8387 (1,23); 1,8323 (1,15); 1,8158 (1,52); 1,8079 (1,18); 1,8015 (1,5); 1,7944 (1,48); 1,7778 (1,23); 1,7681 (0,59); 1,7605 (0,58); 1,7441 (0,37); 1,2098 (0,47); 1,1881 (16); 1,1716 (15,82); 0,0079 (0,33); -0,0002 (7,99); -0,0082 (0,33) |
| 2-59 | 4-methyl-phenyl | CH2 | CH2 | CH(CH3) | H | |
| 2-60 | 2-methyl-phenyl | CH2 | CH2 | CH(CH3) | H | |
| | | | | | | Verbindung Nr. 2-61, Solvent: [DMSO], Spektrometer: 399.95MHz |
| 2-61 | 3,4-dichlor-phenyl | CH2 | CH2 | CH(CH3) | H | 8,3183 (0,58); 7,9722 (2,55); 7,9515 (2,58); 7,8231 (7,37); 7,81 (7,6); 7,5329 (6,66); 7,5227 (0,38); 7,5123 (7,64); 7,4972 (6,4); 7,4924 (6,61); 7,227 (3,66); 7,2222 (3,6); 7,2064 (3,23); 7,2016 (3,16); 7,1458 (7,72); 7,1326 (7,5); 3,9633 (0,57); 3,9427 (1,24); 3,928 (1,62); 3,9124 (1,33); 3,8917 (0,6); 3,3365 (67,26); 3,313 (0,61); 2,7069 (0,4); 2,6917 (0,6); 2,6838 (0,59); 2,6722 (1,93); 2,6509 (2,99); 2,6345 (3,91); 2,6137 (1,84); 2,5968 (0,64); 2,579 (0,51); 2,5446 (23,11); 2,5272 (0,55); 2,5096 (19,92); 2,5052 (26,41); 2,5007 (20,02); 2,0781 (0,85); 1,8847 (0,39); 1,869 (0,51); 1,8628 (0,82); 1,8497 (1,2); 1,8406 (0,78); 1,8354 (1,14); 1,8287 (1,93); 1,8137 (1,66); 1,8072 (1,27); 1,7996 (1,1); 1,7914 (1,12); 1,7826 (1,43); 1,7765 (1,14); 1,7683 (1,47); 1,7635 (1,47); 1,7459 (1,26); 1,7348 (0,57); 1,7292 (0,56); 1,7258 (0,53); 1,712 (0,36); 1,1747 (16); 1,1582 (15,98); - 0,0002 (5,09) |
| | | | | | | Verbindung Nr. 2-62, Solvent: [DMSO], Spektrometer: 399.95MHz |
| 2-62 | 3,5-dichlor-phenyl | CH2 | CH2 | CH2 | H | 8,1498 (1,56); 8,1366 (2,79); 8,1233 (1,56); 7,8286 (8,44); 7,8154 (8,79); 7,4104 (4,01); 7,406 (7,09); 7,4014 (4,27); 7,3289 (16); 7,3244 (14,18); 7,1458 (8,75); 7,1326 (8,5); 3,3373 (40,62); 3,278 (2,6); 3,2612 (6,29); 3,2459 (6,37); 3,2291 (2,75); 2,6748 (4,59); 2,6562 (7,63); 2,6371 (4,76); 2,5459 (24,64); 2,5109 (16,05); 2,5065 (20,86); 2,5021 (15,75); 2,0796 (0,8); 1,8661 (1,37); 1,8481 (4,23); 1,8296 (5,7); 1,8114 (4,02); 1,7937 (1,19); -0,0002 (4,86) |
| 2-63 | 2,6-dimethyl-phenyl | CH2 | CH2 | CH(CH3) | H | Verbindung Nr. 2-63, Solvent: [DMSO], Spektrometer: 399.95MHz 8,032 (0,6); 8,012 (0,61); 7,827 (1,81); 7,8139 (1,91); 7,1533 (1,91); 7,1402 (1,89); 6,9544 (7,38); 4,0757 (0,36); 4,0586 (0,44); 4,0408 (0,37); 3,4371 (0,33); 3,4069 (0,64); 3,3327 (1566,67); 3,2526 (0,79); 2,6753 (2,51); 2,6709 (3,44); 2,6667 (2,56); 2,6427 (0,6); 2,632 (0,64); 2,6164 (0,66); 2,6043 (0,67); 2,5877 (0,81); 2,5412 (8,13); 2,524 (10,07); 2,5063 (342,36); 2,5018 (450,83); 2,4974 (340,1); 2,333 (2,2); 2,3286 (2,98); 2,3242 (2,23); 2,2892 (0,34); 2,2684 (16); 2,074 (1,53); 1,6333 (0,42); 1,6129 (0,75); 1,5953 (0,83); 1,5745 (0,42); 1,2272 (3,92); 1,2105 (3,81); -0,0002 (14,36) |
| | | | | | | Verbindung Nr. 2-64, Solvent: [DMSO], Spektrometer: 399.95MHz |
| 2-64 | 4-trifluormethylphenyl | CH2 | CH2 | CH(CH3) | H | 8,0067 (2,51); 7,9859 (2,51); 7,8239 (7,71); 7,8108 (7,96); 7,6441 (6,59); 7,6238 (8,04); 7,452 (7,31); 7,432 (6,19); 7,147 (8,01); 7,1339 (7,7); 3,9855 (0,56); 3,9649 (1,23); 3,95 (1,59); 3,9344 (1,3); 3,9139 (0,6); 3,3391 (108,58); 3,1416 (0,54); 3,1188 (0,5); 2,7982 (0,34); 2,7829 (0,51); 2,7749 (0,48); 2,7634 (1,56); 2,7433 (2,75); 2,7255 (3,38); 2,705 (1,59); 2,6909 (0,61); 2,67 (0,54); 2,5443 (31,93); 2,5273 (0,69); 2,5136 (12,32); 2,5094 (24,5); 2,5049 (32,71); 2,5005 (24,74); 2,4964 (12,74); 2,077 (0,63); 1,923 (0,4); 1,9073 (0,5); 1,9008 (0,81); 1,8879 (1,13); 1,8787 (0,77); 1,8739 (1,09); 1,8668 (1,76); 1,8519 (1,5); 1,8453 (1,15); 1,8308 (1,44); 1,815 (1,37); 1,8081 (1,1); 1,8008 (1,39); 1,7948 (1,37); 1,7777 (1,19); 1,7672 (0,59); 1,761 (0,57); 1,7439 (0,37); 1,1872 (15,95); 1,1707 (16); - 0,0002 (5,54) |
| 2-65 | 2,5-dichlor-phenyl | CH2 | CH2 | CH(CH3) | H | Verbindung Nr. 2-65, Solvent: [DMSO], Spektrometer: 399.95MHz¬8,0047 (2,66); 7,984 (2,69); 7,8266 (7,82); 7,8135 (8,07); 7,4572 (6,76); 7,4506 (12,88); 7,4292 (8,59); 7,312 (4,56); 7,3056 (4,19); 7,2907 (3,38); 7,2842 (3,13); 7,1485 (8,14); 7,1353 (7,92); 4,03 (0,63); 4,0109 (1,35); 3,9946 (1,87); 3,9783 (1,44); 3,9592 (0,66); 3,337 (33,58); 2,8178 (0,53); 2,8018 (0,69); 2,7949 (0,75); 2,7834 (1,75); 2,7681 (1,8); 2,7601 (2,24); 2,7527 (1,93); 2,7443 (1,99); 2,7351 (2,36); 2,7294 (1,88); 2,7123 (1,81); 2,7002 (0,8); 2,6959 (0,69); 2,6778 (0,67); 2,5459 (21,32); 2,5291 (0,42); 2,5111 (14,05); 2,5066 (18,48); 2,5022 (13,85); 2,0797 (1,06); 1,8417 (0,35); 1,8237 (1,25); 1,8093 (2); 1,8 (2,44); 1,7954 (2,45); 1,7871 (2,82); 1,7808 (2,77); 1,7729 (2,03); 1,7659 (1,51); 1,756 (1,33); 1,7399 (0,34); 1,2061 (16); 1,1895 (15,93); -0,0002 (5,28) |
| 2-66 | 4-phenoxy-phenyl | CH2 | CH2 | CH2 | H | |
| 2-67 | 3-chlor-phenyl | CH2 | CH2 | CH(CH3) | H | |
| 2-68 | 4-phenoxy-phenyl | CH2 | CH2 | CH(CH3) | H | |
| 2-69 | 2,4-dichlor-phenyl | CH2 | CH2 | CH(CH3) | H | |
| 2-70 | 2-difluormethoxy-phenyl | CH2 | CH2 | CH(CH3) | H | Verbindung Nr. 2-70, Solvent: [DMSO], Spektrometer: 399.95MHz¬8,3169 (0,83); 7,9735 (2,48); 7,9528 (2,5); 7,8211 (8,06); 7,8079 (8,36); 7,3656 (3,83); 7,3272 (2,7); 7,3233 (3,07); 7,3086 (3,46); 7,3047 (3,93); 7,2864 (1,41); 7,2821 (1,36); 7,2671 (3,42); 7,2628 (3,05); 7,2477 (2,97); 7,2433 (2,47); 7,1952 (2,86); 7,1925 (3,33); 7,1796 (8,97); 7,174 (5,06); 7,1526 (5,06); 7,1455 (9,38); 7,1323 (11,35); 6,9939 (3,94); 3,9968 (0,59); 3,9773 (1,28); 3,9613 (1,77); 3,9452 (1,36); 3,9256 (0,61); 3,3351 (80,76); 3,3113 (0,69); 2,7451 (0,57); 2,7303 (0,72); 2,7209 (0,8); 2,7108 (1,67); 2,6961 (1,56); 2,6865 (1,8); 2,6709 (2,03); 2,6504 (1,83); 2,6427 (1,62); 2,6268 (1,64); 2,6161 (0,79); 2,6086 (0,76); 2,5924 (0,67); 2,5423 (33,94); 2,5255 (0,69); 2,5118 (11,77); 2,5075 (23,24); 2,503 (30,78); 2,4985 (23,06); 2,4942 (11,62); 2,0754 (1,11); 1,8303 (0,44); 1,8219 (0,49); 1,8114 (1,2); 1,7969 (1,17); 1,7877 (1,66); 1,7771 (1,65); 1,7731 (1,52); 1,7665 (2,34); 1,7505 (1,84); 1,7409 (1,62); 1,7309 (0,81); 1,7252 (1,09); 1,7164 (0,42); 1,7077 (0,54); 1,1919 (16); 1,1753 (15,87); -0,0002 (6,33) |
| 2-71 | 4-methoxy-phenyl | CH2 | CH2 | CH2 | H | |
| 2-72 | 4-chlor-phenyl | CH2 | CH2 | CH(CH3) | H | Verbindung Nr. 2-72, Solvent: [DMSO], Spektrometer: 399.95MHz¬7,9867 (2,32); 7,9661 (2,36); 7,8197 (7,69); 7,8066 (8,01); 7,3355 (7,83); 7,3309 (2,86); 7,3192 (3,58); 7,3145 (12,43); 7,3085 (1,76); 7,2423 (10,76); 7,2214 (6,89); 7,1448 (8,08); 7,1316 (7,83); 3,9585 (0,56); 3,9379 (1,2); 3,9232 (1,56); 3,9074 (1,25); 3,887 (0,57); 3,3692 (0,6); 3,3315 (338,35); 3,2986 (0,6); 2,7118 (0,33); 2,6914 (0,43); 2,6761 (1,11); 2,671 (1,14); 2,6673 (1,05); 2,6571 (1,73); 2,6419 (2); 2,6349 (3,2); 2,6186 (3,68); 2,5962 (1,77); 2,5832 (0,68); 2,5788 (0,73); 2,5608 (0,77); 2,5415 (69,61); 2,5246 (2,52); 2,511 (43,01); 2,5067 (84,57); 2,5022 (111,66); 2,4977 (83,25); 2,4934 (41,77); 2,3334 (0,52); 2,3289 (0,73); 2,3245 (0,53); 2,0746 (0,52); 1,8747 (0,4); 1,8591 (0,51); 1,8526 (0,8); 1,8401 (1,14); 1,8261 (1,03); 1,8186 (1,69); 1,804 (1,42); 1,7971 (1,05); 1,7804 (1,16); 1,7621 (1,26); 1,7553 (0,99); 1,7478 (1,32); 1,7418 (1,29); 1,7245 (1,12); 1,7141 (0,56); 1,7078 (0,57); 1,6909 (0,37); 1,1707 (16); 1,1542 (15,9); 0,0079 (0,47); -0,0002 (12,91); -0,0085 (0,49) |
| 2-73 | 4-chlor-phenyl | CH2 | CH2 | CH(i-propyl) | H | Verbindung Nr. 2-73, Solvent: [DMSO], Spektrometer: 399.95MHz 7,8783 (1,86); 7,8552 (1,89); 7,8267 (4,97); 7,8135 (5,15); 7,3278 (5,08); 7,3234 (1,93); 7,3068 (8,36); 7,3012 (1,31); 7,2428 (7,42); 7,2218 (4,62); 7,1557 (5,24); 7,1426 (5,07); 3,8106 (0,37); 3,7973 (0,6); 3,7872 (1,02); 3,7746 (1,18); 3,7628 (1,01); 3,7518 (0,64); 3,7389 (0,38); 3,3356 (51,87); 2,691 (0,46); 2,6766 (0,69); 2,6686 (0,67); 2,6559 (1,09); 2,6415 (0,96); 2,6351 (0,92); 2,6197 (0,8); 2,5656 (0,8); 2,5434 (16,86); 2,5252 (1,33); 2,5086 (15,1); 2,5041 (19,29); 2,4997 (14,5); 2,4957 (7,55); 2,0766 (0,66); 1,8277 (0,33); 1,8115 (0,99); 1,7946 (1,75); 1,7793 (2,43); 1,7614 (2,44); 1,7442 (1,76); 1,7209 (0,66); 0,8907 (16); 0,8737 (15,72); - 0,0002 (3,83) |
| 2-74 | 4-fluor-phenyl | CH2 | CH2 | CH2 | H | Verbindung Nr. 2-74, Solvent: [DMSO], Spektrometer: 399.95MHz¬8,1571 (2,3); 8,1445 (3,97); 8,1314 (2,32); 7,8233 (13,46); 7,8101 (14); 7,7932 (0,52); 7,7801 (0,41); 7,2736 (6,93); 7,2686 (3,51); 7,2592 (8,85); 7,2522 (10); 7,238 (8,69); 7,1744 (0,47); 7,1614 (0,51); 7,1437 (14,16); 7,1306 (14,46); 7,1211 (10,44); 7,1159 (3,53); 7,0987 (16); 7,0931 (4,15); 7,0813 (3,4); 7,0766 (7,52); 7,069 (0,99); 3,3804 (0,36); 3,3684 (0,65); 3,3369 (163,66); 3,298 (0,48); 3,2807 (4,34); 3,2637 (9,62); 3,2486 (9,89); 3,2314 (4,51); 2,6774 (0,37); 2,6727 (0,48); 2,6685 (0,4); 2,6413 (6,93); 2,6225 (11,06); 2,6031 (7,46); 2,5431 (57,57); 2,5261 (1,62); 2,5122 (25,08); 2,5082 (46,38); 2,5038 (58,81); 2,4993 (43,77); 2,3305 (0,39); 2,0761 (0,8); 1,842 (2,3); 1,8236 (6,48); 1,8051 (8,74); 1,7868 (6,2); 1,7686 (2,03); -0,0002 (7,35) |
| 2-75 | 4-chlor-phenyl | CH2 | CH2 | CH(n-propyl) | H | Verbindung Nr. 2-75, Solvent: [DMSO], Spektrometer: 399.95MHz¬7,913 (2,57); 7,8908 (2,61); 7,8173 (7,32); 7,8042 (7,58); 7,3287 (7,24); 7,3242 (2,78); 7,3123 (3,44); 7,3077 (11,59); 7,3019 (1,79); 7,2372 (10,16); 7,2162 (6,51); 7,1456 (7,76); 7,1325 (7,5); 3,934 (0,76); 3,9158 (1,37); 3,8985 (1,39); 3,8803 (0,8); 3,3318 (66,92); 2,687 (0,51); 2,6681 (1,18); 2,6522 (1,5); 2,6336 (2,24); 2,6145 (2,4); 2,5944 (2,63); 2,574 (1,47); 2,5596 (1,23); 2,5427 (30,42); 2,5259 (0,81); 2,5122 (12,8); 2,5079 (25,23); 2,5034 (33,46); 2,4988 (25,21); 2,4945 (12,78); 2,0761 (0,7); 1,7893 (1,87); 1,7697 (3,93); 1,7521 (4,42); 1,7329 (1,74); 1,5174 (0,93); 1,5077 (1,11); 1,5022 (1,44); 1,4936 (2,52); 1,4751 (3,44); 1,4574 (2); 1,3922 (0,39); 1,3749 (0,68); 1,359 (1,34); 1,3408 (1,88); 1,3332 (1,34); 1,3234 (1,73); 1,3153 (1,85); 1,3097 (1,31); 1,2973 (1,52); 1,2927 (1,32); 1,2815 (0,85); 1,2751 (0,93); 1,2636 (0,48); 1,2594 (0,51); 0,8802 (7,91); 0,862 (16); 0,8437 (6,75); 0,0079 (0,5); -0,0002 (13,95); -0,0084 (0,56) |
| 2-76 | 4-chlor-phenyl | CH2 | CH2 | CH(t-butyl) | H | Verbindung Nr. 2-76, Solvent: [DMSO], Spektrometer: 399.95MHz7,8382 (0,75); 7,8328 (2,07); 7,8196 (2,18); 7,8147 (0,8); 7,3251 (1,73); 7,3205 (0,67); 7,304 (3,07); 7,2498 (2,74); 7,2287 (1,61); 7,1615 (1,96); 7,1484 (1,9); 3,7538 (0,64); 3,7297 (0,33); 3,3375 (20,94); 2,6423 (0,42); 2,5436 (5,53); 2,5129 (2,71); 2,5088 (5,29); 2,5043 (6,98); 2,4998 (5,29); 2,4957 (2,88); 2,4775 (0,4); 2,4722 (0,39); 2,4543 (0,34); 1,8132 (0,35); 0,8896 (16); -0,0002 (1,78) |
| | | | | | | Verbindung Nr. 2-77, Solvent: [DMSO], Spektrometer: 399.95MHz |
| 2-77 | 2-chlor-phenyl | CH2 | CH2 | CH2 | H | 8,3164 (1,67); 8,2073 (2,43); 8,1943 (4,32); 8,1807 (2,43); 7,825 (15,3); 7,8118 (15,85); 7,7946 (0,56); 7,7814 (0,47); 7,4225 (6,56); 7,4191 (6,47); 7,4033 (8,42); 7,3997 (8,42); 7,3877 (5,38); 7,3833 (5,76); 7,369 (7,45); 7,3647 (7,89); 7,3035 (3,37); 7,3001 (3,73); 7,2852 (8,28); 7,2816 (7,74); 7,2668 (5,54); 7,263 (4,67); 7,2539 (6,07); 7,2491 (6,17); 7,2348 (6,74); 7,2303 (6,52); 7,2161 (2,64); 7,2116 (2,32); 7,1756 (0,5); 7,1625 (0,55); 7,1452 (16); 7,132 (15,45); 7,1237 (0,48); 3,3931 (0,48); 3,3741 (1,12); 3,341 (242,72); 3,3129 (11,63); 3,2976 (11); 3,2806 (4,82); 2,77 (8,21); 2,7511 (10,28); 2,7311 (8,72); 2,7137 (0,41); 2,6775 (0,46); 2,6731 (0,59); 2,5434 (63,34); 2,5263 (1,92); 2,5128 (26,19); 2,5086 (50,46); 2,5041 (66,35); 2,4996 (49,74); 2,4953 (25,17); 2,3309 (0,42); 2,0761 (1,44); 1,8538 (2,51); 1,8356 (6,53); 1,8167 (8,34); 1,798 (6,21); 1,78 (2,14); 0,0079 (0,4); -0,0002 (9,67); -0,0085 (0,36) |
| | | | | | | Verbindung Nr. 2-78, Solvent: [DMSO], Spektrometer: 399.95MHz |
| 2-78 | 2-thienyl | CH2 | CH2 | CH2 | H | 8,1901 (2,91); 8,1777 (5); 8,1657 (3,06); 7,8261 (12,85); 7,825 (12,4); 7,8129 (13,52); 7,8119 (13,05); 7,7953 (0,6); 7,7822 (0,44); 7,3203 (8,78); 7,3076 (9,55); 7,1752 (0,42); 7,1621 (0,47); 7,1455 (13,66); 7,1445 (13,08); 7,1323 (13,35); 7,1313 (12,76); 6,9515 (6,41); 6,9427 (8,97); 6,9391 (7,19); 6,9302 (8,23); 6,8872 (10,26); 6,8791 (8,24); 3,3314 (106,28); 3,3073 (13,58); 3,2915 (13,3); 3,2749 (5,96); 2,872 (9,41); 2,853 (16); 2,8339 (10,05); 2,6709 (0,58); 2,5417 (2,78); 2,5407 (2,62); 2,502 (75,43); 2,4981 (60,55); 2,3286 (0,49); 1,9003 (2,93); 1,8821 (9,04); 1,8636 (12,17); 1,8453 (8,71); 1,8273 (2,7); -0,0002 (0,7) |
| | | | | | | [DMSO], Spektrometer: 399,95MHz |
| 2-79 | 2,6-difluor-phenyl | CH2 | CH(CH3) | - | H | 7,9988 (3,15); 7,9778 (3,21); 7,7997 (7,73); 7,7866 (8,07); 7,7711 (0,4); 7,3519 (0,8); 7,3316 (2,07); 7,3143 (3,69); 7,2939 (2,51); 7,2767 (1,05); 7,1338 (0,37); 7,1153 (8,38); 7,1022 (8,12); 7,0815 (0,95); 7,0692 (5,57); 7,0498 (8,8); 7,03 (4,77); 7,0178 (0,82); 4,2991 (0,8); 4,2811 (1,87); 4,263 (2,47); 4,245 (1,98); 4,2275 (0,89); 3,3347 (26,57); 2,9301 (0,44); 2,8921 (5,54); 2,876 (6,4); 2,8396 (0,39); 2,6724 (0,5); 2,5073 (57,25); 2,5032 (75,74); 2,4992 (61,74); 2,33 (0,51); 1,336 (0,44); 1,2491 (0,44); 1,2325 (0,34); 1,1897 (15,85); 1,173 (16); 0,0078 (1,35); -0,0002 (27,44) |
| | | | | | | Verbindung Nr. 2-80, Solvent: [DMSO], Spektrometer: 399.95MHz |
| 2-80 | 4-chlor-phenyl | CH2 | CH2 | - | CH3 | 8,3205 (0,33); 7,7755 (5,45); 7,7655 (5,62); 7,3258 (9,27); 7,1826 (0,48); 7,0807 (9,65); 7,0682 (9,73); 4,0378 (0,69); 4,02 (0,69); 3,6776 (3,09); 3,5874 (0,71); 3,4941 (2,32); 3,3355 (59,17); 3,0207 (4,31); 2,8772 (10,16); 2,6765 (0,74); 2,6724 (0,97); 2,6681 (0,75); 2,5077 (99,47); 2,5033 (128,84); 2,4989 (96,92); 2,3344 (0,65); 2,3302 (0,86); 2,3258 (0,64); 2,08 (0,37); 2,0357 (0,37); 1,9901 (2,95); 1,397 (16); 1,3364 (0,65); 1,2491 (0,76); 1,1929 (0,8); 1,1751 (1,54); 1,1573 (0,76); 0,1459 (0,32); 0,0079 (3,23); -0,0002 (69,14); -0,008 (3,38) |
| | | | | | | Verbindung Nr. 2-81, Solvent: [DMSO], Spektrometer: 399.95MHz |
| 2-81 | 4-chlor-phenyl | CH2 | CH2 | - | CH2CH3 | 7,7744 (5,47); 7,7613 (5,66); 7,3331 (4,15); 7,0949 (4,31); 7,0821 (4,33); 3,6253 (1,16); 3,4958 (1,18); 3,4703 (1,16); 3,461 (1,16); 3,3349 (23,98); 3,1896 (1,01); 2,8679 (1,6); 2,6723 (0,39); 2,5076 (42,89); 2,5032 (56,18); 2,4988 (43,26); 2,3301 (0,37); 1,99 (0,39); 1,3969 (16); 1,1925 (0,58); 1,1747 (1,07); 1,1563 (1,39); 1,1382 (1,45); 1,0351 (1,92); 0,0076 (1,23); -0,0002 (27,9); -0,0081 (1,7) |
| | | | | | | Verbindung Nr. 2-82, Solvent: [DMSO], Spektrometer: 399.95MHz |
| 2-82 | 2,4-dichlor-phenyl | CH2 | CH2 | - | CH3 | 7,7789 (1,34); 7,768 (1,36); 7,5866 (0,34); 7,4841 (0,42); 7,3806 (1,21); 7,3627 (1,15); 7,3 (0,4); 7,0753 (1,27); 3,7028 (0,63); 3,552 (0,68); 3,3362 (10,02); 3,0647 (1,36); 3,0082 (1,02); 2,908 (1,77); 2,5125 (10,77); 2,5084 (20,65); 2,5039 (26,86); 2,4995 (20,26); 2,4955 (10,59); 1,9906 (1,01); 1,3968 (16); 1,1755 (0,52); 0,0079 (0,69); -0,0002 (15,22); -0,0082 (0,77) |
| | | | | | | Verbindung Nr. 2-83, Solvent: [DMSO], Spektrometer: 399.95MHz |
| 2-83 | 4- fluor-phenyl | CH2 | CH2 | CH(CH3) | H | 7,9847 (2,32); 7,964 (2,34); 7,8206 (7,99); 7,8075 (8,3); 7,2518 (3,78); 7,2468 (1,98); 7,2375 (4,78); 7,2304 (5,75); 7,2216 (2,28); 7,2162 (4,99); 7,146 (8,38); 7,1329 (8,13); 7,1185 (0,76); 7,111 (5,83); 7,1059 (1,95); 7,0944 (2,19); 7,0887 (9,22); 7,083 (2,33); 7,0715 (1,62); 7,0665 (4,36); 7,059 (0,52); 3,967 (0,57); 3,9466 (1,26); 3,9312 (1,62); 3,9159 (1,29); 3,8953 (0,59); 3,3367 (86,22); 2,6906 (0,41); 2,6753 (0,71); 2,6674 (0,66); 2,6556 (1,69); 2,6407 (1,87); 2,6329 (3,28); 2,6164 (3,4); 2,5935 (1,7); 2,5808 (0,58); 2,5764 (0,66); 2,5589 (0,64); 2,5432 (28,9); 2,5262 (0,6); 2,5125 (10,86); 2,5083 (21,49); 2,5038 (28,59); 2,4993 (21,7); 2,4951 (11,17); 2,0762 (0,68); 1,8757 (0,44); 1,8603 (0,54); 1,8534 (0,84); 1,841 (1,21); 1,827 (1,09); 1,8194 (1,73); 1,805 (1,45); 1,7979 (1,09); 1,7825 (0,88); 1,777 (0,95); 1,7601 (1,33); 1,7529 (1,04); 1,7459 (1,36); 1,7394 (1,35); 1,7225 (1,17); 1,7123 (0,59); 1,7057 (0,6); 1,7029 (0,57); 1,6888 (0,39); 1,1742 (16); 1,1577 (15,87); 0,0079 (0,37); -0,0002 (10,23); -0,0083 (0,43) |
| | | | | | | Verbindung Nr. 2-84, Solvent: [DMSO], Spektrometer: 399.95MHz |
| 2-84 | 4-chlor-phenyl | CF2 | CH2 | - | H | 8,4971 (0,48); 8,4816 (0,94); 8,466 (0,47); 7,8552 (2,26); 7,842 (2,34); 7,5789 (16); 7,1512 (2,37); 7,138 (2,3); 6,5757 (0,41); 4,0669 (0,61); 4,0511 (0,63); 4,0313 (1,35); 4,0155 (1,31); 3,9954 (0,69); 3,9796 (0,65); 3,3486 (47,96); 2,9452 (2,52); 2,5086 (10,05); 2,5044 (13,29); 2,5001 (10,38) |
| | | | | | | Verbindung Nr. 2-85, Solvent: [DMSO], Spektrometer: 601.6MHz |
| 2-85 | 2-thienyl | CH2 | CH2 | - | CH3 | 19,9671 (0,42); 8,3173 (2,02); 7,7769 (7,1); 7,3518 (10,48); 7,0829 (14,02); 6,9538 (14,91); 6,9485 (14,76); 6,7584 (4,17); 5,4222 (0,41); 4,6286 (0,4); 4,6186 (0,42); 4,2709 (0,36); 4,0466 (0,39); 4,0346 (1,08); 4,0227 (1,07); 4,0108 (0,34); 3,8995 (0,35); 3,6964 (8,82); 3,5095 (7,68); 3,3745 (0,46); 3,3655 (0,42); 3,327 (604,31); 3,2896 (0,8); 3,2808 (0,43); 3,2182 (0,4); 3,1085 (12,03); 3,0584 (12,67); 3,0313 (15,74); 2,8975 (16); 2,7797 (0,49); 2,6166 (4,14); 2,6136 (5,62); 2,6106 (4,1); 2,5414 (1,05); 2,5229 (11,06); 2,5198 (14); 2,5167 (14,69); 2,5079 (270,99); 2,5049 (588,91); 2,5019 (812,04); 2,4988 (583,52); 2,4958 (265,01); 2,389 (3,73); 2,386 (5,26); 2,383 (3,82); 2,3799 (1,78); 1,989 (4,36); 1,3975 (3,17); 1,3358 (1,82); 1,2978 (0,58); 1,2584 (0,62); 1,2492 (2,58); 1,1866 (1,59); 1,1748 (2,59); 1,1629 (1,26); 0,948 (1,65); 0,937 (2,01); 0,8385 (0,77); 0,8266 (1,55); 0,814 (0,87); -0,0002 (6,18) |
| | | | | | | Verbindung Nr. 2-86, Solvent: [DMSO], Spektrometer: 399.95MHz |
| 2-86 | 4-chlor-phenyl | CH(OCH3) | CH3 | - | H | 8,0412 (0,54); 8,0273 (1,04); 8,0134 (0,55); 7,8389 (2,94); 7,8257 (3,07); 7,4555 (2,97); 7,4512 (1,16); 7,4344 (5,07); 7,373 (4,53); 7,3519 (2,79); 7,1478 (3,09); 7,1346 (2,99); 4,4505 (0,88); 4,4347 (1,8); 4,4193 (0,93); 3,4926 (1,9); 3,4779 (3,23); 3,4625 (1,65); 3,3232 (6,6); 3,1925 (16); 2,5248 (0,43); 2,5112 (7,1); 2,507 (13,86); 2,5025 (18,31); 2,4981 (13,72); 2,4939 (6,96); -0,0002 (1,07) |
| | | | | | | Verbindung Nr. 2-87, Solvent: [DMSO], Spektrometer: 399.95MHz |
| 2-87 | 5-chlor-2-thienyl | CH2 | CH2 | - | H | 8,3161 (0,49); 8,2262 (2,13); 8,2126 (3,88); 8,1986 (2,13); 7,835 (15,07); 7,8219 (15,59); 7,8018 (0,43); 7,7888 (0,41); 7,3813 (14,21); 7,3775 (14,34); 7,1746 (0,48); 7,1615 (0,55); 7,1443 (16); 7,1312 (15,45); 6,9241 (11,01); 6,9211 (10,85); 4,056 (0,42); 4,0383 (1,28); 4,0204 (1,29); 4,0027 (0,43); 3,5237 (4,24); 3,5064 (10,83); 3,4917 (11,18); 3,4746 (4,85); 3,3243 (37,3); 3,0576 (0,44); 3,0424 (8,08); 3,0251 (15,23); 3,0079 (6,89); 2,6757 (0,33); 2,6713 (0,45); 2,6668 (0,32); 2,5244 (1,46); 2,5112 (25,96); 2,5068 (51,54); 2,5022 (67,31); 2,4977 (48,22); 2,4932 (23,01); 2,329 (0,43); 1,9892 (5,54); 1,3363 (0,61); 1,2494 (0,75); 1,1929 (1,52); 1,1751 (2,95); 1,1573 (1,45); 0,0079 (2,28); -0,0002 (62,76); -0,0086 (2,09) |
| | | | | | | Verbindung Nr. 2-88, Solvent: [DMSO], Spektrometer: 399.95MHz |
| 2-88 | 4-chlor-phenyl | CH(CF3) | CH2 | - | H | 8,1852 (1,39); 8,171 (2,43); 8,1568 (1,34); 7,8144 (7,54); 7,8013 (7,77); 7,4825 (4,15); 7,4769 (2); 7,4663 (3,34); 7,4608 (16); 7,4444 (11,93); 7,4227 (3,38); 7,4026 (0,33); 7,3938 (0,58); 7,1028 (8); 7,0897 (7,68); 4,1295 (0,78); 4,1145 (1,03); 4,1062 (1,33); 4,0908 (1,46); 4,0825 (1,08); 4,0672 (1,02); 4,0562 (1,19); 4,0382 (2,87); 4,0204 (2,82); 4,0027 (0,95); 3,9342 (0,98); 3,9203 (1,62); 3,9058 (1,18); 3,9002 (1,8); 3,8862 (2,62); 3,8721 (1,41); 3,8153 (1,55); 3,7991 (1,72); 3,7928 (1,52); 3,781 (1,34); 3,7768 (1,59); 3,7653 (1,05); 3,7588 (0,95); 3,7427 (0,82); 3,3234 (33,43); 2,6755 (0,35); 2,6711 (0,48); 2,6666 (0,34); 2,5108 (29,52); 2,5066 (56,22); 2,5021 (72,01); 2,4976 (51,93); 2,4933 (25,34); 2,3334 (0,35); 2,3288 (0,47); 2,3244 (0,35); 1,9892 (11,86); 1,3362 (0,44); 1,2493 (0,48); 1,1925 (3,18); 1,1747 (6,21); 1,1569 (3,05); 0,0078 (0,56); -0,0002 (12,89); -0,0083 (0,46) |
| | | | | | | Verbindung Nr. 2-89, Solvent: [DMSO], Spektrometer: 601.6MHz |
| 2-89 | 4-chlor-2-thienyl | CH2 | CH2 | - | H | 8,2192 (1,85); 8,2101 (3,42); 8,2009 (1,88); 7,8322 (15,73); 7,8234 (16); 7,799 (0,4); 7,7904 (0,42); 7,3809 (14,42); 7,3783 (14,95); 7,1724 (0,39); 7,1638 (0,41); 7,1423 (15,96); 7,1336 (15,48); 6,9235 (9,52); 6,9213 (10,04); 3,5135 (4,02); 3,502 (10,07); 3,4923 (10,33); 3,4809 (4,58); 3,359 (0,44); 3,3471 (1,05); 3,3244 (1184,4); 3,0355 (6,84); 3,0239 (13,54); 3,0125 (6,23); 2,6529 (2,02); 2,619 (0,86); 2,616 (1,93); 2,613 (2,75); 2,6099 (2,01); 2,6069 (0,94); 2,5568 (0,46); 2,5406 (602,61); 2,5223 (5,01); 2,5192 (6,23); 2,5161 (5,92); 2,5073 (142,84); 2,5043 (310,99); 2,5012 (430,85); 2,4982 (317,8); 2,4952 (149,21); 2,4243 (2,08); 2,3914 (0,82); 2,3884 (1,89); 2,3854 (2,66); 2,3823 (1,93); 2,3794 (0,88); 2,0735 (1,8); 0,0052 (0,79); -0,0002 (27,37); -0,0058 (0,82) |
| | | | | | | Verbindung Nr. 2-90, Solvent: [DMSO], Spektrometer: 399.95MHz |
| 2-90 | 4-brom-phenyl | CH2 | CH2 | - | H | 8,1254 (1,64); 8,1114 (2,96); 8,098 (1,58); 7,8191 (11,94); 7,8059 (12,35); 7,7851 (0,39); 7,7721 (0,4); 7,5 (1,64); 7,4936 (13,56); 7,4891 (4,57); 7,4774 (4,83); 7,4728 (16); 7,4665 (1,88); 7,232 (2,14); 7,2256 (13,05); 7,2047 (11,17); 7,1611 (0,44); 7,148 (0,44); 7,1311 (12,58); 7,118 (12,11); 4,0378 (0,35); 4,02 (0,35); 3,4987 (3,04); 3,4814 (6,52); 3,4664 (6,37); 3,4484 (3,36); 3,3228 (89,09); 2,832 (5,84); 2,8139 (10,28); 2,7961 (5,26); 2,6753 (0,74); 2,6707 (1,02); 2,6663 (0,74); 2,662 (0,36); 2,524 (3,23); 2,5106 (57,68); 2,5062 (114,89); 2,5017 (150,1); 2,4971 (107,93); 2,4926 (51,79); 2,333 (0,68); 2,3284 (0,95); 2,3238 (0,69); 2,3193 (0,33); 1,9888 (1,56); 1,3355 (0,36); 1,2492 (0,46); 1,1925 (0,42); 1,1747 (0,81); 1,1569 (0,4); 0,0079 (2,31); -0,0002 (63,05); -0,0085 (2,15) |
| | | | | | | Verbindung Nr. 2-91, Solvent: [DMSO], Spektrometer: 399.95MHz |
| 2-91 | 4-chlor-phenyl | CH2 | C(CH3)2 | - | H | 7,7942 (2); 7,7937 (1,96); 7,781 (2,13); 7,479 (0,83); 7,3998 (1,54); 7,3369 (2,48); 7,3161 (3,41); 7,1964 (3,15); 7,1756 (2,48); 7,116 (2,01); 7,103 (2); 5,3272 (0,4); 3,3499 (30,78); 3,3455 (30,99); 3,3393 (29,64); 3,3302 (12,71); 3,0776 (5,05); 2,5065 (13,57); 2,5026 (18); 2,4987 (15,17); 1,3346 (0,5); 1,3178 (16); -0,0002 (3,62); -0,0012 (3,61) |
| | | | | | | Verbindung Nr. 2-92, Solvent: [DMSO], Spektrometer: 399.95MHz |
| 2-92 | 2,4-dichlor-phenyl | CH2 | C(CH3)2 | - | H | 7,8127 (2,03); 7,8119 (1,97); 7,7996 (2,18); 7,7987 (2,09); 7,5885 (1,94); 7,5833 (2,15); 7,5469 (1,81); 7,366 (0,84); 7,361 (0,84); 7,3452 (1,45); 7,3402 (1,51); 7,2883 (2,5); 7,2674 (1,51); 7,1329 (2,23); 7,1198 (2,2); 4,0387 (0,54); 4,0209 (0,55); 3,3322 (20,83); 3,269 (5,62); 2,5073 (7,09); 2,5032 (9,69); 2,499 (7,85); 1,9896 (2,27); 1,35 (16); 1,1938 (0,62); 1,1929 (0,61); 1,176 (1,2); 1,1751 (1,15); 1,1582 (0,6); -0,0002 (2,55); -0,0013 (2,37) |
| | | | | | | Verbindung Nr. 2-93, Solvent: [DMSO], Spektrometer: 399.95MHz |
| 2-93 | 2-(trifluormethyl)-4-chlorphenyl | CH2 | CH2 | - | H | 8,2642 (1,96); 8,2498 (3,72); 8,2357 (1,92); 7,8279 (15,21); 7,8147 (15,83); 7,7488 (6,83); 7,7434 (10,04); 7,7323 (4,84); 7,7267 (2,66); 7,7115 (5,13); 7,7061 (3,97); 7,5482 (6,98); 7,5275 (5,7); 7,1385 (16); 7,1254 (15,5); 3,5401 (2,97); 3,5233 (6,91); 3,5071 (6,93); 3,4898 (3,3); 3,3246 (34,18); 3,3123 (0,54); 3,304 (4,59); 3,2957 (0,38); 3,2862 (3,62); 3,0151 (4,29); 2,9976 (7,73); 2,98 (3,73); 2,6964 (14,95); 2,6769 (0,42); 2,6724 (0,55); 2,6678 (0,39); 2,5257 (1,76); 2,521 (2,72); 2,5124 (30,57); 2,5079 (60,58); 2,5033 (79,28); 2,4987 (56,52); 2,4941 (26,25); 2,3346 (0,37); 2,3301 (0,52); 2,3253 (0,35); 2,1981 (1,63); 2,1781 (3,1); 2,1576 (2,19); 1,9396 (0,85); 1,9214 (2,12); 1,9182 (1,5); 1,9126 (0,39); 1,9029 (2,26); 1,8923 (0,35); 1,883 (1,91); 1,8798 (0,87); 1,864 (0,57); 1,3365 (2,02); 1,2587 (0,39); 1,2496 (2,47); 1,2334 (0,42); 1,1881 (0,78); 0,008 (0,87); -0,0002 (25,1); - 0,0085 (0,69) |
| | | | | | | Verbindung Nr. 2-94, Solvent: [DMSO], Spektrometer: 399.95MHz |
| 2-94 | 2,4-dichlor-phenyl | O | CH2 | CH(CH3) | H | 8,0435 (2,25); 8,0237 (2,28); 7,8444 (8,97); 7,8313 (9,27); 7,5738 (7,85); 7,5674 (8,35); 7,386 (3,73); 7,3795 (3,44); 7,3638 (5); 7,3574 (4,75); 7,2389 (7,4); 7,2166 (5,53); 7,1533 (9,63); 7,1401 (9,23); 4,3985 (0,56); 4,3832 (1,22); 4,3662 (1,53); 4,3476 (1,31); 4,332 (0,66); 4,1699 (1,65); 4,1548 (1,49); 4,1455 (3,5); 4,1304 (3,06); 4,1114 (3,5); 4,0974 (3,32); 4,087 (1,78); 4,073 (1,45); 3,3802 (0,32); 3,3735 (0,47); 3,3343 (380,72); 3,2974 (0,41); 2,6762 (0,59); 2,6716 (0,8); 2,6672 (0,57); 2,5419 (14,79); 2,525 (2,2); 2,5202 (3,54); 2,5117 (46,3); 2,5072 (93,4); 2,5026 (123); 2,498 (88,09); 2,4934 (41,35); 2,3339 (0,57); 2,3293 (0,81); 2,3247 (0,58); 1,2919 (16); 1,2749 (15,85); 0,008 (0,75); -0,0002 (23,38); -0,0086 (0,66) |
| | | | | | | Verbindung Nr. 2-96, Solvent: [DMSO], Spektrometer: 399.95MHz |
| 2-95 | 4-chlor-phenyl | O | CH2 | CH(CH3) | H | 8,1037 (2,62); 8,0836 (2,64); 7,8406 (8,35); 7,8274 (8,63); 7,6262 (7,86); 7,6205 (8,15); 7,5734 (5,55); 7,5519 (7,26); 7,4258 (4,73); 7,4201 (4,43); 7,4043 (3,64); 7,3986 (3,44); 7,1488 (8,79); 7,1356 (8,49); 4,1854 (0,67); 4,1681 (1,49); 4,1503 (1,96); 4,1328 (1,53); 4,1153 (0,7); 3,3486 (0,62); 3,3298 (110,3); 3,3052 (2,03); 3,2872 (1,82); 3,272 (3,23); 3,2538 (3,07); 3,1986 (3,29); 3,183 (3,35); 3,1653 (2); 3,1496 (1,83); 2,672 (0,36); 2,5422 (20,2); 2,5253 (1,27); 2,5119 (21,46); 2,5075 (42); 2,5029 (54,69); 2,4983 (39,66); 2,4939 (19,17); 2,3296 (0,34); 1,3058 (16); 1,2892 (15,83); -0,0002 (6,66) |
| | | | | | | Verbindung Nr. 2-96, Solvent: [DMSO], Spektrometer: 399.95MHz |
| 2-96 | 2,4-dichlor-phenyl | S | CH2 | CH(CH3) | H | 8,1037 (2,62); 8,0836 (2,64); 7,8406 (8,35); 7,8274 (8,63); 7,6262 (7,86); 7,6205 (8,15); 7,5734 (5,55); 7,5519 (7,26); 7,4258 (4,73); 7,4201 (4,43); 7,4043 (3,64); 7,3986 (3,44); 7,1488 (8,79); 7,1356 (8,49); 4,1854 (0,67); 4,1681 (1,49); 4,1503 (1,96); 4,1328 (1,53); 4,1153 (0,7); 3,3486 (0,62); 3,3298 (110,3); 3,3052 (2,03); 3,2872 (1,82); 3,272 (3,23); 3,2538 (3,07); 3,1986 (3,29); 3,183 (3,35); 3,1653 (2); 3,1496 (1,83); 2,672 (0,36); 2,5422 (20,2); 2,5253 (1,27); 2,5119 (21,46); 2,5075 (42); 2,5029 (54,69); 2,4983 (39,66); 2,4939 (19,17); 2,3296 (0,34); 1,3058 (16); 1,2892 (15,83); -0,0002 (6,66) |
| | | | | | | Verbindung Nr. 2-97, Solvent: [DMSO], Spektrometer: 399.95MHz |
| 2-97 | 4-chlor-phenyl | S | CH2 | CH(CH3) | H | 8,0313 (2,28); 8,0111 (2,27); 7,8351 (7,34); 7,8276 (0,77); 7,822 (7,61); 7,4468 (0,36); 7,4416 (0,39); 7,4184 (5,41); 7,4131 (2,2); 7,4022 (3,25); 7,3966 (13,89); 7,3911 (2,42); 7,3736 (2,38); 7,3681 (14,46); 7,3625 (3,43); 7,3516 (2,29); 7,3463 (5,62); 7,3404 (0,88); 7,3333 (0,67); 7,3158 (0,37); 7,1509 (0,57); 7,1443 (7,83); 7,1379 (0,74); 7,1311 (7,56); 4,1367 (0,71); 4,1196 (1,54); 4,1015 (1,92); 4,0835 (1,56); 4,0664 (0,72); 3,3511 (0,6); 3,3303 (126,34); 3,2924 (0,34); 3,2547 (2,01); 3,2369 (1,94); 3,2209 (3,27); 3,2031 (3,13); 3,1345 (3,43); 3,1187 (3,44); 3,1008 (2,14); 3,0849 (1,99); 2,6713 (0,39); 2,5417 (12,91); 2,5244 (1,23); 2,5112 (23,65); 2,5069 (46,72); 2,5024 (61,31); 2,4978 (44,84); 2,4935 (22,01); 2,3291 (0,39); 1,3123 (0,69); 1,2957 (0,74); 1,2793 (1,26); 1,2672 (16); 1,2505 (15,69); -0,0002 (6,61) |
| | | | | | | Verbindung Nr. 2-98, Solvent: [DMSO], Spektrometer: 399.95MHz |
| 2-98 | 4-chlor-phenyl | N(CH3) | CH2 | CH2 | H | 8,1784 (0,63); 8,1644 (1,19); 8,1511 (0,64); 7,832 (3,85); 7,8188 (3,97); 7,1823 (0,46); 7,1735 (4,39); 7,1681 (1,47); 7,1561 (1,75); 7,1508 (4,9); 7,1399 (4,35); 7,1267 (3,97); 6,78 (0,54); 6,7713 (4,48); 6,766 (1,59); 6,7536 (1,52); 6,7485 (4,03); 6,7396 (0,46); 3,4961 (0,89); 3,4802 (2,47); 3,4646 (2,25); 3,4296 (1,28); 3,4143 (2,21); 3,3992 (1,71); 3,3837 (0,55); 3,3285 (37,94); 2,9175 (16); 2,5417 (14,97); 2,5245 (0,68); 2,5111 (10,02); 2,5068 (18,95); 2,5023 (24,27); 2,4977 (17,83); 2,4934 (8,9); -0,0002 (3,85) |
| | 3-chlor-4-(trifluormethyl)-phenyl | | | | | Verbindung Nr. 2-99, Solvent: [DMSO], Spektrometer: 399.95MHz |
| 2-99 | | CH2 | CH2 | - | H | 7,8169 (0,34); 7,8037 (0,35); 7,1258 (0,35); 7,1126 (0,34); 5,7548 (16); 3,3218 (3,21); 2,5111 (2,65); 2,5067 (5,19); 2,5021 (6,78); 2,4975 (4,91); 2,4931 (2,38) |
| 2-100 | 4-chlor-3-(trifluormethyl)-phenyl | CH2 | CH2 | - | H | Verbindung Nr. 2-100, Solvent: [DMSO], Spektrometer: 399.95MHz 8,1546 (1,81); 8,1411 (3,36); 8,1274 (1,79); 7,8227 (15,02); 7,8095 (15,47); 7,7309 (7,67); 7,7263 (7,96); 7,6656 (5,24); 7,645 (9,1); 7,5935 (5,17); 7,589 (4,84); 7,5729 (2,99); 7,5683 (2,88); 7,1311 (16); 7,1179 (15,44); 3,5543 (3,34); 3,5374 (8,85); 3,5227 (9,01); 3,506 (3,66); 3,3342 (43,7); 2,9569 (6,23); 2,94 (12,45); 2,923 (5,53); 2,679 (0,4); 2,5323 (1,33); 2,5275 (2,04); 2,519 (22,77); 2,5144 (45,98); 2,5099 (60,71); 2,5052 (43,75); 2,5007 (20,76); 2,3367 (0,4); 1,343 (0,48); 1,2558 (0,62) |
| 2-101 | 2-chlor-4-(trifluormethyl)-phenyl | O | CH2 | C(CH3)2 | H | Verbindung Nr. 2-101, Solvent: [DMSO], Spektrometer: 399.95MHz 7,8286 (1,28); 7,8232 (1,39); 7,818 (2,64); 7,8048 (2,63); 7,7272 (1,41); 7,6873 (0,62); 7,6829 (0,58); 7,6655 (0,71); 7,6612 (0,65); 7,3709 (1,12); 7,3493 (1); 7,1335 (2,69); 7,1203 (2,57); 4,3465 (4,75); 3,3445 (101,81); 2,5432 (4,46); 2,5263 (0,44); 2,5129 (9,11); 2,5085 (17,92); 2,5039 (23,2); 2,4993 (16,52); 2,4948 (7,76); 1,4956 (16) |
| 2-102 | 4-chlor-2-(trifluormethyl)-phenyl | O | CH3 | C(CH3)3 | H | Verbindung Nr. 2-102, Solvent: [DMSO], Spektrometer: 399.95MHz 7,8202 (2,3); 7,8071 (2,37); 7,7033 (0,56); 7,6966 (0,73); 7,6811 (0,56); 7,6745 (0,92); 7,659 (1,7); 7,6526 (1,1); 7,6064 (1,44); 7,3238 (1,18); 7,3016 (1,05); 7,1421 (2,41); 7,1289 (2,32); 4,3057 (4,74); 3,3418 (117,7); 2,5428 (6,37); 2,5259 (0,54); 2,5125 (11,26); 2,5081 (22,46); 2,5035 (29,35); 2,4989 (21,13); 2,4944 (10,06); 1,456 (16) |
| 2-103 | 2-chlor-4-(trifluormethyl)-phenyl | O | CH2 | CH2 | H | Verbindung Nr. 2-103, Solvent: [DMSO], Spektrometer: 399.95MHz 8,233 (2,33); 8,2195 (4,37); 8,206 (2,37); 7,8593 (13,55); 7,8461 (15,38); 7,8411 (9,06); 7,8357 (9,06); 7,7032 (4,25); 7,6988 (4,02); 7,6816 (4,96); 7,6771 (4,61); 7,5239 (0,41); 7,417 (7,79); 7,3953 (6,77); 7,1625 (14,26); 7,1493 (13,74); 4,3372 (7,53); 4,3228 (16); 4,3084 (7,71); 3,7244 (4,06); 3,7102 (11,54); 3,6959 (11,21); 3,6816 (3,73); 3,3337 (199,43); 2,6777 (0,45); 2,6733 (0,61); 2,669 (0,44); 2,5432 (2,51); 2,5263 (1,9); 2,5129 (37,99); 2,5086 (72,9); 2,5042 (93,47); 2,4998 (67,94); 2,3355 (0,44); 2,3309 (0,58); 2,3267 (0,42); 1,234 (0,34); -0,0002 (0,46) |

**Tabelle 3**

| | | | | | | |
|---|---|---|---|---|---|---|
| Verbindungen der Formel I-3 | | | | | | |
| 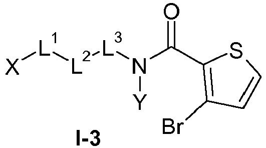 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 3-1 | 3-methyl-2-thienyl | CH2 | CH2 | - | H | WO-A 2006/108791 |
| | | | | | | Verbindung Nr. 3-2, Solvent: [DMSO], Spektrometer: 399.95MHz |
| 3-2 | 2,4-dichlor-phenyl | CH2 | CH2 | CH2 | H | 8,2727 (2,08); 8,2589 (3,84); 8,2451 (2,05); 7,7968 (15,36); 7,7838 (15,9); 7,5676 (10,89); 7,5623 (11,33); 7,4294 (6,33); 7,4087 (14,31); 7,3945 (0,46); 7,3819 (10,21); 7,3766 (9,45); 7,3613 (4,36); 7,356 (4,36); 7,176 (16); 7,163 (15,43); 7,1514 (0,4); 3,5162 (0,4); 3,3364 (45,86); 3,3131 (3,59); 3,2962 (8,61); 3,2811 (8,71); 3,2643 (3,65); 2,7678 (6,35); 2,7489 (7,94); 2,729 (6,83); 2,5457 (19,24); 2,5288 (0,62); 2,5239 (1,02); 2,5154 (10,87); 2,5109 (21,41); 2,5064 (28,22); 2,5018 (20,57); 2,4973 (9,85); 1,8383 (1,86); 1,8208 (4,99); 1,8015 (6,21); 1,7827 (4,82); 1,7651 (1,63); -0,0002 (3,74) |
| 3-3 | 4-chlor-phenyl | CH2 | CH2 | - | H | CAS: 1270975-99-9 |
| 3-4 | 2,4-dichlor-phenyl | CH2 | CH2 | - | H | WO-A 2007/060166 |
| | | | | | | Verbindung Nr. 3-5, Solvent: [DMSO], Spektrometer: 399.95MHz |
| 3-5 | 4-chlor-phenyl | CH(OCH3) | CH(CH3) | - | H | 8,0161 (0,68); 7,9946 (0,69); 7,856 (0,53); 7,8357 (0,53); 7,8077 (1,18); 7,7946 (1,22); 7,7765 (1,55); 7,7635 (1,6); 7,437 (2,83); 7,416 (4,81); 7,3603 (2,38); 7,3542 (3,14); 7,3389 (1,66); 7,333 (1,84); 7,1868 (1,23); 7,1737 (1,18); 7,1492 (1,66); 7,1362 (1,6); 6,5742 (0,41); 4,3623 (0,79); 4,3497 (0,91); 4,305 (1,07); 4,2895 (1,24); 4,1795 (0,33); 4,1314 (0,36); 4,1149 (0,51); 4,0938 (0,49); 3,3227 (9,24); 3,2125 (9,84); 3,2106 (9,39); 2,5065 (21,67); 2,5023 (28,08); 2,4982 (21,85); 1,9892 (0,43); 1,3974(16); 1,131 (3,58); 1,1142 (3,57); 1,0897 (2,69); 1,0728 (2,64); -0,0002 (4,39) |
| 3-6 | 2,4-dichlor-phenyl | CH(OCH3) | CH(CH3) | - | H | |
| 3-7 | 4-chlor-phenyl | CH(CH3) | CH2 | - | H | |
| 3-8 | 2,4-dichlor-phenyl | CH(CH3) | CH2 | - | H | [DMSO], Spektrometer: 399.95MHz 8.2200 (0.51); 8.2055 (1.01); 8.1919 (0.52); 7.7723 (3.38); 7.7593 (3.55); 7.5710 (2.72); 7.5657 (2.98); 7.4893 (1.62); 7.4681 (3.24); 7.4306 (1.96); 7.4253 (1.88); 7.4095 (0.99); 7.4042 (0.99); 7.1453 (3.60); 7.1323 (3.48); 3.9087 (16.00); 3.5751 (0.47); 3.5581 (0.87); 3.5409 (1.02); 3.5251 (0.96); 3.5145 (0.73); 3.4966 (1.18); 3.4815 (1.62); 3.4726 (1.13); 3.4657 (1.16); 3.4579 (1.46); 3.4394 (1.70); 3.3784 (388.06); 3.2888 (0.55); 3.2500 (0.40); 3.1729 (0.36); 2.6830 (0.37); 2.6785 (0.49); 2.6741 (0.37); 2.5485 (0.37); 2.5316 (1.75); 2.5181 (28.84); 2.5139 (56.51); 2.5094 (74.12); 2.5049 (55.80); 2.5006 (28.43); 2.3406 (0.34); 2.3361 (0.47); 2.3316 (0.34); 1.2349 (5.47); 1.2182 (5.38) |
| 3-9 | 4-chlor-phenyl | CH2 | CH(CH3) | - | H | WO-A 2007060164 |
| 3-10 | 2,4-dichlor-phenyl | CH2 | CH(CH3) | - | H | |
| 3-11 | 4-chlor-phenyl | C(CH3)2 | CH2 | - | H | [DMSO], Spektrometer: 399.95MHz 7.8445 (0.41); 7.8294 (0.74); 7.8145 (0.39); 7.7912 (2.73); 7.7781 (2.84); 7.4685 (2.32); 7.4635 (0.88); 7.4519 (1.07); 7.4467 (3.97); 7.4402 (0.57); 7.3902 (0.53); 7.3836 (4.13); 7.3784 (1.11); 7.3669 (0.85); 7.3619 (2.46); 7.1507 (2.90); 7.1376 (2.80); 3.9086 (11.97); 3.4833 (3.04); 3.4678 (3.11); 3.4543 (0.72); 3.3806 (307.12); 3.3012 (0.34); 2.6786 (0.35); 2.5317 (1.03); 2.5183 (20.55); 2.5139 (40.88); 2.5094 (54.15); 2.5049 (40.96); 2.5005 (20.99); 2.3361 (0.34); 1.3245 (16.00) |
| 3-12 | 2,4-dichlor-phenyl | C(CH3)2 | CH2 | - | H | [DMSO], Spektrometer: 399.95MHz 7.8724 (0.40); 7.8577 (0.80); 7.8423 (0.43); 7.7717 (2.77); 7.7587 (2.80); 7.5436 (2.18); 7.5378 (2.41); 7.5058 (1.54); 7.4841 (2.22); 7.3904 (1.46); 7.3845 (1.45); 7.3688 (1.09); 7.3630 (1.08); 7.1284 (2.82); 7.1154 (2.72); 3.9041 (12.41); 3.8138 (2.83); 3.7982 (2.80); 3.5113 (0.43); 3.4965 (0.43); 3.3807 (353.39); 2.6745 (0.43); 2.5141 (24.73); 2.5099 (47.13); 2.5054 (61.05); 2.5009 (45.64); 2.4967 (23.22); 2.3322 (0.37); 1.4735 (16.00) |
| 3-13 | 2-chlor-phenyl | CH2 | CH2 | - | H | [DMSO], Spektrometer: 399.95MHz 8.2809 (0.61); 8.2679 (1.16); 8.2543 (0.64); 7.7889 (4.14); 7.7759 (4.33); 7.4471 (1.48); 7.4430 (1.28); 7.4288 (1.96); 7.4241 (1.98); 7.3873 (1.14); 7.3820 (1.39); 7.3690 (1.55); 7.3641 (2.01); 7.3171 (0.59); 7.3129 (0.83); 7.2987 (1.99); 7.2945 (1.98); 7.2818 (2.41); 7.2772 (2.61); 7.2648 (1.72); 7.2597 (1.56); 7.2463 (0.58); 7.2414 (0.48); 7.1690 (4.39); 7.1560 (4.26); 3.9081 (16.00); 3.5315 (1.41); 3.5145 (3.10); 3.4991 (2.92); 3.4813 (1.92); 3.3866 (495.05); 3.2836 (0.44); 2.9923 (2.25); 2.9743 (3.88); 2.9566 (2.02); 2.6830 (0.39); 2.6785 (0.53); 2.6742 (0.40); 2.5485 (0.40); 2.5317 (1.79); 2.5183 (30.82); 2.5140 (60.87); 2.5095 (80.28); 2.5050 (60.66); 2.5007 (30.97); 2.3406 (0.38); 2.3363 (0.52); 2.3319 (0.39) |
| 3-14 | 3,4-dichlor-phenyl | CH2 | CH2 | - | H | [DMSO], Spektrometer: 399.95MHz 8.2310 (0.72); 8.2172 (1.39); 8.2034 (0.78); 7.7885 (4.47); 7.7755 (4.68); 7.5654 (3.69); 7.5445 (6.89); 7.5388 (3.87); 7.3454 (0.34); 7.3407 (0.34); 7.2753 (1.92); 7.2703 (1.94); 7.2547 (1.70); 7.2498 (1.73); 7.1647 (4.90); 7.1517 (4.79); 3.9071 (16.00); 3.5150 (1.83); 3.4981 (4.09); 3.4832 (4.21); 3.4662 (2.98); 3.3988 (569.18); 3.2438 (0.36); 3.2267 (0.36); 3.1783 (0.44); 3.1665 (0.43); 2.8667 (2.27); 2.8496 (4.53); 2.8324 (2.10); 2.6833 (0.42); 2.6787 (0.57); 2.6741 (0.44); 2.5183 (32.31); 2.5140 (63.85); 2.5095 (84.66); 2.5050 (64.88); 2.5008 (34.15); 2.3406 (0.41); 2.3363 (0.55); 2.3317 (0.41) |
| 3-15 | 3,5-dichlor-phenyl | CH2 | CH2 | - | H | CAS: 1318048-71-3 |
| | | | | | | |
| 3-16 | 3-chlor-phenyl | CH2 | CH2 | - | H | [DMSO], Spektrometer: 399.95MHz 8.2233 (0.45); 8.2093 (0.83); 8.1955 (0.46); 7.7940 (3.28); 7.7810 (3.41); 7.3563 (0.82); 7.3478 (1.31); 7.3433 (2.17); 7.3374 (2.94); 7.3181 (2.00); 7.2909 (1.03); 7.2877 (1.53); 7.2828 (1.18); 7.2709 (0.63); 7.2661 (0.71); 7.2628 (0.50); 7.2409 (1.49); 7.2224 (1.05); 7.1686 (3.58); 7.1556 (3.46); 3.9090 (16.00); 3.5146 (1.04); 3.4973 (2.23); 3.4827 (2.30); 3.4652 (1.28); 3.3763 (317.99); 3.3214 (0.62); 3.2964 (0.38); 2.8720 (1.54); 2.8543 (3.00); 2.8367 (1.46); 2.6786 (0.42); 2.5319 (1.43); 2.5184 (24.35); 2.5140 (48.71); 2.5094 (64.51); 2.5049 (48.63); 2.5005 (24.82); 2.3361 (0.40) |
| 3-17 | 2-fluor-phenyl | CH2 | CH2 | - | H | CAS: 1275755-97-9 |
| 3-18 | 2,6-difluor-phenyl | CH2 | CH2 | - | H | [DMSO], Spektrometer: 399.95MHz 8.3218 (0.53); 8.3074 (1.03); 8.2937 (0.57); 7.7811 (3.58); 7.7680 (3.75); 7.3461 (0.67); 7.3421 (0.65); 7.3252 (1.34); 7.3080 (0.69); 7.3044 (0.88); 7.2876 (0.40); 7.1586 (3.84); 7.1456 (3.69); 7.0840 (1.88); 7.0642 (3.00); 7.0532 (0.43); 7.0443 (1.63); 3.9043 (16.00); 3.4972 (0.35); 3.4725 (1.15); 3.4555 (2.68); 3.4396 (2.90); 3.4228 (1.83); 3.3665 (342.01); 3.1746 (0.55); 3.1618 (0.54); 2.9117 (1.44); 2.8943 (2.75); 2.8770 (1.31); 2.6779 (0.35); 2.6734 (0.48); 2.6689 (0.37); 2.5265 (1.71); 2.5131 (27.34); 2.5088 (53.82); 2.5043 (70.92); 2.4998 (53.48); 2.4955 (27.28); 2.3310 (0.44); -0.0002 (1.23) |
| 3-19 | 2,6-dichlor-phenyl | CH2 | CH2 | - | H | [DMSO], Spektrometer: 399.95MHz 8.3537 (0.50); 8.3393 (1.04); 8.3250 (0.58); 7.7812 (3.52); 7.7681 (3.77); 7.4649 (4.46); 7.4449 (6.20); 7.3049 (2.08); 7.2856 (2.05); 7.2839 (2.10); 7.2646 (1.38); 7.1626 (3.73); 7.1496 (3.67); 3.9043 (16.00); 3.5128 (0.94); 3.4962 (2.13); 3.4794 (2.24); 3.4629 (1.20); 3.3686 (385.44); 3.2638 (0.35); 3.2399 (0.34); 3.1807 (1.93); 3.1627 (3.54); 3.1455 (1.57); 2.6782 (0.40); 2.6736 (0.53); 2.6692 (0.41); 2.5268 (1.86); 2.5134 (29.37); 2.5090 (57.84); 2.5045 (76.07); 2.5000 (57.08); 2.4956 (28.84); 2.3357 (0.33); 2.3312 (0.45); 2.3268 (0.33) |
| 3-20 | 3-(trifluormethyl)-phenyl | CH2 | CH2 | - | H | [DMSO], Spektrometer: 399.95MHz 8.2347 (0.52); 8.2214 (0.99); 8.2078 (0.57); 7.7906 (3.47); 7.7776 (3.67); 7.6157 (2.15); 7.5973 (0.78); 7.5904 (0.84); 7.5753 (3.32); 7.5612 (2.39); 7.5510 (0.68); 7.5432 (0.86); 7.5246 (0.40); 7.1634 (3.68); 7.1504 (3.59); 3.9086 (16.00); 3.5513 (1.14); 3.5341 (2.61); 3.5194 (2.73); 3.5023 (1.34); 3.3800 (440.79); 3.1791 (0.40); 3.1671 (0.37); 2.9689 (1.80); 2.9515 (3.54); 2.9341 (1.65); 2.6829 (0.42); 2.6786 (0.56); 2.6741 (0.43); 2.5317 (1.97); 2.5183 (31.67); 2.5139 (61.89); 2.5094 (81.18); 2.5049 (60.98); 2.5005 (30.95); 2.3405 (0.37); 2.3364 (0.53); 2.3316 (0.36) |
| 3-21 | 4-(trifluormethyl)-phenyl | CH2 | CH2 | - | H | [DMSO], Spektrometer: 399.95MHz 8.2576 (0.53); 8.2434 (1.04); 8.2301 (0.59); 7.7888 (3.63); 7.7757 (3.85); 7.6781 (2.65); 7.6579 (3.41); 7.5038 (3.04); 7.4837 (2.56); 7.1658 (3.90); 7.1528 (3.80); 3.9076 (16.00); 3.5464 (1.21); 3.5291 (2.50); 3.5139 (2.46); 3.4967 (1.49); 3.3908 (499.03); 3.2632 (0.61); 3.1775 (0.42); 2.9594 (1.57); 2.9417 (2.93); 2.9240 (1.43); 2.6829 (0.42); 2.6785 (0.57); 2.6741 (0.42); 2.5482 (0.50); 2.5317 (2.05); 2.5182 (32.24); 2.5139 (62.79); 2.5094 (82.03); 2.5048 (61.25); 2.5005 (30.81); 2.3406 (0.37); 2.3360 (0.51); 2.3314 (0.36) |
| 3-22 | 2-methyl-phenyl | CH2 | CH2 | - | H | [DMSO], Spektrometer: 399.95MHz 8.2822 (0.51); 8.2685 (0.94); 8.2545 (0.50); 7.8035 (3.62); 7.7905 (3.76); 7.1905 (0.80); 7.1822 (4.83); 7.1692 (5.54); 7.1628 (1.58); 7.1536 (1.80); 7.1476 (0.86); 7.1360 (2.33); 7.1318 (2.71); 7.1229 (2.90); 7.1161 (1.08); 7.1107 (1.18); 3.9099 (16.00); 3.4460 (1.27); 3.4307 (2.21); 3.4229 (1.71); 3.4123 (2.38); 3.4080 (2.97); 3.3677 (281.55); 3.1741 (0.41); 2.8542 (1.84); 2.8346 (2.21); 2.8162 (1.66); 2.6788 (0.43); 2.5320 (1.46); 2.5187 (24.89); 2.5142 (49.62); 2.5097 (65.63); 2.5051 (49.58); 2.5007 (25.38); 2.3301 (13.30) |
| 3-23 | 2,4,6-trimethyl-phenyl | CH2 | CH2 | - | H | [DMSO], Spektrometer: 399.95MHz 8.3946 (0.38); 8.3800 (0.77); 8.3655 (0.42); 7.8104 (2.38); 7.7973 (2.52); 7.1900 (2.48); 7.1770 (2.43); 6.8079 (4.13); 3.9039 (11.15); 3.3682 (250.25); 3.2803 (0.75); 3.2657 (1.06); 3.2609 (0.93); 3.2521 (1.00); 3.2449 (0.83); 3.2386 (1.06); 3.2246 (0.72); 3.1748 (0.36); 3.1622 (0.34); 2.8149 (1.08); 2.8015 (0.84); 2.7939 (1.10); 2.7888 (0.91); 2.7740 (0.94); 2.6735 (0.32); 2.5266 (1.04); 2.5132 (18.68); 2.5089 (37.14); 2.5043 (49.26); 2.4998 (37.65); 2.4955 (19.67); 2.3311 (0.38); 2.3265 (0.33); 2.3030 (16.00); 2.1822 (7.09) |
| 3-24 | 3,4-bismethoxy-phenyl | CH2 | CH2 | - | H | [DMSO], Spektrometer: 399.95MHz 8.1597 (0.49); 8.1461 (0.96); 8.1328 (0.53); 7.7919 (3.25); 7.7789 (3.41); 7.1699 (3.46); 7.1569 (3.37); 6.8788 (1.99); 6.8583 (2.87); 6.8433 (2.25); 6.8386 (2.55); 6.7706 (1.43); 6.7659 (1.34); 6.7503 (1.04); 6.7456 (0.98); 3.9042 (14.99); 3.7347 (15.37); 3.7119 (16.00); 3.4798 (0.94); 3.4628 (1.75); 3.4456 (1.90); 3.4293 (1.30); 3.3655 (318.64); 3.2789 (0.42); 3.1746 (0.77); 3.1620 (0.74); 2.7839 (1.49); 2.7655 (2.48); 2.7476 (1.37); 2.6777 (0.32); 2.6733 (0.46); 2.6687 (0.34); 2.5265 (1.65); 2.5131 (26.50); 2.5087 (51.83); 2.5042 (68.11); 2.4996 (51.37); 2.4953 (26.43); 2.3309 (0.43); 2.3263 (0.32) |
| 3-25 | phenyl | CH2 | CH2 | - | H | [DMSO], Spektrometer: 399.95MHz 8.2035 (0.62); 8.1902 (1.10); 8.1767 (0.62); 7.7903 (4.18); 7.7773 (4.38); 7.3256 (1.23); 7.3072 (3.59); 7.2938 (1.16); 7.2892 (3.89); 7.2655 (4.90); 7.2487 (2.19); 7.2316 (0.95); 7.2279 (1.26); 7.2104 (1.89); 7.2046 (0.51); 7.1968 (0.43); 7.1928 (0.66); 7.1669 (4.49); 7.1539 (4.30); 3.9043 (16.00); 3.4997 (1.27); 3.4824 (2.50); 3.4674 (2.42); 3.4638 (2.55); 3.4486 (1.65); 3.4288 (0.62); 3.4125 (0.95); 3.3598 (330.60); 3.1744 (0.41); 3.1616 (0.38); 2.8544 (2.28); 2.8355 (3.54); 2.8175 (2.09); 2.6772 (0.37); 2.6730 (0.51); 2.6684 (0.37); 2.5258 (1.62); 2.5124 (29.32); 2.5082 (57.61); 2.5037 (76.03); 2.4992 (57.88); 2.4949 (30.21); 2.3348 (0.34); 2.3304 (0.47); 2.3260 (0.35); - 0.0002 (5.83) |
| 3-26 | 4-chlor-phenyl | C(CH2-CH2) | CH2 | - | H | |
| 3-27 | 2,4-dichlor-phenyl | C(CH2-CH2) | CH2 | - | H | |
| 3-28 | 4-chlor-phenyl | CH2 | C(CH2-CH2) | - | H | |
| 3-29 | 2,4-dichlor-phenyl | CH2 | C(CH2-CH2) | - | H | |
| 3-30 | 4-chlor-phenyl | O | CH2 | CH2 | H | |
| | | | | | | Verbindung Nr. 3-31, Solvent: [DMSO], Spektrometer: 399.95MHz |
| 3-31 | 2,4-dichlor-phenyl | O | CH2 | CH2 | H | 8,2822 (2,3); 8,269 (4,38); 8,2557 (2,41); 7,8242 (11,81); 7,8111 (12,3); 7,5752 (10,56); 7,5688 (11,67); 7,387 (5,34); 7,3806 (5,18); 7,3649 (7,31); 7,3585 (7,25); 7,2464 (12,19); 7,2241 (9,05); 7,1919 (12,59); 7,1789 (12,26); 4,2268 (7,16); 4,2123 (16); 4,1979 (8,1); 3,679 (4,02); 3,6647 (11,39); 3,6505 (11,04); 3,6362 (3,72); 3,3278 (103,05); 2,6761 (0,4); 2,6719 (0,55); 2,6676 (0,42); 2,5423 (12,35); 2,5249 (1,63); 2,5073 (61,31); 2,5029 (81,63); 2,4985 (62,83); 2,3338 (0,36); 2,3297 (0,5); 2,3255 (0,38); -0,0003 (2,91) |
| 3-32 | 4-chlor-phenyl | CH2 | CH2 | CH2 | H | |
| 3-33 | 4-chlor-phenyl | NCH3 | CH2 | CH2 | H | |
| 3-34 | 2,4-dichlor-phenyl | NCH3 | CH2 | CH2 | H | |
| 3-35 | 4-chlor-phenyl | CH(OCH3) | CH2 | - | H | |
| 3-36 | 2,4-dichlor-phenyl | CH(OCH3) | CH2 | - | H | |
| | | | | | | Verbindung Nr. 3-37, Solvent: [DMSO], Spektrometer: 399.95MHz |
| 3-37 | 2-thienyl | CH2 | CH2 | - | H | 8,2709 (2,47); 8,2578 (4,4); 8,2445 (2,58); 7,8021 (13,53); 7,7891 (14,28); 7,3587 (7,31); 7,3558 (7,48); 7,3461 (8,13); 7,3431 (7,99); 7,1762 (14,66); 7,1632 (14,2); 6,9754 (5,68); 6,9668 (9,25); 6,9629 (5,93); 6,9542 (9,21); 6,9359 (9,39); 6,9294 (6,21); 3,5179 (4,53); 3,5002 (10,66); 3,4854 (10,84); 3,4677 (5,39); 3,3265 (108,64); 3,0742 (8,92); 3,0562 (16); 3,0383 (7,79); 2,675 (0,54); 2,6706 (0,72); 2,6667 (0,55); 2,5409 (1,07); 2,5238 (2,27); 2,506 (81,37); 2,5015 (109,31); 2,4972 (84,86); 2,3326 (0,54); 2,3283 (0,72); 2,3242 (0,55); - 0,0002 (2,19) |
| 3-38 | 3-thienyl | CH2 | CH2 | - | H | |
| 3-39 | 2-furyl | CH2 | CH2 | - | H | |
| 3-40 | 3-furyl | CH2 | CH2 | - | H | |
| 3-41 | phenyl | CH2 | CH2 | CH(CH3) | H | |
| 3-42 | phenyl | CH2 | CH2 | CH2 | H | |
| 3-43 | 2-Cl-phenyl | CH2 | CH2 | CH(CH3) | H | |
| 3-44 | 4-t-butyl-phenyl | CH2 | CH2 | CH2 | H | |
| 3-45 | 4-methyl-phenyl | CH2 | CH2 | CH2 | H | |
| 3-46 | phenyl | CH2 | CH2 | CH(CH2CH3) | H | |
| 3-47 | 2-methoxy-phenyl | CH2 | CH2 | CH2 | H | |
| 3-48 | 2-methyl-phenyl | CH2 | CH2 | CH2 | H | |
| 3-49 | 3 -methyl-phenyl | CH2 | CH2 | CH2 | H | |
| 3-50 | 3-chlor-phenyl | CH2 | CH2 | CH2 | H | |
| | | | | | | Verbindung Nr. 3-51, Solvent: [DMSO], Spektrometer: 399.95MHz |
| 3-51 | 2,6-difluor-phenyl | CH2 | CH2 | CH2 | H | 8,2507 (3,15); 8,2373 (5,71); 8,2241 (3,3); 7,8265 (0,35); 7,8135 (0,47); 7,7965 (14,71); 7,7835 (15,45); 7,3512 (1,55); 7,3339 (3,69); 7,3306 (3,69); 7,3134 (7,02); 7,2929 (4,7); 7,2758 (2,05); 7,2022 (0,4); 7,1894 (0,43); 7,1745 (15,6); 7,1614 (15,22); 7,1442 (0,47); 7,1223 (0,48); 7,1015 (1,47); 7,0884 (10,05); 7,0688 (16); 7,0491 (8,53); 7,0355 (1,22); 6,5622 (0,48); 3,3305 (79,53); 3,3 (5,1); 3,2829 (11,73); 3,2669 (11,92); 3,2501 (5,38); 2,7104 (6,98); 2,6911 (11,79); 2,6719 (8,33); 2,543 (4,06); 2,5078 (60,23); 2,5037 (79,89); 2,4996 (63,31); 2,3306 (0,52); 1,8103 (2,64); 1,7917 (7,39); 1,7729 (10,06); 1,7542 (7,14); 1,7362 (2,4); -0,0002 (1,04) |
| 3-52 | 4-chlor-phenyl | CH2 | CH2 | CH2 | H | |
| 3-53 | 2,6-dichlor-phenyl | CH2 | CH2 | CH(CH3) | H | |
| 3-54 | 3,5-dichlor-phenyl | CH2 | CH2 | CH(CH3) | H | |
| 3-55 | 2,6-dimethyl-phenyl | CH2 | CH2 | CH2 | H | |
| 3-56 | 2,5-dichlor-phenyl | CH2 | CH2 | CH2 | H | |
| 3-57 | 4-i-propoxy-phenyl | CH2 | CH2 | CH2 | H | |
| 3-58 | 3-trifluormethyl-phenyl | CH2 | CH2 | CH(CH3) | H | |
| 3-59 | 4-methyl-phenyl | CH2 | CH2 | CH(CH3) | H | |
| 3-60 | 2-methyl-phenyl | CH2 | CH2 | CH(CH3) | H | |
| 3-61 | 3,4-dichlor-phenyl | CH2 | CH2 | CH(CH3) | H | |
| 3-62 | 3,5-dichlor-phenyl | CH2 | CH2 | CH2 | H | |
| 3-63 | 2,6-dimethyl-phenyl | CH2 | CH2 | CH(CH3) | H | |
| 3-64 | 4-trifluormethyl-phenyl | CH2 | CH2 | CH(CH3) | H | |
| 3-65 | 2,5-dichlor-phenyl | CH2 | CH2 | CH(CH3) | H | |
| 3-66 | 4-phenoxy-phenyl | CH2 | CH2 | CH2 | H | |
| 3-67 | 3-chlor-phenyl | CH2 | CH2 | CH(CH3) | H | |
| 3-68 | 4-phenoxy-phenyl | CH2 | CH2 | CH(CH3) | H | |
| 3-69 | 2,4-dichlor-phenyl | CH2 | CH2 | CH(CH3) | H | |
| 3-70 | 2-difluormethoxy-phenyl | CH2 | CH2 | CH(CH3) | H | |
| 3-71 | 4-methoxy-phenyl | CH2 | CH2 | CH2 | H | |
| 3-72 | 4-chlor-phenyl | CH2 | CH2 | CH(CH3) | H | |
| 3-73 | 4-chlor-phenyl | CH2 | CH2 | CH(i-propyl) | H | |
| 3-74 | 4-fluor-phenyl | CH2 | CH2 | CH2 | H | |
| 3-75 | 4-chlor-phenyl | CH2 | CH2 | CH(n-propyl) | H | |
| 3-76 | 4-chlor-phenyl | CH2 | CH2 | CH(t-butyl) | H | |
| 3-77 | 2-chlor-phenyl | CH2 | CH2 | CH2 | H | |
| | | | | | | Verbindung Nr. 3-78, Solvent: [DMSO], Spektrometer: 399.95MHz |
| 3-78 | 2-thienyl | CH2 | CH2 | CH2 | H | 8,2574 (2,91); 8,2444 (5,08); 8,2319 (3,03); 7,795 (15,03); 7,782 (15,69); 7,3229 (8,64); 7,3203 (8,19); 7,3101 (9,55); 7,3076 (8,65); 7,175 (16); 7,162 (15,51); 6,9532 (6,85); 6,9446 (9,61); 6,9406 (7,2); 6,932 (8,69); 6,8918 (9,94); 6,8839 (8,05); 3,3293 (77,96); 3,3137 (5,96); 3,2968 (13,1); 3,2815 (13,26); 3,2648 (5,7); 2,8872 (9,12); 2,8681 (15,69); 2,8491 (9,99); 2,6712 (0,52); 2,5413 (3,88); 2,5061 (54,67); 2,5019 (71,28); 2,4977 (54,62); 2,3289 (0,45); 1,8988 (2,88); 1,8809 (8,81); 1,8623 (11,75); 1,8439 (8,49); 1,826 (2,61); -0,0002 (0,81) |
| | | | | | | Verbindung Nr. 3-79, Solvent: [DMSO], Spektrometer: 399.95MHz |
| 3-79 | 4-chlor-phenyl | CF2 | CH2 | - | H | 8,576 (0,68); 8,5604 (1,35); 8,5448 (0,7); 7,8206 (3,69); 7,8075 (3,86); 7,6075 (0,57); 7,5835 (16); 7,5685 (0,76); 7,5596 (0,63); 7,1796 (3,87); 7,1666 (3,76); 6,5757 (0,42); 4,0637 (0,84); 4,048 (0,87); 4,028 (1,88); 4,0122 (1,84); 3,9922 (0,97); 3,9764 (0,91); 3,3393 (75,51); 2,9456 (1,74); 2,5078 (20,84); 2,5034 (27,3); 2,4989 (20,68) |
| | | | | | | Verbindung Nr. 3-80, Solvent: [DMSO], Spektrometer: 601.6MHz |
| 3-80 | 2-thienyl | CH2 | CH2 | - | CH3 | 8,3171 (0,72); 7,7515 (6,42); 7,3562 (7,76); 7,1159 (11,38); 6,9622 (12,26); 6,8603 (0,55); 6,7564 (4,07); 5,7572 (0,42); 3,7 (8,1); 3,4844 (7,36); 3,3293 (377,32); 3,2181 (0,36); 3,1186 (10,28); 3,038 (16); 2,8784 (15,2); 2,6168 (1,63); 2,6138 (2,24); 2,6108 (1,63); 2,5414 (0,34); 2,5231 (3,99); 2,52 (4,92); 2,5169 (4,71); 2,508 (104,72); 2,5051 (230,25); 2,5021 (319,21); 2,499 (230,97); 2,4961 (106,28); 2,3892 (1,51); 2,3862 (2,11); 2,3832 (1,51); 1,336 (1,49); 1,2984 (0,4); 1,2582 (0,56); 1,2492 (1,84); 1,1875 (0.32); -0,0002(2,45) |
| | | | | | | Verbindung Nr. 3-81, Solvent: [DMSO], Spektrometer: 399.95MHz |
| 3-81 | 5-chlor-2-thienyl | CH2 | CH2 | - | H | 8,316 (0,52); 8,2988 (2,07); 8,2851 (3,82); 8,2712 (2,08); 7,8017 (15,07); 7,7887 (15,57); 7,3823 (13,33); 7,3785 (13,5); 7,1745 (16); 7,1615 (15,29); 6,9307 (10,42); 6,9277 (10,44); 3,5147 (4,05); 3,4975 (10,45); 3,4829 (10,74); 3,4658 (4,65); 3,3232 (27,64); 3,0565 (0,4); 3,0404 (7,54); 3,023 (14,29); 3,006 (6,44); 2,6756 (0,33); 2,6711 (0,46); 2,6665 (0,33); 2,5244 (1,35); 2,5197 (2,08); 2,5111 (25,96); 2,5066 (52,36); 2,5021 (68,84); 2,4975 (49,18); 2,4929 (23,27); 2,3288 (0,44); 1,3363 (0,55); 1,2494 (0,68); 0,008 (2,43); -0,0002 (67,03); - 0,0085 (2,09) |
| | | | | | | Verbindung Nr. 3-82, Solvent: [DMSO], Spektrometer: 399.95MHz |
| 3-82 | 4-chlor-phenyl | CH(CF3) | CH2 | - | H | 8,2644 (1,3); 8,2504 (2,34); 8,2362 (1,31); 7,7767 (7,71); 7,7637 (8,01); 7,4848 (3,65); 7,479 (1,78); 7,4688 (2,95); 7,4629 (16); 7,4485 (11,94); 7,4267 (3,09); 7,3972 (0,55); 7,1347 (8,26); 7,1217 (7,96); 4,112 (0,79); 4,0969 (0,99); 4,0884 (1,3); 4,0734 (1,39); 4,0645 (1,07); 4,0559 (1,23); 4,0497 (1); 4,0382 (2,79); 4,0204 (2,67); 4,0026 (0,88); 3,9198 (0,92); 3,906 (1,52); 3,8918 (1,06); 3,8858 (1,74); 3,872 (2,54); 3,8581 (1,32); 3,8106 (1,49); 3,7944 (1,68); 3,7877 (1,44); 3,7763 (1,23); 3,7718 (1,5); 3,7605 (0,98); 3,7539 (0,88); 3,7377 (0,77); 3,3243 (51,99); 2,6755 (0,36); 2,6711 (0,49); 2,6665 (0,36); 2,541 (0,33); 2,5241 (1,83); 2,5109 (28,37); 2,5065 (55,26); 2,502 (71,53); 2,4975 (51,75); 2,4931 (25,32); 2,3332 (0,34); 2,3288 (0,46); 2,3241 (0,35); 1,9892 (10,92); 1,3362 (0,43); 1,2492 (0,51); 1,1925 (2,96); 1,1747 (5,8); 1,1569 (2,85); 0,0079 (0,55); -0,0002 (13,71); -0,0085 (0,49) |
| | | | | | | Verbindung Nr. 3-83, Solvent: [DMSO], Spektrometer: 601.6MHz |
| 3-83 | 4-chlor-2-thienyl | CH2 | CH2 | - | H | 8,2915 (1,78); 8,2824 (3,27); 8,2731 (1,76); 7,7989 (15,81); 7,7902 (15,77); 7,3818 (15,13); 7,3793 (15,15); 7,1725 (15,74); 7,1638 (16); 6,9301 (9,11); 6,929 (8,54); 6,9279 (9,01); 3,5052 (3,7); 3,4937 (9,16); 3,484 (9,36); 3,4726 (4,09); 3,3547 (0,52); 3,3501 (0,72); 3,3266 (979,51); 3,3046 (1,02); 3,3 (0,57); 3,2923 (0,32); 3,0339 (6,1); 3,0223 (12,02); 3,0112 (5,63); 2,6531 (1,55); 2,6192 (0,58); 2,6163 (1,28); 2,6132 (1,78); 2,6101 (1,26); 2,6071 (0,58); 2,5541 (0,65); 2,5408 (489,74); 2,5345 (2,17); 2,5292 (0,67); 2,5225 (3,38); 2,5194 (4,3); 2,5163 (4,19); 2,5075 (96,46); 2,5045 (205,3); 2,5014 (283,65); 2,4984 (204,29); 2,4953 (94,77); 2,4247 (1,54); 2,3917 (0,57); 2,3887 (1,26); 2,3856 (1,74); 2,3826 (1,24); 2,3796 (0,55); 2,0735 (1,17); 0,0052 (0,62); -0,0002 (19,04); -0,0058 (0,54) |
| | | | | | | Verbindung Nr. 3-84, Solvent: [DMSO], Spektrometer: 399.95MHz |
| 3-84 | 4-brom-phenyl | CH2 | CH2 | - | H | 8,1965 (1,91); 8,1834 (3,45); 8,1699 (1,88); 7,785 (11,42); 7,7719 (11,87); 7,4945 (13,49); 7,4902 (5,13); 7,478 (5,31); 7,4737 (16); 7,2336 (13,63); 7,2127 (11,65); 7,1609 (12,19); 7,1479 (11,79); 3,4906 (3,21); 3,4734 (7,36); 3,4581 (7,32); 3,4406 (3,55); 3,3229 (66,74); 2,8314 (6,16); 2,8135 (11,19); 2,7957 (5,56); 2,6753 (0,67); 2,6707 (0,91); 2,6664 (0,69); 2,5062 (103,94); 2,5017 (134,01); 2,4973 (98,17); 2,3327 (0,63); 2,3285 (0,85); 2,3241 (0,61); 1,9889 (1,26); 1,3355 (0,37); 1,2494 (0,45); 1,1925 (0,33); 1,1748 (0,66); 1,157 (0,34); 0,0079 (2,32); -0,0002 (52,25); -0,0084 (2,1) |
| | | | | | | Verbindung Nr. 3-85, Solvent: [DMSO], Spektrometer: 399.95MHz |
| 3-85 | 2-(trifluormethyl)-4-chlorphenyl | CH2 | CH2 | - | H | 8,5752 (2,51); 8,5607 (4,96); 8,5462 (2,48); 7,8976 (15,64); 7,8929 (15,71); 7,7434 (7,7); 7,7381 (11,7); 7,7281 (5,7); 7,7228 (3,05); 7,7074 (5,8); 7,702 (4,53); 7,5824 (0,4); 7,5239 (8,07); 7,5033 (6,71); 6,8431 (16); 6,8384 (15,81); 5,7576 (1,8); 3,4777 (3,27); 3,461 (7,38); 3,4428 (7,25); 3,4266 (3,69); 3,3266 (36,26); 2,9946 (5,04); 2,9767 (8,59); 2,9587 (4,31); 2,6736 (0,44); 2,6692 (0,33); 2,5269 (1,37); 2,5136 (24,65); 2,5092 (48,76); 2,5047 (64,15); 2,5001 (46,88); 2,4957 (22,73); 2,3315 (0,42); 1,3372 (1,37); 1,25 (1,65); 1,2333 (0,39); 0,0079 (0,66); -0,0002 (18,26); -0,0084 (0,6) |
| | | | | | | Verbindung Nr. 3-86, Solvent: [DMSO], Spektrometer: |
| 3-86 | 2,4-dichlor-phenyl | O | CH2 | CH(CH3) | H | 399.95MHz8,1467 (2,33); 8,1269 (2,35); 7,8124 (9,53); 7,8046 (0,44); 7,7994 (9,96); 7,5734 (8,49); 7,567 (9,01); 7,3855 (3,9); 7,379 (3,61); 7,3633 (5,2); 7,3568 (4,97); 7,2379 (7,37); 7,2156 (5,51); 7,1821 (10,39); 7,1691 (9,94); 4,3873 (0,55); 4,3721 (1,22); 4,355 (1,55); 4,3363 (1,3); 4,3206 (0,66); 4,1536 (1,46); 4,1384 (1,32); 4,1292 (3,54); 4,114 (3,17); 4,1024 (3,57); 4,0882 (3,34); 4,078 (1,6); 4,0639 (1,26); 3,3812 (0,35); 3,3642 (0,72); 3,3295 (564,99); 3,2865 (0,37); 2,6803 (0,48); 2,6757 (1,03); 2,671 (1,43); 2,6665 (1,02); 2,662 (0,47); 2,5413 (16,36); 2,5245 (4,04); 2,5197 (6,26); 2,5111 (80,2); 2,5066 (162,64); 2,502 (215,3); 2,4974 (154,32); 2,4928 (72,38); 2,3378 (0,48); 2,3333 (1,01); 2,3288 (1,41); 2,3242 (0,99); 2,3196 (0,45); 1,2912 (16); 1,2743 (15,84); 0,008 (0,41); - 0,0002 (13,21); -0,0085 (0,38) |
| | | | | | | Verbindung Nr. 3-87, Solvent: [DMSO], Spektrometer: 399.95MHz |
| 3-87 | 4-chlor-phenyl | O | CH2 | CH(CH3) | H | 8,1732 (2,21); 8,1535 (2,23); 7,8088 (9,31); 7,7957 (9,67); 7,3463 (0,9); 7,3374 (10,82); 7,3318 (3,17); 7,3205 (3,39); 7,3149 (12,46); 7,306 (1,07); 7,1808 (9,77); 7,1678 (9,3); 7,0183 (1,08); 7,0094 (11,04); 7,0038 (3,18); 6,9925 (2,96); 6,9869 (9,63); 6,978 (0,84); 4,3431 (0,52); 4,3274 (1,18); 4,3103 (1,51); 4,2927 (1,25); 4,2766 (0,62); 4,0704 (1,79); 4,0549 (1,68); 4,0461 (3,05); 4,0306 (2,67); 3,9864 (3,11); 3,9725 (3,07); 3,962 (2,01); 3,9481 (1,72); 3,3724 (0,35); 3,3657 (0,44); 3,33 (343,39); 3,2974 (0,4); 2,6757 (0,58); 2,671 (0,81); 2,6665 (0,57); 2,5413 (7,01); 2,5244 (2,47); 2,5196 (3,96); 2,5111 (46,34); 2,5065 (93,01); 2,5019 (122,87); 2,4973 (88,34); 2,4928 (41,73); 2,3333 (0,58); 2,3287 (0,8); 2,3242 (0,58); 1,2637 (16); 1,2468 (15,88); 0,008 (0,36); -0,0002 (11,39); -0,0085 (0,34) |
| | | | | | | Verbindung Nr. 3-88, Solvent: [DMSO], Spektrometer: 399.95MHz |
| 3-88 | 2,4-dichlor-phenyl | S | CH2 | CH(CH3) | H | 8,2127 (2,51); 8,1927 (2,51); 7,8093 (8,4); 7,7962 (8,74); 7,629 (7,53); 7,6233 (7,85); 7,5753 (5,55); 7,5538 (7,31); 7,4289 (4,64); 7,4232 (4,33); 7,4075 (3,56); 7,4018 (3,38); 7,1795 (8,94); 7,1665 (8,56); 4,1732 (0,66); 4,1562 (1,46); 4,1382 (1,88); 4,1206 (1,5); 4,1032 (0,69); 3,3331 (88,06); 3,2873 (1,73); 3,2693 (1,69); 3,254 (3,22); 3,236 (3,06); 3,1907 (3,27); 3,175 (3,32); 3,1574 (1,88); 3,1417 (1,71); 2,5427 (22,56); 2,5259 (0,78); 2,5211 (1,24); 2,5126 (15,06); 2,5081 (30,14); 2,5035 (39,5); 2,4989 (28,24); 2,4943 (13,19); 1,3053 (16); 1,2886 (15,72); -0,0002 (4,02) |
| | | | | | | Verbindung Nr. 3-89, Solvent: [DMSO], Spektrometer: |
| 3-89 | 4-chlor-phenyl | S | CH2 | CH(CH3) | H | 399.95MHz-8,143 (2,14); 8,1229 (2,13); 7,8094 (0,61); 7,8038 (8,51); 7,7965 (0,76); 7,7908 (8,78); 7,4512 (0,33); 7,4293 (0,67); 7,4233 (5,42); 7,4179 (2,19); 7,4071 (3,22); 7,4014 (14,11); 7,3957 (2,32); 7,3894 (0,37); 7,3783 (2,34); 7,3726 (14,83); 7,3669 (3,31); 7,3561 (2,31); 7,3507 (5,74); 7,3448 (0,86); 7,3386 (0,62); 7,3211 (0,36); 7,1814 (0,6); 7,1752 (9); 7,1684 (0,71); 7,1622 (8,62); 4,1245 (0,67); 4,1076 (1,46); 4,0887 (1,73); 4,0713 (1,48); 4,0544 (0,68); 3,3341 (98,05); 3,239 (1,95); 3,2214 (1,87); 3,2053 (3,23); 3,1877 (3,09); 3,1258 (3,35); 3,1098 (3,38); 3,0921 (2,06); 3,0762 (1,91); 2,5424 (14); 2,5255 (0,62); 2,5207 (1); 2,5122 (13,34); 2,5077 (27,12); 2,5031 (36,01); 2,4985 (25,94); 2,4939 (12,29); 1,3121 (0,7); 1,2954 (0,73); 1,2784 (1,18); 1,266 (16); 1,2493 (15,71); - 0,0002 (3,14) |
| | | | | | | Verbindung Nr. 3-90, Solvent: [DMSO], Spektrometer: 399.95MHz |
| 3-90 | 4-chlor-phenyl | N(CH3) | CH2 | CH2 | H | 8,2498 (0,67); 8,2363 (1,24); 8,2229 (0,65); 7,7972 (3,49); 7,7842 (3,63); 7,1863 (0,53); 7,1775 (4,44); 7,1709 (4,92); 7,1576 (5,8); 6,7757 (0,57); 6,7671 (4,46); 6,7444 (3,95); 3,4981 (1,03); 3,4816 (2,75); 3,4655 (2,31); 3,4179 (1,25); 3,4033 (2,51); 3,3874 (1,93); 3,3716 (0,77); 3,3291 (101,38); 2,9226 (16); 2,6713 (0,33); 2,5414 (19,11); 2,5065 (40,27); 2,5021 (50,72); 2,4977 (36,93); 2,329 (0,32); 0,0078 (0,37); - 0,0002 (7,39) |

**Tabelle 4**

| | | | | | | |
|---|---|---|---|---|---|---|
| Verbindungen der Formel 1-4 | | | | | | |
| 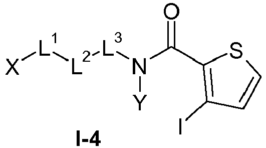 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 4-1 | 3-methyl-2-thienyl | CH2 | CH2 | - | H | WO-A 2006/108791 |
| 4-2 | 3-chlor-2-thienyl | CH2 | CH2 | - | H | WO-A 2006/108791 |
| 4-3 | 2,4-dichlor-phenyl | CH2 | CH2 | CH2 | H | WO-A 2008/101976 |
| 4-4 | 4-chlor-phenyl | CH2 | CH2 | - | H | |
| 4-5 | 2,4-dichlor-phenyl | CH2 | CH2 | - | H | WO-A 2007/108483 |
| 4-6 | 4-chlor-phenyl | CH(OCH3) | CH(CH3) | - | H | |
| 4-7 | 2,4-dichlor-phenyl | CH(OCH3) | CH(CH3) | - | H | |
| | | | | | | Verbindung Nr. 4-8, Solvent: [DMSO], Spektrometer: 399.95MHz |
| 4-8 | 4-chlor-phenyl | CH(CH3) | CH2 | - | H | 8,1978 (1,28); 8,1836 (2,49); 8,1694 (1,27); 7,6731 (9,43); 7,6604 (9,81); 7,3745 (0,77); 7,3688 (6,87); 7,3638 (2,5); 7,3527 (3,53); 7,3475 (15,13); 7,3419 (2,2); 7,3161 (2,24); 7,3108 (13,08); 7,3056 (3,36); 7,2943 (2,33); 7,2894 (6,14); 7,1873 (10,07); 7,1745 (9,59); 3,4045 (0,36); 3,3904 (3); 3,3871 (2,85); 3,3719 (5,93); 3,3549 (4,25); 3,3334 (135,39); 3,0838 (1,26); 3,0659 (2,5); 3,0481 (2,41); 3,0302 (1,12); 2,6716 (0,37); 2,5418 (34,95); 2,5249 (1,18); 2,5201 (1,86); 2,5115 (22,13); 2,507 (44,23); 2,5025 (58,05); 2,4979 (41,77); 2,4934 (19,73); 2,3293 (0,37); 1,2521 (16); 1,2346 (15,76); -0,0002 (7,44) |
| | | | | | | Verbindung Nr. 4-9, Solvent: [DMSO], Spektrometer: 399.95MHz |
| 4-9 | 2,4-dichlor-phenyl | CH(CH3) | CH2 | - | H | 8,2659 (1,43); 8,2517 (2,78); 8,2379 (1,37); 7,6708 (11,05); 7,6581 (11,5); 7,5626 (8,69); 7,5572 (9,18); 7,4833 (4,64); 7,4621 (8,93); 7,4228 (5,83); 7,4173 (5,36); 7,4017 (2,99); 7,3962 (2,83); 7,1832 (11,46); 7,1705 (10,82); 3,5655 (0,99); 3,5479 (2,21); 3,5307 (2,6); 3,5134 (1,78); 3,5092 (1,48); 3,4948 (1,4); 3,4771 (2,63); 3,4615 (3); 3,4453 (3,26); 3,4306 (2,49); 3,4125 (2,42); 3,3982 (1,06); 3,3796 (0,79); 3,3691 (0,46); 3,3297 (402,14); 3,2983 (0,41); 2,6802 (0,32); 2,6755 (0,69); 2,671 (0,95); 2,6664 (0,69); 2,6618 (0,32); 2,5413 (62,58); 2,5243 (2,87); 2,5195 (4,52); 2,511 (53,88); 2,5065 (108,84); 2,5019 (144,25); 2,4973 (104,06); 2,4927 (49,39); 2,3332 (0,66); 2,3286 (0,93); 2,3241 (0,65); 1,2414 (16); 1,2245 (15,82); 0,008 (0,5); -0,0002 (14,84); -0,0085 (0,45) |
| | | | | | | Verbindung Nr. 4-10, Solvent: [DMSO], Spektrometer: 399.95MHz |
| 4-10 | 4-chlor-phenyl | CH2 | CH(CH3) | - | H | 8,1201 (2,15); 8,0997 (2,16); 7,6702 (8,96); 7,6575 (9,35); 7,3518 (0,84); 7,3459 (7,05); 7,3409 (2,57); 7,3299 (3,49); 7,3247 (14,43); 7,319 (2,05); 7,2817 (11,16); 7,2767 (3,02); 7,2653 (2,24); 7,2605 (5,63); 7,1814 (9,66); 7,1687 (9,22); 4,1703 (0,59); 4,1538 (1,11); 4,1508 (1,23); 4,1341 (1,75); 4,1176 (1,27); 4,0979 (0,57); 3,3292 (373,27); 2,856 (1,31); 2,8363 (1,27); 2,8225 (2,79); 2,8028 (2,75); 2,7753 (2,78); 2,7597 (2,85); 2,7417 (1,32); 2,7261 (1,19); 2,6797 (0,41); 2,6754 (0,91); 2,6707 (1,26); 2,6662 (0,89); 2,6616 (0,42); 2,541 (11,06); 2,5242 (4,05); 2,5194 (6,28); 2,5108 (70,9); 2,5063 (142,56); 2,5017 (188,15); 2,497 (134,78); 2,4925 (63,12); 2,3376 (0,41); 2,333 (0,89); 2,3284 (1,22); 2,3238 (0,87); 2,3193 (0,4); 1,1641 (16); 1,1476 (15,9); 0,008 (0,56); -0,0002 (17,08); -0,0086 (0,5) |
| | | | | | | Verbindung Nr. 4-11, Solvent: [DMSO], Spektrometer: 399.95MHz |
| 4-11 | 2,4-dichlor-phenyl | CH2 | CH(CH3) | - | H | 8,1715 (2,47); 8,1501 (2,54); 7,6701 (9,62); 7,6574 (10,06); 7,5702 (6,37); 7,5652 (6,68); 7,4002 (2,84); 7,3795 (9,34); 7,365 (7,41); 7,3599 (6,8); 7,3444 (2,34); 7,3393 (2,44); 7,1782 (10,2); 7,1655 (9,72); 4,3099 (0,56); 4,2893 (1,25); 4,273 (1,72); 4,2568 (1,19); 4,2523 (1,03); 4,2363 (0,58); 3,3755 (0,44); 3,3304 (524,28); 3,2932 (0,66); 3,28 (0,37); 2,995 (0,76); 2,9591 (0,59); 2,944 (0,89); 2,9247 (4,16); 2,9161 (4,42); 2,9099 (4,06); 2,8956 (3,55); 2,8819 (0,7); 2,8614 (0,77); 2,6801 (0,53); 2,6755 (1,11); 2,6709 (1,52); 2,6663 (1,09); 2,6616 (0,5); 2,5412 (72,37); 2,5243 (4,63); 2,5196 (7,08); 2,511 (85,7); 2,5064 (173,71); 2,5018 (229,57); 2,4972 (164,3); 2,4926 (76,96); 2,3377 (0,52); 2,3332 (1,11); 2,3286 (1,52); 2,324 (1,08); 2,3195 (0,49); 1,2349 (0,56); 1,213 (16); 1,1963 (15,76); 0,0081 (0,66); -0,0002 (19,69); -0,0085 (0,52) |
| 4-12 | 4-chlor-phenyl | C(CH3)2 | CH2 | - | H | |
| 4-13 | 2,4-dichlor-phenyl | C(CH3)2 | CH2 | - | H | |
| 4-14 | 2-chlor-phenyl | CH2 | CH2 | - | H | |
| | | | | | | Verbindung Nr. 4-15, Solvent: [DMSO], Spektrometer: 399.95MHz |
| 4-15 | 3,4-dichlor-phenyl | CH2 | CH2 | - | H | 8,2748 (1,63); 8,2609 (3,12); 8,247 (1,61); 7,6903 (12,52); 7,6776 (12,97); 7,5589 (9,91); 7,5486 (0,57); 7,5381 (16); 7,5316 (8,43); 7,2734 (4,72); 7,2683 (4,54); 7,2528 (4,08); 7,2478 (3,97); 7,2011 (13,09); 7,1884 (12,55); 3,4944 (2,7); 3,4772 (6,94); 3,4626 (7,1); 3,4456 (2,94); 3,3623 (0,52); 3,3573 (0,55); 3,3317 (219,19); 3,31 (0,65); 2,8624 (5,18); 2,8452 (10,47); 2,8279 (4,63); 2,6761 (0,45); 2,6716 (0,61); 2,6669 (0,47); 2,5418 (54,74); 2,5248 (1,69); 2,5201 (2,77); 2,5116 (34,23); 2,5071 (68,94); 2,5025 (90,9); 2,4979 (65,17); 2,4933 (30,68); 2,3337 (0,43); 2,3292 (0,6); 2,3246 (0,41); -0,0002 (8,21) |
| 4-16 | 3,5-dichlor-phenyl | CH2 | CH2 | - | H | |
| 4-17 | 3-chlor-phenyl | CH2 | CH2 | - | H | |
| 4-18 | 2-fluor-phenyl | CH2 | CH2 | - | H | |
| 4-19 | 2,6-difluor-phenyl | CH2 | CH2 | - | H | Verbindung Nr. 4-19, Solvent: [DMSO], Spektrometer: 39995MHz¬8,3762 (1,84); 8,3617 (3,57); 8,3473 (1,83); 7,6858 (15,23); 7,6731 (15,98); 7,3627 (1,16); 7,3459 (2,52); 7,3418 (2,17); 7,3289 (1,79); 7,325 (4,81); 7,321 (1,91); 7,308 (2,29); 7,3041 (3,05); 7,2874 (1,45); 7,2011 (16); 7,1884 (15,11); 7,1023 (0,61); 7,0979 (0,9); 7,0858 (6,51); 7,0776 (1,25); 7,0659 (10,15); 7,0547 (1,26); 7,0458 (5,46); 7,0333 (0,71); 7,03 (0,52); 3,4505 (3); 3,4337 (7,14); 3,4175 (7,17); 3,4006 (3,27); 3,3603 (0,48); 3,3506 (0,96); 3,3316 (197,25); 3,3076 (0,42); 2,9097 (4,61); 2,8922 (8,53); 2,8747 (4,12); 2,6762 (0,37); 2,6717 (0,52); 2,6671 (0,37); 2,5418 (14,06); 2,525 (1,6); 2,5202 (2,5); 2,5117 (29,79); 2,5072 (59,77); 2,5026 (78,58); 2,4979 (56,22); 2,4934 (26,35); 2,3339 (0,37); 2,3293 (0,52); 2,3247 (0,36); -0,0002 (6,98) |
| 4-20 | 2,6-dichlor-phenyl | CH2 | CH2 | - | H | |
| 4-21 | 3-(trifluormethyl)-phenyl | CH2 | CH2 | - | H | |
| 4-22 | 4-(trifluormethyl)-phenyl | CH2 | CH2 | - | H | |
| 4-23 | 2-methyl-phenyl | CH2 | CH2 | - | H | |
| 4-24 | 2,4,6-trimethyl-phenyl | CH2 | CH2 | - | H | |
| 4-25 | 3,4-bismethoxy-phenyl | CH2 | CH2 | - | H | |
| 4-26 | phenyl | CH2 | CH2 | - | H | |
| 4-27 | 4-chlor-phenyl | C(CH2-CH2) | CH2 | - | H | |
| 4-28 | 2,4-dichlor-phenyl | C(CH2-CH2) | CH2 | - | H | |
| 4-29 | 4-chlor-phenyl | CH2 | C(CH2-CH2) | - | H | |
| 4-30 | 2,4-dichlor-phenyl | CH2 | C(CH2-CH2) | - | H | |
| 4-31 | 4-chlor-phenyl | O | CH2 | CH2 | H | |
| 4-32 | 2,4-dichlor-phenyl | O | CH2 | CH2 | H | Verbindung Nr. 4-32, Solvent: [DMSO], Spektrometer: 39995MHz¬8,3422 (2,49); 8,3288 (4,8); 8,3156 (2,57); 7,7222 (12,7); 7,7094 (13,41); 7,5727 (11,06); 7,5663 (12,06); 7,3863 (5,61); 7,3799 (5,35); 7,3642 (7,64); 7,3577 (7,48); 7,2441 (12,4); 7,2298 (14,09); 7,2218 (10,16); 7,2171 (14,12); 4,2214 (7,21); 4,2069 (16); 4,1924 (8); 3,6582 (4,04); 3,6439 (11,17); 3,6297 (10,84); 3,6154 (3,67); 3,3273 (96,57); 2,6761 (0,38); 2,6718 (0,51); 2,6675 (0,39); 2,542 (5,91); 2,5246 (1,61); 2,5071 (57,98); 2,5027 (76,93); 2,4983 (58,65); 2,3339 (0,36); 2,3294 (0,48); 2,325 (0,37); -0,0002 (3,39) |
| 4-33 | 4-chlor-phenyl | CH2 | CH2 | CH2 | H | |
| 4-34 | 4-chlor-phenyl | NCH3 | CH2 | CH2 | H | |
| 4-35 | 2,4-dichlor-phenyl | NCH3 | CH2 | CH2 | H | |
| 4-36 | 4-chlor-phenyl | CH(OCH3) | CH2 | - | H | |
| 4-37 | 2,4-dichlor-phenyl | CH(OCH3) | CH2 | - | H | |
| 4-38 | 2-thienyl | CH2 | CH2 | - | H | Verbindung Nr. 4-38, Solvent: [DMSO], Spektrometer: 399.95MHz 8,3451 (2,74); 8,3316 (5,03); 8,3181 (2,84); 7,7043 (15,02); 7,6916 (15,87); 7,3568 (8,12); 7,3538 (8); 7,3442 (9,03); 7,3411 (8,48); 7,2174 (16); 7,2047 (15,26); 6,9763 (6,01); 6,9677 (10,12); 6,9638 (5,89); 6,9551 (10,17); 6,9408 (9,96); 6,9335 (6,3); 3,4995 (4,52); 3,4819 (10,28); 3,4671 (10,58); 3,4493 (5,31); 3,3305 (31,33); 3,0738 (9,05); 3,0558 (15,86); 3,0378 (7,85); 2,5411 (5,79); 2,524 (0,86); 2,5063 (28,38); 2,5018 (37,56); 2,4974 (28,52); -0,0002 (0,8) |
| 4-39 | 3-thienyl | CH2 | CH2 | - | H | |
| 4-40 | 2-furyl | CH2 | CH2 | - | H | |
| 4-41 | 3-furyl | CH2 | CH2 | - | H | |
| 4-42 | phenyl | CH2 | CH2 | CH(CH3) | H | |
| 4-43 | phenyl | CH2 | CH2 | CH2 | H | |
| 4-44 | 2-Cl-phenyl | CH2 | CH2 | CH(CH3) | H | |
| 4-45 | 4-t-butyl-phenyl | CH2 | CH2 | CH2 | H | |
| 4-46 | 4-methyl-phenyl | CH2 | CH2 | CH2 | H | |
| 4-47 | phenyl | CH2 | CH2 | CH(CH2CH3) | H | |
| 4-48 | 2-methoxy-phenyl | CH2 | CH2 | CH2 | H | |
| 4-49 | 2-methyl-phenyl | CH2 | CH2 | CH2 | H | |
| 4-50 | 3-methyl-phenyl | CH2 | CH2 | CH2 | H | |
| 4-51 | 3-chlor-phenyl | CH2 | CH2 | CH2 | H | |
| 4-52 | 2,6-difluor-phenyl | CH2 | CH2 | CH2 | H | Verbindung Nr. 4-52, Solvent: [DMSO], Spektrometer: 39995MHz¬8,289 (3,25); 8,2754 (6,02); 8,2617 (3,36); 7,7312 (0,36); 7,7184 (0,45); 7,7002 (14,81); 7,6875 (15,63); 7,3533 (1,47); 7,3359 (3,65); 7,3329 (3,54); 7,3156 (6,68); 7,2952 (4,43); 7,278 (1,94); 7,2407 (0,44); 7,228 (0,48); 7,2108 (16); 7,198 (15,4); 7,1445 (0,34); 7,1223 (0,48); 7,1034 (1,35); 7,0904 (9,74); 7,0708 (15,16); 7,0513 (8,19); 7,0373 (1,2); 6,5636 (0,55); 3,3363 (59,45); 3,2877 (4,72); 3,2706 (10,64); 3,2546 (10,82); 3,2377 (5,03); 2,7211 (6,7); 2,7019 (11,21); 2,6825 (7,59); 2,5446 (8,86); 2,5095 (32,46); 2,5053 (42,82); 2,501 (33,42); 1,815 (2,56); 1,7961 (6,96); 1,7775 (9,62); 1,7588 (6,71); 1,7405 (2,35); -0,0002 (0,39) |
| 4-53 | 4-chlor-phenyl | CH2 | CH2 | CH2 | H | |
| 4-54 | 2,6-dichlor-phenyl | CH2 | CH2 | CH(CH3) | H | |
| 4-55 | 3,5-dichlor-phenyl | CH2 | CH2 | CH(CH3) | H | |
| 4-56 | 2,6-dimethyl-phenyl | CH2 | CH2 | CH2 | H | |
| 4-57 | 2,5-dichlor-phenyl | CH2 | CH2 | CH2 | H | |
| 4-58 | 4-i-propoxy-phenyl | CH2 | CH2 | CH2 | H | |
| 4-59 | 3-trifluormethyl-phenyl | CH2 | CH2 | CH(CH3) | H | |
| 4-60 | 4-methyl-phenyl | CH2 | CH2 | CH(CH3) | H | |
| 4-61 | 2-methyl-phenyl | CH2 | CH2 | CH(CH3) | H | |
| 4-62 | 3,4-dichlor-phenyl | CH2 | CH2 | CH(CH3) | H | |
| 4-63 | 3,5-dichlor-phenyl | CH2 | CH2 | CH2 | H | |
| 4-64 | 2,6-dimethyl-phenyl | CH2 | CH2 | CH(CH3) | H | |
| 4-65 | 4-trifluormethyl-phenyl | CH2 | CH2 | CH(CH3) | H | |
| 4-66 | 2,5-dichlor-phenyl | CH2 | CH2 | CH(CH3) | H | |
| 4-67 | 4-phenoxy-phenyl | CH2 | CH2 | CH2 | H | |
| 4-68 | 3-chlor-phenyl | CH2 | CH2 | CH(CH3) | H | |
| 4-69 | 4-phenoxy-phenyl | CH2 | CH2 | CH(CH3) | H | |
| 4-70 | 2,4-dichlor-phenyl | CH2 | CH2 | CH(CH3) | H | |
| 4-71 | 2-difluormethoxy-phenyl | CH2 | CH2 | CH(CH3) | H | |
| 4-72 | 4-methoxy-phenyl | CH2 | CH2 | CH2 | H | |
| 4-73 | 4-chlor-phenyl | CH2 | CH2 | CH(CH3) | H | |
| 4-74 | 4-chlor-phenyl | CH2 | CH2 | CH(i-propyl) | H | |
| 4-75 | 4-fluor-phenyl | CH2 | CH2 | CH2 | H | |
| 4-76 | 4-chlor-phenyl | CH2 | CH2 | CH(n-propyl) | H | |
| 4-77 | 4-chlor-phenyl | CH2 | CH2 | CH(t-butyl) | H | |
| 4-78 | 2-chlor-phenyl | CH2 | CH2 | CH2 | H | |
| 4-79 | 4-chlor-phenyl | CH2 | CH(CH3) | - | cyclopropyl | WO-A 2007/060164 |
| 4-80 | 2-thienyl | CH2 | CH2 | CH2 | H | Verbindung Nr. 4-80, Solvent: [DMSO], Spektrometer: 39995MHz¬8,2883 (3,21); 8,2753 (5,82); 8,2622 (3,42); 7,6997 (14,19); 7,6984 (13,56); 7,687 (14,99); 7,6858 (14,27); 7,3213 (9,12); 7,3093 (9,89); 7,2101 (15,06); 7,209 (14,6); 7,1974 (14,74); 7,1962 (14,13); 6,9542 (6,4); 6,9453 (9,34); 6,9423 (7,53); 6,9333 (8,71); 6,8987 (10,86); 6,8907 (8,47); 3,3289 (59,48); 3,2994 (5,47); 3,2828 (13,42); 3,2674 (13,55); 3,2509 (5,79); 2,9046 (9,38); 2,8856 (16); 2,8664 (10,3); 2,6709 (0,49); 2,5411 (5,09); 2,5398 (4,82); 2,5011 (68,2); 2,4974 (55,76); 2,3281 (0,44); 1,8989 (2,91); 1,881 (9,05); 1,8622 (12,17); 1,844 (8,84); 1,8262 (2,7); -0,0002 (0,92); - 0,0015 (0,88) |
| | | | | | | Verbindung Nr. 4-81, Solvent: [DMSO], Spektrometer: 399.95MHz |
| 4-81 | 4-brom-phenyl | CH2 | CH2 | - | H | 8,2659 (1,89); 8,2521 (3,57); 8,2382 (1,87); 7,6881 (11,61); 7,6754 (12,18); 7,5003 (1,69); 7,494 (13,45); 7,4896 (4,69); 7,4777 (4,96); 7,4732 (16); 7,4671 (2,05); 7,2381 (13,04); 7,2173 (11,31); 7,2023 (12,33); 7,1896 (11,63); 4,0378 (0,54); 4,02 (0,55); 3,4707 (2,96); 3,4535 (6,6); 3,4383 (6,52); 3,4207 (3,18); 3,3229 (55,32); 2,8306 (5,69); 2,8126 (10,26); 2,7948 (5,12); 2,6751 (0,53); 2,6707 (0,71); 2,6662 (0,53); 2,5238 (2,53); 2,5104 (42,3); 2,5061 (82,96); 2,5017 (107,91); 2,4972 (78,3); 2,4929 (38,31); 2,3328 (0,51); 2,3283 (0,69); 2,324 (0,52); 1,9889 (2,44); 1,2584 (0,37); 1,2491 (0,34); 1,1925 (0,64); 1,1747 (1,25); 1,1569 (0,63); 0,0079 (1,74); -0,0002 (44,61); -0,0085 (1,59) |
| | | | | | | Verbindung Nr. 4-82, Solvent: [DMSO], Spektrometer: 399.95MHz |
| 4-82 | 4-chlor-phenyl | CH2 | C(CH3)2 | - | H | 7,6667 (2,46); 7,654 (2,62); 7,5355 (1,71); 7,3362 (2,54); 7,3317 (1,01); 7,3151 (3,63); 7,2185 (3,4); 7,1975 (2,5); 7,1805 (2,68); 7,1678 (2,52); 3,3365 (20,24); 3,3294 (25,49); 3,0901 (4,92); 2,5063 (12,56); 2,502 (15,88); 2,4976 (11,94); 1,3354 (0,48); 1,314 (16); -0,0002 (4,44) |
| 4-83 | 2,4-dichlor-phenyl | CH2 | C(CH3)2 | - | H | Verbindung Nr. 4-83, Solvent: [DMSO], Spektrometer: 39995MHz¬7,6871 (2,66); 7,6797 (2,07); 7,675 (3,39); 7,5904 (1,85); 7,5854 (2,22); 7,3686 (0,59); 7,3636 (0,58); 7,3479 (1,79); 7,3428 (2,06); 7,3286 (3,03); 7,3078 (0,97); 7,1961 (2,31); 7,1834 (2,26); 3,3548 (62,71); 3,3461 (49,33); 3,3409 (49,17); 3,3339 (34,47); 3,2763 (5,46); 2,5073 (23,38); 2,5032 (31,78); 2,4991 (26,77); 1,9891 (1); 1,3507 (16); 1,1752 (0,54); -0,0002 (7,13) |
| | | | | | | Verbindung Nr. 4-84, Solvent: [DMSO], Spektrometer: 399.95MHz |
| 4-84 | 2-(trifluormethyl)-4-chlorphenyl | CH2 | CH2 | - | H | 8,383 (2,04); 8,3687 (3,97); 8,3546 (1,99); 8,3164 (0,35); 7,7528 (6,84); 7,7474 (9,86); 7,7346 (4,64); 7,7293 (2,73); 7,714 (5,2); 7,7084 (4,18); 7,6994 (15,25); 7,6866 (15,78); 7,5619 (6,76); 7,5412 (5,43); 7,2103 (16); 7,1976 (15,19); 7,187 (0,43); 7,1816 (0,43); 5,7568 (1,06); 3,509 (2,76); 3,4923 (6,41); 3,4763 (6,43); 3,4591 (3,03); 3,323 (95,71); 3,3035 (4,12); 3,2952 (0,44); 3,2857 (3,28); 3,0133 (4,15); 2,996 (7,49); 2,9784 (3,61); 2,6956 (13,62); 2,6802 (0,39); 2,6758 (0,78); 2,6712 (1,07); 2,6666 (0,76); 2,6621 (0,35); 2,5246 (3,29); 2,5199 (5,09); 2,5113 (57,87); 2,5067 (115,78); 2,5021 (152,53); 2,4975 (109,23); 2,493 (50,69); 2,3381 (0,36); 2,3335 (0,75); 2,3289 (1,01); 2,3243 (0,72); 2,3198 (0,33); 2,1972 (1,42); 2,1773 (2,71); 2,1569 (1,91); 1,9391 (0,72); 1,9208 (1,87); 1,9176 (1,37); 1,9118 (0,34); 1,9024 (1,96); 1,8994 (1,59); 1,8852 (0,65); 1,8823 (1,68); 1,8792 (0,77); 1,8635 (0,5); 1,3358 (4,81); 1,2984 (0,49); 1,2586 (0,86); 1,2495 (5,9); 1,2344 (1,02); 1,1874 (1,93); 0,008 (1,56); -0,0002 (47,94); -0,0085 (1,37) |
| | | | | | | Verbindung Nr. 4-85, Solvent: [DMSO], Spektrometer: 399.95MHz |
| 4-85 | 4-chlor-phenyl | N(CH3) | CH2 | CH2 | H | 8,3095 (0,73); 8,2956 (1,36); 8,2814 (0,7); 7,6991 (3,86); 7,6864 (4); 7,2124 (4,09); 7,1997 (3,94); 7,1915 (0,59); 7,1826 (4,31); 7,1599 (4,68); 7,1511 (0,53); 6,7634 (4,39); 6,7406 (3,91); 3,4974 (1,23); 3,4807 (2,99); 3,4642 (2,31); 3,3992 (1,39); 3,3836 (2,95); 3,3677 (3,11); 3,3355 (303,57); 3,2852 (0,41); 3,2753 (0,34); 2,9276 (16); 2,676 (0,43); 2,6715 (0,57); 2,5417 (28,45); 2,5068 (68,26); 2,5024 (86,24); 2,4979 (63,01); 2,3337 (0,41); 2,3293 (0,56); 2,3247 (0,41); (0,0078) (0,65); -0,0002 (13,43); -0,0084 -(0.57) |
| | | | | | | Verbindung Nr. 4-86, Solvent: [DMSO], Spektrometer: 399.95MHz |
| 4-86 | 3-chlor-4-(trifluormethyl)-phenyl | CH2 | CH2 | - | H | 8,3159 (1,13); 8,3109 (1,84); 8,2967 (3,57); 8,2828 (1,84); 7,7891 (7,25); 7,7689 (8,48); 7,7483 (0,44); 7,6916 (15,23); 7,6789 (16); 7,6296 (8,18); 7,546 (0,53); 7,4507 (4,39); 7,4307 (3,86); 7,358 (0,32); 7,2005 (15,96); 7,1877 (15,29); 3,539 (2,93); 3,5219 (7,7); 3,5073 (7,92); 3,4905 (3,23); 3,3239 (53,87); 3,2047 (0,7); 3,1898 (0,72); 2,9553 (4,99); 2,9383 (9,85); 2,9212 (4,4); 2,7977 (0,33); 2,7802 (0,59); 2,6765 (0,5); 2,6718 (0,68); 2,6672 (0,49); 2,5251 (2,19); 2,5204 (3,32); 2,5118 (37,85); 2,5073 (76,06); 2,5027 (100,26); 2,4981 (71,49); 2,4935 (33,32); 2,3341 (0,47); 2,3295 (0,64); 2,3249 (0,44); 1,336 (3,03); 1,3299 (5,74); 1,299 (0,49); 1,2697 (0,39); 1,2589 (0,85); 1,2498 (2,68); 1,2339 (0,51); 1,2116 (0,42); 1,188 (0,49) |
| | | | | | | Verbindung Nr. 4-87, Solvent: [DMSO], Spektrometer: 399.95MHz |
| 4-87 | 4-chlor-3-(trifluormethyl)-phenyl | CH2 | CH2 | - | H | 8,2897 (2,17); 8,276 (4,12); 8,2624 (2,16); 7,736 (8,36); 7,7315 (8,67); 7,6972 (15,06); 7,6844 (15,74); 7,6656 (5,48); 7,645 (10,25); 7,6029 (5,8); 7,5986 (5,48); 7,5824 (3,08); 7,5779 (2,96); 7,2045 (16); 7,1918 (15,22); 5,7641 (0,79); 3,5293 (3,38); 3,5124 (8,83); 3,4976 (9); 3,481 (3,6); 3,3339 (42,56); 2,9747 (0,38); 2,9521 (6,46); 2,9352 (12,81); 2,9181 (5,67); 2,6835 (0,35); 2,6789 (0,47); 2,6743 (0,35); 2,5321 (1,78); 2,5188 (26,97); 2,5144 (53,04); 2,5098 (69,14); 2,5052 (49,99); 2,5007 (23,99); 2,3365 (0,43); 1,9964 (0,51); 1,3435 (0,51); 1,2563 (0,69) |
| | | | | | | Verbindung Nr. 4-88, Solvent: [DMSO], Spektrometer: 399.95MHz |
| 4-88 | 2-chlor-4-(trifluormethyl)-phenyl | O | CH2 | C(CH3)2 | H | 7,8748 (1,62); 7,8251 (1,38); 7,8201 (1,46); 7,6895 (2,54); 7,6769 (2,47); 7,6643 (0,8); 7,6599 (0,74); 7,3646 (1,25); 7,343 (1,13); 7,1933 (2,36); 7,1806 (2,22); 4,3632 (4,84); 3,3302 (38,37); 2,5421 (4,27); 2,5251 (0,4); 2,5117 (8,48); 2,5074 (16,74); 2,5029 (21,85); 2,4984 (16,2); 2,4944 (8,12); 1,4942 (16); 1,4718 (0,33); 1,4664 (0,61) |
| | | | | | | Verbindung Nr. 4-89, Solvent: [DMSO], Spektrometer: 399.95MHz |
| 4-89 | 4-chlor-2-(trifluormethyl)-phenyl | O | CH3 | C(CH3)3 | H | 7,8044 (1,53); 7,7025 (0,59); 7,6957 (0,8); 7,6883 (2,87); 7,6803 (0,7); 7,6755 (3,55); 7,6604 (1,81); 7,6539 (1,1); 7,3165 (1,19); 7,2943 (1,07); 7,1974 (2,96); 7,1847 (2,85); 4,3156 (4,63); 3,3281 (49,55); 2,5417 (6,3); 2,5248 (0,58); 2,52 (0,91); 2,5115 (11,41); 2,5069 (22,83); 2,5023 (29,89); 2,4977 (21,3); 2,4932 (9,93); 1,4545 (16); 1,4259 (0,69) |
| | | | | | | Verbindung Nr. 4-90, Solvent: [DMSO], Spektrometer: 399.95MHz |
| 4-90 | 2-chlor-4-(trifluormethyl)-phenyl | O | CH2 | CH2 | H | 8,3684 (1,98); 8,3548 (3,86); 8,3416 (2,02); 7,8377 (7,1); 7,8328 (7,68); 7,7229 (15,04); 7,7102 (16); 7,7044 (3,72); 7,6989 (3,42); 7,6814 (4,1); 7,6771 (3,85); 7,6674 (0,48); 7,5236 (0,32); 7,5183 (0,34); 7,4413 (0,38); 7,4093 (6,44); 7,3876 (5,68); 7,2302 (15,98); 7,2175 (15,09); 4,3275 (5,82); 4,3131 (12,95); 4,2988 (6,26); 3,6945 (3,18); 3,6803 (9,06); 3,6662 (8,84); 3,652 (3); 3,365 (0,37); 3,3286 (261,25); 2,6806 (0,33); 2,6762 (0,72); 2,6716 (0,97); 2,6671 (0,7); 2,5419 (19,71); 2,525 (2,86); 2,5202 (4,63); 2,5117 (56,62); 2,5072 (112,56); 2,5026 (146,97); 2,498 (105,33); 2,4935 (49,52); 2,3385 (0,34); 2,3339 (0,7); 2,3294 (0,94); 2,3248 (0,68); 1,2343 (0,52); - 0,0002 (1,06) |

**Tabelle 5**

| | | | | | | |
|---|---|---|---|---|---|---|
| Verbindungen der Formel I-5 | | | | | | |
| 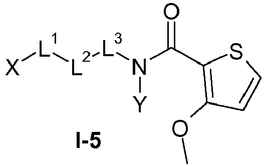 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 5-1 | 3-methyl-2-thienyl | CH2 | CH2 | - | H | |
| | | | | | | Verbindung Nr. 5-2, Solvent: [DMSO], Spektrometer: 399.95MHz |
| 5-2 | 2,4-dichlor-phenyl | CH2 | CH2 | CH2 | H | 7,697 (3,12); 7,6832 (3,24); 7,5658 (2,74); 7,5605 (2,68); 7,5518 (0,84); 7,5372 (0,43); 7,4228 (1,38); 7,4021 (3,09); 7,3743 (2,13); 7,369 (1,95); 7,3537 (0,91); 7,3484 (0,9); 7,1176 (3,41); 7,1038 (3,28); 3,9577 (16); 3,337 (35,89); 3,3209 (1,01); 3,3037 (1,78); 3,2879 (1,77); 3,2709 (0,81); 2,7282 (1,35); 2,7097 (1,8); 2,6899 (1,45); 2,5436 (4,79); 2,5218 (0,45); 2,5133 (5); 2,5088 (9,88); 2,5042 (13,04); 2,4996 (9,47); 2,4951 (4,5); 1,8224 (0,39); 1,8039 (1,03); 1,7855 (1,37); 1,7673 (0,97); 1,749 (0,34); -0,0002 (0,56) |
| 5-3 | 4-chlor-phenyl | CH2 | CH2 | - | H | |
| 5-4 | 2,4-dichlor-phenyl | CH2 | CH2 | - | H | |
| 5-5 | 4-chlor-phenyl | CH(OCH3) | CH(CH3) | - | H | |
| 5-6 | 2,4-dichlor-phenyl | CH(OCH3) | CH(CH3) | - | H | |
| 5-7 | 4-chlor-phenyl | CH(CH3) | CH2 | - | H | |
| 5-8 | 2,4-dichlor-phenyl | CH(CH3) | CH2 | - | H | |
| 5-9 | 4-chlor-phenyl | CH2 | CH(CH3) | - | H | |
| 5-10 | 2,4-dichlor-phenyl | CH2 | CH(CH3) | - | H | |
| 5-11 | 4-chlor-phenyl | C(CH3)2 | CH2 | - | H | |
| 5-12 | 2,4-dichlor-phenyl | C(CH3)2 | CH2 | - | H | |
| 5-13 | 2-chlor-phenyl | CH2 | CH2 | - | H | |
| 5-14 | 3,4-dichlor-phenyl | CH2 | CH2 | - | H | |
| 5-15 | 3,5-dichlor-phenyl | CH2 | CH2 | - | H | |
| 5-16 | 3-chlor-phenyl | CH2 | CH2 | - | H | |
| 5-17 | 2-fluor-phenyl | CH2 | CH2 | - | H | |
| 5-18 | 2,6-difluor-phenyl | CH2 | CH2 | - | H | |
| 5-19 | 2,6-dichlor-phenyl | CH2 | CH2 | - | H | |
| 5-20 | 3-(trifluormethyl)-phenyl | CH2 | CH2 | - | H | |
| 5-21 | 4-(trifluormethyl)-phenyl | CH2 | CH2 | - | H | |
| 5-22 | 2-methyl-phenyl | CH2 | CH2 | - | H | |
| 5-23 | 2,4,6-trimethyl-phenyl | CH2 | CH2 | - | H | |
| 5-24 | 3,4-bismethoxy-phenyl | CH2 | CH2 | - | H | |
| 5-25 | phenyl | CH2 | CH2 | - | H | |
| 5-26 | 4-chlor-phenyl | C(CH2-CH2) | CH2 | - | H | |
| 5-27 | 2,4-dichlor-phenyl | C(CH2-CH2) | CH2 | - | H | |
| 5-28 | 4-chlor-phenyl | CH2 | C(CH2-CH2) | - | H | |
| 5-29 | 2,4-dichlor-phenyl | CH2 | C(CH2-CH2) | - | H | |
| 5-30 | 4-chlor-phenyl | O | CH2 | CH2 | H | |
| | | | | | | Verbindung Nr. 5-31, Solvent: [DMSO], Spektrometer: 399.95MHz |
| 5-31 | 2,4-dichlor-phenyl | O | CH2 | CH2 | H | 7,7368 (3,04); 7,723 (3,67); 7,7075 (1,23); 7,6932 (0,64); 7,5902 (2,45); 7,5838 (2,66); 7,388 (1,32); 7,3815 (1,26); 7,3658 (1,81); 7,3593 (1,79); 7,2512 (3,06); 7,2289 (2,26); 7,1361 (3,32); 7,1223 (3,24); 4,1952 (1,81); 4,1806 (4,03); 4,1662 (2,05); 3,9646 (16); 3,7044 (1,03); 3,69 (2,92); 3,6754 (2,84); 3,6608 (0,95); 3,3343 (12,36); 2,5452 (8,06); 2,5101 (6,38); 2,5057 (8,56); 2,5013 (6,61); -0,0002 (0,35) |
| 5-32 | 4-chlor-phenyl | CH2 | CH2 | CH2 | H | |
| 5-33 | 4-chlor-phenyl | NCH3 | CH2 | CH2 | H | |
| 5-34 | 2,4-dichlor-phenyl | NCH3 | CH2 | CH2 | H | |
| 5-35 | 4-chlor-phenyl | CH(OCH3) | CH2 | - | H | |
| 5-36 | 2,4-dichlor-phenyl | CH(OCH3) | CH2 | - | H | |
| | | | | | | Verbindung Nr. 5-37, Solvent: [DMSO], Spektrometer: 399.95MHz |
| 5-37 | 2-thienyl | CH2 | CH2 | - | H | 7,7084 (2,75); 7,6946 (2,9); 7,6176 (0,54); 7,6034 (1,01); 7,5895 (0,58); 7,3724 (1,54); 7,3698 (1,55); 7,3597 (1,72); 7,357 (1,64); 7,1144 (3,13); 7,1006 (3,03); 6,9939 (1,3); 6,9853 (1,79); 6,9813 (1,37); 6,9726 (1,66); 6,9295 (1,84); 6,9221 (1,5); 3,9191 (16); 3,5354 (1); 3,5179 (2,4); 3,5026 (2,47); 3,4851 (1,17); 3,3301 (19,26); 3,0521 (1,89); 3,0343 (3,43); 3,0166 (1,68); 2,5415 (0,53); 2,5065 (10,07); 2,5021 (13,37); 2,4978 (10,39) |
| 5-38 | 3-thienyl | CH2 | CH2 | - | H | |
| 5-39 | 2-furyl | CH2 | CH2 | - | H | |
| 5-40 | 3-furyl | CH2 | CH2 | - | H | |
| 5-41 | phenyl | CH2 | CH2 | CH(CH3) | H | |
| 5-42 | phenyl | CH2 | CH2 | CH2 | H | |
| 5-43 | 2-Cl-phenyl | CH2 | CH2 | CH(CH3) | H | |
| 5-44 | 4-t-butyl-phenyl | CH2 | CH2 | CH2 | H | |
| 5-45 | 4-methyl-phenyl | CH2 | CH2 | CH2 | H | |
| 5-46 | phenyl | CH2 | CH2 | CH(CH2CH3) | H | |
| 5-47 | 2-methoxy-phenyl | CH2 | CH2 | CH2 | H | |
| 5-48 | 2-methyl-phenyl | CH2 | CH2 | CH2 | H | |
| 5-49 | 3-methyl-phenyl | CH2 | CH2 | CH2 | H | |
| 5-50 | 3-chlor-phenyl | CH2 | CH2 | CH2 | H | |
| | | | | | | Verbindung Nr. 5-51, Solvent: [DMSO], Spektrometer: 399.95MHz |
| 5-51 | 2,6-difluor-phenyl | CH2 | CH2 | CH2 | H | 7,699 (2,76); 7,6853 (2,89); 7,6047 (0,51); 7,5902 (0,96); 7,5757 (0,53); 7,3315 (0,63); 7,3283 (0,61); 7,3109 (1,18); 7,2902 (0,78); 7,2733 (0,35); 7,1187 (3,18); 7,1048 (3,18); 7,0898 (1,68); 7,0698 (2,67); 7,05 (1,37); 3,9774 (0,42); 3,9593 (16); 3,3301 (31,44); 3,2955 (0,86); 3,2788 (1,98); 3,2619 (1,97); 3,2452 (0,87); 2,6756 (1,24); 2,6567 (2,12); 2,6377 (1,25); 2,5074 (16,9); 2,503 (22,52); 2,4986 (17,23); 1,7884 (0,44); 1,7699 (1,29); 1,7515 (1,78); 1,7332 (1,22); 1,7151 (0,38) |
| 5-52 | 4-chlor-phenyl | CH2 | CH2 | CH2 | H | |
| 5-53 | 2,6-dichlor-phenyl | CH2 | CH2 | CH(CH3) | H | |
| 5-54 | 3,5-dichlor-phenyl | CH2 | CH2 | CH(CH3) | H | |
| 5-55 | 2,6-dimethyl-phenyl | CH2 | CH2 | CH2 | H | |
| 5-56 | 2,5-dichlor-phenyl | CH2 | CH2 | CH2 | H | |
| 5-57 | 4-i-propoxy-phenyl | CH2 | CH2 | CH2 | H | |
| 5-58 | 3-trifluormethyl-phenyl | CH2 | CH2 | CH(CH3) | H | |
| 5-59 | 4-methyl-phenyl | CH2 | CH2 | CH(CH3) | H | |
| 5-60 | 2-methyl-phenyl | CH2 | CH2 | CH(CH3) | H | |
| 5-61 | 3,4-dichlor-phenyl | CH2 | CH2 | CH(CH3) | H | |
| 5-62 | 3,5-dichlor-phenyl | CH2 | CH2 | CH2 | H | |
| 5-63 | 2,6-dimethyl-phenyl | CH2 | CH2 | CH(CH3) | H | |
| 5-64 | 4-trifluormethyl-phenyl | CH2 | CH2 | CH(CH3) | H | |
| 5-65 | 2,5-dichlor-phenyl | CH2 | CH2 | CH(CH3) | H | |
| 5-66 | 4-phenoxy-phenyl | CH2 | CH2 | CH2 | H | |
| 5-67 | 3-chlor-phenyl | CH2 | CH2 | CH(CH3) | H | |
| 5-68 | 4-phenoxy-phenyl | CH2 | CH2 | CH(CH3) | H | |
| 5-69 | 2,4-dichlor-phenyl | CH2 | CH2 | CH(CH3) | H | |
| 5-70 | 2-difluormethoxy-phenyl | CH2 | CH2 | CH(CH3) | H | |
| 5-71 | 4-methoxy-phenyl | CH2 | CH2 | CH2 | H | |
| 5-72 | 4-chlor-phenyl | CH2 | CH2 | CH(CH3) | H | |
| 5-73 | 4-chlor-phenyl | CH2 | CH2 | CH(i-propyl) | H | |
| 5-74 | 4-fluor-phenyl | CH2 | CH2 | CH2 | H | |
| 5-75 | 4-chlor-phenyl | CH2 | CH2 | CH(n-propyl) | H | |
| 5-76 | 4-chlor-phenyl | CH2 | CH2 | CH(t-butyl) | H | |
| 5-77 | 2-chlor-phenyl | CH2 | CH2 | CH2 | H | |
| | | | | | | Verbindung Nr. 5-78, Solvent: [DMSO], Spektrometer: 399.95MHz |
| 5-78 | 2-thienyl | CH2 | CH2 | CH2 | H | 7,6992 (2,67); 7,6854 (2,81); 7,5655 (0,5); 7,5515 (0,93); 7,5369 (0,53); 7,3207 (1,41); 7,318 (1,5); 7,308 (1,58); 7,3052 (1,59); 7,1195 (3,07); 7,1056 (2,96); 6,9505 (1,23); 6,942 (1,68); 6,9379 (1,28); 6,9293 (1,57); 6,8857 (1,69); 6,8793 (1,35); 3,9627 (16); 3,3285 (39,07); 3,315 (2,61); 3,2972 (2,23); 3,2808 (1,03); 2,8407 (1,59); 2,8217 (2,79); 2,8027 (1,71); 2,5064 (18,69); 2,5019 (25,1); 2,4975 (19,4); 1,8814 (0,51); 1,8627 (1,48); 1,8444 (1,98); 1,8264 (1,4); 1,8079 (0,46); -0,0002 (0,35) |
| | | | | | | [DMSO], Spektrometer: 399,95MHz |
| 5-79 | 2,6-difluor-phenyl | CH2 | CH(CH3) | - | H | 7,6897 (2,73); 7,6759 (2,87); 7,3397 (0,77); 7,3225 (1,32); 7,3021 (0,99); 7,2848 (0,44); 7,263 (1,32); 7,2424 (1,34); 7,1118 (3,25); 7,098 (3,3); 7,091 (2,38); 7,0715 (3,23); 7,0517 (1,74); 4,2493 (0,71); 4,2314 (0,93); 4,2136 (0,74); 4,1963 (0,33); 3,9594 (16); 3,3402 (27,46); 2,8908 (2,98); 2,8731 (2,73); 2,5041 (17,82); 2,5 (15,57); 1,1927 (5,75); 1,1763 (5,8); -0,0002 (6,51) |
| 5-80 | 4-chlor-phenyl | N(CH3) | CH2 | CH2 | H | |

**Tabelle 6**

| | | | | | | |
|---|---|---|---|---|---|---|
| Verbindungen der Formel 1-6 | | | | | | |
| 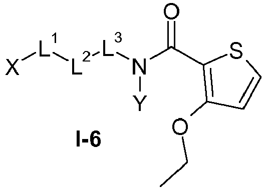 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 6-1 | 3-methyl-2-thienyl | CH2 | CH2 | - | H | |
| | | | | | | Verbindung Nr. 6-2, Solvent: [DMSO], Spektrometer: 399.95MHz |
| 6-2 | 2,4-dichlor-phenyl | CH2 | CH2 | CH2 | H | 7,6931 (5,88); 7,6793 (6,11); 7,5677 (4,29); 7,5625 (4,38); 7,5055 (0,8); 7,4911 (1,52); 7,4767 (0,79); 7,4167 (2,38); 7,396 (5,89); 7,3731 (4,19); 7,3679 (3,82); 7,3525 (1,62); 7,3472 (1,61); 7,1121 (6,09); 7,0982 (5,83); 4,2841 (2,28); 4,2666 (7,51); 4,2491 (7,58); 4,2317 (2,34); 3,3386 (81,64); 3,3287 (4,96); 3,3131 (3,75); 3,2963 (1,48); 2,7431 (2,5); 2,7244 (3,13); 2,7046 (2,67); 2,5439 (4,54); 2,5271 (0,54); 2,5223 (0,86); 2,5137 (9,69); 2,5092 (19,29); 2,5046 (25,6); 2,5 (18,54); 2,4954 (8,72); 1,8254 (0,72); 1,8079 (1,93); 1,7888 (2,4); 1,77 (1,87); 1,7525 (0,63); 1,3855 (7,72); 1,3681 (16); 1,3506 (7,5); -0,0002 (0,89) |
| 6-3 | 4-chlor-phenyl | CH2 | CH2 | - | H | |
| 6-4 | 2,4-dichlor-phenyl | CH2 | CH2 | - | H | |
| 6-5 | 4-chlor-phenyl | CH(OCH3) | CH(CH3) | - | H | |
| 6-6 | 2,4-dichlor-phenyl | CH(OCH3) | CH(CH3) | - | H | |
| 6-7 | 4-chlor-phenyl | CH(CH3) | CH2 | - | H | [DMSO]: 7.69, d, 1H; 7.37, d, 2H; 7.25, d, 2H; 7.18, d, NH; 7.09, d, 1H; 4.25-4.14, m, 3H; 2.82, d, 2H; 1.29, t, 3H; 1.14, d, 3H |
| 6-8 | 2,4-dichlor-phenyl | CH(CH3) | CH2 | - | H | |
| 6-9 | 4-chlor-phenyl | CH2 | CH(CH3) | - | H | |
| 6-10 | 2,4-dichlor-phenyl | CH2 | CH(CH3) | - | H | |
| 6-11 | 4-chlor-phenyl | C(CH3)2 | CH2 | - | H | |
| 6-12 | 2,4-dichlor-phenyl | C(CH3)2 | CH2 | - | H | |
| 6-13 | 2-chlor-phenyl | CH2 | CH2 | - | H | |
| 6-14 | 3,4-dichlor-phenyl | CH2 | CH2 | - | H | |
| 6-15 | 3,5-dichlor-phenyl | CH2 | CH2 | - | H | |
| 6-16 | 3-chlor-phenyl | CH2 | CH2 | - | H | |
| 6-17 | 2-fluor-phenyl | CH2 | CH2 | - | H | |
| 6-18 | 2,6-difluor-phenyl | CH2 | CH2 | - | H | |
| 6-19 | 2,6-dichlor-phenyl | CH2 | CH2 | - | H | |
| 6-20 | 3 -(trifluormethyl)-phenyl | CH2 | CH2 | - | H | CAS: 1022443-07-7 |
| 6-21 | 4-(trifluormethyl)-phenyl | CH2 | CH2 | - | H | |
| 6-22 | 2-methyl-phenyl | CH2 | CH2 | - | H | |
| 6-23 | 2,4,6- trimethyl-phenyl | CH2 | CH2 | - | H | |
| 6-24 | 3,4-bismethoxy-phenyl | CH2 | CH2 | - | H | |
| 6-25 | phenyl | CH2 | CH2 | - | H | |
| 6-26 | 4-chlor-phenyl | C(CH2-CH2) | CH2 | - | H | |
| 6-27 | 2,4-dichlor-phenyl | C(CH2-CH2) | CH2 | - | H | |
| 6-28 | 4-chlor-phenyl | CH2 | C(CH2-CH2) | - | H | |
| 6-29 | 2,4-dichlor-phenyl | CH2 | C(CH2-CH2) | - | H | |
| 6-30 | 4-chlor-phenyl | O | CH2 | CH2 | H | |
| | | | | | | Verbindung Nr. 6-31, Solvent: [DMSO], Spektrometer: 399.95MHz |
| 6-31 | 2,4-dichlor-phenyl | O | CH2 | CH2 | H | 7,7264 (5,52); 7,7126 (5,85); 7,6937 (1,09); 7,6796 (2,14); 7,6653 (1,13); 7,5916 (5,02); 7,5851 (5,41); 7,3872 (2,53); 7,3807 (2,41); 7,365 (3,43); 7,3586 (3,35); 7,2439 (5,76); 7,2216 (4,3); 7,1218 (5,95); 7,108 (5,75); 4,2743 (2,4); 4,2568 (7,71); 4,2393 (7,81); 4,2218 (2,54); 4,1994 (3,23); 4,1857 (6,99); 4,1721 (3,66); 3,7205 (1,81); 3,7067 (4,99); 3,6927 (4,87); 3,6788 (1,67); 3,3285 (79,46); 2,5424 (7,29); 2,5254 (0,83); 2,5117 (17,49); 2,5076 (34,69); 2,5031 (46,14); 2,4987 (35,2); 1,3303 (7,85); 1,3128 (16); 1,2954 (7,69); -0,0002 (1,17) |
| 6-32 | 4-chlor-phenyl | CH2 | CH2 | CH2 | H | |
| 6-33 | 4-chlor-phenyl | NCH3 | CH2 | CH2 | H | |
| 6-34 | 2,4-dichlor-phenyl | NCH3 | CH2 | CH2 | H | |
| 6-35 | 4-chlor-phenyl | CH(OCH3) | CH2 | - | H | |
| 6-36 | 2,4-dichlor-phenyl | CH(OCH3) | CH2 | - | H | |
| | | | | | | Verbindung Nr. 6-37, Solvent: [DMSO], Spektrometer: 399.95MHz |
| 6-37 | 2-thienyl | CH2 | CH2 | - | H | 7,6972 (5,59); 7,6834 (5,88); 7,5202 (1,04); 7,5062 (1,95); 7,4922 (1,09); 7,3773 (3,09); 7,3745 (3,05); 7,3646 (3,39); 7,3617 (3,18); 7,0945 (6,15); 7,0806 (5,93); 6,9891 (2,46); 6,9805 (3,59); 6,9765 (2,5); 6,9678 (3,37); 6,9381 (3,6); 6,9305 (2,69); 4,2217 (2,47); 4,2041 (7,85); 4,1866 (7,97); 4,1692 (2,6); 3,5802 (1,96); 3,5634 (5,28); 3,5481 (5,52); 3,5315 (2,23); 3,3282 (54,19); 3,0541 (3,58); 3,0371 (7); 3,0201 (3,22); 2,5064 (28,37); 2,502 (37,77); 2,4976 (28,92); 1,2767 (7,89); 1,2592 (16); 1,2417 (7,77); - 0,0002 (0,57) |
| 6-38 | 3-thienyl | CH2 | CH2 | - | H | |
| 6-39 | 2-furyl | CH2 | CH2 | - | H | |
| 6-40 | 3-furyl | CH2 | CH2 | - | H | |
| 6-41 | phenyl | CH2 | CH2 | CH(CH3) | H | |
| 6-42 | phenyl | CH2 | CH2 | CH2 | H | |
| 6-43 | 2-Cl-phenyl | CH2 | CH2 | CH(CH3) | H | |
| 6-44 | 4-t-butyl-phenyl | CH2 | CH2 | CH2 | H | |
| 6-45 | 4-methyl-phenyl | CH2 | CH2 | CH2 | H | |
| 6-46 | phenyl | CH2 | CH2 | CH(CH2CH3) | H | |
| 6-47 | 2-methoxy-phenyl | CH2 | CH2 | CH2 | H | |
| 6-48 | 2-methyl-phenyl | CH2 | CH2 | CH2 | H | |
| 6-49 | 3-methyl-phenyl | CH2 | CH2 | CH2 | H | |
| 6-50 | 3-chlor-phenyl | CH2 | CH2 | CH2 | H | |
| | | | | | | Verbindung Nr. 6-51, Solvent: [DMSO], Spektrometer: 399.95MHz |
| 6-51 | 2,6-difluor-phenyl | CH2 | CH2 | CH2 | H | 7,6975 (5,44); 7,6837 (5,71); 7,5395 (1,16); 7,5254 (2,18); 7,5113 (1,2); 7,3505 (0,57); 7,3333 (1,35); 7,33 (1,3); 7,3127 (2,54); 7,2921 (1,67); 7,2751 (0,75); 7,1166 (6,24); 7,1028 (6,27); 7,091 (3,65); 7,0712 (5,73); 7,0514 (2,99); 7,038 (0,41); 4,2897 (2,53); 4,2722 (7,97); 4,2547 (8,09); 4,2373 (2,63); 3,332 (25,51); 3,3187 (2,12); 3,302 (4,8); 3,2862 (4,85); 3,2697 (1,88); 2,6936 (2,46); 2,6748 (4,71); 2,656 (2,72); 2,5441 (0,45); 2,509 (18,73); 2,5046 (24,99); 2,5003 (19,41); 1,7933 (0,94); 1,7757 (2,93); 1,7569 (3,9); 1,7387 (2,84); 1,7212 (0,84); 1,3934 (7,91); 1,3759 (16); 1,3664 (1,16); 1,3584 (7,85); -0,0002 (0,45) |
| 6-52 | 4-chlor-phenyl | CH2 | CH2 | CH2 | H | |
| 6-53 | 2,6-dichlor-phenyl | CH2 | CH2 | CH(CH3) | H | |
| 6-54 | 3,5-dichlor-phenyl | CH2 | CH2 | CH(CH3) | H | |
| 6-55 | 2,6-dimethyl-phenyl | CH2 | CH2 | CH2 | H | |
| 6-56 | 2,5-dichlor-phenyl | CH2 | CH2 | CH2 | H | |
| 6-57 | 4-i-propoxy-phenyl | CH2 | CH2 | CH2 | H | |
| 6-58 | 3-trifluormethyl-phenyl | CH2 | CH2 | CH(CH3) | H | |
| 6-59 | 4-methyl-phenyl | CH2 | CH2 | CH(CH3) | H | |
| 6-60 | 2-methyl-phenyl | CH2 | CH2 | CH(CH3) | H | |
| 6-61 | 3,4-dichlor-phenyl | CH2 | CH2 | CH(CH3) | H | |
| 6-62 | 3,5-dichlor-phenyl | CH2 | CH2 | CH2 | H | |
| 6-63 | 2,6-dimethyl-phenyl | CH2 | CH2 | CH(CH3) | H | |
| 6-64 | 4-trifluormethyl-phenyl | CH2 | CH2 | CH(CH3) | H | |
| 6-65 | 2,5-dichlor-phenyl | CH2 | CH2 | CH(CH3) | H | |
| 6-66 | 4-phenoxy-phenyl | CH2 | CH2 | CH2 | H | |
| 6-67 | 3-chlor-phenyl | CH2 | CH2 | CH(CH3) | H | |
| 6-68 | 4-phenoxy-phenyl | CH2 | CH2 | CH(CH3) | H | |
| 6-69 | 2,4-dichlor-phenyl | CH2 | CH2 | CH(CH3) | H | |
| 6-70 | 2-difluormethoxy-phenyl | CH2 | CH2 | CH(CH3) | H | |
| 6-71 | 4-methoxy-phenyl | CH2 | CH2 | CH2 | H | |
| 6-72 | 4-chlor-phenyl | CH2 | CH2 | CH(CH3) | H | |
| 6-73 | 4-chlor-phenyl | CH2 | CH2 | CH(i-propyl) | H | |
| 6-74 | 4-fluor-phenyl | CH2 | CH2 | CH2 | H | |
| 6-75 | 4-chlor-phenyl | CH2 | CH2 | CH(n-propyl) | H | |
| 6-76 | 4-chlor-phenyl | CH2 | CH2 | CH(t-butyl) | H | |
| 6-77 | 2-chlor-phenyl | CH2 | CH2 | CH2 | H | |
| | | | | | | Verbindung Nr. 6-78, Solvent: [DMSO], Spektrometer: 399.95MHz |
| 6-78 | 2-thienyl | CH2 | CH2 | CH2 | H | 7,696 (5,55); 7,6822 (5,86); 7,4984 (1,13); 7,4843 (2,09); 7,4701 (1,17); 7,325 (3,18); 7,3232 (2,97); 7,3223 (3,01); 7,3122 (3,52); 7,3104 (3,17); 7,3095 (3,19); 7,1147 (6,22); 7,1008 (5,96); 6,9534 (2,59); 6,9448 (3,55); 6,9408 (2,68); 6,9322 (3,22); 6,8846 (3,65); 6,8775 (2,97); 4,2878 (2,52); 4,2703 (7,94); 4,2528 (8,07); 4,2354 (2,65); 3,347 (2,19); 3,3295 (31,18); 3,3146 (5,41); 3,2979 (2,23); 2,8611 (3,39); 2,842 (5,86); 2,8229 (3,7); 2,5246 (0,61); 2,5069 (19,67); 2,5026 (25,81); 2,4984 (19,91); 1,8865 (1,08); 1,8686 (3,25); 1,8498 (4,32); 1,8316 (3,17); 1,8137 (1); 1,3951 (7,95); 1,3776 (16); 1,3601 (7,86); -0,0002 (0,45) |

**Tabelle 7**

| | | | | | | |
|---|---|---|---|---|---|---|
| Verbindungen der Formel 1-7 | | | | | | |
| 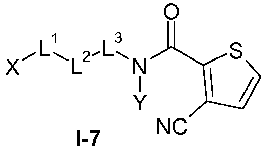 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 7-1 | 3-methyl-2-thienyl | CH2 | CH2 | - | H | |
| 7-2 | 2,4-dichlor-phenyl | CH2 | CH2 | CH2 | H | |
| 7-3 | 4-chlor-phenyl | CH2 | CH2 | - | H | [DMSO], Spektrometer: 399,95MHz |
| | | | | | | 8,7637 (1,66); 8,7502 (3,17); 8,7364 (1,7); 7,9252 (10,03); 7,9123 (10,75); 7,5535 (10,65); 7,5407 (10,07); 7,3609 (8,8); 7,3562 (3,53); 7,3448 (4,38); 7,3398 (16); 7,334 (2,76); 7,2856 (13,93); 7,2645 (8,1); 3,4844 (2,63); 3,4672 (5,67); 3,4522 (5,54); 3,4342 (2,92); 3,3357 (35,3); 2,8535 (5,02); 2,8353 (8,73); 2,8174 (4,53); 2,6767 (0,4); 2,6723 (0,55); 2,6679 (0,42); 2,5256 (1,69); 2,5119 (27,98); 2,5077 (55,22); 2,5032 (72,9); 2,4987 (54,91); 2,4945 (28,18); 2,3347 (0,34); 2,33 (0,48); 2,3255 (0,35); 0,0079 (0,49); -0,0002 (13,72); -0,0084 (0,58) |
| 7-4 | 2,4-dichlor-phenyl | CH2 | CH2 | - | H | [DMSO], Spektrometer: 399,95MHz |
| | | | | | | 8,791 (1,29); 8,7773 (2,55); 8,7634 (1,36); 7,9238 (7,38); 7,911 (7,94); 7,5902 (5,62); 7,5516 (7,8); 7,5388 (7,38); 7,392 (0,43); 7,3731 (16); 7,3526 (0,37); 7,3486 (0,5); 5,7585 (0,68); 3,5117 (1,9); 3,4947 (4,75); 3,4796 (4,87); 3,4626 (2,18); 3,4101 (0,4); 3,3554 (231,44); 2,9775 (3,9); 2,96 (7,5); 2,9426 (3,52); 2,6739 (0,39); 2,5272 (1,22); 2,5135 (21,16); 2,5094 (42,13); 2,5049 (55,83); 2,5004 (42,57); 2,4962 (22,27); 2,3316 (0,38); 1,3362 (0,33); 1,2493 (0,41); 0,0079 (0,4); -0,0002 (10,88); - 0,0084 (0,49) |
| 7-5 | 4-chlor-phenyl | CH(OCH3) | CH(CH3) | - | H | |
| 7-6 | 2,4-dichlor-phenyl | CH(OCH3) | CH(CH3) | - | H | |
| 7-7 | 4-chlor-phenyl | CH(CH3) | CH2 | - | H | |
| 7-8 | 2,4-dichlor-phenyl | CH(CH3) | CH2 | - | H | |
| 7-9 | 4-chlor-phenyl | CH2 | CH(CH3) | - | H | |
| 7-10 | 2,4-dichlor-phenyl | CH2 | CH(CH3) | - | H | |
| 7-11 | 4-chlor-phenyl | C(CH3)2 | CH2 | - | H | |
| 7-12 | 2,4-dichlor-phenyl | C(CH3)2 | CH2 | - | H | |
| 7-13 | 2-chlor-phenyl | CH2 | CH2 | - | H | |
| 7-14 | 3,4-dichlor-phenyl | CH2 | CH2 | - | H | |
| 7-15 | 3,5-dichlor-phenyl | CH2 | CH2 | - | H | |
| 7-16 | 3-chlor-phenyl | CH2 | CH2 | - | H | |
| 7-17 | 2-fluor-phenyl | CH2 | CH2 | - | H | |
| 7-18 | 2,6-difluor-phenyl | CH2 | CH2 | - | H | [DMSO], Spektrometer: 399,95MHz |
| | | | | | | 8,8536 (2,47); 8,8394 (4,77); 8,8253 (2,54); 7,9229 (14,9); 7,9101 (15,96); 7,552 (16); 7,5391 (15,07); 7,3681 (1,28); 7,3512 (2,93); 7,3473 (2,78); 7,3303 (5,68); 7,3131 (2,98); 7,3096 (3,65); 7,2927 (1,65); 7,1043 (0,78); 7,0998 (1,07); 7,0876 (8,02); 7,0679 (12,59); 7,057 (1,95); 7,048 (6,83); 7,0356 (1,02); 5,7606 (0,46); 3,4601 (3,72); 3,4432 (9,39); 3,4273 (9,56); 3,4105 (4,19); 3,3369 (50,68); 2,9207 (5,93); 2,9033 (11,25); 2,8858 (5,46); 2,6772 (0,44); 2,6729 (0,61); 2,6685 (0,45); 2,5261 (2,15); 2,5125 (34,74); 2,5083 (68,66); 2,5038 (90,7); 2,4993 (68,58); 2,495 (35,71); 2,3349 (0,43); 2,3305 (0,61); 2,326 (0,45); 1,3363 (0,51); 1,2496 |
| 7-19 | 2,6-dichlor-phenyl | CH2 | CH2 | - | H | [DMSO], Spektrometer: 399,95MHz |
| | | | | | | 8,8706 (1,58); 8,8566 (3,12); 8,8422 (1,63); 7,923 (9,28); 7,9102 (9,9); 7,5517 (10,16); 7,5388 (9,57); 7,4689 (11,6); 7,4489 (16); 7,4285 (0,32); 7,3095 (5,44); 7,2903 (5,71); 7,2884 (5,66); 7,2803 (0,56); 7,2692 (3,63); 7,2618 (0,38); 5,7598 (1,47); 3,5105 (2,09); 3,4939 (5,53); 3,4771 (5,61); 3,4609 (2,57); 3,3736 (0,52); 3,3434 (148,2); 3,189 (4,65); 3,1712 (8,14); 3,154 (3,8); 2,9768 (0,44); 2,6733 (0,43); 2,6688 (0,33); 2,5086 (47,99); 2,5042 (63,51); 2,4997 (48,43); 2,4956 (25,51); 2,331 (0,42); 1,2494 (0,37); -0,0002 (8,94); -0,0084 (0,43) |
| 7-20 | 3-(trifluormethyl)-phenyl | CH2 | CH2 | - | H | |
| 7-21 | 4-(trifluormethyl)-phenyl | CH2 | CH2 | - | H | |
| 7-22 | 2-methyl-phenyl | CH2 | CH2 | - | H | |
| 7-23 | 2,4,6-trimethyl-phenyl | CH2 | CH2 | - | H | |
| 7-24 | 3,4-bismethoxy-phenyl | CH2 | CH2 | - | H | |
| 7-25 | phenyl | CH2 | CH2 | - | H | |
| 7-26 | 4-chlor-phenyl | C(CH2-CH2) | CH2 | - | H | |
| 7-27 | 2,4-dichlor-phenyl | C(CH2-CH2) | CH2 | - | H | |
| 7-28 | 4-chlor-phenyl | CH2 | C(CH2-CH2) | - | H | |
| 7-29 | 2,4-dichlor-phenyl | CH2 | C(CH2-CH2) | - | H | |
| 7-30 | 4-chlor-phenyl | O | CH2 | CH2 | H | |
| 7-31 | 2,4-dichlor-phenyl | O | CH2 | CH2 | H | [DMSO], Spektrometer: 399,95MHz |
| | | | | | | 8,8522 (1,07); 8,8389 (2,09); 8,8258 (1,1); 8,1029 (1,26); 8,0993 (1,04); 8,0903 (1,05); 8,0868 (1,31); 7,9491 (6,61); 7,9363 (7,1); 7,919 (0,55); 7,6307 (0,37); 7,5719 (14,11); 7,5656 (7,94); 7,5591 (7,26); 7,5441 (0,38); 7,5377 (0,35); 7,5041 (0,57); 7,4912 (0,49); 7,3874 (2,9); 7,3809 (2,79); 7,3773 (1,56); 7,3705 (1,38); 7,3652 (4,09); 7,3587 (4,04); 7,3551 (2,09); 7,3485 (1,63); 7,3406 (0,34); 7,3351 (0,33); 7,2462 (6,01); 7,2239 (4,49); 7,2093 (0,34); 7,1932 (0,47); 7,1872 (2,26); 7,1649 (1,81); 6,5864 (1,34); 6,5827 (1,08); 6,5777 (0,8); 6,574 (1,09); 6,5702 (1,33); 5,7607 (1,41); 4,3737 (0,33); 4,2346 (3,15); 4,2201 (7,07); 4,2057 (3,51); 4,1882 (0,39); 4,1142 (0,42); 4,1045 (0,37); 4,0894 (0,39); 4,0184 (1,55); 4,0041 (3,2); 3,9898 (1,62); 3,7464 (0,48); 3,6652 (1,75); 3,6511 (4,89); 3,637 (4,76); 3,6227 (1,68); 3,5065 (0,59); 3,4891 (0,33); 3,3356 (11,99); 3,0722 (0,34); 3,0672 (0,41); 3,0541 (0,37); 2,9425 (16); 2,9058 (1,58); 2,8915 (3,16); 2,8771 (1,58); 2,677 (0,39); 2,6725 (0,55); 2,668 (0,4); 2,5259 (1,55); 2,5122 (30,72); 2,5079 (61,96); 2,5034 (82,32); 2,4989 (62,24); 2,4946 (32,17); 2,4309 (0,33); 2,3481 (0,55); 2,3303 (0,78); 2,3257 (0,52); 2,1891 (0,75); 2,1818 (3,87); 2,1725 (0,55); 2,0965 (0,49); 2,0722 (3,69); 2,0592 (1,1); 1,8245 (0,48); 1,7375 (2,42); 1,6581 (0,35); 1,325 (0,48); 1,2271 (6,23); 1,1838 (0,45); 1,1659 (0,92); 1,148 (0,44); 1,0533 (0,61); 1,0352 (1,28); 1,0171 (0,58); 0,9532 (1,71); 0,9368 (1,74); 0,0079 (0,58); -0,0002 (17,26); -0,0085 (0,78) |
| 7-32 | 4-chlor-phenyl | CH2 | CH2 | CH2 | H | |
| 7-33 | 4-chlor-phenyl | NCH3 | CH2 | CH2 | H | |
| 7-34 | 2,4-dichlor-phenyl | NCH3 | CH2 | CH2 | H | |
| 7-35 | 4-chlor-phenyl | CH(OCH3) | CH2 | - | H | |
| 7-36 | 2,4-dichlor-phenyl | CH(OCH3) | CH2 | - | H | |
| 7-37 | 2-thienyl | CH2 | CH2 | - | H | Verbindung Nr. 7-37, Solvent: [DMSO], Spektrometer: 399.95MHz |
| | | | | | | 8,839 (2,59); 8,8256 (4,79); 8,8124 (2,64); 8,3189 (0,4); 7,9395 (14,92); 7,9266 (16); 7,5661 (15,86); 7,5532 (14,91); 7,3585 (7,66); 7,3555 (7,79); 7,3459 (8,58); 7,3429 (8,24); 6,9743 (6,13); 6,9657 (9,59); 6,9617 (6,17); 6,9531 (9,46); 6,9321 (9,27); 6,9259 (6,25); 3,5065 (4,36); 3,4888 (10,03); 3,4742 (10,22); 3,4564 (5,21); 3,3375 (161,8); 3,0819 (8,6); 3,064 (15,33); 3,046 (7,4); 2,6765 (0,71); 2,672 (0,95); 2,6676 (0,73); 2,5252 (3,11); 2,5074 (102,9); 2,503 (135,05); 2,4985 (102,17); 2,334 (0,66); 2,3298 (0,9); 2,3252 (0,67); 1,336 (0,54); 1,2493 (0,67); 0,0079 (0,65); -0,0002 (17,56); -0,0083 (0,81) |
| 7-38 | 3-thienyl | CH2 | CH2 | - | H | |
| 7-39 | 2-furyl | CH2 | CH2 | - | H | |
| 7-40 | 3-furyl | CH2 | CH2 | - | H | |
| 7-41 | phenyl | CH2 | CH2 | CH(CH3) | H | |
| 7-42 | phenyl | CH2 | CH2 | CH2 | H | |
| 7-43 | 2-Cl-phenyl | CH2 | CH2 | CH(CH3) | H | |
| 7-44 | 4-t-butyl-phenyl | CH2 | CH2 | CH2 | H | |
| 7-45 | 4-methyl-phenyl | CH2 | CH2 | CH2 | H | |
| 7-46 | phenyl | CH2 | CH2 | CH(CH2CH3) | H | |
| 7-47 | 2-methoxy-phenyl | CH2 | CH2 | CH2 | H | |
| 7-48 | 2-methyl-phenyl | CH2 | CH2 | CH2 | H | |
| 7-49 | 3-methyl-phenyl | CH2 | CH2 | CH2 | H | |
| 7-50 | 3-chlor-phenyl | CH2 | CH2 | CH2 | H | |
| 7-51 | 2,6-difluor-phenyl | CH2 | CH2 | CH2 | H | [DMSO], Spektrometer: 399,95MHz |
| | | | | | | 8,7549 (2,05); 8,7414 (3,86); 8,7278 (2,09); 8,3188 (0,37); 7,9666 (0,33); 7,9538 (0,41); 7,9341 (14,91); 7,9269 (0,85); 7,9212 (15,91); 7,5855 (0,37); 7,5726 (0,39); 7,5573 (16); 7,5444 (15,04); 7,3519 (1,2); 7,335 (2,69); 7,3311 (2,41); 7,3141 (5,04); 7,3104 (2,27); 7,2967 (2,47); 7,2933 (3,34); 7,2765 (1,51); 7,105 (0,76); 7,1006 (0,94); 7,088 (6,68); 7,0679 (10,99); 7,0559 (1,48); 7,0478 (5,67); 7,0347 (0,81); 6,5468 (0,52); 3,3468 (249,02); 3,2962 (3,34); 3,2789 (6,79); 3,2634 (6,76); 3,246 (3,44); 2,7069 (4,52); 2,6878 (7,65); 2,6688 (5,31); 2,5441 (0,41); 2,5273 (1,94); 2,5225 (2,98); 2,5139 (33,76); 2,5094 (68,38); 2,5049 (90,84); 2,5003 (67,84); 2,4959 (34,13); 2,3361 (0,43); 2,3317 (0,61); 2,3271 (0,44); 1,8234 (1,73); 1,8044 (4,61); 1,786 (6,51); 1,7672 (4,31); 1,7488 (1,53); 1,3367 (0,51); 1,2498 (0,63); 0,008 (0,5); -0,0002 (15,55); -0,0085 (0,6) |
| 7-52 | 4-chlor-phenyl | CH2 | CH2 | CH2 | H | [DMSO], Spektrometer: 399,95MHz |
| | | | | | | 8,7403 (1,6); 8,7272 (2,97); 8,7136 (1,63); 7,9373 (9,86); 7,9244 (10,54); 7,5592 (10,41); 7,5463 (9,86); 7,3552 (1,07); 7,3492 (8,86); 7,3444 (3,56); 7,3331 (4,47); 7,3281 (16); 7,3223 (2,8); 7,309 (0,44); 7,273 (14); 7,2519 (8,27); 7,2312 (0,34); 3,345 (103,91); 3,2734 (2,65); 3,2562 (6,23); 3,2416 (6,24); 3,2245 (2,8); 2,678 (0,38); 2,6736 (0,52); 2,6593 (4,6); 2,6406 (7,03); 2,621 (5,08); 2,5271 (1,2); 2,5135 (20,13); 2,5092 (40,08); 2,5047 (52,88); 2,5002 (39,87); 2,4959 (20,52); 2,3314 (0,35); 1,8483 (1,39); 1,8303 (4); 1,8114 (5,31); 1,793 (3,88); 1,7751 (1,25); 1,337 (0,39); 1,2494 (0,47); -0,0002 (7,86); -0,0084 (0,33) |
| 7-53 | 2,6-dichlor-phenyl | CH2 | CH2 | CH(CH3) | H | |
| 7-54 | 3,5-dichlor-phenyl | CH2 | CH2 | CH(CH3) | H | |
| 7-55 | 2,6-dimethyl-phenyl | CH2 | CH2 | CH2 | H | |
| 7-56 | 2,5-dichlor-phenyl | CH2 | CH2 | CH2 | H | |
| 7-57 | 4-i-propoxy-phenyl | CH2 | CH2 | CH2 | H | |
| 7-58 | 3-trifluormethyl-phenyl | CH2 | CH2 | CH(CH3) | H | |
| 7-59 | 4-methyl-phenyl | CH2 | CH2 | CH(CH3) | H | |
| 7-60 | 2-methyl-phenyl | CH2 | CH2 | CH(CH3) | H | |
| 7-61 | 3,4-dichlor-phenyl | CH2 | CH2 | CH(CH3) | H | |
| 7-62 | 3,5-dichlor-phenyl | CH2 | CH2 | CH2 | H | |
| 7-63 | 2,6-dimethyl-phenyl | CH2 | CH2 | CH(CH3) | H | |
| 7-64 | 4-trifluormethyl-phenyl | CH2 | CH2 | CH(CH3) | H | |
| 7-65 | 2,5-dichlor-phenyl | CH2 | CH2 | CH(CH3) | H | |
| 7-66 | 4-phenoxy-phenyl | CH2 | CH2 | CH2 | H | |
| 7-67 | 3-chlor-phenyl | CH2 | CH2 | CH(CH3) | H | |
| 7-68 | 4-phenoxy-phenyl | CH2 | CH2 | CH(CH3) | H | |
| 7-69 | 2,4-dichlor-phenyl | CH2 | CH2 | CH(CH3) | H | |
| 7-70 | 2-difluormethoxy-phenyl | CH2 | CH2 | CH(CH3) | H | |
| 7-71 | 4-methoxy-phenyl | CH2 | CH2 | CH2 | H | |
| 7-72 | 4-chlor-phenyl | CH2 | CH2 | CH(CH3) | H | |
| 7-73 | 4-chlor-phenyl | CH2 | CH2 | CH(i-propyl) | H | |
| 7-74 | 4-fluor-phenyl | CH2 | CH2 | CH2 | H | |
| 7-75 | 4-chlor-phenyl | CH2 | CH2 | CH(n-propyl) | H | |
| 7-76 | 4-chlor-phenyl | CH2 | CH2 | CH(t-butyl) | H | |
| 7-77 | 2-chlor-phenyl | CH2 | CH2 | CH2 | H | |
| 7-78 | 4-chlor-phenyl | N(CH3) | CH2 | CH2 | H | Verbindung Nr. 7-78, Solvent: [DMSO], Spektrometer: 399.95MHz |
| | | | | | | 8,7475 (0,68); 8,7339 (1,34); 8,7201 (0,71); 7,9284 (3,31); 7,9155 (3,52); 7,554 (3,57); 7,5412 (3,35); 7,1828 (0,44); 7,1742 (4,37); 7,1691 (1,48); 7,1516 (4,8); 7,1428 (0,53); 6,7631 (0,63); 6,7544 (4,51); 6,7318 (4,02); 6,7229 (0,47); 3,5155 (1,15); 3,4986 (2,94); 3,4826 (2,24); 3,4183 (1,22); 3,4033 (2,51); 3,3878 (2,05); 3,3716 (0,74); 3,3307 (60,01); 2,9199 (16); 2,8746 (0,79); 2,542 (19); 2,507 (25,15); 2,5027 (31,73); 2,4983 (23,27); 2,3743 (0,39); -0,0002 (3,58) |

**Tabelle 8**

| | | | | | | |
|---|---|---|---|---|---|---|
| Verbindungen der Formel 1-8 | | | | | | |
| 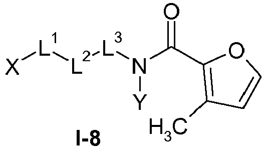 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 8-1 | 3-methyl-2-thienyl | CH2 | CH2 | - | H | |
| 8-2 | 2,4-dichlor-phenyl | CH2 | CH2 | CH2 | H | Verbindung Nr. 8-2, Solvent: [DMSO], Spektrometer: 399.95MHz 8,2244 (0,52); 8,2099 (1); 8,1955 (0,52); 7,6582 (2,82); 7,6541 (2,8); 7,5613 (3,05); 7,556 (3,13); 7,4276 (1,91); 7,4069 (3,86); 7,3725 (2,72); 7,3672 (2,5); 7,3518 (1,3); 7,3465 (1,28); 6,4852 (3); 6,4811 (2,98); 3,337 (46,88); 3,254 (0,97); 3,2368 (2,09); 3,2211 (2,08); 3,2039 (0,99); 2,7131 (1,76); 2,6946 (2,19); 2,6745 (1,94); 2,5436 (10,74); 2,5267 (0,35); 2,5219 (0,56); 2,5134 (6,94); 2,5089 (13,88); 2,5042 (18,41); 2,4996 (13,29); 2,4951 (6,22); 2,2699 (16); 1,8033 (0,5); 1,7844 (1,26); 1,7662 (1,7); 1,7479 (1,18); 1,7294 (0,44); -0,0002 (1,59) |
| 8-3 | 4-chlor-phenyl | CH2 | CH2 | - | H | CAS: 1330848-74-2 |
| 8-4 | 2,4-dichlor-phenyl | CH2 | CH2 | - | H | [DMSO], Spektrometer: 601.6MHz 8.2533 (0.55); 8.2437 (1.08); 8.2341 (0.56); 7.6534 (2.78); 7.6506 (2.81); 7.5762 (2.58); 7.5730 (2.69); 7.3713 (0.79); 7.3680 (0.66); 7.3575 (3.11); 7.3543 (3.31); 7.3480 (4.41); 7.3343 (1.03); 6.4800 (2.88); 6.4772 (2.88); 3.4549 (0.97); 3.4436 (1.99); 3.4330 (1.93); 3.4213 (1.06); 3.3283 (23.69); 2.9365 (1.93); 2.9244 (3.25); 2.9126 (1.79); 2.5434 (7.05); 2.5101 (3.42); 2.5071 (7.69); 2.5041 (10.83); 2.5010 (7.79); 2.4980 (3.55); 2.2571 (16.00); -0.0002 (8.63) |
| 8-5 | 4-chlor-phenyl | CH(OCH3) | CH(CH3) | - | H | |
| 8-6 | 2,4-dichlor-phenyl | CH(OCH3) | CH(CH3) | - | H | |
| 8-7 | 4-chlor-phenyl | CH(CH3) | CH2 | - | H | [DMSO], Spektrometer: 601.6MHz 8.1009 (0.58); 8.0913 (1.11); 8.0817 (0.58); 7.6309 (2.83); 7.6281 (2.77); 7.3559 (0.46); 7.3515 (3.81); 7.3483 (1.30); 7.3407 (1.56); 7.3374 (5.63); 7.3332 (0.69); 7.2773 (0.73); 7.2731 (5.14); 7.2699 (1.46); 7.2621 (1.26); 7.2591 (3.56); 7.2547 (0.39); 6.4671 (2.96); 6.4644 (2.85); 3.3811 (0.42); 3.3708 (0.53); 3.3689 (0.56); 3.3590 (1.16); 3.3489 (1.02); 3.3471 (1.10); 3.3367 (1.16); 3.3269 (67.15); 3.3167 (1.04); 3.3069 (0.97); 3.3042 (0.96); 3.2946 (1.27); 3.2851 (0.47); 3.2824 (0.52); 3.2728 (0.45); 3.0742 (0.56); 3.0622 (1.11); 3.0503 (1.09); 3.0384 (0.51); 2.5417 (12.80); 2.5233 (0.33); 2.5203 (0.40); 2.5171 (0.43); 2.5083 (7.97); 2.5054 (16.93); 2.5023 (23.20); 2.4993 (16.75); 2.4963 (7.73); 2.2443 (16.00); 1.1791 (7.28); 1.1675 (7.25); 0.0050 (0.39); -0.0002 (9.65); -0.0057 (0.32) |
| 8-8 | 2,4-dichlor-phenyl | CH(CH3) | CH2 | - | H | [DMSO], Spektrometer: 601.6MHz 8.1820 (0.66); 8.1723 (1.24); 8.1625 (0.64); 7.6318 (2.92); 7.6291 (2.79); 7.5490 (2.92); 7.5455 (2.93); 7.4479 (1.92); 7.4338 (3.89); 7.4107 (2.32); 7.4071 (2.09); 7.3967 (1.11); 7.3931 (1.08); 6.4670 (3.04); 6.4642 (2.92); 3.5460 (0.52); 3.5341 (1.13); 3.5223 (1.24); 3.5105 (0.69); 3.4201 (1.37); 3.4143 (1.36); 3.4096 (1.70); 3.4044 (1.53); 3.4019 (1.35); 3.3986 (1.21); 3.3919 (1.19); 3.3269 (69.14); 3.3091 (0.97); 2.5421 (9.32); 2.5236 (0.75); 2.5206 (0.93); 2.5175 (1.05); 2.5087 (9.41); 2.5058 (18.83); 2.5028 (25.35); 2.4997 (18.16); 2.4968 (8.55); 2.4842 (0.97); 2.2433 (16.00); 1.1680 (7.51); 1.1565 (7.49); 0.0050 (0.37); - 0.0002 (8.14); -0.0056 (0.33) |
| 8-9 | 4-chlor-phenyl | CH2 | CH(CH3) | - | H | [DMSO], Spektrometer: 601.6MHz 7.9941 (1.08); 7.9798 (1.10); 7.6414 (2.71); 7.6386 (2.78); 7.3234 (0.34); 7.3191 (3.63); 7.3160 (1.24); 7.3084 (1.40); 7.3052 (5.46); 7.3011 (0.68); 7.2467 (0.59); 7.2427 (4.72); 7.2396 (1.42); 7.2318 (1.12); 7.2287 (3.33); 7.2246 (0.39); 6.4645 (2.83); 6.4617 (2.85); 4.1700 (0.46); 4.1672 (0.56); 4.1562 (0.83); 4.1454 (0.58); 4.1426 (0.48); 3.3297 (111.93); 2.8835 (0.86); 2.8699 (0.86); 2.8610 (1.11); 2.8475 (1.08); 2.7225 (1.08); 2.7122 (1.11); 2.7001 (0.86); 2.6897 (0.83); 2.5418 (13.67); 2.5204 (0.33); 2.5085 (7.69); 2.5055 (17.49); 2.5024 (24.75); 2.4994 (17.97); 2.4964 (8.28); 2.2240 (16.00); 1.1286 (7.02); 1.1176 (7.03); - 0.0002 (9.01) |
| 8-10 | 2,4-dichlor-phenyl | CH2 | CH(CH3) | - | H | |
| 8-11 | 4-chlor-phenyl | C(CH3)2 | CH2 | - | H | [DMSO], Spektrometer: 601.6MHz 7.6613 (0.33); 7.6509 (0.67); 7.6399 (0.39); 7.6352 (1.85); 7.6324 (1.81); 7.4262 (2.18); 7.4228 (0.77); 7.4153 (0.95); 7.4117 (3.81); 7.4073 (0.46); 7.3731 (0.44); 7.3686 (3.79); 7.3651 (0.95); 7.3575 (0.77); 7.3542 (2.28); 6.4718 (1.81); 6.4690 (1.80); 3.4008 (2.66); 3.3900 (2.66); 3.3311 (73.50); 2.5420 (6.69); 2.5086 (5.02); 2.5056 (11.04); 2.5026 (15.37); 2.4996 (11.02); 2.4966 (5.02); 2.2348 (10.09); 1.2561 (16.00); -0.0002 (4.16) |
| 8-12 | 2,4-dichlor-phenyl | C(CH3)2 | CH2 | - | H | [DMSO], Spektrometer: 601.6MHz 7.7625 (0.35); 7.7518 (0.69); 7.7412 (0.39); 7.6249 (1.80); 7.6221 (1.78); 7.5364 (2.21); 7.5325 (2.32); 7.4594 (1.56); 7.4450 (2.10); 7.3650 (1.43); 7.3611 (1.36); 7.3507 (1.11); 7.3468 (1.08); 6.4625 (1.84); 6.4598 (1.83); 3.7328 (2.62); 3.7220 (2.61); 3.3285 (246.39); 3.3149 (0.46); 2.5413 (8.83); 2.5230 (0.53); 2.5199 (0.63); 2.5168 (0.57); 2.5080 (14.39); 2.5050 (32.45); 2.5019 (45.35); 2.4989 (32.42); 2.4958 (14.43); 2.2178 (10.38); 1.4075 (16.00); 0.0052 (0.51); -0.0002 (19.26); -0.0057 (0.57) |
| 8-13 | 2-chlor-phenyl | CH2 | CH2 | - | H | [DMSO], Spektrometer: 601.6MHz 8.2580 (0.48); 8.2487 (0.92); 8.2392 (0.49); 7.6540 (2.63); 7.6512 (2.61); 7.4295 (1.40); 7.4270 (1.30); 7.4168 (1.75); 7.4142 (1.69); 7.3410 (1.01); 7.3379 (1.18); 7.3287 (1.47); 7.3256 (1.67); 7.2908 (0.70); 7.2884 (0.81); 7.2786 (1.79); 7.2761 (1.72); 7.2665 (1.22); 7.2638 (1.05); 7.2605 (1.35); 7.2572 (1.36); 7.2478 (1.43); 7.2447 (1.42); 7.2354 (0.55); 7.2324 (0.49); 6.4816 (2.73); 6.4788 (2.73); 3.4608 (1.04); 3.4505 (1.70); 3.4389 (1.55); 3.4364 (1.82); 3.4264 (1.13); 3.3310 (181.76); 2.9524 (1.91); 2.9397 (2.58); 2.9278 (1.77); 2.5416 (15.14); 2.5233 (0.38); 2.5202 (0.47); 2.5171 (0.44); 2.5083 (10.29); 2.5053 (23.29); 2.5022 (32.98); 2.4992 (23.49); 2.4962 (10.64); 2.2634 (16.00); -0.0002 (4.03) |
| 8-14 | 3,4-dichlor-phenyl | CH2 | CH2 | - | H | [DMSO], Spektrometer: 601.6MHz 8.2047 (0.54); 8.1951 (1.03); 8.1858 (0.54); 7.6519 (2.75); 7.6491 (2.78); 7.5429 (3.21); 7.5292 (3.54); 7.5015 (2.66); 7.4981 (2.76); 7.2266 (1.51); 7.2232 (1.50); 7.2129 (1.39); 7.2096 (1.40); 6.4806 (2.86); 6.4778 (2.82); 3.4456 (1.01); 3.4340 (2.18); 3.4239 (2.16); 3.4122 (1.10); 3.3316 (204.25); 3.3131 (0.35); 2.8338 (1.79); 2.8219 (3.35); 2.8101 (1.68); 2.5418 (15.80); 2.5235 (0.42); 2.5204 (0.51); 2.5173 (0.47); 2.5084 (12.10); 2.5055 (27.47); 2.5024 (39.05); 2.4994 (28.35); 2.4964 (13.25); 2.2537 (16.00); -0.0002 (4.73) |
| 8-15 | 3,5-dichlor-phenyl | CH2 | CH2 | - | H | [DMSO], Spektrometer: 601.6MHz 8.2134 (0.53); 8.2041 (1.03); 8.1946 (0.53); 7.6572 (2.71); 7.6544 (2.70); 7.4295 (1.39); 7.4264 (2.74); 7.4232 (1.50); 7.2998 (5.88); 7.2966 (5.65); 6.4830 (2.85); 6.4802 (2.76); 3.4567 (0.97); 3.4451 (2.22); 3.4350 (2.23); 3.4234 (1.06); 3.3345 (121.94); 2.8476 (1.71); 2.8359 (3.32); 2.8242 (1.61); 2.5426 (11.75); 2.5093 (6.44); 2.5063 (14.44); 2.5032 (20.30); 2.5002 (14.66); 2.4972 (6.66); 2.2543 (16.00); -0.0002 (3.03) |
| 8-16 | 3-chlor-phenyl | CH2 | CH2 | - | H | CAS: 1043374-79-3 |
| 8-17 | 2-fluor-phenyl | CH2 | CH2 | - | H | CAS: 1043270-08-1 |
| 8-18 | 2,6-difluor-phenyl | CH2 | CH2 | - | H | [DMSO], Spektrometer: 601.6MHz 8.2999 (0.51); 8.2901 (0.98); 8.2805 (0.51); 7.6472 (2.70); 7.6444 (2.68); 7.3275 (0.64); 7.3248 (0.61); 7.3136 (1.21); 7.3024 (0.64); 7.2998 (0.72); 7.0656 (1.69); 7.0526 (2.62); 7.0449 (0.34); 7.0394 (1.51); 6.4765 (2.83); 6.4737 (2.81); 3.4012 (0.93); 3.3903 (1.99); 3.3777 (1.95); 3.3672 (1.02); 3.3395 (142.03); 2.8751 (1.33); 2.8630 (2.35); 2.8512 (1.24); 2.5430 (13.70); 2.5097 (6.17); 2.5067 (13.45); 2.5036 (18.62); 2.5006 (13.27); 2.4976 (5.94); 2.2531 (16.00); -0.0002 (0.79) |
| 8-19 | 2,6-dichlor-phenyl | CH2 | CH2 | - | H | [DMSO], Spektrometer: 601.6MHz 8.3392 (0.53); 8.3294 (1.05); 8.3197 (0.54); 7.6538 (2.65); 7.6511 (2.71); 7.4513 (5.52); 7.4379 (6.67); 7.2889 (2.12); 7.2756 (2.61); 7.2621 (1.59); 6.4793 (2.75); 6.4765 (2.82); 3.4316 (0.94); 3.4212 (1.85); 3.4172 (1.06); 3.4097 (1.51); 3.4071 (1.92); 3.3972 (1.09); 3.3352 (90.12); 3.3346 (94.30); 3.1452 (1.99); 3.1323 (2.48); 3.1207 (1.72); 2.5427 (10.35); 2.5094 (6.80); 2.5064 (15.36); 2.5034 (21.69); 2.5003 (15.57); 2.4973 (7.13); 2.2610 (16.00); -0.0002 (4.04) |
| 8-20 | 3 -(trifluormethyl)-phenyl | CH2 | CH2 | - | H | [DMSO], Spektrometer: 601.6MHz 8.2219 (0.57); 8.2125 (1.05); 8.2030 (0.56); 7.6499 (2.92); 7.6472 (2.84); 7.5688 (2.78); 7.5578 (0.96); 7.5546 (1.52); 7.5401 (2.57); 7.5376 (3.85); 7.5253 (1.05); 6.4792 (3.00); 6.4764 (2.89); 3.4759 (1.07); 3.4645 (2.14); 3.4542 (2.10); 3.4421 (1.20); 3.3360 (129.83); 2.9322 (1.87); 2.9200 (3.20); 2.9081 (1.78); 2.5428 (14.60); 2.5214 (0.32); 2.5094 (7.43); 2.5064 (16.06); 2.5034 (22.23); 2.5004 (16.13); 2.4974 (7.56); 2.2605 (0.69); 2.2520 (16.00); -0.0002 (3.79) |
| 8-21 | 4-(trifluormethyl)-phenyl | CH2 | CH2 | - | H | CAS: 1325832-12-9 |
| 8-22 | 2-methyl-phenyl | CH2 | CH2 | - | H | CAS: 1043269-68-6 |
| 8-23 | 2,4,6-trimethyl-phenyl | CH2 | CH2 | - | H | [DMSO], Spektrometer: 601.6MHz 8.3712 (0.61); 7.6759 (1.40); 7.6732 (1.40); 6.7975 (3.13); 6.4963 (1.46); 6.4936 (1.46); 3.3334 (97.86); 3.2078 (0.43); 3.1980 (0.70); 3.1944 (0.55); 3.1888 (0.67); 3.1839 (0.54); 3.1797 (0.71); 3.1701 (0.49); 2.7659 (0.85); 2.7570 (0.61); 2.7518 (0.83); 2.7476 (0.62); 2.7383 (0.77); 2.5415 (5.51); 2.5081 (5.27); 2.5052 (11.49); 2.5022 (15.98); 2.4991 (11.53); 2.4962 (5.33); 2.2934 (16.00); 2.1786 (5.69); -0.0002 (2.22) |
| 8-24 | 3,4-bismethoxy-phenyl | CH2 | CH2 | - | H | CAS: 1036695-62-1 |
| 8-25 | phenyl | CH2 | CH2 | - | H | CAS: 1060941-00-5 |
| 8-26 | 4-chlor-phenyl | C(CH2-CH2) | CH2 | - | H | |
| 8-27 | 2,4-dichlor-phenyl | C(CH2-CH2) | CH2 | - | H | |
| 8-28 | 4-chlor-phenyl | CH2 | C(CH2-CH2) | - | H | |
| 8-29 | 2,4-dichlor-phenyl | CH2 | C(CH2-CH2) | - | H | |
| 8-30 | 4-chlor-phenyl | O | CH2 | CH2 | H | CAS: 1325531-65-4 |
| 8-31 | 2,4-dichlor-phenyl | O | CH2 | CH2 | H | Verbindung Nr. 8-31, Solvent: [DMSO], Spektrometer: 399.95MHz 8,2551 (0,73); 8,2413 (1,4); 8,2273 (0,74); 7,6878 (3,16); 7,6842 (3,17); 7,5671 (2,97); 7,5607 (3,19); 7,3816 (1,54); 7,3752 (1,45); 7,3594 (2,09); 7,353 (2,01); 7,24 (3,54); 7,2177 (2,63); 6,5049 (3,33); 6,5013 (3,32); 4,1896 (2,12); 4,1741 (4,72); 4,1587 (2,35); 3,6071 (1,2); 3,5921 (3,32); 3,5772 (3,21); 3,562 (1,08); 3,334 (25,86); 2,5438 (13,28); 2,5267 (0,33); 2,5089 (10,22); 2,5046 (13,23); 2,5002 (10,08); 2,2826 (16); - 0,0002 (0,33) |
| 8-32 | 4-chlor-phenyl | CH2 | CH2 | CH2 | H | |
| 8-33 | 4-chlor-phenyl | NCH3 | CH2 | CH2 | H | |
| 8-34 | 2,4-dichlor-phenyl | NCH3 | CH2 | CH2 | H | |
| 8-35 | 4-chlor-phenyl | CH(OCH3) | CH2 | - | H | |
| 8-36 | 2,4-dichlor-phenyl | CH(OCH3) | CH2 | - | H | |
| 8-37 | 2-thienyl | CH2 | CH2 | - | H | CAS: 1015949-54-8 |
| 8-38 | 3-thienyl | CH2 | CH2 | - | H | |
| 8-39 | 2-furyl | CH2 | CH2 | - | H | CAS: 1016026-83-7 |
| 8-40 | 3-furyl | CH2 | CH2 | - | H | |
| 8-41 | phenyl | CH2 | CH2 | CH(CH3) | H | |
| 8-42 | phenyl | CH2 | CH2 | CH2 | H | |
| 8-43 | 2-Cl-phenyl | CH2 | CH2 | CH(CH3) | H | |
| 8-44 | 4-t-butyl-phenyl | CH2 | CH2 | CH2 | H | |
| 8-45 | 4-methyl-phenyl | CH2 | CH2 | CH2 | H | |
| 8-46 | phenyl | CH2 | CH2 | CH(CH2CH3) | H | |
| 8-47 | 2-methoxy-phenyl | CH2 | CH2 | CH2 | H | |
| 8-48 | 2-methyl-phenyl | CH2 | CH2 | CH2 | H | |
| 8-49 | 3 -methyl-phenyl | CH2 | CH2 | CH2 | H | |
| 8-50 | 3-chlor-phenyl | CH2 | CH2 | CH2 | H | |
| 8-51 | 2,6-difluor-phenyl | CH2 | CH2 | CH2 | H | Verbindung Nr. 8-51, Solvent: [DMSO], Spektrometer: 399.95MHz 8,2012 (0,65); 8,1868 (1,21); 8,173 (0,66); 7,6566 (3); 7,653 (2,98); 7,3419 (0,33); 7,3249 (0,79); 7,3215 (0,73); 7,3042 (1,45); 7,2835 (0,94); 7,2666 (0,42); 7,0797 (2,03); 7,0598 (3,28); 7,0399 (1,68); 6,4825 (3,22); 6,4789 (3,24); 3,3291 (43,27); 3,2425 (1,02); 3,2257 (2,16); 3,2081 (2,18); 3,1914 (1,07); 2,6559 (1,43); 2,6367 (2,4); 2,6173 (1,57); 2,5071 (24,4); 2,5027 (32,29); 2,4983 (24,78); 2,2874 (0,37); 2,2627 (16); 1,7727 (0,55); 1,7532 (1,42); 1,7349 (2,05); 1,7162 (1,33); 1,6975 (0,48); -0,0002 (0,34) |
| | | | | | | |
| 8-52 | 4-chlor-phenyl | CH2 | CH2 | CH2 | H | |
| 8-53 | 2,6-dichlor-phenyl | CH2 | CH2 | CH(CH3) | H | |
| 8-54 | 3,5-dichlor-phenyl | CH2 | CH2 | CH(CH3) | H | |
| 8-55 | 2,6-dimethyl-phenyl | CH2 | CH2 | CH2 | H | |
| 8-56 | 2,5-dichlor-phenyl | CH2 | CH2 | CH2 | H | |
| 8-57 | 4-i-propoxy-phenyl | CH2 | CH2 | CH2 | H | |
| 8-58 | 3-trifluormethyl-phenyl | CH2 | CH2 | CH(CH3) | H | |
| 8-59 | 4-methyl-phenyl | CH2 | CH2 | CH(CH3) | H | |
| 8-60 | 2-methyl-phenyl | CH2 | CH2 | CH(CH3) | H | |
| 8-61 | 3,4-dichlor-phenyl | CH2 | CH2 | CH(CH3) | H | |
| 8-62 | 3,5-dichlor-phenyl | CH2 | CH2 | CH2 | H | |
| 8-63 | 2,6-dimethyl-phenyl | CH2 | CH2 | CH(CH3) | H | |
| 8-64 | 4-trifluormethyl-phenyl | CH2 | CH2 | CH(CH3) | H | |
| 8-65 | 2,5-dichlor-phenyl | CH2 | CH2 | CH(CH3) | H | |
| 8-66 | 4-phenoxy-phenyl | CH2 | CH2 | CH2 | H | |
| 8-67 | 3-chlor-phenyl | CH2 | CH2 | CH(CH3) | H | |
| 8-68 | 4-phenoxy-phenyl | CH2 | CH2 | CH(CH3) | H | |
| 8-69 | 2,4-dichlor-phenyl | CH2 | CH2 | CH(CH3) | H | |
| 8-70 | 2-difluormethoxy-phenyl | CH2 | CH2 | CH(CH3) | H | |
| 8-71 | 4-methoxy-phenyl | CH2 | CH2 | CH2 | H | |
| 8-72 | 4-chlor-phenyl | CH2 | CH2 | CH(CH3) | H | |
| 8-73 | 4-chlor-phenyl | CH2 | CH2 | CH(i-propyl) | H | |
| 8-74 | 4-fluor-phenyl | CH2 | CH2 | CH2 | H | |
| 8-75 | 4-chlor-phenyl | CH2 | CH2 | CH(n-propyl) | H | |
| 8-76 | 4-chlor-phenyl | CH2 | CH2 | CH(t-butyl) | H | |
| 8-77 | 2-chlor-phenyl | CH2 | CH2 | CH2 | H | |
| 8-78 | 4-chlor-phenyl | CH2 | CH(CH3) | - | cyclopropyl | |
| | | | | | | Verbindung Nr. 8-79, Solvent: [DMSO], Spektrometer: 399.95MHz |
| 8-79 | 2-thienyl | CH2 | CH2 | CH2 | H | 8,221 (0,63); 8,2071 (1,15); 8,1929 (0,64); 7,6601 (2,99); 7,6565 (3,01); 7,3135 (1,85); 7,3107 (1,85); 7,3007 (2,05); 7,2979 (1,95); 6,9452 (1,53); 6,9367 (2,1); 6,9325 (1,56); 6,924 (1,94); 6,8841 (2,07); 6,8822 (2,07); 6,876 (1,67); 6,487 (3,15); 6,4834 (3,17); 3,3298 (24,93); 3,2682 (1,16); 3,2515 (2,62); 3,235 (2,63); 3,2183 (1,23); 2,8303 (1,94); 2,8111 (3,31); 2,792 (2,12); 2,5418 (1,02); 2,5245 (0,38); 2,5068 (13,21); 2,5024 (17,54); 2,498 (13,39); 2,2729 (16); 1,8665 (0,62); 1,848 (1,79); 1,8294 (2,39); 1,8113 (1,72); 1,7928 (0,57) |
| | | | | | | Verbindung Nr. 8-80, Solvent: [DMSO], Spektrometer: 601.6MHz |
| 8-80 | 4-chlor-2-thienyl | CH2 | CH2 | - | H | 8,2854 (0,55); 8,2761 (1,03); 8,2669 (0,54); 7,6694 (2,74); 7,6667 (2,72); 7,3628 (3,72); 7,3602 (3,83); 6,8983 (2,61); 6,8971 (2,5); 6,896 (2,67); 6,492 (2,8); 6,4894 (2,87); 3,4515 (1,07); 3,4397 (2,48); 3,4298 (2,5); 3,4181 (1,16); 3,3249 (297,13); 3,0042 (1,78); 2,9924 (3,36); 2,9811 (1,58); 2,6529 (0,53); 2,6162 (0,46); 2,6131 (0,62); 2,6101 (0,45); 2,5408 (159,37); 2,5224 (1,46); 2,5193 (1,94); 2,5162 (2,27); 2,5074 (35,62); 2,5044 (73,23); 2,5014 (100,26); 2,4983 (73,35); 2,4953 (34,47); 2,4244 (0,53); 2,3886 (0,45); 2,3856 (0,62); 2,3825 (0,45); 2,2685 (16); 2,0736 (0,38); -0,0002 (6,04) |
| | | | | | | Verbindung Nr. 8-81, Solvent: [DMSO], Spektrometer: 399.95MHz |
| 8-81 | 4-chlor-phenyl | N(CH3) | CH2 | CH2 | H | 8,2378 (0,68); 8,2238 (1,29); 8,2099 (0,68); 7,6642 (3,05); 7,6607 (2,89); 7,1804 (0,49); 7,1717 (4,21); 7,1665 (1,43); 7,149 (4,63); 7,1402 (0,51); 6,774 (0,59); 6,7656 (4,48); 6,7429 (3,98); 6,4866 (3,24); 6,4831 (3,01); 3,4418 (1,08); 3,4248 (2,45); 3,4071 (2,29); 3,3521 (1,68); 3,3299 (50,37); 3,3052 (0,87); 2,9004 (16); 2,5418 (11,39); 2,5068 (22,85); 2,5024 (28,29); 2,498 (20,57); 2,2758 (15,31); -0,0002 (3,19) |

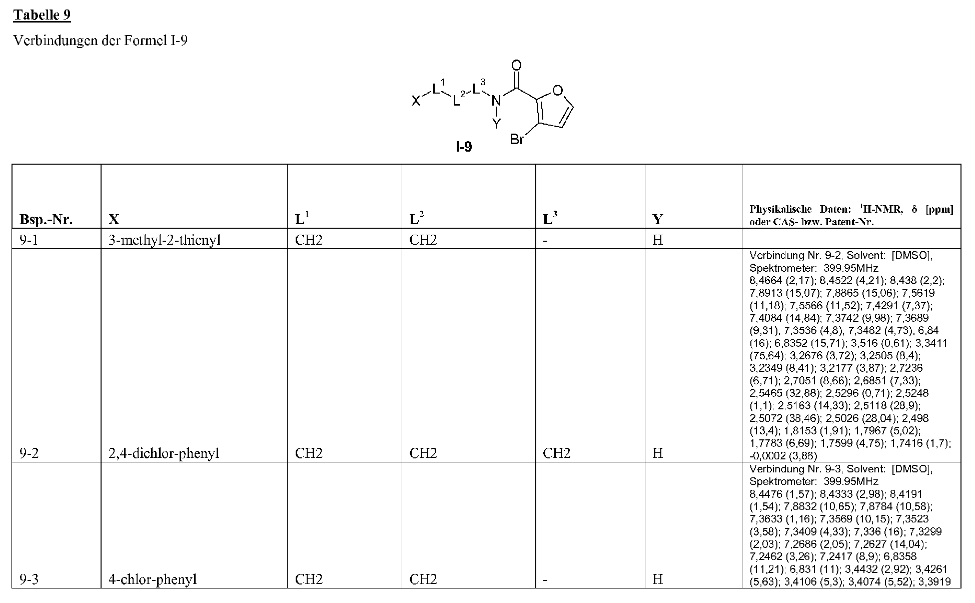

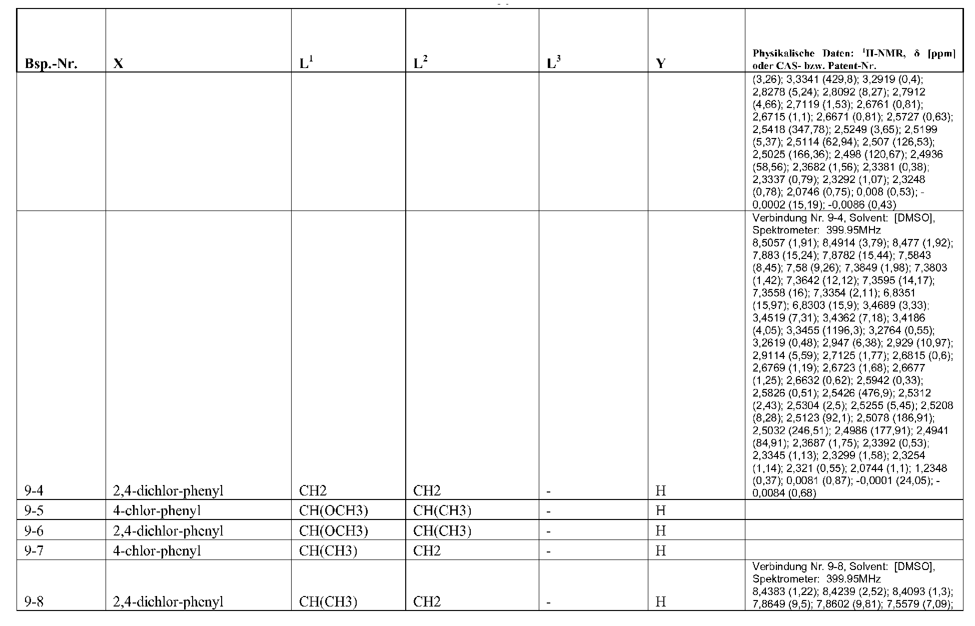

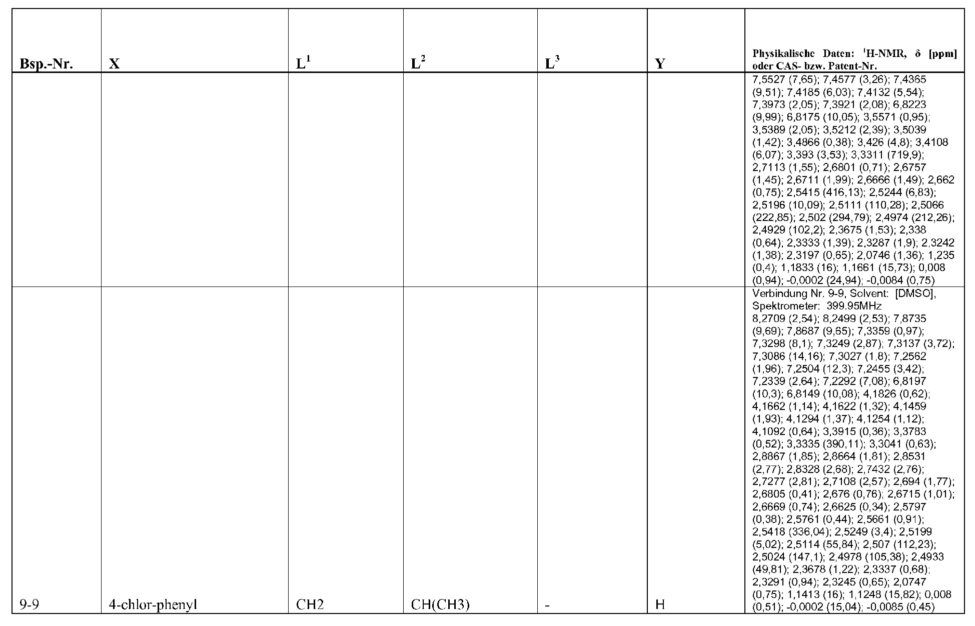

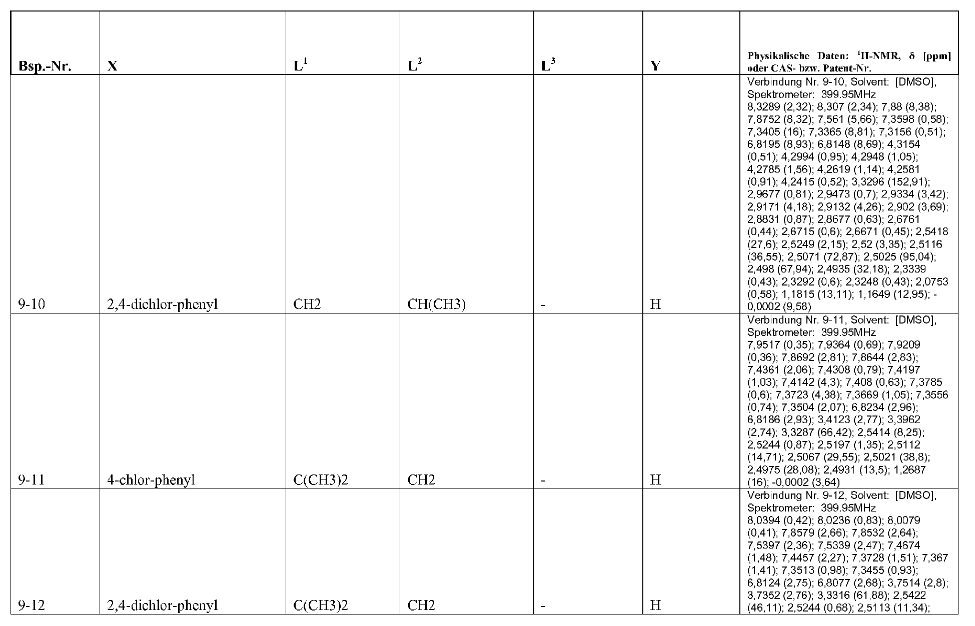

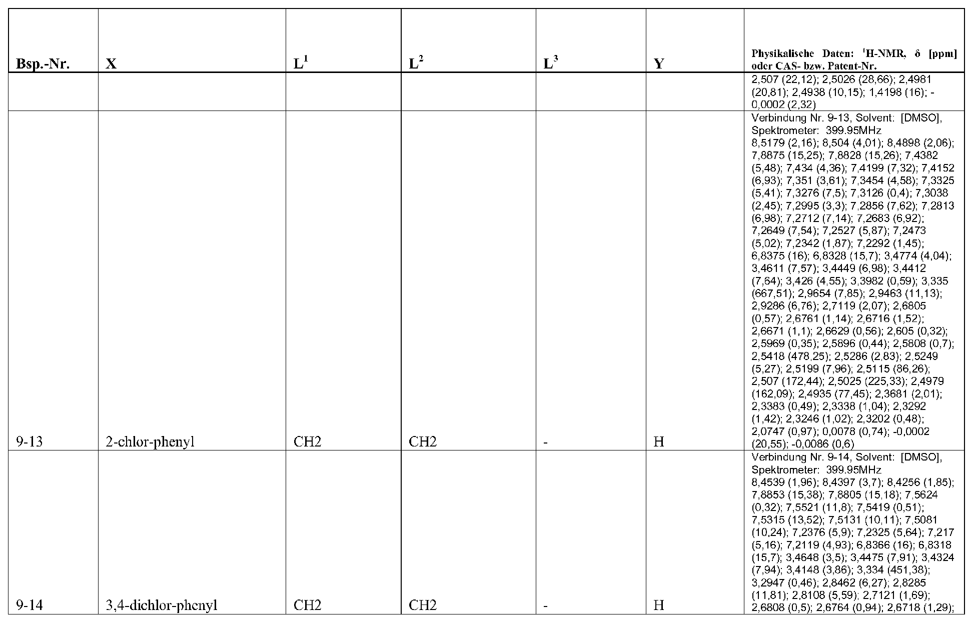

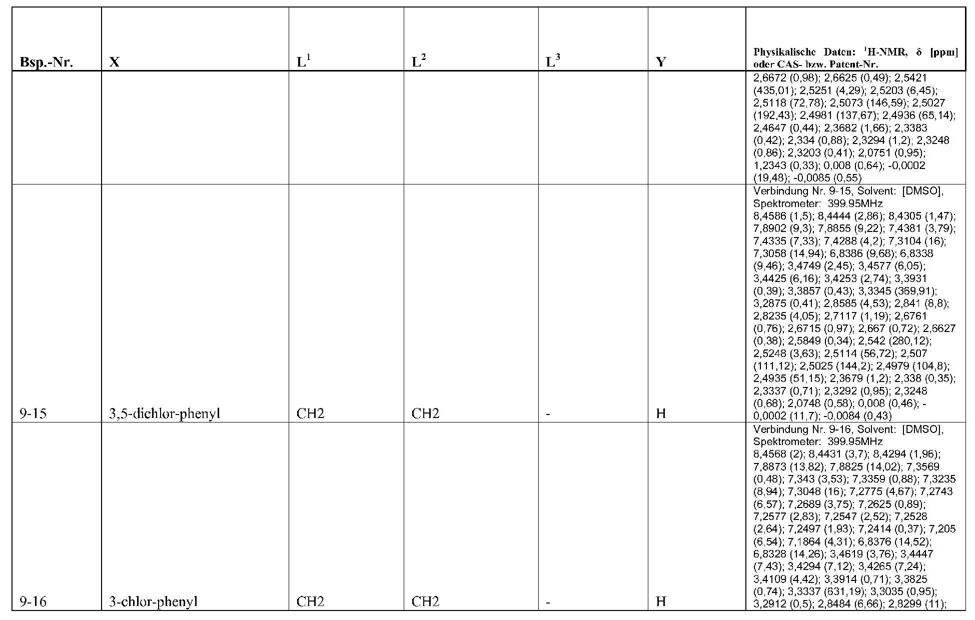

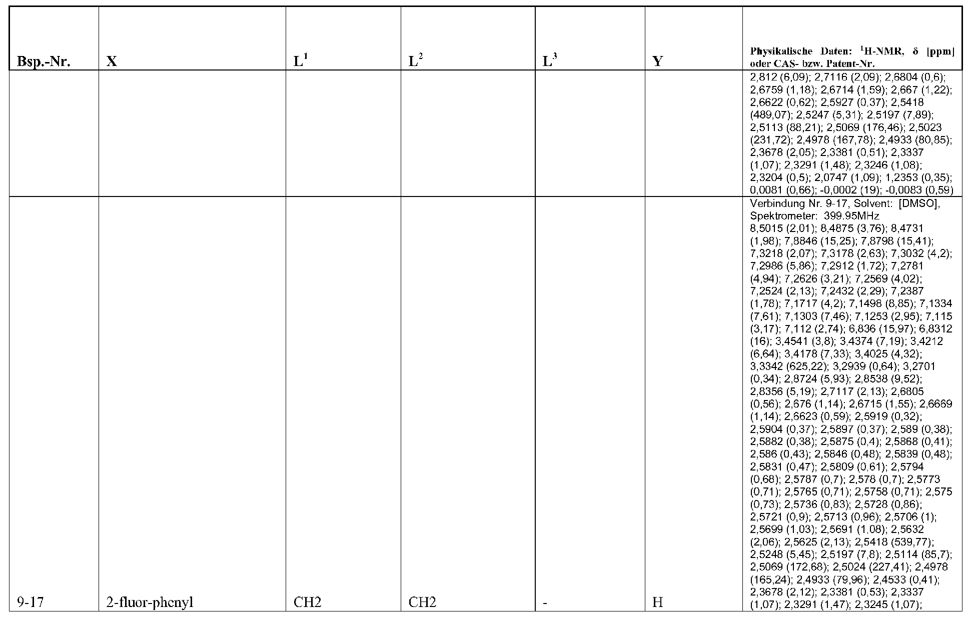

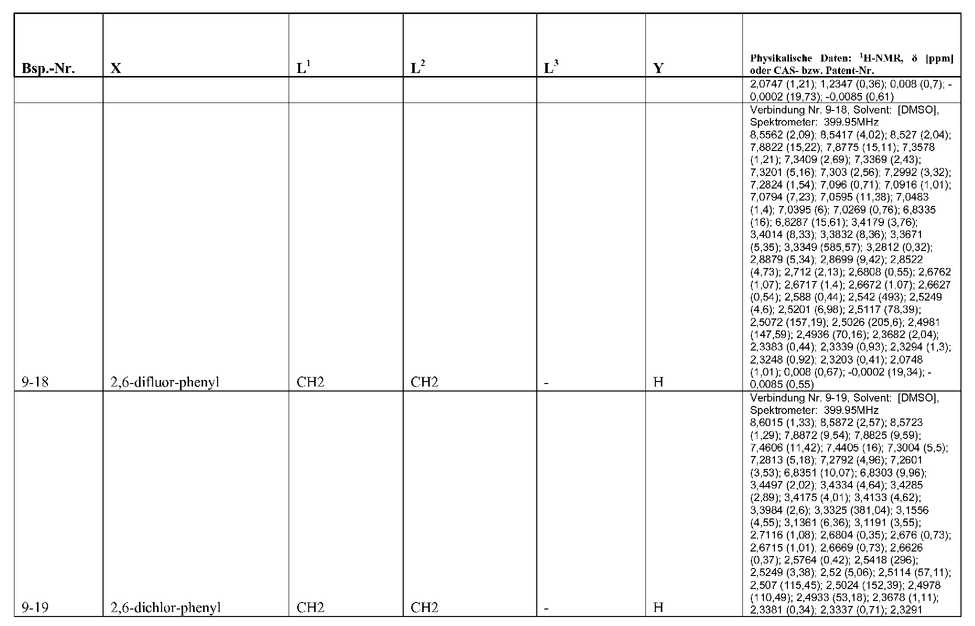

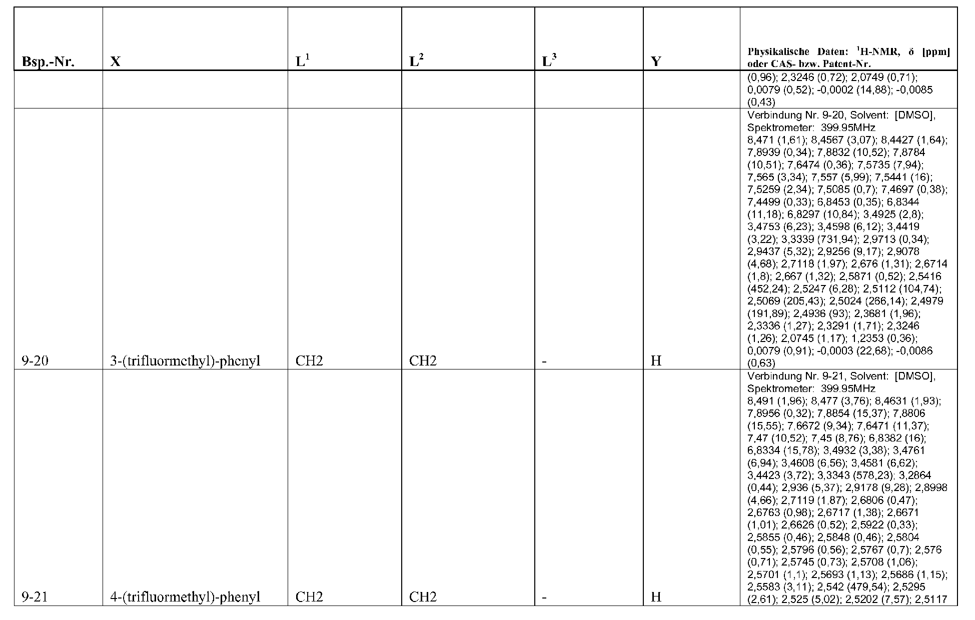

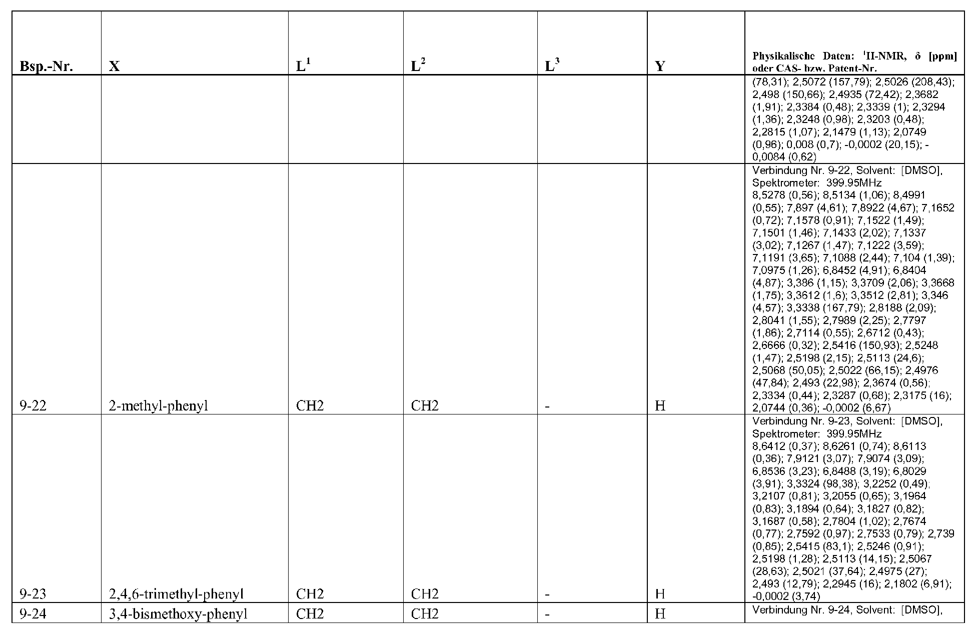

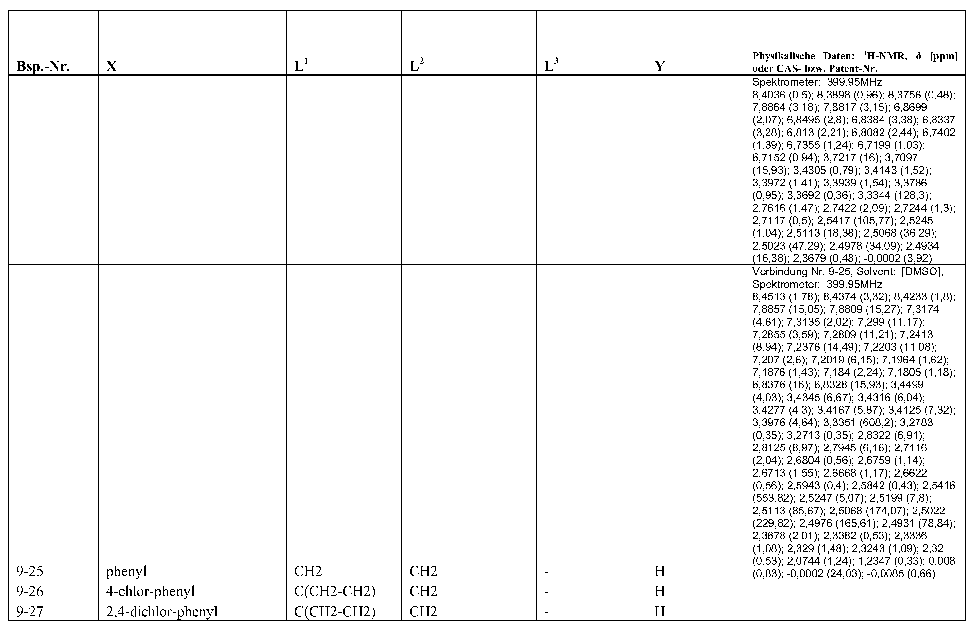

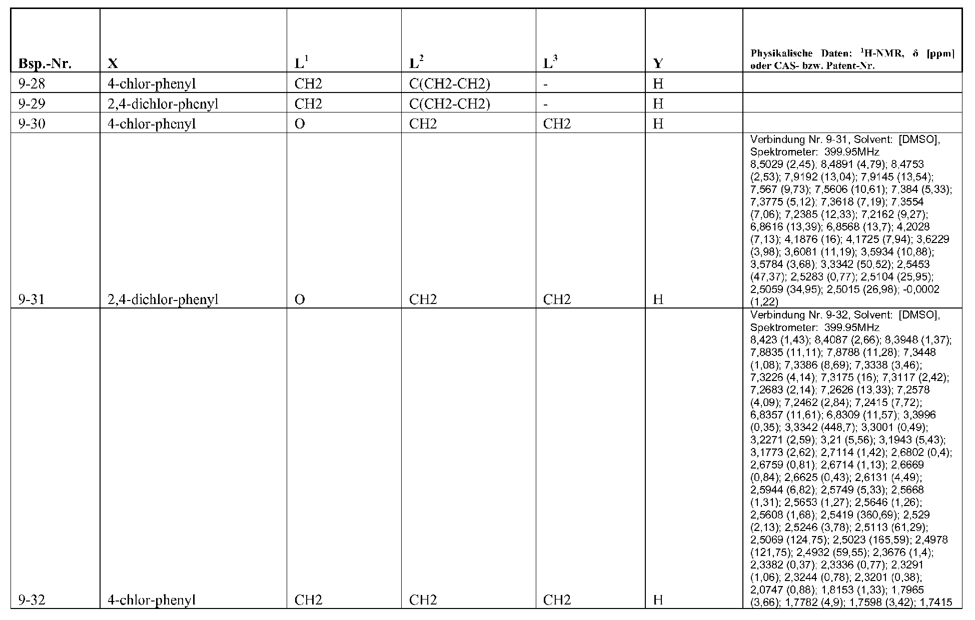

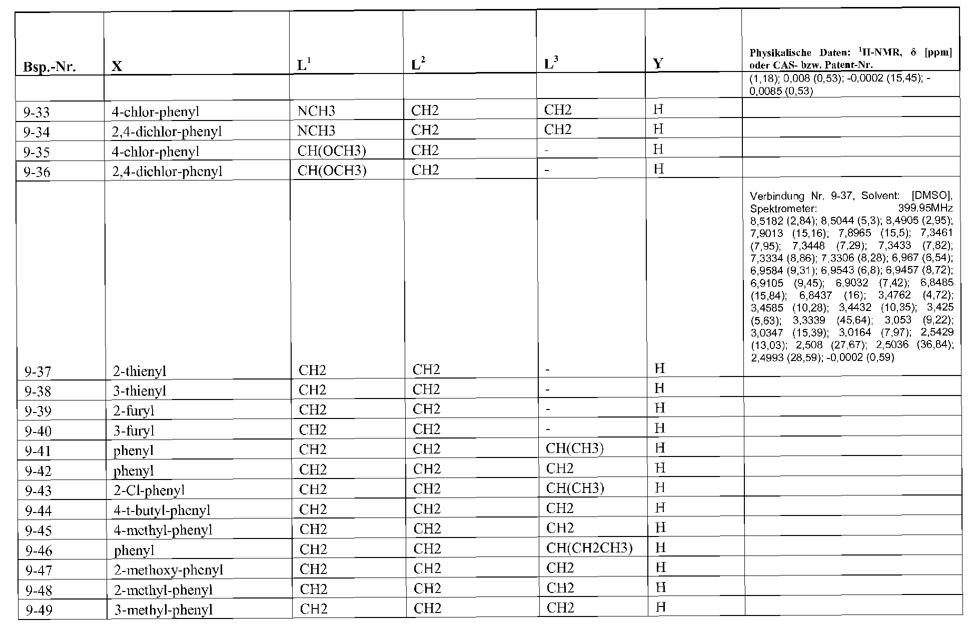

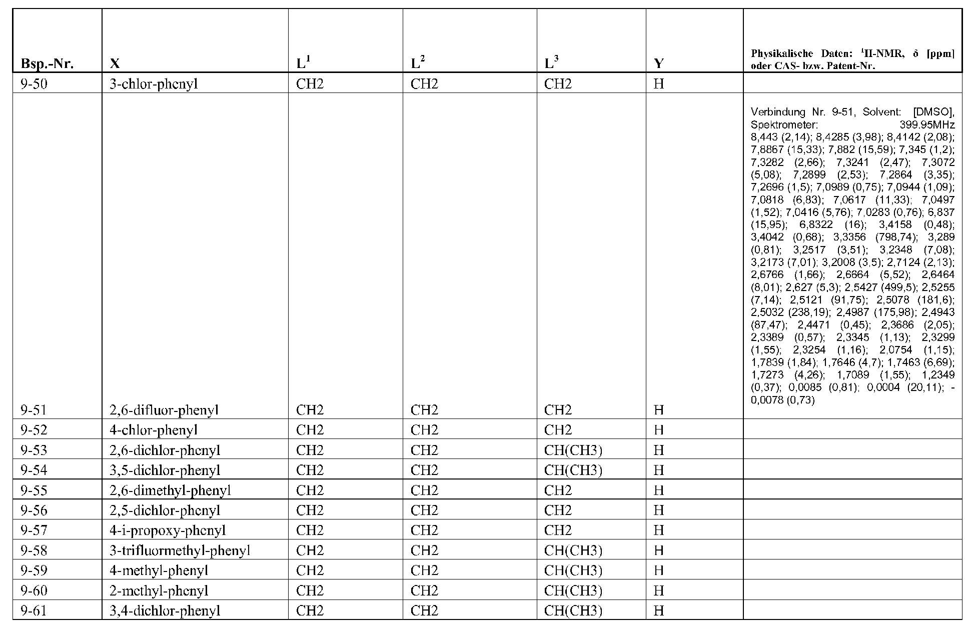

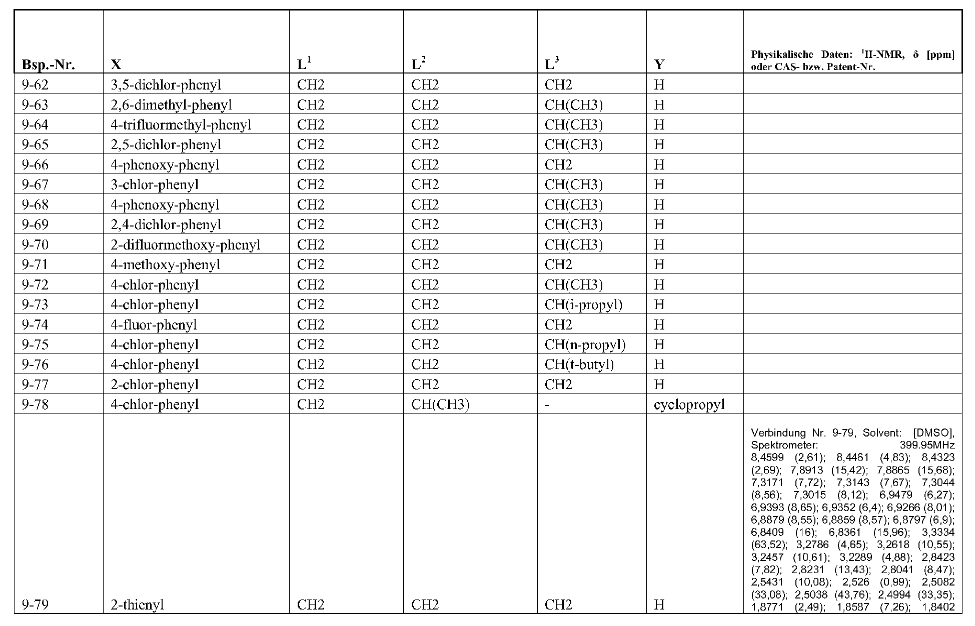

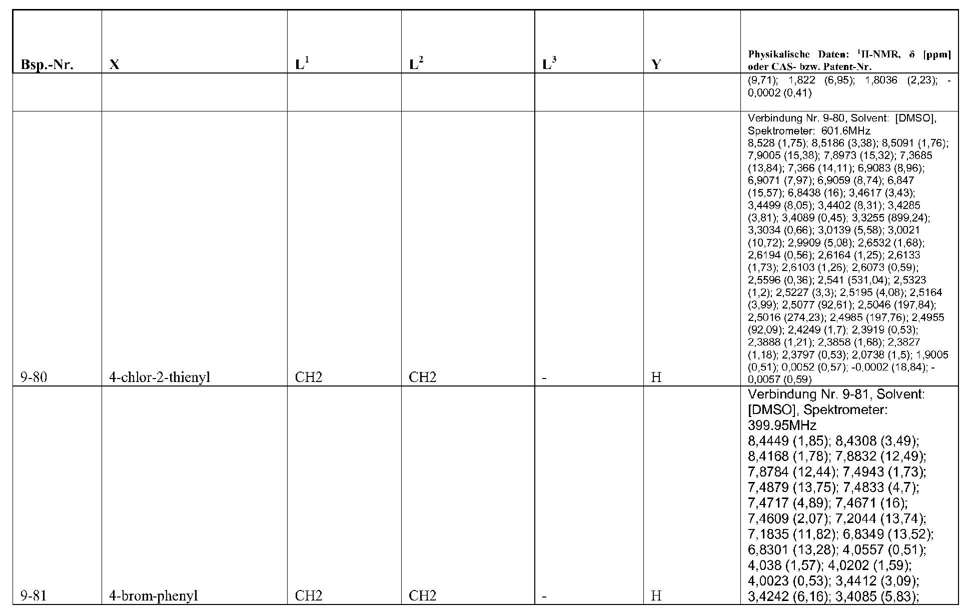

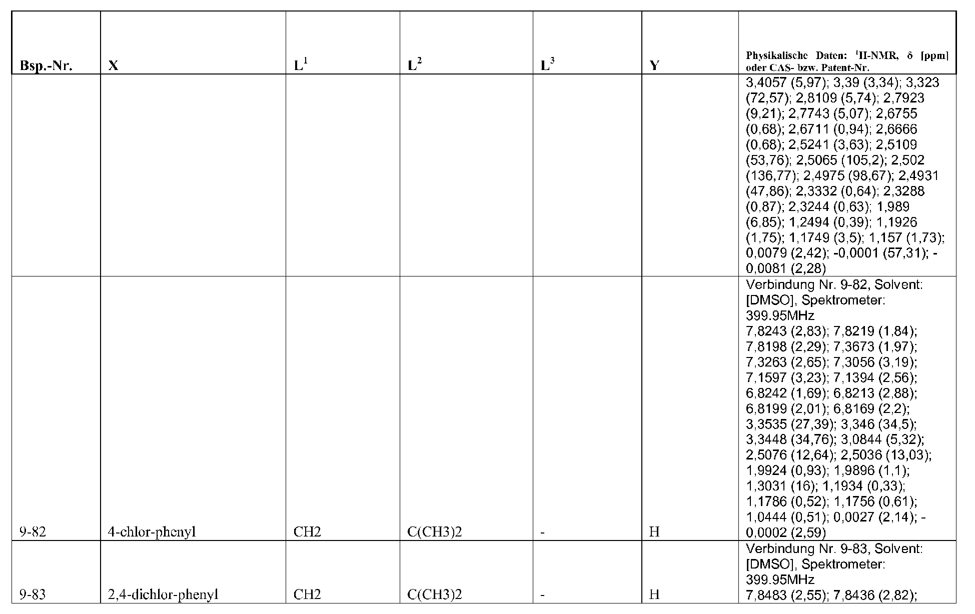

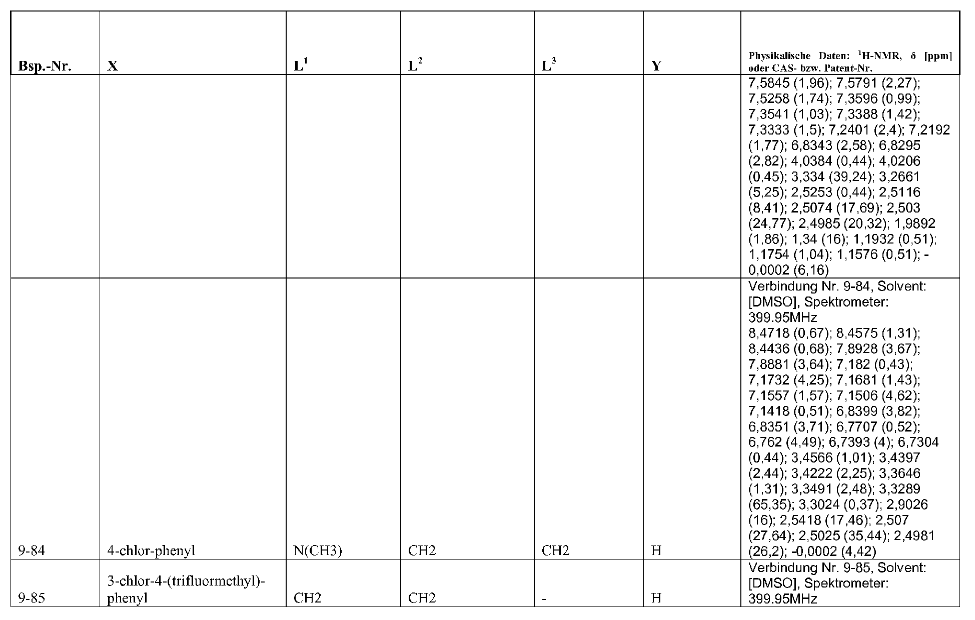

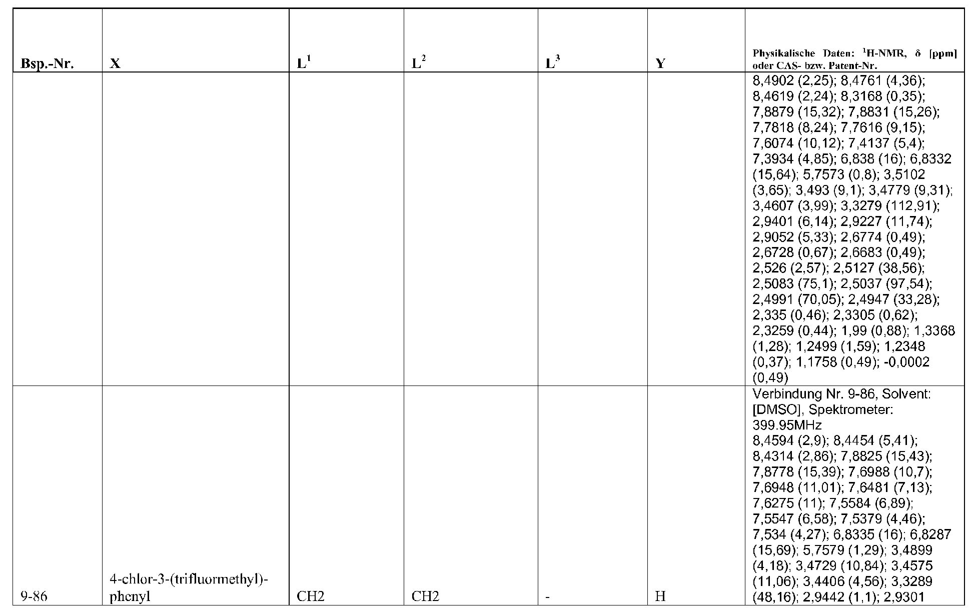

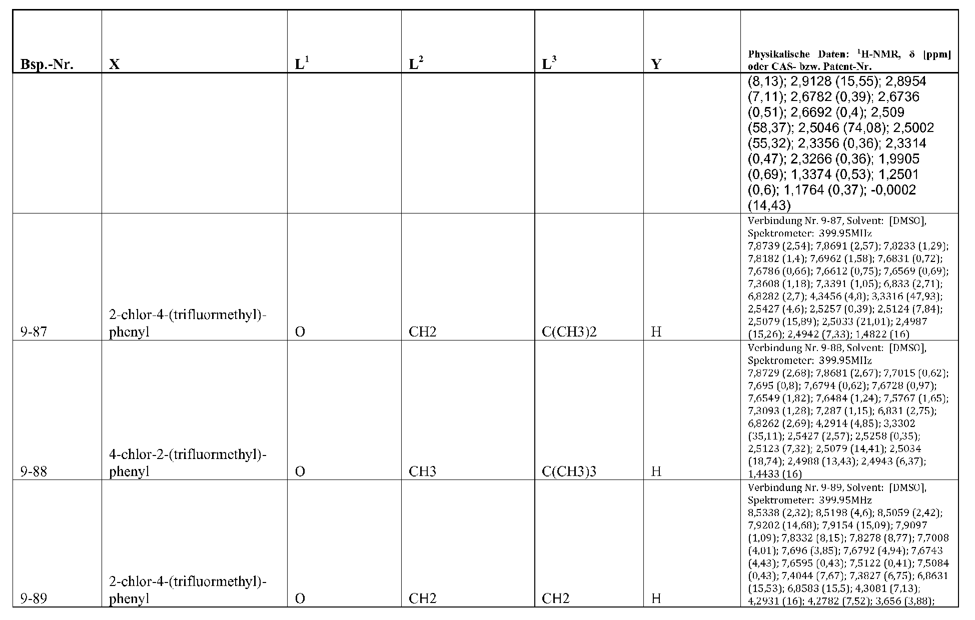

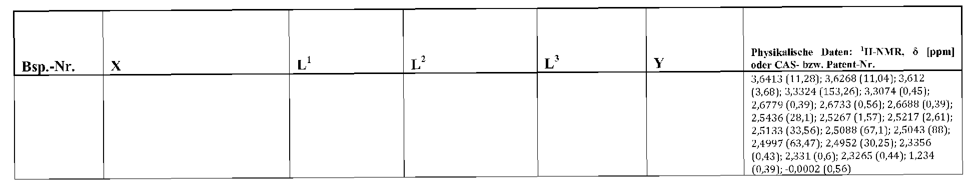

**Tabelle 10**

| | | | | | | |
|---|---|---|---|---|---|---|
| Verbindungen der Formel 1-10 | | | | | | |
| 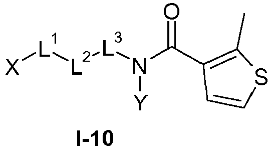 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 10-1 | 3-methl-2-thienyl | CH2 | CH2 | - | H | |
| 10-2 | 2,4-dichlor-phenyl | CH2 | CH2 | CH2 | H | |
| 10-3 | 4-chlor-phenyl | CH2 | CH2 | - | H | |
| 10-4 | 2,4-dichlor-phenyl | CH2 | CH2 | - | H | |
| 10-5 | 4-chlor-phenyl | CH(OCH3) | CH(CH3) | - | H | |
| 10-6 | 2,4-dichlor-phenyl | CH(OCH3) | CH(CH3) | - | H | |
| 10-7 | 4-chlor-phenyl | CH(CH3) | CH2 | - | H | |
| 10-8 | 2,4-dichlor-phenyl | CH(CH3) | CH2 | - | H | |
| 10-9 | 4-chlor-phenyl | CH2 | CH(CH3) | - | H | |
| 10-10 | 2,4-dichlor-phenyl | CH2 | CH(CH3) | - | H | |
| 10-11 | 4-chlor-phenyl | C(CH3)2 | CH2 | - | H | |
| 10-12 | 2,4-dichlor-phenyl | C(CH3)2 | CH2 | - | H | |
| 10-13 | 2-chlor-phenyl | CH2 | CH2 | - | H | |
| 10-14 | 3,4-dichlor-phenyl | CH2 | CH2 | - | H | |
| 10-15 | 3,5-dichlor-phenyl | CH2 | CH2 | - | H | |
| 10-16 | 3-chlor-phenyl | CH2 | CH2 | - | H | |
| 10-17 | 2-fluor-phenyl | CH2 | CH2 | - | H | |
| 10-18 | 2,6-difluor-phenyl | CH2 | CH2 | - | H | |
| 10-19 | 2,6-dichlor-phenyl | CH2 | CH2 | - | H | |
| 10-20 | 3 -(trifluormethyl)-phenyl | CH2 | CH2 | - | H | |
| 10-21 | 4-(trifluormethyl)-phenyl | CH2 | CH2 | - | H | |
| 10-22 | 2-methyl-phenyl | CH2 | CH2 | - | H | |
| 10-23 | 2,4,6-trimethyl-phenyl | CH2 | CH2 | - | H | |
| 10-24 | 3,4-bismethoxy-phenyl | CH2 | CH2 | - | H | |
| 10-25 | phenyl | CH2 | CH2 | - | H | |
| 10-26 | 4-chlor-phenyl | C(CH2-CH2) | CH2 | - | H | |
| 10-27 | 2,4-dichlor-phenyl | C(CH2-CH2) | CH2 | - | H | |
| 10-28 | 4-chlor-phenyl | CH2 | C(CH2-CH2) | - | H | |
| 10-29 | 2,4-dichlor-phenyl | CH2 | C(CH2-CH2) | - | H | |
| 10-30 | 4-chlor-phenyl | O | CH2 | CH2 | H | |
| 10-31 | 2,4-dichlor-phenyl | O | CH2 | CH2 | H | |
| 10-32 | 4-chlor-phenyl | CH2 | CH2 | CH2 | H | |
| 10-33 | 4-chlor-phenyl | NCH3 | CH2 | CH2 | H | |
| 10-34 | 2,4-dichlor-phenyl | NCH3 | CH2 | CH2 | H | |
| 10-35 | 4-chlor-phenyl | CH(OCH3) | CH2 | - | H | |
| 10-36 | 2,4-dichlor-phenyl | CH(OCH3) | CH2 | - | H | |
| 10-37 | 2-thienyl | CH2 | CH2 | - | H | |
| 10-38 | 3-thienyl | CH2 | CH2 | - | H | |
| 10-39 | 2-furyl | CH2 | CH2 | - | H | |
| 10-40 | 3-furyl | CH2 | CH2 | - | H | |
| 10-41 | phenyl | CH2 | CH2 | CH(CH3) | H | |
| 10-42 | phenyl | CH2 | CH2 | CH2 | H | |
| 10-43 | 2-Cl-phenyl | CH2 | CH2 | CH(CH3) | H | |
| 10-44 | 4-t-butyl-phenyl | CH2 | CH2 | CH2 | H | |
| 10-45 | 4-methyl-phenyl | CH2 | CH2 | CH2 | H | |
| 10-46 | phenyl | CH2 | CH2 | CH(CH2CH3) | H | |
| 10-47 | 2-methoxy-phenyl | CH2 | CH2 | CH2 | H | |
| 10-48 | 2-methyl-phenyl | CH2 | CH2 | CH2 | H | |
| 10-49 | 3-methyl-phenyl | CH2 | CH2 | CH2 | H | |
| 10-50 | 3-chlor-phenyl | CH2 | CH2 | CH2 | H | |
| 10-51 | 2,6-difluor-phenyl | CH2 | CH2 | CH2 | H | |
| 10-52 | 4-chlor-phenyl | CH2 | CH2 | CH2 | H | |
| 10-53 | 2,6-dichlor-phenyl | CH2 | CH2 | CH(CH3) | H | |
| 10-54 | 3,5-dichlor-phenyl | CH2 | CH2 | CH(CH3) | H | |
| 10-55 | 2,6-dimethyl-phenyl | CH2 | CH2 | CH2 | H | |
| 10-56 | 2,5-dichlor-phenyl | CH2 | CH2 | CH2 | H | |
| 10-57 | 4-i-propoxy-phenyl | CH2 | CH2 | CH2 | H | |
| 10-58 | 3-trifluormethyl-phenyl | CH2 | CH2 | CH(CH3) | H | |
| 10-59 | 4-methyl-phenyl | CH2 | CH2 | CH(CH3) | H | |
| 10-60 | 2-methyl-phenyl | CH2 | CH2 | CH(CH3) | H | |
| 10-61 | 3,4-dichlor-phenyl | CH2 | CH2 | CH(CH3) | H | |
| 10-62 | 3,5-dichlor-phenyl | CH2 | CH2 | CH2 | H | |
| 10-63 | 2,6-dimethyl-phenyl | CH2 | CH2 | CH(CH3) | H | |
| 10-64 | 4-trifluormethyl-phenyl | CH2 | CH2 | CH(CH3) | H | |
| 10-65 | 2,5-dichlor-phenyl | CH2 | CH2 | CH(CH3) | H | |
| 10-66 | 4-phenoxy-phenyl | CH2 | CH2 | CH2 | H | |
| 10-67 | 3-chlor-phenyl | CH2 | CH2 | CH(CH3) | H | |
| 10-68 | 4-henox-phenyl | CH2 | CH2 | CH(CH3) | H | |
| 10-69 | 2,4-dichlor-phenyl | CH2 | CH2 | CH(CH3) | H | |
| 10-70 | 2-difluormethoxy-phenyl | CH2 | CH2 | CH(CH3) | H | |
| 10-71 | 4-methoxy-phenyl | CH2 | CH2 | CH2 | H | |
| 10-72 | 4-chlor-phenyl | CH2 | CH2 | CH(CH3) | H | |
| 10-73 | 4-chlor-phenyl | CH2 | CH2 | CH(i-propyl) | H | |
| 10-74 | 4-fluor-phenyl | CH2 | CH2 | CH2 | H | |
| 10-75 | 4-chlor-phenyl | CH2 | CH2 | CH(n-propyl) | H | |
| 10-76 | 4-chlor-phenyl | CH2 | CH2 | CH(t-butyl) | H | |
| 10-77 | 2-chlor-phenyl | CH2 | CH2 | CH2 | H | |
| 10-78 | 4-chlor-phenyl | CH2 | CH(CH3) | - | cyclopropyl | WO-A 2007/060164 |

**Tabelle 11**

| | | | | | | |
|---|---|---|---|---|---|---|
| Verbindungen der Formel I-11 | | | | | | |
| 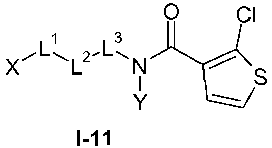 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 11-1 | 3-methyl-2-thienyl | CH2 | CH2 | - | H | |
| 11-2 | 2,4-dichlor-phenyl | CH2 | CH2 | CH2 | H | |
| 11-3 | 4-chlor-phenyl | CH2 | CH2 | - | H | Verbindung Nr. 11-3, Solvent: [DMSO], Spektrometer: 399.95MHz |
| | | | | | | 8,324 (1,4); 8,3105 (2,59); 8,2971 (1,42); 7,4819 (9,92); 7,4674 (10,93); 7,3651 (1); 7,3588 (9,04); 7,3541 (3,3); 7,3429 (4,14); 7,3378 (16); 7,3319 (2,25); 7,2844 (2,06); 7,2787 (13,94); 7,2739 (3,99); 7,2623 (3,01); 7,2576 (8,33); 7,1841 (11,46); 7,1696 (10,53); 3,454 (2,82); 3,4366 (5,79); 3,4219 (5,7); 3,4038 (3,12); 3,3268 (91,94); 2,8304 (5,17); 2,8122 (9,02); 2,7944 (4,63); 2,6754 (0,35); 2,6708 (0,48); 2,6663 (0,37); 2,5411 (16,41); 2,5241 (1,28); 2,5107 (25,83); 2,5062 (51,84); 2,5017 (69,01); 2,4971 (50,97); 2,4926 (24,86); 2,3331 (0,32); 2,3284 (0,44); 2,3238 (0,32); 2,0747 (0,45); -0,0002 (7,87) |
| 11-4 | 2,4-dichlor-phenyl | CH2 | CH2 | - | H | Verbindung Nr. 11-4, Solvent: [DMSO], Spektrometer: 399.95MHz |
| | | | | | | 8,3622 (1,12); 8,3484 (2,12); 8,3344 (1,12); 7,5825 (6,36); 7,5799 (3,86); 7,4827 (7,29); 7,4682 (7,98); 7,3726 (16); 7,3695 (15,8); 7,1861 (8,69); 7,1716 (8,04); 3,4845 (1,92); 3,4673 (4,73); 3,4524 (4,82); 3,4352 (2,13); 3,33 (28,07); 2,9532 (4,09); 2,9358 (7,84); 2,9183 (3,59); 2,5426 (10,86); 2,5256 (0,54); 2,5123 (9,2); 2,5078 (18,09); 2,5032 (23,82); 2,4987 (17,46); 2,4942 (8,49); -0,0002 (2,82) |
| 11-5 | 4-chlor-phenyl | CH(OCH3) | CH(CH3) | - | H | |
| 11-6 | 2,4-dichlor-phenyl | CH(OCH3) | CH(CH3) | - | H | |
| 11-7 | 4-chlor-phenyl | CH(CH3) | CH2 | - | H | Verbindung Nr. 11-7, Solvent: [DMSO], Spektrometer: 399.95MHz |
| | | | | | | 8,2745 (1,71); 8,2608 (3,17); 8,247 (1,75); 7,4645 (7,07); 7,45 (7,68); 7,3619 (7,04); 7,3409 (13,53); 7,2937 (13,14); 7,2726 (7,41); 7,1296 (8); 7,1151 (7,43); 3,4038 (0,52); 3,3856 (0,82); 3,3709 (2,68); 3,3532 (5,26); 3,3369 (5,77); 3,3265 (12,28); 3,3033 (0,9); 3,2886 (0,6); 3,0809 (0,35); 3,0631 (1,51); 3,0454 (2,93); 3,0275 (2,85); 3,0097 (1,37); 2,5023 (34,43); 2,4985 (28,1); 1,9893 (0,45); 1,3969 (1,19); 1,2488 (0,38); 1,2259 (16); 1,2084 (15,92); 1,1753 (0,37); -0,0002 (17,97) |
| 11-8 | 2,4-dichlor-phenyl | CH(CH3) | CH2 | - | H | Verbindung Nr. 11-8, Solvent: [DMSO], Spektrometer: 399.95MHz |
| | | | | | | 8,3291 (1,78); 8,3149 (3,46); 8,3011 (1,85); 7,558 (6,34); 7,5528 (6,84); 7,4712 (4,61); 7,4635 (7,51); 7,4495 (15,86); 7,4173 (5,42); 7,4122 (5,15); 7,3962 (2,53); 7,3911 (2,49); 7,1338 (7,92); 7,1192 (7,36); 4,0385 (0,77); 4,0208 (0,78); 3,5511 (1,19); 3,5336 (2,64); 3,5162 (3,05); 3,4991 (1,9); 3,4859 (1,53); 3,4717 (1,35); 3,4541 (2,88); 3,4385 (3,59); 3,4224 (3,86); 3,4074 (2,92); 3,3895 (2,56); 3,3749 (1,09); 3,3567 (0,7); 3,3263 (22,58); 2,5028 (45,05); 1,9897 (3,21); 1,3972 (12,85); 1,2492 (0,33); 1,2167 (15,99); 1,1997 (16); 1,1755 (1,91); 1,1576 (0,94); -0,0002 (20,68) |
| 11-9 | 4-chlor-phenyl | CH2 | CH(CH3) | - | H | Verbindung Nr. 11-9, Solvent: [DMSO], Spektrometer: 399.95MHz |
| | | | | | | 8,1383 (2,47); 8,1177 (2,5); 7,4695 (8,58); 7,4551 (9,27); 7,3752 (0,42); 7,3469 (0,87); 7,3408 (7,51); 7,3361 (2,74); 7,3247 (3,61); 7,3198 (13,82); 7,3141 (2,01); 7,2672 (12,32); 7,2461 (6,96); 7,1445 (9,23); 7,13 (8,54); 4,1754 (0,66); 4,1557 (1,41); 4,1392 (2,05); 4,1226 (1,49); 4,103 (0,67); 3,3281 (112,12); 2,9958 (0,65); 2,8303 (1,33); 2,8106 (1,27); 2,7967 (3,33); 2,7769 (3,33); 2,7601 (3,34); 2,7445 (3,38); 2,7265 (1,35); 2,711 (1,31); 2,671 (0,39); 2,5413 (31,42); 2,5244 (1,22); 2,5108 (22,6); 2,5065 (44,94); 2,5019 (59,63); 2,4974 (44,12); 2,493 (21,73); 2,3286 (0,38); 1,1422 (16); 1,1256 (15,88); - 0,0002 (6,23) |
| 11-10 | 2,4-dichlor-phenyl | CH2 | CH(CH3) | - | H | Verbindung Nr. 11-11, Solvent: [DMSO], Spektrometer: 399.95MHz |
| | | | | | | 8,0755 (0,35); 8,0602 (0,68); 8,0447 (0,35); 7,4556 (2,62); 7,4409 (4,82); 7,4352 (0,88); 7,424 (1,07); 7,4186 (4,21); 7,412 (0,54); 7,3689 (0,53); 7,3623 (4,21); 7,3569 (1,03); 7,3456 (0,79); 7,3405 (2,34); 7,1089 (2,97); 7,0945 (2,75); 3,4168 (2,88); 3,4009 (2,86); 3,326 (22,44); 2,5411 (5,12); 2,5241 (0,37); 2,5193 (0,58); 2,5108 (7,15); 2,5063 (14,26); 2,5017 (18,89); 2,4971 (13,79); 2,4927 (6,62); 1,2964 (16); -0,0002 (2,31) |
| 11-11 | 4-chlor-phenyl | C(CH3)2 | CH2 | - | H | Verbindung Nr. 11-12, Solvent: [DMSO], Spektrometer: 399.95MHz |
| | | | | | | 8,0743 (0,37); 8,0588 (0,74); 8,0432 (0,38); 7,5223 (2,56); 7,5165 (2,75); 7,4778 (1,68); 7,4561 (2,46); 7,4438 (3,17); 7,4294 (3,37); 7,3712 (1,69); 7,3654 (1,57); 7,3497 (1,17); 7,3438 (1,11); 7,0774 (3,61); 7,0629 (3,37); 3,7598 (2,9); 3,7439 (2,88); 3,3254 (19,78); 2,5413 (3,21); 2,5244 (0,34); 2,5197 (0,51); 2,511 (6,19); 2,5065 (12,62); 2,5019 (16,92); 2,4972 (12,39); 2,4927 (5,95); 1,4489 (16); -0,0002 (2,31) |
| 11-12 | 2,4-dichlor-phenyl | C(CH3)2 | CH2 | - | H | Verbindung Nr. 11-13, Solvent: [DMSO], Spektrometer: 399.95MHz |
| | | | | | | 8,3829 (2,11); 8,3691 (3,81); 8,3557 (2,11); 7,4823 (13,81); 7,4678 (15,22); 7,458 (0,49); 7,4401 (5,35); 7,4361 (4,23); 7,4219 (7,19); 7,4171 (6,67); 7,4 (0,38); 7,368 (4,01); 7,3625 (4,87); 7,3495 (5,47); 7,3447 (7,06); 7,3074 (2,33); 7,3032 (3,01); 7,2891 (7,34); 7,2848 (6,8); 7,2742 (7,49); 7,272 (7,63); 7,2681 (8,03); 7,2557 (6); 7,2504 (5,12); 7,2372 (2,01); 7,2322 (1,55); 7,2012 (16); 7,1867 (14,67); 3,4886 (4,03); 3,4715 (8,18); 3,4566 (8,03); 3,4537 (7,57); 3,4382 (4,56); 3,3673 (0,33); 3,327 (146,98); 2,9718 (7,96); 2,9534 (12,73); 2,9358 (6,97); 2,6753 (0,51); 2,671 (0,66); 2,6665 (0,49); 2,5412 (37,44); 2,5242 (2,74); 2,5108 (39,44); 2,5064 (75,52); 2,5019 (97,85); 2,4973 (71,6); 2,4929 (35,07); 2,3332 (0,48); 2,3286 (0,64); 2,3241 (0,46); 2,0747 (0,38); 0,0079 (0,48); -0,0002 (11,2); - 0,0085 (0,4) |
| 11-13 | 2-chlor-phenyl | CH2 | CH2 | - | H | Verbindung Nr. 11-14, Solvent: [DMSO], Spektrometer: 399.95MHz |
| | | | | | | 8,3194 (2,09); 8,3057 (3,86); 8,292 (2,08); 7,5558 (12,09); 7,5453 (0,61); 7,5352 (14,39); 7,5286 (11,07); 7,5236 (11,23); 7,4847 (14,74); 7,4702 (16); 7,257 (6,32); 7,2519 (6,09); 7,2364 (5,54); 7,2313 (5,33); 7,1627 (15,77); 7,1482 (14,57); 3,477 (3,74); 3,4599 (9,58); 3,4452 (9,79); 3,4283 (4,08); 3,3274 (110,78); 2,8471 (7,04); 2,8299 (14,14); 2,8126 (6,27); 2,676 (0,41); 2,6714 (0,55); 2,6667 (0,46); 2,5417 (32,72); 2,5247 (1,78); 2,5114 (31,21); 2,5069 (61,08); 2,5024 (80,34); 2,4978 (58,66); 2,4933 (28,25); 2,3337 (0,38); 2,3291 (0,52); 2,3246 (0,38); 2,0755 (0,34); 0,008 (0,34); -0,0002 (9,07) |
| 11-14 | 3,4-dichlor-phenyl | CH2 | CH2 | - | H | Verbindung Nr. 11-15, Solvent: [DMSO], Spektrometer: 399.95MHz |
| | | | | | | 8,3216 (1,26); 8,3082 (2,37); 8,2947 (1,26); 7,4877 (10,15); 7,4732 (11,05); 7,4407 (3,67); 7,436 (7,35); 7,4312 (4,19); 7,3284 (16); 7,3236 (14,68); 7,1511 (11,7); 7,1366 (10,87); 3,487 (2,32); 3,47 (6,1); 3,4555 (6,24); 3,4388 (2,55); 3,3465 (0,34); 3,3273 (80,76); 3,3112 (0,45); 2,8597 (4,25); 2,8427 (8,64); 2,8257 (3,84); 2,6713 (0,38); 2,5416 (8,3); 2,5248 (1,09); 2,5199 (1,67); 2,5113 (20,85); 2,5068 (41,83); 2,5022 (55,49); 2,4976 (40,29); 2,493 (19,11); 2,3289 (0,36); -0,0002 (7,54) |
| 11-15 | 3,5-dichlor-phenyl | CH2 | CH2 | - | H | Verbindung Nr. 11-16, Solvent: [DMSO], Spektrometer: 399.95MHz |
| | | | | | | 8,3321 (1,58); 8,3187 (2,87); 8,3052 (1,58); 7,4839 (14,27); 7,4694 (15,38); 7,359 (0,35); 7,3448 (3,19); 7,3378 (0,88); 7,3247 (15,14); 7,3198 (5,47); 7,3066 (7,88); 7,2798 (4,31); 7,2767 (6,14); 7,2745 (4,01); 7,2716 (4,33); 7,26 (2,29); 7,2564 (2,53); 7,2549 (2,51); 7,2516 (1,75); 7,225 (3,71); 7,2216 (5,76); 7,2064 (2,76); 7,2028 (4,04); 7,1998 (2,42); 7,1841 (0,59); 7,174 (16); 7,1651 (0,58); 7,1595 (14,79); 3,473 (3,4); 3,4555 (7,36); 3,4411 (7,38); 3,4232 (3,88); 3,3282 (144,3); 3,3127 (0,6); 2,8515 (6,04); 2,8337 (11,28); 2,816 (5,48); 2,6755 (0,43); 2,6709 (0,63); 2,6664 (0,43); 2,554 (0,38); 2,5412 (42,89); 2,5243 (1,78); 2,5196 (2,86); 2,511 (31,99); 2,5064 (64,24); 2,5018 (85,45); 2,4972 (61,99); 2,4926 (29,23); 2,3332 (0,42); 2,3286 (0,56); 2,3241 (0,4); 2,0746 (0,37); 0,008 (0,37); -0,0002 (11,24); -0,0085 (0,32) |
| 11-16 | 3-chlor-phenyl | CH2 | CH2 | - | H | Verbindung Nr. 11-17, Solvent: [DMSO], Spektrometer: 399.95MHz |
| | | | | | | 8,3767 (1,9); 8,3634 (3,37); 8,3495 (1,86); 7,4811 (11,89); 7,4666 (13,01); 7,339 (2,1); 7,3352 (2,47); 7,3205 (4,24); 7,316 (5,16); 7,2973 (3,78); 7,2847 (1,38); 7,2799 (2,86); 7,2653 (2,86); 7,2597 (3,62); 7,2552 (1,99); 7,246 (2,03); 7,2415 (1,67); 7,1895 (13,78); 7,175 (16); 7,1526 (8,37); 7,1361 (6,92); 7,1334 (6,68); 7,1178 (2,86); 7,115 (2,54); 3,4647 (3,72); 3,4477 (7,58); 3,4323 (7,23); 3,4141 (4,05); 3,3283 (141,3); 2,878 (5,94); 2,8599 (10,29); 2,8418 (5,24); 2,6755 (0,46); 2,6709 (0,58); 2,6664 (0,42); 2,5413 (37,08); 2,5107 (34,65); 2,5064 (67,03); 2,5019 (88,23); 2,4974 (65,69); 2,4931 (32,79); 2,3333 (0,42); 2,3286 (0,56); 2,3242 (0,41); -0,0002 (7,45) |
| 11-17 | 2-fluor-phenyl | CH2 | CH2 | - | H | Verbindung Nr. 11-18, Solvent: [DMSO], Spektrometer: 399.95MHz |
| | | | | | | 8,4295 (1,87); 8,4151 (3,52); 8,4009 (1,86); 7,4813 (14,56); 7,4668 (16); 7,3601 (1,18); 7,3433 (2,58); 7,3391 (2,31); 7,3224 (5,03); 7,3185 (2,04); 7,3053 (2,42); 7,3016 (3,18); 7,2848 (1,5); 7,1731 (15,19); 7,1586 (14,02); 7,0998 (0,65); 7,0953 (0,93); 7,0831 (6,82); 7,0751 (1,31); 7,0632 (10,87); 7,0518 (1,34); 7,0431 (5,77); 7,0305 (0,76); 3,4342 (3,37); 3,4174 (8,24); 3,4011 (8,25); 3,3842 (3,75); 3,3542 (0,4); 3,3485 (0,67); 3,3276 (165,07); 3,3094 (0,55); 3,3036 (0,36); 2,8962 (5,11); 2,8786 (9,51); 2,8611 (4,56); 2,6757 (0,41); 2,6711 (0,56); 2,6666 (0,41); 2,5415 (1,8); 2,5243 (1,8); 2,5111 (32,75); 2,5066 (64,62); 2,502 (85,44); 2,4974 (62,13); 2,4929 (29,71); 2,3334 (0,39); 2,3288 (0,55); 2,3243 (0,39); - 0,0002 (8,23) |
| 11-18 | 2,6-difluor-phenyl | CH2 | CH2 | - | H | Verbindung Nr. 11-19, Solvent: [DMSO], Spektrometer: 399.95MHz |
| | | | | | | 8,4545 (1,91); 8,4408 (3,47); 8,4271 (1,91); 7,484 (6,03); 7,4822 (6,36); 7,4675 (16); 7,4458 (13,27); 7,3032 (4,03); 7,2836 (6,08); 7,2637 (2,64); 7,2223 (6,73); 7,2208 (6,83); 7,2078 (6,04); 7,2063 (6,15); 3,4725 (2,41); 3,4559 (6,31); 3,4397 (6,75); 3,4227 (3,01); 3,3312 (92,15); 3,1678 (5,53); 3,1503 (9,02); 3,1324 (4,42); 2,675 (0,38); 2,5437 (6,69); 2,5419 (6,94); 2,5061 (57,13); 2,5027 (58,98); 2,3299 (0,38); 0,0015 (2,92); -0,0002 (3,07) |
| 11-19 | 2,6-dichlor-phenyl | CH2 | CH2 | - | H | Verbindung Nr. 11-20, Solvent: [DMSO], Spektrometer: 399.95MHz |
| | | | | | | 8,3374 (2,21); 8,3242 (3,94); 8,3107 (2,11); 7,6684 (0,41); 7,6481 (0,56); 7,597 (8,84); 7,5858 (3,27); 7,5768 (3,53); 7,5622 (16); 7,5494 (9,65); 7,5371 (3,76); 7,5256 (1,78); 7,5133 (1,26); 7,4932 (0,47); 7,4801 (14,24); 7,4656 (15,49); 7,1809 (0,59); 7,1663 (0,71); 7,156 (15,92); 7,1415 (14,7); 3,5098 (4,08); 3,4924 (10,09); 3,4779 (10,2); 3,4606 (4,58); 3,3562 (0,52); 3,3286 (201,14); 3,3061 (0,64); 2,949 (7,48); 2,9315 (14,75); 2,9141 (6,7); 2,6761 (0,54); 2,6714 (0,73); 2,6668 (0,53); 2,5416 (50,7); 2,5246 (2,16); 2,5113 (41,94); 2,5069 (82,27); 2,5023 (108,16); 2,4978 (78,98); 2,4933 (38,15); 2,3335 (0,48); 2,329 (0,68); 2,3245 (0,48); 2,0749 (0,47); 0,0079 (0,44); - 0,0002 (11,06); -0,0084 (0,35) |
| 11-20 | 3 -(trifluormethyl)-phenyl | CH2 | CH2 | - | H | Verbindung Nr. 11-21, Solvent: [DMSO], Spektrometer: 399.95MHz |
| | | | | | | 8,3587 (1,15); 8,345 (2,15); 8,331 (1,14); 7,6683 (5,85); 7,6483 (7,14); 7,4864 (7,11); 7,4832 (14,15); 7,4686 (16); 7,1809 (11,51); 7,1664 (10,68); 3,5062 (2,2); 3,4889 (4,78); 3,4743 (4,78); 3,4564 (2,44); 3,3283 (114,84); 2,9386 (3,41); 2,9209 (6,32); 2,9031 (3); 2,6714 (0,4); 2,5416 (1,14); 2,5248 (1,33); 2,5201 (2,07); 2,5115 (22,77); 2,5069 (45,71); 2,5023 (60,81); 2,4977 (44,06); 2,4931 (20,85); 2,3291 (0,4); -0,0002 (8,15) |
| 11-21 | 4-(trifluormethyl)-phenyl | CH2 | CH2 | - | H | Verbindung Nr. 11-22, Solvent: [DMSO], Spektrometer: 399.95MHz |
| | | | | | | 8,3927 (0,54); 8,3785 (1); 8,3649 (0,54); 7,4914 (4,51); 7,4769 (4,99); 7,224 (5,11); 7,2095 (4,71); 7,1729 (0,82); 7,1649 (1,47); 7,1617 (1,1); 7,1537 (2,34); 7,1505 (2,52); 7,144 (2,2); 7,1362 (1,07); 7,1301 (0,82); 7,125 (3); 7,1214 (3,18); 7,1183 (1,49); 7,1122 (2,9); 7,1059 (1,29); 7,1 (1,47); 3,4032 (1,17); 3,3881 (1,96); 3,3846 (1,6); 3,3798 (1,13); 3,3727 (1,28); 3,3698 (1,58); 3,365 (2,11); 3,3504 (1,4); 3,3279 (50,23); 2,8321 (2,25); 2,8168 (1,73); 2,8124 (2,5); 2,7938 (2,01); 2,5408 (12,42); 2,524 (0,59); 2,5191 (0,91); 2,5106 (11,37); 2,5061 (22,79); 2,5015 (30,32); 2,4968 (22,05); 2,4923 (10,49); 2,3193 (16); -0,0002 (3,87) |
| 11-22 | 2-methyl-phenyl | CH2 | CH2 | - | H | Verbindung Nr. 11-23, Solvent: [DMSO], Spektrometer: 399.95MHz |
| | | | | | | 8,4846 (0,48); 8,4704 (0,88); 8,4564 (0,48); 7,5054 (1,96); 7,4908 (2,16); 7,2682 (2,2); 7,2537 (2); 6,8072 (4,44); 3,3271 (31,98); 3,2443 (0,62); 3,2295 (1,12); 3,2158 (1,08); 3,2087 (0,96); 3,2031 (1,11); 3,1888 (0,7); 2,8004 (1,25); 2,7867 (1,05); 2,7792 (1,32); 2,7596 (1,02); 2,5409 (4,06); 2,5056 (16,7); 2,5014 (20,02); 2,4971 (15,02); 2,297 (16); 2,1819 (7,29); -0,0002 (1,53) |
| 11-23 | 2,4,6-trimethyl-phenyl | CH2 | CH2 | - | H | Verbindung Nr. 11-25, Solvent: [DMSO], Spektrometer: 399.95MHz |
| | | | | | | 8,3377 (1,59); 8,3247 (2,81); 8,3109 (1,58); 7,4827 (13,01); 7,4682 (14,31); 7,3191 (3,86); 7,3153 (1,65); 7,3008 (10,29); 7,2874 (3,19); 7,2828 (10,7); 7,256 (9,52); 7,252 (13,53); 7,2467 (2,78); 7,2352 (6,55); 7,2256 (3,21); 7,2219 (3,53); 7,2181 (1,9); 7,2092 (2,15); 7,204 (5,95); 7,1998 (16); 7,1853 (15,1); 3,4592 (3,82); 3,4413 (5,92); 3,4268 (5,78); 3,4227 (6,18); 3,4078 (4,19); 3,3276 (137,01); 2,8379 (6,65); 2,8187 (9,34); 2,8007 (5,92); 2,6752 (0,47); 2,6705 (0,61); 2,666 (0,43); 2,5408 (30,04); 2,5239 (1,92); 2,519 (2,92); 2,5105 (34,19); 2,506 (68,14); 2,5014 (90,31); 2,4968 (65,75); 2,4923 (31,3); 2,3327 (0,42); 2,3282 (0,58); 2,3236 (0,41); 2,0741 (0,68); 0,008 (0,38); -0,0002 (11,51); -0,0085 (0,32) |
| 11-24 | 3,4-bismethoxy-phenyl | CH2 | CH2 | - | H | Verbindung Nr. 11-7, Solvent: [DMSO], Spektrometer: 399.95MHz |
| | | | | | | 8,2745 (1,71); 8,2608 (3,17); 8,247 (1,75); 7,4645 (7,07); 7,45 (7,68); 7,3619 (7,04); 7,3409 (13,53); 7,2937 (13,14); 7,2726 (7,41); 7,1296 (8); 7,1151 (7,43); 3,4038 (0,52); 3,3856 (0,82); 3,3709 (2,68); 3,3532 (5,26); 3,3369 (5,77); 3,3265 (12,28); 3,3033 (0,9); 3,2886 (0,6); 3,0809 (0,35); 3,0631 (1,51); 3,0454 (2,93); 3,0275 (2,85); 3,0097 (1,37); 2,5023 (34,43); 2,4985 (28,1); 1,9893 (0,45); 1,3969 (1,19); 1,2488 (0,38); 1,2259 (16); 1,2084 (15,92); 1,1753 (0,37); -0,0002 (17,97) |
| 11-25 | phenyl | CH2 | CH2 | - | H | Verbindung Nr. 11-8, Solvent: [DMSO], Spektrometer: 399.95MHz |
| | | | | | | 8,3291 (1,78); 8,3149 (3,46); 8,3011 (1,85); 7,558 (6,34); 7,5528 (6,84); 7,4712 (4,61); 7,4635 (7,51); 7,4495 (15,86); 7,4173 (5,42); 7,4122 (5,15); 7,3962 (2,53); 7,3911 (2,49); 7,1338 (7,92); 7,1192 (7,36); 4,0385 (0,77); 4,0208 (0,78); 3,5511 (1,19); 3,5336 (2,64); 3,5162 (3,05); 3,4991 (1,9); 3,4859 (1,53); 3,4717 (1,35); 3,4541 (2,88); 3,4385 (3,59); 3,4224 (3,86); 3,4074 (2,92); 3,3895 (2,56); 3,3749 (1,09); 3,3567 (0,7); 3,3263 (22,58); 2,5028 (45,05); 1,9897 (3,21); 1,3972 (12,85); 1,2492 (0,33); 1,2167 (15,99); 1,1997 (16); 1,1755 (1,91); 1,1576 (0,94); -0,0002 (20,68) |
| 11-26 | 4-chlor-phenyl | C(CH2-CH2) | CH2 | - | H | |
| 11-27 | 2,4-dichlor-phenyl | C(CH2-CH2) | CH2 | - | H | |
| 11-28 | 4-chlor-phenyl | CH2 | C(CH2-CH2) | - | H | |
| 11-29 | 2,4-dichlor-phenyl | CH2 | C(CH2-CH2) | - | H | |
| 11-30 | 4-chlor-phenyl | O | CH2 | CH2 | H | |
| 11-31 | 2,4-dichlor-phenyl | O | CH2 | CH2 | H | |
| 11-32 | 4-chlor-phenyl | CH2 | CH2 | CH2 | H | |
| 11-33 | 4-chlor-phenyl | NCH3 | CH2 | CH2 | H | |
| 11-34 | 2,4-dichlor-phenyl | NCH3 | CH2 | CH2 | H | |
| 11-35 | 4-chlor-phenyl | CH(OCH3) | CH2 | - | H | |
| 11-36 | 2,4-dichlor-phenyl | CH(OCH3) | CH2 | - | H | |
| 11-37 | 2-thienyl | CH2 | CH2 | - | H | Verbindung Nr. 11-37, Solvent: [DMSO], Spektrometer: 399.95MHz 8,4093 (1,8); 8,3956 (3,2); 8,3822 (1,77); 7,4959 (13,95); 7,4813 (15,41); 7,3512 (6,88); 7,3481 (7,15); 7,3384 (7,63); 7,3354 (7,39); 7,2335 (16); 7,219 (14,45); 6,9708 (5,81); 6,9623 (8,32); 6,9581 (5,41); 6,9496 (8,12); 6,9238 (6,96); 6,9215 (7,3); 6,9155 (4,9); 6,913 (4,71); 3,4761 (4,02); 3,4583 (8,51); 3,4439 (8,66); 3,4404 (6,22); 3,4258 (4,68); 3,3277 (170,52); 3,3062 (0,39); 3,0561 (7,24); 3,0383 (12,69); 3,0202 (6,06); 2,675 (0,47); 2,6705 (0,64); 2,6658 (0,47); 2,5408 (45,14); 2,5238 (1,96); 2,5189 (3,11); 2,5104 (36,74); 2,5059 (73,63); 2,5013 (97,89); 2,4968 (71,63); 2,4923 (34,49); 2,3327 (0,44); 2,3281 (0,61); 2,3235 (0,45); 2,0742 (0,49); 0,008 (0,35); -0,0002 (10,89) |
| 11-38 | 3-thienyl | CH2 | CH2 | - | H | |
| 11-39 | 2-furyl | CH2 | CH2 | - | H | |
| 11-40 | 3-furyl | CH2 | CH2 | - | H | |
| 11-41 | phenyl | CH2 | CH2 | CH(CH3) | H | |
| 11-42 | phenyl | CH2 | CH2 | CH2 | H | |
| 11-43 | 2-Cl-phenyl | CH2 | CH2 | CH(CH3) | H | |
| 11-44 | 4-t-butyl-phenyl | CH2 | CH2 | CH2 | H | |
| 11-45 | 4-methyl-phenyl | CH2 | CH2 | CH2 | H | |
| 11-46 | phenyl | CH2 | CH2 | CH(CH2CH3) | H | |
| 11-47 | 2-methoxy-phenyl | CH2 | CH2 | CH2 | H | |
| 11-48 | 2-methyl-phenyl | CH2 | CH2 | CH2 | H | |
| 11-49 | 3-methyl-phenyl | CH2 | CH2 | CH2 | H | |
| 11-50 | 3-chlor-phenyl | CH2 | CH2 | CH2 | H | |
| 11-51 | 2,6-difluor-phenyl | CH2 | CH2 | CH2 | H | |
| 11-52 | 4-chlor-phenyl | CH2 | CH2 | CH2 | H | |
| 11-53 | 2,6-dichlor-phenyl | CH2 | CH2 | CH(CH3) | H | |
| 11-54 | 3,5-dichlor-phenyl | CH2 | CH2 | CH(CH3) | H | |
| 11-55 | 2,6-dimethyl-phenyl | CH2 | CH2 | CH2 | H | |
| 11-56 | 2,5-dichlor-phenyl | CH2 | CH2 | CH2 | H | |
| 11-57 | 4-i-propoxy-phenyl | CH2 | CH2 | CH2 | H | |
| 11-58 | 3-trifluormethyl-phenyl | CH2 | CH2 | CH(CH3) | H | |
| 11-59 | 4-methyl-phenyl | CH2 | CH2 | CH(CH3) | H | |
| 11-60 | 2-methyl-phenyl | CH2 | CH2 | CH(CH3) | H | |
| 11-61 | 3,4-dichlor-phenyl | CH2 | CH2 | CH(CH3) | H | |
| 11-62 | 3,5-dichlor-phenyl | CH2 | CH2 | CH2 | H | |
| 11-63 | 2,6-dimethyl-phenyl | CH2 | CH2 | CH(CH3) | H | |
| 11-64 | 4-trifluormethyl-phenyl | CH2 | CH2 | CH(CH3) | H | |
| 11-65 | 2,5-dichlor-phenyl | CH2 | CH2 | CH(CH3) | H | |
| 11-66 | 4-phenoxy-phenyl | CH2 | CH2 | CH2 | H | |
| 11-67 | 3-chlor-phenyl | CH2 | CH2 | CH(CH3) | H | |
| 11-68 | 4-phenoxy-phenyl | CH2 | CH2 | CH(CH3) | H | |
| 11-69 | 2,4-dichlor-phenyl | CH2 | CH2 | CH(CH3) | H | |
| 11-70 | 2-difluormethoxy-phenyl | CH2 | CH2 | CH(CH3) | H | |
| 11-71 | 4-methoxy-phenyl | CH2 | CH2 | CH2 | H | |
| 11-72 | 4-chlor-phenyl | CH2 | CH2 | CH(CH3) | H | |
| 11-73 | 4-chlor-phenyl | CH2 | CH2 | CH(i-propyl) | H | |
| 11-74 | 4-fluor-phenyl | CH2 | CH2 | CH2 | H | |
| 11-75 | 4-chlor-phenyl | CH2 | CH2 | CH(n-propyl) | H | |
| 11-76 | 4-chlor-phenyl | CH2 | CH2 | CH(t-butyl) | H | |
| 11-77 | 2-chlor-phenyl | CH2 | CH2 | CH2 | H | |
| 11-78 | 4-chlor-phenyl | CH2 | CH(CH3) | - | cyclopropyl | |
| 11-79 | 4-chlor-phenyl | CH(OCH3) | CH2 | - | H | Verbindung Nr. 11-79, Solvent: [DMSO], Spektrometer: 399.95MHz |
| | | | | | | 8,346 (0,51); 8,3319 (0,98); 8,3169 (0,52); 7,4778 (2,93); 7,4632 (3,31); 7,4523 (2,92); 7,4479 (1,11); 7,436 (1,26); 7,4312 (4,69); 7,4257 (0,71); 7,36 (4,42); 7,3389 (2,8); 7,1917 (3,31); 7,1772 (3,03); 4,4049 (0,83); 4,3894 (1,47); 4,373 (0,88); 3,4211 (0,97); 3,4123 (1,06); 3,4068 (1,24); 3,4041 (1,33); 3,3976 (1,8); 3,3899 (1,01); 3,3829 (0,95); 3,3214 (14,07); 3,1713 (16); 2,5238 (0,82); 2,5104 (12,71); 2,506 (25,27); 2,5015 (33,73); 2,497 (25,32); 2,4927 (12,7); -0,0002 (1,91) |
| 11-80 | 4-chlor-phenyl | N(CH3) | CH2 | CH2 | H | Verbindung Nr. 11-80, Solvent: [DMSO], Spektrometer: 399.95MHz |
| | | | | | | 8,3571 (0,64); 8,3429 (1,2); 8,3295 (0,65); 8,3151 (0,39); 7,4802 (3,24); 7,4657 (3,5); 7,1859 (0,54); 7,1768 (4,22); 7,1716 (1,62); 7,156 (6,44); 7,1418 (3,48); 6,764 (0,58); 6,7555 (4,42); 6,7328 (3,91); 6,7239 (0,45); 3,4846 (1,24); 3,4676 (2,95); 3,4512 (2,23); 3,378 (1,52); 3,3628 (3,09); 3,3268 (337,58); 3,2827 (0,56); 2,9343 (0,72); 2,9132 (16); 2,6751 (0,92); 2,6707 (1,23); 2,6666 (0,91); 2,5409 (7,25); 2,5061 (147,5); 2,5017 (188,35); 2,4973 (139,99); 2,3328 (0,95); 2,3285 (1,25); 2,3241 (0,93); 0,0077 (1,16); - 0,0003 (24,24); -0,0086 (1,12) |

**Tabelle 12**

| | | | | | | |
|---|---|---|---|---|---|---|
| Verbindungen der Formel I-12 | | | | | | |
| 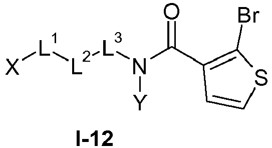 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 12-1 | 3-methyl-2-thienyl | CH2 | CH2 | - | H | |
| 12-2 | 2,4-dichlor-phenyl | CH2 | CH2 | CH2 | H | Verbindung Nr. 12-2, Solvent: [DMSO], Spektrometer: 399.95MHz 8,3521 (1,86); 8,3384 (3,5); 8,3246 (1,85); 7,6354 (13,63); 7,6211 (14,47); 7,5685 (9); 7,5632 (9,4); 7,433 (6,12); 7,4123 (13,13); 7,3828 (8,55); 7,3775 (7,98); 7,3621 (3,81); 7,3568 (3,76); 7,207 (16); 7,1988 (0,63); 7,1927 (15,22); 3,5145 (0,52); 3,3343 (52,66); 3,2728 (3,31); 3,2557 (7,93); 3,2409 (7,97); 3,2238 (3,44); 2,7614 (5,95); 2,7425 (7,24); 2,7226 (6,35); 2,544 (28,04); 2,5271 (0,65); 2,5222 (0,99); 2,5136 (12,37); 2,5091 (25,04); 2,5045 (33,42); 2,4999 (24,37); 2,4954 (11,58); 1,819 (1,71); 1,8011 (4,5); 1,7821 (5,68); 1,7634 (4,33); 1,7456 (1,51); -0,0002 (4,14) |
| 12-3 | 4-chlor-phenyl | CH2 | CH2 | - | H | [DMSO], Spektrometer: 399.95MHz 8.3531 (1.66); 8.3395 (3.09); 8.3258 (1.65); 7.6271 (10.73); 7.6128 (11.25); 7.3689 (1.14); 7.3627 (9.27); 7.3579 (3.66); 7.3466 (4.48); 7.3416 (16.00); 7.3357 (2.73); 7.2857 (14.23); 7.2692 (3.39); 7.2645 (8.46); 7.1601 (11.40); 7.1458 (10.88); 3.4522 (2.83); 3.4349 (5.97); 3.4200 (5.81); 3.4020 (3.12); 3.3459 (18.77); 2.8345 (5.41); 2.8164 (9.21); 2.7985 (4.75); 2.5436 (4.58); 2.5266 (0.41); 2.5218 (0.61); 2.5131 (7.31); 2.5087 (14.83); 2.5042 (19.72); 2.4997 (14.85); 2.4953 (7.53); -0.0002 (6.24) |
| 12-4 | 2,4-dichlor-phenyl | CH2 | CH2 | - | H | [DMSO], Spektrometer: 399.95MHz 8.3827 (1.20); 8.3689 (2.31); 8.3551 (1.19); 7.6272 (7.73); 7.6130 (8.21); 7.5924 (3.75); 7.5898 (6.55); 7.5869 (4.05); 7.3796 (16.00); 7.3766 (15.68); 7.1564 (8.02); 7.1421 (7.63); 3.4760 (1.81); 3.4588 (4.60); 3.4437 (4.66); 3.4267 (2.04); 3.3355 (126.32); 3.3131 (0.35); 2.9517 (3.91); 2.9342 (7.57); 2.9168 (3.44); 2.6759 (0.61); 2.6713 (0.84); 2.6668 (0.62); 2.5418 (12.45); 2.5247 (2.68); 2.5112 (45.90); 2.5068 (93.39); 2.5022 (124.88); 2.4977 (93.92); 2.4933 (47.45); 2.3336 (0.59); 2.3290 (0.83); 2.3246 (0.62); 0.0080 (1.23); -0.0002 (39.06); -0.0085 (1.53) |
| 12-5 | 4-chlor-phenyl | CH(OCH3) | CH(CH3) | - | H | [DMSO], Spektrometer: 399.95MHz 8.1677 (1.14); 8.1461 (1.16); 8.0316 (0.91); 8.0115 (0.92); 7.6200 (2.69); 7.6058 (2.90); 7.5969 (3.53); 7.5827 (3.63); 7.4445 (2.70); 7.4403 (1.05); 7.4270 (4.17); 7.4235 (5.00); 7.4107 (1.49); 7.4059 (5.32); 7.3445 (4.85); 7.3238 (6.23); 7.3031 (2.59); 7.1741 (2.82); 7.1598 (2.70); 7.0188 (3.55); 7.0046 (3.45); 4.3154 (1.44); 4.3003 (1.83); 4.2305 (1.96); 4.2139 (2.72); 4.1965 (0.69); 4.1777 (0.64); 4.1611 (0.39); 4.0941 (0.57); 4.0773 (0.89); 4.0728 (0.70); 4.0604 (0.63); 4.0559 (0.90); 4.0391 (0.52); 3.3426 (14.99); 3.1828 (16.00); 3.1636 (12.86); 2.5435 (4.51); 2.5130 (5.58); 2.5087 (11.35); 2.5041 (15.15); 2.4996 (11.48); 2.4953 (5.90); 1.1524 (6.20); 1.1355 (6.18); 0.9758 (4.73); 0.9587 (4.70); -0.0002 (4.29) |
| 12-6 | 2,4-dichlor-phenyl | CH(OCH3) | CH(CH3) | - | H | |
| 12-7 | 4-chlor-phenyl | CH(CH3) | CH2 | - | H | [DMSO], Spektrometer: 399.95MHz 8.3039 (1.48); 8.2899 (2.87); 8.2756 (1.51); 7.6091 (9.53); 7.5948 (10.00); 7.3722 (0.88); 7.3663 (7.56); 7.3614 (2.91); 7.3501 (3.85); 7.3450 (14.62); 7.3393 (2.53); 7.3055 (2.22); 7.3002 (13.56); 7.2953 (4.05); 7.2835 (2.78); 7.2789 (7.33); 7.1006 (9.98); 7.0863 (9.59); 3.4004 (0.53); 3.3850 (0.69); 3.3818 (0.76); 3.3673 (2.66); 3.3438 (21.19); 3.3339 (5.14); 3.3169 (2.93); 3.3022 (0.66); 3.2985 (0.77); 3.2842 (0.59); 3.0643 (1.39); 3.0465 (2.77); 3.0287 (2.68); 3.0108 (1.26); 2.5434 (3.93); 2.5265 (0.36); 2.5215 (0.54); 2.5130 (7.13); 2.5086 (14.55); 2.5040 (19.46); 2.4995 (14.72); 2.4952 (7.56); 1.2340 (16.00); 1.2165 (15.81); -0.0002 (5.65) |
| 12-8 | 2,4-dichlor-phenyl | CH(CH3) | CH2 | - | H | [DMSO], Spektrometer: 399.95MHz 8.3540 (1.52); 8.3397 (2.97); 8.3255 (1.54); 8.3223 (1.47); 7.6089 (9.43); 7.5946 (9.98); 7.5638 (7.41); 7.5585 (7.90); 7.4769 (4.67); 7.4557 (9.53); 7.4215 (5.64); 7.4161 (5.24); 7.4004 (2.71); 7.3951 (2.63); 7.1065 (10.03); 7.0922 (9.64); 3.5477 (1.10); 3.5303 (2.51); 3.5130 (2.89); 3.4958 (1.76); 3.4830 (1.42); 3.4687 (1.31); 3.4511 (2.77); 3.4356 (3.09); 3.4170 (2.67); 3.4016 (2.58); 3.3835 (2.49); 3.3691 (1.09); 3.3660 (1.00); 3.3405 (28.82); 2.5434 (5.88); 2.5264 (0.75); 2.5129 (12.07); 2.5086 (24.01); 2.5040 (31.64); 2.4995 (23.61); 2.4952 (11.92); 1.4586 (0.34); 1.2241 (16.00); 1.2071 (15.79); -0.0002 (8.22); -0.0085 (0.34) |
| 12-9 | 4-chlor-phenyl | CH2 | CH(CH3) | - | H | [DMSO], Spektrometer: 399.95MHz 8.1645 (2.67); 8.1440 (2.71); 7.6151 (9.36); 7.6009 (9.83); 7.4696 (0.47); 7.3522 (0.85); 7.3462 (7.80); 7.3415 (2.95); 7.3301 (3.80); 7.3251 (14.28); 7.3194 (2.28); 7.2725 (12.62); 7.2514 (7.20); 7.1143 (9.87); 7.1001 (9.41); 4.1697 (0.66); 4.1503 (1.45); 4.1336 (2.09); 4.1169 (1.53); 4.0976 (0.70); 3.3395 (50.45); 2.9978 (0.55); 2.8343 (1.45); 2.8147 (1.41); 2.8007 (3.21); 2.7811 (3.19); 2.7553 (3.23); 2.7396 (3.31); 2.7217 (1.50); 2.7060 (1.35); 2.5423 (26.31); 2.5254 (0.90); 2.5205 (1.33); 2.5118 (17.41); 2.5074 (35.27); 2.5029 (46.85); 2.4983 (35.21); 2.4939 (17.89); 1.1401 (16.00); 1.1236 (15.93); 0.0080 (0.38); -0.0002 (12.17); -0.0085 (0.50) |
| 12-10 | 2,4-dichlor-phenyl | CH2 | CH(CH3) | - | H | [DMSO], Spektrometer: 399.95MHz 8.2004 (2.78); 8.1790 (2.87); 7.6185 (9.67); 7.6042 (10.31); 7.5727 (6.11); 7.5682 (6.35); 7.5573 (0.58); 7.5523 (0.33); 7.3941 (1.81); 7.3735 (10.64); 7.3678 (8.82); 7.3630 (8.00); 7.3471 (1.37); 7.3423 (1.67); 7.1163 (9.94); 7.1021 (9.58); 4.3105 (0.60); 4.2898 (1.42); 4.2735 (1.87); 4.2576 (1.31); 4.2367 (0.64); 3.3379 (793.49); 2.9388 (0.95); 2.9239 (1.21); 2.9045 (4.14); 2.8893 (7.23); 2.8679 (3.64); 2.8546 (1.03); 2.8338 (1.09); 2.6759 (2.15); 2.6713 (3.02); 2.6668 (2.27); 2.5417 (6.08); 2.5248 (8.92); 2.5200 (13.44); 2.5112 (158.61); 2.5068 (325.54); 2.5023 (436.98); 2.4977 (330.10); 2.4933 (167.93); 2.3379 (0.97); 2.3336 (2.09); 2.3290 (2.90); 2.3245 (2.19); 1.1868 (16.00); 1.1702 (15.87); 0.0080 (2.04); -0.0002 (61.12); -0.0085 (2.26) |
| 12-11 | 4-chlor-phenyl | C(CH3)2 | CH2 | - | H | [DMSO], Spektrometer: 399.95MHz 8.1110 (0.51); 8.0956 (1.00); 8.0801 (0.51); 7.5962 (3.37); 7.5820 (3.53); 7.4555 (0.35); 7.4487 (2.98); 7.4436 (1.15); 7.4321 (1.42); 7.4269 (4.94); 7.4203 (0.77); 7.3711 (0.69); 7.3645 (4.97); 7.3593 (1.46); 7.3477 (1.09); 7.3427 (2.99); 7.3360 (0.37); 7.0825 (3.55); 7.0683 (3.41); 3.4141 (3.37); 3.3982 (3.35); 3.3440 (6.40); 2.5433 (2.11); 2.5128 (2.24); 2.5084 (4.44); 2.5039 (5.84); 2.4994 (4.38); 2.4951 (2.20); 1.3075 (16.00); -0.0002 (1.51) |
| 12-12 | 2,4-dichlor-phenyl | C(CH3)2 | CH2 | - | H | [DMSO], Spektrometer: 399.95MHz 8.1079 (0.60); 8.0923 (1.19); 8.0767 (0.60); 7.5849 (3.47); 7.5707 (3.62); 7.5197 (2.71); 7.5139 (2.93); 7.4941 (2.06); 7.4724 (2.84); 7.3706 (1.81); 7.3648 (1.73); 7.3490 (1.33); 7.3432 (1.29); 7.0520 (3.91); 7.0378 (3.78); 3.7589 (3.57); 3.7430 (3.55); 3.3450 (6.00); 2.5443 (2.75); 2.5138 (1.93); 2.5095 (3.90); 2.5050 (5.19); 2.5005 (3.94); 2.4963 (2.03); 1.4603 (16.00); - 0.0002 (1.31) |
| 12-13 | 2-chlor-phenyl | CH2 | CH2 | - | H | [DMSO], Spektrometer: 399.95MHz 8.4127 (2.48); 8.3990 (4.61); 8.3852 (2.47); 7.6280 (15.19); 7.6137 (16.00); 7.4427 (5.76); 7.4388 (5.01); 7.4243 (7.64); 7.4197 (7.42); 7.3771 (4.40); 7.3719 (5.36); 7.3585 (6.21); 7.3539 (7.66); 7.3115 (2.49); 7.3074 (3.25); 7.2932 (7.49); 7.2891 (7.31); 7.2759 (11.33); 7.2705 (10.51); 7.2574 (6.18); 7.2523 (5.81); 7.2388 (2.14); 7.2340 (1.79); 7.1805 (15.75); 7.1662 (14.99); 3.4881 (4.06); 3.4711 (8.61); 3.4557 (8.12); 3.4531 (8.32); 3.4376 (4.61); 3.3496 (21.16); 2.9774 (8.27); 2.9589 (13.18); 2.9413 (7.23); 2.5443 (11.48); 2.5273 (0.45); 2.5137 (7.95); 2.5094 (16.09); 2.5049 (21.36); 2.5004 (16.25); 2.4962 (8.34); -0.0002 (5.49) |
| 12-14 | 3,4-dichlor-phenyl | CH2 | CH2 | - | H | [DMSO], Spektrometer: 399.95MHz 8.3492 (2.08); 8.3354 (3.92); 8.3217 (2.09); 7.6322 (11.80); 7.6179 (12.44); 7.5574 (8.96); 7.5363 (16.00); 7.5302 (9.94); 7.2643 (5.49); 7.2593 (5.34); 7.2438 (4.80); 7.2387 (4.73); 7.1441 (13.25); 7.1298 (12.72); 3.4797 (3.29); 3.4626 (8.30); 3.4479 (8.48); 3.4309 (3.63); 3.3536 (12.70); 2.8534 (6.22); 2.8362 (12.12); 2.8188 (5.64); 2.5468 (8.28); 2.5249 (0.46); 2.5163 (5.42); 2.5120 (10.84); 2.5075 (14.28); 2.5029 (10.66); 2.4986 (5.37); -0.0002 (3.77) |
| 12-15 | 3,5-dichlor-phenyl | CH2 | CH2 | - | H | [DMSO], Spektrometer: 399.95MHz 8.3492 (1.63); 8.3356 (3.08); 8.3218 (1.63); 7.6359 (9.82); 7.6216 (10.28); 7.4403 (3.65); 7.4356 (7.02); 7.4309 (4.37); 7.3343 (16.00); 7.3296 (15.16); 7.1307 (10.96); 7.1165 (10.51); 3.4879 (2.44); 3.4710 (6.52); 3.4563 (6.56); 3.4395 (2.73); 3.3475 (9.73); 2.8644 (4.63); 2.8474 (9.16); 2.8302 (4.22); 2.5452 (0.46); 2.5148 (5.16); 2.5104 (10.48); 2.5059 (13.97); 2.5014 (10.59); 2.4972 (5.44); -0.0002 (4.02) |
| 12-16 | 3-chlor-phenyl | CH2 | CH2 | - | H | [DMSO], Spektrometer: 399.95MHz 8.3626 (2.09); 8.3491 (3.90); 8.3356 (2.16); 7.6299 (12.83); 7.6156 (13.56); 7.3625 (0.43); 7.3479 (3.43); 7.3411 (1.31); 7.3290 (16.00); 7.3096 (8.19); 7.2816 (4.61); 7.2786 (6.59); 7.2738 (4.89); 7.2618 (2.62); 7.2572 (3.02); 7.2539 (2.13); 7.2285 (6.48); 7.2100 (4.52); 7.1617 (0.57); 7.1525 (14.20); 7.1383 (13.63); 3.4730 (3.57); 3.4556 (7.97); 3.4409 (8.00); 3.4232 (4.10); 3.3497 (19.51); 2.8568 (6.66); 2.8390 (12.06); 2.8211 (6.12); 2.5439 (12.22); 2.5270 (0.35); 2.5134 (6.93); 2.5091 (13.86); 2.5046 (18.26); 2.5000 (13.73); 2.4958 (6.97); -0.0002 (5.23) |
| 12-17 | 2-fluor-phenyl | CH2 | CH2 | - | H | [DMSO], Spektrometer: 399.95MHz 8.4105 (1.69); 8.3967 (3.11); 8.3828 (1.70); 7.6274 (10.97); 7.6132 (11.61); 7.3487 (1.78); 7.3447 (2.10); 7.3300 (3.59); 7.3255 (4.34); 7.3097 (2.13); 7.3064 (2.55); 7.3014 (1.24); 7.2967 (1.00); 7.2874 (1.15); 7.2827 (2.44); 7.2681 (2.42); 7.2624 (3.15); 7.2579 (1.77); 7.2487 (1.77); 7.2442 (1.47); 7.1789 (3.54); 7.1696 (11.64); 7.1554 (16.00); 7.1408 (6.06); 7.1379 (5.90); 7.1324 (2.58); 7.1225 (2.63); 7.1195 (2.29); 3.4660 (2.91); 3.4489 (5.89); 3.4334 (5.60); 3.4306 (5.72); 3.4151 (3.26); 3.3508 (9.28); 2.8851 (4.76); 2.8669 (8.04); 2.8487 (4.26); 2.5445 (10.46); 2.5140 (5.33); 2.5097 (10.66); 2.5051 (14.07); 2.5006 (10.63); 2.4963 (5.45); -0.0002 (4.31) |
| 12-18 | 2,6-difluor-phenyl | CH2 | CH2 | - | H | [DMSO], Spektrometer: 399.95MHz 8.4664 (2.25); 8.4520 (4.28); 8.4375 (2.26); 7.6305 (14.40); 7.6162 (15.19); 7.3635 (1.19); 7.3466 (2.69); 7.3427 (2.51); 7.3257 (5.15); 7.3083 (2.69); 7.3049 (3.37); 7.2881 (1.52); 7.1557 (16.00); 7.1414 (15.19); 7.1059 (0.68); 7.1016 (0.98); 7.0893 (7.19); 7.0695 (11.20); 7.0584 (1.62); 7.0495 (6.17); 7.0368 (0.91); 3.4332 (3.52); 3.4165 (8.35); 3.3996 (8.31); 3.3830 (3.90); 3.3493 (15.48); 3.0039 (0.62); 2.9017 (5.53); 2.8839 (9.98); 2.8664 (5.00); 2.5457 (48.45); 2.5291 (0.44); 2.5241 (0.53); 2.5152 (6.36); 2.5109 (13.14); 2.5063 (17.60); 2.5018 (13.32); 2.4974 (6.83); -0.0002 (5.32) |
| 12-19 | 2,6-dichlor-phenyl | CH2 | CH2 | - | H | [DMSO], Spektrometer: 399.95MHz 8.4813 (5.51); 8.4717 (6.76); 7.6432 (4.18); 7.6408 (4.68); 7.6373 (4.42); 7.6306 (11.07); 7.6165 (8.63); 7.4771 (8.04); 7.4674 (13.54); 7.4605 (11.53); 7.4577 (11.13); 7.4473 (16.00); 7.3162 (2.79); 7.3138 (3.13); 7.3034 (5.48); 7.2951 (5.19); 7.2929 (4.95); 7.2840 (7.19); 7.2634 (3.36); 7.2160 (4.84); 7.2136 (5.48); 7.2102 (5.22); 7.2039 (11.34); 7.1997 (7.87); 7.1901 (8.73); 3.4516 (11.11); 3.4373 (10.24); 3.4202 (4.23); 3.3591 (7.73); 3.3569 (7.79); 3.3501 (14.35); 3.1542 (12.93); 3.1373 (6.04); 2.5588 (3.42); 2.5564 (3.78); 2.5531 (3.35); 2.5493 (4.50); 2.5466 (5.87); 2.5116 (26.16); 0.0128 (1.90); 0.0103 (2.09); 0.0069 (1.81); 0.0031 (2.34); -0.0002 (2.93) |
| 12-20 | 3-(trifluormethyl)-phenyl | CH2 | CH2 | - | H | [DMSO], Spektrometer: 399.95MHz 8.3732 (2.25); 8.3596 (4.09); 8.3459 (2.18); 7.6733 (0.40); 7.6533 (0.57); 7.6414 (0.52); 7.6293 (14.31); 7.6150 (16.00); 7.6061 (8.70); 7.5919 (2.71); 7.5885 (2.82); 7.5824 (3.33); 7.5670 (13.64); 7.5542 (9.11); 7.5429 (2.88); 7.5367 (2.18); 7.5178 (1.30); 7.4960 (0.47); 7.4759 (0.38); 7.1613 (0.56); 7.1470 (0.66); 7.1370 (14.42); 7.1227 (13.77); 3.5114 (3.59); 3.4940 (8.81); 3.4794 (8.80); 3.4620 (4.13); 3.3522 (15.80); 2.9556 (6.78); 2.9380 (12.80); 2.9204 (6.09); 2.5461 (16.30); 2.5292 (0.41); 2.5244 (0.61); 2.5157 (7.12); 2.5113 (14.54); 2.5068 (19.44); 2.5022 (14.72); 2.4979 (7.52); -0.0002 (6.35) |
| 12-21 | 4-(trifluormethyl)-phenyl | CH2 | CH2 | - | H | [DMSO], Spektrometer: 399.95MHz 8.3905 (2.28); 8.3768 (4.30); 8.3631 (2.28); 7.6732 (10.42); 7.6531 (12.81); 7.6305 (14.27); 7.6162 (15.04); 7.4955 (11.97); 7.4755 (9.94); 7.1598 (16.00); 7.1455 (15.29); 3.5068 (3.65); 3.4894 (8.31); 3.4746 (8.25); 3.4569 (4.11); 3.3486 (17.26); 2.9447 (6.12); 2.9269 (11.15); 2.9091 (5.42); 2.5456 (0.65); 2.5287 (0.50); 2.5239 (0.76); 2.5152 (8.81); 2.5108 (17.77); 2.5062 (23.54); 2.5017 (17.68); 2.4973 (8.95); -0.0002 (8.01) |
| 12-22 | 2-methyl-phenyl | CH2 | CH2 | - | H | [DMSO], Spektrometer: 399.95MHz 8.4223 (0.79); 8.4084 (1.48); 8.3945 (0.80); 7.6351 (4.68); 7.6209 (4.99); 7.2019 (5.30); 7.1876 (5.10); 7.1795 (1.17); 7.1714 (1.73); 7.1677 (1.68); 7.1561 (3.52); 7.1462 (2.76); 7.1407 (1.34); 7.1286 (3.25); 7.1236 (3.88); 7.1150 (3.97); 7.1072 (1.73); 7.1056 (1.79); 7.1024 (1.59); 7.0897 (0.34); 3.4021 (1.35); 3.3869 (2.36); 3.3788 (1.50); 3.3687 (1.99); 3.3639 (2.51); 3.3479 (7.52); 2.8379 (2.66); 2.8180 (3.12); 2.7994 (2.37); 2.5420 (3.48); 2.5114 (2.36); 2.5072 (4.75); 2.5027 (6.30); 2.4982 (4.76); 2.4941 (2.44); 2.3229 (16.00); -0.0002 (1.93) |
| 12-23 | 2,4,6-trimethyl-phenyl | CH2 | CH2 | - | H | [DMSO], Spektrometer: 399.95MHz 8.5114 (0.56); 8.4970 (1.11); 8.4825 (0.58); 7.6494 (2.65); 7.6352 (2.84); 7.2473 (2.77); 7.2330 (2.63); 6.8076 (5.31); 3.3461 (4.45); 3.2423 (0.67); 3.2276 (1.18); 3.2139 (1.17); 3.2070 (1.00); 3.2006 (1.27); 3.1866 (0.81); 2.8069 (1.42); 2.7936 (1.11); 2.7858 (1.44); 2.7809 (1.23); 2.7659 (1.21); 2.5424 (3.52); 2.5075 (3.25); 2.5031 (4.33); 2.4989 (3.39); 2.3006 (16.00); 2.1828 (8.30); -0.0002 (1.07) |
| 12-24 | 3,4-bismethoxy-phenyl | CH2 | CH2 | - | H | [DMSO], Spektrometer: 399.95MHz 8.3289 (0.63); 8.3153 (1.23); 8.3016 (0.64); 7.6315 (3.39); 7.6172 (3.58); 7.1806 (3.65); 7.1663 (3.47); 6.8748 (2.32); 6.8544 (3.19); 6.8313 (2.61); 6.8267 (2.95); 6.7596 (1.68); 6.7550 (1.56); 6.7393 (1.22); 6.7347 (1.15); 3.7316 (16.00); 3.7117 (16.00); 3.4332 (0.90); 3.4165 (1.79); 3.3974 (1.80); 3.3820 (1.00); 3.3407 (7.66); 2.7700 (1.76); 2.7510 (2.68); 2.7331 (1.59); 2.5419 (0.54); 2.5112 (3.87); 2.5071 (7.71); 2.5026 (10.21); 2.4982 (7.74); - 0.0002 (2.88) |
| 12-25 | phenyl | CH2 | CH2 | - | H | [DMSO], Spektrometer: 399.95MHz 8.3699 (2.08); 8.3564 (3.75); 8.3429 (2.10); 7.6266 (14.06); 7.6123 (14.88); 7.3222 (4.38); 7.3185 (1.98); 7.3038 (11.81); 7.2904 (3.91); 7.2859 (12.24); 7.2617 (11.43); 7.2578 (15.34); 7.2410 (7.56); 7.2272 (3.32); 7.2236 (4.14); 7.2199 (2.35); 7.2108 (2.48); 7.2059 (6.20); 7.2002 (1.68); 7.1921 (1.59); 7.1881 (2.32); 7.1844 (1.48); 7.1769 (16.00); 7.1626 (15.25); 3.4580 (4.10); 3.4419 (6.80); 3.4403 (6.74); 3.4255 (6.55); 3.4214 (7.14); 3.4065 (4.58); 3.3472 (15.79); 2.8433 (7.45); 2.8240 (10.47); 2.8060 (6.70); 2.5419 (5.14); 2.5250 (0.37); 2.5115 (7.00); 2.5071 (14.03); 2.5026 (18.53); 2.4980 (13.81); 2.4936 (6.90); -0.0002 (6.21) |
| 12-26 | 4-chlor-phenyl | C(CH2-CH2) | CH2 | - | H | |
| 12-27 | 2,4-dichlor-phenyl | C(CH2-CH2) | CH2 | - | H | |
| 12-28 | 4-chlor-phenyl | CH2 | C(CH2-CH2) | - | H | |
| 12-29 | 2,4-dichlor-phenyl | CH2 | C(CH2-CH2) | - | H | |
| 12-30 | 4-chlor-phenyl | O | CH2 | CH2 | H | |
| 12-31 | 2,4-dichlor-phenyl | O | CH2 | CH2 | H | Verbindung Nr. 12-31, Solvent: [DMSO], Spektrometer: 399.95MHz 8,4586 (2,37); 8,4454 (4,52); 8,4321 (2,46); 7,6461 (12,06); 7,6319 (12,91); 7,5719 (10,04); 7,5654 (11,06); 7,386 (5,31); 7,3795 (5,1); 7,3638 (7,1); 7,3574 (7,07); 7,2393 (12,88); 7,2345 (15,08); 7,2199 (15,12); 4,2102 (7,19); 4,1955 (16); 4,181 (7,99); 3,6247 (4,03); 3,6104 (11,36); 3,5962 (11,04); 3,5818 (3,73); 3,3308 (89,55); 2,6724 (0,35); 2,5426 (64,52); 2,5253 (1,19); 2,5075 (39,39); 2,5031 (53); 2,4987 (41,18); 2,33 (0,34); -0,0002 (1,92) |
| 12-32 | 4-chlor-phenyl | CH2 | CH2 | CH2 | H | |
| 12-33 | 4-chlor-phenyl | NCH3 | CH2 | CH2 | H | |
| 12-34 | 2,4-dichlor-phenyl | NCH3 | CH2 | CH2 | H | |
| 12-35 | 4-chlor-phenyl | CH(OCH3) | CH2 | - | H | |
| 12-36 | 2,4-dichlor-phenyl | CH(OCH3) | CH2 | - | H | |
| 12-37 | 2-thienyl | CH2 | CH2 | - | H | Verbindung Nr. 12-37, Solvent: [DMSO], Spektrometer: 399.95MHz 8,4262 (2,73); 8,413 (4,89); 8,3996 (2,79); 7,6376 (13,67); 7,6233 (14,65); 7,3506 (7,69); 7,3483 (7,69); 7,338 (8,41); 7,3355 (8,13); 7,2098 (15,14); 7,1955 (14,48); 6,9721 (6,1); 6,9635 (9,35); 6,9596 (6,31); 6,9509 (8,87); 6,9273 (9,83); 6,9205 (6,8); 3,4749 (4,72); 3,4571 (10,55); 3,4423 (10,87); 3,4245 (5,66); 3,3326 (27,99); 3,0595 (9,29); 3,0414 (16); 3,0234 (8); 2,5408 (9,54); 2,5056 (22,54); 2,5013 (29,54); 2,497 (22,77); -0,0002 (0,5) |
| 12-38 | 3-thienyl | CH2 | CH2 | - | H | |
| 12-39 | 2-furyl | CH2 | CH2 | - | H | |
| 12-40 | 3-furyl | CH2 | CH2 | - | H | |
| 12-41 | phenyl | CH2 | CH2 | CH(CH3) | H | |
| 12-42 | phenyl | CH2 | CH2 | CH2 | H | |
| 12-43 | 2-Cl-phenyl | CH2 | CH2 | CH(CH3) | H | |
| 12-44 | 4-t-butyl-phenyl | CH2 | CH2 | CH2 | H | |
| 12-45 | 4-methyl-phenyl | CH2 | CH2 | CH2 | H | |
| 12-46 | phenyl | CH2 | CH2 | CH(CH2CH3) | H | |
| 12-47 | 2-methoxy-phenyl | CH2 | CH2 | CH2 | H | |
| 12-48 | 2-methyl-phenyl | CH2 | CH2 | CH2 | H | |
| 12-49 | 3-methyl-phenyl | CH2 | CH2 | CH2 | H | |
| 12-50 | 3-chlor-phenyl | CH2 | CH2 | CH2 | H | |
| 12-51 | 2,6-difluor-phenyl | CH2 | CH2 | CH2 | H | Verbindung Nr. 12-51, Solvent: [DMSO], Spektrometer: 399.95MHz 8,3439 (3,57); 8,3309 (6,36); 8,3178 (3,69); 7,6616 (0,33); 7,6471 (0,66); 7,634 (13,3); 7,6212 (13,4); 7,6198 (14,21); 7,3509 (1,47); 7,333 (4,06); 7,3131 (6,87); 7,2937 (4,81); 7,2758 (2,27); 7,2618 (0,44); 7,197 (15,35); 7,1827 (14,55); 7,1425 (0,32); 7,1208 (0,5); 7,1009 (1,56); 7,0882 (10,47); 7,0686 (16); 7,0493 (8,79); 7,0349 (1,36); 6,5486 (0,63); 3,3361 (29,78); 3,2664 (5,24); 3,2496 (11,89); 3,2332 (12,11); 3,2164 (5,54); 2,7079 (7,58); 2,6887 (12,66); 2,6694 (8,51); 2,5446 (9,47); 2,5056 (37,84); 1,7988 (2,81); 1,7801 (7,74); 1,7614 (10,65); 1,7426 (7,42); 1,7243 (2,52); -0,0002 (0,54) |
| 12-52 | 4-chlor-phenyl | CH2 | CH2 | CH2 | H | |
| 12-53 | 2,6-dichlor-phenyl | CH2 | CH2 | CH(CH3) | H | |
| 12-54 | 3,5-dichlor-phenyl | CH2 | CH2 | CH(CH3) | H | |
| 12-55 | 2,6-dimethyl-phenyl | CH2 | CH2 | CH2 | H | |
| 12-56 | 2,5-dichlor-phenyl | CH2 | CH2 | CH2 | H | |
| 12-57 | 4-i-propoxy-phenyl | CH2 | CH2 | CH2 | H | |
| 12-58 | 3-trifluormethyl-phenyl | CH2 | CH2 | CH(CH3) | H | |
| 12-59 | 4-methyl-phenyl | CH2 | CH2 | CH(CH3) | H | |
| 12-60 | 2-methyl-phenyl | CH2 | CH2 | CH(CH3) | H | |
| 12-61 | 3,4-dichlor-phenyl | CH2 | CH2 | CH(CH3) | H | |
| 12-62 | 3,5-dichlor-phenyl | CH2 | CH2 | CH2 | H | |
| 12-63 | 2,6-dimethyl-phenyl | CH2 | CH2 | CH(CH3) | H | |
| 12-64 | 4-trifluormethyl-phenyl | CH2 | CH2 | CH(CH3) | H | |
| 12-65 | 2,5-dichlor-phenyl | CH2 | CH2 | CH(CH3) | H | |
| 12-66 | 4-phenoxy-phenyl | CH2 | CH2 | CH2 | H | |
| 12-67 | 3-chlor-phenyl | CH2 | CH2 | CH(CH3) | H | |
| 12-68 | 4-phenoxy-phenyl | CH2 | CH2 | CH(CH3) | H | |
| 12-69 | 2,4-dichlor-phenyl | CH2 | CH2 | CH(CH3) | H | |
| 12-70 | 2-difluormethoxy-phenyl | CH2 | CH2 | CH(CH3) | H | |
| 12-71 | 4-methoxy-phenyl | CH2 | CH2 | CH2 | H | |
| 12-72 | 4-chlor-phenyl | CH2 | CH2 | CH(CH3) | H | |
| 12-73 | 4-chlor-phenyl | CH2 | CH2 | CH(i-propyl) | H | |
| 12-74 | 4-fluor-phenyl | CH2 | CH2 | CH2 | H | |
| 12-75 | 4-chlor-phenyl | CH2 | CH2 | CH(n-propyl) | H | |
| 12-76 | 4-chlor-phenyl | CH2 | CH2 | CH(t-butyl) | H | |
| 12-77 | 2-chlor-phenyl | CH2 | CH2 | CH2 | H | |
| 12-78 | 4-chlor-phenyl | CH2 | CH(CH3) | - | cyclopropyl | WO-A 2007/060164 |
| 12-79 | 2-thienyl | CH2 | CH2 | CH2 | H | Verbindung Nr. 12-79, Solvent: [DMSO], Spektrometer: 399.95MHz 8,3298 (2,96); 8,3169 (5,26); 8,3042 (3,1); 7,6325 (14,47); 7,6183 (15,49); 7,3211 (8,42); 7,3186 (7,65); 7,3083 (9,3); 7,3058 (8,14); 7,2108 (16); 7,1966 (15,18); 6,9528 (6,49); 6,9442 (9,31); 6,9403 (6,93); 6,9316 (8,34); 6,8914 (10,08); 6,8833 (8,16); 3,328 (186,81); 3,2777 (5,37); 3,2609 (12,9); 3,2458 (13,23); 3,229 (5,65); 2,8831 (9,11); 2,864 (15,57); 2,8449 (9,91); 2,6708 (0,98); 2,5408 (3,11); 2,5058 (106,56); 2,5016 (136,82); 2,4976 (105,49); 2,3285 (0,9); 1,8804 (2,82); 1,8625 (8,73); 1,8438 (11,62); 1,8255 (8,42); 1,8075 (2,56); -0,0002 (1,36) |
| 12-80 | 5-chlor-2-thienyl | CH2 | CH2 | - | H | Verbindung Nr. 12-80, Solvent: [DMSO], Spektrometer: 399.95MHz 8,432 (1,82); 8,4184 (3,38); 8,4047 (1,79); 8,3155 (1,88); 7,6414 (15,17); 7,6271 (16); 7,3751 (14,36); 7,3713 (14,43); 7,3638 (0,37); 7,2076 (0,36); 7,1958 (15,71); 7,1815 (14,83); 6,9256 (9,98); 6,9239 (8,92); 6,922 (9,73); 3,4729 (3,5); 3,4555 (8,91); 3,441 (9,18); 3,4239 (4,02); 3,3222 (44,18); 3,0393 (0,33); 3,0233 (6,68); 3,0058 (12,63); 2,9887 (5,62); 2,6753 (0,39); 2,6707 (0,53); 2,6661 (0,38); 2,5239 (1,8); 2,5191 (2,81); 2,5106 (32,21); 2,5062 (64,19); 2,5016 (83,82); 2,497 (59,36); 2,4924 (27,72); 2,3329 (0,4); 2,3283 (0,55); 2,3237 (0,41); 1,9887 (0,99); 1,3357 (0,35); 1,2494 (0,43); 1,1749 (0,53); 0,1459 (0,33); 0,008 (2,83); -0,0002 (78,68); -0,0085 (2,37); -0,1496 (0,33) |
| 12-81 | 4-chlor-2-thienyl | CH2 | CH2 | - | H | Verbindung Nr. 12-81, Solvent: [DMSO], Spektrometer: 601.6MHz 8,4249 (1,78); 8,4158 (3,3); 8,4066 (1,74); 7,639 (15,4); 7,6295 (16); 7,3751 (13,88); 7,3725 (13,92); 7,1923 (15,77); 7,1828 (15,35); 6,9252 (9,32); 6,924 (8,25); 6,9228 (9,13); 3,464 (3,67); 3,4524 (8,92); 3,4428 (9,08); 3,4314 (4,07); 3,3535 (0,49); 3,3256 (836,97); 3,3031 (0,84); 3,0173 (5,99); 3,0056 (11,69); 2,9947 (5,41); 2,6531 (1,58); 2,6192 (0,57); 2,6162 (1,19); 2,6131 (1,66); 2,61 (1,22); 2,607 (0,58); 2,5407 (486,63); 2,5224 (3,63); 2,5193 (4,8); 2,5161 (5,78); 2,5075 (92,82); 2,5044 (192,64); 2,5014 (263,85); 2,4983 (189,46); 2,4953 (88,15); 2,4245 (1,58); 2,3916 (0,56); 2,3886 (1,18); 2,3855 (1,63); 2,3825 (1,15); 2,3795 (0,51); 2,0735 (0,57); 0,0053 (0,56); - 0,0002 (15,95); -0,0057 (0,45) |
| 12-82 | 4-chlor-phenyl | N(CH3) | CH2 | CH2 | H | Verbindung Nr. 12-82, Solvent: [DMSO], Spektrometer: 399.95MHz 8,2813 (0,73); 8,2674 (1,4); 8,2535 (0,75); 7,8316 (3,37); 7,8264 (3,35); 7,1794 (4,23); 7,157 (4,61); 7,1488 (0,56); 6,8767 (3,52); 6,8715 (3,44); 6,7524 (4,57); 6,7299 (4,09); 3,4651 (1,31); 3,4485 (3,06); 3,4314 (2,35); 3,3556 (1,46); 3,3279 (46,87); 2,9073 (16); 2,5415 (20,17); 2,5064 (30,6); 2,5024 (38,46); -0,0002 (4,87) |
| 12-83 | 3-(trifluormethyl)-phenyl | O | CH2 | CH(CH2CH3) | H | Verbindung Nr. 12-83, Solvent: [DMSO], Spektrometer: 399.95MHz 8,3165 (0,38); 8,1187 (2,84); 8,099 (2,84); 7,8058 (6,33); 7,7928 (6,56); 7,785 (1,18); 7,5428 (1,9); 7,5232 (4,2); 7,5036 (2,82); 7,4898 (0,46); 7,3461 (0,34); 7,2982 (6,29); 7,2784 (5,13); 7,2686 (8,11); 7,1795 (6,69); 7,1665 (6,51); 7,15 (0,77); 4,2211 (0,85); 4,2081 (1,31); 4,2001 (1,72); 4,1867 (2,03); 4,1687 (3,2); 4,1455 (4,52); 4,1306 (3,15); 4,1162 (3,62); 4,1033 (3,3); 4,0927 (2,02); 4,0795 (1,14); 3,3274 (85,66); 2,6715 (0,48); 2,5421 (8,14); 2,5025 (75,15); 2,4986 (60,82); 2,329 (0,51); 1,7771 (0,84); 1,7587 (1,27); 1,7434 (1,92); 1,7301 (1,65); 1,7247 (1,75); 1,712 (1,37); 1,6939 (0,56); 1,6778 (0,34); 1,6605 (0,5); 1,6457 (1,3); 1,6263 (1,94); 1,6077 (1,72); 1,5919 (1,41); 1,5727 (0,89); 0,9905 (8,04); 0,9722 (16); 0,9536 (7,45); 0,9419 (1,2); -0,0002 (9,15); -0,0018 (7,67) |

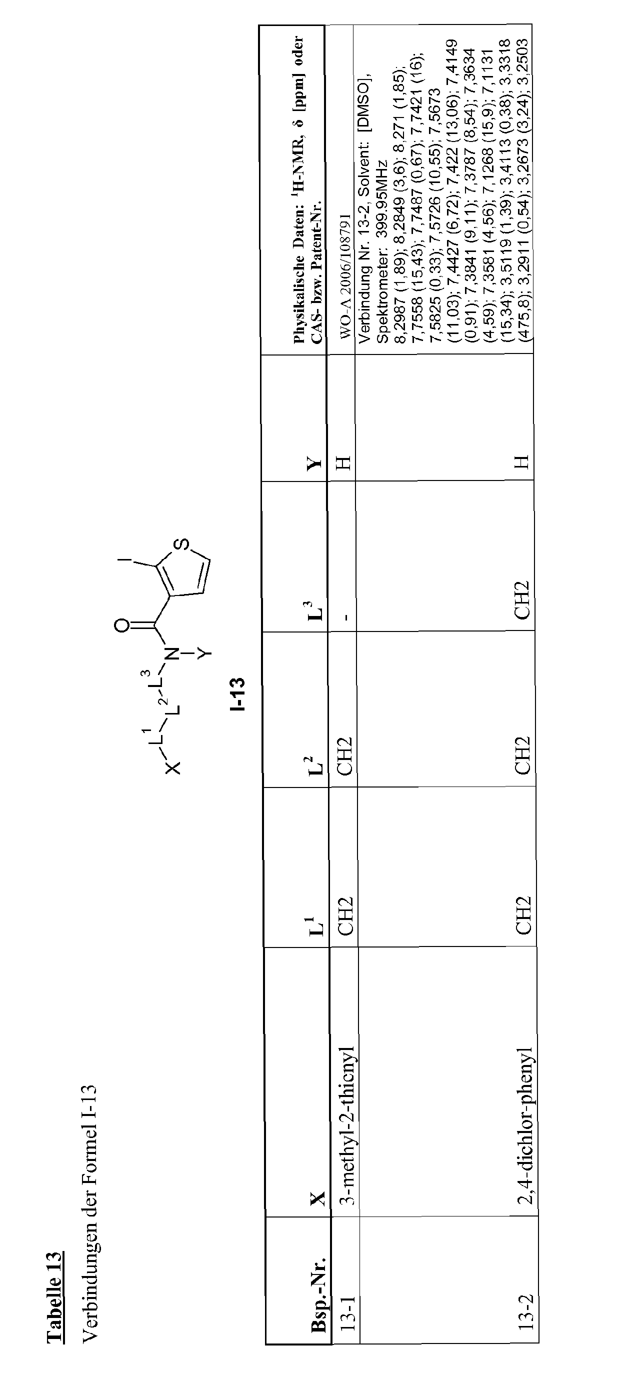

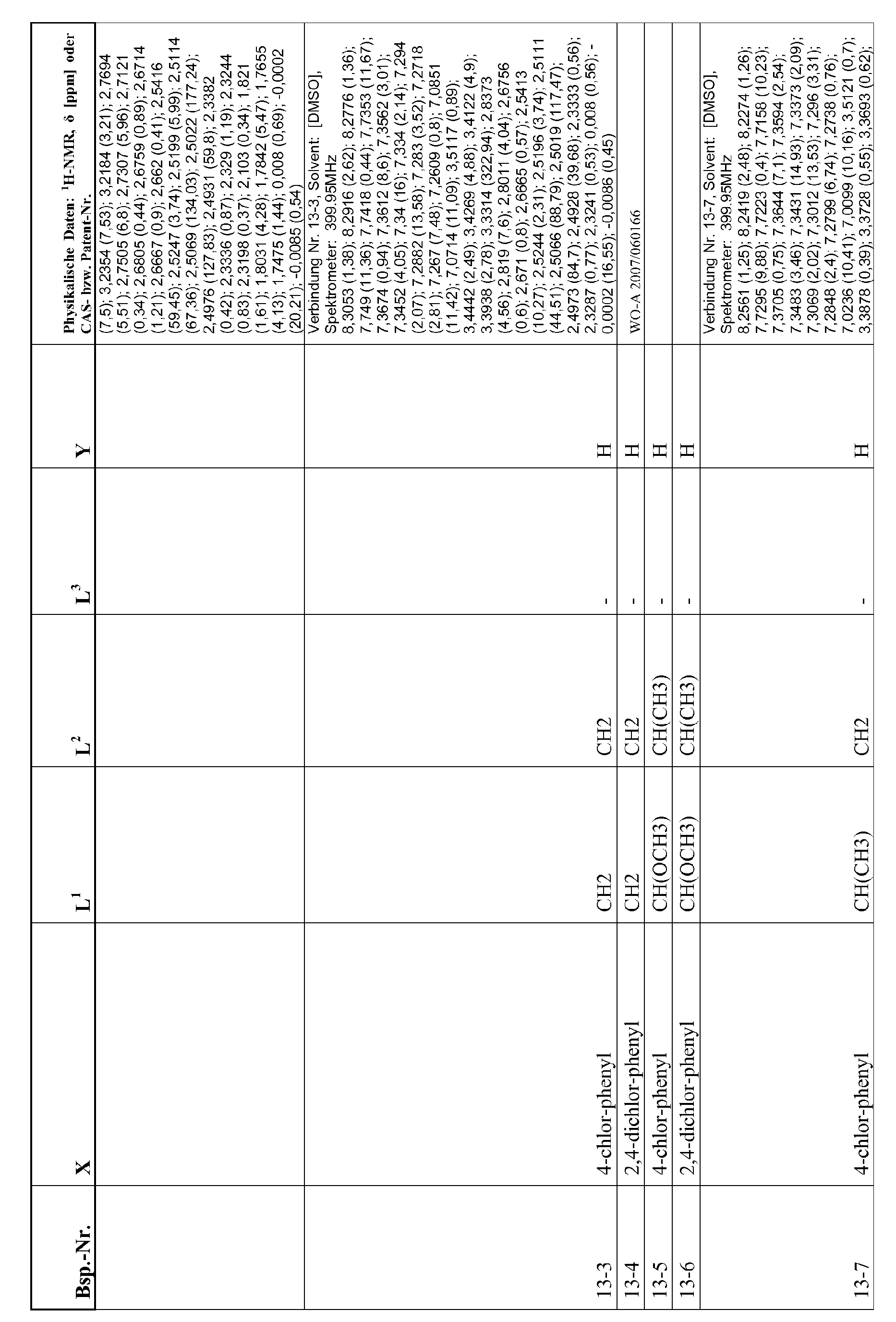

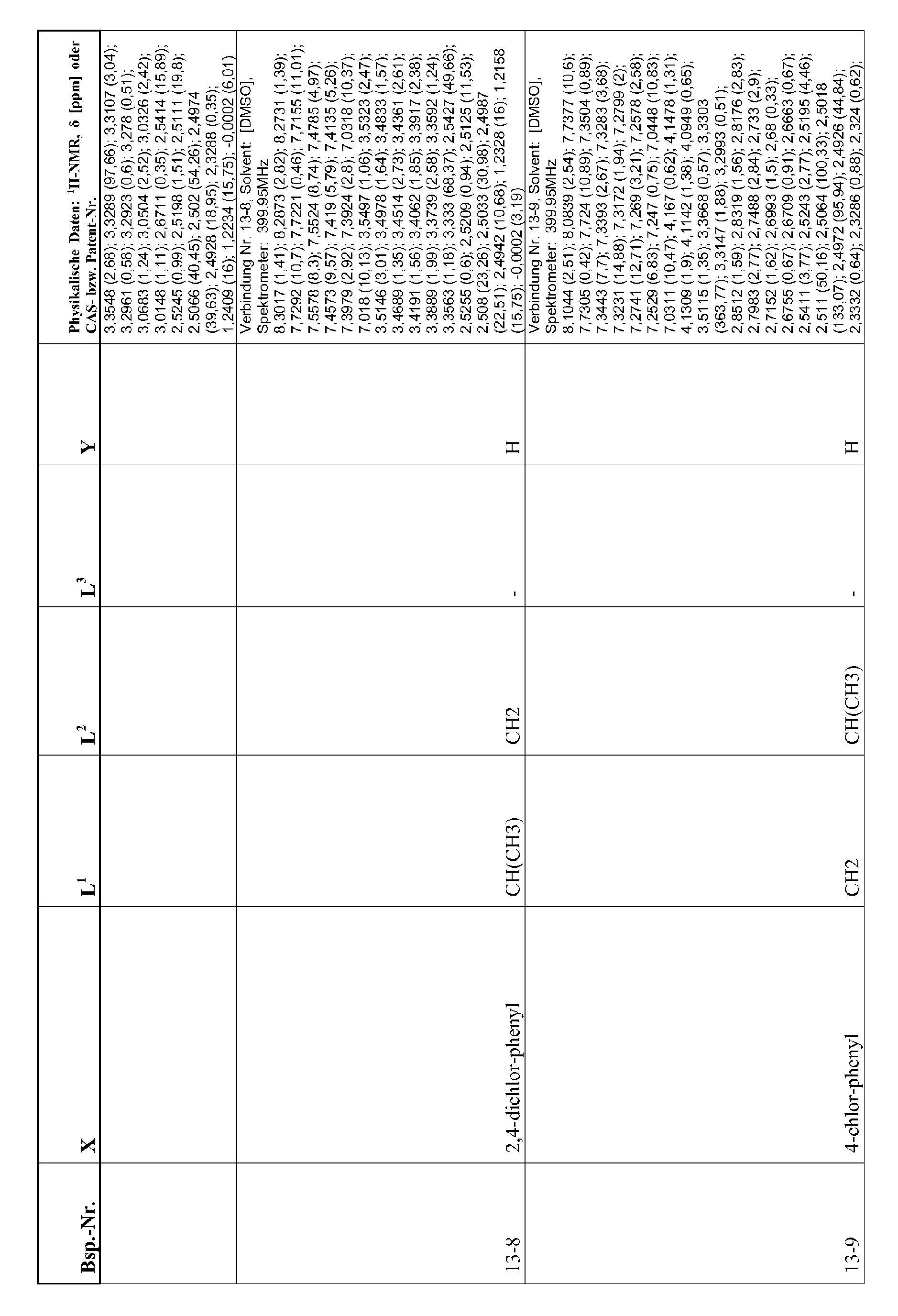

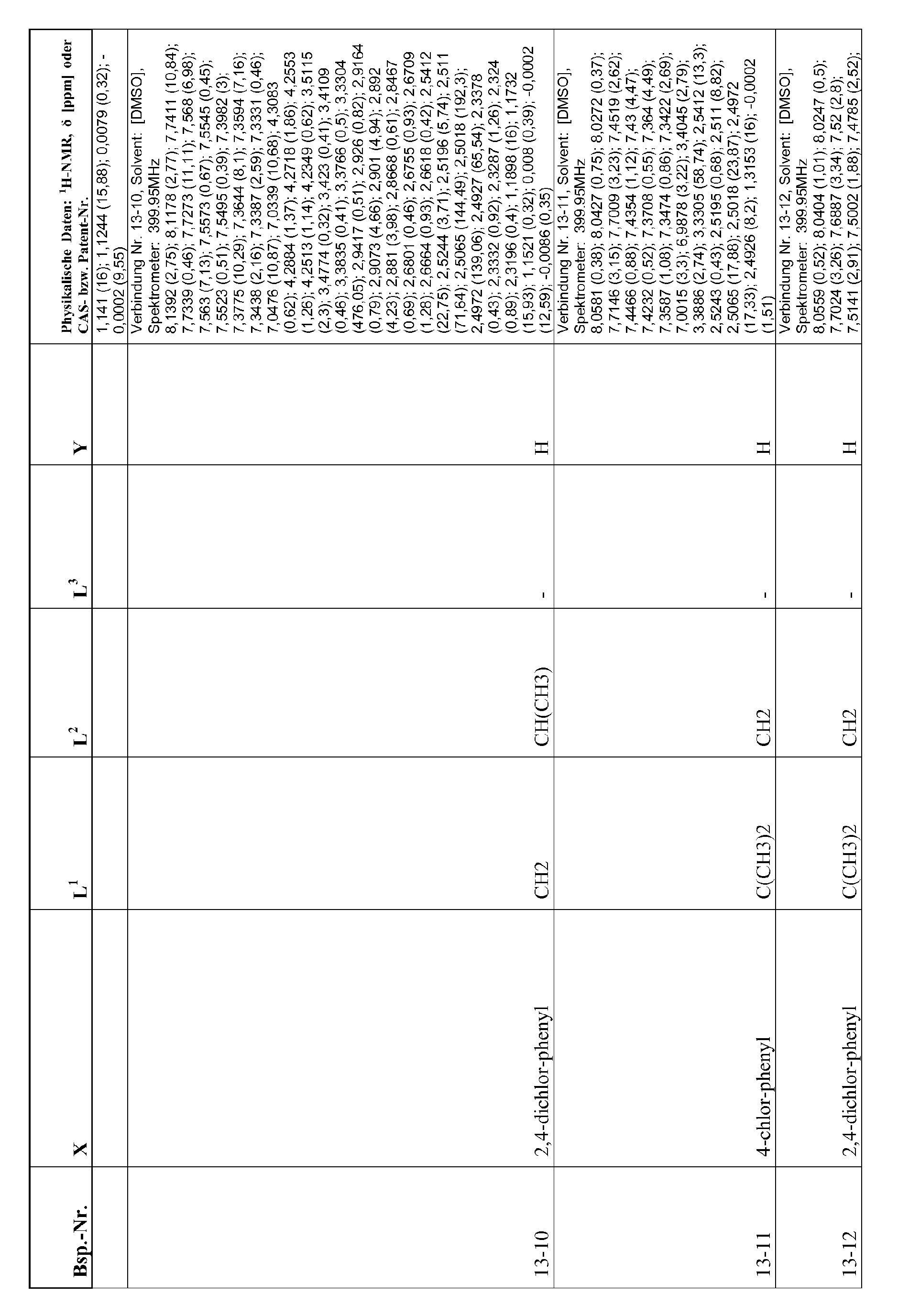

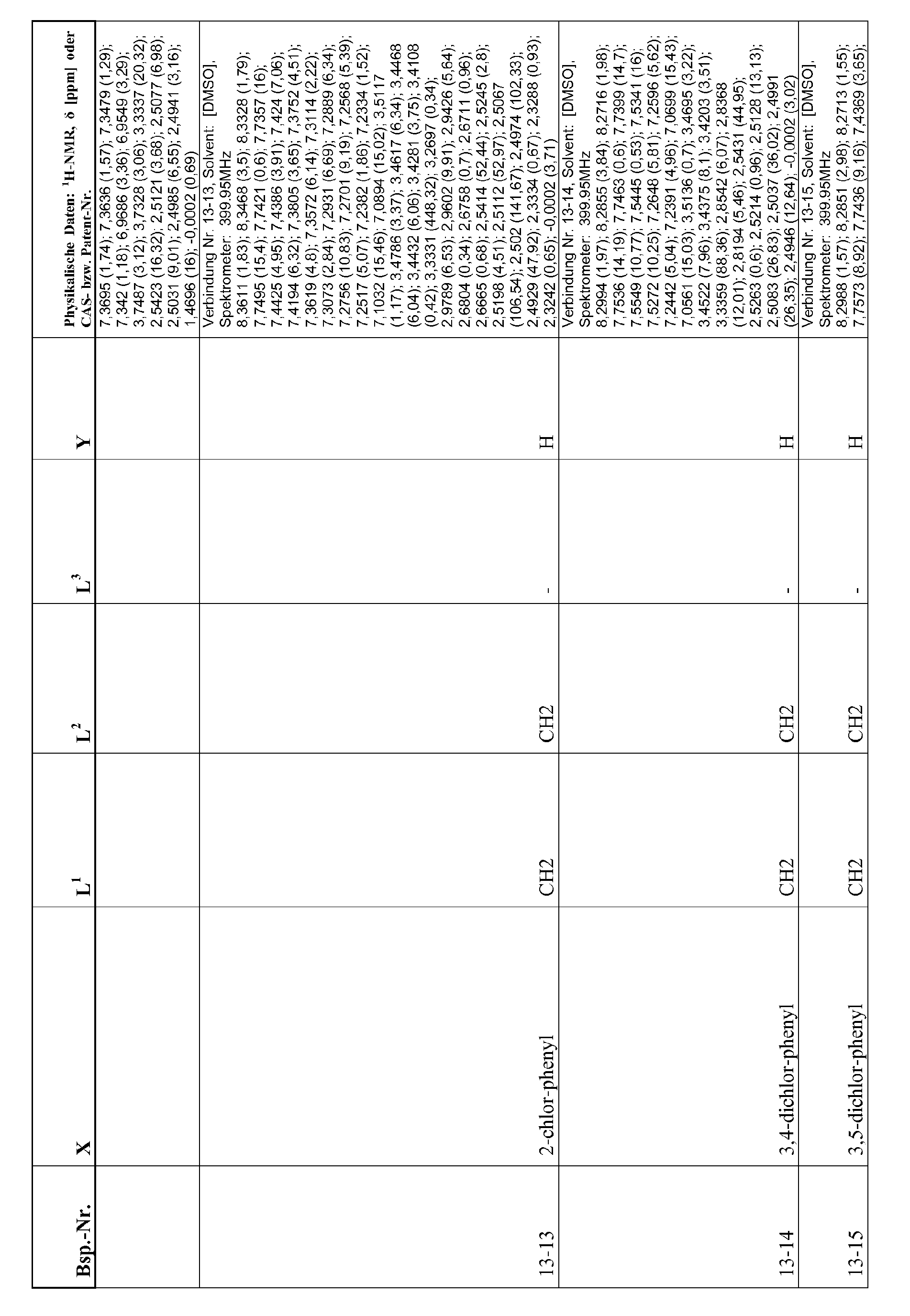

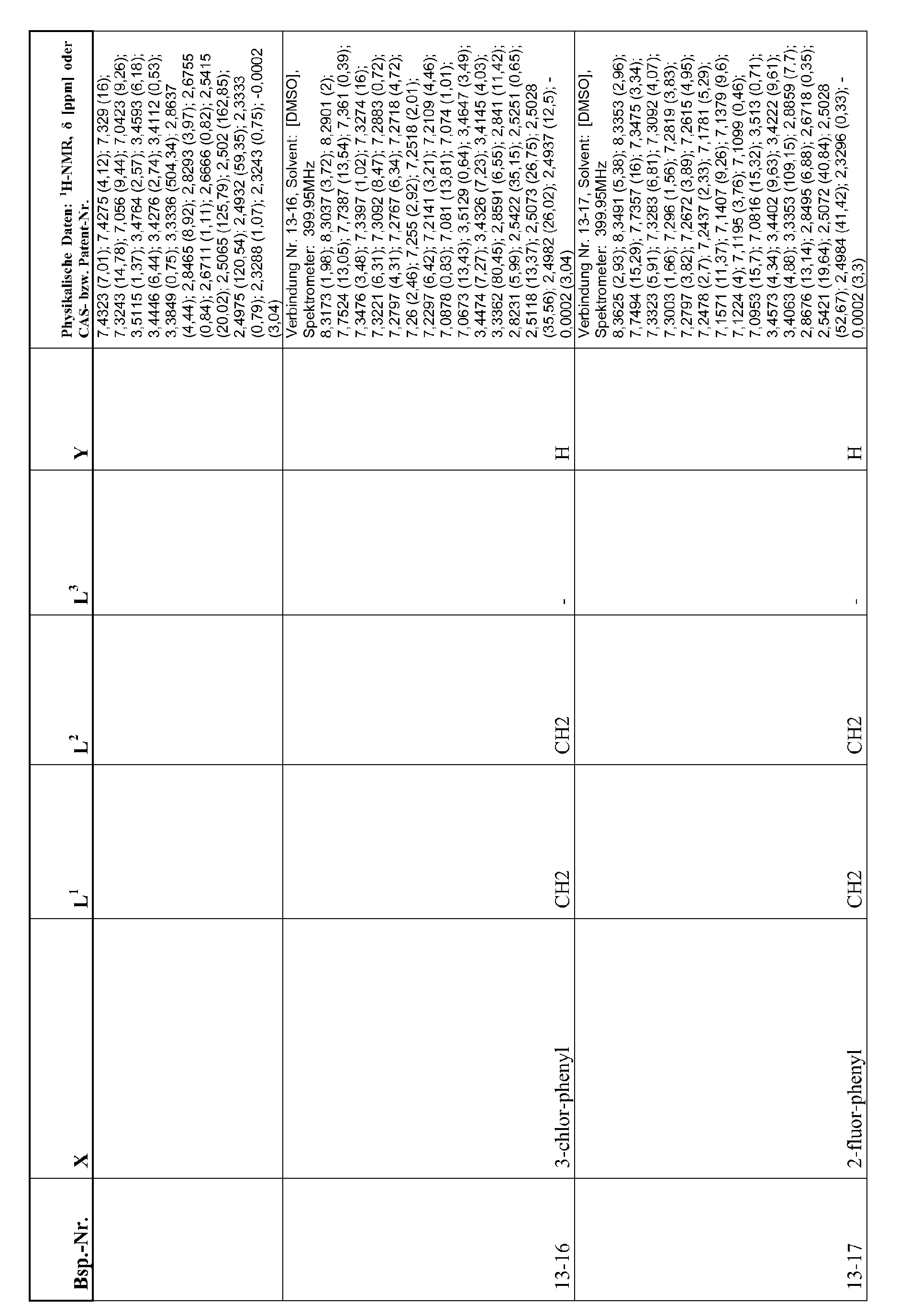

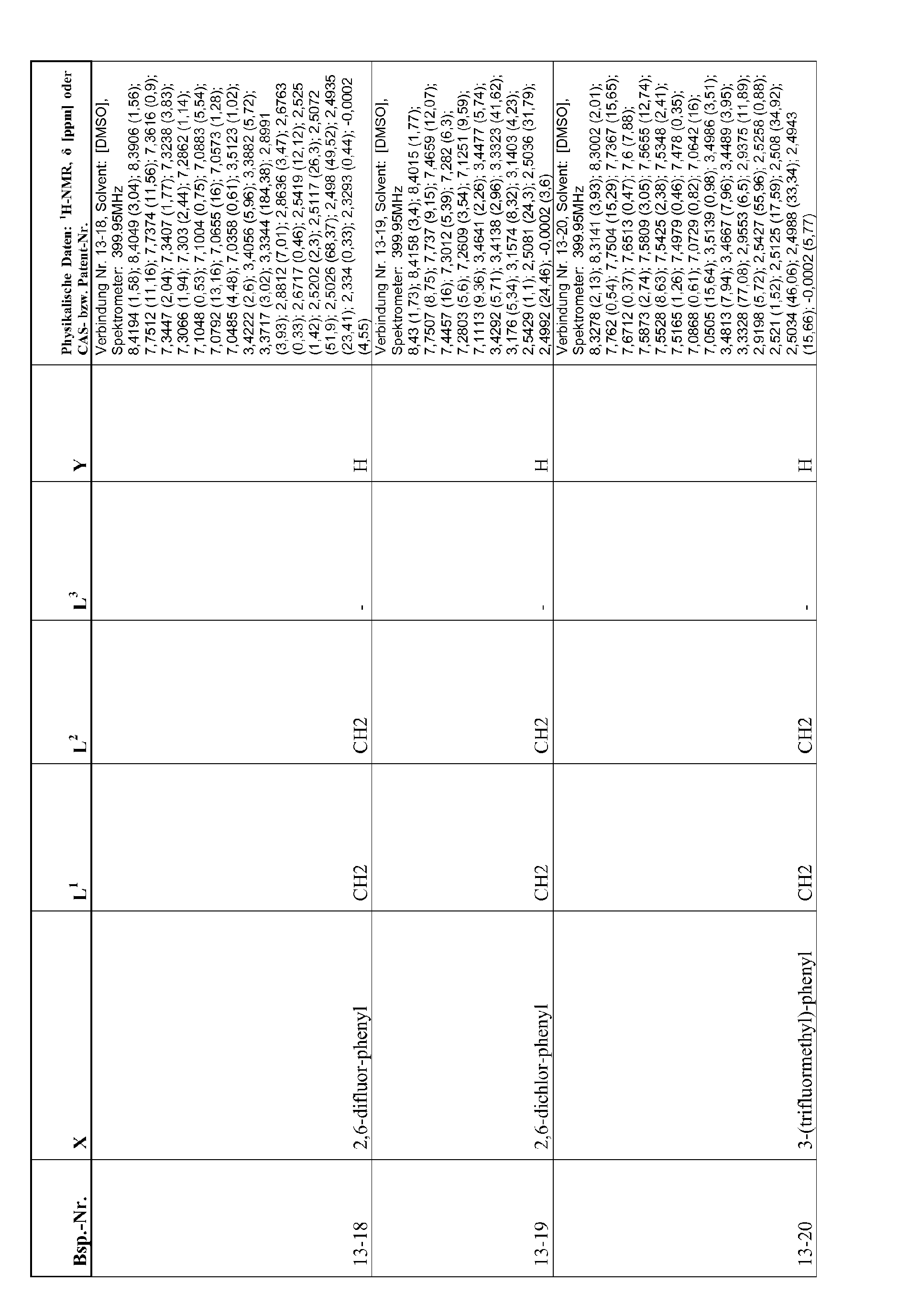

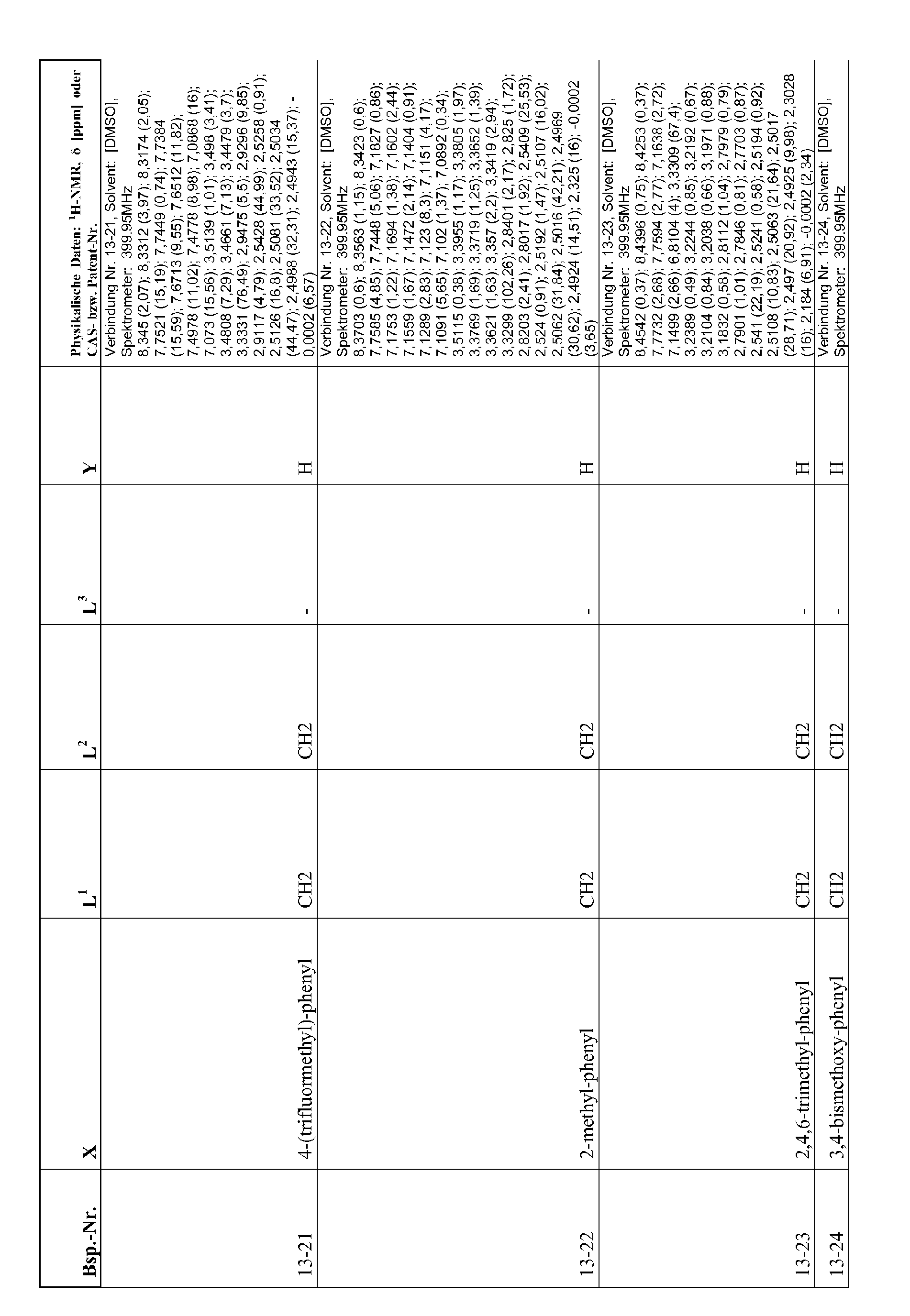

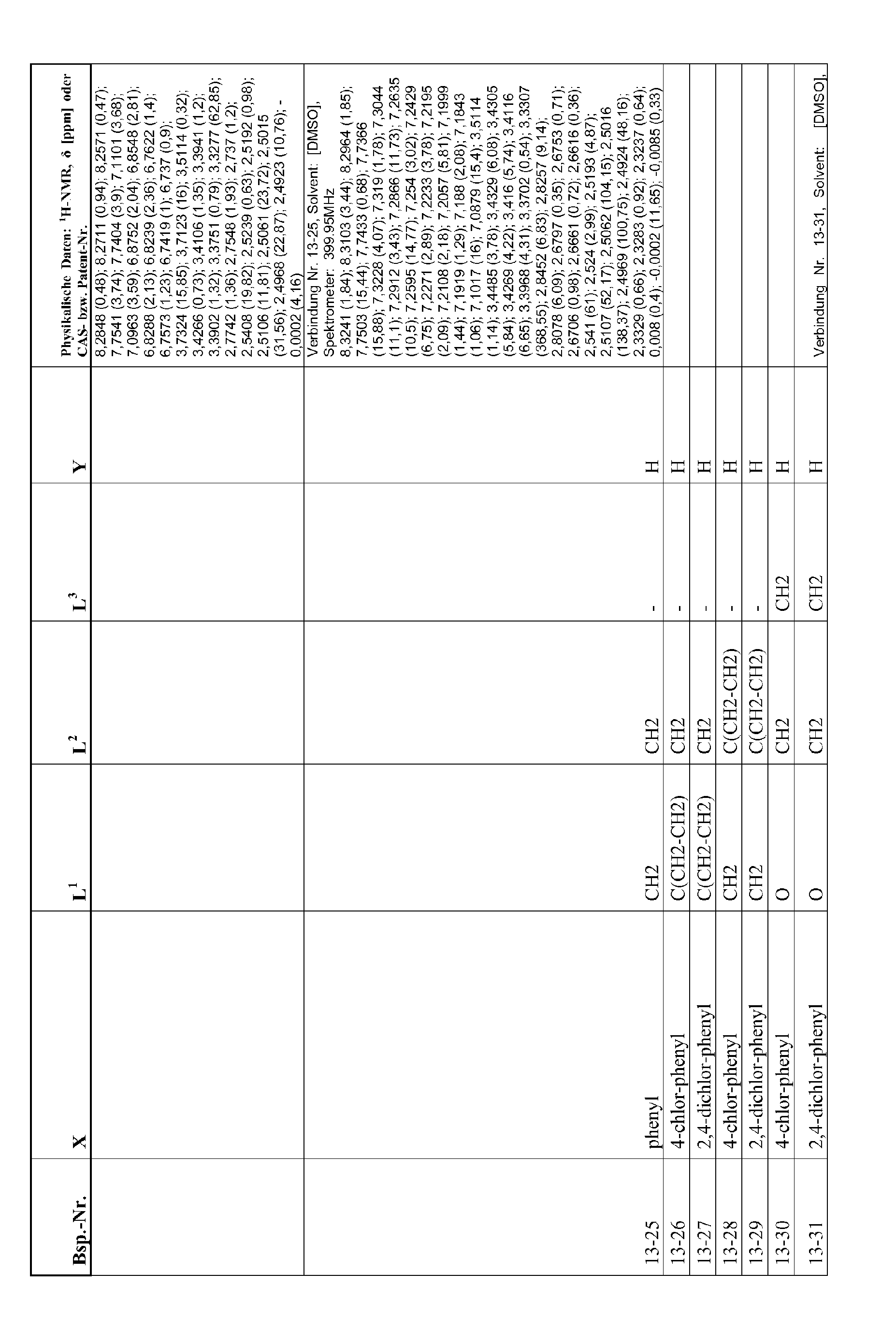

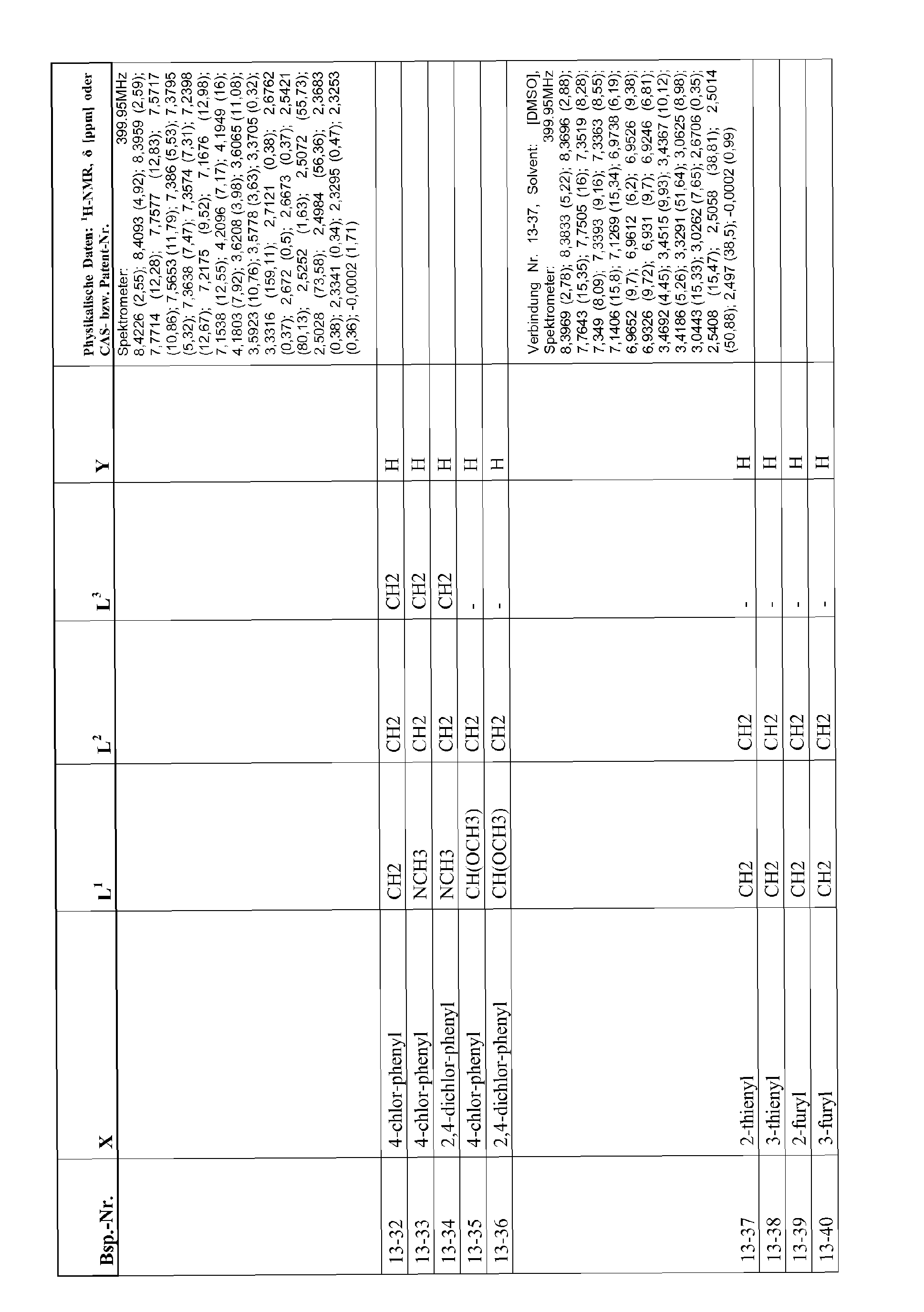

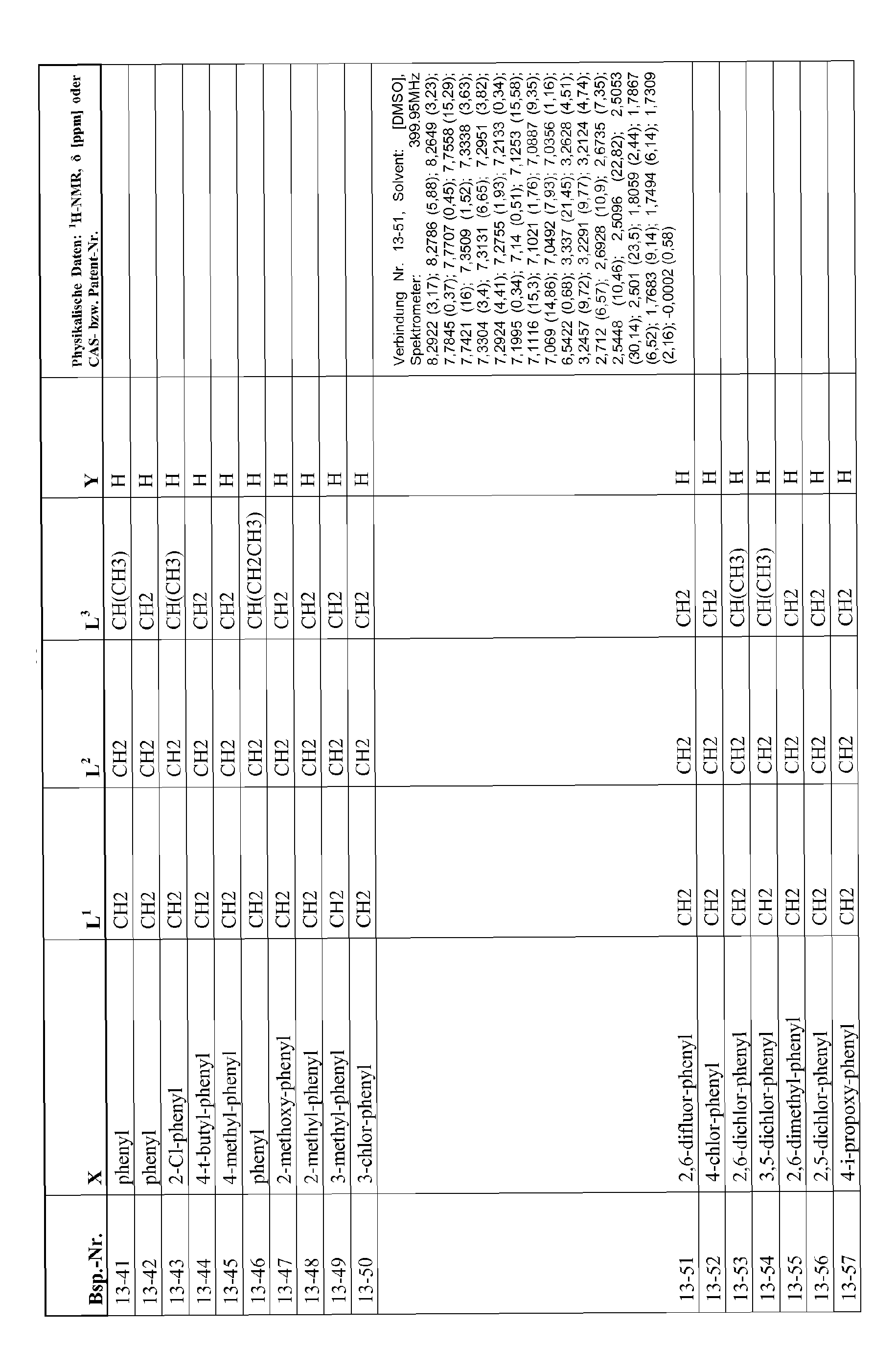

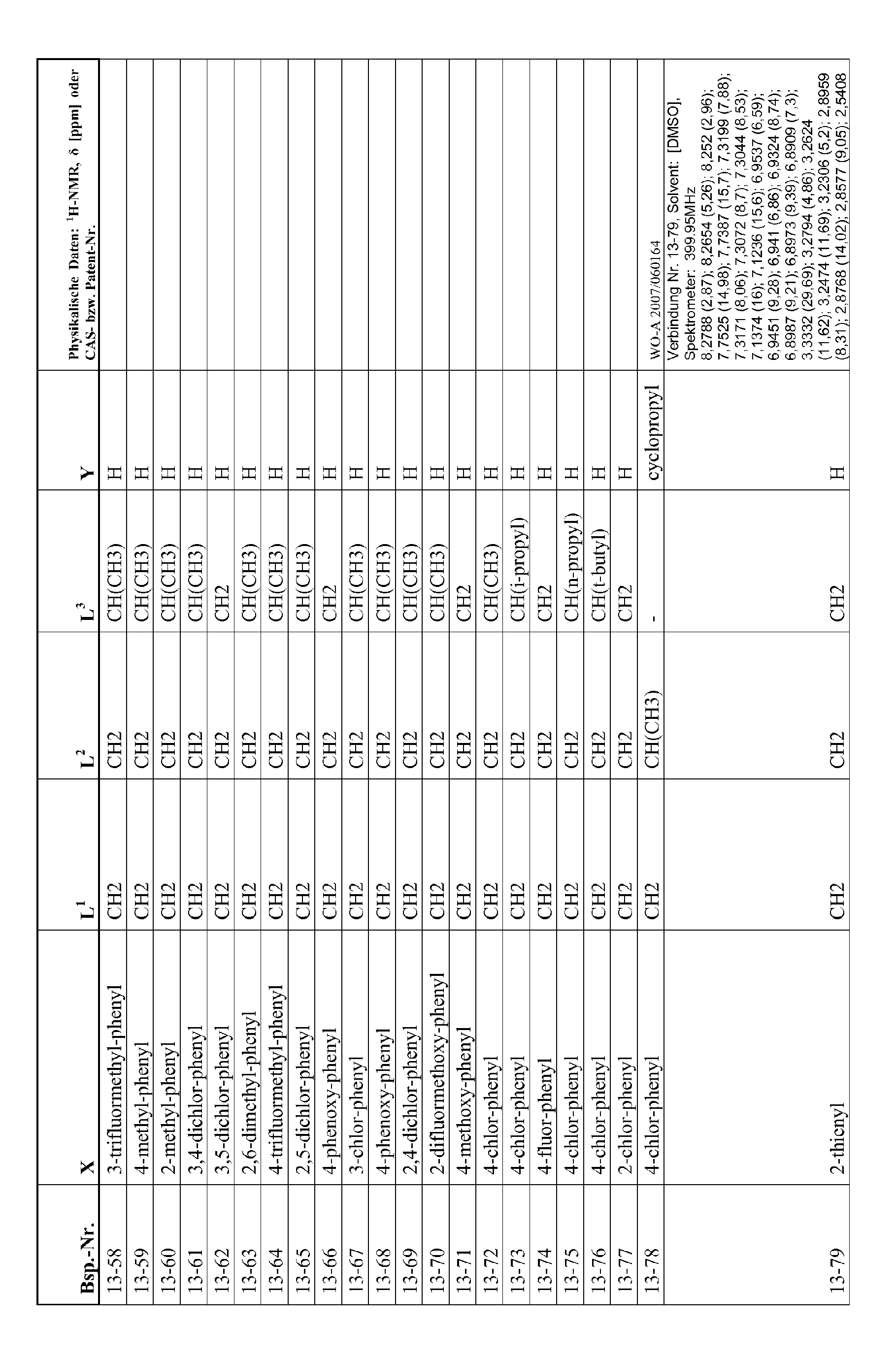

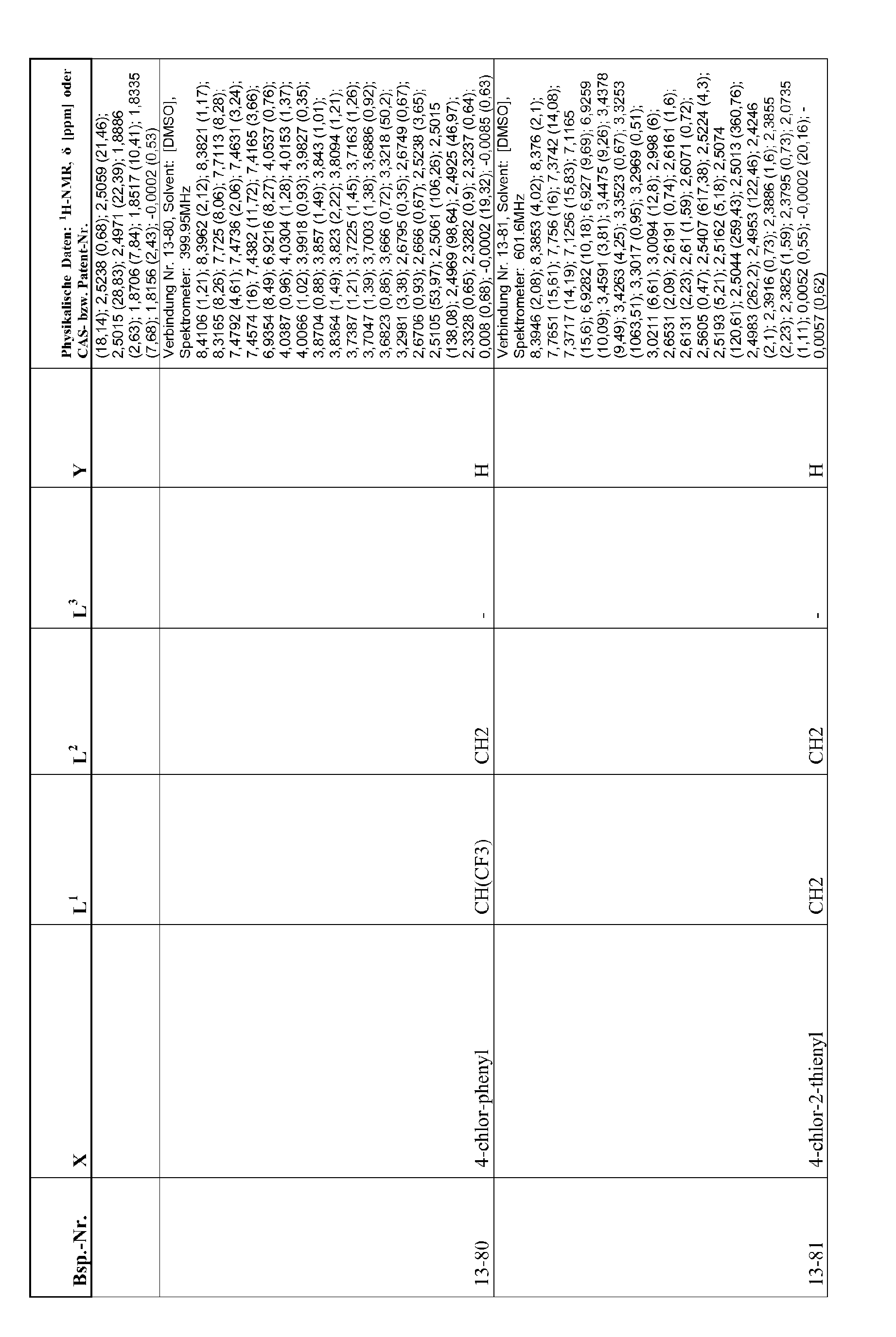

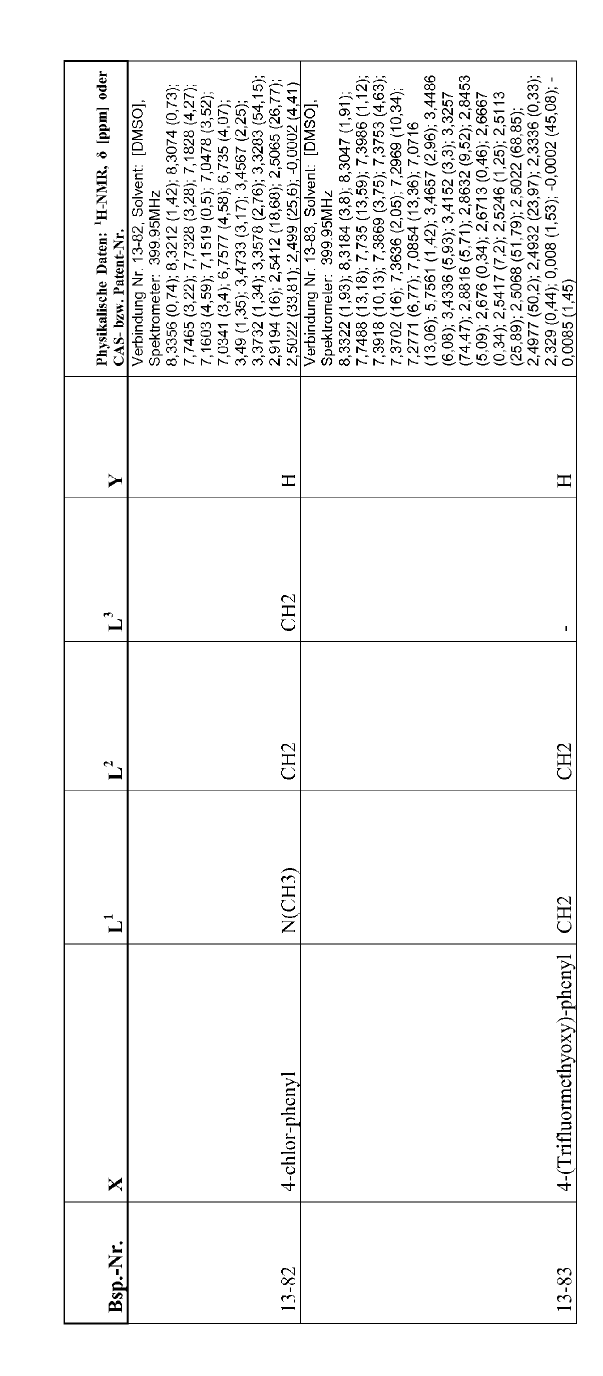

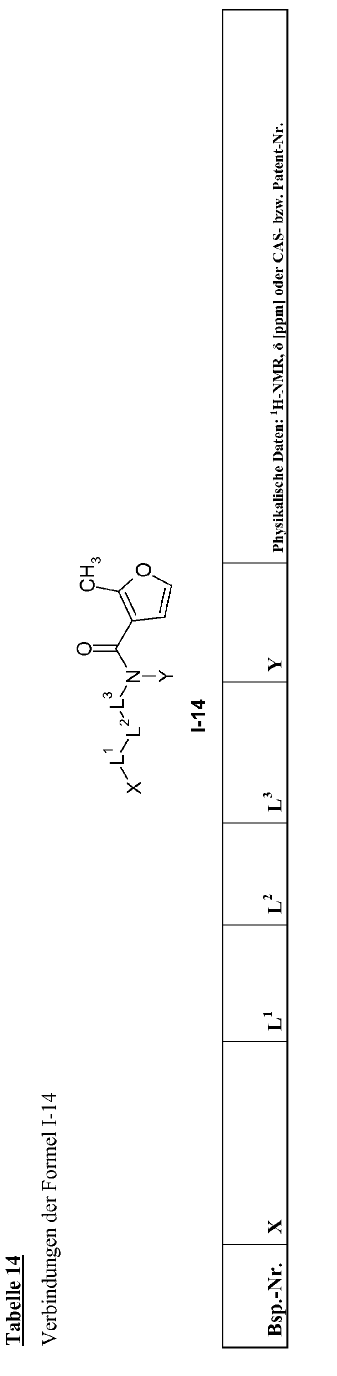

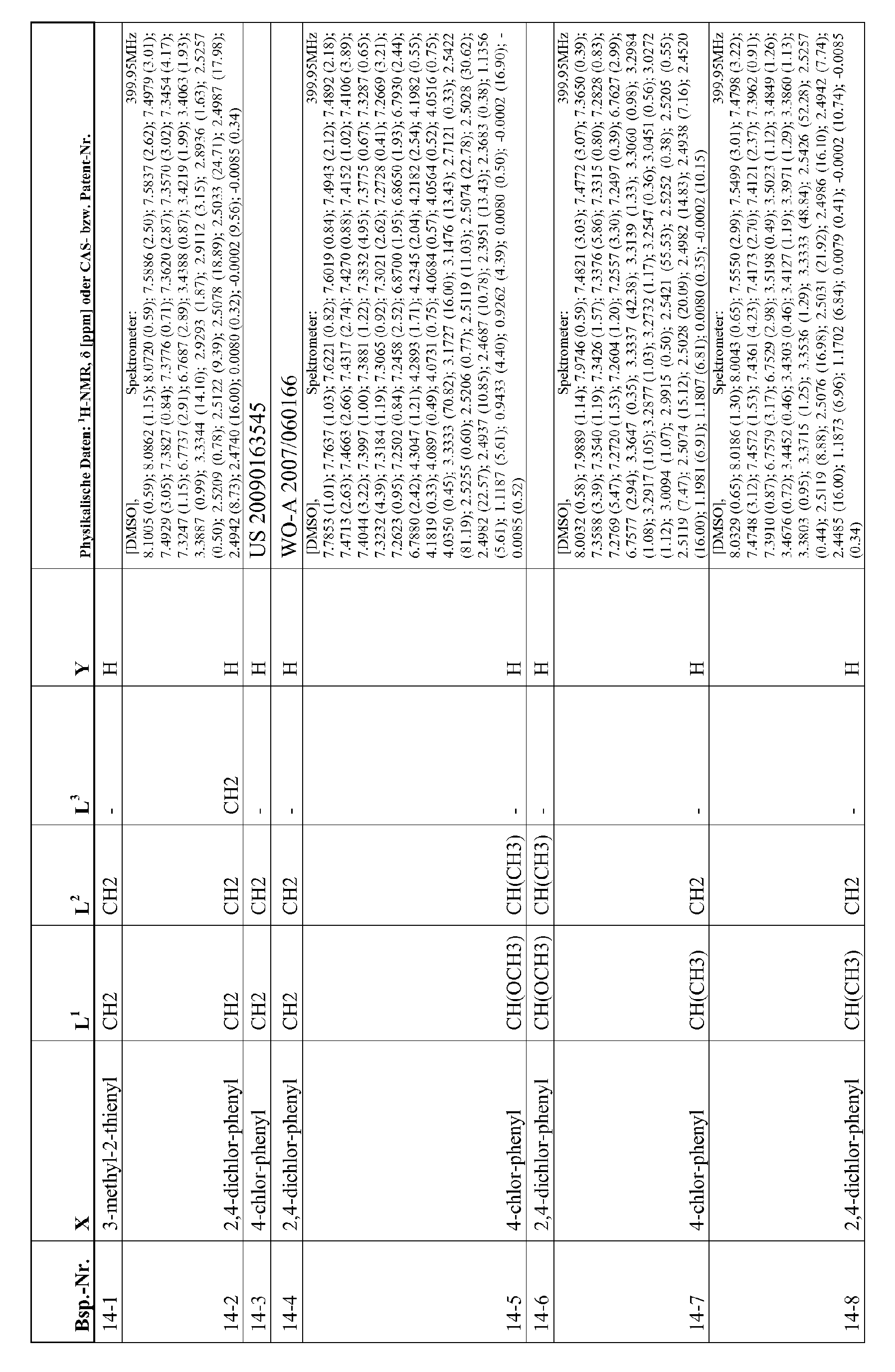

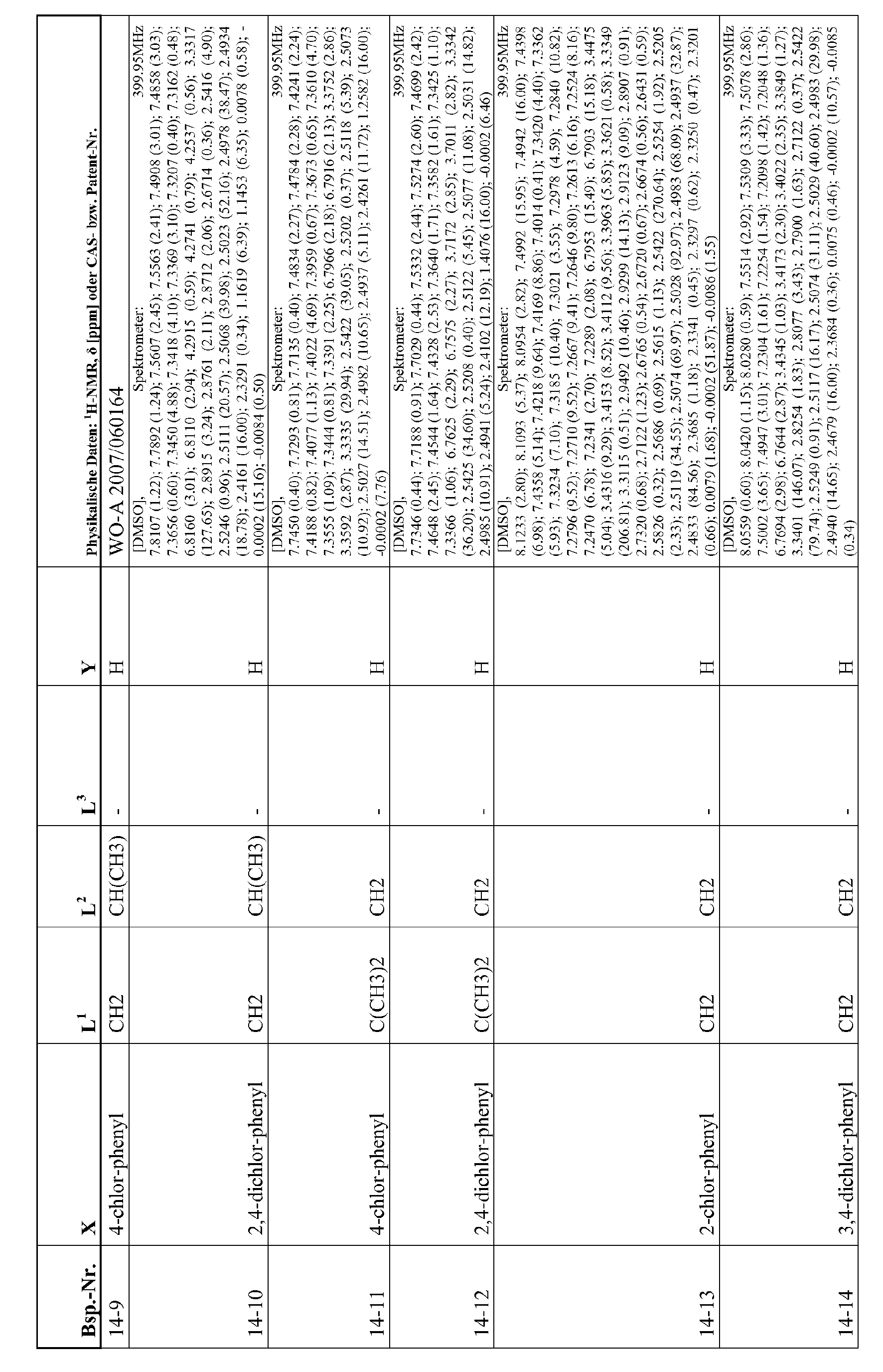

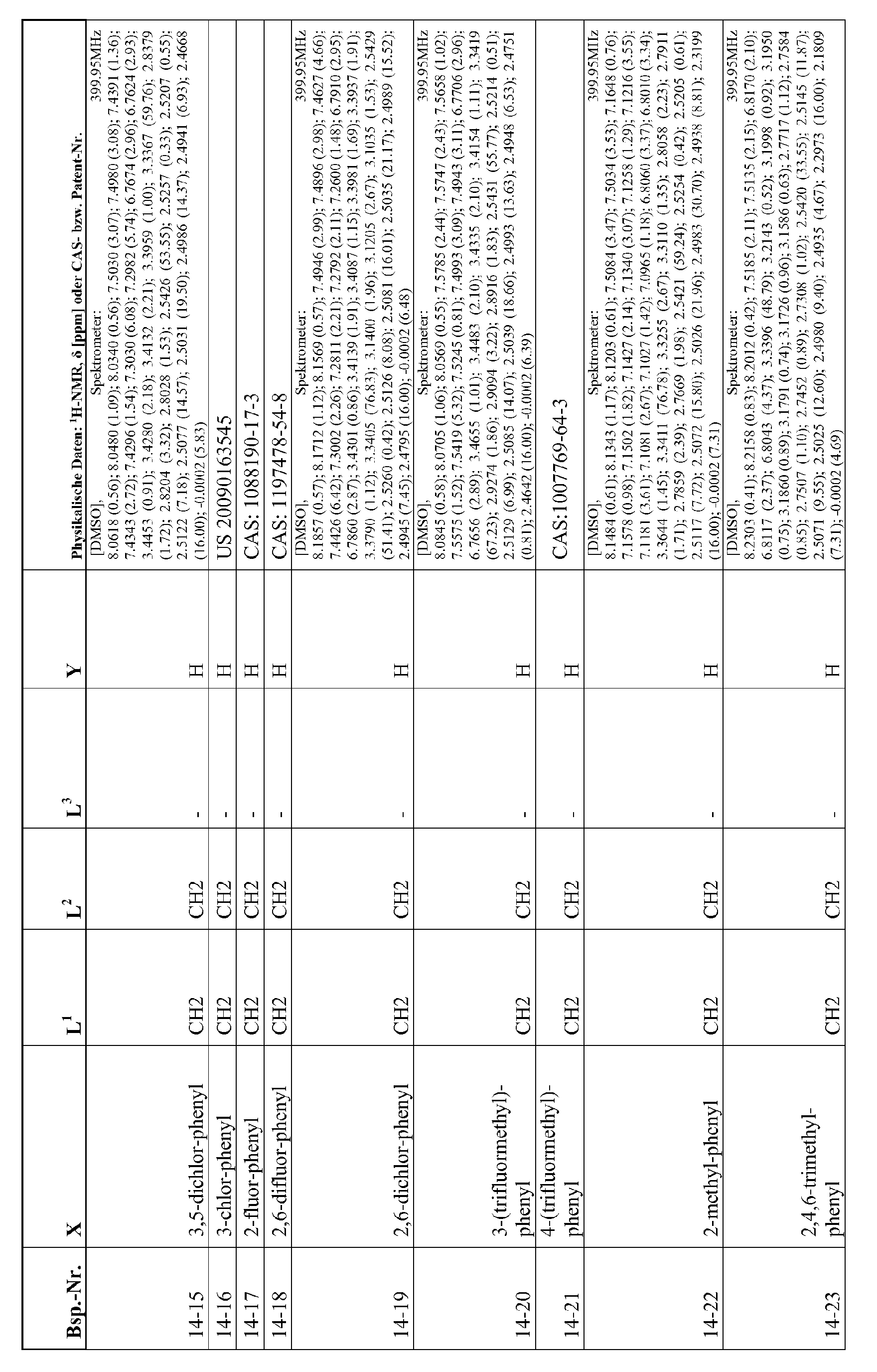

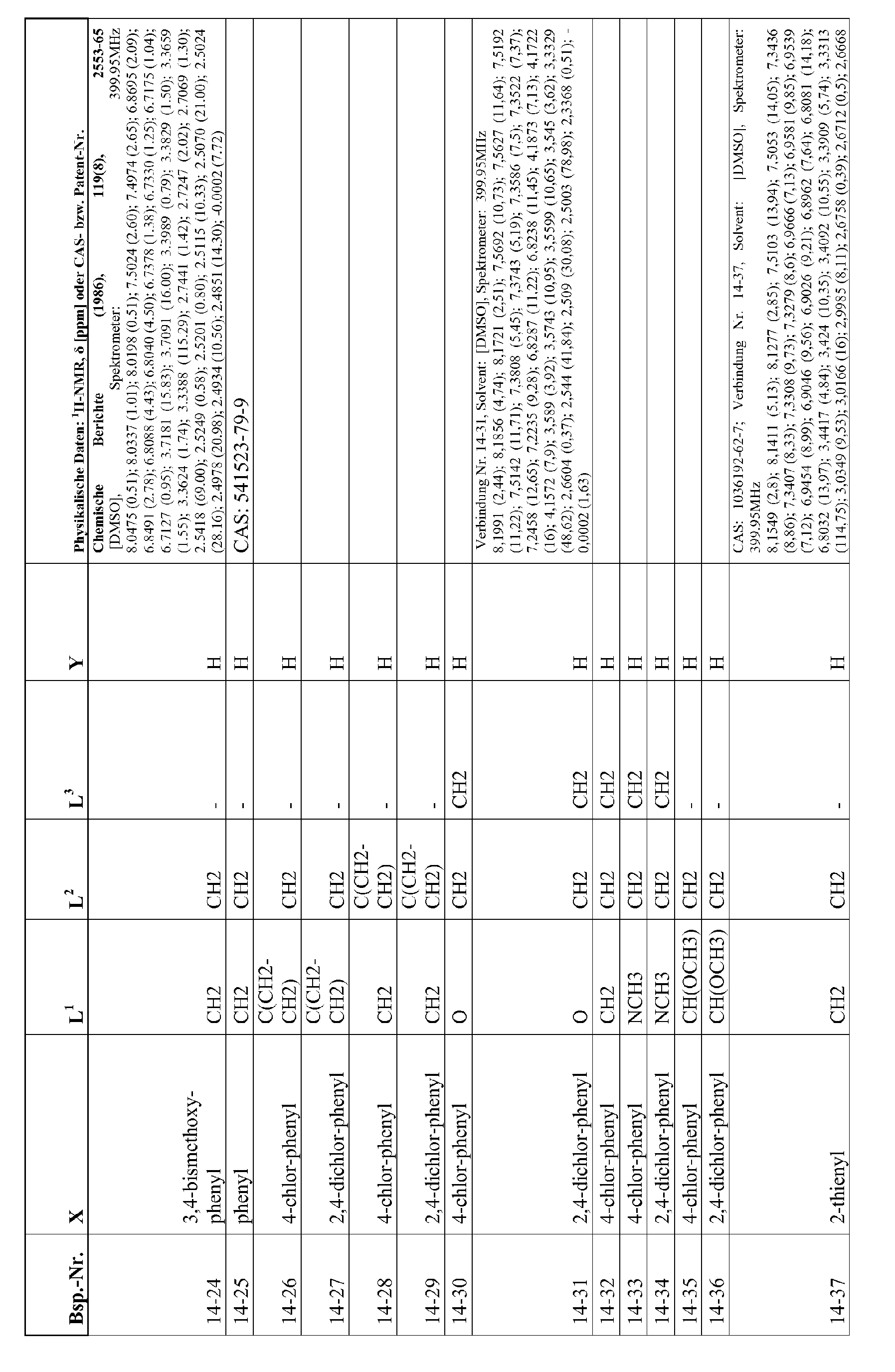

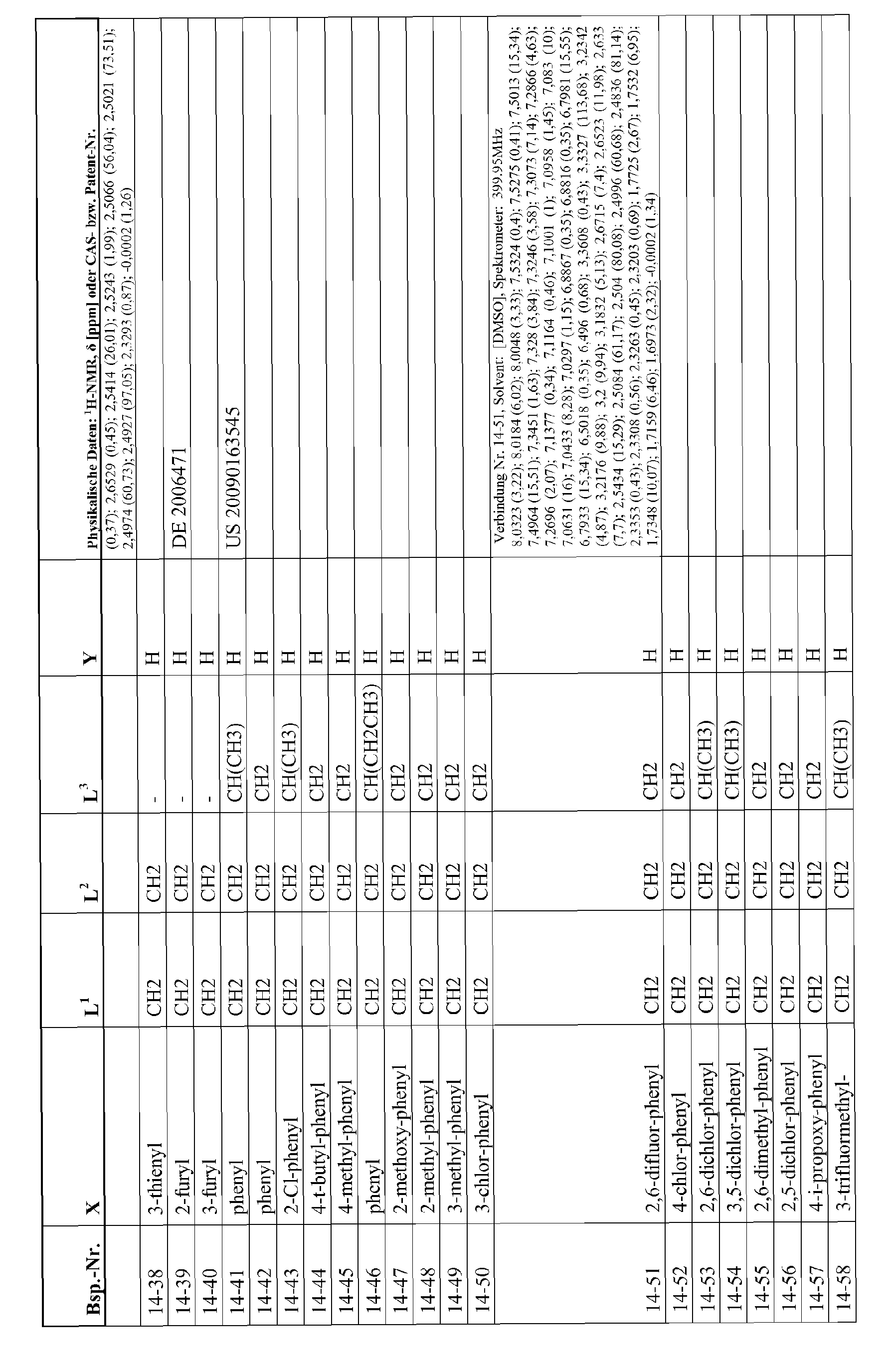

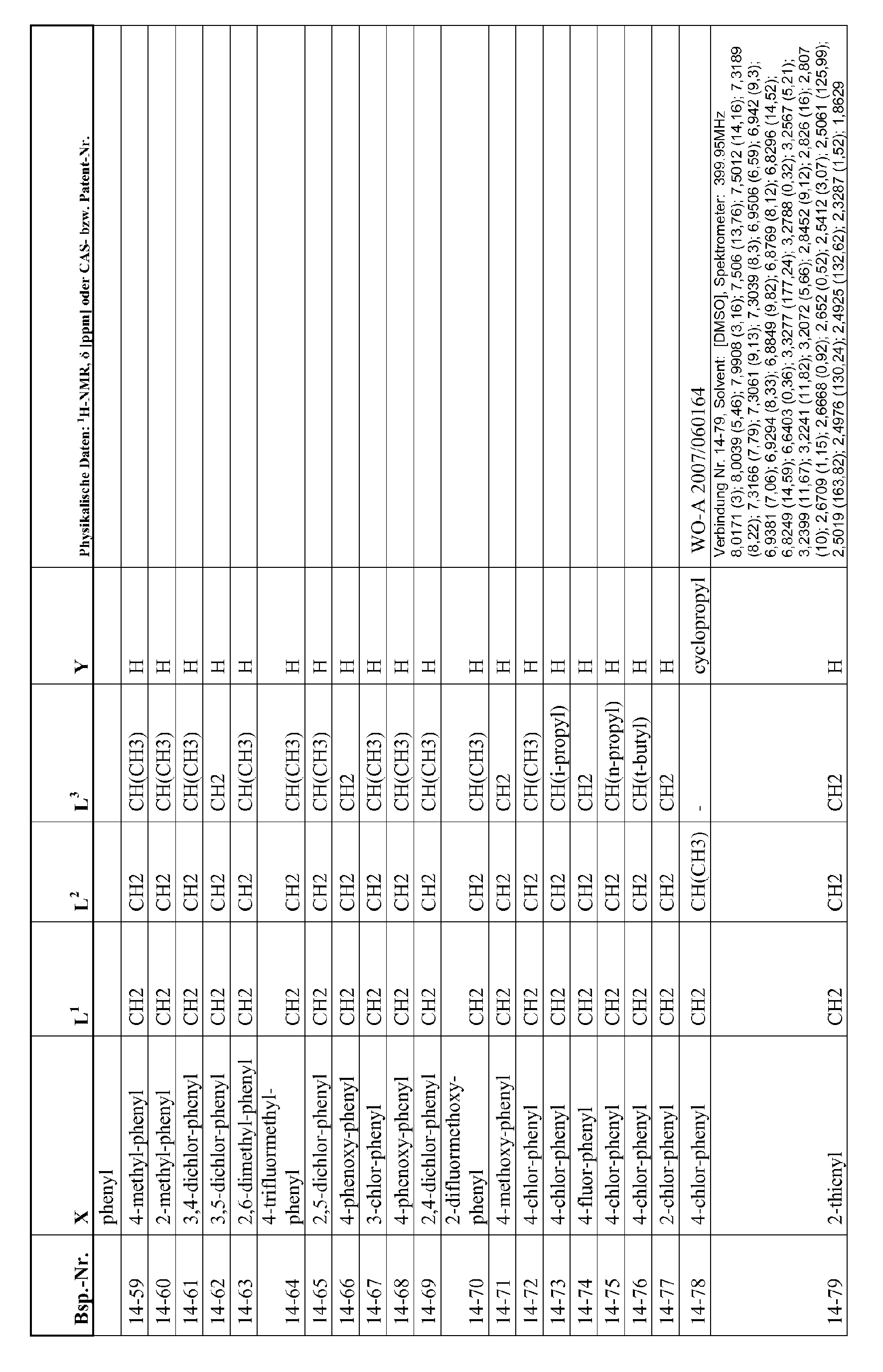

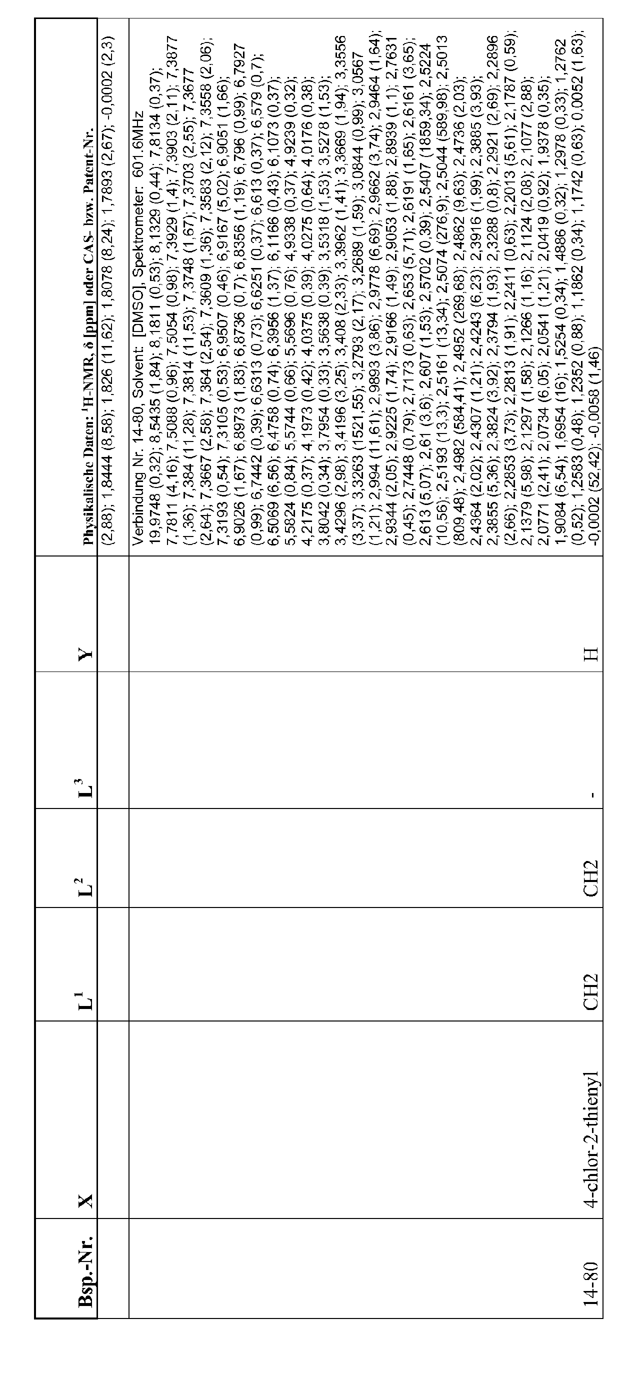

**Tabelle 15**

| | | | | | | |
|---|---|---|---|---|---|---|
| Verbindungen der Formel I-15 | | | | | | |
| | | | | | | 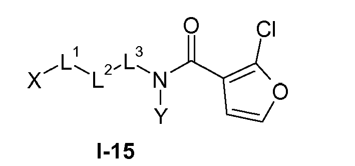 |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 15-1 | 3-methyl-2-thienyl | CH2 | CH2 | - | H | |
| 15-2 | 2,4-dichlor-phenyl | CH2 | CH2 | CH2 | H | |
| 15-3 | 4-chlor-phenyl | CH2 | CH2 | - | H | |
| 15-4 | 2,4-dichlor-phenyl | CH2 | CH2 | - | H | |
| 15-5 | 4-chlor-phenyl | CH(OCH3) | CH(CH3) | - | H | |
| 15-6 | 2,4-dichlor-phenyl | CH(OCH3) | CH(CH3) | - | H | |
| 15-7 | 4-chlor-phenyl | CH(CH3) | CH2 | - | H | |
| 15-8 | 2,4-dichlor-phenyl | CH(CH3) | CH2 | - | H | |
| 15-9 | 4-chlor-phenyl | CH2 | CH(CH3) | - | H | |
| 15-10 | 2,4-dichlor-phenyl | CH2 | CH(CH3) | - | H | |
| 15-11 | 4-chlor-phenyl | C(CH3)2 | CH2 | - | H | |
| 15-12 | 2,4-dichlor-phenyl | C(CH3)2 | CH2 | - | H | |
| 15-13 | 2-chlor-phenyl | CH2 | CH2 | - | H | |
| 15-14 | 3,4-dichlor-phenyl | CH2 | CH2 | - | H | |
| 15-15 | 3,5-dichlor-phenyl | CH2 | CH2 | - | H | |
| 15-16 | 3-chlor-phenyl | CH2 | CH2 | - | H | |
| 15-17 | 2-fluor-phenyl | CH2 | CH2 | - | H | |
| 15-18 | 2,6-difluor-phenyl | CH2 | CH2 | - | H | |
| 15-19 | 2,6-dichlor-phenyl | CH2 | CH2 | - | H | |
| 15-20 | 3-(trifluormethyl)-phenyl | CH2 | CH2 | - | H | |
| 15-21 | 4-(trifluormethyl)-phenyl | CH2 | CH2 | - | H | |
| 15-22 | 2-methyl-phenyl | CH2 | CH2 | - | H | |
| 15-23 | 2,4,6-trimethyl-phenyl | CH2 | CH2 | - | H | |
| 15-24 | 3,4-bismethoxy-phenyl | CH2 | CH2 | - | H | |
| 15-25 | phenyl | CH2 | CH2 | - | H | |
| 15-26 | 4-chlor-phenyl | C(CH2-CH2) | CH2 | - | H | |
| 15-27 | 2,4-dichlor-phenyl | C(CH2-CH2) | CH2 | - | H | |
| 15-28 | 4-chlor-phenyl | CH2 | C(CH2-CH2) | - | H | |
| 15-29 | 2,4-dichlor-phenyl | CH2 | C(CH2-CH2) | - | H | |
| 15-30 | 4-chlor-phenyl | O | CH2 | CH2 | H | |
| 15-31 | 2,4-dichlor-phenyl | O | CH2 | CH2 | H | |
| 15-32 | 4-chlor-phenyl | CH2 | CH2 | CH2 | H | |
| 15-33 | 4-chlor-phenyl | NCH3 | CH2 | CH2 | H | |
| 15-34 | 2,4-dichlor-phenyl | NCH3 | CH2 | CH2 | H | |
| 15-35 | 4-chlor-phenyl | CH(OCH3) | CH2 | - | H | |
| 15-36 | 2,4-dichlor-phenyl | CH(OCH3) | CH2 | - | H | |
| 15-37 | 2-thienyl | CH2 | CH2 | - | H | |
| 15-38 | 3-thienyl | CH2 | CH2 | - | H | |
| 15-39 | 2-furyl | CH2 | CH2 | - | H | |
| 15-40 | 3-furyl | CH2 | CH2 | - | H | |
| 15-41 | phenyl | CH2 | CH2 | CH(CH3) | H | |
| 15-42 | phenyl | CH2 | CH2 | CH2 | H | |
| 15-43 | 2-Cl-phenyl | CH2 | CH2 | CH(CH3) | H | |
| 15-44 | 4-t-butyl-phenyl | CH2 | CH2 | CH2 | H | |
| 15-45 | 4-methyl-phenyl | CH2 | CH2 | CH2 | H | |
| 15-46 | phenyl | CH2 | CH2 | CH(CH2CH3) | H | |
| 15-47 | 2-methoxy-phenyl | CH2 | CH2 | CH2 | H | |
| 15-48 | 2-methyl-phenyl | CH2 | CH2 | CH2 | H | |
| 15-49 | 3-methyl-phenyl | CH2 | CH2 | CH2 | H | |
| 15-50 | 3-chlor-phenyl | CH2 | CH2 | CH2 | H | |
| 15-51 | 2,6-difluor-phenyl | CH2 | CH2 | CH2 | H | |
| 15-52 | 4-chlor-phenyl | CH2 | CH2 | CH2 | H | |
| 15-53 | 2,6-dichlor-phenyl | CH2 | CH2 | CH(CH3) | H | |
| 15-54 | 3,5-dichlor-phenyl | CH2 | CH2 | CH(CH3) | H | |
| 15-55 | 2,6-dimethyl-phenyl | CH2 | CH2 | CH2 | H | |
| 15-56 | 2,5-dichlor-phenyl | CH2 | CH2 | CH2 | H | |
| 15-57 | 4-i-propoxy-phenyl | CH2 | CH2 | CH2 | H | |
| 15-58 | 3-trifluormethyl-phenyl | CH2 | CH2 | CH(CH3) | H | |
| 15-59 | 4-methyl-phenyl | CH2 | CH2 | CH(CH3) | H | |
| 15-60 | 2-methyl-phenyl | CH2 | CH2 | CH(CH3) | H | |
| 15-61 | 3,4-dichlor-phenyl | CH2 | CH2 | CH(CH3) | H | |
| 15-62 | 3,5-dichlor-phenyl | CH2 | CH2 | CH2 | H | |
| 15-63 | 2,6-dimethyl-phenyl | CH2 | CH2 | CH(CH3) | H | |
| 15-64 | 4-trifluormethyl-phenyl | CH2 | CH2 | CH(CH3) | H | |
| 15-65 | 2,5-dichlor-phenyl | CH2 | CH2 | CH(CH3) | H | |
| 15-66 | 4-phenoxy-phenyl | CH2 | CH2 | CH2 | H | |
| 15-67 | 3-chlor-phenyl | CH2 | CH2 | CH(CH3) | H | |
| 15-68 | 4-phenoxy-phenyl | CH2 | CH2 | CH(CH3) | H | |
| 15-69 | 2,4-dichlor-phenyl | CH2 | CH2 | CH(CH3) | H | |
| 15-70 | 2-difluormethoxy-phenyl | CH2 | CH2 | CH(CH3) | H | |
| 15-71 | 4-methoxy-phenyl | CH2 | CH2 | CH2 | H | |
| 15-72 | 4-chlor-phenyl | CH2 | CH2 | CH(CH3) | H | |
| 15-73 | 4-chlor-phenyl | CH2 | CH2 | CH(i-propyl) | H | |
| 15-74 | 4-fluor-phenyl | CH2 | CH2 | CH2 | H | |
| 15-75 | 4-chlor-phenyl | CH2 | CH2 | CH(n-propyl) | H | |
| 15-76 | 4-chlor-phenyl | CH2 | CH2 | CH(t-butyl) | H | |
| 15-77 | 2-chlor-phenyl | CH2 | CH2 | CH2 | H | |
| 15-78 | 4-chlor-phenyl | CH2 | CH(CH3) | - | cyclopropyl | |

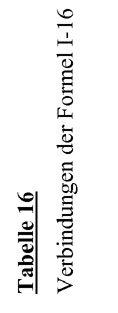

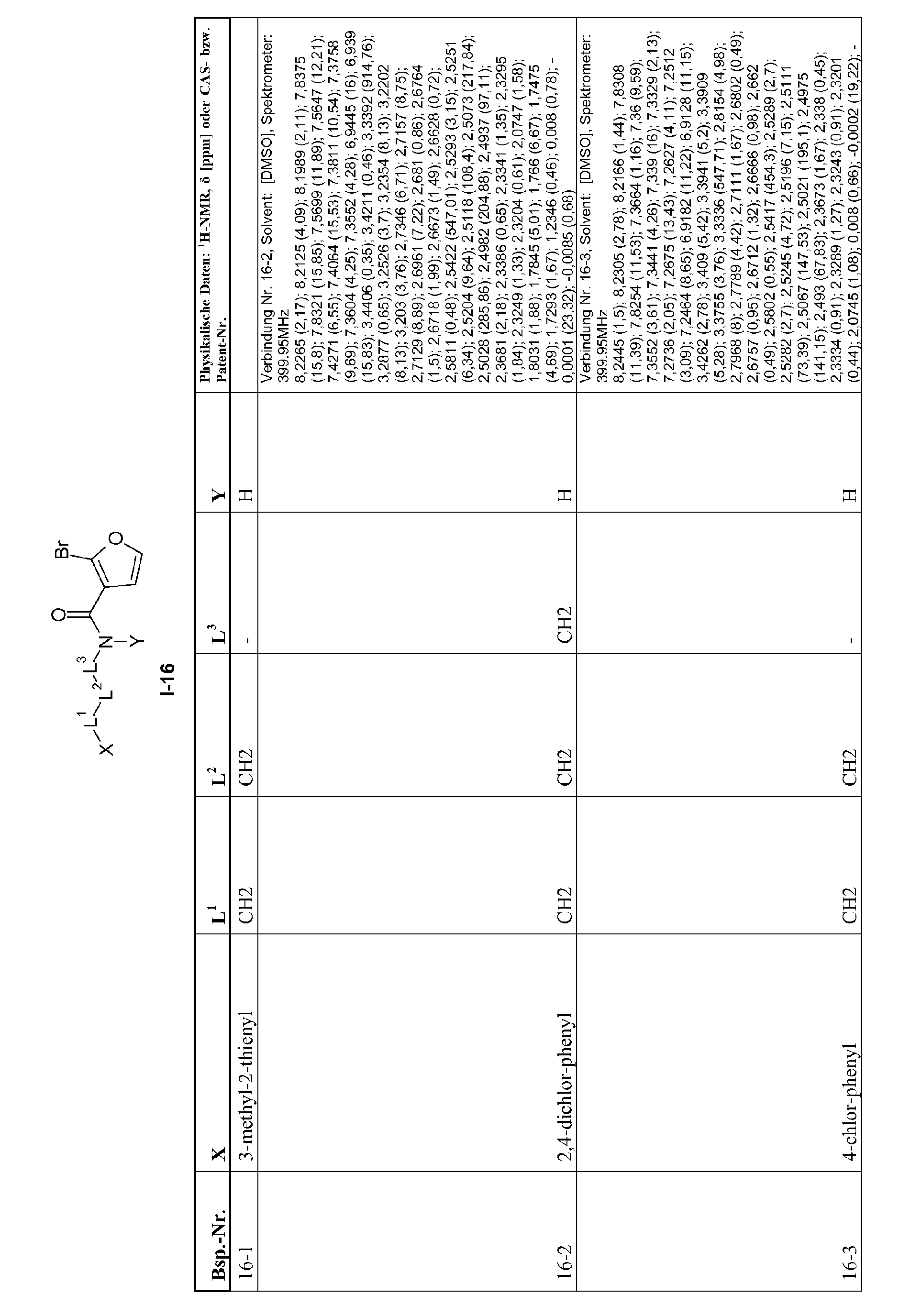

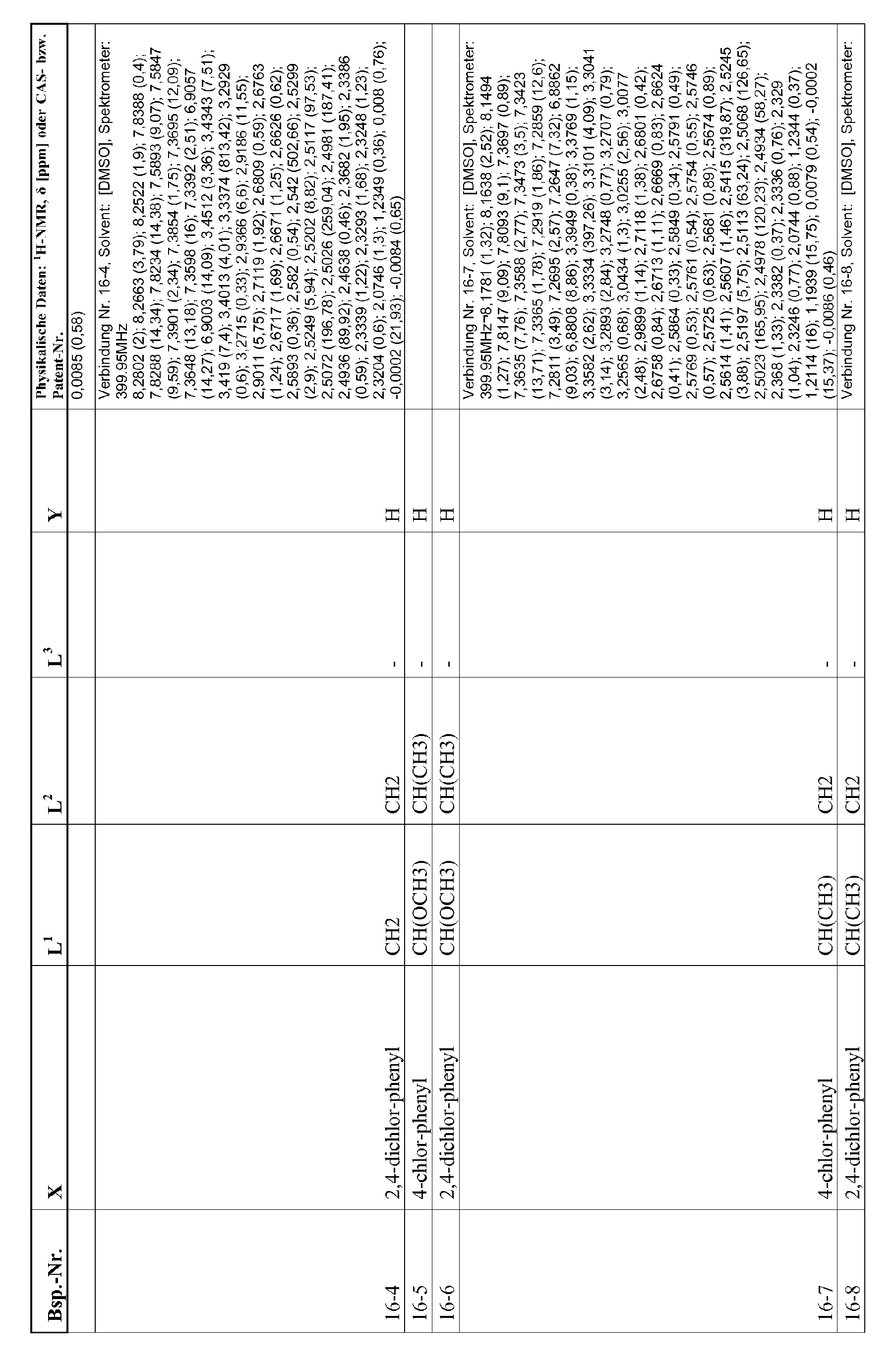

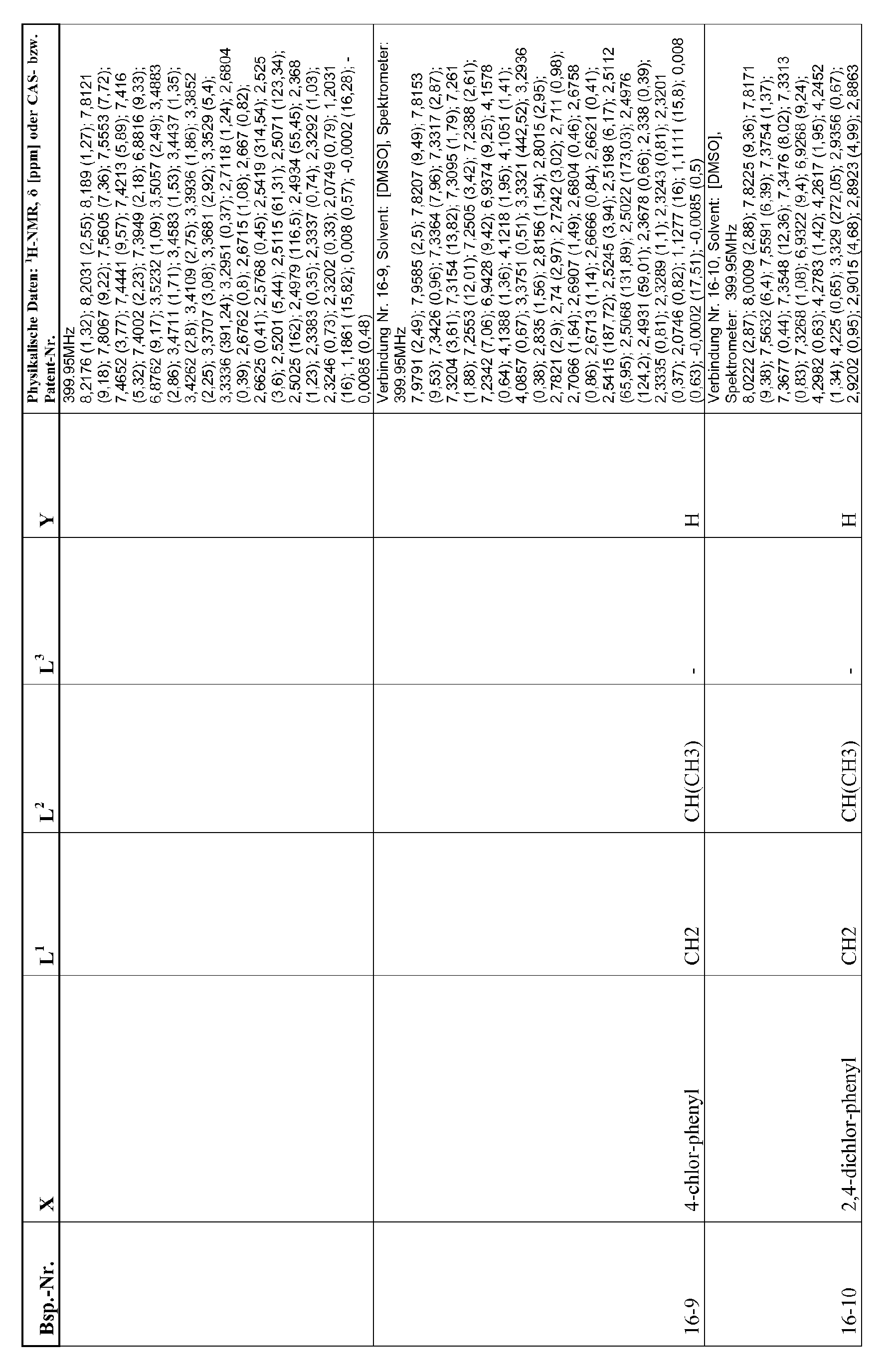

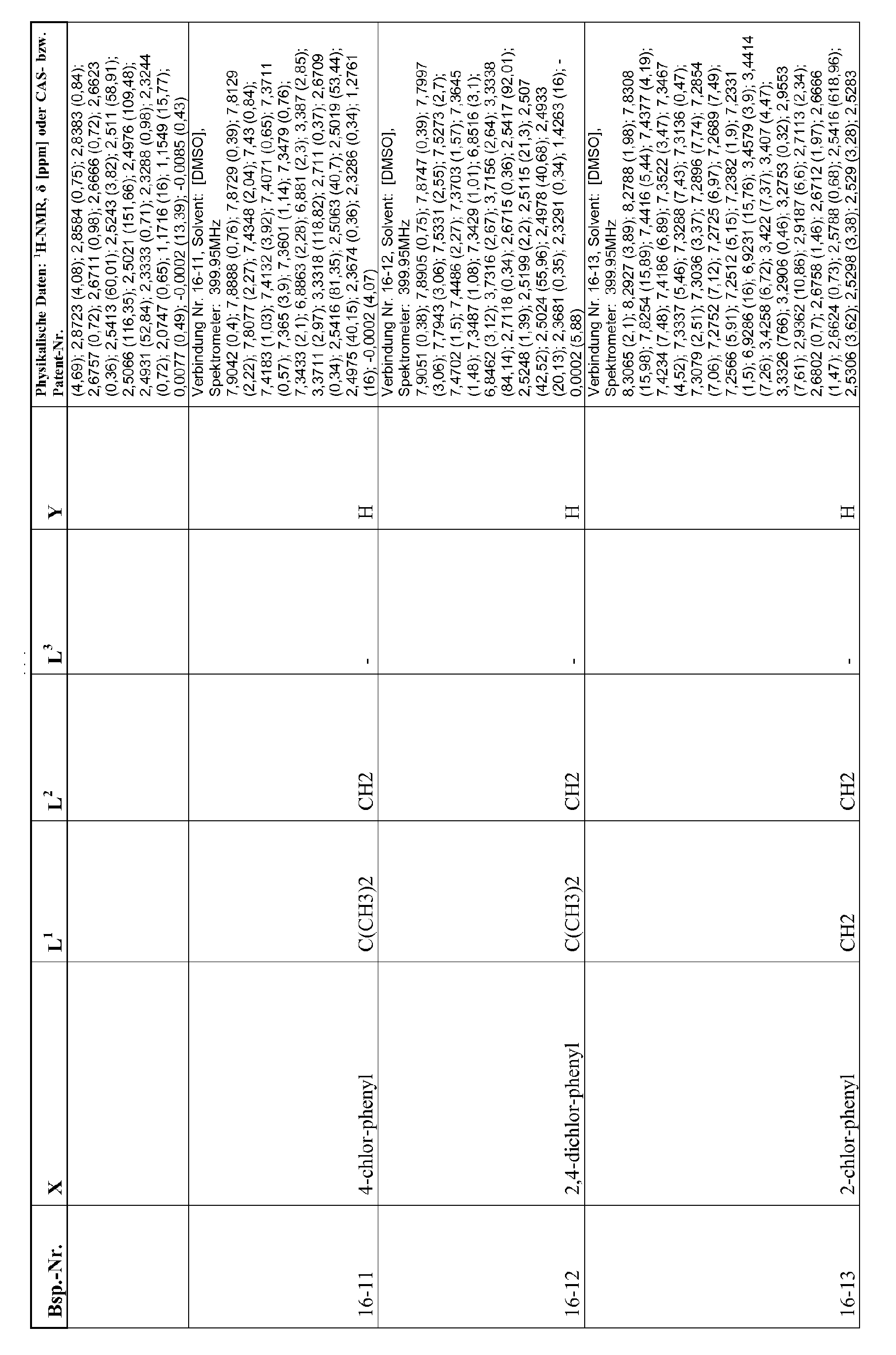

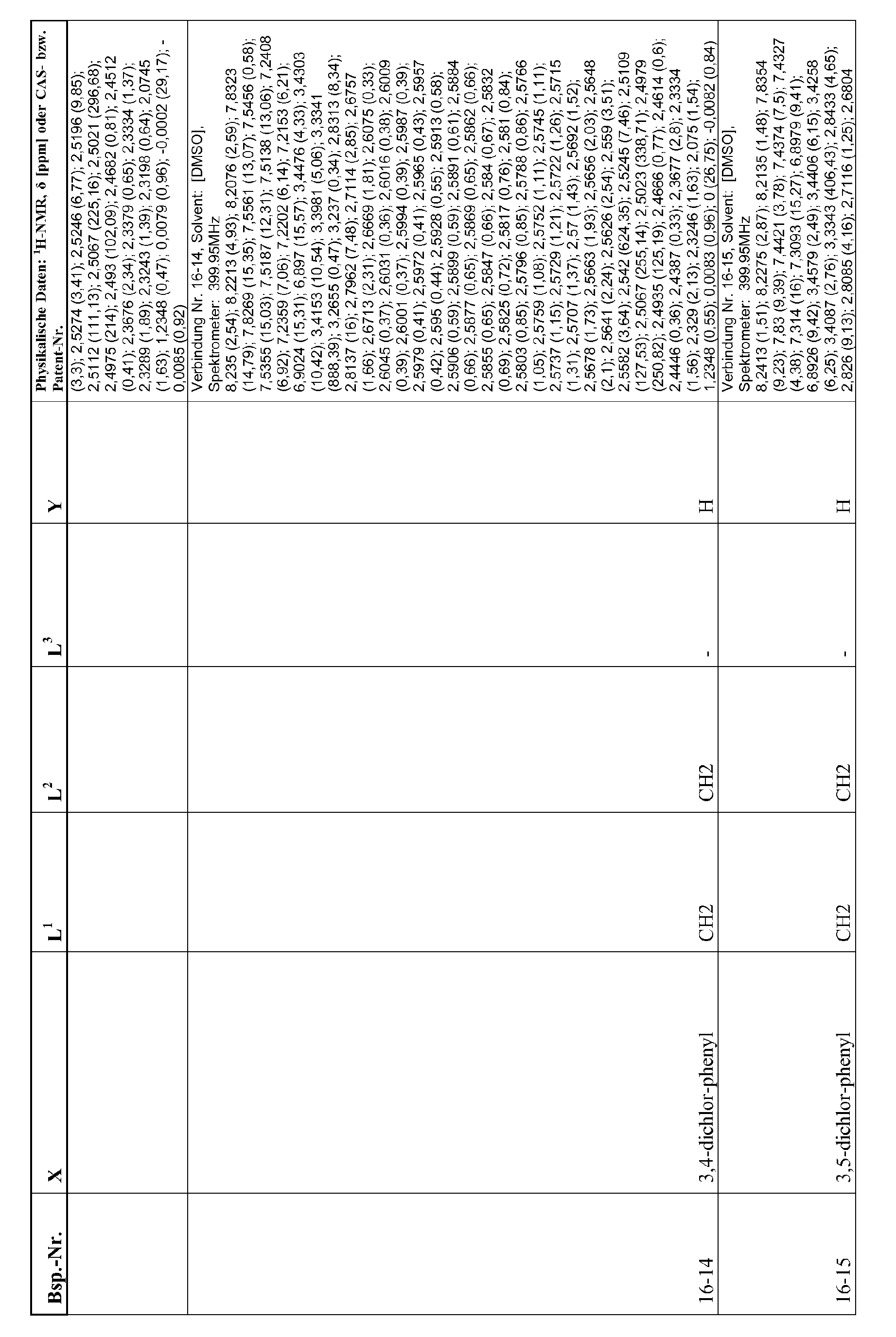

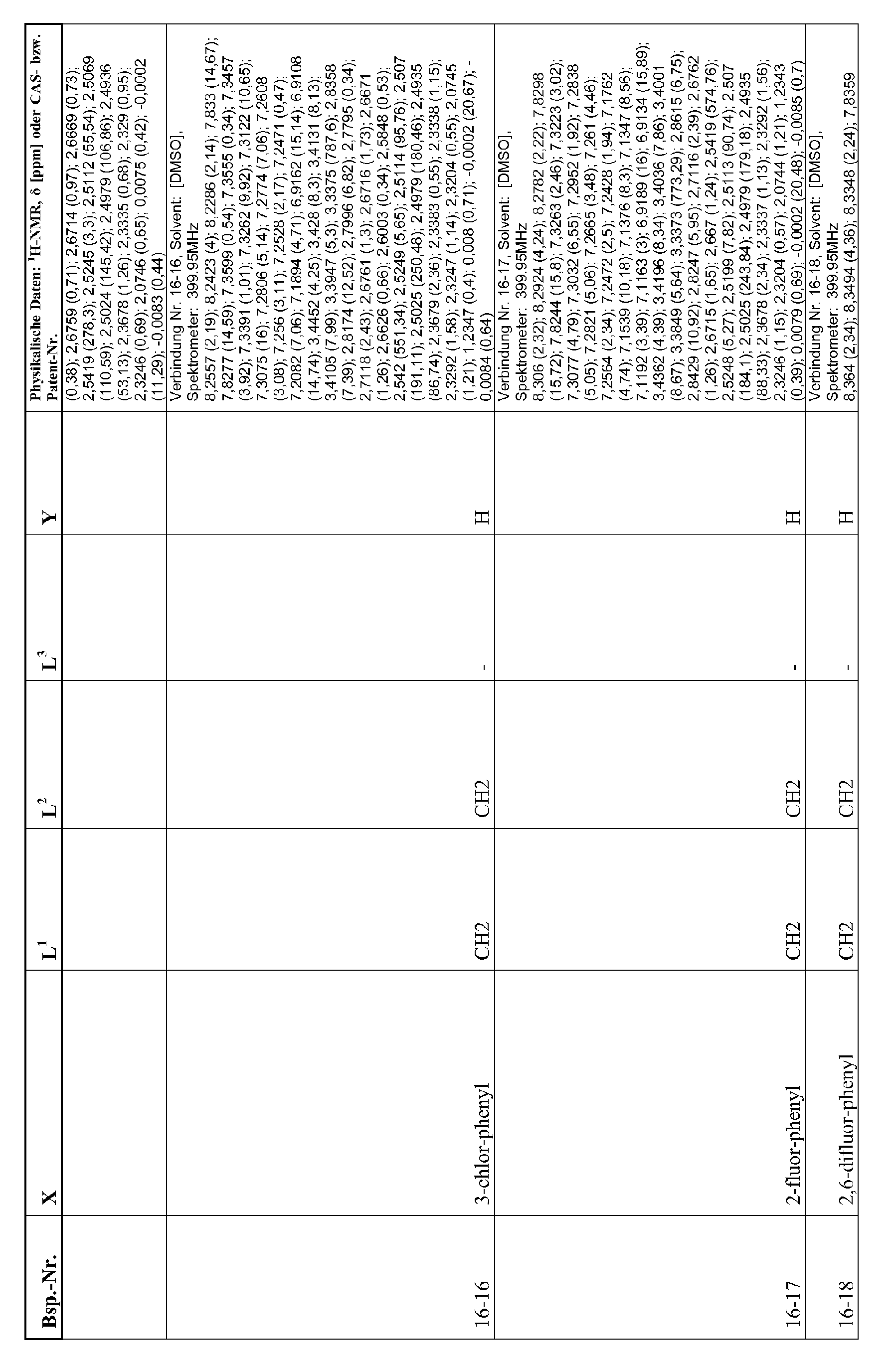

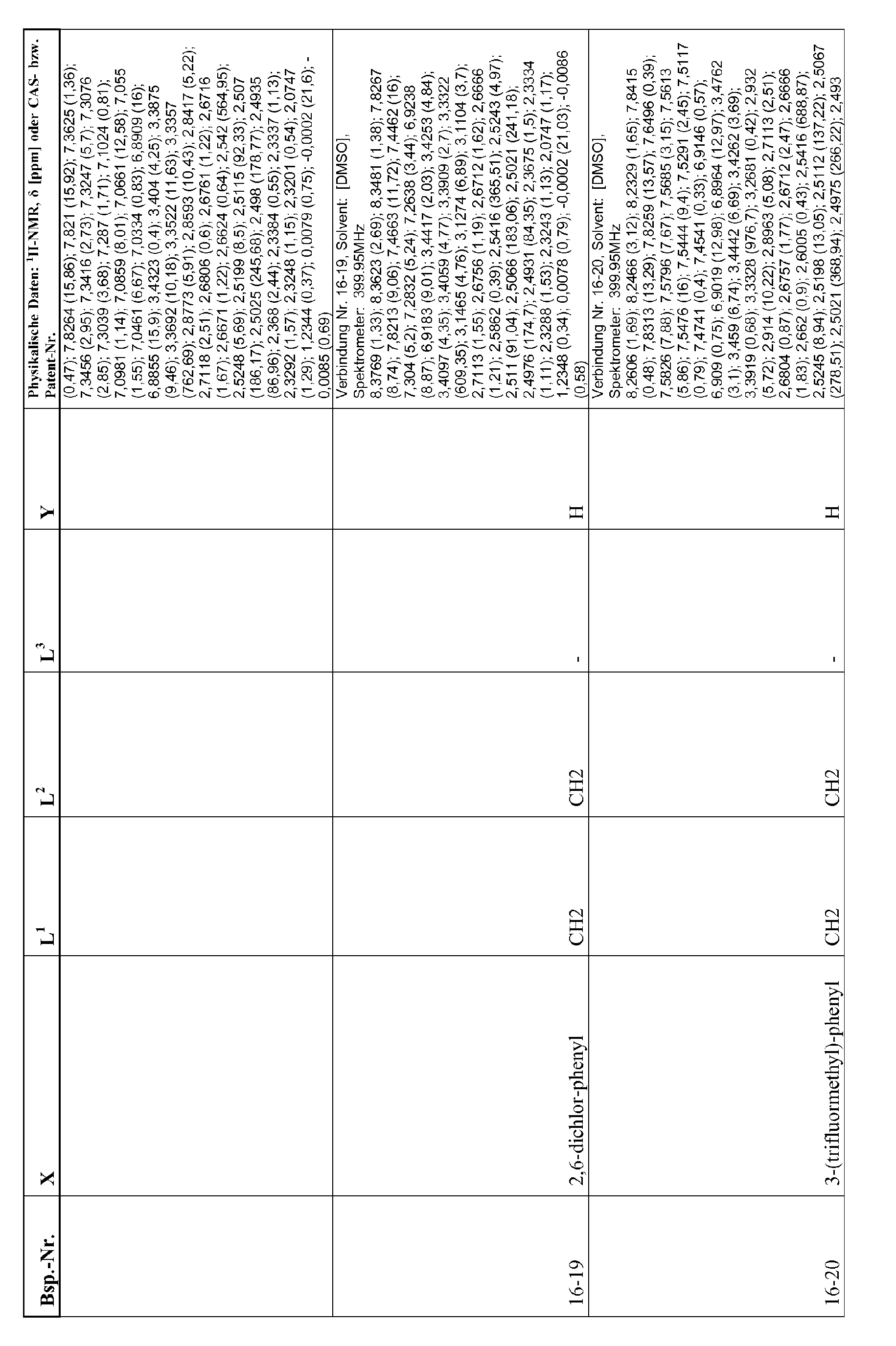

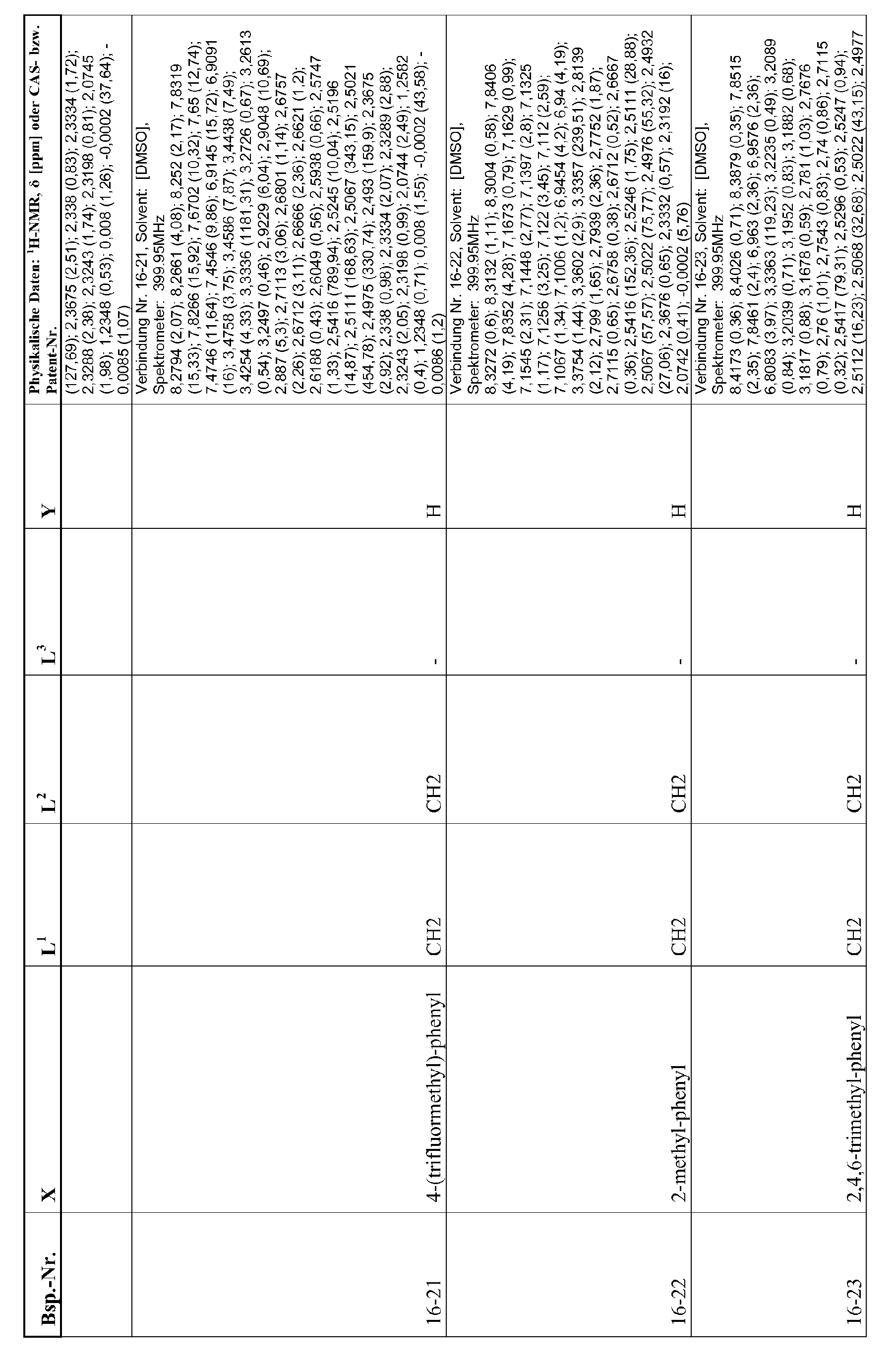

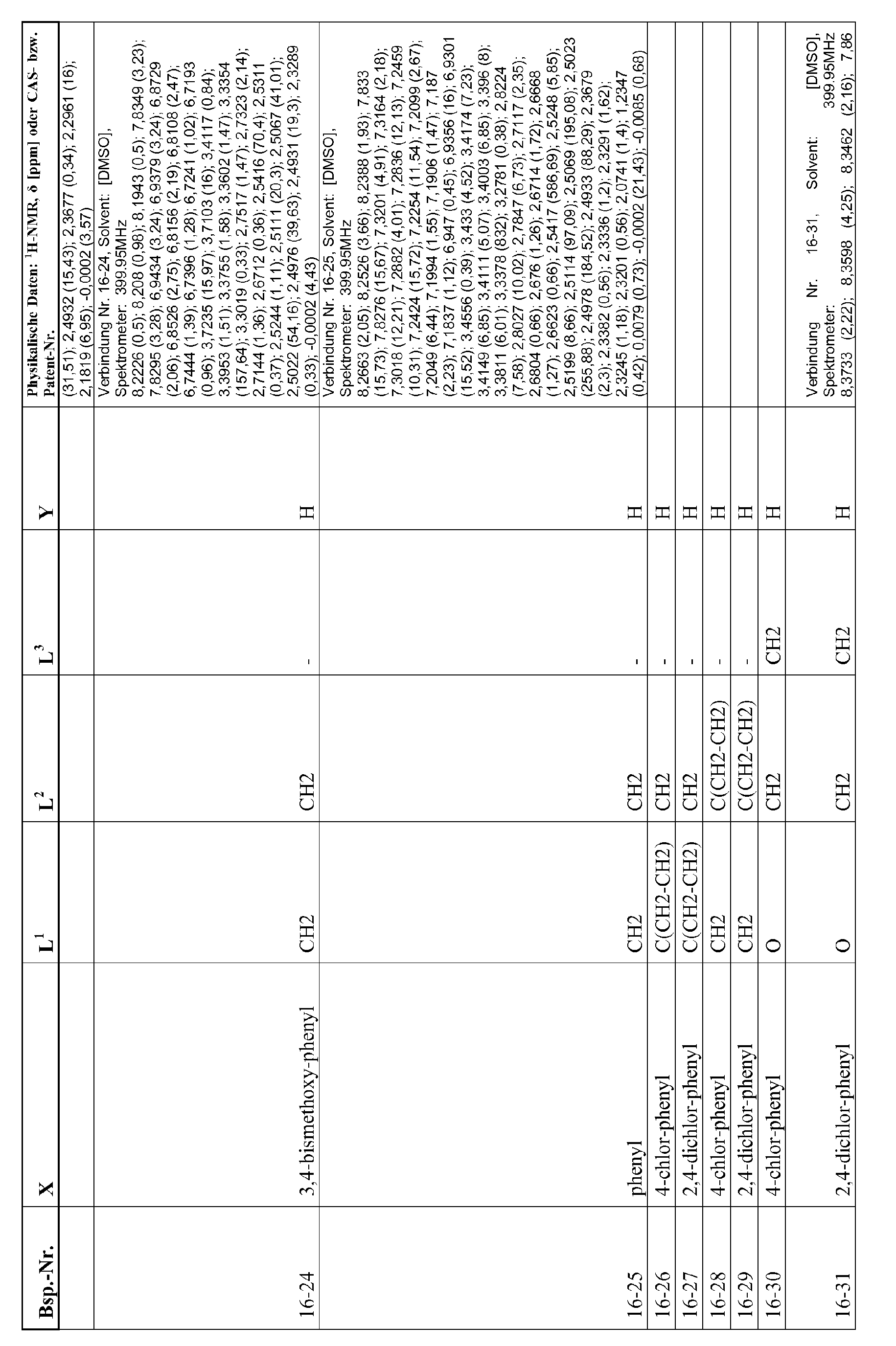

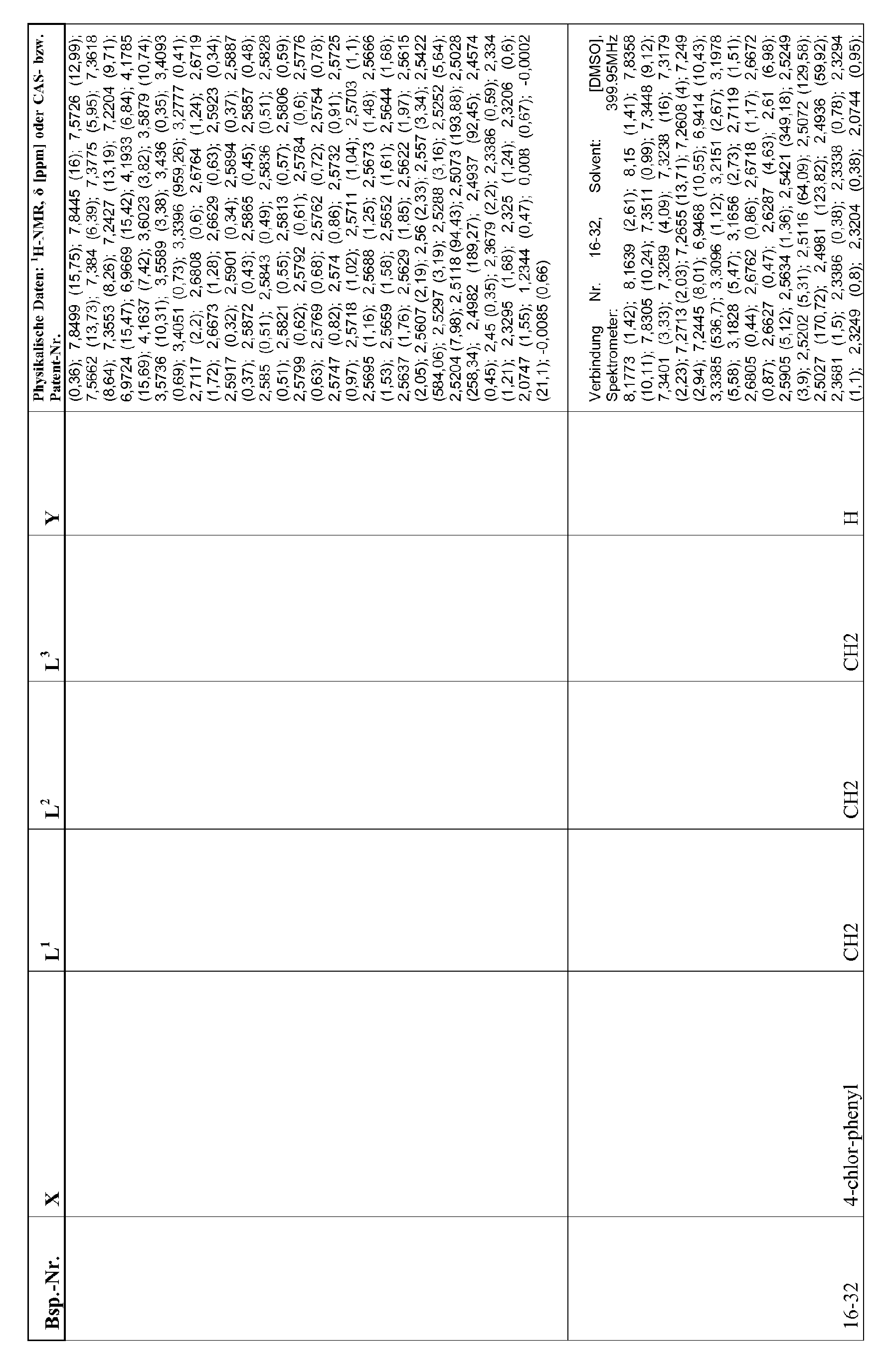

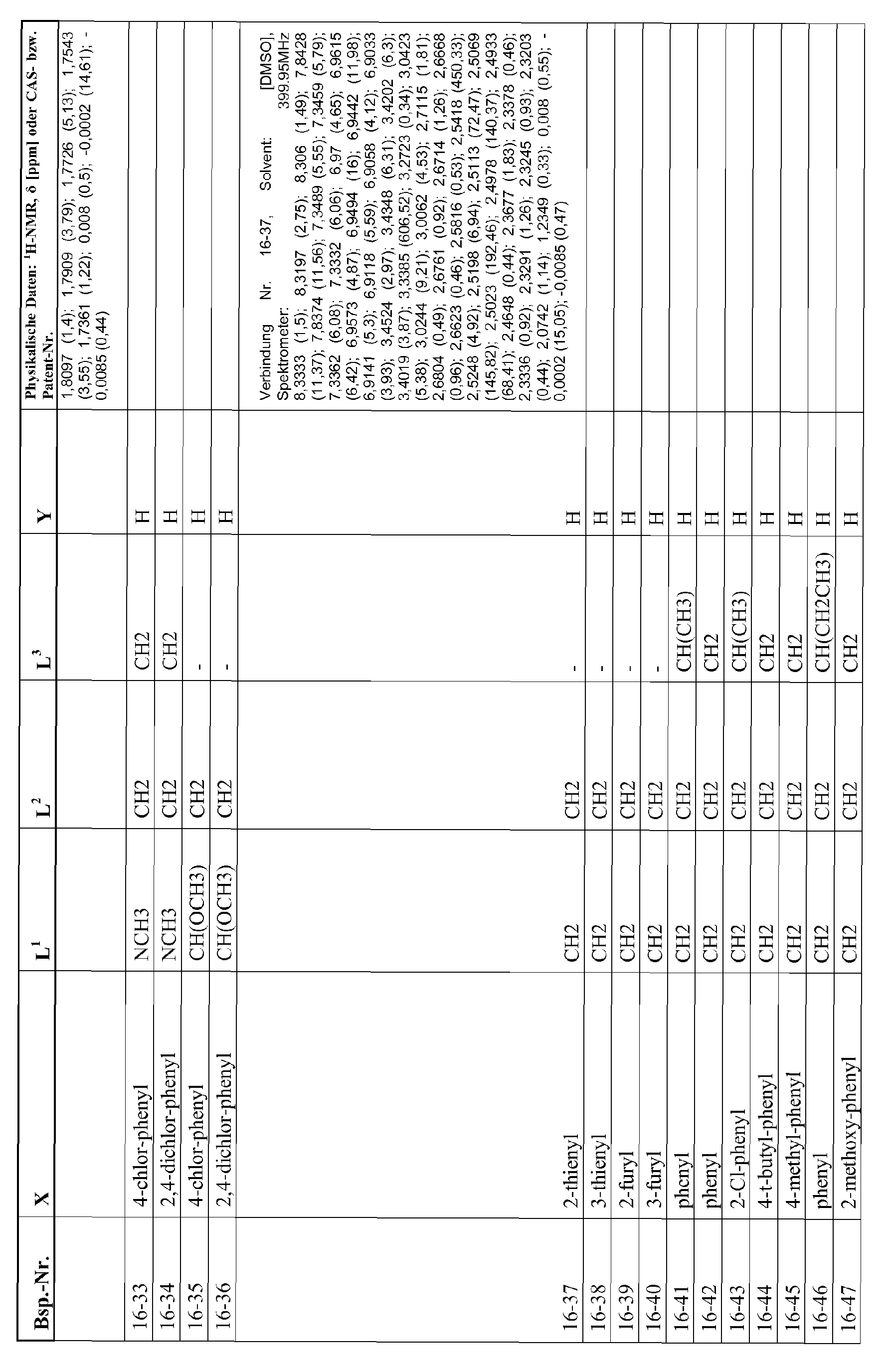

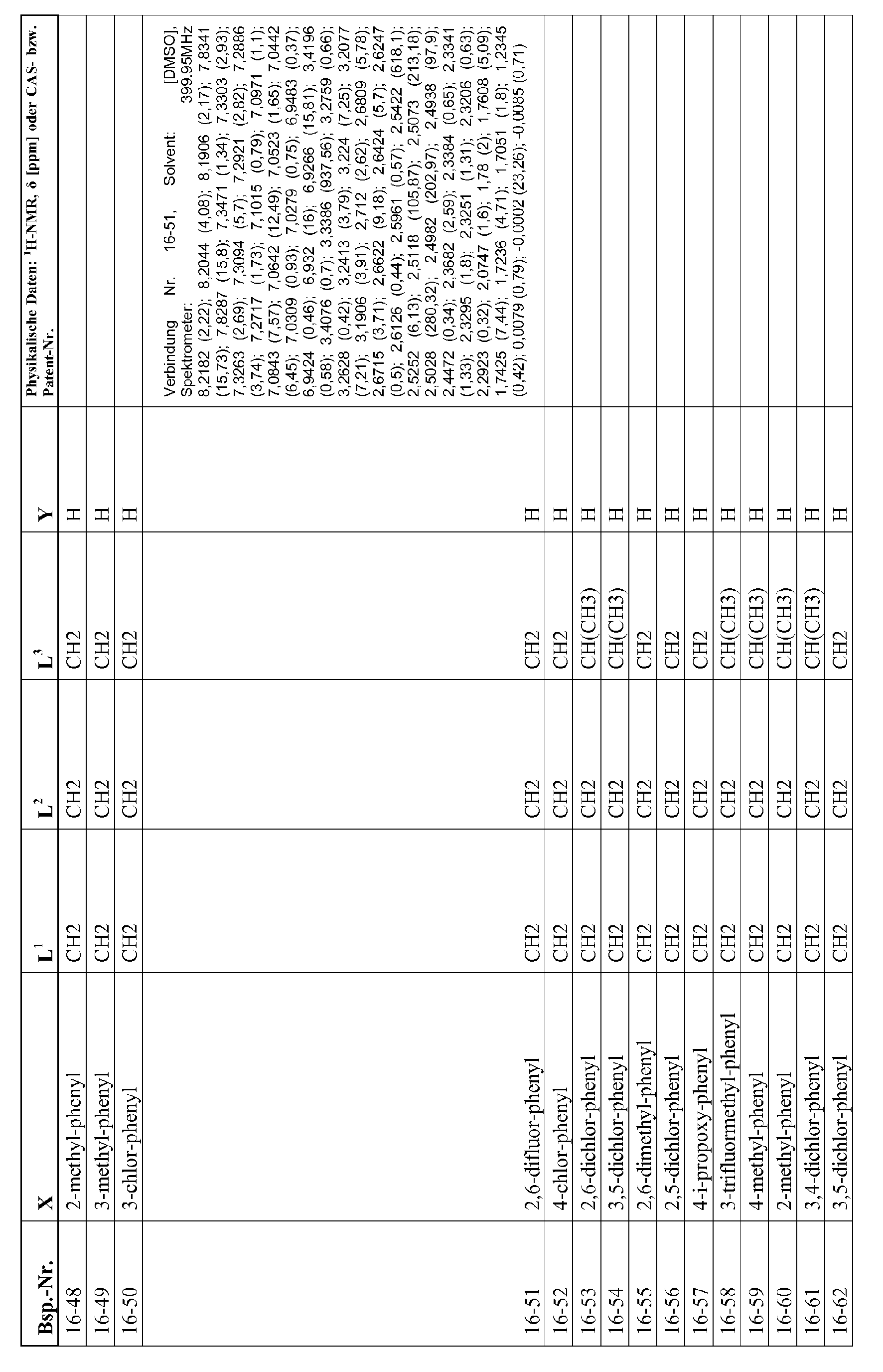

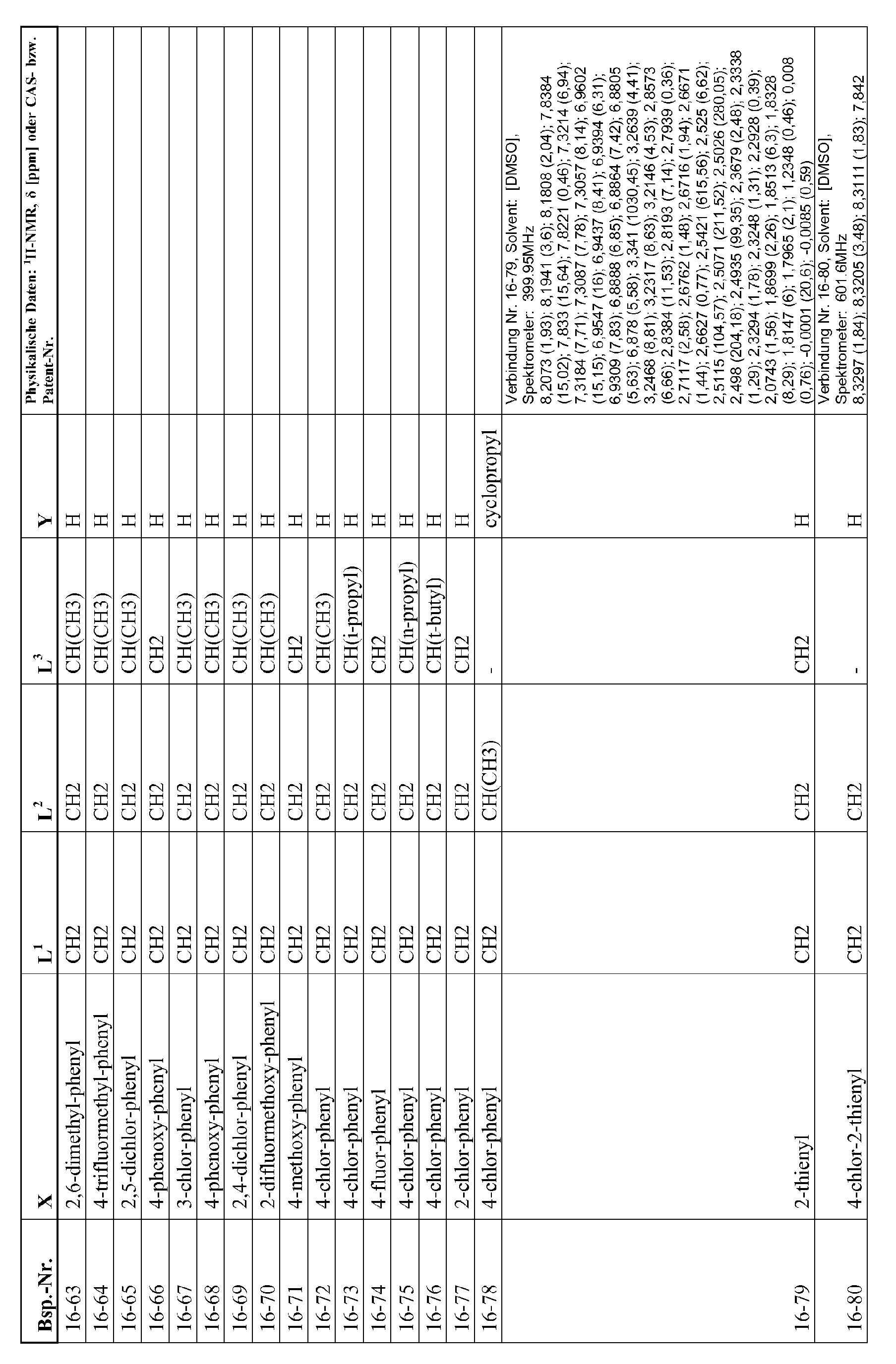

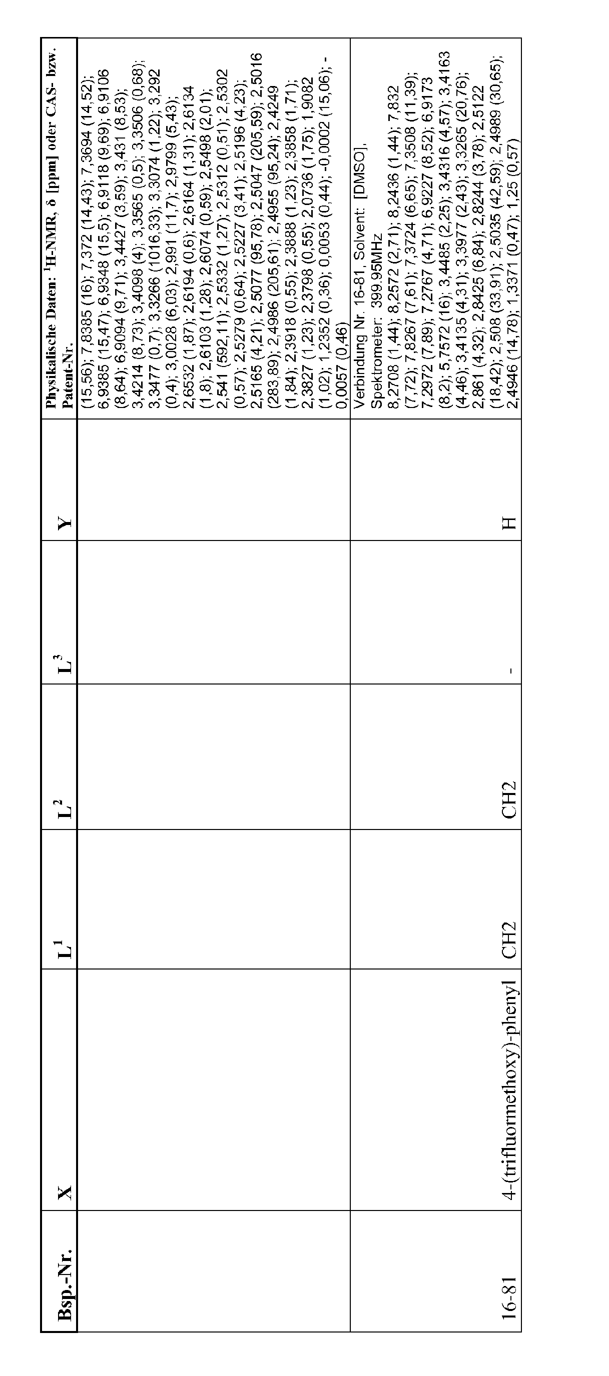

**Tabelle 17**

| | | | | | | |
|---|---|---|---|---|---|---|
| Verbindungen der Formel I-17 | | | | | | |
| 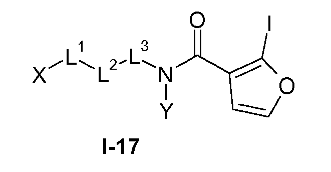 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 17-1 | 3-methyl-2-thienyl | CH2 | CH2 | - | H | |
| 17-2 | 2,4-dichlor-phenyl | CH2 | CH2 | CH2 | H | Verbindung Nr. 17-2, Solvent: [DMSO], Spektrometer: 399.95MHz 8,17 (2,06); 8,156 (3,99); 8,1419 (2,02); 7,9082 (15,62); 7,903 (15,74); 7,5666 (8,93); 7,5614 (9,26); 7,4288 (6,04); 7,4081 (13,21); 7,3941 (0,33); 7,38 (8,59); 7,3747 (7,99); 7,3593 (3,77); 7,354 (3,73); 6,8835 (0,33); 6,8734 (16); 6,8681 (15,76); 3,5158 (0,64); 3,3468 (21,57); 3,2552 (3,13); 3,238 (6,82); 3,2229 (6,82); 3,2056 (3,19); 2,7416 (5,76); 2,723 (7,22); 2,703 (6,2); 2,676 (0,33); 2,5461 (50,15); 2,5294 (0,61); 2,5246 (0,91); 2,5159 (11,4); 2,5114 (23,3); 2,5068 (31,17); 2,5021 (22,71); 2,4976 (10,75); 1,8075 (1,66); 1,7887 (4,29); 1,7703 (5,72); 1,752 (4,03); 1,7335 (1,48); -0,0002 (3,44) |
| 17-3 | 4-chlor-phenyl | CH2 | CH2 | - | H | [DMSO], Spektrometer: 399.95MHz |
| | | | | | | 8.2153 (0.36); 7.9050 (0.97); 7.8998 (0.97); 7.3625 (0.89); 7.3416 (1.44); 7.2769 (1.35); 7.2559 (0.85); 6.8470 (0.97); 6.8418 (0.97); 3.4907 (16.00); 3.4304 (0.43); 3.4133 (0.64); 3.3952 (0.63); 3.3796 (0.36); 2.8214 (0.50); 2.8027 (0.81); 2.7848 (0.45); 2.5552 (2.54); 2.5209 (1.35); 2.5165 (1.79); 2.5121 (1.37) |
| 17-4 | 2,4-dichlor-phenyl | CH2 | CH2 | - | H | Verbindung Nr. 17-4, Solvent: [DMSO], Spektrometer: 399.95MHz 8,2259 (1,64); 8,2118 (3,23); 8,1976 (1,63); 7,8966 (9,93); 7,8914 (10,05); 7,5854 (5,98); 7,582 (6,61); 7,3878 (1,24); 7,3834 (0,84); 7,3672 (9,54); 7,3621 (16); 7,3481 (0,42); 7,3404 (1,25); 6,8333 (10); 6,8281 (9,95); 5,7559 (1,39); 3,4479 (2,31); 3,4311 (5,33); 3,4153 (5,28); 3,3981 (2,6); 3,3352 (133,8); 2,9377 (4,89); 2,9197 (8,43); 2,9022 (4,3); 2,5262 (1,13); 2,513 (17,16); 2,5085 (33,81); 2,504 (44,7); 2,4994 (33,06); 2,495 (16,26); 1,9898 (0,89); 1,2497 (0,4); 1,1757 (0,5); -0,0002 (3,15) |
| 17-5 | 4-chlor-phenyl | CH(OCH3) | CH(CH3) | - | H | [DMSO], Spektrometer: 399.95MHz |
| | | | | | | 7.9346 (1.29); 7.9132 (1.34); 7.8986 (3.17); 7.8935 (3.16); 7.8787 (3.81); 7.8736 (3.79); 7.7754 (1.06); 7.7551 (1.08); 7.4397 (3.09); 7.4182 (5.40); 7.3952 (5.46); 7.3336 (4.88); 7.3125 (3.00); 7.2906 (3.65); 7.2696 (2.79); 6.9125 (3.03); 6.9073 (2.99); 6.8343 (3.71); 6.8291 (3.66); 4.3054 (1.47); 4.2903 (1.95); 4.2506 (1.89); 4.2351 (2.29); 4.2212 (0.50); 4.2041 (0.70); 4.1850 (0.65); 4.1687 (0.39); 4.0859 (0.54); 4.0694 (0.82); 4.0650 (0.67); 4.0527 (0.62); 4.0483 (0.81); 4.0319 (0.50); 3.3646 (214.06); 3.1876 (16.00); 3.1570 (13.27); 2.5446 (23.99); 2.5274 (0.78); 2.5141 (16.57); 2.5098 (32.70); 2.5053 (42.93); 2.5008 (32.15); 2.4965 (16.12); 1.1261 (6.08); 1.1092 (6.08); 0.9615 (4.80); 0.9444 (4.80); -0.0002 (5.26) |
| 17-6 | 2,4-dichlor-phenyl | CH(OCH3) | CH(CH3) | - | H | |
| 17-7 | 4-chlor-phenyl | CH(CH3) | CH2 | - | H | [DMSO], Spektrometer: 399.95MHz |
| | | | | | | 8.1462 (1.98); 8.1321 (3.29); 8.1178 (1.61); 7.9073 (0.57); 7.9023 (0.70); 7.8892 (10.09); 7.8840 (9.22); 7.3662 (8.69); 7.3614 (3.54); 7.3497 (5.91); 7.3449 (13.90); 7.3390 (2.04); 7.2904 (13.43); 7.2856 (4.05); 7.2735 (4.00); 7.2692 (7.52); 6.8214 (9.19); 6.8162 (8.32); 3.3817 (2.45); 3.3590 (43.45); 3.3418 (3.25); 3.3234 (4.50); 3.3080 (4.52); 3.2889 (2.72); 3.2744 (0.78); 3.2705 (0.79); 3.2561 (0.61); 3.0661 (0.45); 3.0486 (1.60); 3.0308 (2.90); 3.0130 (2.69); 2.9952 (1.22); 2.5650 (0.87); 2.5468 (23.00); 2.5162 (8.64); 2.5120 (14.46); 2.5075 (17.35); 2.5030 (12.20); 2.4987 (5.59); 2.0801 (0.53); 1.2175 (16.00); 1.2000 (14.95); -0.0002 (1.94) |
| 17-8 | 2,4-dichlor-phenyl | CH(CH3) | CH2 | - | H | [DMSO], Spektrometer: 399.95MHz |
| | | | | | | 8.1758 (1.77); 8.1613 (3.49); 8.1473 (1.75); 7.8860 (10.63); 7.8808 (10.75); 7.5603 (7.55); 7.5551 (8.06); 7.4693 (4.38); 7.4482 (10.22); 7.4326 (0.35); 7.4228 (6.21); 7.4175 (5.75); 7.4017 (2.58); 7.3964 (2.55); 6.8132 (9.78); 6.8079 (9.75); 3.5263 (1.20); 3.5088 (2.78); 3.4916 (3.14); 3.4744 (1.94); 3.4651 (1.63); 3.4505 (1.57); 3.4331 (2.82); 3.4176 (2.76); 3.4003 (1.59); 3.3809 (1.92); 3.3657 (3.32); 3.3534 (31.95); 3.3342 (1.68); 3.3171 (0.90); 2.5478 (30.07); 2.5310 (0.41); 2.5260 (0.57); 2.5174 (6.98); 2.5131 (14.06); 2.5085 (18.69); 2.5040 (14.09); 2.4997 (7.17); 2.0819 (0.45); 1.2090 (16.00); 1.1921 (15.93); -0.0002 (1.94) |
| 17-9 | 4-chlor-phenyl | CH2 | CH(CH3) | - | H | [DMSO], Spektrometer: 399.95MHz |
| | | | | | | 7.9341 (3.31); 7.9135 (3.39); 7.8967 (10.25); 7.8916 (10.27); 7.3383 (8.48); 7.3337 (3.34); 7.3218 (4.35); 7.3172 (14.38); 7.2595 (13.04); 7.2384 (7.87); 6.8857 (9.59); 6.8805 (9.52); 4.1614 (0.73); 4.1429 (1.62); 4.1255 (2.26); 4.1083 (1.70); 4.0898 (0.77); 3.3513 (29.33); 2.8463 (1.85); 2.8271 (1.84); 2.8129 (3.17); 2.7937 (3.09); 2.7358 (3.12); 2.7198 (3.22); 2.7023 (1.91); 2.6863 (1.79); 2.5461 (5.63); 2.5292 (0.35); 2.5156 (7.20); 2.5113 (14.43); 2.5068 (19.08); 2.5022 (14.38); 2.4980 (7.33); 1.1269 (16.00); 1.1103 (15.91); -0.0002 (1.86) |
| 17-10 | 2,4-dichlor-phenyl | CH2 | CH(CH3) | - | H | |
| 17-11 | 4-chlor-phenyl | C(CH3)2 | CH2 | - | H | [DMSO], Spektrometer: 399.95MHz |
| | | | | | | 7.8855 (3.71); 7.8804 (3.81); 7.8730 (0.69); 7.8567 (1.17); 7.8411 (0.58); 7.4474 (0.35); 7.4411 (2.83); 7.4360 (1.12); 7.4244 (1.45); 7.4192 (5.07); 7.4129 (0.85); 7.3742 (0.75); 7.3679 (5.04); 7.3629 (1.50); 7.3510 (1.08); 7.3460 (2.83); 6.8181 (3.59); 6.8129 (3.58); 3.3884 (3.34); 3.3724 (3.35); 3.3506 (6.88); 2.5459 (7.32); 2.5154 (2.28); 2.5111 (4.56); 2.5066 (6.03); 2.5021 (4.54); 2.4980 (2.31); 1.2846 (16.00); -0.0002 (0.52) |
| 17-12 | 2,4-dichlor-phenyl | C(CH3)2 | CH2 | - | H | [DMSO], Spektrometer: 399.95MHz |
| | | | | | | 7.8724 (3.99); 7.8673 (4.35); 7.8503 (1.31); 7.8345 (0.64); 7.5338 (2.43); 7.5281 (2.59); 7.4753 (1.85); 7.4536 (2.70); 7.3702 (1.69); 7.3644 (1.63); 7.3486 (1.18); 7.3428 (1.13); 6.7823 (3.77); 6.7771 (3.77); 3.7319 (3.38); 3.7159 (3.35); 3.3496 (8.21); 2.5471 (10.07); 2.5166 (2.36); 2.5123 (4.73); 2.5078 (6.26); 2.5033 (4.73); 2.4992 (2.42); 1.4336 (16.00); -0.0002 (0.46) |
| 17-13 | 2-chlor-phenyl | CH2 | CH2 | - | H | [DMSO], Spektrometer: 399.95MHz |
| | | | | | | 8.2730 (3.03); 8.2592 (5.74); 8.2452 (3.03); 7.9069 (15.65); 7.9018 (16.00); 7.4453 (6.11); 7.4414 (5.58); 7.4270 (7.86); 7.4226 (7.88); 7.4054 (0.35); 7.3586 (4.31); 7.3532 (5.37); 7.3400 (7.02); 7.3354 (9.00); 7.3203 (0.55); 7.3104 (2.79); 7.3064 (3.62); 7.2922 (8.16); 7.2882 (7.83); 7.2770 (8.74); 7.2751 (8.85); 7.2712 (9.60); 7.2588 (6.59); 7.2536 (5.94); 7.2404 (2.24); 7.2355 (1.85); 6.8678 (15.76); 6.8626 (15.98); 3.4600 (4.27); 3.4436 (8.81); 3.4274 (8.22); 3.4242 (8.90); 3.4090 (4.93); 3.3540 (25.43); 2.9612 (8.94); 2.9420 (13.13); 2.9244 (7.88); 2.5472 (38.06); 2.5301 (0.65); 2.5124 (18.26); 2.5080 (24.12); 2.5035 (18.65); 2.0811 (0.64); -0.0002 (1.98) |
| 17-14 | 3,4-dichlor-phenyl | CH2 | CH2 | - | H | Verbindung Nr. 17-14, Solvent: [DMSO], Spektrometer: 399.95MHz |
| | | | | | | 8,1863 (2,74); 8,1724 (5,32); 8,1584 (2,79); 7,9015 (15,73); 7,8963 (15,92); 7,5632 (0,34); 7,5532 (12,94); 7,5433 (0,69); 7,5327 (14,85); 7,5167 (12,06); 7,5118 (12,61); 7,2422 (7,04); 7,2372 (6,94); 7,2216 (6,22); 7,2166 (6,15); 6,8352 (16); 6,8299 (15,99); 5,7581 (3,62); 3,4468 (3,88); 3,4296 (9,23); 3,4144 (9,26); 3,3972 (4,27); 3,3301 (14,93); 2,8348 (7,68); 2,817 (14,34); 2,7994 (6,95); 2,5273 (0,91); 2,5139 (15,91); 2,5096 (31,41); 2,505 (41,48); 2,5005 (30,86); 2,4962 (15,39); 1,9909 (0,36); 1,3379 (0,44); 1,25 (0,54); -0,0002 (2,91) |
| 17-15 | 3,5-dichlor-phenyl | CH2 | CH2 | - | H | Verbindung Nr. 17-15, Solvent: [DMSO], Spektrometer: 399.95MHz |
| | | | | | | 8,1892 (1,68); 8,1751 (3,13); 8,1612 (1,62); 7,9024 (9,49); 7,8973 (9,57); 7,4372 (3,64); 7,4325 (7,17); 7,4277 (4,15); 7,3257 (0,45); 7,3109 (16); 7,3062 (15,6); 6,8462 (0,45); 6,841 (0,52); 6,8277 (9,35); 6,8225 (9,31); 3,4538 (2,27); 3,4366 (5,62); 3,4216 (5,74); 3,4045 (2,62); 3,3236 (18,65); 2,8444 (4,43); 2,8268 (8,57); 2,8093 (4,05); 2,6719 (0,42); 2,5251 (1,34); 2,5115 (21,91); 2,5072 (42,99); 2,5027 (56,75); 2,4982 (42,05); 2,4939 (20,75); 2,3296 (0,37); 1,3363 (0,39); 1,2496 (0,48); -0,0002 (3,95) |
| 17-16 | 3-chlor-phenyl | CH2 | CH2 | - | H | [DMSO], Spektrometer: 399.95MHz |
| | | | | | | 8.2249 (2.76); 8.2111 (5.34); 8.1973 (2.89); 7.9082 (15.77); 7.9031 (16.00); 7.3626 (0.46); 7.3478 (3.64); 7.3419 (1.08); 7.3285 (9.45); 7.3152 (11.43); 7.3100 (14.43); 7.2796 (7.56); 7.2629 (3.23); 7.2581 (3.52); 7.2126 (7.54); 7.1938 (5.32); 6.8562 (15.88); 6.8510 (15.93); 3.4459 (3.89); 3.4290 (8.27); 3.4112 (8.16); 3.3953 (4.50); 3.3551 (29.48); 2.8405 (7.57); 2.8220 (12.55); 2.8041 (7.01); 2.7848 (0.33); 2.5467 (37.80); 2.5302 (0.53); 2.5118 (17.03); 2.5074 (22.52); 2.5029 (17.23); 2.0805 (0.60); -0.0002 (1.57) |
| 17-17 | 2-fluor-phenyl | CH2 | CH2 | - | H | [DMSO], Spektrometer: 399.95MHz |
| | | | | | | 8.2765 (2.49); 8.2625 (4.74); 8.2484 (2.48); 7.9060 (15.79); 7.9008 (16.00); 7.3334 (2.38); 7.3295 (2.94); 7.3148 (4.61); 7.3105 (6.26); 7.3033 (1.99); 7.2983 (2.42); 7.2908 (4.50); 7.2847 (3.66); 7.2698 (3.00); 7.2645 (4.26); 7.2601 (2.35); 7.2507 (2.44); 7.2463 (1.93); 7.1821 (4.50); 7.1585 (10.87); 7.1415 (8.18); 7.1388 (9.95); 7.1233 (3.36); 7.1205 (2.96); 6.8587 (14.47); 6.8534 (14.58); 3.4380 (3.67); 3.4216 (7.24); 3.4051 (6.62); 3.4017 (7.33); 3.3865 (4.13); 3.3538 (21.37); 2.8670 (6.33); 2.8483 (9.90); 2.8300 (5.64); 2.5471 (40.41); 2.5304 (0.47); 2.5255 (0.62); 2.5167 (7.78); 2.5124 (15.88); 2.5078 (21.29); 2.5033 (16.19); 2.4991 (8.35); 2.0807 (0.44); -0.0002 (1.98) |
| 17-18 | 2,6-difluor-phenyl | CH2 | CH2 | - | H | [DMSO], Spektrometer: 399.95MHz |
| | | | | | | 8.3342 (2.88); 8.3196 (5.58); 8.3051 (2.87); 7.9026 (15.84); 7.8975 (16.00); 7.3664 (1.21); 7.3493 (2.96); 7.3460 (2.91); 7.3287 (5.48); 7.3081 (3.63); 7.2912 (1.61); 7.1040 (1.12); 7.0917 (8.22); 7.0720 (12.64); 7.0609 (2.02); 7.0523 (6.99); 7.0393 (1.04); 6.8308 (15.75); 6.8256 (15.91); 3.4058 (3.86); 3.3893 (8.82); 3.3709 (8.98); 3.3545 (32.02); 2.8820 (6.36); 2.8638 (10.95); 2.8461 (5.71); 2.5491 (41.65); 2.5323 (0.55); 2.5142 (16.83); 2.5098 (22.31); 2.5054 (17.15); 2.0827 (0.67); -0.0002 (1.48) |
| 17-19 | 2,6-dichlor-phenyl | CH2 | CH2 | - | H | [DMSO], Spektrometer: 399.95MHz |
| | | | | | | 8.3288 (1.55); 8.3145 (3.12); 8.3000 (1.57); 7.8978 (9.08); 7.8927 (9.37); 7.4685 (11.72); 7.4484 (16.00); 7.3046 (5.36); 7.2854 (5.37); 7.2835 (5.45); 7.2643 (3.55); 6.8498 (9.10); 6.8446 (9.27); 3.4357 (1.95); 3.4193 (4.69); 3.4001 (4.74); 3.3850 (2.68); 3.3443 (255.13); 3.3124 (0.63); 3.1460 (4.54); 3.1268 (6.78); 3.1098 (3.60); 2.6771 (0.46); 2.6727 (0.64); 2.6681 (0.49); 2.5429 (48.46); 2.5260 (1.90); 2.5124 (34.75); 2.5080 (70.93); 2.5035 (95.37); 2.4990 (72.98); 2.4948 (38.02); 2.3347 (0.46); 2.3303 (0.64); 2.3259 (0.48); 2.0770 (0.45); -0.0002 (2.21) |
| 17-20 | 3-(trifluormethyl)-phenyl | CH2 | CH2 | - | H | [DMSO], Spektrometer: 399.95MHz |
| | | | | | | 8.2350 (2.48); 8.2211 (4.60); 8.2072 (2.38); 7.9189 (0.55); 7.9089 (14.27); 7.9037 (14.51); 7.6742 (0.38); 7.6541 (0.53); 7.5885 (10.40); 7.5657 (7.60); 7.5527 (16.00); 7.5341 (3.06); 7.5164 (0.99); 7.4822 (0.46); 7.4624 (0.43); 6.8563 (0.76); 6.8452 (13.05); 6.8400 (13.21); 3.4790 (3.41); 3.4619 (7.67); 3.4465 (7.57); 3.4288 (3.97); 3.3577 (26.92); 2.9395 (6.60); 2.9214 (11.46); 2.9036 (5.97); 2.5490 (41.45); 2.5323 (0.52); 2.5274 (0.63); 2.5185 (8.02); 2.5142 (16.31); 2.5097 (21.74); 2.5052 (16.57); 2.5010 (8.61); 2.0822 (0.92); -0.0002 (1.78) |
| 17-21 | 4-(trifluormethyl)-phenyl | CH2 | CH2 | - | H | [DMSO], Spektrometer: 399.95MHz |
| | | | | | | 8.2544 (2.57); 8.2406 (4.94); 8.2266 (2.54); 7.9113 (15.88); 7.9061 (16.00); 7.6742 (10.65); 7.6540 (12.96); 7.4830 (12.41); 7.4629 (10.58); 6.8595 (13.45); 6.8543 (13.40); 3.4818 (3.52); 3.4648 (7.59); 3.4474 (7.40); 3.4313 (3.94); 3.3601 (18.84); 2.9325 (6.19); 2.9142 (10.62); 2.8963 (5.49); 2.5504 (41.32); 2.5337 (0.44); 2.5289 (0.58); 2.5201 (6.63); 2.5157 (13.54); 2.5112 (18.14); 2.5066 (13.67); 2.5023 (6.94); 2.0836 (1.04); -0.0002 (1.85) |
| 17-22 | 2-methyl-phenyl | CH2 | CH2 | - | H | [DMSO], Spektrometer: 399.95MHz |
| | | | | | | 8.2893 (0.78); 8.2753 (1.48); 8.2612 (0.77); 7.9142 (4.40); 7.9091 (4.51); 7.1690 (1.52); 7.1570 (2.32); 7.1455 (4.19); 7.1396 (1.94); 7.1285 (3.49); 7.1242 (4.10); 7.1147 (3.00); 7.1085 (1.64); 7.1054 (1.45); 7.1028 (1.43); 6.8805 (4.36); 6.8752 (4.36); 3.3764 (1.19); 3.3612 (2.28); 3.3566 (2.37); 3.3480 (8.87); 3.3377 (2.36); 3.3231 (1.32); 2.8169 (2.38); 2.8019 (2.00); 2.7969 (2.70); 2.7781 (2.12); 2.5442 (10.51); 2.5136 (3.09); 2.5093 (6.18); 2.5048 (8.22); 2.5003 (6.24); 2.4961 (3.22); 2.3238 (16.00); -0.0002 (1.07) |
| 17-23 | 2,4,6-trimethyl-phenyl | CH2 | CH2 | - | H | [DMSO], Spektrometer: 399.95MHz |
| | | | | | | 8.3716 (0.56); 8.3571 (1.13); 8.3425 (0.56); 7.9246 (3.20); 7.9195 (3.24); 6.8969 (3.16); 6.8916 (3.15); 6.8093 (5.25); 3.3493 (5.65); 3.2268 (0.63); 3.2122 (1.13); 3.1985 (1.13); 3.1914 (0.95); 3.1852 (1.19); 3.1712 (0.75); 2.7842 (1.35); 2.7708 (1.09); 2.7631 (1.36); 2.7581 (1.16); 2.7433 (1.14); 2.5445 (14.01); 2.5140 (1.84); 2.5097 (3.72); 2.5052 (4.96); 2.5007 (3.75); 2.3013 (16.00); 2.1841 (8.24); -0.0002 (0.61) |
| 17-24 | 3,4-bismethoxy-phenyl | CH2 | CH2 | - | H | [DMSO], Spektrometer: 399.95MHz |
| | | | | | | 8.1934 (0.77); 8.1795 (1.52); 8.1655 (0.79); 7.9109 (4.42); 7.9057 (4.46); 6.8844 (4.25); 6.8791 (4.46); 6.8754 (3.05); 6.8548 (3.36); 6.8202 (2.95); 6.8155 (3.33); 6.7501 (1.89); 6.7454 (1.75); 6.7298 (1.41); 6.7250 (1.34); 3.7297 (16.00); 3.7147 (15.91); 3.4141 (0.94); 3.3979 (1.88); 3.3779 (1.94); 3.3621 (1.32); 3.3532 (6.27); 2.7580 (1.87); 2.7385 (2.73); 2.7207 (1.71); 2.5453 (11.49); 2.5149 (2.00); 2.5106 (4.07); 2.5061 (5.45); 2.5016 (4.16); - 0.0002 (0.64) |
| 17-25 | phenyl | CH2 | CH2 | - | H | [DMSO], Spektrometer: 399.95MHz |
| | | | | | | 8.2349 (2.45); 8.2211 (4.64); 8.2072 (2.45); 7.9072 (15.16); 7.9020 (15.41); 7.3229 (4.79); 7.3045 (12.58); 7.2862 (12.23); 7.2470 (16.00); 7.2285 (11.44); 7.2071 (6.59); 7.1926 (1.60); 7.1892 (2.35); 6.8730 (15.16); 6.8677 (15.29); 3.4314 (3.88); 3.4157 (6.90); 3.3985 (6.16); 3.3942 (7.30); 3.3793 (4.36); 3.3488 (27.85); 2.8251 (7.51); 2.8053 (10.01); 2.7873 (6.71); 2.5441 (33.83); 2.5272 (0.54); 2.5136 (9.76); 2.5093 (19.52); 2.5048 (25.88); 2.5003 (19.58); 2.4961 (10.02); 2.0776 (0.54); -0.0002 (3.28) |
| 17-26 | 4-chlor-phenyl | C(CH2-CH2) | CH2 | - | H | |
| 17-27 | 2,4-dichlor-phenyl | C(CH2-CH2) | CH2 | - | H | |
| 17-28 | 4-chlor-phenyl | CH2 | C(CH2-CH2) | - | H | |
| 17-29 | 2,4-dichlor-phenyl | CH2 | C(CH2-CH2) | - | H | |
| 17-30 | 4-chlor-phenyl | O | CH2 | CH2 | H | |
| 17-31 | 2,4-dichlor-phenyl | O | CH2 | CH2 | H | Verbindung Nr. 17-31, Solvent: [DMSO], Spektrometer: 399.95MHz 8,3198 (2,68); 8,3063 (5,28); 8,2928 (2,73); 7,919 (13,13); 7,9139 (13,58); 7,5705 (11,09); 7,5641 (12,19); 7,3831 (5,65); 7,3766 (5,45); 7,3609 (7,7); 7,3545 (7,63); 7,2428 (12,91); 7,2205 (9,58); 6,9026 (13,27); 6,8974 (13,37); 4,1934 (7,15); 4,1785 (16); 4,1638 (7,93); 3,6025 (3,89); 3,5881 (10,79); 3,5737 (10,5); 3,5591 (3,6); 3,3328 (59,36); 2,7136 (0,32); 2,5439 (68,35); 2,5274 (1,08); 2,509 (33,15); 2,5046 (44,31); 2,5002 (34,03); 2,3703 (0,33); -0,0002 (1,74) |
| 17-32 | 4-chlor-phenyl | CH2 | CH2 | CH2 | H | |
| 17-33 | 4-chlor-phenyl | NCH3 | CH2 | CH2 | H | |
| 17-34 | 2,4-dichlor-phenyl | NCH3 | CH2 | CH2 | H | |
| 17-35 | 4-chlor-phenyl | CH(OCH3) | CH2 | - | H | |
| 17-36 | 2,4-dichlor-phenyl | CH(OCH3) | CH2 | - | H | |
| 17-37 | 2-thienyl | CH2 | CH2 | - | H | [DMSO], Spektrometer: 399.95MHz |
| | | | | | | 8.2894 (2.91); 8.2758 (5.41); 8.2620 (2.89); 7.9117 (15.02); 7.9066 (14.86); 7.7491 (0.38); 7.7366 (0.42); 7.7308 (0.43); 7.7212 (0.44); 7.3494 (7.92); 7.3467 (7.79); 7.3367 (8.65); 7.3340 (8.01); 7.1526 (0.35); 7.1430 (0.39); 6.9710 (6.64); 6.9625 (9.30); 6.9585 (7.00); 6.9498 (8.54); 6.9151 (9.96); 6.9070 (7.48); 6.8780 (16.00); 6.8728 (15.62); 3.4726 (0.57); 3.4472 (4.55); 3.4297 (9.92); 3.4147 (10.07); 3.3965 (5.26); 3.3313 (111.52); 3.0670 (0.51); 3.0409 (9.27); 3.0226 (15.47); 3.0045 (7.69); 2.6758 (0.66); 2.6713 (0.85); 2.6671 (0.63); 2.5067 (96.28); 2.5024 (121.05); 2.4980 (87.99); 2.3290 (0.81); 2.3246 (0.60); 1.3974 (3.19); 0.0078 (1.41); -0.0002 (26.06); -0.0084 (1.01) |
| 17-38 | 3-thienyl | CH2 | CH2 | - | H | |
| 17-39 | 2-furyl | CH2 | CH2 | - | H | |
| 17-40 | 3-furyl | CH2 | CH2 | - | H | |
| 17-41 | phenyl | CH2 | CH2 | CH(CH3) | H | [DMSO], Spektrometer: 399.95MHz |
| | | | | | | 7.9127 (9.50); 7.9076 (9.66); 7.8923 (3.15); 7.8716 (3.10); 7.2928 (3.11); 7.2742 (8.22); 7.2560 (7.67); 7.2169 (9.97); 7.1996 (5.58); 7.1882 (2.37); 7.1850 (2.88); 7.1671 (4.16); 7.1525 (1.07); 7.1492 (1.57); 6.9296 (9.21); 6.9244 (9.27); 3.9844 (0.63); 3.9644 (1.41); 3.9487 (1.90); 3.9328 (1.47); 3.9127 (0.67); 3.3512 (9.38); 2.6692 (0.44); 2.6533 (0.55); 2.6454 (0.47); 2.6346 (1.92); 2.6197 (3.59); 2.6113 (2.75); 2.6028 (2.93); 2.5963 (3.75); 2.5804 (2.06); 2.5688 (0.52); 2.5629 (0.67); 2.5442 (27.24); 2.5274 (0.48); 2.5134 (7.30); 2.5093 (14.43); 2.5048 (18.94); 2.5003 (14.42); 1.8535 (0.47); 1.8371 (0.61); 1.8313 (0.86); 1.8196 (1.20); 1.8100 (0.88); 1.8042 (1.22); 1.7971 (1.74); 1.7821 (1.43); 1.7765 (1.30); 1.7605 (1.65); 1.7444 (1.58); 1.7364 (1.11); 1.7292 (1.54); 1.7217 (1.54); 1.7096 (1.05); 1.7053 (1.30); 1.6957 (0.64); 1.6887 (0.68); 1.6722 (0.41); 1.1507 (16.00); 1.1341 (15.91); -0.0002 (2.95) |
| 17-42 | phenyl | CH2 | CH2 | CH2 | H | [DMSO], Spektrometer: 399.95MHz |
| | | | | | | 8.1368 (2.49); 8.1231 (4.66); 8.1094 (2.50); 7.9090 (14.35); 7.9038 (14.46); 7.3063 (4.74); 7.2877 (13.13); 7.2695 (12.50); 7.2351 (16.00); 7.2178 (8.62); 7.1973 (4.41); 7.1795 (6.60); 7.1616 (2.42); 6.8846 (14.75); 6.8794 (14.76); 3.3474 (19.82); 3.2286 (3.90); 3.2114 (8.33); 3.1960 (8.33); 3.1853 (1.91); 3.1788 (4.15); 2.6414 (7.03); 2.6225 (10.63); 2.6029 (7.70); 2.5441 (42.85); 2.5275 (0.71); 2.5093 (22.43); 2.5048 (29.37); 2.5004 (22.20); 1.8273 (2.15); 1.8084 (5.85); 1.7900 (8.00); 1.7714 (5.53); 1.7531 (1.93); - 0.0002 (4.50) |
| 17-43 | 2-Cl-phenyl | CH2 | CH2 | CH(CH3) | H | [DMSO], Spektrometer: 399.95MHz |
| | | | | | | 7.9294 (3.33); 7.9154 (10.19); 7.9102 (11.89); 7.4127 (3.68); 7.4094 (3.74); 7.3935 (4.75); 7.3900 (4.76); 7.3633 (2.79); 7.3589 (3.23); 7.3446 (4.33); 7.3403 (4.71); 7.2924 (1.90); 7.2890 (2.15); 7.2741 (4.59); 7.2706 (4.36); 7.2558 (2.99); 7.2519 (2.61); 7.2437 (3.44); 7.2388 (3.45); 7.2246 (3.72); 7.2200 (3.70); 7.2059 (1.49); 7.2014 (1.35); 6.9319 (9.09); 6.9267 (9.20); 4.0286 (0.72); 4.0116 (1.55); 3.9926 (2.08); 3.9751 (1.67); 3.9575 (0.77); 3.3505 (12.19); 2.7561 (0.48); 2.7433 (2.36); 2.7376 (2.68); 2.7261 (3.82); 2.7159 (4.23); 2.7036 (2.81); 2.6978 (2.63); 2.6850 (0.55); 2.6768 (0.33); 2.5467 (22.42); 2.5297 (0.50); 2.5158 (6.75); 2.5118 (13.34); 2.5073 (17.62); 2.5028 (13.49); 1.7964 (0.38); 1.7844 (1.32); 1.7755 (1.58); 1.7658 (3.15); 1.7483 (3.08); 1.7459 (3.09); 1.7367 (2.84); 1.7262 (1.66); 1.7194 (1.44); 1.7086 (0.37); 1.1794 (16.00); 1.1628 (15.97); -0.0002 (2.80) |
| 17-44 | 4-t-butyl-phenyl | CH2 | CH2 | CH2 | H | [DMSO], Spektrometer: 399.95MHz |
| | | | | | | 8.1086 (0.61); 7.9056 (1.74); 7.9005 (1.76); 7.3061 (1.74); 7.2855 (2.27); 7.1515 (2.05); 7.1309 (1.61); 6.8779 (1.72); 6.8727 (1.74); 3.3462 (2.94); 3.2216 (0.43); 3.2046 (0.95); 3.1888 (0.95); 3.1719 (0.45); 2.5985 (0.77); 2.5797 (1.25); 2.5605 (0.84); 2.5442 (1.83); 2.5093 (2.63); 2.5049 (3.45); 2.5005 (2.61); 1.7923 (0.67); 1.7740 (0.91); 1.7556 (0.63); 1.2603 (16.00); - 0.0002 (0.55) |
| 17-45 | 4-methyl-phenyl | CH2 | CH2 | CH2 | H | [DMSO], Spektrometer: 399.95MHz |
| | | | | | | 8.1177 (0.84); 8.1040 (1.61); 8.0903 (0.85); 7.9061 (4.81); 7.9010 (4.85); 7.1188 (1.26); 7.1127 (0.73); 7.0976 (11.87); 7.0932 (11.01); 7.0718 (1.22); 6.8801 (4.97); 6.8749 (4.98); 3.3461 (6.49); 3.2100 (1.25); 3.1929 (2.69); 3.1773 (2.70); 3.1602 (1.33); 2.5896 (2.25); 2.5708 (3.58); 2.5511 (2.73); 2.5438 (14.58); 2.5090 (7.74); 2.5046 (10.12); 2.5001 (7.69); 2.2601 (16.00); 1.7966 (0.68); 1.7777 (1.91); 1.7595 (2.63); 1.7409 (1.81); 1.7227 (0.62); -0.0002 (1.63) |
| 17-46 | phenyl | CH2 | CH2 | CH(CH2CH3) | H | [DMSO], Spektrometer: 399.95MHz |
| | | | | | | 7.9184 (8.52); 7.9133 (8.64); 7.7938 (3.22); 7.7719 (3.28); 7.2878 (3.07); 7.2691 (8.26); 7.2509 (7.54); 7.2127 (10.10); 7.1952 (5.70); 7.1806 (2.98); 7.1626 (4.18); 7.1447 (1.57); 6.9428 (8.82); 6.9377 (9.07); 3.8539 (1.00); 3.8401 (1.66); 3.8339 (1.41); 3.8198 (1.75); 3.8061 (0.98); 3.3471 (13.64); 2.6696 (0.69); 2.6524 (0.92); 2.6482 (0.87); 2.6349 (1.70); 2.6159 (2.42); 2.5958 (1.85); 2.5907 (1.82); 2.5685 (2.70); 2.5447 (23.34); 2.5097 (13.17); 2.5054 (17.07); 2.5011 (13.39); 1.7774 (2.16); 1.7576 (4.32); 1.7388 (3.59); 1.7239 (1.48); 1.7170 (1.31); 1.5828 (0.69); 1.5686 (1.07); 1.5650 (1.06); 1.5493 (1.95); 1.5353 (1.66); 1.5305 (1.79); 1.5170 (1.55); 1.4940 (1.64); 1.4747 (2.32); 1.4557 (1.94); 1.4401 (1.34); 1.4213 (0.83); 0.8711 (7.89); 0.8527 (16.00); 0.8341 (7.15); -0.0002 (2.47) |
| 17-47 | 2-methoxy-phenyl | CH2 | CH2 | CH2 | H | [DMSO], Spektrometer: 399.95MHz |
| | | | | | | 8.1191 (0.73); 8.1052 (1.39); 8.0914 (0.74); 7.9055 (3.72); 7.9003 (3.84); 7.1997 (0.68); 7.1959 (0.87); 7.1764 (1.97); 7.1702 (1.96); 7.1571 (1.61); 7.1515 (2.23); 6.9530 (2.31); 6.9330 (2.00); 6.8845 (4.80); 6.8793 (4.01); 6.8679 (2.33); 6.8494 (1.04); 3.7736 (16.00); 3.3485 (7.03); 3.2187 (0.99); 3.2016 (2.09); 3.1848 (2.13); 3.1682 (1.07); 2.6032 (1.84); 2.5844 (2.84); 2.5652 (2.04); 2.5445 (9.41); 2.5094 (5.94); 2.5051 (7.84); 2.5009 (6.13); 1.7730 (0.53); 1.7537 (1.48); 1.7357 (2.10); 1.7168 (1.40); 1.6986 (0.49); - 0.0002 (0.98) |
| 17-48 | 2-methyl-phenyl | CH2 | CH2 | CH2 | H | [DMSO], Spektrometer: 399.95MHz |
| | | | | | | 8.1622 (0.78); 8.1484 (1.45); 8.1346 (0.77); 7.9092 (4.51); 7.9041 (4.61); 7.1697 (0.97); 7.1650 (1.27); 7.1475 (2.45); 7.1254 (2.49); 7.1218 (2.59); 7.1119 (2.40); 7.0959 (3.43); 7.0908 (2.68); 7.0787 (1.31); 7.0746 (1.21); 7.0609 (0.43); 7.0568 (0.36); 6.8814 (4.59); 6.8763 (4.67); 3.3412 (12.11); 3.2653 (1.11); 3.2482 (2.59); 3.2328 (2.58); 3.2158 (1.19); 2.6194 (2.08); 2.6005 (2.71); 2.5803 (2.29); 2.5427 (15.63); 2.5258 (0.38); 2.5078 (13.05); 2.5033 (17.27); 2.4989 (13.26); 2.2571 (16.00); 1.7732 (0.61); 1.7544 (1.62); 1.7357 (2.13); 1.7170 (1.52); 1.6987 (0.55); -0.0002 (2.60) |
| 17-49 | 3-methyl-phenyl | CH2 | CH2 | CH2 | H | [DMSO], Spektrometer: 399.95MHz |
| | | | | | | 8.1261 (0.87); 8.1124 (1.65); 8.0986 (0.88); 7.9076 (4.97); 7.9024 (5.13); 7.1833 (1.42); 7.1646 (3.33); 7.1459 (2.14); 7.0322 (3.37); 7.0168 (2.30); 6.9972 (3.66); 6.9779 (1.70); 6.8828 (5.15); 6.8776 (5.29); 3.3453 (10.30); 3.2197 (1.29); 3.2025 (2.80); 3.1868 (2.80); 3.1699 (1.38); 2.5970 (2.32); 2.5781 (3.54); 2.5585 (2.58); 2.5436 (12.31); 2.5087 (8.59); 2.5042 (11.48); 2.4998 (8.92); 2.2758 (16.00); 1.8111 (0.70); 1.7921 (1.94); 1.7738 (2.66); 1.7551 (1.84); 1.7370 (0.65); -0.0002 (1.52) |
| 17-50 | 3-chlor-phenyl | CH2 | CH2 | CH2 | H | [DMSO], Spektrometer: 399.95MHz |
| | | | | | | 8.1375 (2.05); 8.1238 (3.89); 8.1101 (2.08); 7.9127 (11.29); 7.9075 (11.48); 7.3386 (3.08); 7.3190 (16.00); 7.3000 (6.92); 7.2569 (5.46); 7.2366 (2.93); 7.2157 (5.86); 7.1966 (4.09); 6.8827 (11.57); 6.8775 (11.66); 3.3505 (14.94); 3.2206 (3.03); 3.2035 (6.85); 3.1881 (6.98); 3.1712 (3.18); 2.6569 (5.36); 2.6381 (8.51); 2.6187 (5.85); 2.5466 (34.11); 2.5298 (0.52); 2.5117 (16.03); 2.5073 (20.99); 2.5029 (16.00); 1.8282 (1.62); 1.8096 (4.68); 1.7911 (6.33); 1.7727 (4.47); 1.7546 (1.47); -0.0002 (2.94) |
| 17-51 | 2,6-difluor-phenyl | CH2 | CH2 | CH2 | H | [DMSO], Spektrometer: 399.95MHz |
| | | | | | | 8.1763 (2.84); 8.1625 (5.46); 8.1486 (2.86); 7.9383 (0.32); 7.9332 (0.36); 7.9093 (15.44); 7.9041 (15.62); 7.3514 (1.34); 7.3344 (3.16); 7.3310 (3.01); 7.3137 (5.89); 7.2930 (3.89); 7.2761 (1.74); 7.1232 (0.33); 7.1063 (0.84); 7.1021 (1.22); 7.0893 (8.41); 7.0694 (13.19); 7.0496 (6.96); 7.0360 (0.98); 6.8676 (15.70); 6.8624 (16.00); 6.5148 (0.56); 3.3516 (23.80); 3.2443 (3.92); 3.2273 (8.04); 3.2098 (8.05); 3.1934 (4.14); 2.6878 (5.86); 2.6688 (9.90); 2.6494 (6.31); 2.5483 (49.22); 2.5313 (0.73); 2.5134 (22.83); 2.5090 (30.10); 2.5046 (23.13); 2.0825 (0.49); 1.7846 (2.16); 1.7653 (5.68); 1.7470 (8.12); 1.7281 (5.29); 1.7096 (1.91); -0.0002 (4.53) |
| 17-52 | 4-chlor-phenyl | CH2 | CH2 | CH2 | H | [DMSO], Spektrometer: 399.95MHz |
| | | | | | | 8.1353 (1.90); 8.1216 (3.60); 8.1078 (1.91); 7.9108 (10.72); 7.9057 (10.81); 7.3480 (9.46); 7.3434 (3.76); 7.3315 (4.99); 7.3269 (16.00); 7.2703 (14.47); 7.2492 (8.63); 6.8796 (10.90); 6.8744 (10.94); 3.3507 (15.76); 3.2175 (2.79); 3.2005 (6.27); 3.1852 (6.32); 3.1681 (2.92); 2.6374 (4.98); 2.6186 (7.80); 2.5992 (5.41); 2.5465 (32.60); 2.5296 (0.49); 2.5117 (14.92); 2.5072 (19.57); 2.5028 (14.83); 1.8128 (1.51); 1.7942 (4.29); 1.7758 (5.78); 1.7573 (4.06); 1.7392 (1.34); -0.0002 (3.23) |
| 17-53 | 2,6-dichlor-phenyl | CH2 | CH2 | CH(CH3) | H | [DMSO], Spektrometer: 399.95MHz |
| | | | | | | 7.9701 (3.26); 7.9492 (3.32); 7.9137 (9.17); 7.9086 (9.30); 7.4476 (12.36); 7.4274 (16.00); 7.2717 (5.17); 7.2522 (5.98); 7.2315 (3.53); 6.9302 (9.18); 6.9251 (9.17); 4.1088 (0.72); 4.0919 (1.62); 4.0745 (1.94); 4.0552 (1.61); 4.0384 (0.74); 3.3437 (20.97); 2.9196 (0.41); 2.9041 (3.32); 2.8834 (6.19); 2.8696 (1.83); 2.8624 (3.70); 2.8464 (0.44); 2.5456 (27.97); 2.5285 (0.67); 2.5107 (23.37); 2.5062 (30.62); 2.5018 (23.06); 1.7293 (0.38); 1.7136 (1.61); 1.7077 (1.48); 1.6987 (2.26); 1.6902 (2.74); 1.6776 (2.43); 1.6700 (2.57); 1.6574 (1.45); 1.6485 (1.28); 1.6332 (0.41); 1.2153 (15.51); 1.1986 (15.42); -0.0002 (4.88) |
| 17-54 | 3,5-dichlor-phenyl | CH2 | CH2 | CH(CH3) | H | [DMSO], Spektrometer: 399.95MHz |
| | | | | | | 7.9154 (9.25); 7.9103 (9.32); 7.8677 (3.43); 7.8470 (3.49); 7.3985 (3.80); 7.3942 (6.77); 7.3898 (4.20); 7.3043 (15.61); 7.2998 (14.40); 6.9135 (9.28); 6.9083 (9.30); 3.9652 (0.67); 3.9456 (1.43); 3.9297 (1.98); 3.9136 (1.53); 3.8938 (0.71); 3.3504 (12.26); 2.6914 (0.37); 2.6697 (0.64); 2.6568 (1.91); 2.6414 (3.71); 2.6339 (2.49); 2.6197 (3.97); 2.6028 (2.04); 2.5894 (0.69); 2.5680 (0.47); 2.5481 (23.90); 2.5310 (0.42); 2.5133 (13.84); 2.5089 (18.18); 2.5044 (13.87); 1.8187 (0.39); 1.8135 (0.60); 1.8011 (1.23); 1.7803 (3.13); 1.7629 (3.66); 1.7503 (1.72); 1.7430 (2.69); 1.7261 (1.32); 1.7141 (0.46); 1.7070 (0.42); 1.1463 (16.00); 1.1297 (15.94); -0.0002 (2.95) |
| 17-55 | 2,6-dimethyl-phenyl | CH2 | CH2 | CH2 | H | [DMSO], Spektrometer: 399.95MHz |
| | | | | | | 8.2139 (0.48); 8.1999 (0.94); 8.1859 (0.48); 7.9149 (2.54); 7.9097 (2.55); 6.9585 (8.29); 6.8842 (2.65); 6.8790 (2.68); 3.3478 (3.48); 3.3182 (0.64); 3.3015 (1.64); 3.2862 (1.65); 3.2695 (0.66); 2.6167 (1.17); 2.6033 (0.97); 2.5962 (1.23); 2.5881 (0.97); 2.5752 (1.26); 2.5441 (6.22); 2.5093 (3.69); 2.5049 (4.78); 2.5006 (3.64); 2.2571 (16.00); 1.6572 (0.33); 1.6395 (0.82); 1.6275 (0.83); 1.6191 (0.96); 1.6117 (0.84); 1.5990 (0.79); -0.0002 (0.77) |
| 17-56 | 2,5-dichlor-phenyl | CH2 | CH2 | CH2 | H | [DMSO], Spektrometer: 399.95MHz |
| | | | | | | 8.1790 (2.75); 8.1651 (5.42); 8.1511 (2.80); 7.9110 (15.55); 7.9058 (16.00); 7.5056 (11.96); 7.4992 (12.95); 7.4642 (11.69); 7.4428 (15.63); 7.3259 (8.07); 7.3194 (7.73); 7.3045 (6.07); 7.2980 (5.83); 6.8747 (15.71); 6.8695 (15.98); 3.3409 (54.02); 3.2545 (3.74); 3.2375 (9.03); 3.2220 (9.06); 3.2051 (3.98); 2.7480 (7.09); 2.7293 (9.79); 2.7096 (7.77); 2.6778 (0.48); 2.6734 (0.61); 2.6691 (0.46); 2.5438 (60.65); 2.5264 (1.86); 2.5087 (62.90); 2.5043 (83.39); 2.4998 (63.85); 2.3354 (0.40); 2.3311 (0.54); 2.3267 (0.41); 1.8158 (1.98); 1.7974 (5.61); 1.7788 (7.53); 1.7601 (5.39); 1.7422 (1.81); 0.0080 (0.47); -0.0002 (13.70); -0.0083 (0.63) |
| 17-57 | 4-i-propoxy-phenyl | CH2 | CH2 | CH2 | H | [DMSO], Spektrometer: 399.95MHz |
| | | | | | | 8.1129 (0.67); 8.0991 (1.30); 8.0853 (0.68); 7.9061 (3.64); 7.9010 (3.71); 7.1187 (3.51); 7.0973 (4.07); 6.8816 (3.64); 6.8764 (3.67); 6.8325 (0.54); 6.8255 (4.39); 6.8041 (3.88); 4.5717 (0.50); 4.5566 (1.28); 4.5415 (1.73); 4.5265 (1.30); 4.5114 (0.52); 3.3459 (5.03); 3.2119 (0.89); 3.1949 (1.96); 3.1790 (1.96); 3.1622 (0.95); 2.5618 (1.61); 2.5448 (11.46); 2.5237 (1.88); 2.5143 (2.54); 2.5100 (4.88); 2.5056 (6.48); 2.5011 (4.98); 1.7872 (0.47); 1.7683 (1.36); 1.7501 (1.85); 1.7317 (1.29); 1.7135 (0.43); 1.2464 (16.00); 1.2313 (16.00); -0.0002 (1.10) |
| 17-58 | 3-trifluormethyl-phenyl | CH2 | CH2 | CH(CH3) | H | [DMSO], Spektrometer: 399.95MHz |
| | | | | | | 7.9177 (9.64); 7.9126 (10.03); 7.9032 (3.64); 7.8824 (3.43); 7.6218 (0.39); 7.5774 (6.16); 7.5595 (1.29); 7.5335 (12.02); 7.5299 (12.17); 7.5189 (2.87); 7.5029 (0.62); 7.4926 (0.64); 7.4584 (0.35); 6.9287 (9.37); 6.9235 (9.60); 6.9149 (0.77); 3.9956 (0.63); 3.9755 (1.44); 3.9600 (1.90); 3.9443 (1.49); 3.9240 (0.69); 3.3534 (16.27); 2.7807 (0.46); 2.7649 (0.63); 2.7569 (0.64); 2.7461 (1.87); 2.7292 (3.15); 2.7228 (2.93); 2.7064 (3.72); 2.6879 (2.03); 2.6768 (0.69); 2.6712 (0.71); 2.6534 (0.48); 2.5487 (28.87); 2.5319 (0.45); 2.5138 (13.94); 2.5094 (18.54); 2.5050 (14.40); 1.8741 (0.36); 1.8583 (0.50); 1.8515 (0.72); 1.8399 (1.22); 1.8250 (1.32); 1.8178 (1.92); 1.8030 (2.44); 1.7972 (1.75); 1.7867 (1.97); 1.7799 (1.97); 1.7718 (1.81); 1.7647 (1.72); 1.7483 (1.35); 1.7389 (0.59); 1.7307 (0.61); 1.7150 (0.34); 1.1817 (0.51); 1.1608 (16.00); 1.1442 (15.88); -0.0002 (2.85) |
| 17-59 | 4-methyl-phenyl | CH2 | CH2 | CH(CH3) | H | [DMSO], Spektrometer: 399.95MHz |
| | | | | | | 7.9137 (9.40); 7.9086 (9.59); 7.8870 (3.27); 7.8662 (3.28); 7.2596 (4.34); 7.2452 (5.33); 7.2381 (6.47); 7.2292 (2.57); 7.2239 (5.66); 7.1180 (0.82); 7.1106 (6.27); 7.1055 (2.21); 7.0940 (2.46); 7.0882 (9.93); 7.0828 (2.71); 7.0711 (1.87); 7.0661 (4.85); 7.0587 (0.64); 6.9256 (9.30); 6.9204 (9.37); 3.9715 (0.65); 3.9514 (1.44); 3.9358 (1.92); 3.9201 (1.50); 3.8999 (0.69); 3.3558 (9.54); 2.6623 (0.36); 2.6466 (0.55); 2.6388 (0.50); 2.6275 (1.88); 2.6124 (3.53); 2.6047 (2.75); 2.5894 (3.81); 2.5731 (2.03); 2.5467 (26.29); 2.5299 (0.51); 2.5158 (6.09); 2.5118 (12.17); 2.5074 (16.17); 2.5029 (12.44); 1.8366 (0.46); 1.8202 (0.60); 1.8146 (0.91); 1.8025 (1.23); 1.7932 (0.90); 1.7872 (1.25); 1.7806 (1.89); 1.7655 (1.57); 1.7596 (1.36); 1.7448 (1.37); 1.7303 (1.60); 1.7229 (1.16); 1.7154 (1.59); 1.7085 (1.57); 1.6918 (1.34); 1.6819 (0.66); 1.6753 (0.67); 1.6586 (0.41); 1.1462 (16.00); 1.1297 (15.96); -0.0002 (2.69) |
| 17-60 | 2-methyl-phenyl | CH2 | CH2 | CH(CH3) | H | |
| 17-61 | 3,4-dichlor-phenyl | CH2 | CH2 | CH(CH3) | H | [DMSO], Spektrometer: 399.95MHz |
| | | | | | | 7.9133 (9.29); 7.9082 (9.50); 7.8746 (3.42); 7.8538 (3.49); 7.5303 (7.20); 7.5201 (0.45); 7.5097 (8.46); 7.5008 (7.28); 7.4960 (7.56); 7.2337 (4.03); 7.2288 (4.02); 7.2130 (3.57); 7.2081 (3.56); 6.9111 (9.56); 6.9059 (9.71); 3.9618 (0.63); 3.9415 (1.40); 3.9263 (1.87); 3.9105 (1.49); 3.8901 (0.69); 3.3482 (20.76); 2.6811 (0.43); 2.6647 (0.54); 2.6584 (0.49); 2.6461 (1.92); 2.6315 (3.40); 2.6236 (2.86); 2.6145 (3.20); 2.6094 (3.54); 2.5934 (2.08); 2.5770 (0.63); 2.5581 (0.54); 2.5473 (16.67); 2.5305 (0.46); 2.5165 (7.65); 2.5125 (15.43); 2.5080 (20.67); 2.5036 (15.94); 1.8368 (0.35); 1.8201 (0.49); 1.8153 (0.74); 1.8027 (1.20); 1.7942 (0.77); 1.7814 (2.15); 1.7658 (2.21); 1.7605 (1.96); 1.7530 (1.90); 1.7452 (1.81); 1.7379 (1.92); 1.7333 (1.73); 1.7157 (1.34); 1.7046 (0.52); 1.6990 (0.52); 1.1441 (15.89); 1.1276 (16.00); -0.0002 (2.67) |
| 17-62 | 3,5-dichlor-phenyl | CH2 | CH2 | CH2 | H | [DMSO], Spektrometer: 399.95MHz |
| | | | | | | 8.1253 (1.68); 8.1116 (3.23); 8.0981 (1.69); 7.9117 (9.06); 7.9066 (9.20); 7.4187 (3.58); 7.4142 (7.05); 7.4095 (4.40); 7.3363 (16.00); 7.3317 (14.77); 6.8716 (9.31); 6.8664 (9.38); 3.3428 (25.62); 3.2070 (2.32); 3.1901 (5.61); 3.1749 (5.61); 3.1581 (2.46); 2.6645 (4.20); 2.6458 (6.99); 2.6266 (4.51); 2.5445 (29.25); 2.5275 (0.91); 2.5096 (28.36); 2.5052 (37.26); 2.5007 (28.41); 1.8263 (1.22); 1.8081 (3.76); 1.7896 (5.10); 1.7714 (3.60); 1.7535 (1.11); -0.0002 (5.72) |
| 17-63 | 2,6-dimethyl-phenyl | CH2 | CH2 | CH(CH3) | H | [DMSO], Spektrometer: 399.95MHz |
| | | | | | | 7.9458 (1.00); 7.9201 (3.12); 7.9149 (2.95); 6.9495 (8.61); 6.9341 (2.86); 6.9289 (2.85); 4.0731 (0.46); 4.0552 (0.57); 4.0370 (0.46); 3.3484 (3.90); 2.5991 (0.60); 2.5875 (0.80); 2.5765 (0.82); 2.5684 (0.82); 2.5582 (0.92); 2.5443 (12.30); 2.5095 (3.78); 2.5050 (4.97); 2.5006 (3.79); 2.2564 (16.00); 1.5993 (0.59); 1.5780 (1.35); 1.5604 (1.10); 1.5426 (0.44); 1.5389 (0.43); 1.2042 (4.47); 1.1875 (4.46); -0.0002 (0.86) |
| 17-64 | 4-trifluormethylphenyl | CH2 | CH2 | CH(CH3) | H | [DMSO], Spektrometer: 399.95MHz |
| | | | | | | 7.9159 (9.37); 7.9108 (11.30); 7.8886 (3.51); 7.6422 (7.44); 7.6220 (9.02); 7.4590 (8.52); 7.4389 (7.15); 6.9214 (8.61); 6.9162 (8.74); 3.9910 (0.66); 3.9710 (1.47); 3.9556 (1.95); 3.9398 (1.54); 3.9196 (0.71); 3.3562 (15.85); 2.7561 (0.50); 2.7376 (1.86); 2.7235 (3.17); 2.7150 (2.91); 2.7024 (3.32); 2.6863 (2.01); 2.6701 (0.60); 2.6515 (0.35); 2.5494 (19.48); 2.5327 (0.40); 2.5145 (11.93); 2.5101 (15.65); 2.5058 (12.13); 1.8834 (0.42); 1.8616 (0.90); 1.8492 (1.22); 1.8464 (1.20); 1.8404 (0.92); 1.8277 (2.04); 1.8117 (1.77); 1.8062 (1.76); 1.7887 (2.02); 1.7792 (1.30); 1.7709 (1.71); 1.7650 (1.66); 1.7481 (1.40); 1.7375 (0.64); 1.7317 (0.64); 1.7146 (0.38); 1.1605 (16.00); 1.1440 (15.96); -0.0002 (2.35) |
| 17-65 | 2,5-dichlor-phenyl | CH2 | CH2 | CH(CH3) | H | [DMSO], Spektrometer: 399.95MHz |
| | | | | | | 7.9161 (12.47); 7.9109 (10.86); 7.8956 (3.51); 7.4585 (7.10); 7.4509 (10.82); 7.4285 (9.20); 7.3111 (4.80); 7.3046 (4.53); 7.2897 (3.59); 7.2833 (3.42); 6.9247 (9.47); 6.9195 (9.66); 4.0229 (0.74); 4.0060 (1.67); 3.9863 (2.07); 3.9691 (1.75); 3.9521 (0.79); 3.3489 (14.21); 2.7499 (0.38); 2.7350 (2.34); 2.7299 (2.64); 2.7175 (4.40); 2.7076 (4.32); 2.6956 (2.73); 2.6902 (2.62); 2.6758 (0.52); 2.5475 (26.27); 2.5306 (0.47); 2.5259 (0.62); 2.5170 (7.43); 2.5127 (15.17); 2.5082 (20.37); 2.5037 (15.67); 2.4997 (8.27); 1.7760 (2.20); 1.7587 (4.45); 1.7371 (4.35); 1.7190 (2.12); 1.1764 (16.00); 1.1598 (15.96); -0.0002 (3.49) |
| 17-66 | 4-phenoxy-phenyl | CH2 | CH2 | CH2 | H | [DMSO], Spektrometer: 399.95MHz |
| | | | | | | 8.1416 (2.26); 8.1278 (4.38); 8.1140 (2.25); 7.9119 (12.71); 7.9067 (12.91); 7.4000 (1.05); 7.3943 (7.31); 7.3891 (2.86); 7.3816 (1.62); 7.3757 (11.30); 7.3730 (10.98); 7.3592 (3.48); 7.3543 (9.31); 7.3483 (1.35); 7.2570 (12.44); 7.2357 (14.15); 7.1346 (2.60); 7.1323 (4.38); 7.1299 (2.80); 7.1139 (7.39); 7.0978 (2.03); 7.0954 (3.31); 7.0930 (2.02); 6.9878 (10.15); 6.9852 (12.61); 6.9660 (11.14); 6.9637 (9.80); 6.9565 (2.71); 6.9486 (16.00); 6.9438 (5.72); 6.9321 (4.90); 6.9273 (14.23); 6.9203 (1.89); 6.8901 (12.33); 6.8849 (12.57); 3.3529 (18.81); 3.2413 (3.04); 3.2243 (6.83); 3.2088 (6.79); 3.1918 (3.21); 2.6380 (5.28); 2.6192 (8.32); 2.5998 (5.76); 2.5459 (37.02); 2.5293 (0.53); 2.5242 (0.73); 2.5154 (8.42); 2.5111 (17.04); 2.5066 (22.70); 2.5021 (17.21); 2.4980 (8.84); 1.8327 (1.62); 1.8139 (4.61); 1.7956 (6.24); 1.7771 (4.38); 1.7590 (1.46); -0.0002 (4.51) |
| 17-67 | 3-chlor-phenyl | CH2 | CH2 | CH(CH3) | H | [DMSO], Spektrometer: 399.95MHz |
| | | | | | | 7.9148 (9.31); 7.9097 (9.61); 7.8856 (3.41); 7.8649 (3.46); 7.3233 (2.54); 7.3038 (6.89); 7.2938 (7.18); 7.2894 (5.52); 7.2850 (6.59); 7.2426 (4.38); 7.2386 (3.61); 7.2255 (2.04); 7.2210 (2.42); 7.1934 (4.78); 7.1746 (3.43); 6.9228 (9.33); 6.9176 (9.55); 3.9731 (0.65); 3.9530 (1.45); 3.9373 (1.94); 3.9216 (1.51); 3.9015 (0.69); 3.3503 (14.85); 2.6819 (0.44); 2.6665 (0.63); 2.6588 (0.55); 2.6477 (1.94); 2.6320 (3.64); 2.6247 (2.88); 2.6090 (4.03); 2.5920 (2.07); 2.5749 (0.70); 2.5570 (0.74); 2.5461 (26.91); 2.5290 (0.48); 2.5109 (14.21); 2.5066 (18.95); 2.5022 (14.74); 2.0803 (0.33); 1.8429 (0.40); 1.8267 (0.54); 1.8209 (0.80); 1.8088 (1.23); 1.7993 (0.85); 1.7934 (1.30); 1.7871 (2.01); 1.7690 (2.21); 1.7511 (2.30); 1.7368 (1.72); 1.7301 (1.65); 1.7135 (1.33); 1.7036 (0.57); 1.6963 (0.60); 1.6803 (0.35); 1.1483 (16.00); 1.1318 (15.96); -0.0002 (3.04) |
| 17-68 | 4-phenoxy-phenyl | CH2 | CH2 | CH(CH3) | H | |
| 17-69 | 2,4-dichlor-phenyl | CH2 | CH2 | CH(CH3) | H | [DMSO], Spektrometer: 399.95MHz |
| | | | | | | 7.9226 (3.71); 7.9138 (9.65); 7.9087 (9.95); 7.9019 (3.90); 7.5560 (6.70); 7.5513 (6.84); 7.3970 (2.67); 7.3764 (10.70); 7.3663 (7.40); 7.3615 (6.72); 7.3456 (1.65); 7.3408 (1.81); 6.9205 (9.31); 6.9153 (9.31); 4.0118 (0.73); 3.9948 (1.57); 3.9759 (2.10); 3.9585 (1.67); 3.9409 (0.76); 3.3505 (15.85); 2.7400 (0.47); 2.7269 (2.46); 2.7221 (2.75); 2.7098 (4.10); 2.7004 (4.42); 2.6875 (2.87); 2.6824 (2.74); 2.5472 (24.81); 2.5300 (0.52); 2.5122 (16.58); 2.5078 (21.72); 2.5034 (16.52); 2.0817 (0.33); 1.7801 (0.37); 1.7676 (1.32); 1.7600 (1.59); 1.7492 (3.26); 1.7375 (2.74); 1.7297 (3.24); 1.7219 (2.83); 1.7095 (1.62); 1.7046 (1.45); 1.6927 (0.33); 1.1715 (16.00); 1.1549 (15.92); -0.0002 (3.53) |
| 17-70 | 2-difluormethoxyphenyl | CH2 | CH2 | CH(CH3) | H | [DMSO], Spektrometer: 399.95MHz |
| | | | | | | 7.9109 (10.54); 7.9057 (12.57); 7.8833 (3.48); 7.3646 (4.15); 7.3332 (3.10); 7.3294 (3.60); 7.3146 (3.94); 7.3106 (4.51); 7.2863 (1.47); 7.2820 (1.50); 7.2671 (3.68); 7.2629 (3.45); 7.2476 (3.22); 7.2433 (2.79); 7.1958 (3.12); 7.1931 (3.69); 7.1785 (11.34); 7.1749 (5.90); 7.1523 (5.24); 7.1317 (3.64); 6.9929 (4.28); 6.9275 (9.62); 6.9223 (9.76); 3.9923 (0.70); 3.9737 (1.52); 3.9567 (2.11); 3.9398 (1.60); 3.9211 (0.73); 3.3517 (14.29); 2.6712 (0.41); 2.6546 (3.38); 2.6344 (6.18); 2.6151 (3.77); 2.5987 (0.37); 2.5454 (33.18); 2.5285 (0.38); 2.5239 (0.48); 2.5149 (5.61); 2.5106 (11.41); 2.5061 (15.28); 2.5017 (11.65); 1.7864 (0.74); 1.7726 (1.18); 1.7674 (1.04); 1.7521 (3.00); 1.7331 (3.96); 1.7150 (2.78); 1.6950 (1.44); 1.6822 (0.50); 1.1649 (16.00); 1.1483 (15.94); -0.0002 (2.99) |
| 17-71 | 4-methoxy-phenyl | CH2 | CH2 | CH2 | H | [DMSO], Spektrometer: 399.95MHz |
| | | | | | | 8.1154 (0.58); 8.1017 (1.12); 8.0879 (0.59); 7.9071 (3.45); 7.9019 (3.52); 7.1541 (0.38); 7.1470 (3.26); 7.1255 (3.78); 6.8825 (3.56); 6.8773 (3.59); 6.8643 (0.54); 6.8569 (4.21); 6.8520 (1.51); 6.8402 (1.29); 6.8354 (3.69); 6.8281 (0.48); 3.7181 (16.00); 3.3485 (4.93); 3.3464 (4.93); 3.2082 (0.83); 3.1911 (1.79); 3.1755 (1.78); 3.1585 (0.88); 2.5734 (1.51); 2.5546 (2.49); 2.5443 (10.09); 2.5355 (1.82); 2.5137 (2.55); 2.5095 (5.10); 2.5050 (6.78); 2.5005 (5.18); 2.4964 (2.71); 1.7871 (0.45); 1.7681 (1.27); 1.7499 (1.73); 1.7315 (1.19); 1.7133 (0.40); -0.0002 (1.21) |
| 17-72 | 4-chlor-phenyl | CH2 | CH2 | CH(CH3) | H | [DMSO], Spektrometer: 399.95MHz |
| | | | | | | 7.9102 (9.37); 7.9051 (9.64); 7.8835 (3.31); 7.8627 (3.32); 7.3334 (8.44); 7.3291 (3.40); 7.3172 (3.89); 7.3124 (14.01); 7.2482 (12.42); 7.2272 (7.78); 6.9151 (9.38); 6.9099 (9.55); 3.9599 (0.62); 3.9398 (1.38); 3.9239 (1.85); 3.9085 (1.44); 3.8883 (0.66); 3.3429 (33.50); 3.3398 (28.83); 2.6732 (0.32); 2.6623 (0.40); 2.6464 (0.52); 2.6390 (0.46); 2.6272 (1.90); 2.6126 (3.44); 2.6044 (2.81); 2.5960 (3.05); 2.5900 (3.56); 2.5742 (2.06); 2.5569 (0.68); 2.5433 (4.69); 2.5263 (0.94); 2.5085 (32.46); 2.5040 (43.18); 2.4996 (33.25); 1.8325 (0.42); 1.8106 (0.87); 1.7981 (1.16); 1.7895 (0.86); 1.7826 (1.23); 1.7766 (1.89); 1.7611 (1.58); 1.7557 (1.39); 1.7469 (1.11); 1.7394 (1.10); 1.7306 (1.61); 1.7235 (1.14); 1.7152 (1.56); 1.7088 (1.53); 1.6922 (1.30); 1.6820 (0.60); 1.6747 (0.62); 1.6586 (0.36); 1.1407 (16.00); 1.1242 (15.98); -0.0002 (6.79); -0.0080 (0.33) |
| 17-73 | 4-chlor-phenyl | CH2 | CH2 | CH(i-propyl) | H | [DMSO], Spektrometer: 399.95MHz |
| | | | | | | 7.9210 (6.88); 7.9158 (6.99); 7.7394 (2.51); 7.7167 (2.55); 7.3268 (6.28); 7.3222 (2.46); 7.3103 (3.03); 7.3057 (10.26); 7.2427 (9.13); 7.2216 (5.74); 6.9487 (6.87); 6.9435 (6.92); 3.7815 (0.43); 3.7716 (0.56); 3.7670 (0.62); 3.7573 (1.28); 3.7440 (1.27); 3.7340 (1.27); 3.7246 (0.63); 3.7197 (0.65); 3.7141 (0.46); 3.7101 (0.47); 3.3484 (11.94); 2.6643 (0.55); 2.6508 (0.72); 2.6401 (0.70); 2.6288 (1.30); 2.6164 (1.12); 2.6064 (1.04); 2.5928 (0.93); 2.5468 (12.85); 2.5308 (1.09); 2.5257 (0.72); 2.5120 (10.55); 2.5075 (13.85); 2.5031 (10.20); 2.4988 (5.64); 2.4800 (0.82); 2.4733 (0.88); 2.4559 (0.61); 1.7941 (0.58); 1.7889 (0.52); 1.7768 (1.47); 1.7727 (1.39); 1.7607 (2.36); 1.7553 (1.81); 1.7452 (3.03); 1.7275 (2.17); 1.7249 (2.15); 1.7103 (1.66); 1.6990 (0.89); 1.6861 (0.78); 1.6760 (0.45); 0.8728 (16.00); 0.8557 (15.46); -0.0002 (2.35) |
| 17-74 | 4-fluor-phenyl | CH2 | CH2 | CH2 | H | [DMSO], Spektrometer: 399.95MHz |
| | | | | | | 8.1333 (2.48); 8.1196 (4.75); 8.1058 (2.53); 7.9105 (15.11); 7.9053 (15.43); 7.2880 (0.80); 7.2806 (6.94); 7.2755 (3.27); 7.2663 (8.40); 7.2591 (10.04); 7.2503 (3.89); 7.2449 (8.86); 7.2380 (1.36); 7.1319 (1.17); 7.1243 (10.12); 7.1191 (3.30); 7.1079 (3.67); 7.1020 (16.00); 7.0962 (4.07); 7.0849 (2.87); 7.0798 (7.78); 7.0723 (1.00); 6.8921 (0.37); 6.8819 (15.19); 6.8767 (15.35); 3.3496 (21.88); 3.2187 (3.76); 3.2016 (8.28); 3.1864 (8.41); 3.1691 (4.01); 2.6336 (6.42); 2.6147 (10.23); 2.5953 (7.03); 2.5461 (45.68); 2.5292 (0.68); 2.5155 (10.63); 2.5112 (21.36); 2.5068 (28.40); 2.5023 (21.58); 2.4983 (11.28); 2.0803 (0.35); 1.8108 (2.05); 1.7920 (5.74); 1.7736 (7.78); 1.7551 (5.47); 1.7370 (1.86); -0.0002 (4.63) |
| 17-75 | 4-chlor-phenyl | CH2 | CH2 | CH(n-propyl) | H | [DMSO], Spektrometer: 399.95MHz |
| | | | | | | 7.9144 (8.00); 7.9092 (8.05); 7.7826 (3.02); 7.7605 (3.08); 7.3256 (7.35); 7.3211 (2.86); 7.3092 (3.60); 7.3045 (12.10); 7.2426 (10.83); 7.2215 (6.74); 6.9230 (8.00); 6.9178 (8.02); 3.9277 (0.83); 3.9094 (1.48); 3.8925 (1.44); 3.8735 (0.83); 3.3498 (11.45); 2.6567 (0.45); 2.6392 (0.76); 2.6217 (1.49); 2.6033 (2.26); 2.5885 (1.77); 2.5846 (1.81); 2.5684 (2.50); 2.5468 (11.14); 2.5330 (1.00); 2.5252 (0.62); 2.5161 (6.07); 2.5120 (11.61); 2.5075 (15.13); 2.5031 (11.40); 1.7562 (1.82); 1.7366 (3.72); 1.7176 (2.97); 1.7027 (1.20); 1.6966 (1.07); 1.4837 (1.46); 1.4643 (4.13); 1.4466 (4.92); 1.4289 (2.37); 1.3625 (0.35); 1.3452 (0.66); 1.3292 (1.47); 1.3112 (2.42); 1.2927 (2.57); 1.2899 (2.50); 1.2708 (2.00); 1.2528 (1.08); 1.2366 (0.62); 0.8749 (8.05); 0.8567 (16.00); 0.8384 (6.91); -0.0002 (2.85) |
| 17-76 | 4-chlor-phenyl | CH2 | CH2 | CH(t-butyl) | H | [DMSO], Spektrometer: 399.95MHz |
| | | | | | | 7.9228 (2.58); 7.9177 (2.61); 7.6241 (0.93); 7.6000 (0.94); 7.3261 (2.22); 7.3216 (0.86); 7.3050 (3.70); 7.2455 (3.30); 7.2244 (2.03); 6.9470 (2.57); 6.9419 (2.58); 3.7681 (0.35); 3.7642 (0.37); 3.7400 (0.76); 3.7159 (0.38); 3.7113 (0.34); 3.3497 (4.20); 2.6099 (0.50); 2.5990 (0.37); 2.5868 (0.36); 2.5467 (4.96); 2.5119 (3.53); 2.5074 (4.65); 2.5030 (3.51); 2.4388 (0.40); 2.4345 (0.43); 2.4164 (0.38); 1.7974 (0.33); 1.7918 (0.41); 1.6760 (0.35); 0.8714 (16.00); -0.0002 (0.79) |
| 17-77 | 2-chlor-phenyl | CH2 | CH2 | CH2 | H | [DMSO], Spektrometer: 399.95MHz |
| | | | | | | 8.1852 (2.64); 8.1712 (5.09); 8.1573 (2.64); 7.9117 (15.90); 7.9065 (16.00); 7.4255 (6.16); 7.4221 (6.15); 7.4062 (7.97); 7.4027 (7.94); 7.3933 (5.09); 7.3889 (5.58); 7.3746 (6.93); 7.3703 (7.57); 7.3057 (3.11); 7.3022 (3.47); 7.2873 (7.71); 7.2837 (7.22); 7.2689 (5.15); 7.2651 (4.34); 7.2559 (5.61); 7.2510 (5.68); 7.2368 (6.20); 7.2322 (6.12); 7.2180 (2.41); 7.2136 (2.18); 6.8936 (0.38); 6.8836 (15.85); 6.8784 (15.89); 3.3493 (25.35); 3.2650 (3.86); 3.2478 (8.57); 3.2323 (8.54); 3.2152 (4.03); 2.7566 (7.33); 2.7379 (9.57); 2.7179 (8.01); 2.5463 (46.61); 2.5295 (0.65); 2.5157 (11.43); 2.5115 (22.76); 2.5070 (30.08); 2.5025 (22.68); 1.8236 (2.10); 1.8047 (5.55); 1.7863 (7.53); 1.7676 (5.20); 1.7493 (1.87); -0.0002 (5.43) |
| 17-78 | 4-chlor-phenyl | CH2 | CH(CH3) | - | cyclopropyl | |
| 17-79 | 2-brom-phenyl | CH2 | CH2 | CH(CH3) | H | [DMSO], Spektrometer: 399.95MHz |
| | | | | | | 7.9328 (3.45); 7.9150 (11.67); 7.9099 (12.20); 7.5802 (4.63); 7.5786 (4.57); 7.5605 (5.06); 7.5585 (4.91); 7.3661 (1.84); 7.3613 (2.38); 7.3471 (5.66); 7.3422 (5.54); 7.3332 (3.78); 7.3308 (3.66); 7.3153 (4.51); 7.3131 (4.49); 7.2966 (1.75); 7.2940 (1.68); 7.1626 (2.45); 7.1576 (2.50); 7.1427 (3.33); 7.1396 (3.31); 7.1250 (1.93); 7.1201 (1.83); 6.9353 (9.53); 6.9301 (9.50); 4.0370 (0.76); 4.0202 (1.71); 4.0006 (2.07); 3.9833 (1.77); 3.9662 (0.79); 3.3495 (13.99); 2.7573 (0.38); 2.7434 (2.39); 2.7378 (2.66); 2.7257 (4.41); 2.7154 (4.49); 2.7035 (2.77); 2.6978 (2.61); 2.6827 (0.46); 2.5467 (22.88); 2.5296 (0.35); 2.5160 (6.46); 2.5119 (12.88); 2.5074 (17.03); 2.5029 (12.89); 1.7731 (2.25); 1.7559 (4.51); 1.7505 (3.17); 1.7337 (4.25); 1.7158 (2.15); 1.1849 (16.00); 1.1683 (15.89); -0.0002 (3.03) |
| 17-80 | 3,4-bismethoxyphenyl | CH2 | CH2 | CH2 | H | [DMSO], Spektrometer: 399.95MHz 8.1153 (0.66); 8.1015 (1.31); 8.0877 (0.68); 7.9095 (3.89); 7.9043 (3.99); 6.8878 (3.91); 6.8826 (3.95); 6.8581 (2.42); 6.8377 (3.28); 6.8180 (2.67); 6.8134 (3.01); 6.7371 (1.69); 6.7325 (1.60); 6.7168 (1.25); 6.7121 (1.20); 3.7419 (15.94); 3.7117 (16.00); 3.3469 (7.03); 3.2211 (0.87); 3.2040 (1.97); 3.1883 (1.97); 3.1715 (0.93); 2.5721 (1.57); 2.5532 (2.64); 2.5442 (10.06); 2.5342 (1.87); 2.5230 (0.36); 2.5136 (2.95); 2.5093 (5.98); 2.5048 (8.02); 2.5004 (6.16); 2.4963 (3.25); 1.8073 (0.46); 1.7885 (1.35); 1.7703 (1.84); 1.7519 (1.27); 1.7338 (0.41); -0.0002 (1.30) |
| 17-81 | 4-methoxy-phenyl | CH2 | CH2 | CH(CH3) | H | Verbindung Nr. 17-81, Solvent: [DMSO], Spektrometer: 399.95MHz 7,9111 (3,22); 7,906 (3,26); 7,8715 (1,27); 7,8508 (1,28); 7,1285 (3,39); 7,1071 (3,93); 6,9286 (3,26); 6,9235 (3,27); 6,8512 (0,55); 6,8442 (4,26); 6,8227 (3,73); 3,9429 (0,56); 3,9271 (0,76); 3,9111 (0,58); 3,8908 (0,36); 3,7132 (16); 3,3486 (4,51); 2,5671 (0,78); 2,545 (9,89); 2,531 (1,41); 2,51 (4,9); 2,5056 (6,27); 2,5012 (4,9); 1,7975 (0,37); 1,7827 (0,47); 1,7767 (0,37); 1,7635 (0,7); 1,748 (0,58); 1,743 (0,5); 1,7264 (0,33); 1,7189 (0,35); 1,7025 (0,59); 1,6953 (0,42); 1,687 (0,61); 1,6805 (0,6); 1,664 (0,52); 1,1391 (5,97); 1,1226 (5,94); -0,0002 (0,97) |
| 17-82 | 2-thienyl | CH2 | CH2 | - | CH3 | Verbindung Nr. 17-82, Solvent: [DMSO], Spektrometer: 399.95MHz 7,9051 (3,98); 7,8564 (3,87); 7,367 (2,33); 7,3568 (3,14); 7,3412 (3,18); 7,3294 (2,53); 6,9643 (7,41); 6,9431 (3,26); 6,9317 (2,05); 6,786 (3,02); 6,5777 (4,12); 6,2593 (3,96); 3,6581 (2,6); 3,64 (4,75); 3,6216 (3,24); 3,4874 (2,55); 3,4701 (4,58); 3,4522 (2,77); 3,3222 (32,1); 3,1073 (2,88); 3,089 (4,93); 3,0707 (3,5); 3,0489 (3,48); 3,0311 (4,81); 3,0136 (2,48); 2,9694 (15,44); 2,8893 (16); 2,675 (0,7); 2,6705 (0,96); 2,666 (0,7); 2,5403 (0,64); 2,5237 (3,17); 2,5103 (52,17); 2,5059 (102,88); 2,5014 (135,96); 2,4968 (100,49); 2,4925 (49,43); 2,3325 (0,66); 2,3282 (0,92); 2,3237 (0,67); 1,3357 (0,6); 1,2585 (0,34); 1,2494 (0,77); 1,235 (0,58); 0,008 (0,32); -0,0002 (8,75) |
| 17-83 | 4-chlor-phenyl | CH(OCH3) | CH3 | - | H | Verbindung Nr. 17-83, Solvent: [DMSO], Spektrometer: 399.95MHz 8,2417 (0,6); 8,2275 (1,19); 8,2131 (0,61); 7,8976 (3,22); 7,8924 (3,36); 7,4559 (3,18); 7,4517 (1,33); 7,4395 (1,41); 7,4349 (4,93); 7,3531 (4,57); 7,332 (3,12); 6,8935 (3,26); 6,8883 (3,34); 4,3888 (0,87); 4,3749 (1,08); 4,371 (1,3); 4,3574 (0,92); 3,3811 (0,96); 3,3671 (1,72); 3,3579 (1,33); 3,3536 (1,62); 3,3399 (1,04); 3,3228 (7,27); 3,1652 (16); 2,5247 (0,5); 2,5112 (8,17); 2,5069 (16,23); 2,5024 (21,68); 2,4979 (16,58); 2,4937 (8,63); -0,0002 (1,22) |
| 17-84 | 4-chlor-2-thienyl | CH2 | CH2 | - | H | Verbindung Nr. 17-84, Solvent: [DMSO], Spektrometer: 601.6MHz 8,2799 (1,85); 8,2706 (3,61); 8,2612 (1,84); 7,9098 (15,38); 7,9064 (16); 7,3715 (14,22); 7,3689 (14,34); 6,9118 (9,2); 6,9106 (8,17); 6,9093 (9,31); 6,8643 (14,88); 6,8609 (15,08); 3,4375 (3,23); 3,4258 (7,59); 3,4162 (7,82); 3,4046 (3,63); 3,3616 (0,32); 3,3259 (1025,33); 3,3013 (0,62); 3,0015 (5,58); 2,9896 (10,71); 2,9783 (5,05); 2,653 (2,1); 2,6193 (0,61); 2,6163 (1,35); 2,6132 (1,88); 2,6102 (1,36); 2,6071 (0,61); 2,5541 (0,78); 2,5409 (672,55); 2,5226 (3,72); 2,5194 (4,5); 2,5163 (4,32); 2,5076 (99,89); 2,5046 (217,41); 2,5015 (301,87); 2,4984 (218,95); 2,4954 (101,36); 2,4247 (2,05); 2,3917 (0,6); 2,3887 (1,32); 2,3856 (1,84); 2,3826 (1,32); 2,3796 (0,6); 2,0736 (1,18); 1,9083 (1,46); 0,0052 (0,48); -0,0002 (17,52); -0,0058 (0,49) |
| 17-85 | 4-chlor-phenyl | N(CH3) | CH2 | CH2 | H | Verbindung Nr. 17-85, Solvent: [DMSO], Spektrometer: 399.95MHz 8,2286 (0,82); 8,2146 (1,51); 8,2008 (0,77); 7,8992 (3,63); 7,8941 (3,51); 7,1819 (4,35); 7,1593 (4,68); 7,1504 (0,54); 6,7994 (3,77); 6,7942 (3,59); 6,7502 (4,64); 6,7275 (4,1); 3,4632 (1,4); 3,4467 (3,07); 3,4297 (2,27); 3,3503 (2,29); 3,3287 (50,01); 3,3035 (0,97); 2,9083 (16); 2,5414 (28,34); 2,5064 (28,93); 2,5021 (36,24); 2,4979 (27,16); 0,0072 (0,37); -0,0002 (5,12) |

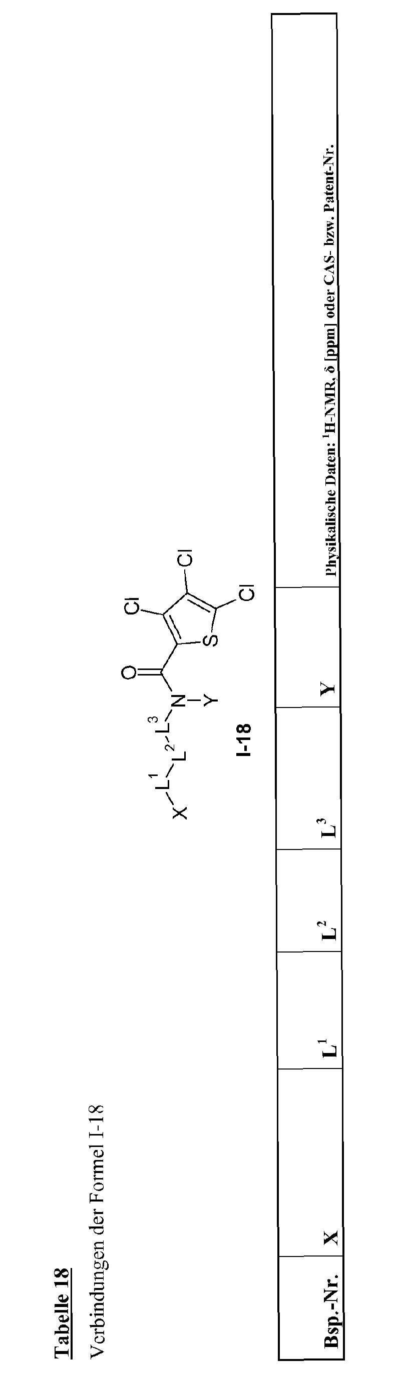

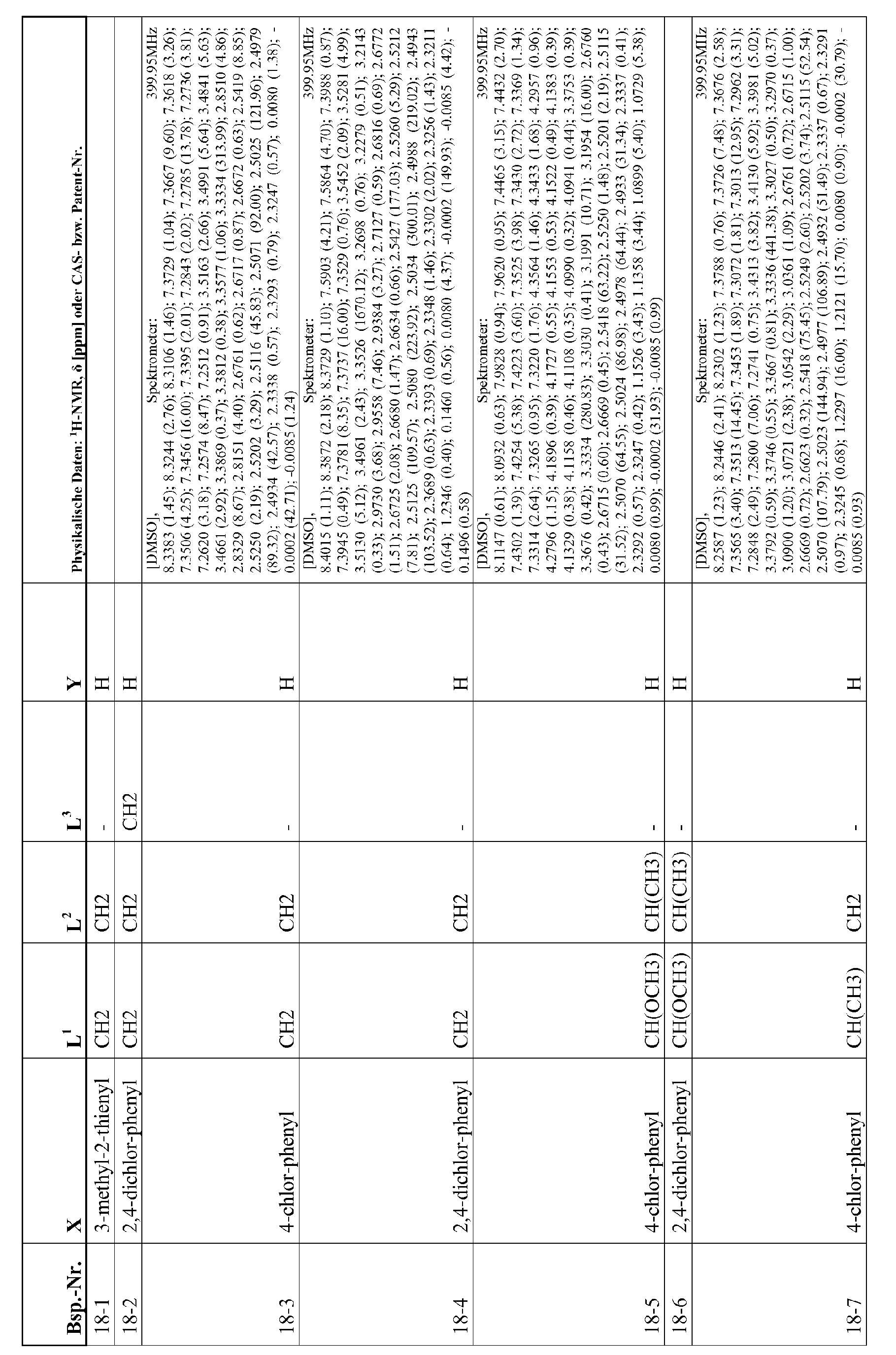

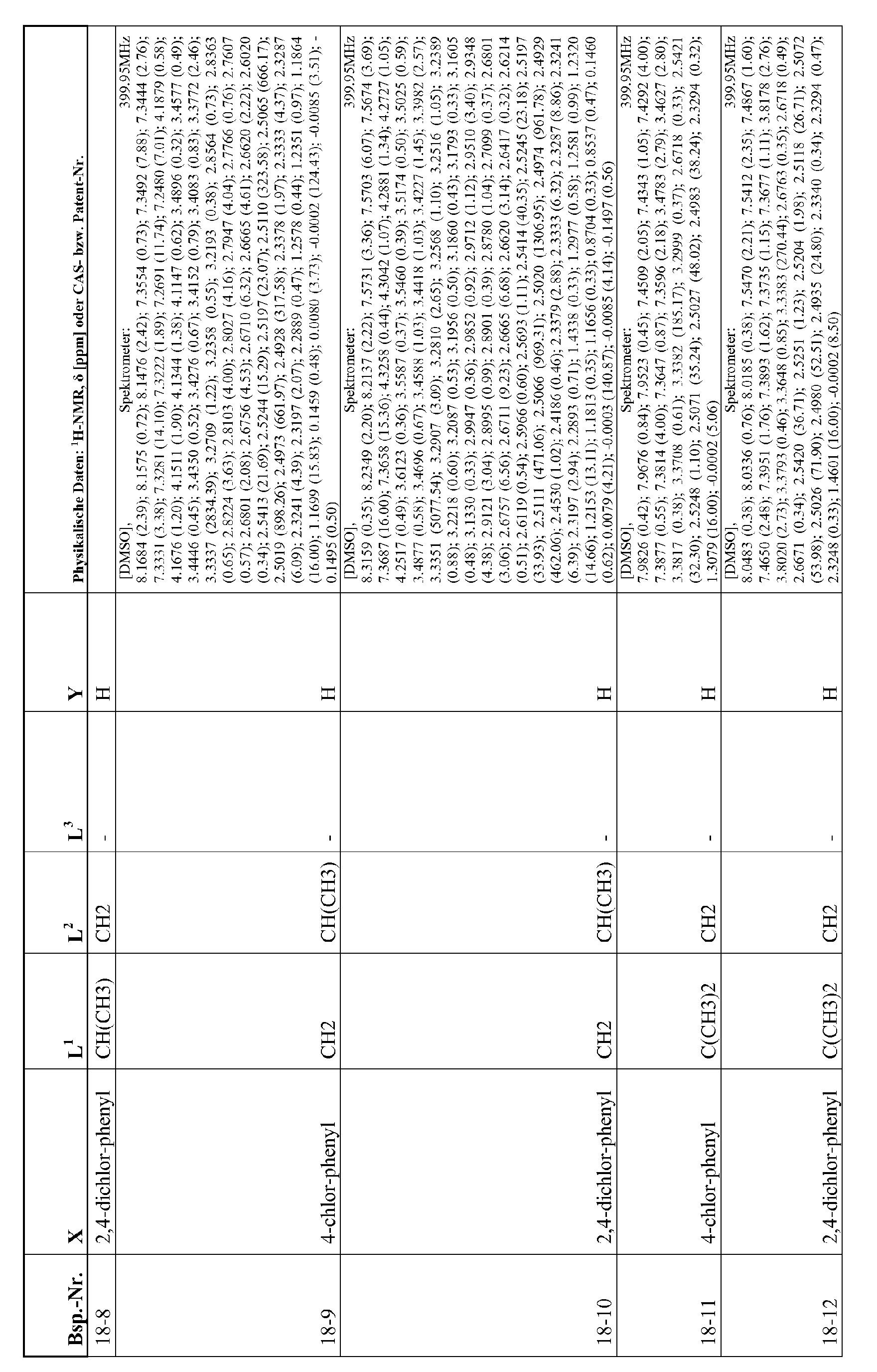

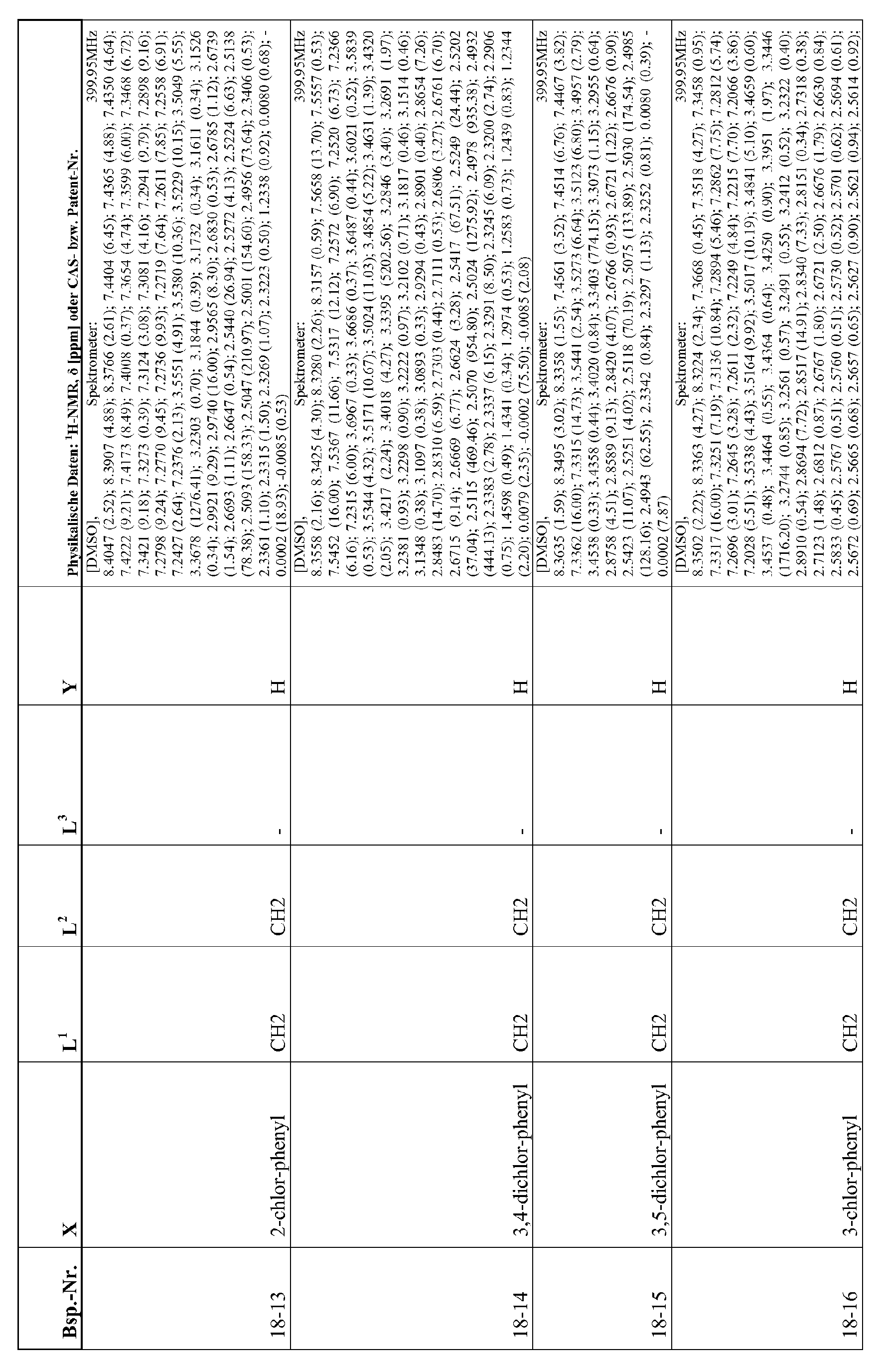

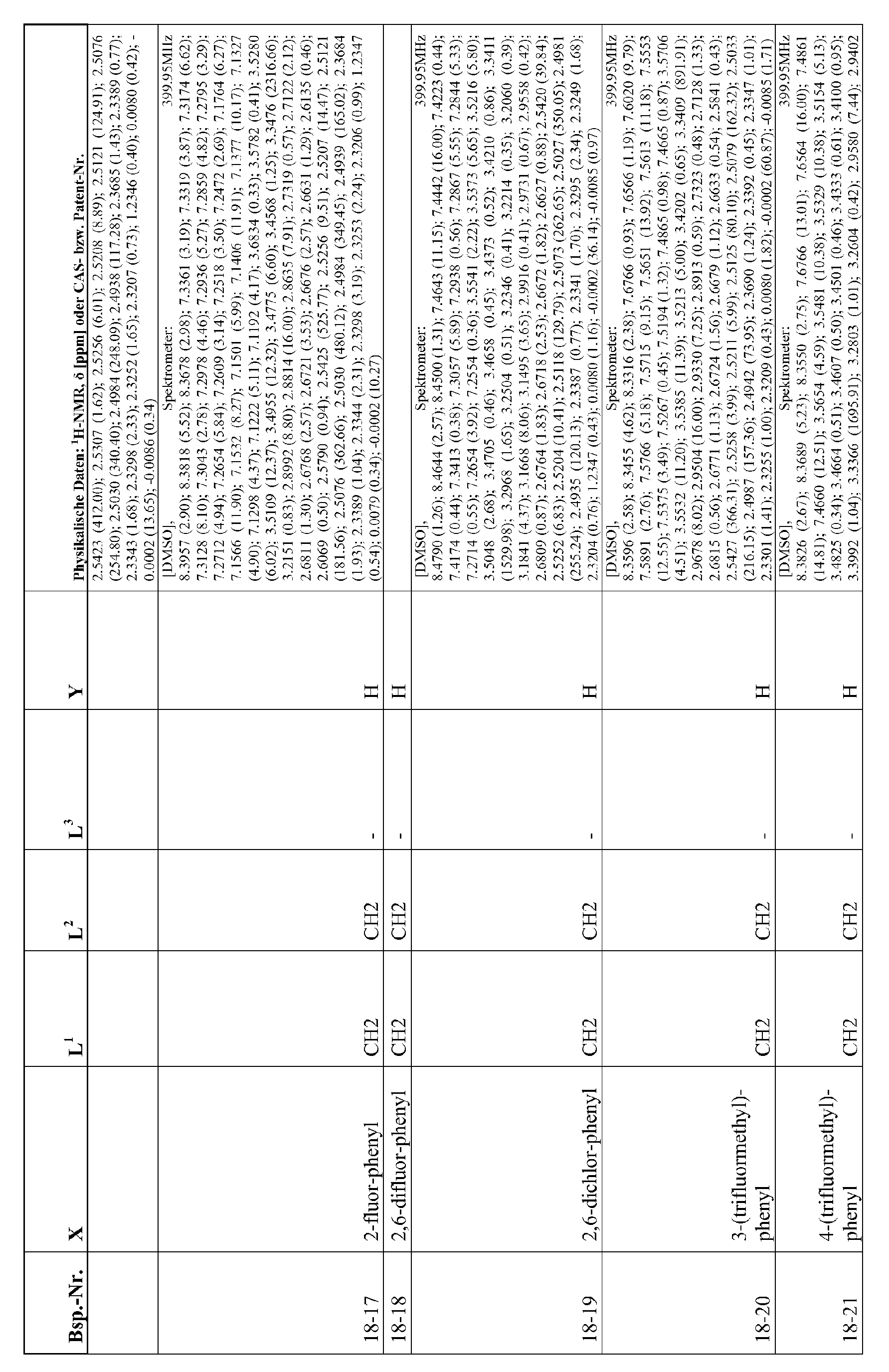

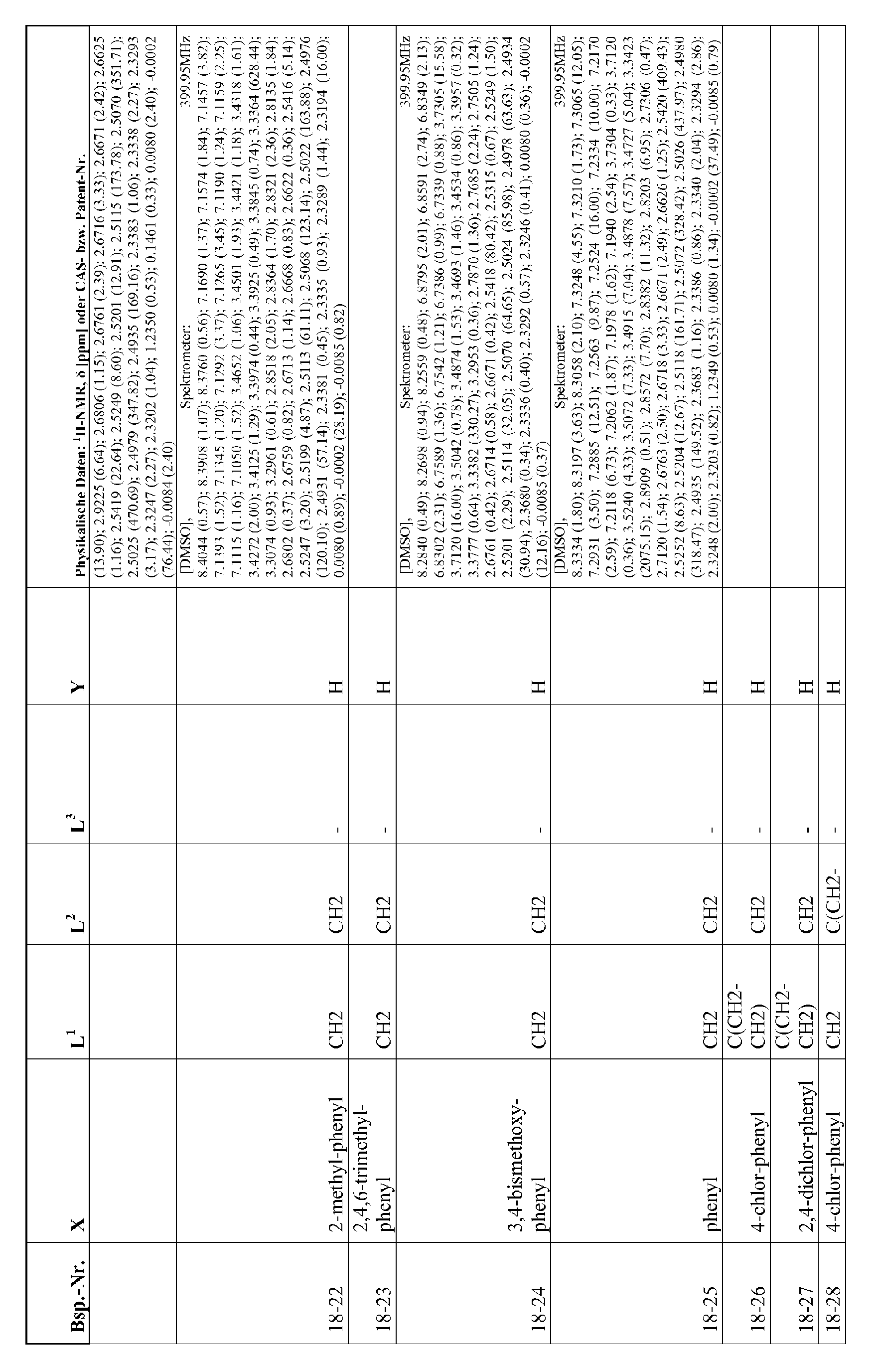

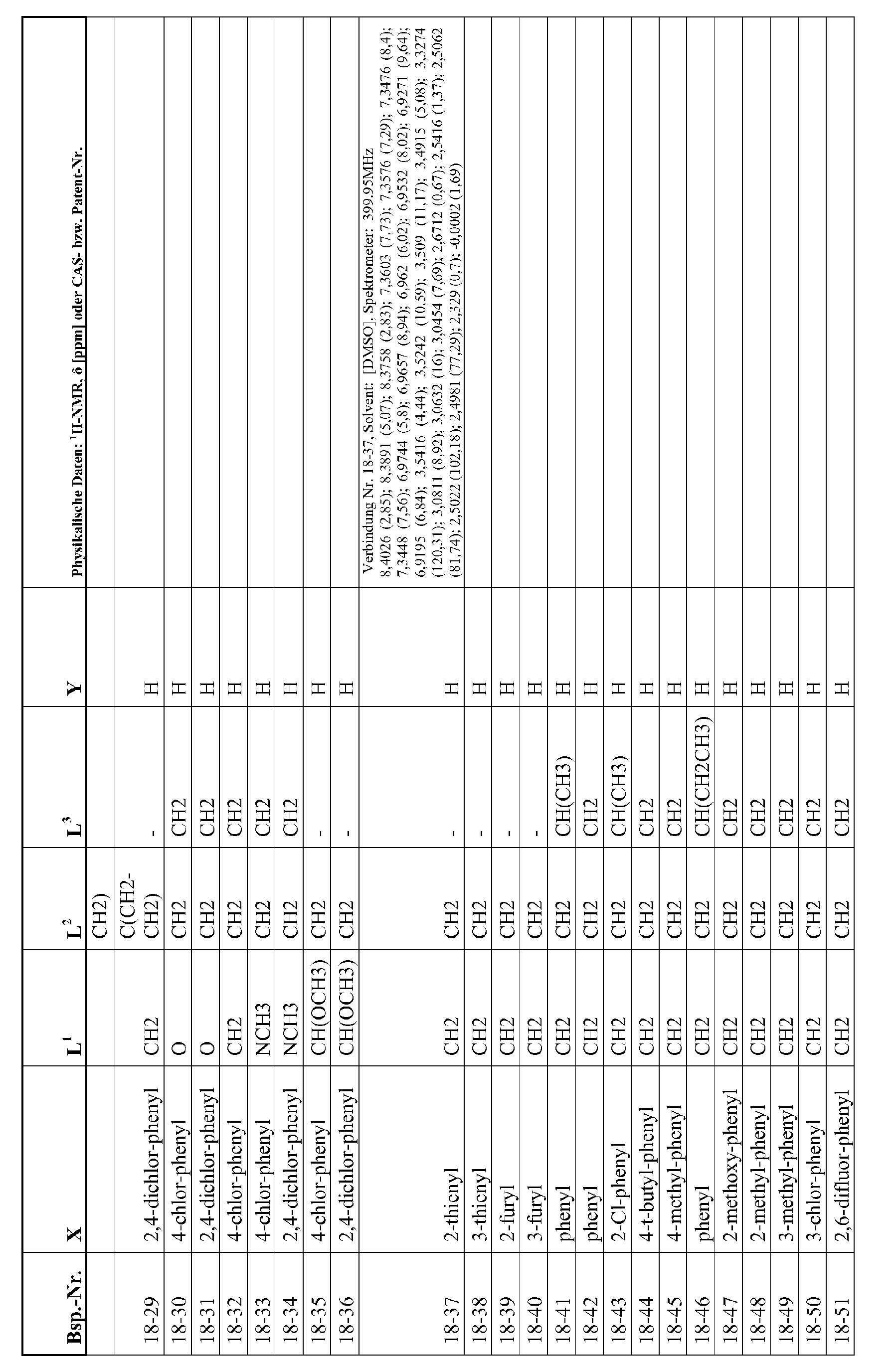

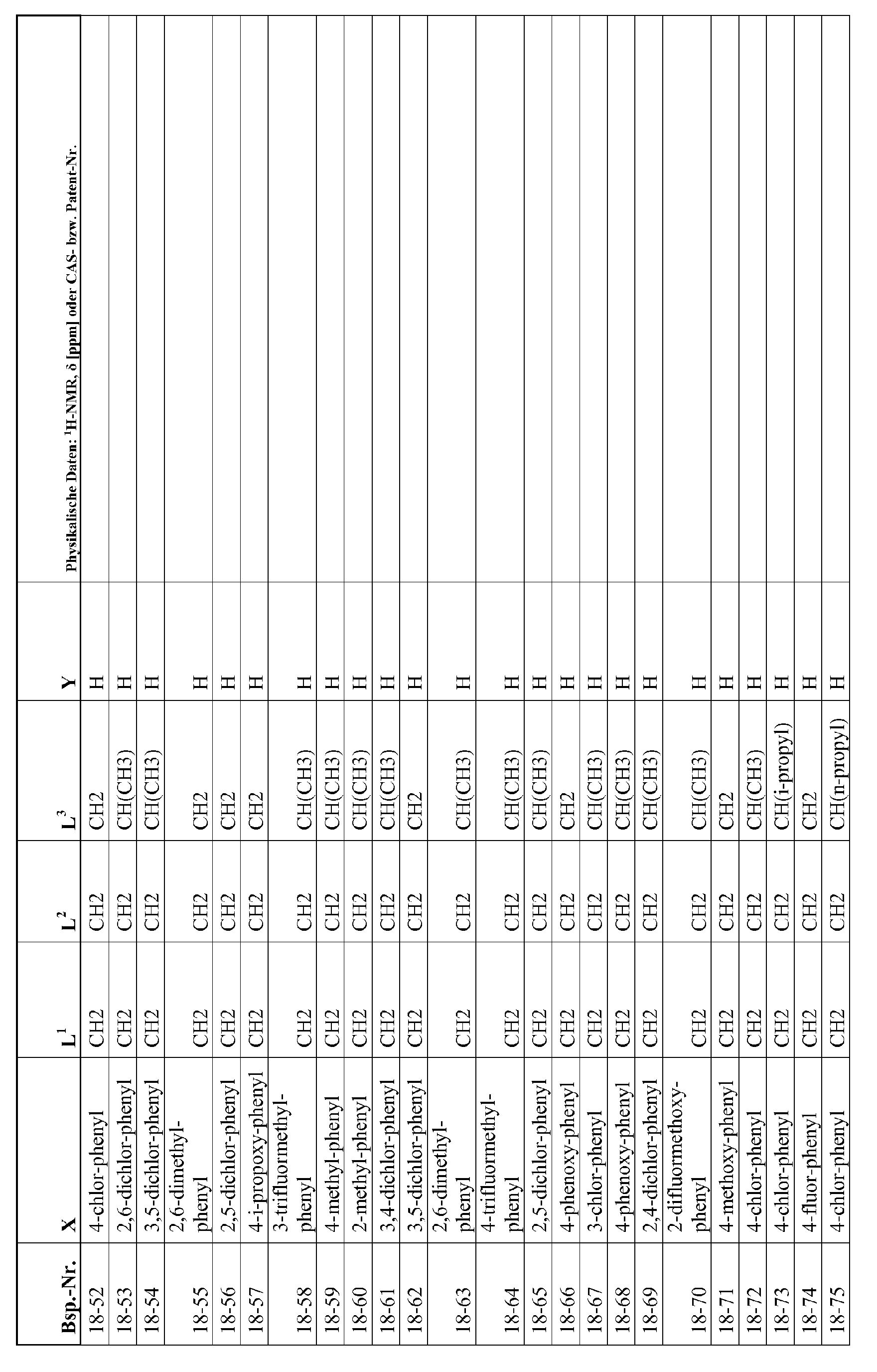

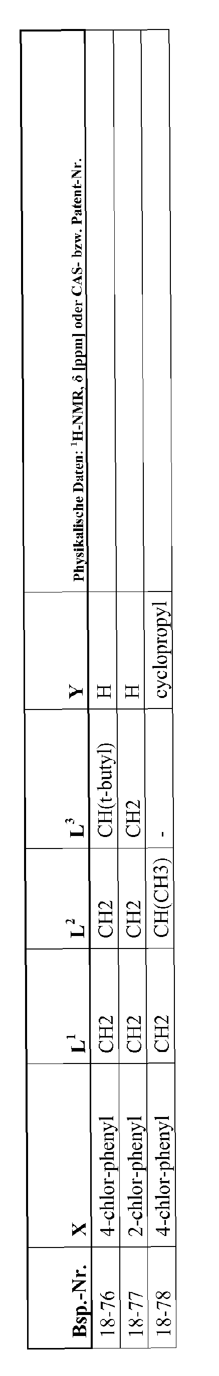

**Tabelle 19**

| | | | | | | |
|---|---|---|---|---|---|---|
| Verbindungen der Formel I-19 | | | | | | |
| | | | | | 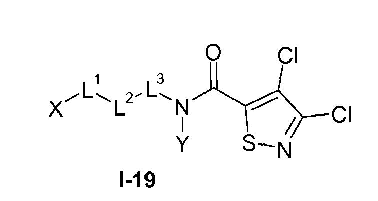 | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 19-1 | 3-methyl-2-thienyl | CH2 | CH2 | - | H | |
| 19-2 | 2,4-dichlor-phenyl | CH2 | CH2 | CH2 | H | WO-A 2008/101976 |
| 19-3 | 4-chlor-phenyl | CH2 | CH2 | - | H | [DMSO], Spektrometer: 399.95MHz 8.7804 (1.61); 8.7666 (2.98); 8.7533 (1.69); 7.3729 (9.38); 7.3684 (3.79); 7.3566 (4.52); 7.3519 (16.00); 7.3464 (2.86); 7.2909 (14.36); 7.2699 (8.86); 3.5300 (2.87); 3.5127 (6.87); 3.4975 (6.94); 3.4802 (3.25); 3.3432 (53.59); 2.8588 (5.46); 2.8410 (10.32); 2.8233 (5.05); 2.5440 (6.08); 2.5090 (29.64); 2.5046 (39.14); 2.5002 (29.94); 2.0786 (1.17); -0.0002 (4.99) |
| 19-4 | 2,4-dichlor-phenyl | CH2 | CH2 | - | H | [DMSO], Spektrometer: 399.95MHz 8.8319 (1.09); 8.8182 (2.04); 8.8041 (1.10); 7.6008 (3.91); 7.5980 (5.81); 7.4114 (0.32); 7.3901 (12.93); 7.3871 (16.00); 7.3689 (0.34); 3.5560 (1.82); 3.5389 (4.91); 3.5238 (5.01); 3.5070 (2.05); 3.3452 (150.16); 2.9809 (3.79); 2.9637 (7.76); 2.9464 (3.42); 2.6728 (0.41); 2.5432 (6.83); 2.5262 (1.41); 2.5127 (23.77); 2.5083 (47.67); 2.5038 (62.98); 2.4992 (47.09); 2.4948 (23.94); 2.3306 (0.41); 2.0773 (1.79); 0.0080 (0.34); -0.0002 (10.17); - 0.0085 (0.44) |
| 19-5 | 4-chlor-phenyl | CH(OCH3) | CH(CH3) | - | H | [DMSO], Spektrometer: 399.95MHz 8.6720 (0.83); 8.6503 (0.84); 8.5858 (1.06); 8.5647 (1.07); 7.4562 (3.64); 7.4520 (3.91); 7.4351 (6.33); 7.4309 (4.83); 7.3606 (4.59); 7.3404 (5.97); 7.3199 (2.51); 4.3370 (1.82); 4.3233 (2.21); 4.2540 (1.43); 4.2378 (1.90); 4.2202 (0.54); 4.2031 (0.72); 4.1996 (0.64); 4.1890 (0.56); 4.1854 (0.66); 4.1827 (0.62); 4.1683 (0.46); 4.1301 (0.46); 4.1135 (0.69); 4.1087 (0.53); 4.0968 (0.48); 4.0920 (0.68); 4.0754 (0.40); 3.3409 (73.80); 3.1983 (13.14); 3.1836 (16.00); 2.5431 (4.17); 2.5261 (0.83); 2.5213 (1.23); 2.5127 (14.60); 2.5083 (29.47); 2.5037 (39.10); 2.4992 (29.18); 2.4948 (14.65); 2.0775 (2.35); 1.1695 (4.92); 1.1527 (4.90); 1.0737 (5.89); 1.0566 (5.86); -0.0002 (5.08) |
| 19-6 | 2,4-dichlor-phenyl | CH(OCH3) | CH(CH3) | - | H | |
| 19-7 | 4-chlor-phenyl | CH(CH3) | CH2 | - | H | [DMSO], Spektrometer: 399.95MHz 8.7346 (1.37); 8.7208 (2.60); 8.7066 (1.41); 7.3845 (0.88); 7.3786 (7.46); 7.3738 (2.91); 7.3624 (3.84); 7.3573 (14.36); 7.3515 (2.43); 7.3103 (13.32); 7.3055 (3.96); 7.2935 (2.86); 7.2890 (7.26); 3.4569 (0.35); 3.4429 (2.88); 3.4379 (2.86); 3.4235 (6.11); 3.4081 (3.74); 3.4062 (3.84); 3.3906 (0.38); 3.3874 (0.38); 3.3432 (43.13); 3.0839 (1.34); 3.0661 (2.65); 3.0482 (2.58); 3.0303 (1.24); 2.5442 (5.28); 2.5273 (0.65); 2.5225 (0.99); 2.5137 (11.38); 2.5094 (22.74); 2.5048 (29.96); 2.5003 (22.48); 2.4960 (11.55); 2.0788 (1.96); 1.2453 (16.00); 1.2278 (15.80); -0.0002 (4.41) |
| 19-8 | 2,4-dichlor-phenyl | CH(CH3) | CH2 | - | H | [DMSO], Spektrometer: 399.95MHz 8.8147 (1.76); 8.8006 (3.14); 8.7893 (1.62); 7.5801 (9.54); 7.5748 (10.09); 7.4937 (5.81); 7.4726 (11.68); 7.4585 (0.32); 7.4369 (7.10); 7.4315 (6.55); 7.4159 (3.48); 7.4104 (3.35); 3.5791 (1.06); 3.5625 (2.52); 3.5467 (3.86); 3.5373 (2.96); 3.5270 (3.67); 3.5124 (3.99); 3.4968 (1.82); 3.4874 (1.70); 3.4733 (1.37); 3.4631 (2.34); 3.4491 (2.88); 3.4355 (1.44); 3.4251 (0.61); 3.4175 (0.49); 3.4109 (0.34); 3.3420 (126.95); 2.6732 (0.41); 2.5437 (7.71); 2.5266 (1.34); 2.5131 (24.03); 2.5087 (48.16); 2.5042 (63.58); 2.4996 (47.39); 2.4952 (24.03); 2.3309 (0.42); 2.0782 (3.45); 1.2396 (15.60); 1.2233 (16.00); -0.0002 (7.21) |
| 19-9 | 4-chlor-phenyl | CH2 | CH(CH3) | - | H | [DMSO], Spektrometer: 399.95MHz 8.6741 (2.64); 8.6534 (2.68); 7.3663 (0.98); 7.3604 (8.41); 7.3560 (3.27); 7.3442 (3.93); 7.3394 (13.89); 7.3337 (2.30); 7.2747 (12.38); 7.2537 (7.77); 4.2068 (0.71); 4.1886 (1.65); 4.1712 (2.18); 4.1521 (1.64); 4.1349 (0.79); 3.3435 (51.80); 2.8243 (0.45); 2.8066 (8.20); 2.7880 (5.81); 2.7530 (0.34); 2.5442 (5.12); 2.5270 (0.63); 2.5135 (12.25); 2.5093 (24.47); 2.5048 (32.40); 2.5003 (24.57); 2.4962 (12.91); 2.0787 (3.43); 1.1902 (16.00); 1.1736 (15.92); -0.0002 (4.17) |
| 19-10 | 2,4-dichlor-phenyl | CH2 | CH(CH3) | - | H | [DMSO], Spektrometer: 399.95MHz 8.7231 (2.60); 8.7020 (2.65); 7.5839 (5.94); 7.5803 (6.44); 7.3996 (1.21); 7.3951 (0.92); 7.3789 (9.06); 7.3736 (14.41); 7.3517 (1.29); 4.3427 (0.56); 4.3210 (1.30); 4.3046 (1.60); 4.2997 (1.06); 4.2905 (1.28); 4.2690 (0.62); 3.3395 (91.34); 2.9978 (0.50); 2.9928 (1.78); 2.9792 (1.95); 2.9586 (3.54); 2.9451 (3.31); 2.9337 (0.34); 2.8990 (3.28); 2.8769 (3.20); 2.8648 (1.81); 2.8427 (1.73); 2.6726 (0.44); 2.6680 (0.32); 2.5431 (14.37); 2.5261 (1.28); 2.5125 (25.11); 2.5081 (50.63); 2.5036 (67.19); 2.4990 (50.39); 2.4946 (25.68); 2.3303 (0.44); 2.3257 (0.33); 2.0776 (0.56); 1.2350 (16.00); 1.2184 (15.83); 0.0080 (0.33); - 0.0002 (9.83); -0.0085 (0.42) |
| 19-11 | 4-chlor-phenyl | C(CH3)2 | CH2 | - | H | [DMSO], Spektrometer: 399.95MHz 8.5244 (0.45); 8.5093 (0.87); 8.4941 (0.46); 7.4515 (2.38); 7.4299 (4.35); 7.3812 (4.42); 7.3639 (0.95); 7.3596 (2.39); 3.4804 (3.09); 3.4647 (3.08); 3.3427 (14.46); 2.5438 (1.18); 2.5090 (7.49); 2.5047 (9.63); 2.5006 (7.32); 2.0785 (0.56); 1.3199 (16.00); -0.0002 (1.03) |
| 19-12 | 2,4-dichlor-phenyl | C(CH3)2 | CH2 | - | H | [DMSO], Spektrometer: 399.95MHz 8.5462 (0.47); 8.5307 (0.91); 8.5155 (0.47); 7.5449 (2.56); 7.5392 (2.75); 7.4817 (1.58); 7.4600 (2.57); 7.3986 (1.75); 7.3928 (1.64); 7.3770 (1.09); 7.3712 (1.04); 3.8267 (3.08); 3.8109 (3.06); 3.3406 (18.90); 2.5439 (1.33); 2.5133 (4.42); 2.5090 (8.72); 2.5045 (11.48); 2.5000 (8.64); 2.4959 (4.44); 2.0787 (0.62); 1.4697 (16.00); -0.0002 (1.49) |
| 19-13 | 2-chlor-phenyl | CH2 | CH2 | - | H | [DMSO], Spektrometer: 399.95MHz 8.8495 (2.31); 8.8362 (4.26); 8.8228 (2.56); 7.4483 (5.85); 7.4440 (4.95); 7.4299 (8.03); 7.4253 (7.77); 7.4076 (0.52); 7.3783 (4.41); 7.3729 (5.50); 7.3599 (6.47); 7.3550 (8.44); 7.3210 (2.68); 7.3168 (3.45); 7.3027 (8.15); 7.2985 (7.66); 7.2865 (9.83); 7.2809 (10.41); 7.2685 (7.22); 7.2633 (6.22); 7.2500 (2.55); 7.2450 (1.96); 3.5642 (4.49); 3.5470 (10.21); 3.5319 (10.47); 3.5142 (5.54); 3.3436 (133.06); 3.0006 (8.81); 2.9828 (16.00); 2.9651 (8.39); 2.6780 (0.36); 2.6733 (0.48); 2.6688 (0.36); 2.5435 (12.04); 2.5266 (1.84); 2.5131 (28.79); 2.5087 (57.00); 2.5042 (75.11); 2.4996 (56.62); 2.4952 (30.49); 2.3356 (0.36); 2.3309 (0.49); 2.3263 (0.37); 2.0778 (1.98); -0.0002 (8.63); -0.0085 (0.47) |
| 19-14 | 3,4-dichlor-phenyl | CH2 | CH2 | - | H | [DMSO], Spektrometer: 399.95MHz 8.7831 (1.79); 8.7697 (3.26); 8.7563 (1.81); 8.0167 (0.44); 8.0142 (0.46); 7.7686 (0.56); 7.7640 (1.10); 7.5729 (9.10); 7.5518 (16.00); 7.5455 (9.40); 7.5300 (1.00); 7.2724 (5.03); 7.2675 (4.89); 7.2519 (4.43); 7.2469 (4.33); 3.5517 (2.97); 3.5348 (7.98); 3.5198 (8.24); 3.5032 (3.53); 3.4913 (0.56); 3.3421 (68.88); 2.8729 (5.77); 2.8558 (11.58); 2.8387 (5.30); 2.6739 (0.36); 2.5442 (20.13); 2.5269 (1.23); 2.5092 (39.28); 2.5048 (50.84); 2.5003 (38.03); 2.3314 (0.32); -0.0002 (6.05) |
| 19-15 | 3,5-dichlor-phenyl | CH2 | CH2 | - | H | [DMSO], Spektrometer: 399.95MHz 8.7911 (1.51); 8.7773 (2.84); 8.7639 (1.57); 7.4621 (3.80); 7.4574 (7.53); 7.4527 (4.51); 7.3510 (16.00); 7.3463 (15.06); 3.5626 (2.41); 3.5462 (6.56); 3.5309 (6.73); 3.5146 (2.74); 3.3448 (76.07); 2.8833 (4.62); 2.8665 (9.15); 2.8497 (4.27); 2.5445 (6.75); 2.5270 (0.83); 2.5096 (27.94); 2.5051 (36.59); 2.5008 (27.67); 2.0790 (3.29); -0.0002 (3.41) |
| 19-16 | 3-chlor-phenyl | CH2 | CH2 | - | H | [DMSO], Spektrometer: 399.95MHz 8.7918 (2.09); 8.7786 (3.77); 8.7654 (2.21); 7.3726 (0.45); 7.3575 (3.69); 7.3382 (16.00); 7.3192 (8.92); 7.2947 (4.95); 7.2916 (7.69); 7.2869 (5.41); 7.2749 (2.80); 7.2703 (3.49); 7.2671 (2.30); 7.2343 (6.99); 7.2158 (4.98); 3.5505 (3.89); 3.5331 (9.69); 3.5183 (9.79); 3.5011 (4.46); 3.3496 (96.29); 2.8786 (7.20); 2.8611 (14.18); 2.8435 (6.87); 2.8251 (0.35); 2.5450 (9.80); 2.5280 (0.84); 2.5144 (14.12); 2.5101 (28.09); 2.5056 (37.06); 2.5011 (27.82); 2.4969 (14.31); 2.0791 (2.50); -0.0002 (3.45) |
| 19-17 | 2-fluor-phenyl | CH2 | CH2 | - | H | [DMSO], Spektrometer: 399.95MHz 8.8382 (2.72); 8.8249 (4.94); 8.8114 (2.83); 7.3493 (3.12); 7.3453 (3.77); 7.3307 (6.31); 7.3261 (7.78); 7.3178 (1.32); 7.3110 (5.26); 7.3070 (6.13); 7.2978 (2.28); 7.2930 (4.44); 7.2868 (3.36); 7.2783 (4.56); 7.2725 (5.67); 7.2681 (3.20); 7.2589 (3.29); 7.2544 (2.70); 7.1846 (6.09); 7.1640 (12.14); 7.1479 (10.66); 7.1451 (10.10); 7.1411 (5.38); 7.1380 (4.57); 7.1295 (4.67); 7.1266 (4.17); 3.5382 (5.31); 3.5210 (12.15); 3.5055 (12.02); 3.4881 (5.99); 3.3396 (151.75); 2.9086 (8.60); 2.8908 (16.00); 2.8730 (7.82); 2.6771 (0.50); 2.6726 (0.70); 2.6680 (0.54); 2.5430 (16.86); 2.5338 (0.86); 2.5258 (2.20); 2.5124 (39.67); 2.5080 (79.89); 2.5035 (106.15); 2.4989 (79.67); 2.4945 (40.94); 2.3347 (0.48); 2.3302 (0.67); 2.3257 (0.51); 2.0774 (1.75); 0.0080 (0.57); -0.0002 (16.34); -0.0084 (0.71) |
| 19-18 | 2,6-difluor-phenyl | CH2 | CH2 | - | H | [DMSO], Spektrometer: 399.95MHz 8.9007 (2.81); 8.8864 (5.25); 8.8721 (2.89); 7.3756 (1.65); 7.3588 (3.71); 7.3546 (3.45); 7.3378 (7.21); 7.3205 (3.71); 7.3170 (4.72); 7.3002 (2.18); 7.1144 (0.95); 7.1101 (1.39); 7.0980 (10.03); 7.0899 (2.24); 7.0782 (16.00); 7.0673 (2.34); 7.0581 (8.70); 7.0458 (1.29); 3.5129 (4.77); 3.4960 (13.06); 3.4802 (13.39); 3.4636 (5.42); 3.3679 (0.36); 3.3420 (107.12); 2.9327 (7.50); 2.9155 (14.53); 2.8983 (6.88); 2.6738 (0.44); 2.6694 (0.33); 2.5443 (0.46); 2.5272 (1.38); 2.5137 (25.06); 2.5094 (50.22); 2.5048 (66.45); 2.5003 (49.73); 2.4958 (25.33); 2.3316 (0.42); 2.3271 (0.32); 0.0079 (0.33); -0.0002 (9.53); - 0.0086 (0.39) |
| 19-19 | 2,6-dichlor-phenyl | CH2 | CH2 | - | H | [DMSO], Spektrometer: 399.95MHz 8.9373 (1.36); 8.9229 (2.62); 8.9083 (1.40); 7.4765 (11.58); 7.4564 (16.00); 7.3161 (5.43); 7.2970 (5.29); 7.2951 (5.32); 7.2758 (3.58); 3.5576 (2.10); 3.5408 (5.68); 3.5246 (5.85); 3.5080 (2.59); 3.3415 (57.81); 3.1951 (4.63); 3.1777 (8.55); 3.1604 (3.93); 2.5441 (5.45); 2.5270 (0.80); 2.5136 (14.24); 2.5092 (28.83); 2.5046 (38.37); 2.5001 (28.84); 2.4957 (14.83); 2.0788 (2.44); -0.0002 (5.53) |
| 19-20 | 3-(trifluormethyl)-phenyl | CH2 | CH2 | - | H | [DMSO], Spektrometer: 399.95MHz 8.7964 (2.23); 8.7835 (3.96); 8.7703 (2.26); 7.6628 (0.40); 7.6154 (9.26); 7.6001 (3.27); 7.5966 (3.26); 7.5908 (3.88); 7.5760 (16.00); 7.5625 (9.95); 7.5502 (3.78); 7.5387 (1.89); 7.5262 (1.44); 7.4994 (0.38); 3.5867 (3.96); 3.5695 (10.42); 3.5548 (10.75); 3.5378 (4.57); 3.3419 (134.70); 2.9749 (7.43); 2.9576 (14.97); 2.9403 (6.88); 2.6775 (0.51); 2.6729 (0.68); 2.6685 (0.52); 2.5433 (17.34); 2.5263 (2.41); 2.5128 (40.93); 2.5084 (80.03); 2.5039 (104.05); 2.4994 (77.50); 2.4950 (39.59); 2.3351 (0.50); 2.3307 (0.68); 2.3262 (0.49); 2.0778 (0.93); 0.0080 (0.69); -0.0002 (17.79); -0.0085 (0.76) |
| 19-21 | 4-(trifluormethyl)-phenyl | CH2 | CH2 | - | H | [DMSO], Spektrometer: 399.95MHz 8.8177 (2.60); 8.8041 (4.86); 8.7904 (2.63); 7.6831 (13.11); 7.6630 (16.00); 7.4994 (14.89); 7.4794 (12.39); 3.5812 (4.57); 3.5638 (11.19); 3.5489 (11.29); 3.5316 (5.14); 3.3418 (176.25); 2.9659 (7.54); 2.9483 (14.53); 2.9307 (6.82); 2.6778 (0.52); 2.6731 (0.70); 2.6686 (0.55); 2.5436 (18.36); 2.5266 (2.35); 2.5218 (3.55); 2.5131 (40.06); 2.5087 (80.58); 2.5041 (106.74); 2.4996 (79.60); 2.4952 (40.28); 2.3355 (0.48); 2.3310 (0.68); 2.3266 (0.48); 2.0780 (1.55); 0.0080 (0.58); -0.0002 (17.65); -0.0085 (0.75) |
| 19-22 | 2-methyl-phenyl | CH2 | CH2 | - | H | [DMSO], Spektrometer: 399.95MHz 8.8388 (0.56); 8.8252 (1.02); 8.8110 (0.57); 7.1762 (1.10); 7.1680 (1.57); 7.1600 (2.17); 7.1529 (3.48); 7.1464 (1.52); 7.1401 (1.15); 7.1353 (3.36); 7.1325 (3.75); 7.1270 (1.42); 7.1222 (2.19); 7.1175 (1.38); 7.1110 (1.65); 3.4793 (1.11); 3.4639 (1.94); 3.4568 (1.23); 3.4460 (1.65); 3.4415 (2.06); 3.4267 (1.23); 3.3398 (36.67); 2.8624 (2.18); 2.8427 (2.65); 2.8243 (1.97); 2.5424 (3.80); 2.5253 (0.57); 2.5119 (9.67); 2.5074 (19.48); 2.5029 (25.78); 2.4983 (19.12); 2.4938 (9.54); 2.3216 (16.00); 2.0768 (0.40); -0.0002 (4.25) |
| 19-23 | 2,4,6-trimethyl-phenyl | CH2 | CH2 | - | H | |
| 19-24 | 3,4-bismethoxy-phenyl | CH2 | CH2 | - | H | WO-A 1999/24413 |
| 19-25 | phenyl | CH2 | CH2 | - | H | [DMSO], Spektrometer: 399.95MHz 8.7893 (1.92); 8.7766 (3.37); 8.7637 (1.96); 7.3294 (4.40); 7.3257 (2.01); 7.3110 (11.94); 7.2976 (3.73); 7.2929 (12.13); 7.2650 (10.76); 7.2613 (16.00); 7.2443 (7.92); 7.2347 (4.70); 7.2217 (2.41); 7.2166 (6.34); 7.2110 (1.64); 7.2026 (1.43); 7.1989 (2.22); 7.1955 (1.20); 3.5342 (4.15); 3.5169 (7.84); 3.5019 (7.46); 3.4986 (7.53); 3.4832 (4.68); 3.3438 (186.18); 2.8650 (7.54); 2.8463 (12.09); 2.8284 (6.88); 2.6766 (0.41); 2.6723 (0.56); 2.6677 (0.44); 2.5427 (12.25); 2.5257 (1.71); 2.5209 (2.48); 2.5121 (30.51); 2.5078 (62.13); 2.5032 (82.86); 2.4987 (62.25); 2.4942 (31.74); 2.3346 (0.36); 2.3300 (0.52); 2.3255 (0.38); 2.0768 (0.71); -0.0002 (9.93); -0.0084 (0.41) |
| 19-26 | 4-chlor-phenyl | C(CH2-CH2) | CH2 | - | H | |
| 19-27 | 2,4-dichlor-phenyl | C(CH2-CH2) | CH2 | - | H | |
| 19-28 | 4-chlor-phenyl | CH2 | C(CH2-CH2) | - | H | |
| 19-29 | 2,4-dichlor-phenyl | CH2 | C(CH2-CH2) | - | H | |
| 19-30 | 4-chlor-phenyl | O | CH2 | CH2 | H | |
| 19-31 | 2,4-dichlor-phenyl | O | CH2 | CH2 | H | |
| 19-32 | 4-chlor-phenyl | CH2 | CH2 | CH2 | H | |
| 19-33 | 4-chlor-phenyl | NCH3 | CH2 | CH2 | H | |
| 19-34 | 2,4-dichlor-phenyl | NCH3 | CH2 | CH2 | H | |
| 19-35 | 4-chlor-phenyl | CH(OCH3) | CH2 | - | H | |
| 19-36 | 2,4-dichlor-phenyl | CH(OCH3) | CH2 | - | H | |
| 19-37 | 2-thienyl | CH2 | CH2 | - | H | Verbindung Nr. 19-37, Solvent: [DMSO], Spektrometer: 399.95MHz 8,8336 (4,16); 7,3653 (6,75); 7,3528 (7,52); 6,9787 (4,74); 6,97 (7,86); 6,9576 (7,44); 6,937 (9,26); 6,9294 (6,33); 3,5478 (4,27); 3,5305 (10,7); 3,5153 (11,05); 3,4981 (5,03); 3,3931 (0,38); 3,3266 (812,02); 3,2311 (0,42); 3,0886 (8,69); 3,0709 (16); 3,0532 (7,58); 2,6701 (3,3); 2,5397 (2,96); 2,5047 (374,78); 2,501 (476,09); 2,3279 (3,13); 1,2346 (0,43); -0,0002 (5,83 |
| 19-38 | 3-thienyl | CH2 | CH2 | - | H | |
| 19-39 | 2-furyl | CH2 | CH2 | - | H | |
| 19-40 | 3-furyl | CH2 | CH2 | - | H | |
| 19-41 | phenyl | CH2 | CH2 | CH(CH3) | H | |
| 19-42 | phenyl | CH2 | CH2 | CH2 | H | |
| 19-43 | 2-Cl-phenyl | CH2 | CH2 | CH(CH3) | H | |
| 19-44 | 4-t-butyl-phenyl | CH2 | CH2 | CH2 | H | |
| 19-45 | 4-methyl-phenyl | CH2 | CH2 | CH2 | H | |
| 19-46 | phenyl | CH2 | CH2 | CH(CH2CH3) | H | |
| 19-47 | 2-methoxy-phenyl | CH2 | CH2 | CH2 | H | |
| 19-48 | 2-methyl-phenyl | CH2 | CH2 | CH2 | H | |
| 19-49 | 3-methyl-phenyl | CH2 | CH2 | CH2 | H | |
| 19-50 | 3-chlor-phenyl | CH2 | CH2 | CH2 | H | |
| 19-51 | 2,6-difluor-phenyl | CH2 | CH2 | CH2 | H | |
| 19-52 | 4-chlor-phenyl | CH2 | CH2 | CH2 | H | |
| 19-53 | 2,6-dichlor-phenyl | CH2 | CH2 | CH(CH3) | H | |
| 19-54 | 3,5-dichlor-phenyl | CH2 | CH2 | CH(CH3) | H | |
| 19-55 | 2,6-dimethyl-phenyl | CH2 | CH2 | CH2 | H | |
| 19-56 | 2,5-dichlor-phenyl | CH2 | CH2 | CH2 | H | |
| 19-57 | 4-i-propoxy-phenyl | CH2 | CH2 | CH2 | H | |
| 19-58 | 3-trifluormethyl-phenyl | CH2 | CH2 | CH(CH3) | H | |
| 19-59 | 4-methyl-phenyl | CH2 | CH2 | CH(CH3) | H | |
| 19-60 | 2-methyl-phenyl | CH2 | CH2 | CH(CH3) | H | |
| 19-61 | 3,4-dichlor-phenyl | CH2 | CH2 | CH(CH3) | H | |
| 19-62 | 3,5-dichlor-phenyl | CH2 | CH2 | CH2 | H | |
| 19-63 | 2,6-dimethyl-phenyl | CH2 | CH2 | CH(CH3) | H | |
| 19-64 | 4-trifluormethyl-phenyl | CH2 | CH2 | CH(CH3) | H | |
| 19-65 | 2,5-dichlor-phenyl | CH2 | CH2 | CH(CH3) | H | |
| 19-66 | 4-phenoxy-phenyl | CH2 | CH2 | CH2 | H | |
| 19-67 | 3-chlor-phenyl | CH2 | CH2 | CH(CH3) | H | |
| 19-68 | 4-phenoxy-phenyl | CH2 | CH2 | CH(CH3) | H | |
| 19-69 | 2,4-dichlor-phenyl | CH2 | CH2 | CH(CH3) | H | |
| 19-70 | 2-difluormethoxy-phenyl | CH2 | CH2 | CH(CH3) | H | |
| 19-71 | 4-methoxy-phenyl | CH2 | CH2 | CH2 | H | |
| 19-72 | 4-chlor-phenyl | CH2 | CH2 | CH(CH3) | H | |
| 19-73 | 4-chlor-phenyl | CH2 | CH2 | CH(i-propyl) | H | |
| 19-74 | 4-fluor-phenyl | CH2 | CH2 | CH2 | H | |
| 19-75 | 4-chlor-phenyl | CH2 | CH2 | CH(n-propyl) | H | |
| 19-76 | 4-chlor-phenyl | CH2 | CH2 | CH(t-butyl) | H | |
| 19-77 | 2-chlor-phenyl | CH2 | CH2 | CH2 | H | |
| 19-78 | 4-chlor-phenyl | CH2 | CH(CH3) | - | cyclopropyl | |
| 19-79 | 5-chlor-2-thienyl | CH2 | CH2 | - | H | Verbindung Nr. 19-79, Solvent: [DMSO], Spektrometer: 399.95MHz 8,8608 (2,19); 8,8474 (4,01); 8,8336 (2,24); 7,3913 (15,36); 7,3875 (15,49); 6,9401 (11,63); 6,9369 (11,98); 6,5738 (0,41); 3,5496 (4,49); 3,5324 (11,85); 3,5178 (12,11); 3,5008 (5,14); 3,3233 (38,67); 3,0726 (0,4); 3,0536 (8,3); 3,0363 (16); 3,0194 (7,18); 2,6761 (0,37); 2,6716 (0,5); 2,667 (0,36); 2,525 (1,55); 2,5203 (2,32); 2,5116 (28,26); 2,5072 (57,01); 2,5026 (75,01); 2,498 (53,73); 2,4935 (25,57); 2,334 (0,36); 2,3294 (0,5); 2,3247 (0,37); 1,3361 (1,12); 1,2988 (0,33); 1,2586 (0,47); 1,2494 (1,29); 1,2326 (0,32); 1,1878 (0,37); 0,008 (2,52); -0,0002 (72,28); -0,0085 (2,36) |
| 19-80 | 4-chlor-phenyl | CH(CF3) | CH2 | - | H | Verbindung Nr. 19-80, Solvent: [DMSO], Spektrometer: 399.95MHz |
| | | | | | | 8,8846 (2,51); 7,4971 (3,7); 7,4915 (1,85); 7,4753 (16); 7,4611 (12,36); 7,4393 (3,16); 7,4048 (0,67); 4,1116 (0,8); 4,0968 (1,06); 4,0885 (1,33); 4,0736 (1,47); 4,0651 (1,07); 4,0501 (1,02); 4,0414 (0,4); 3,9563 (1,09); 3,9418 (0,97); 3,922 (1,72); 3,9084 (1,42); 3,8182 (1,33); 3,7947 (1,33); 3,763 (0,73); 3,3264 (41,38); 2,6724 (0,32); 2,5117 (19,46); 2,5075 (37,26); 2,5031 (48,06); 2,4986 (35,03); 2,4944 (17,36); 1,3365 (0,91); 1,2492 (0,84); 0,0078 (0,39); -0,0002 (9,03); -0,0084 (0,34) |

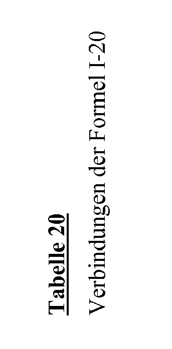

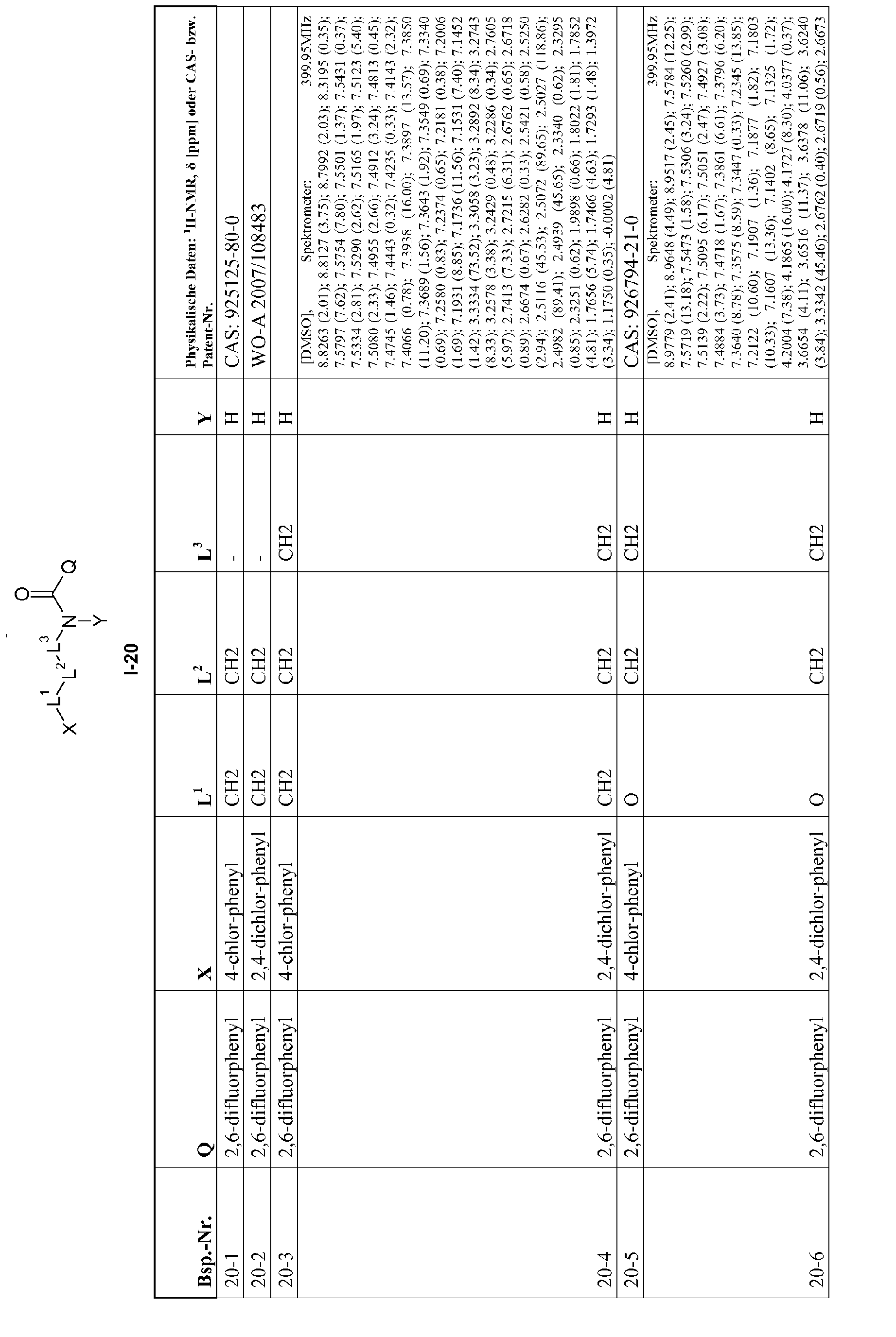

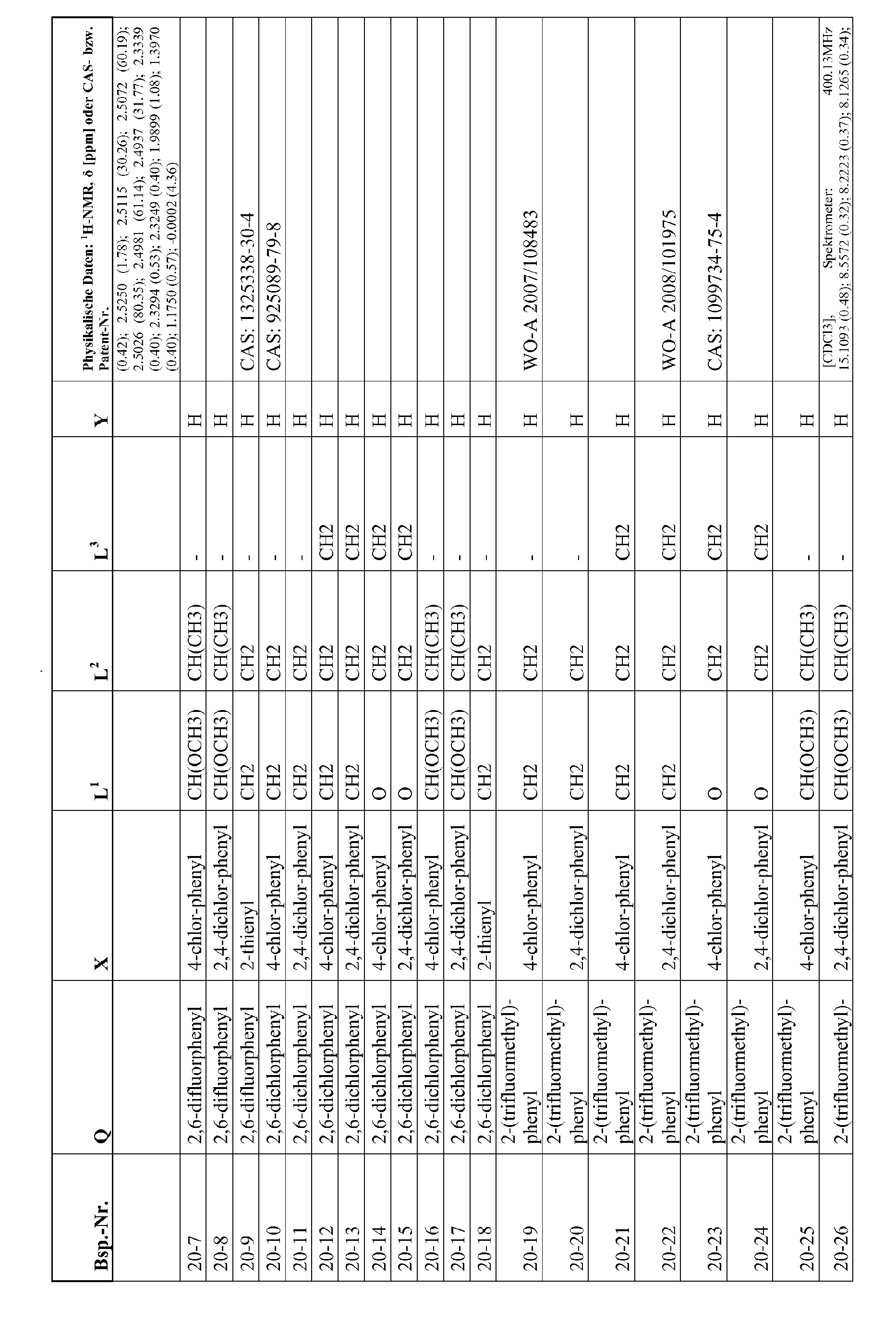

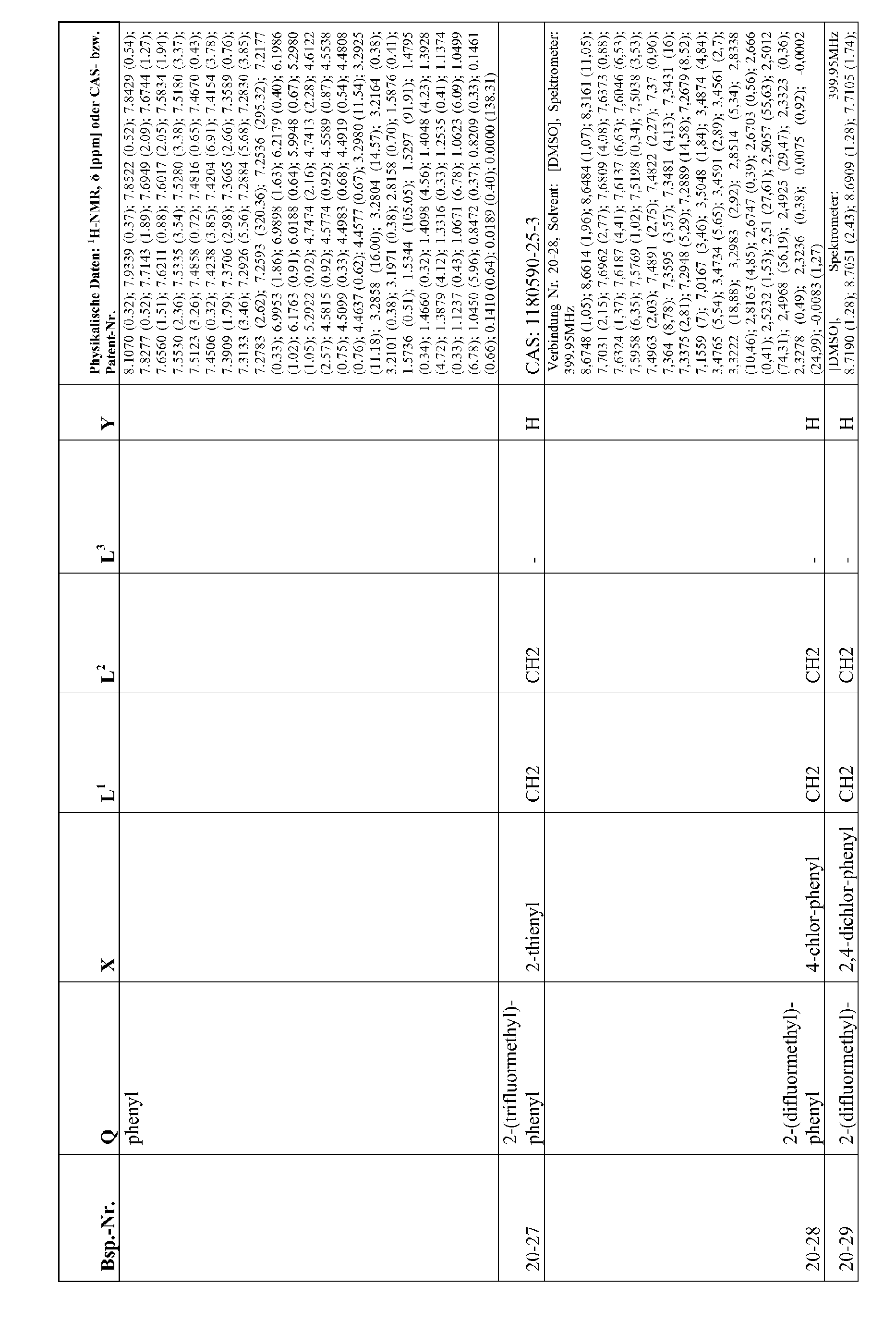

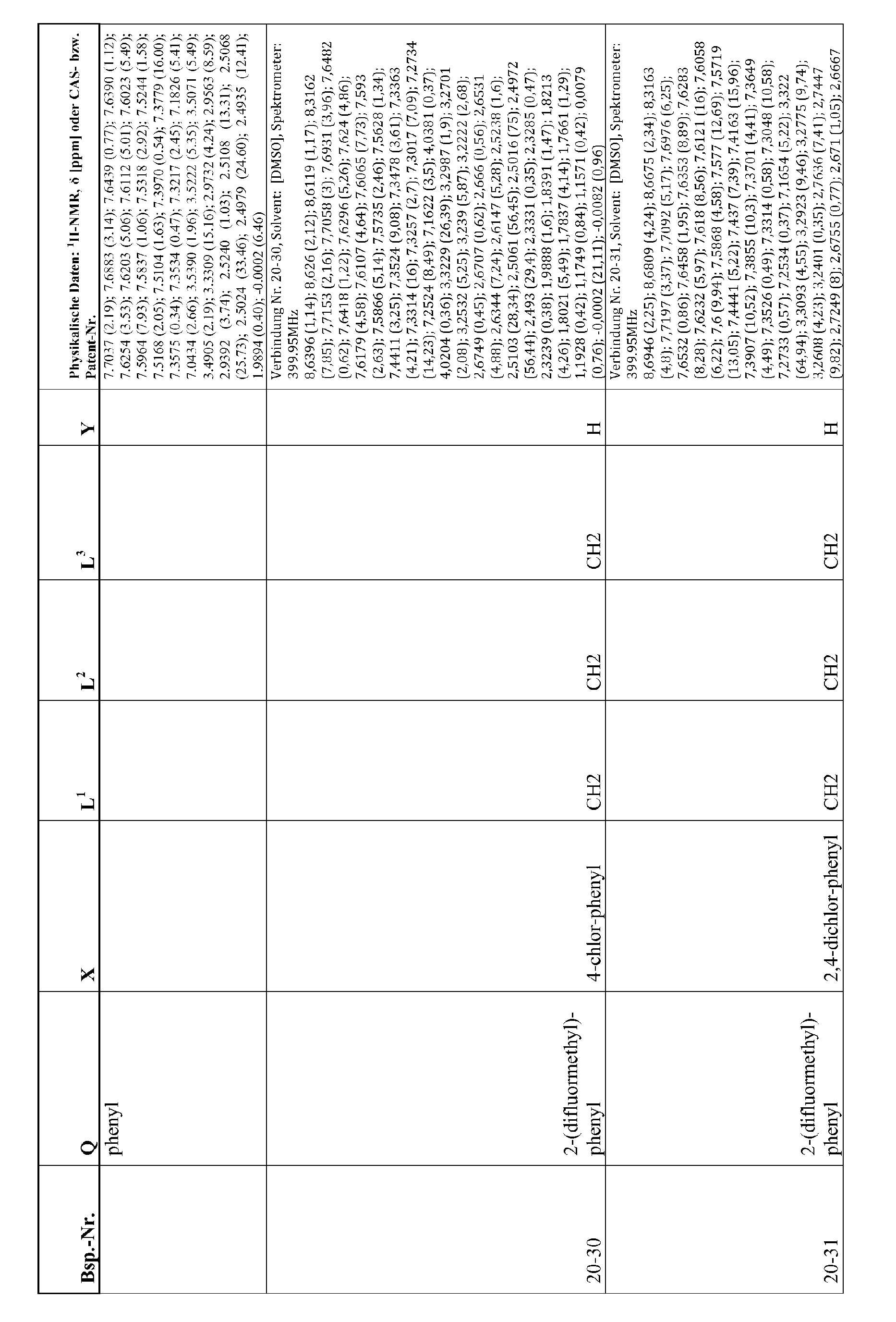

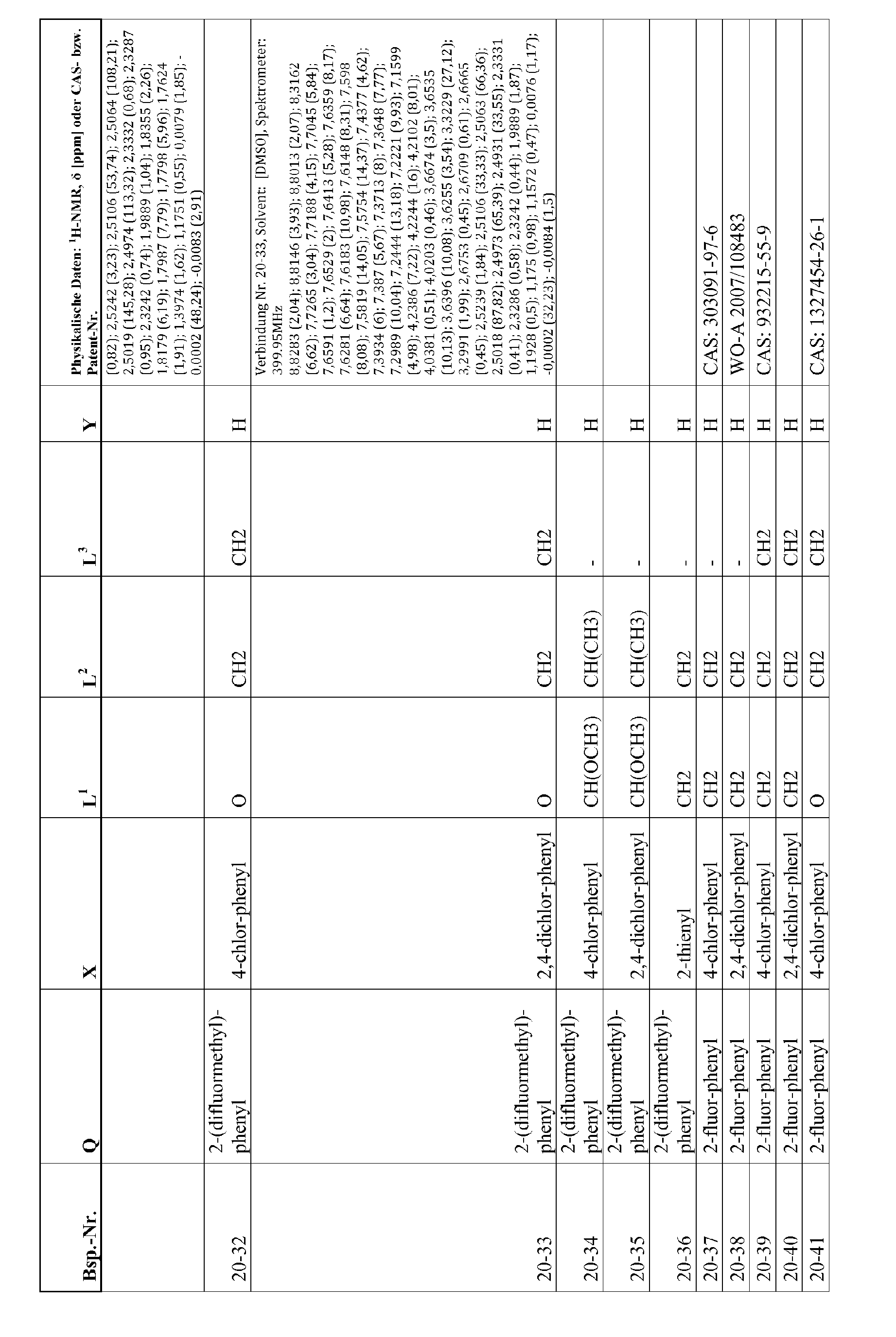

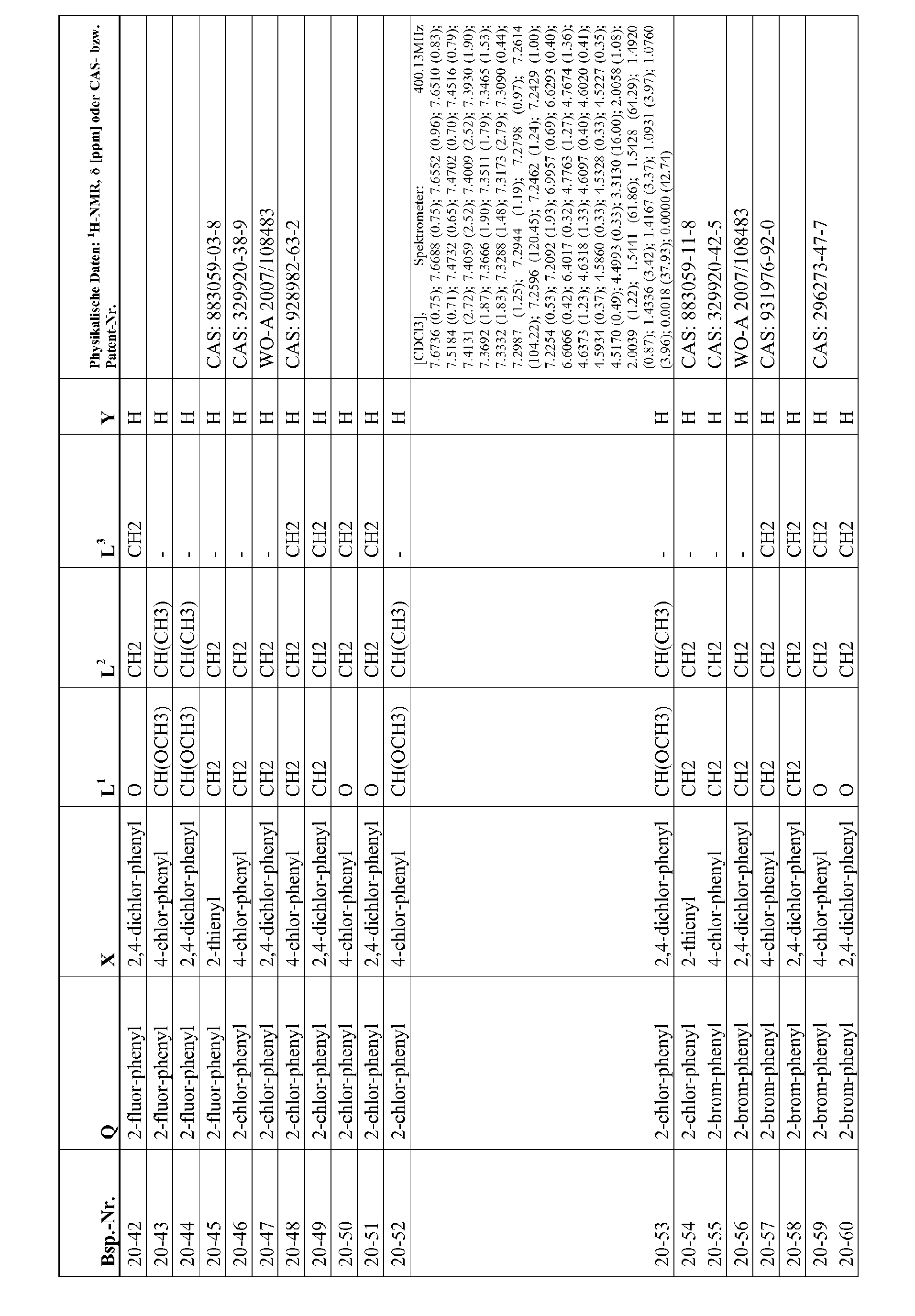

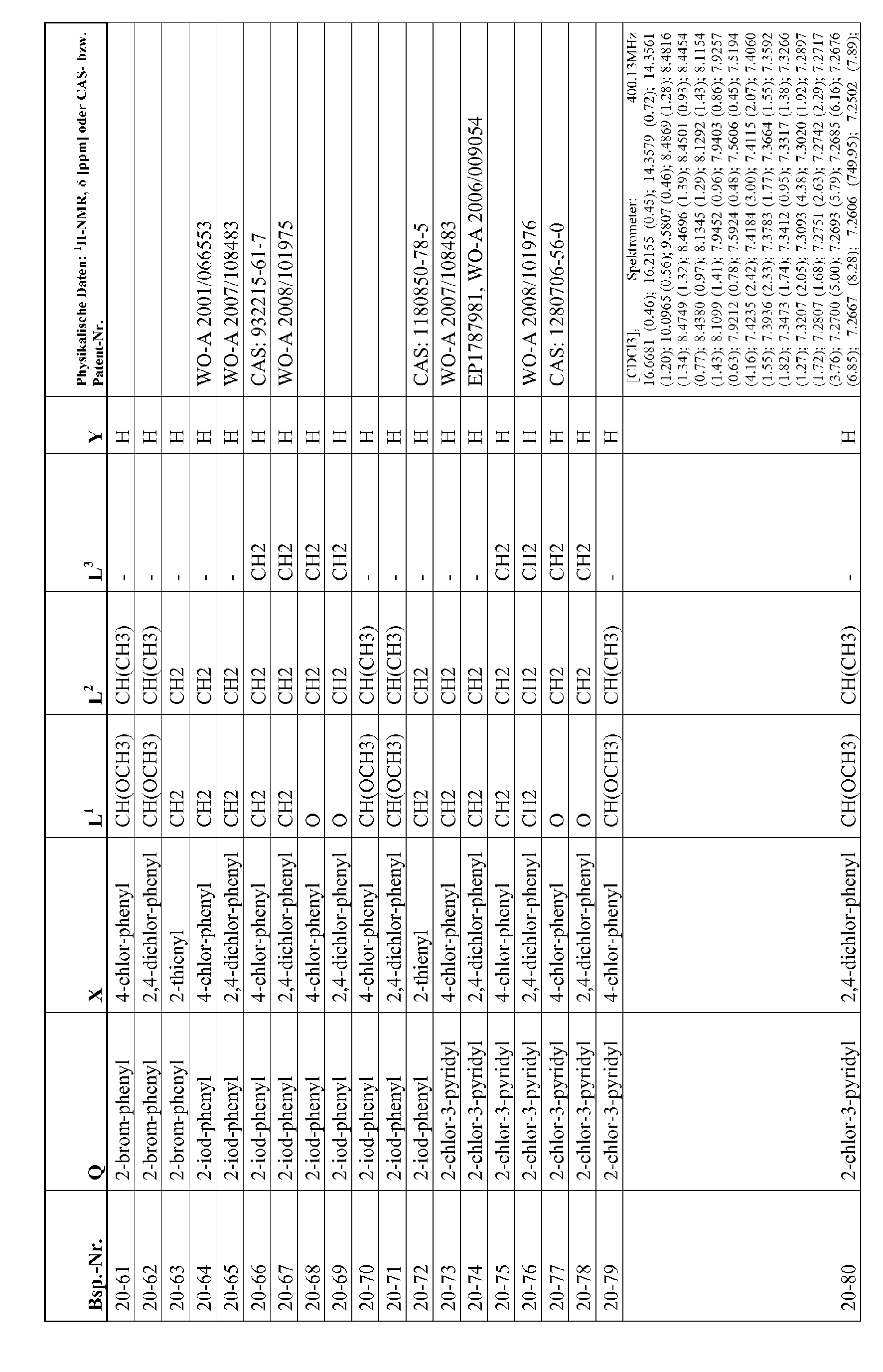

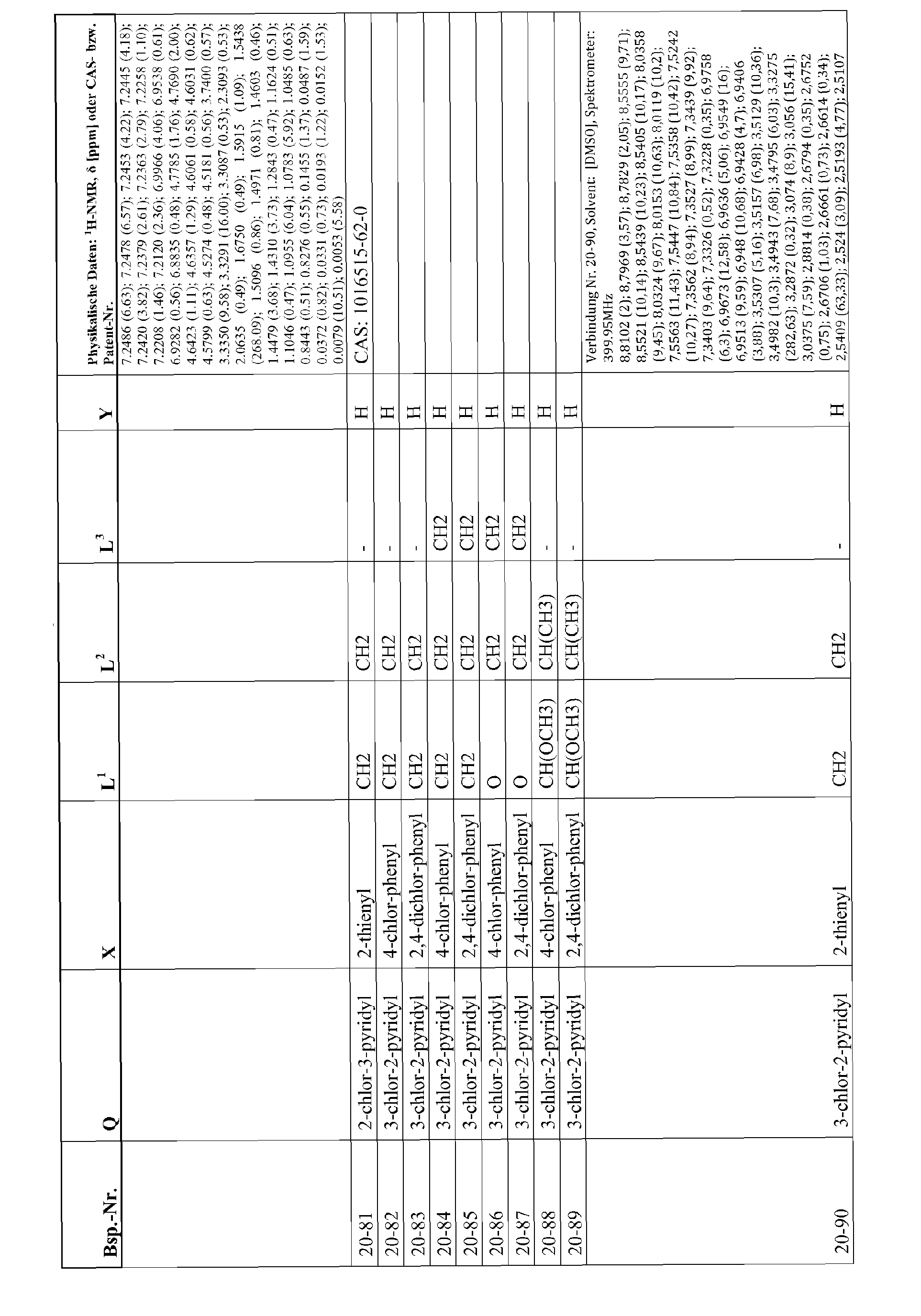

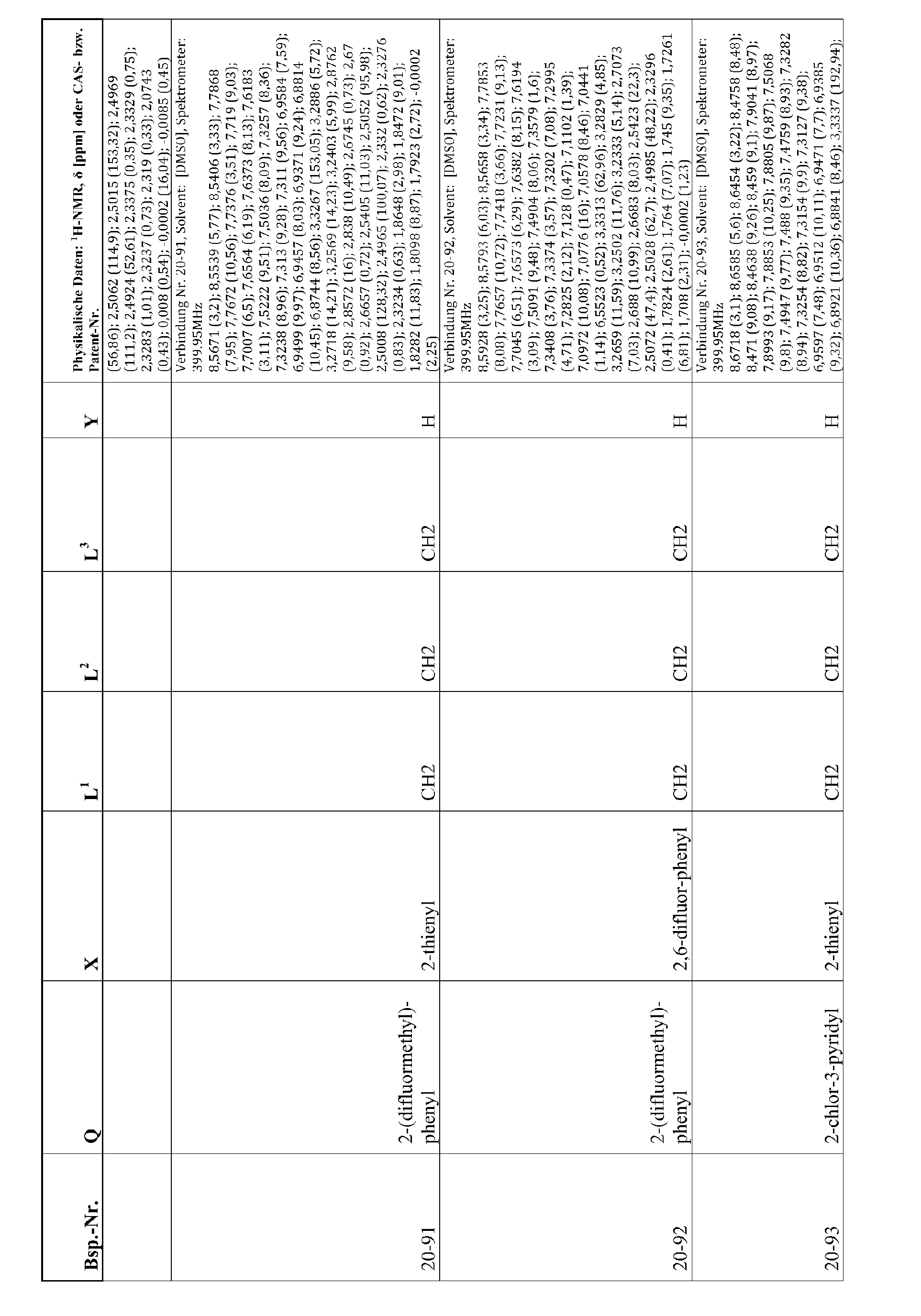

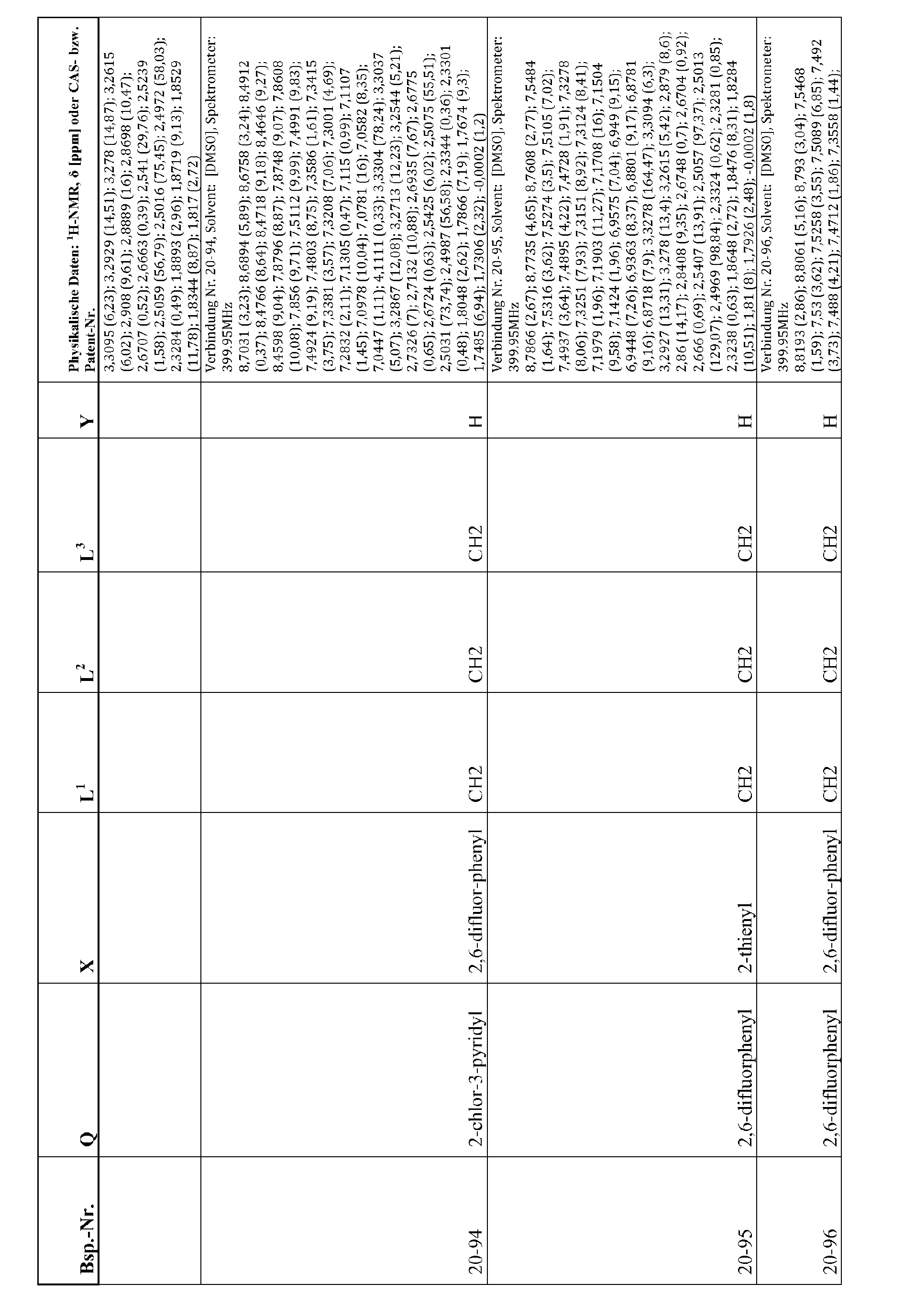

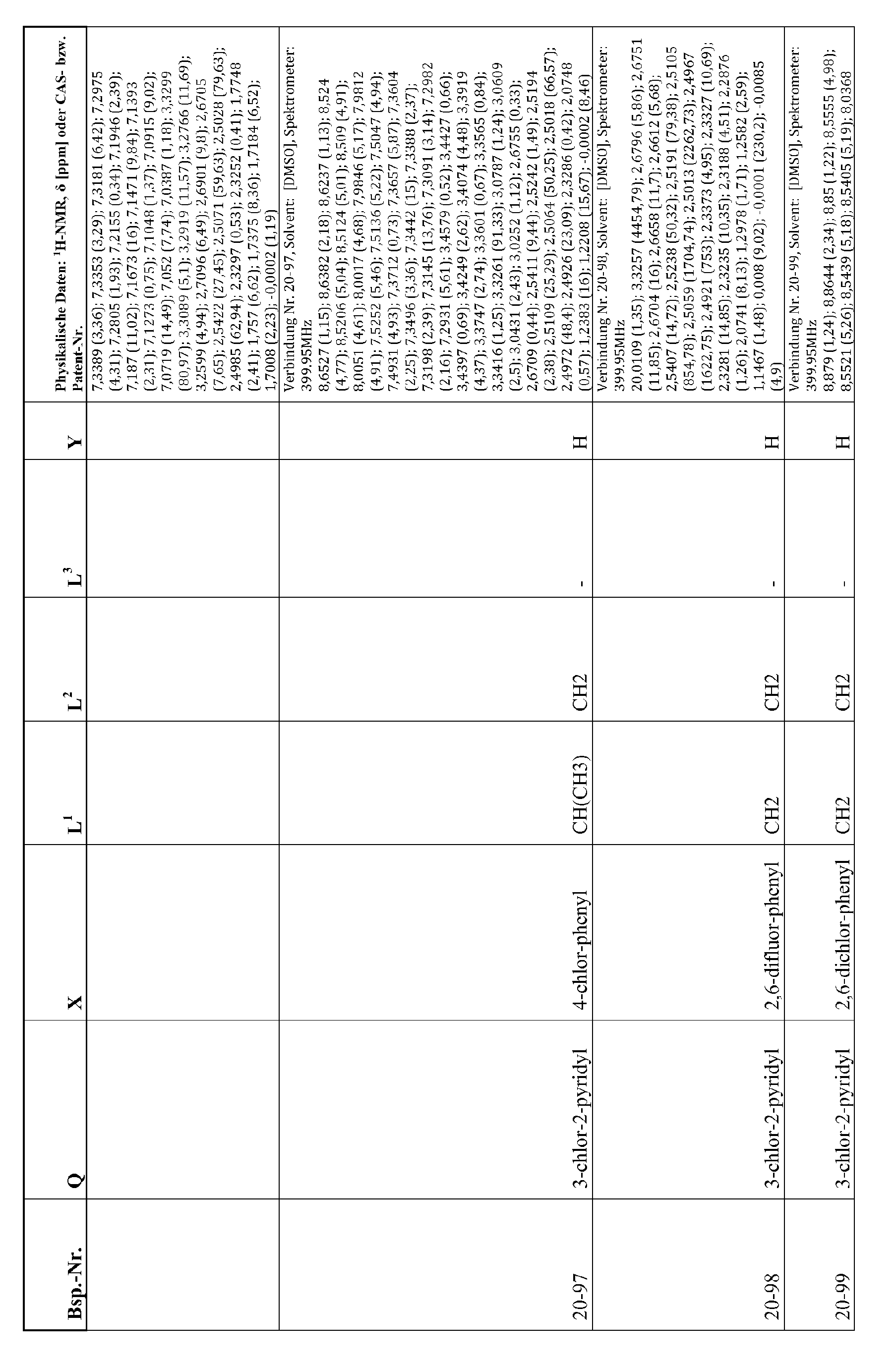

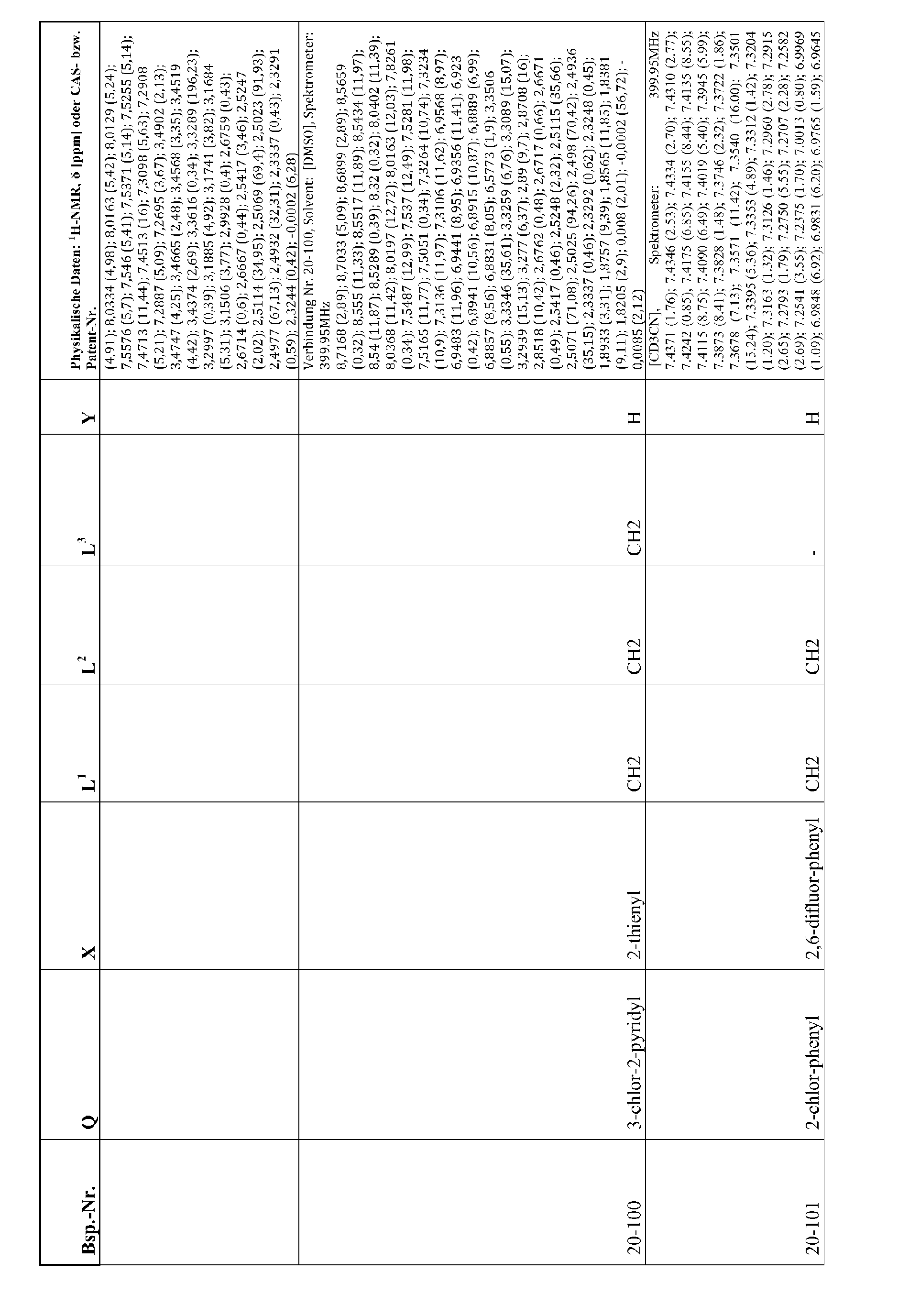

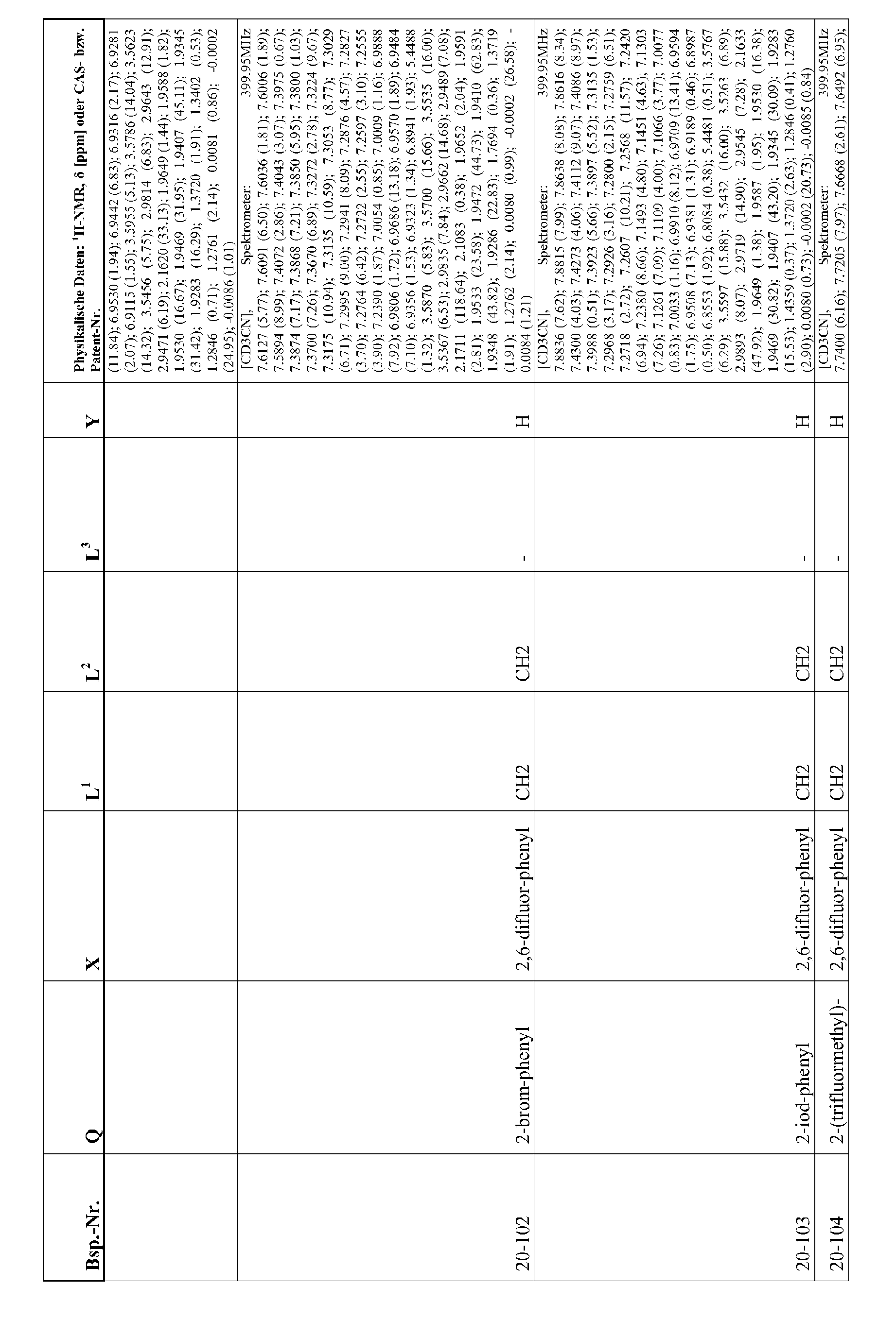

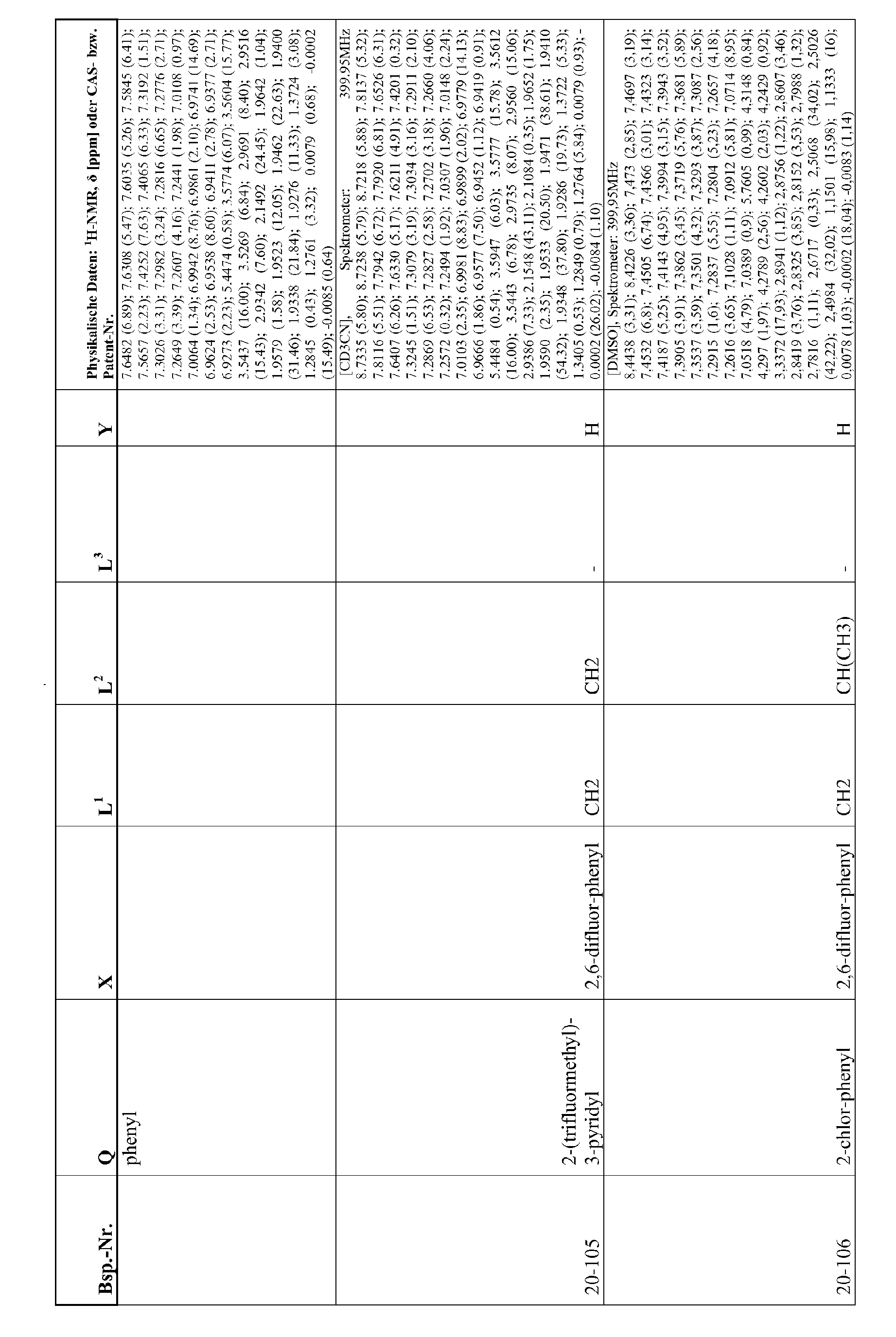

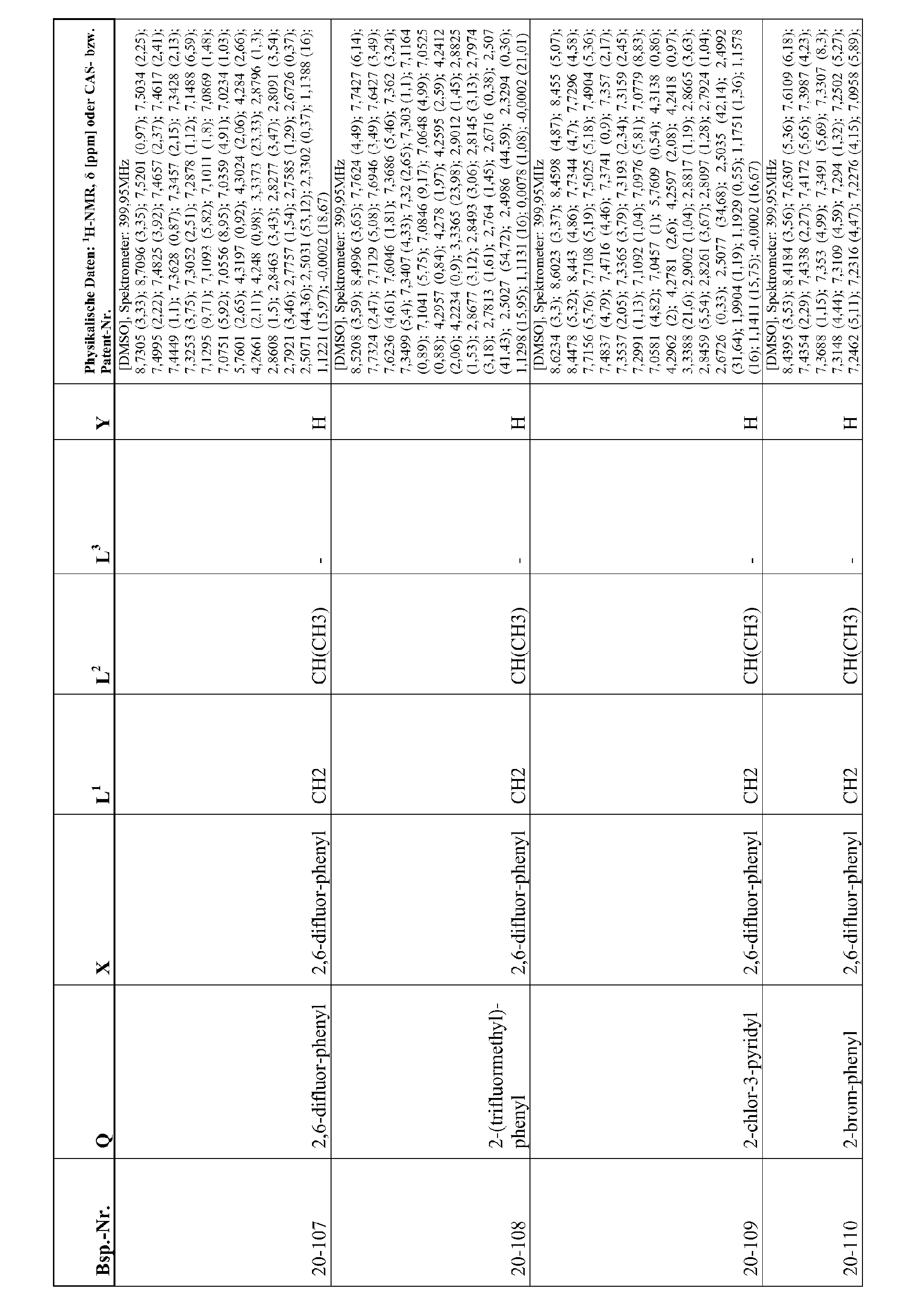

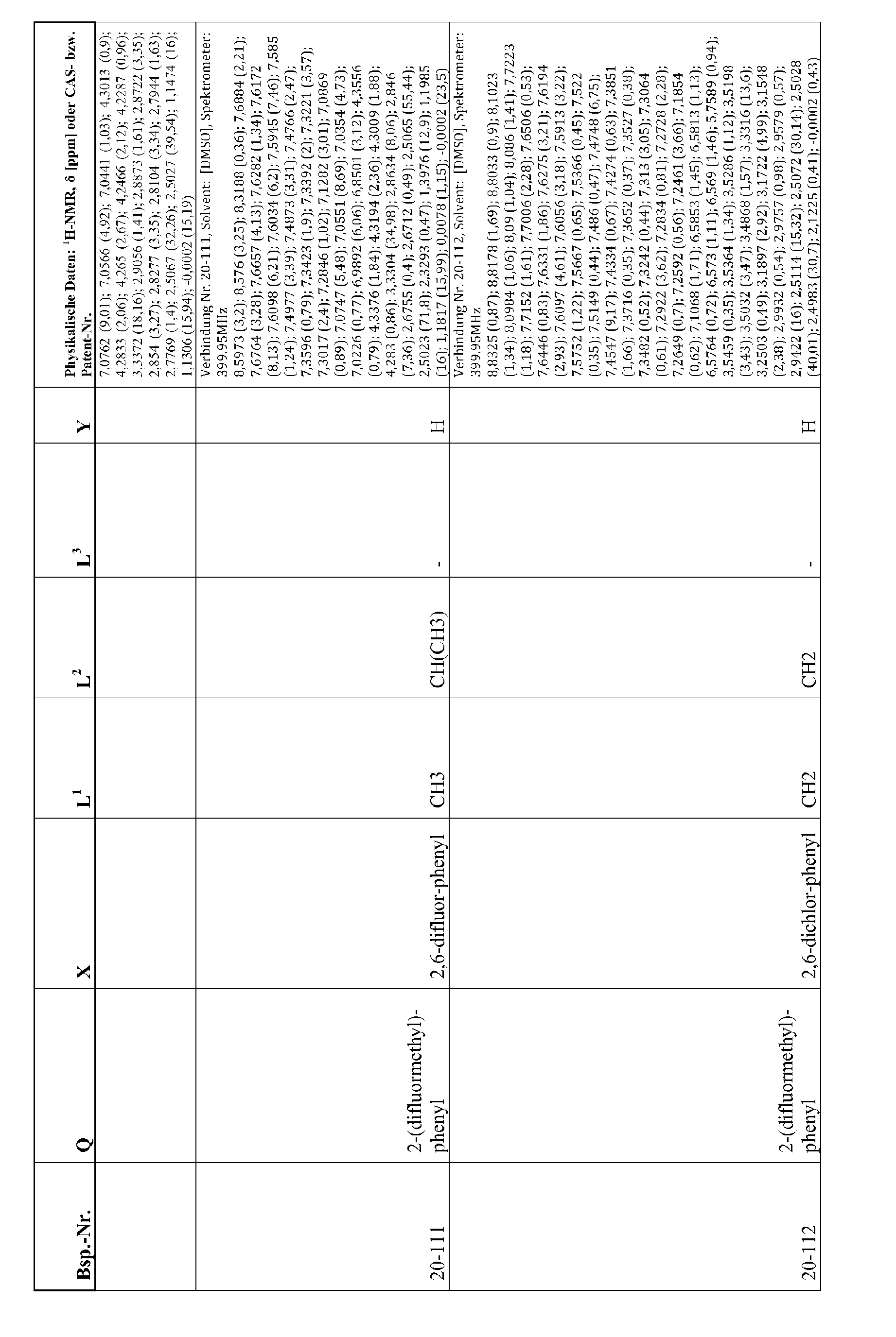

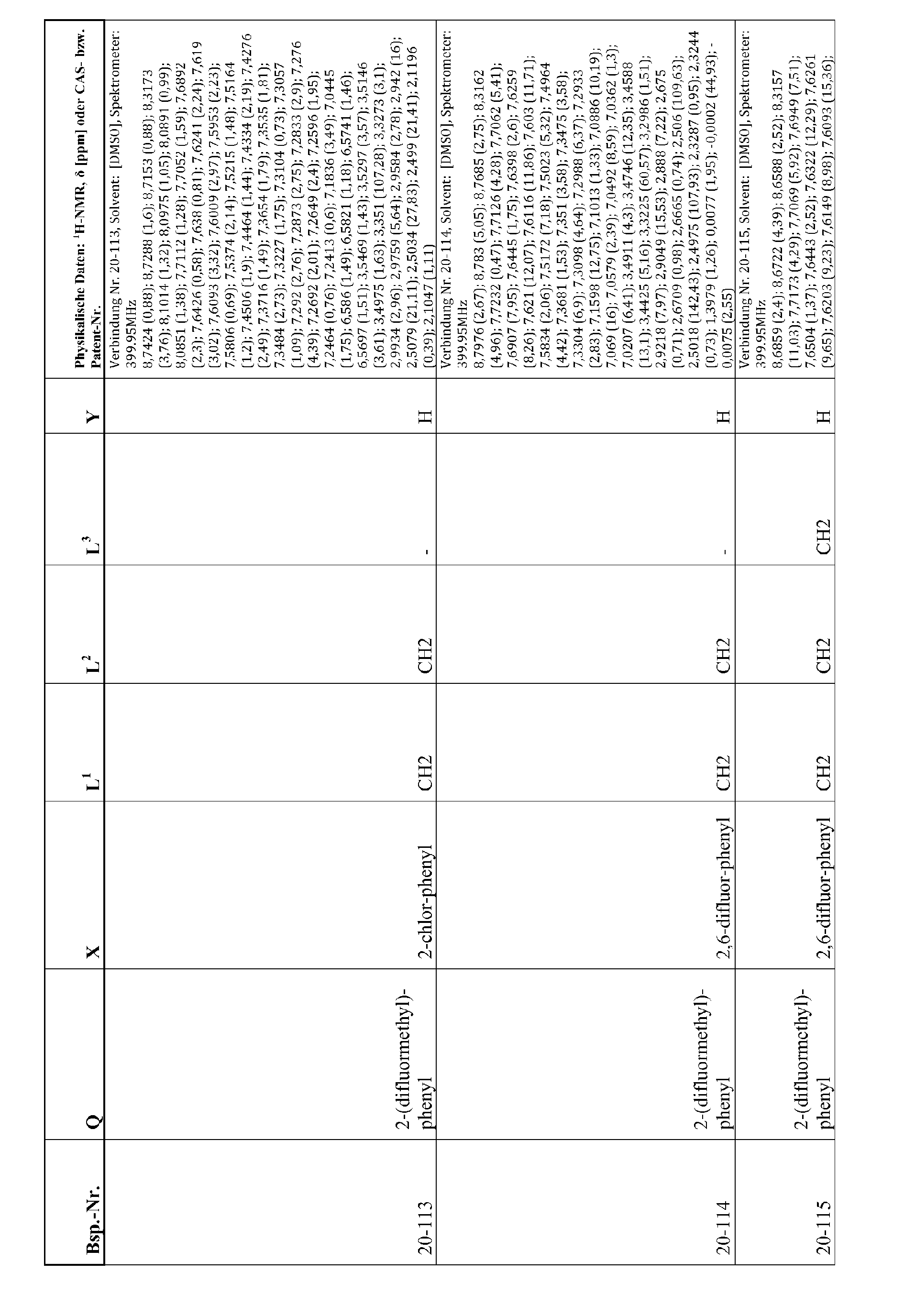

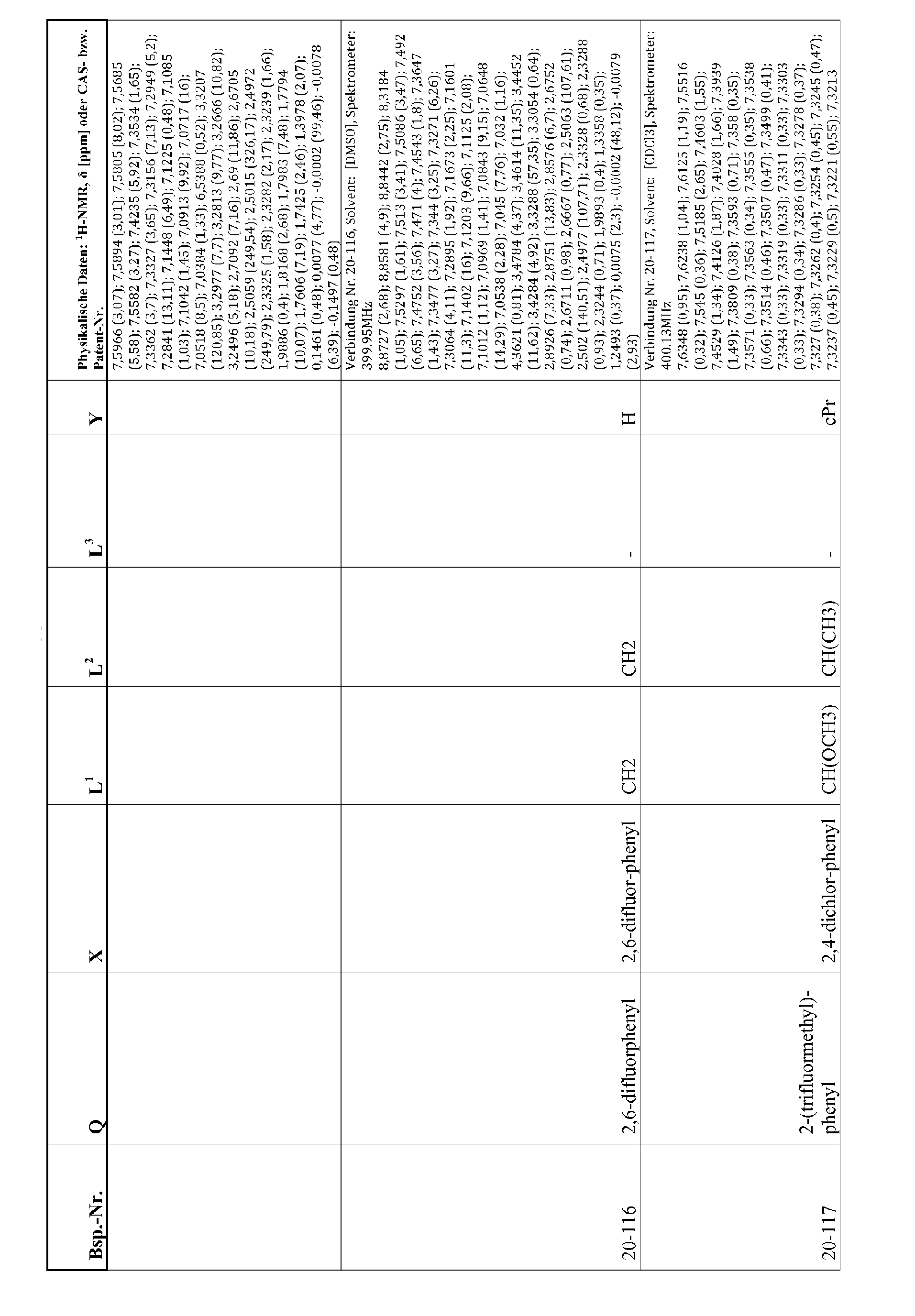

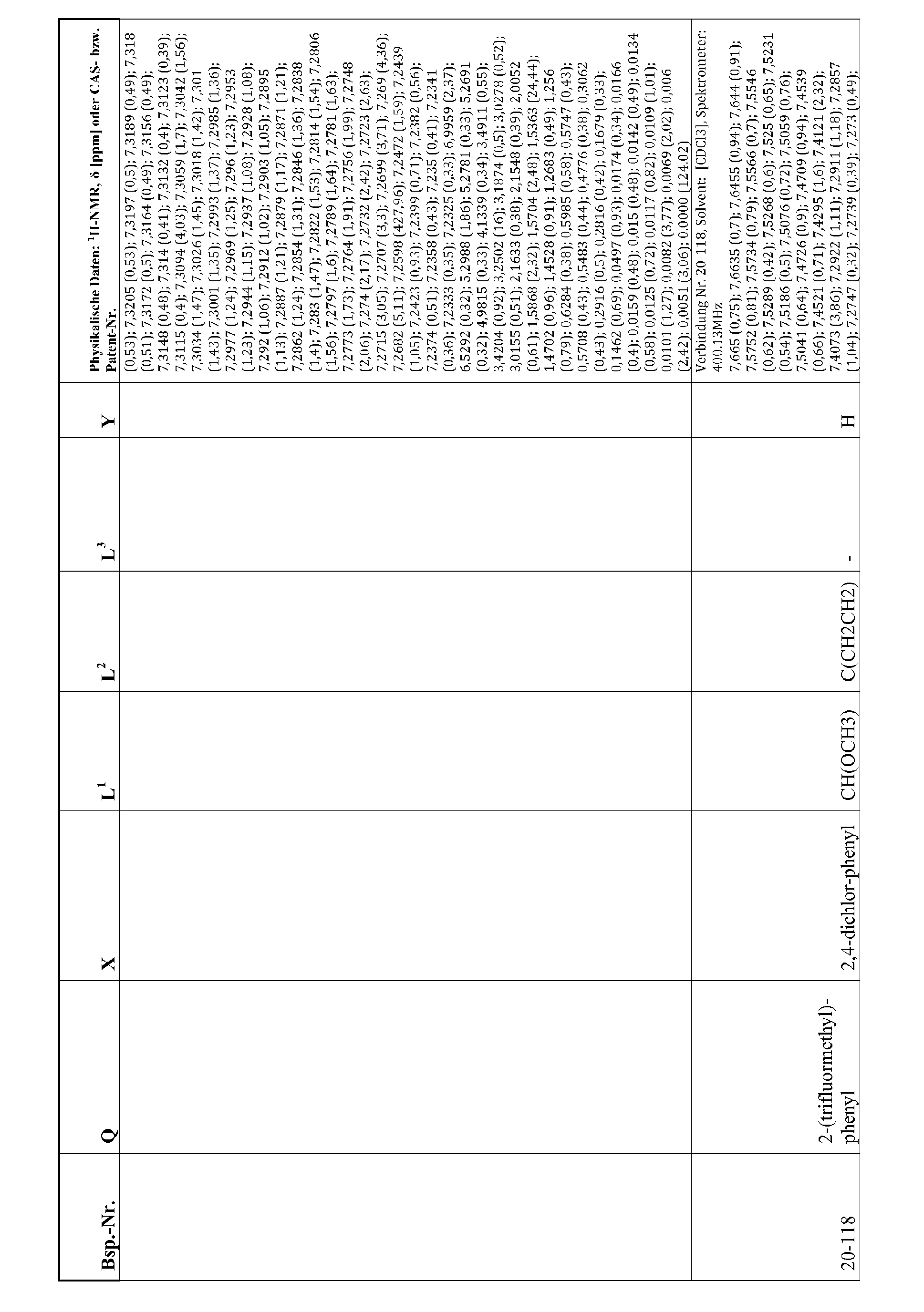

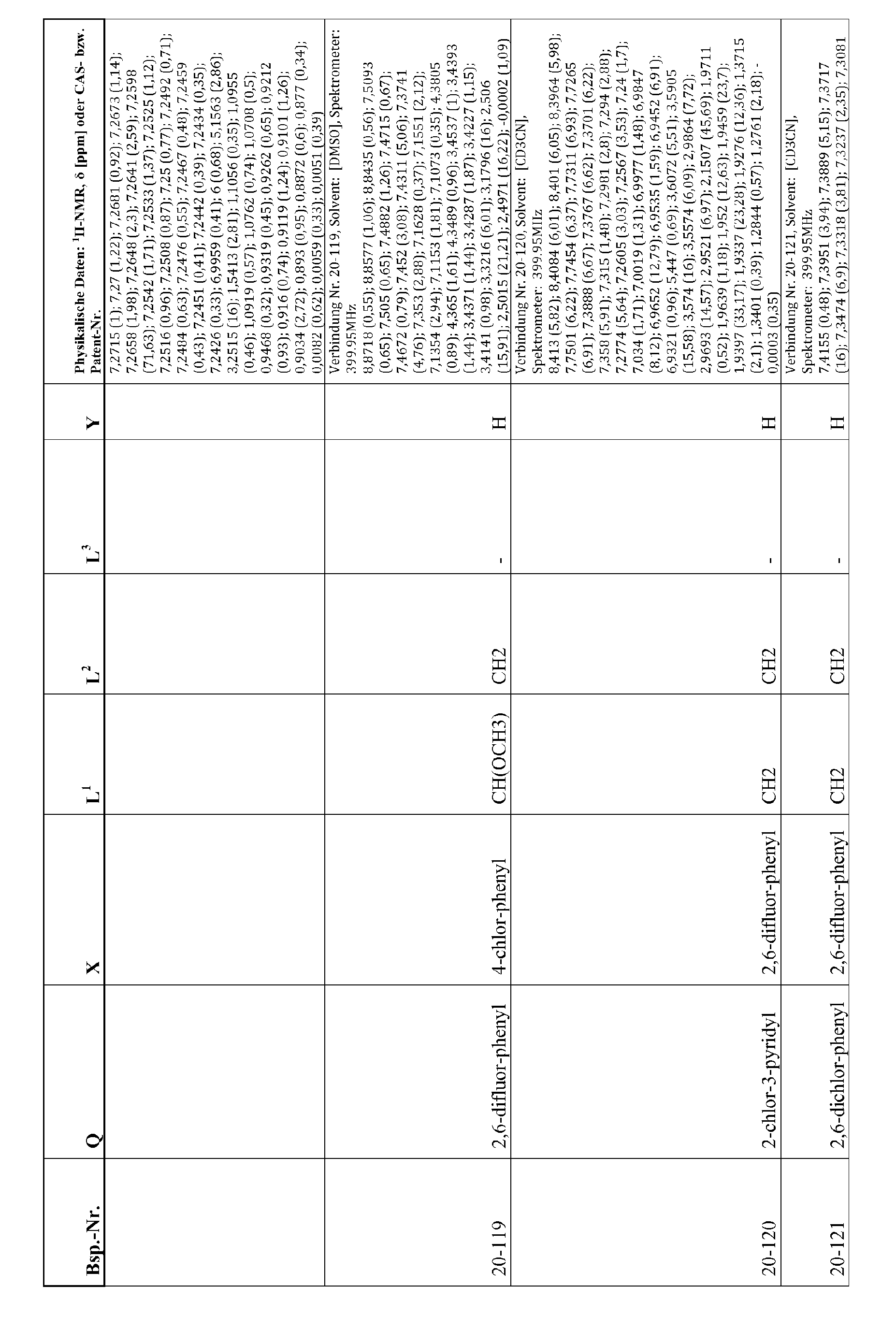

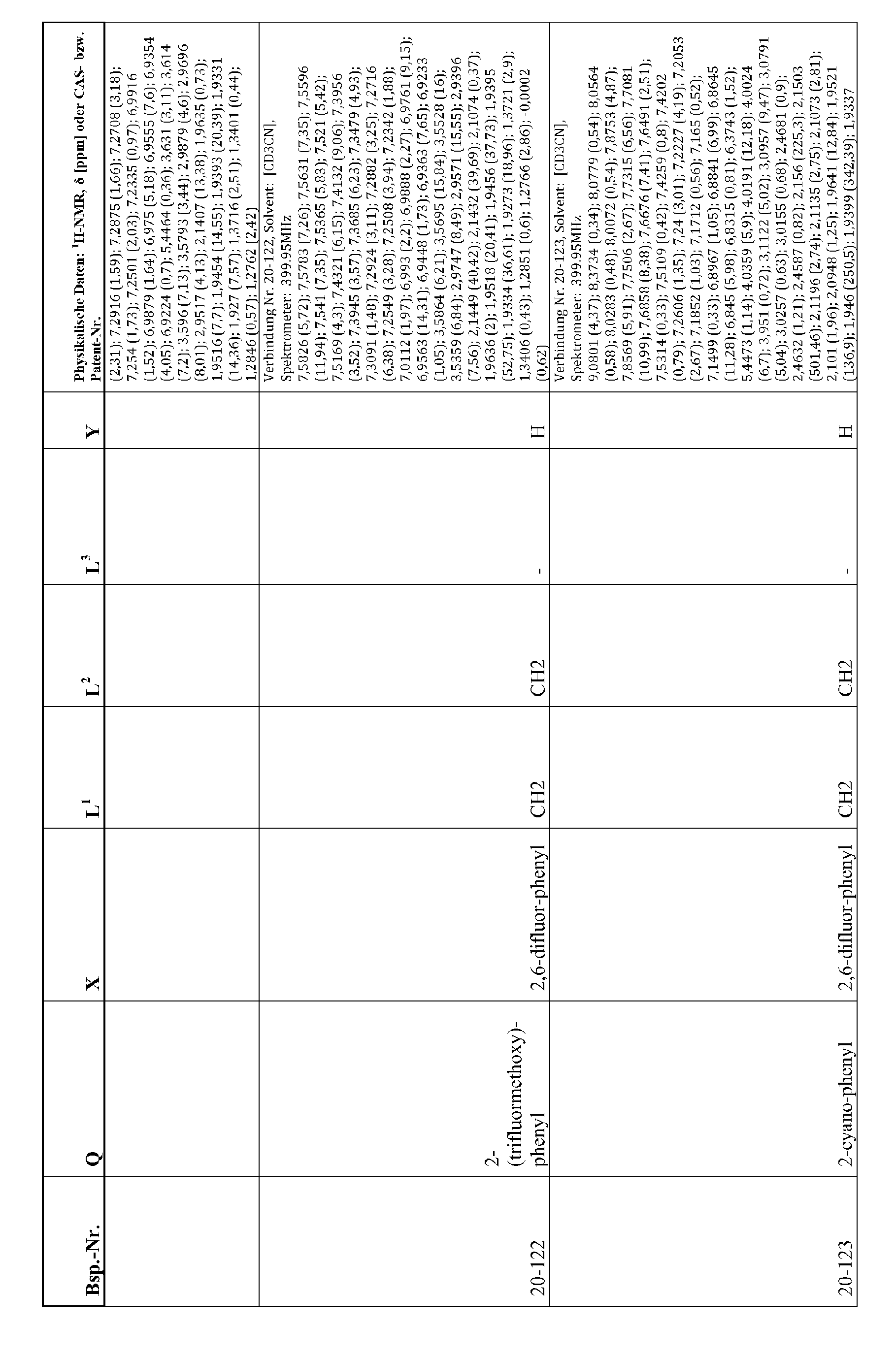

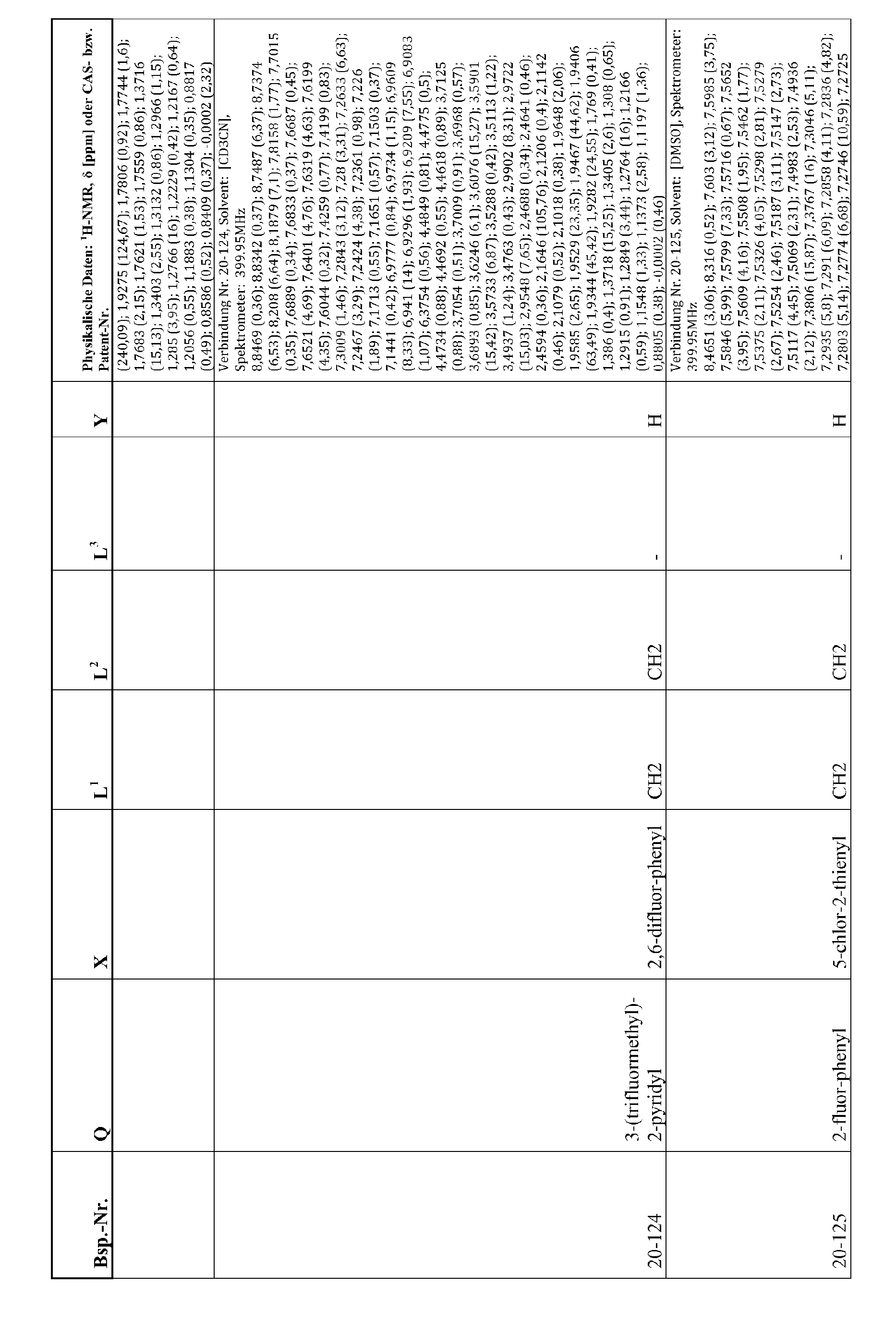

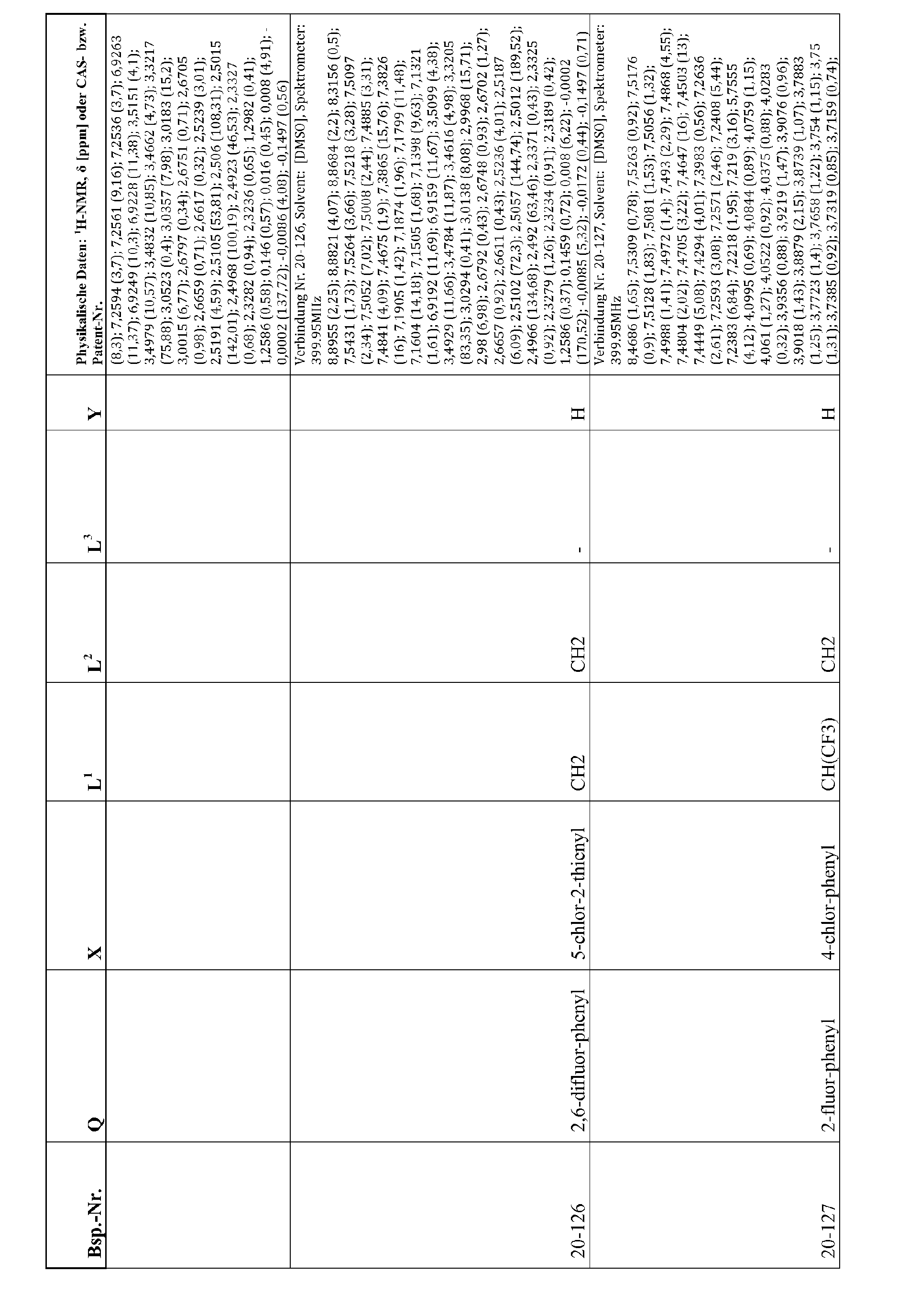

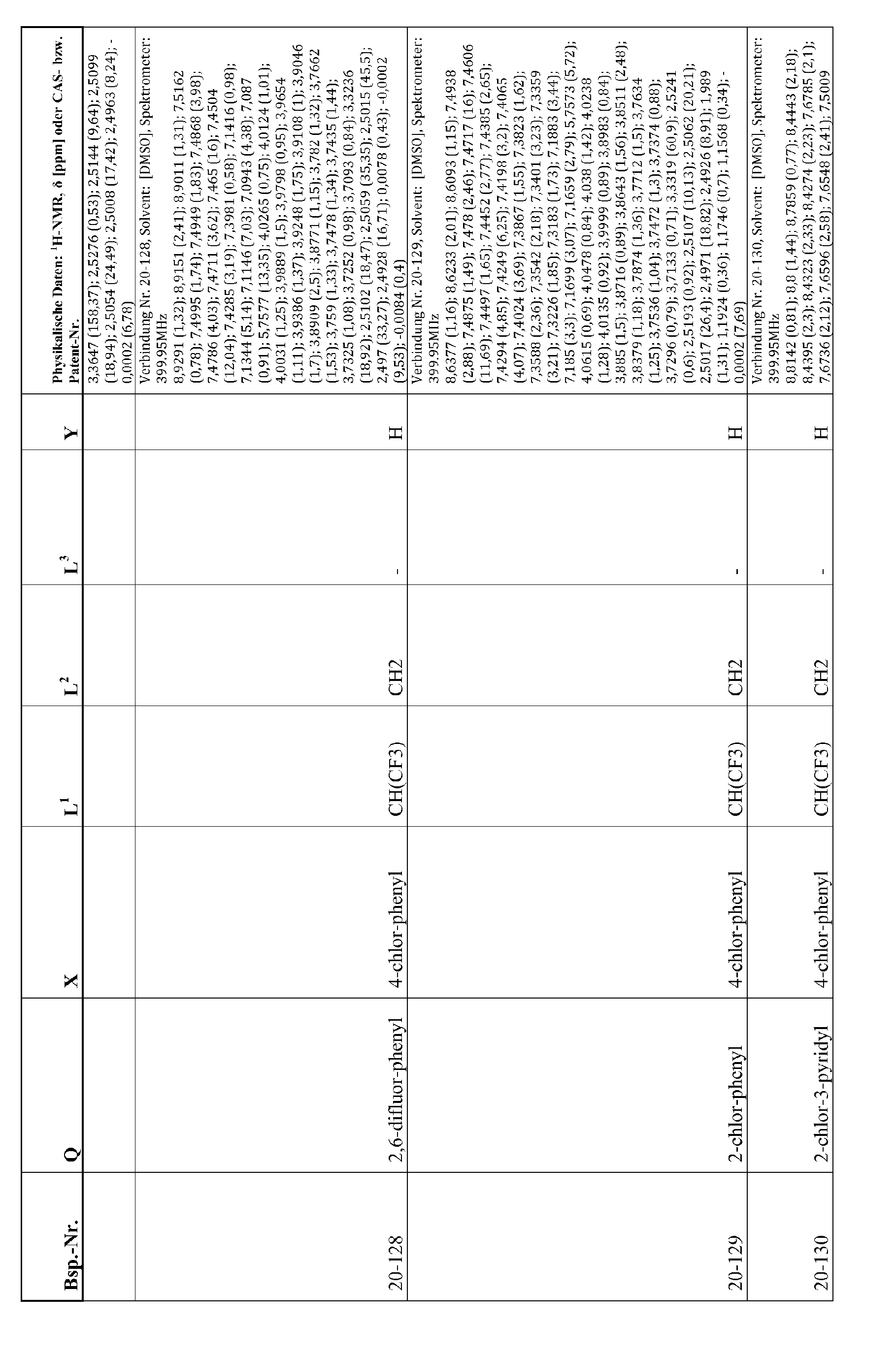

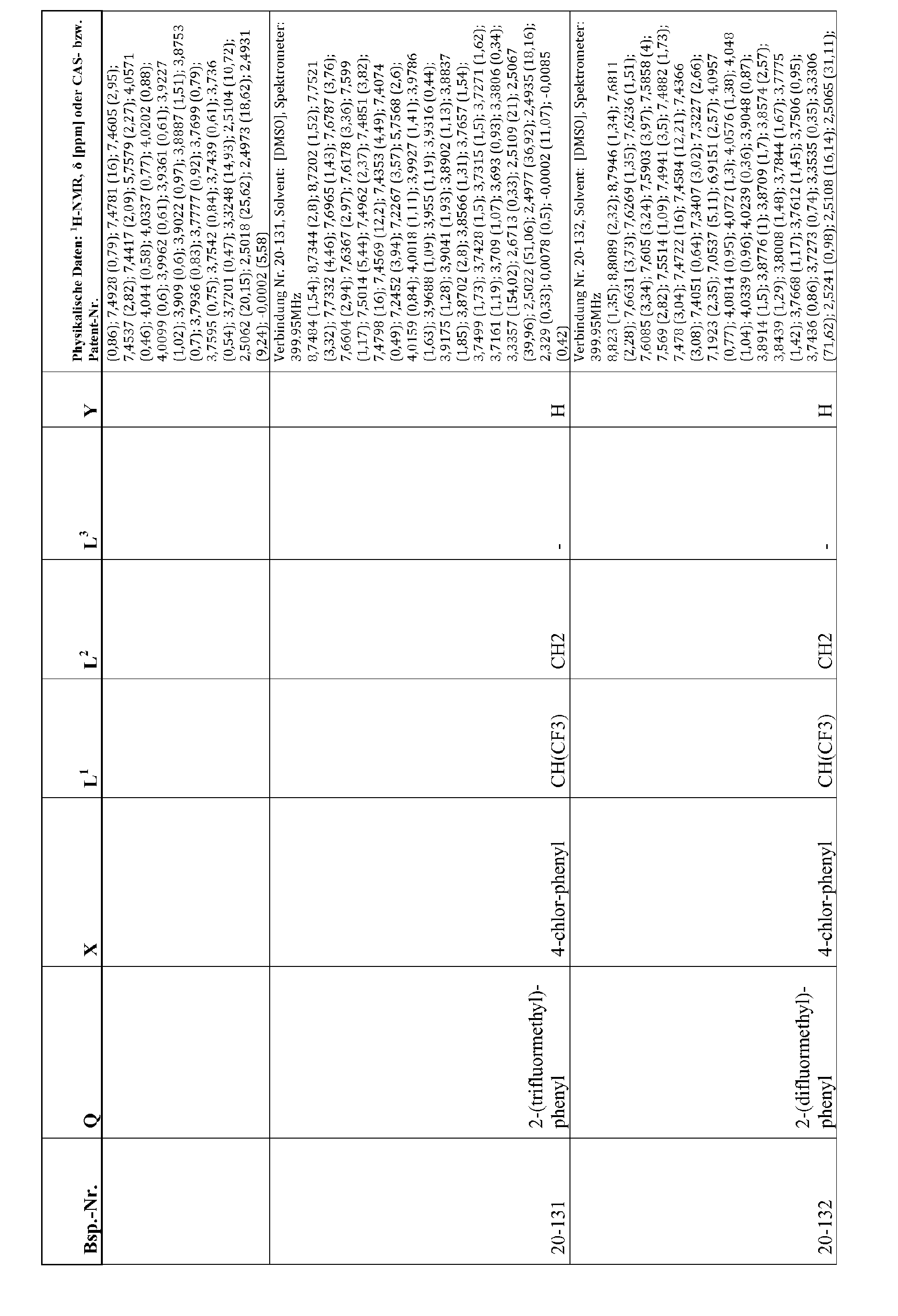

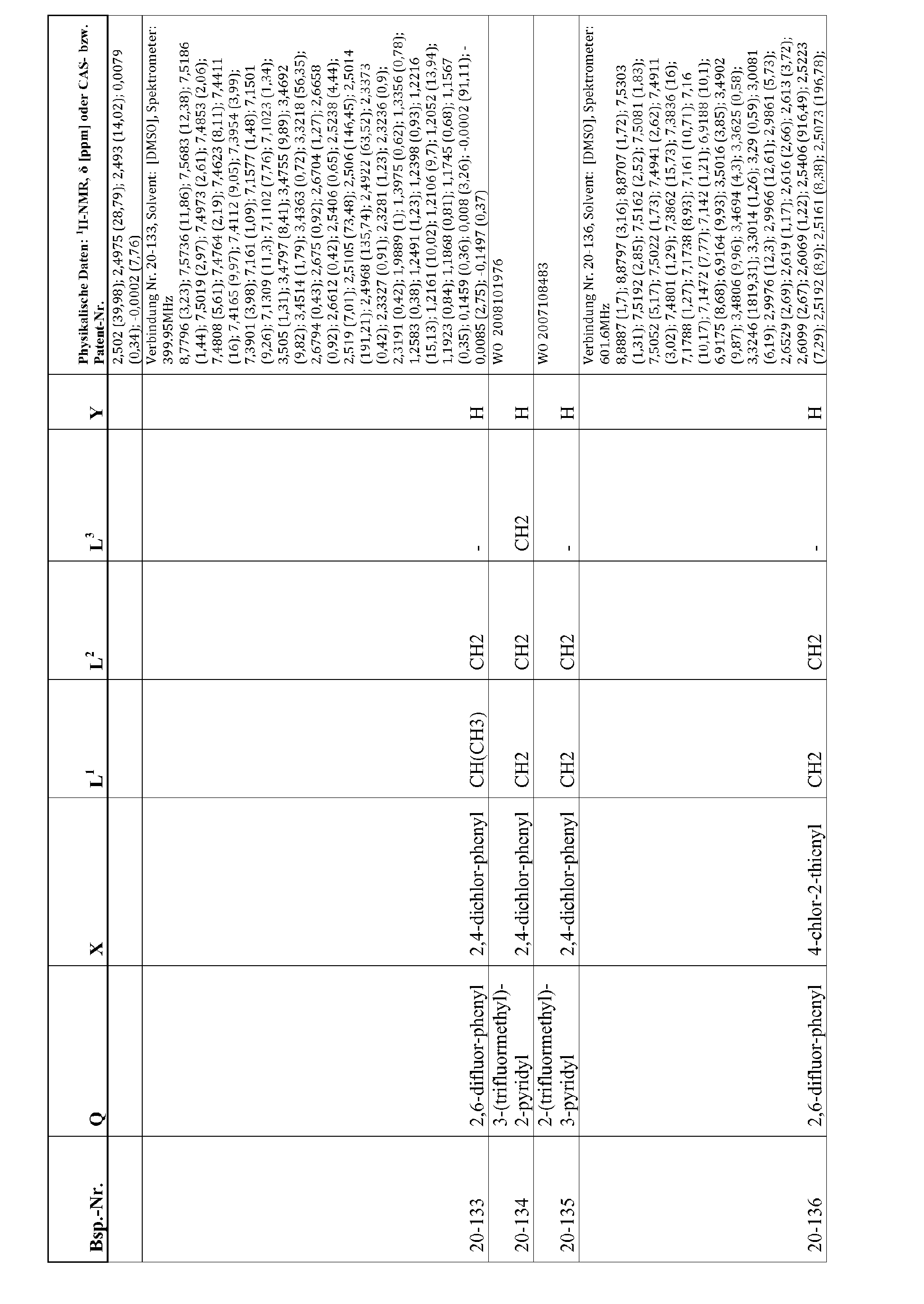

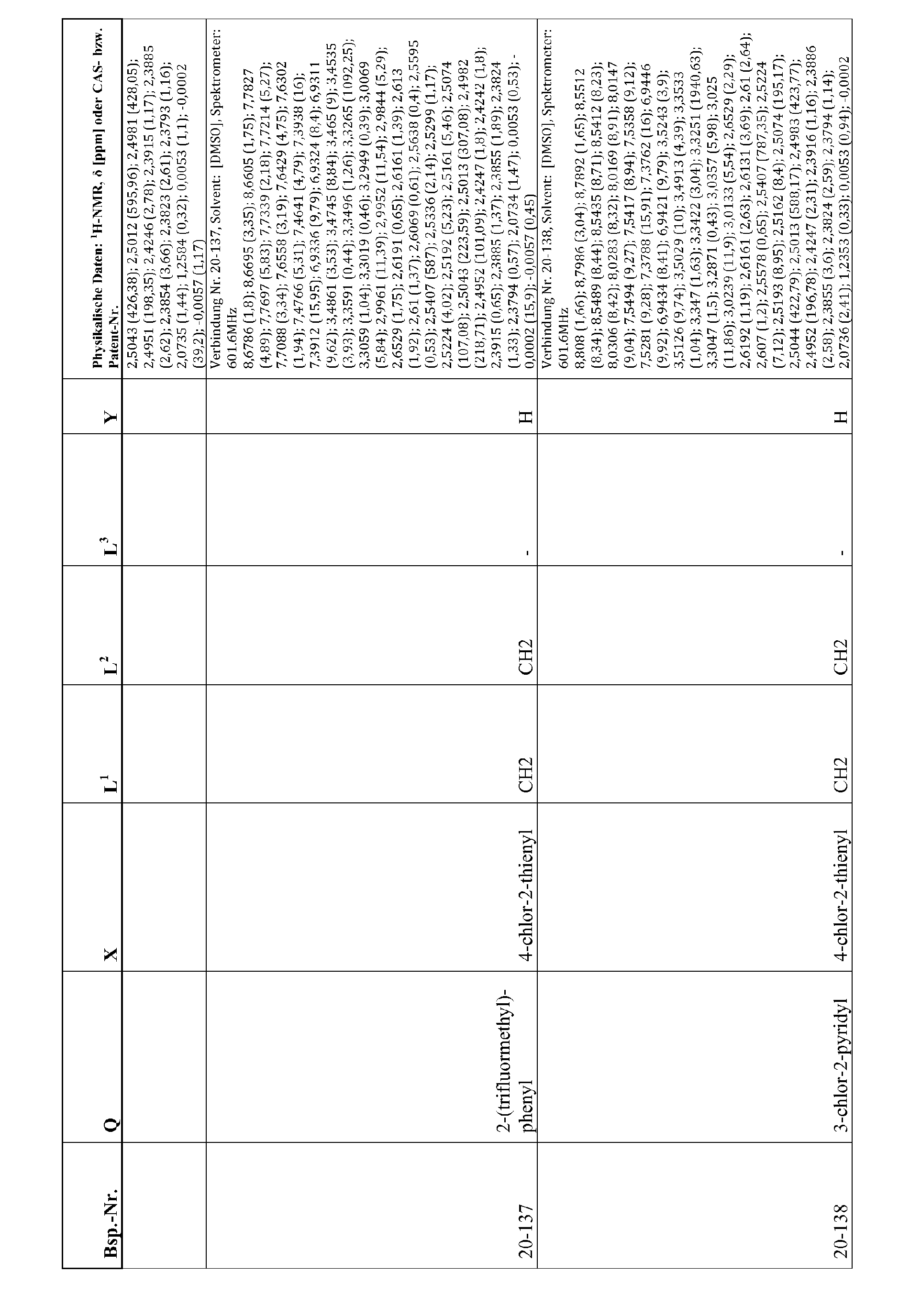

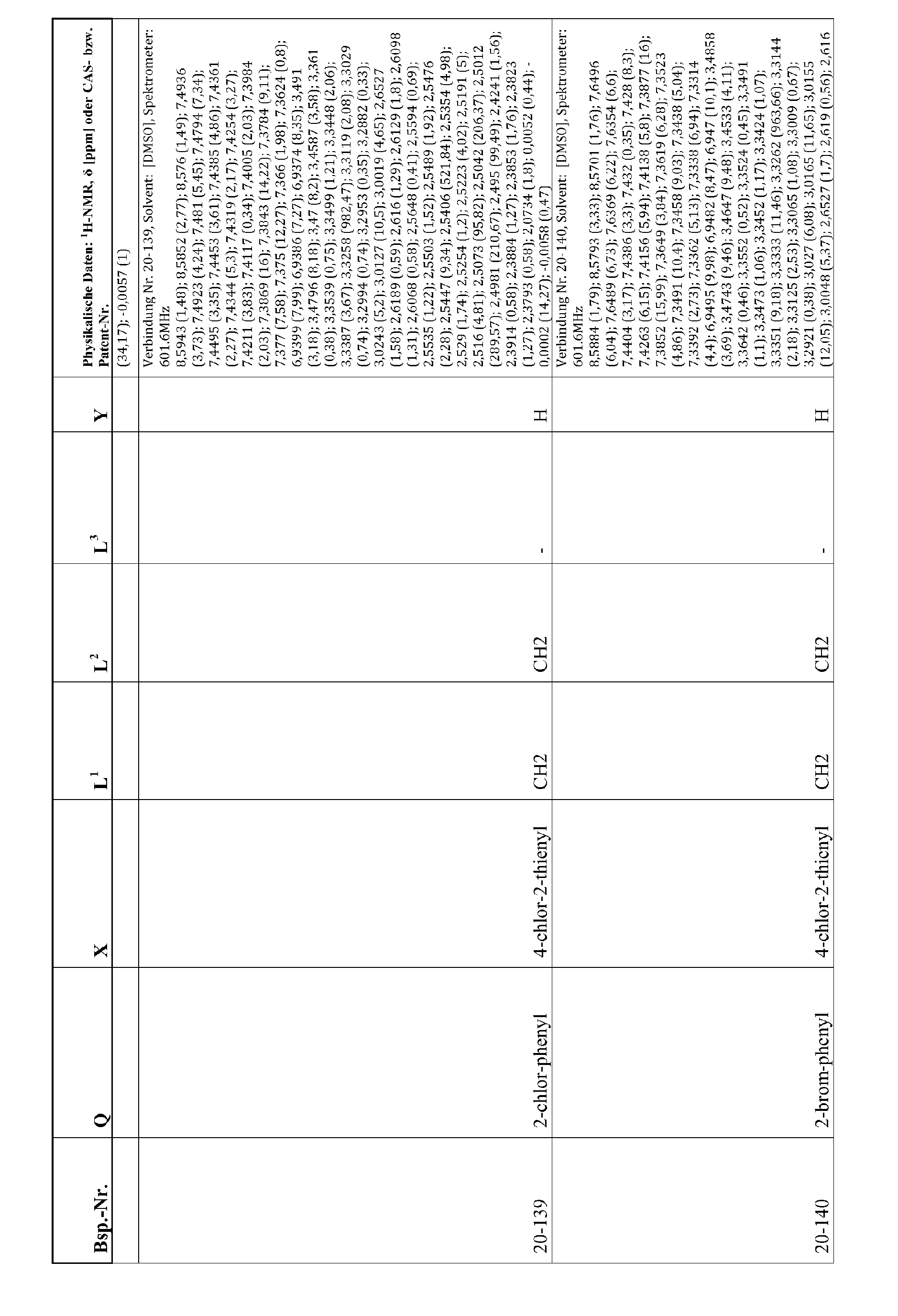

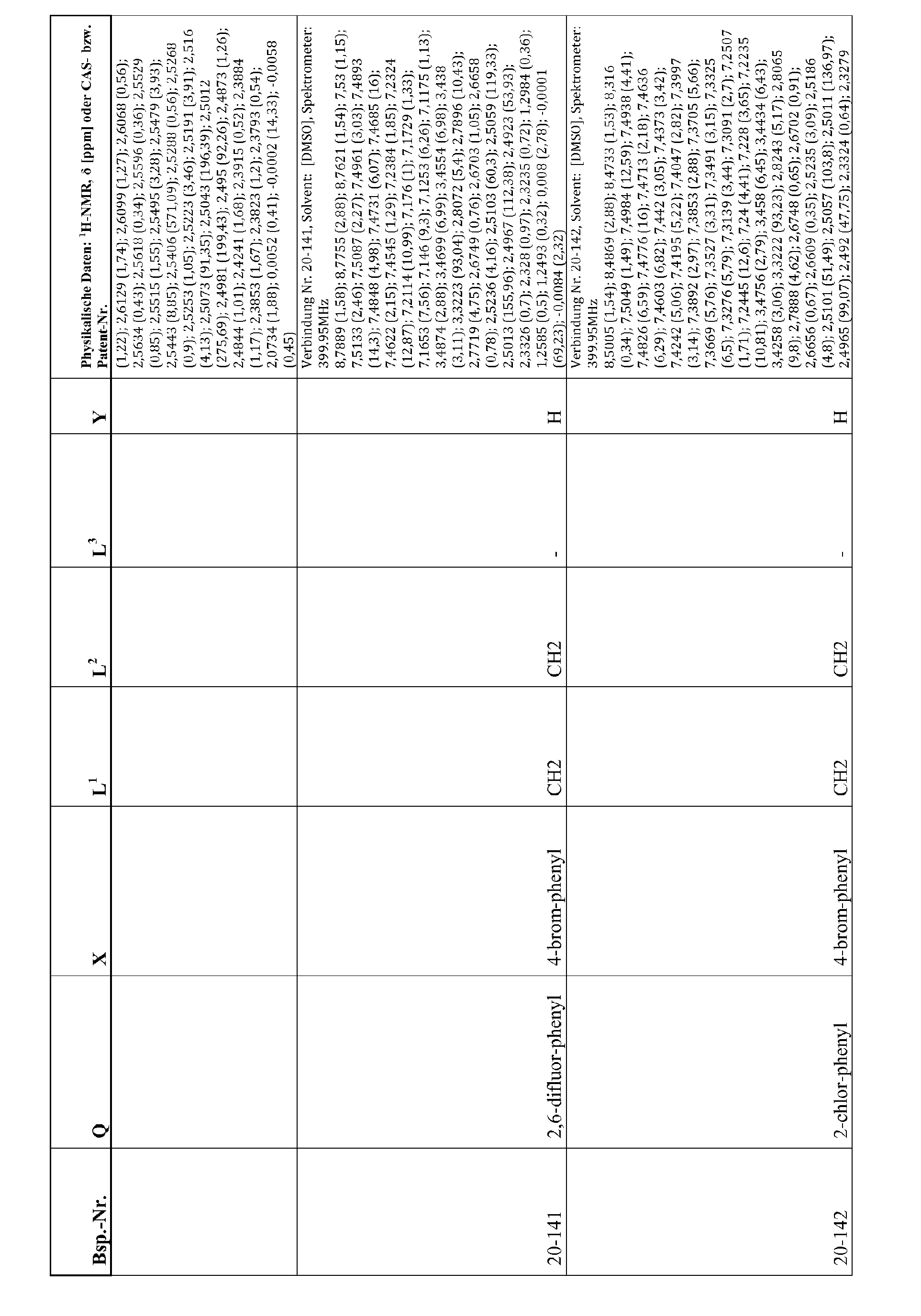

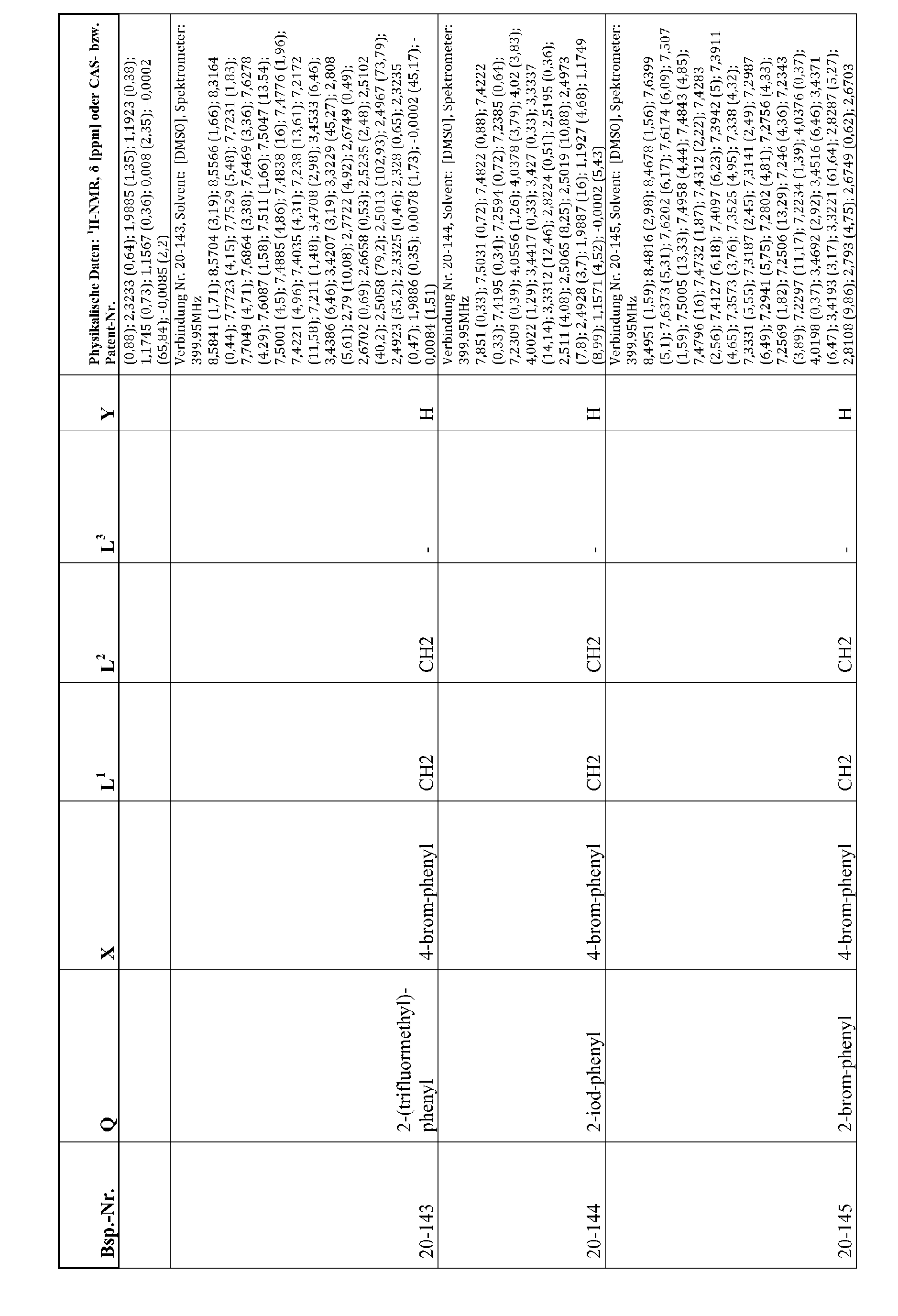

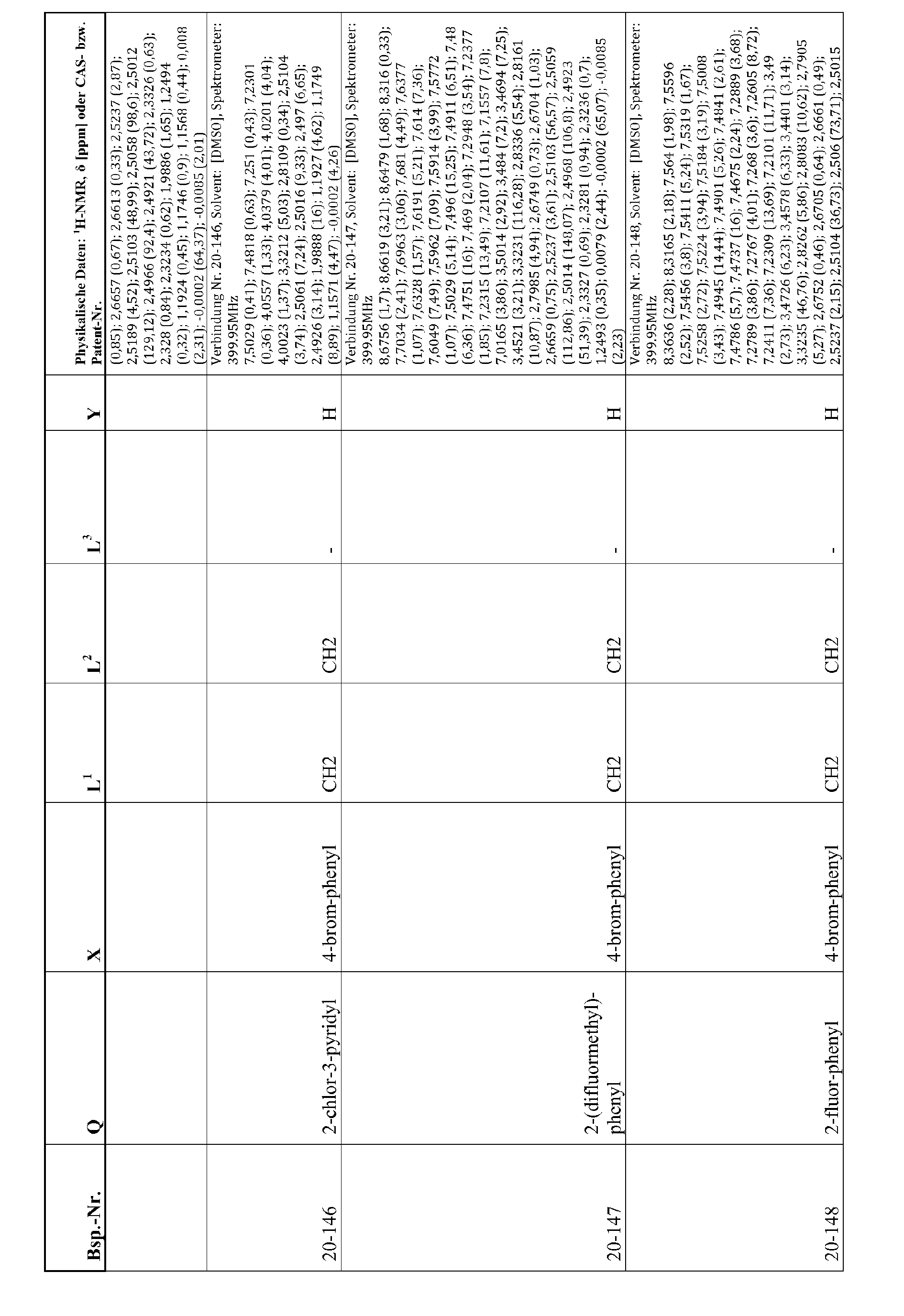

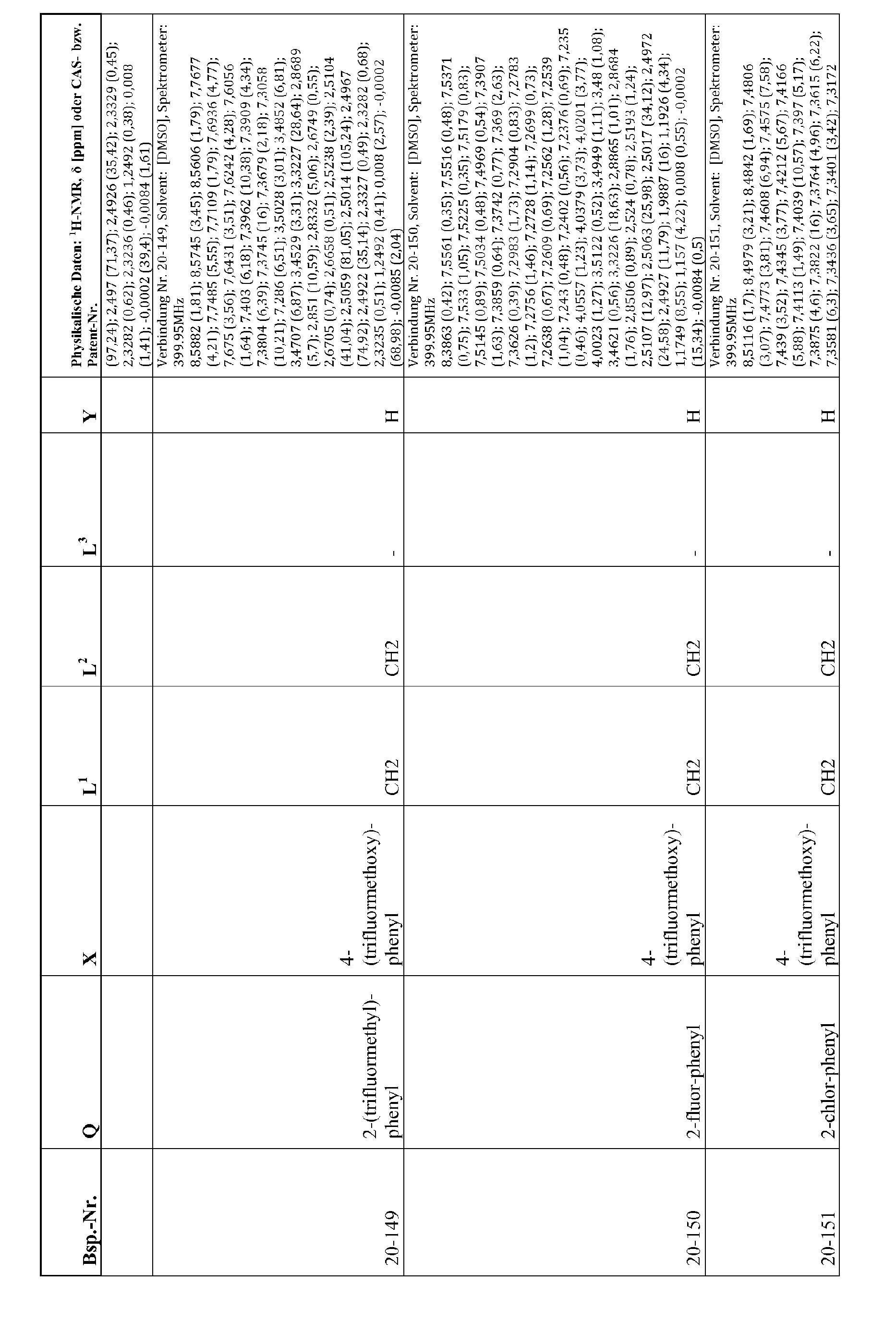

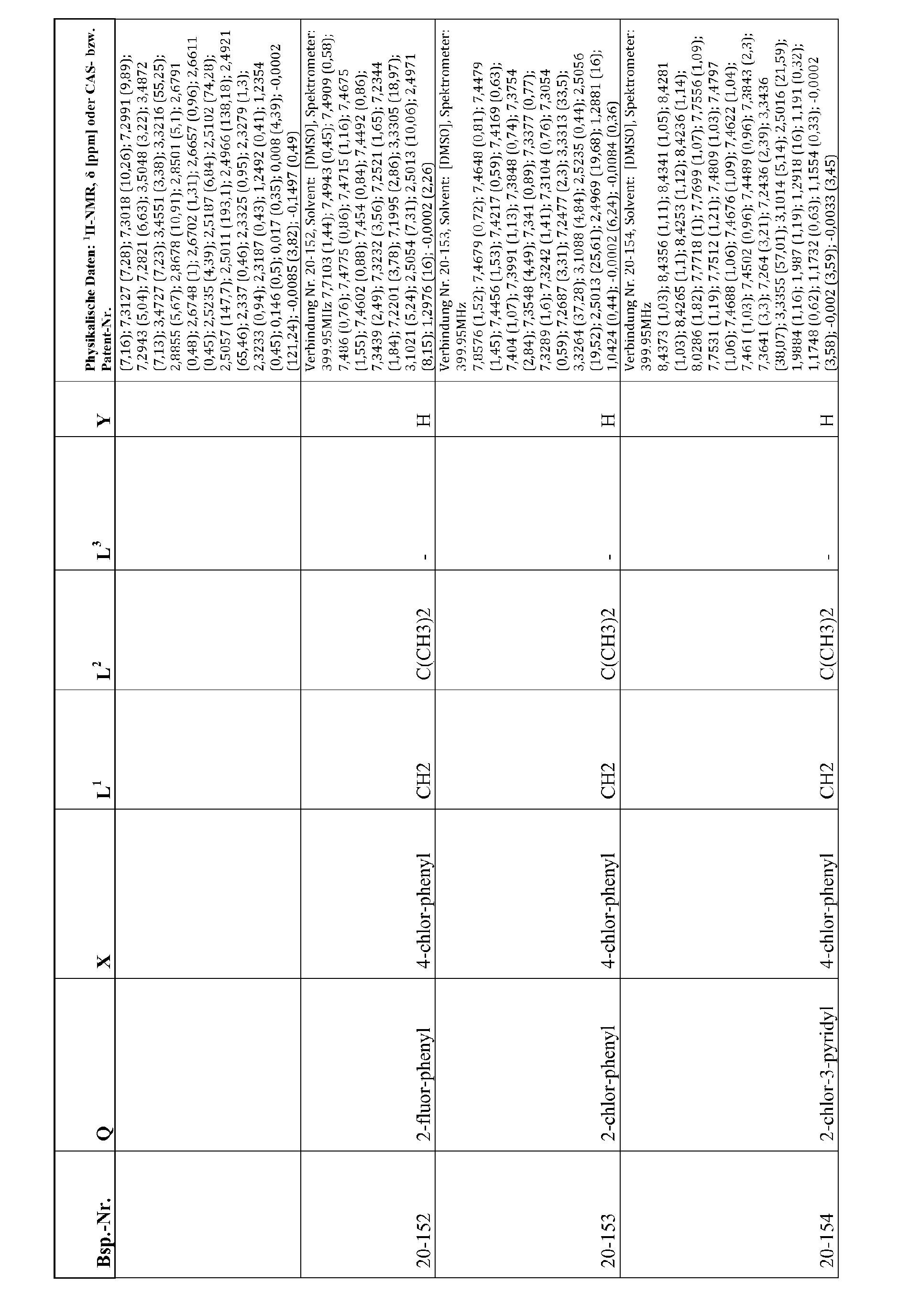

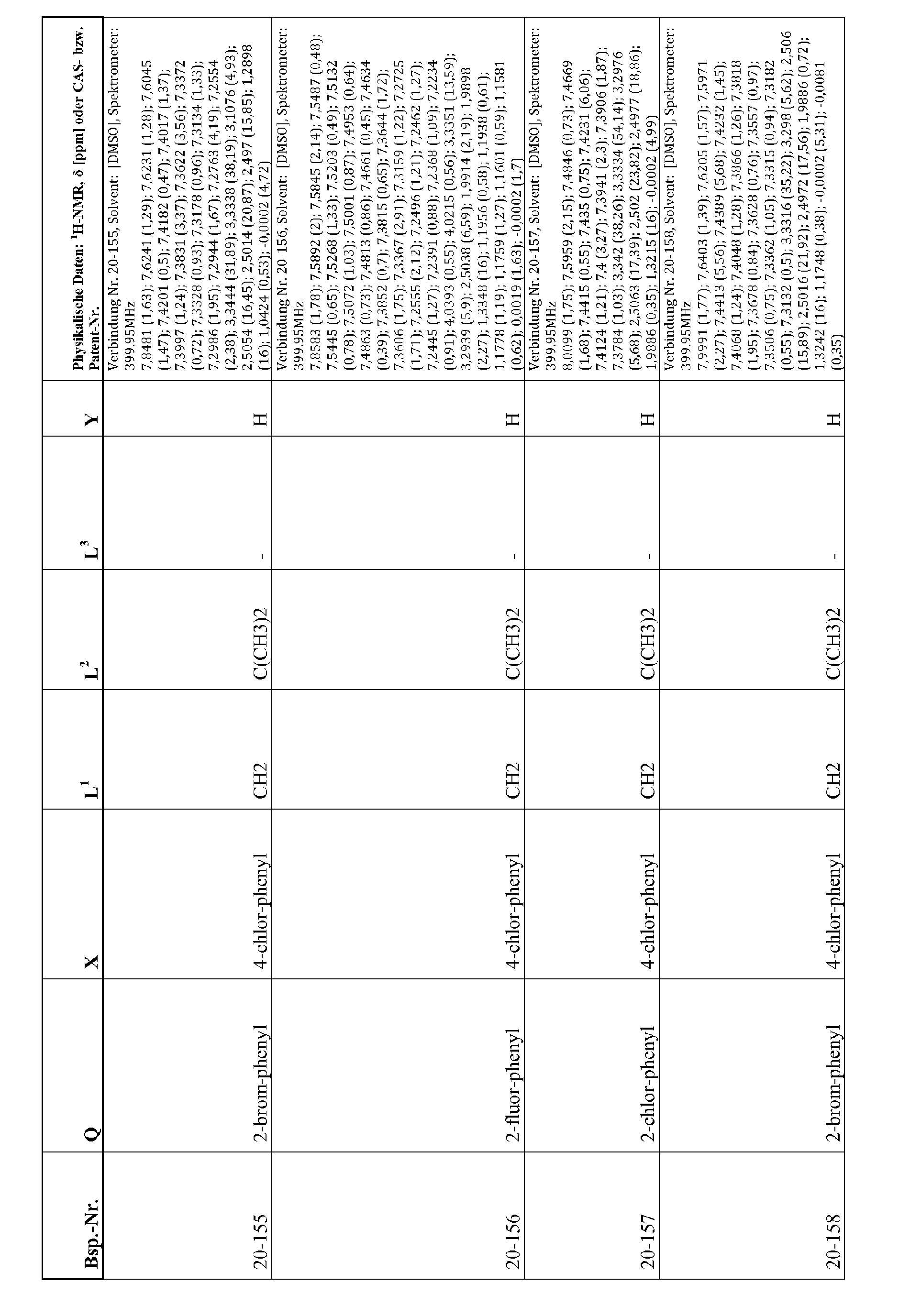

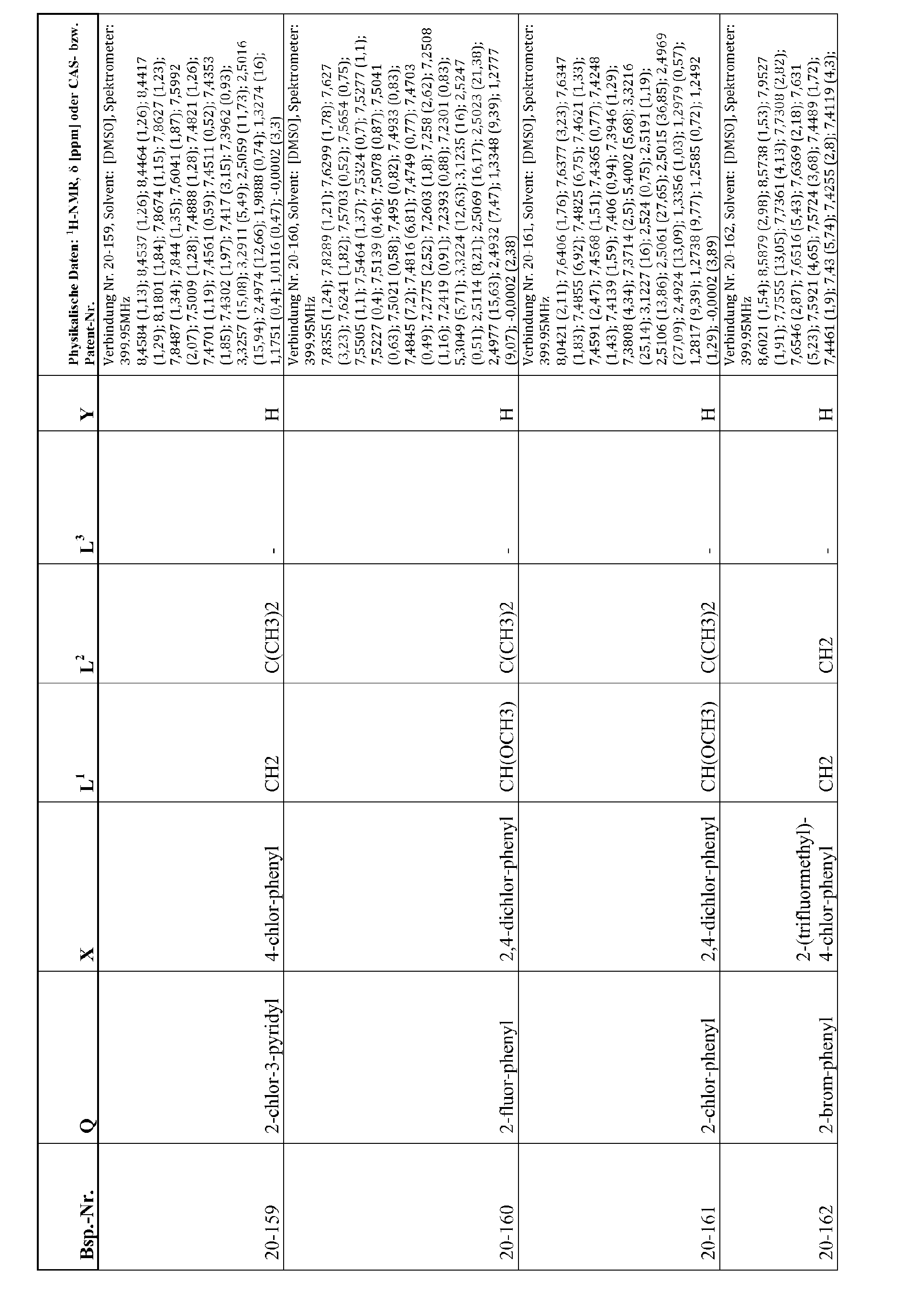

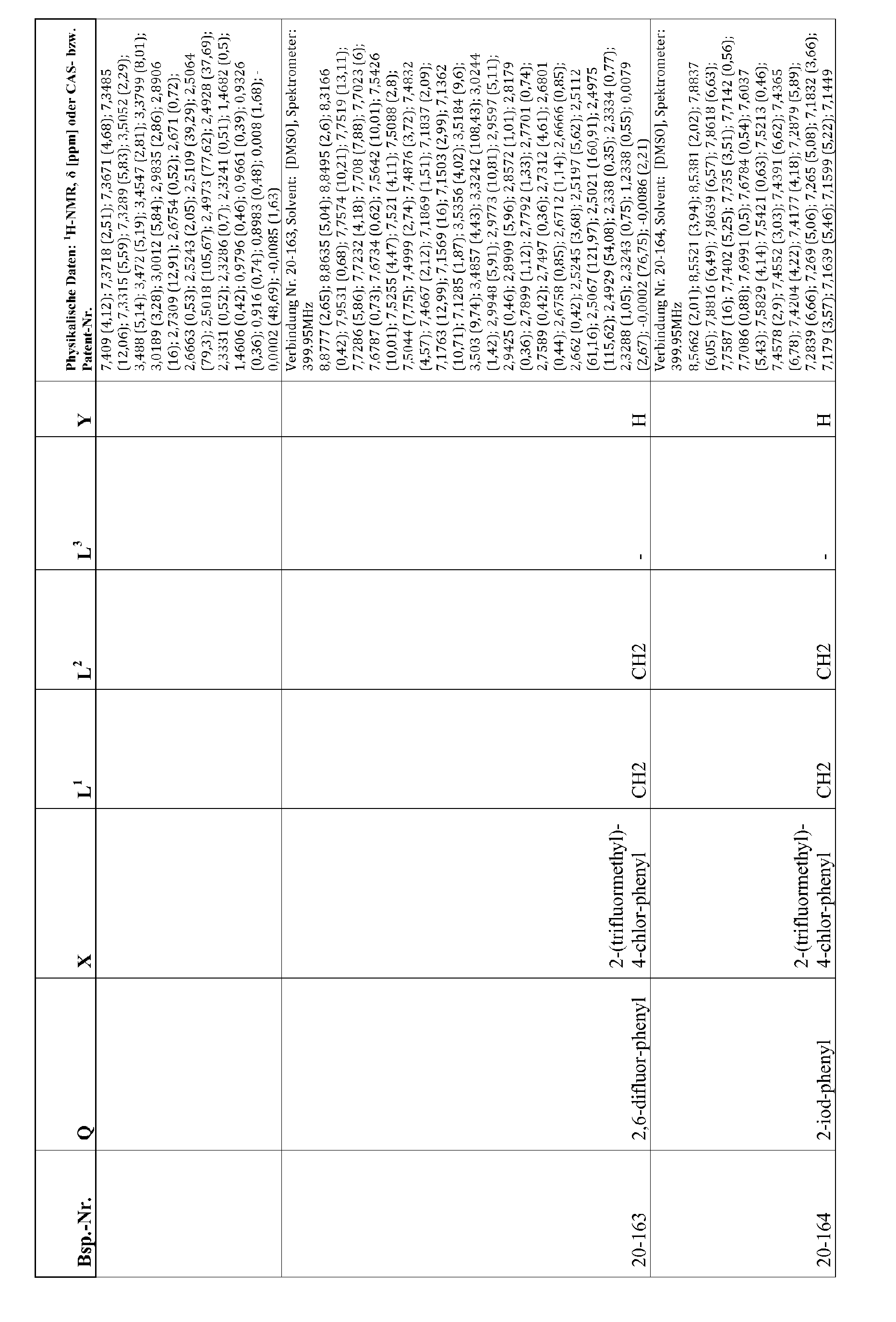

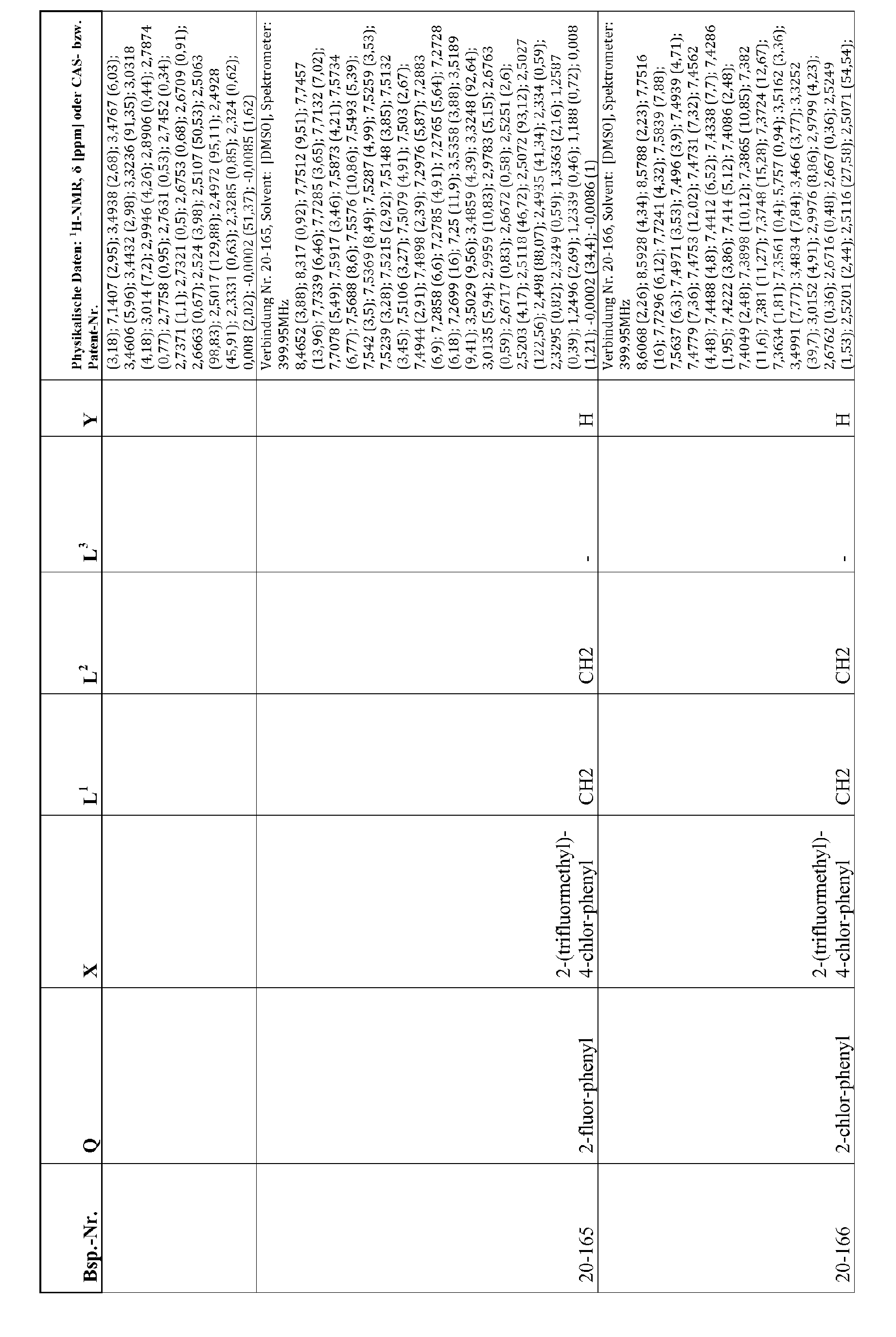

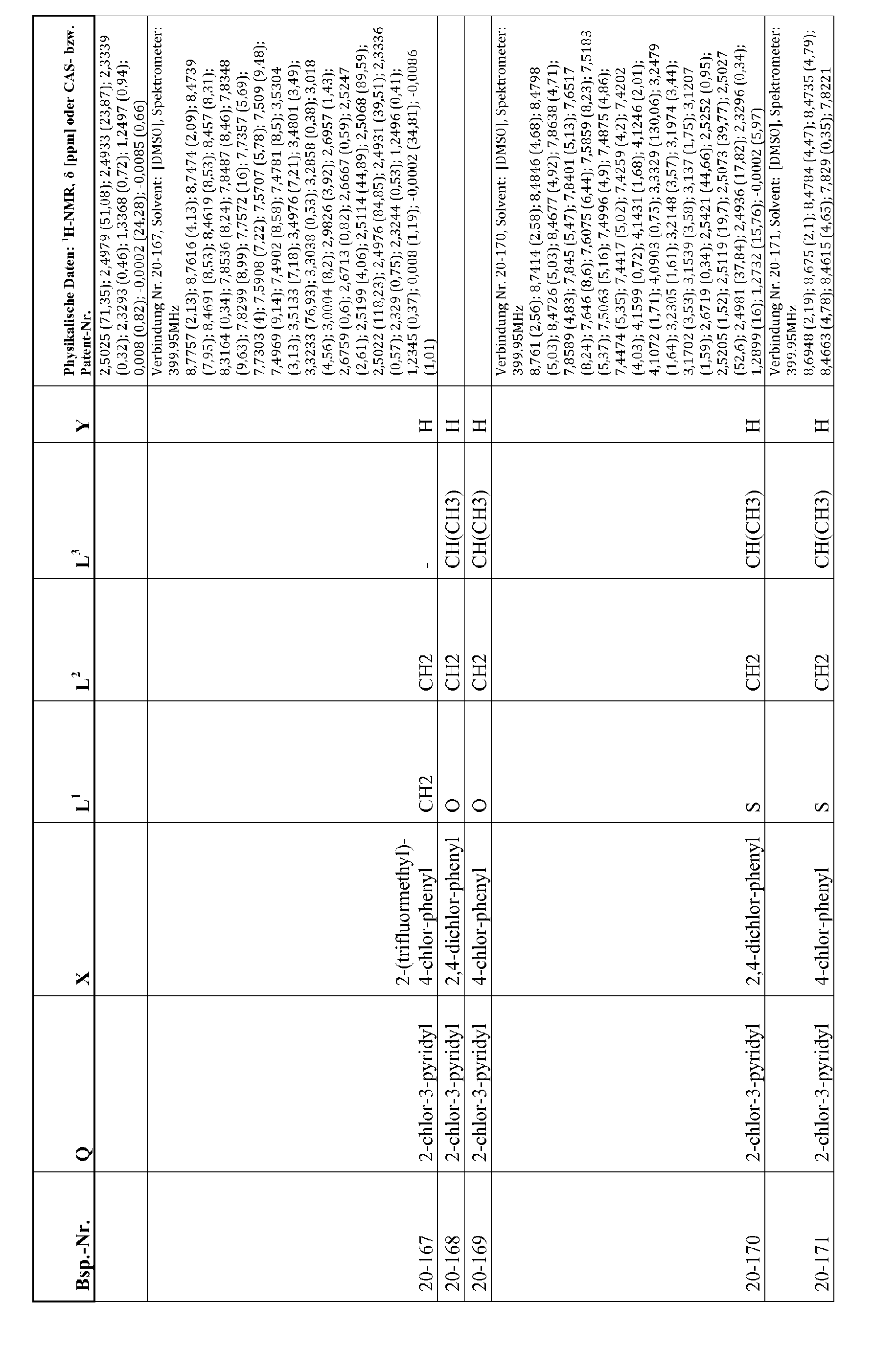

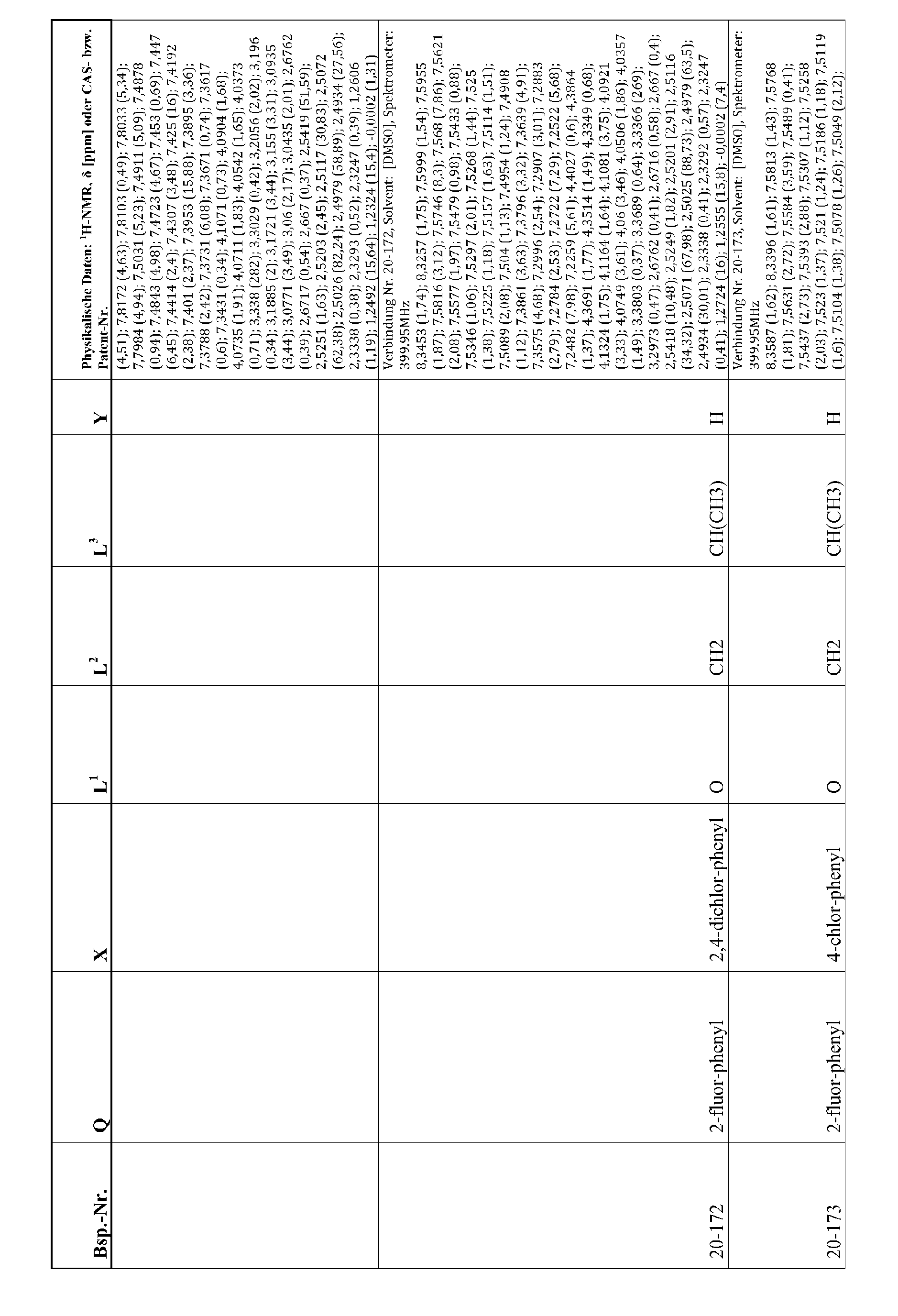

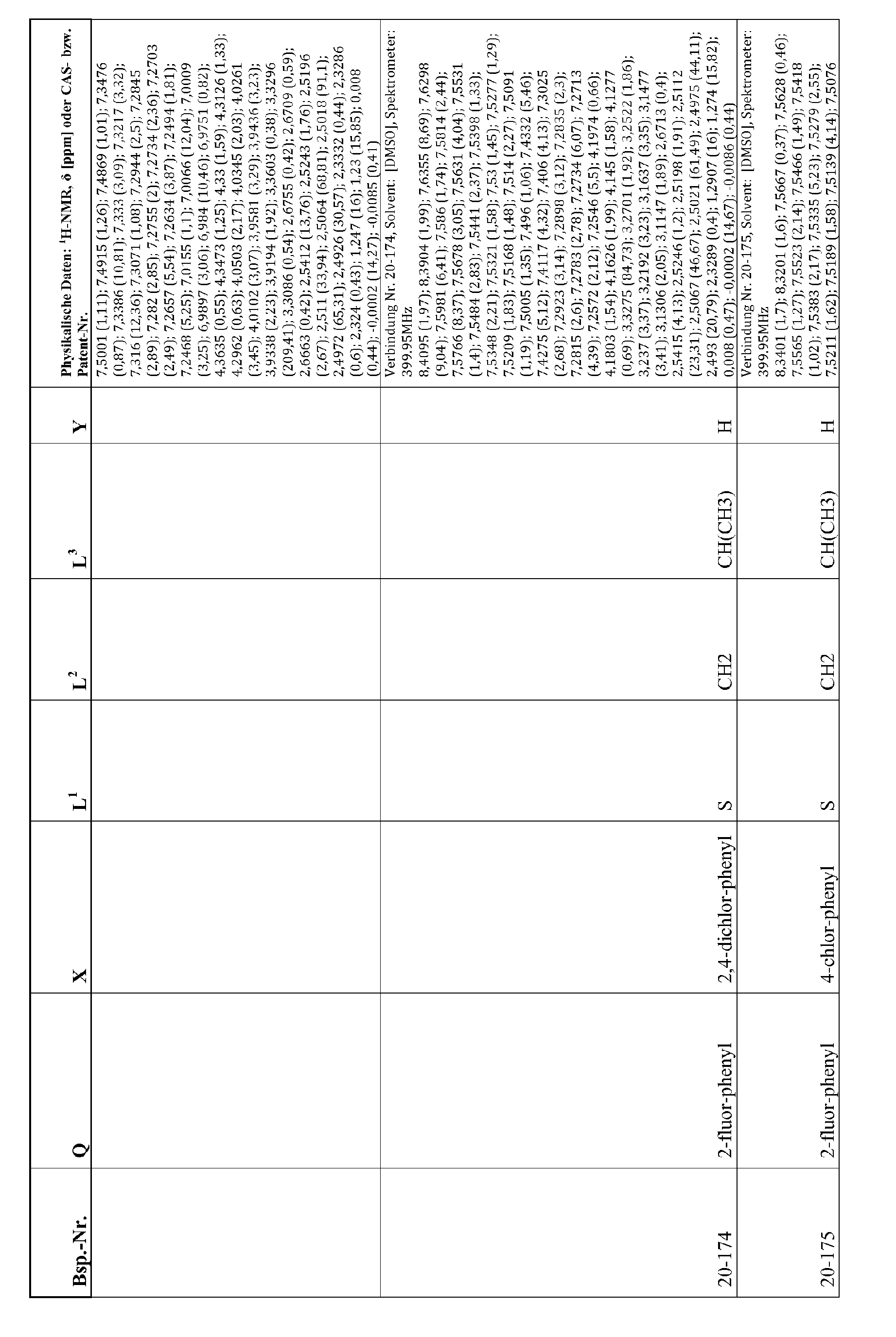

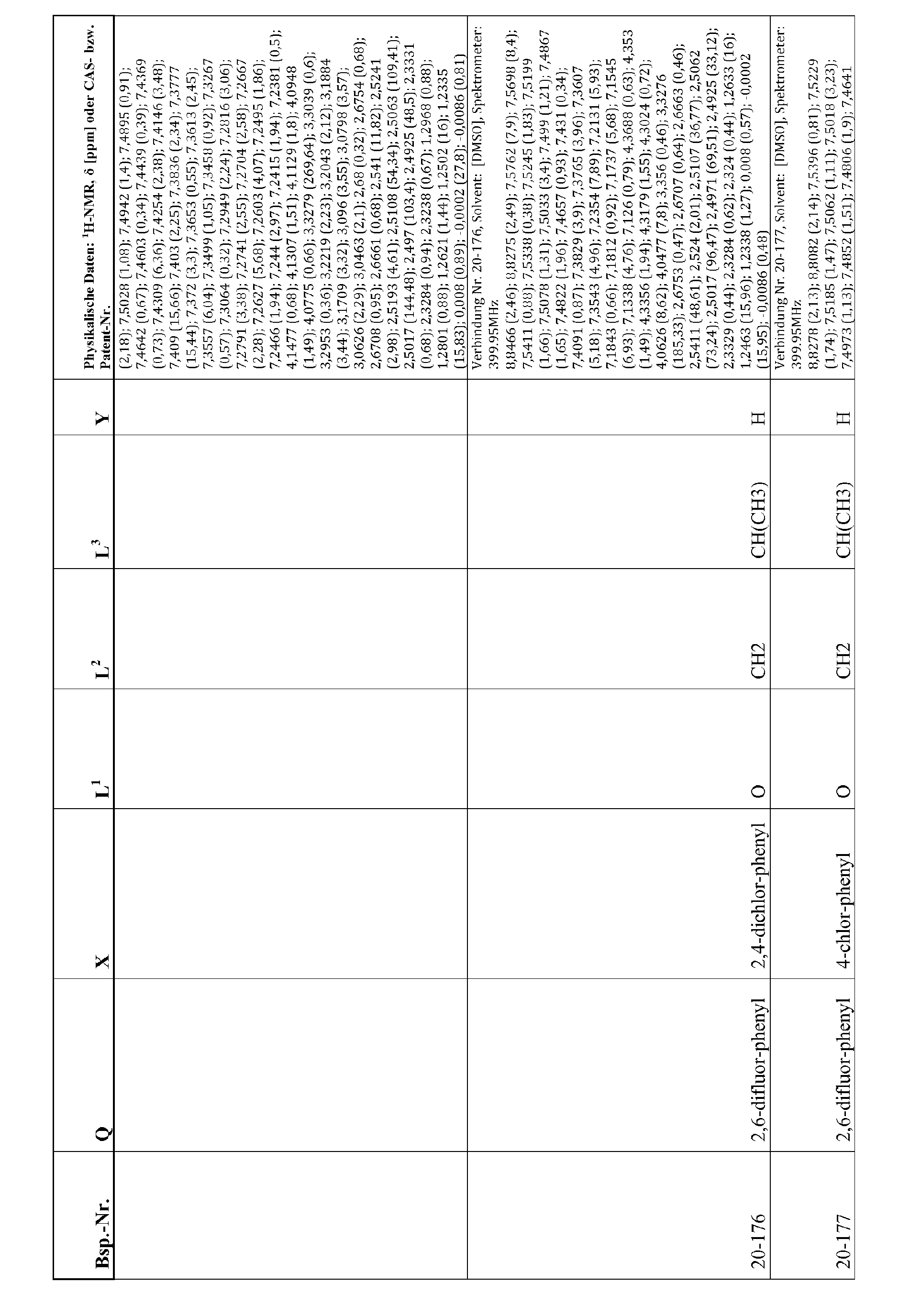

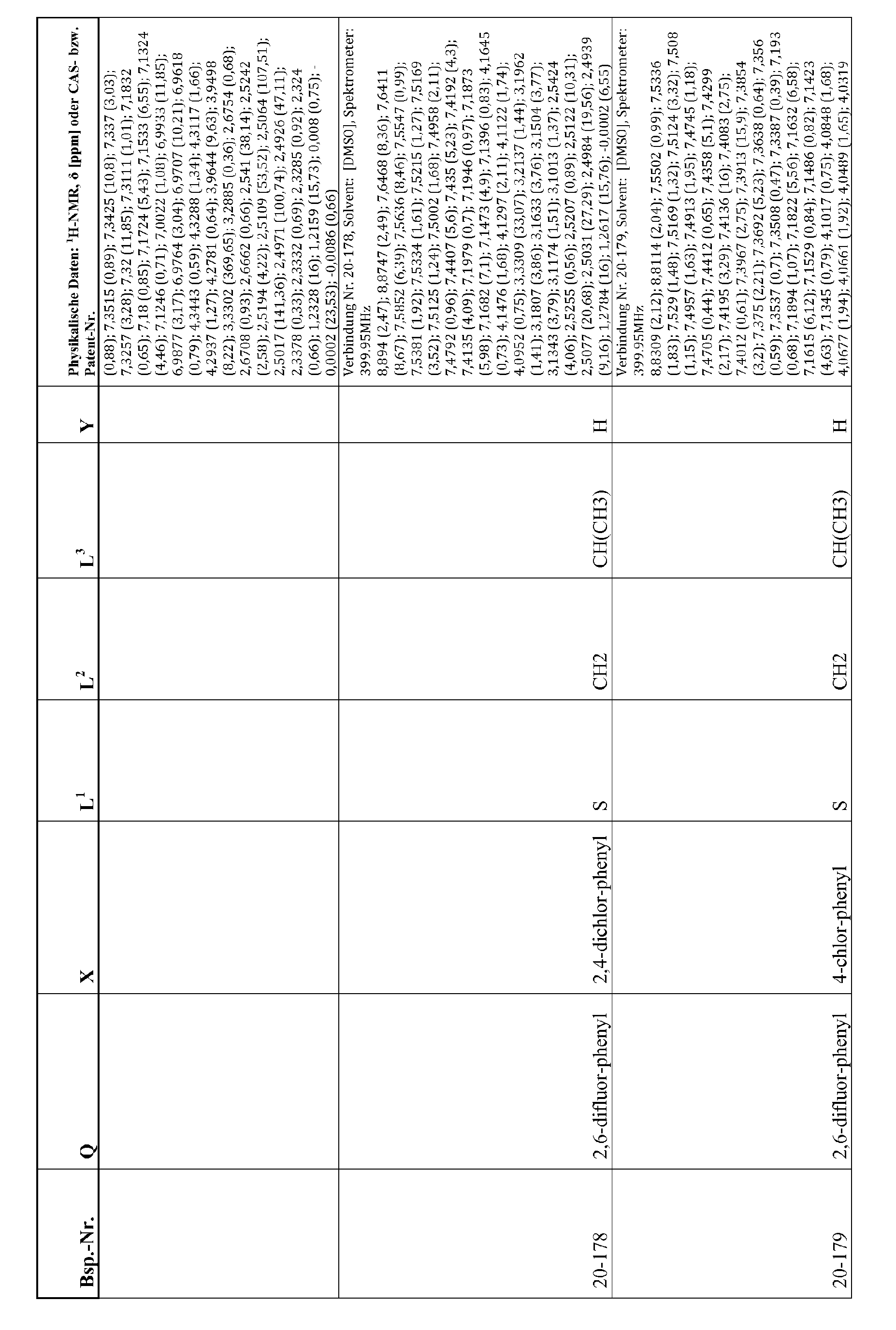

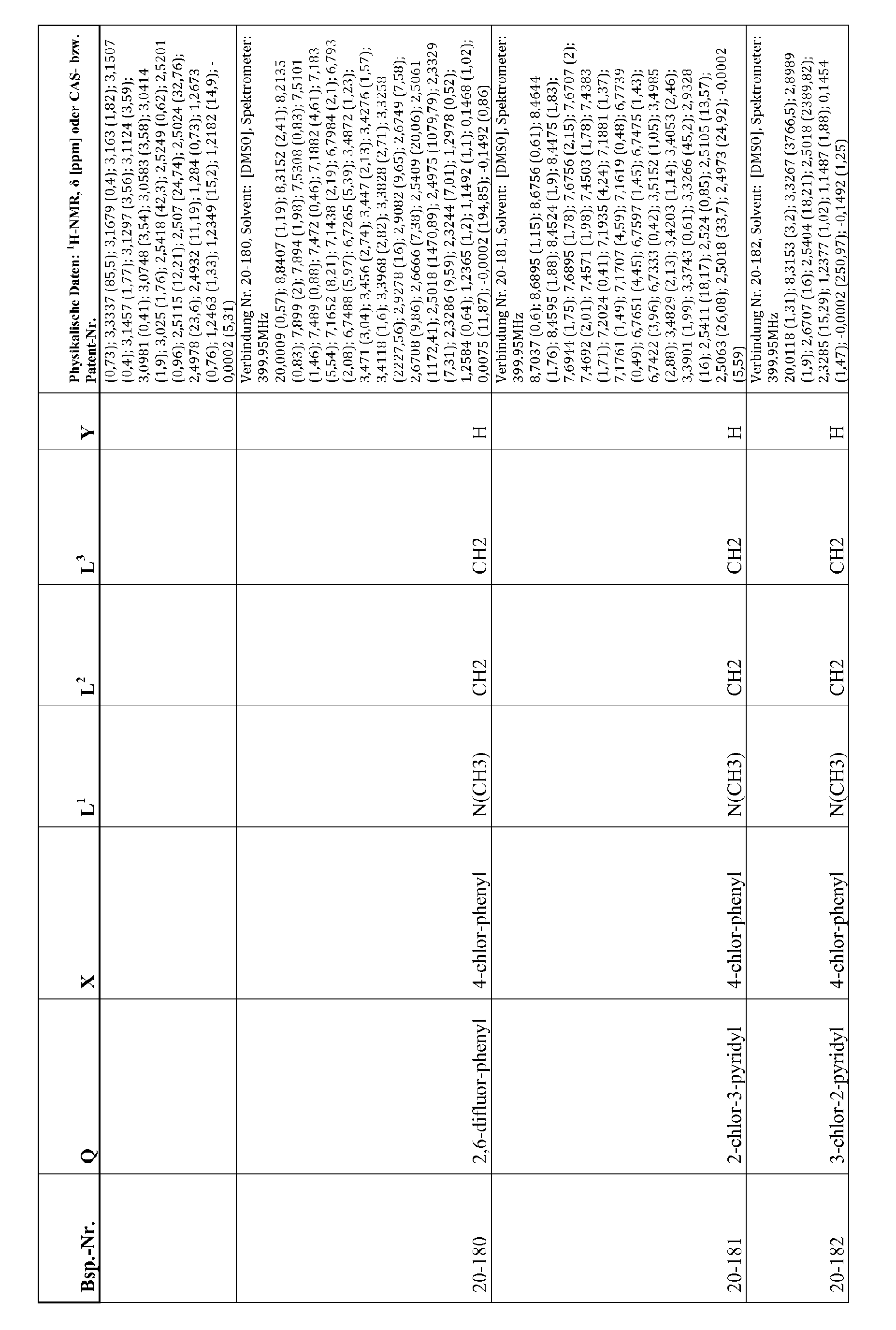

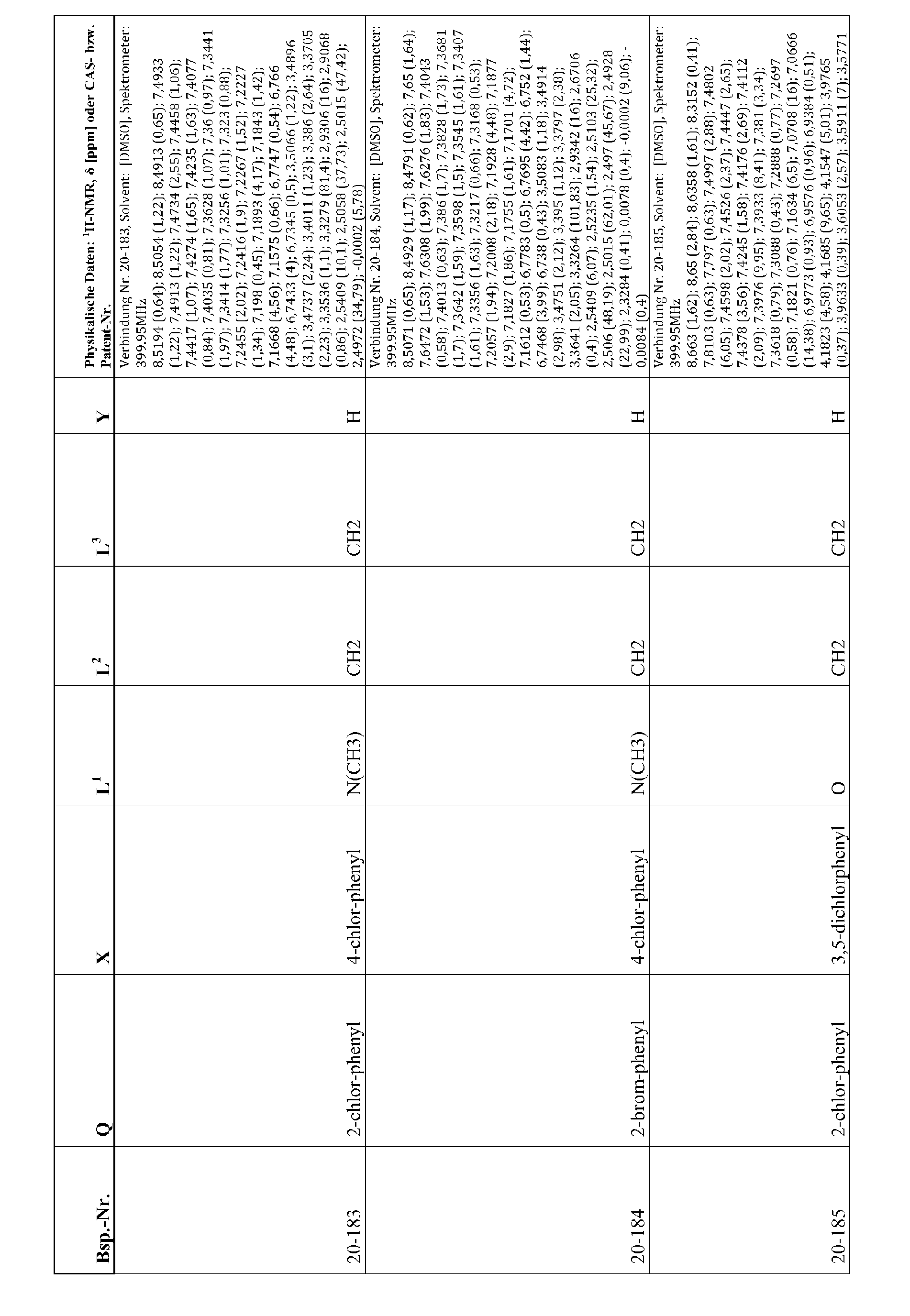

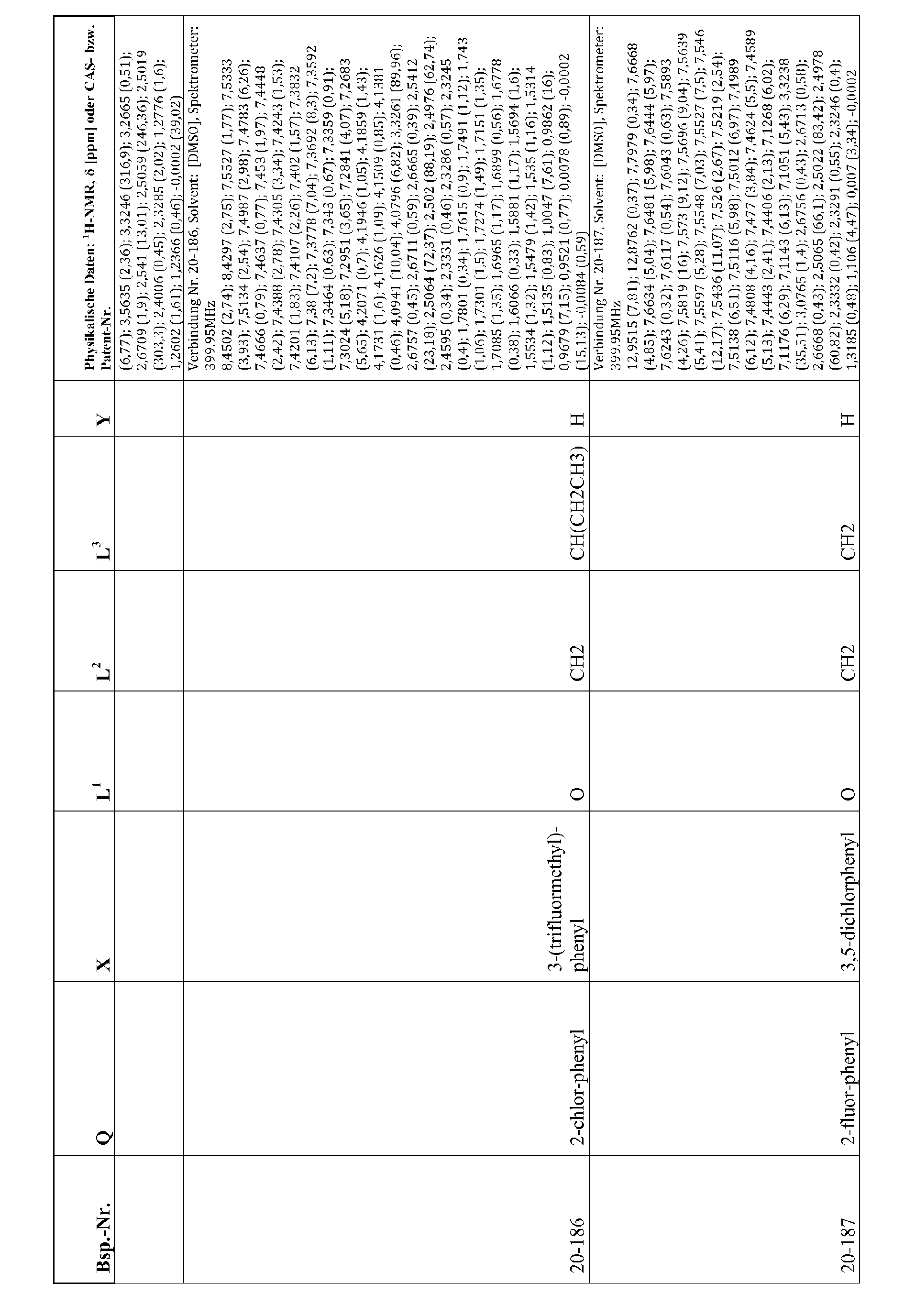

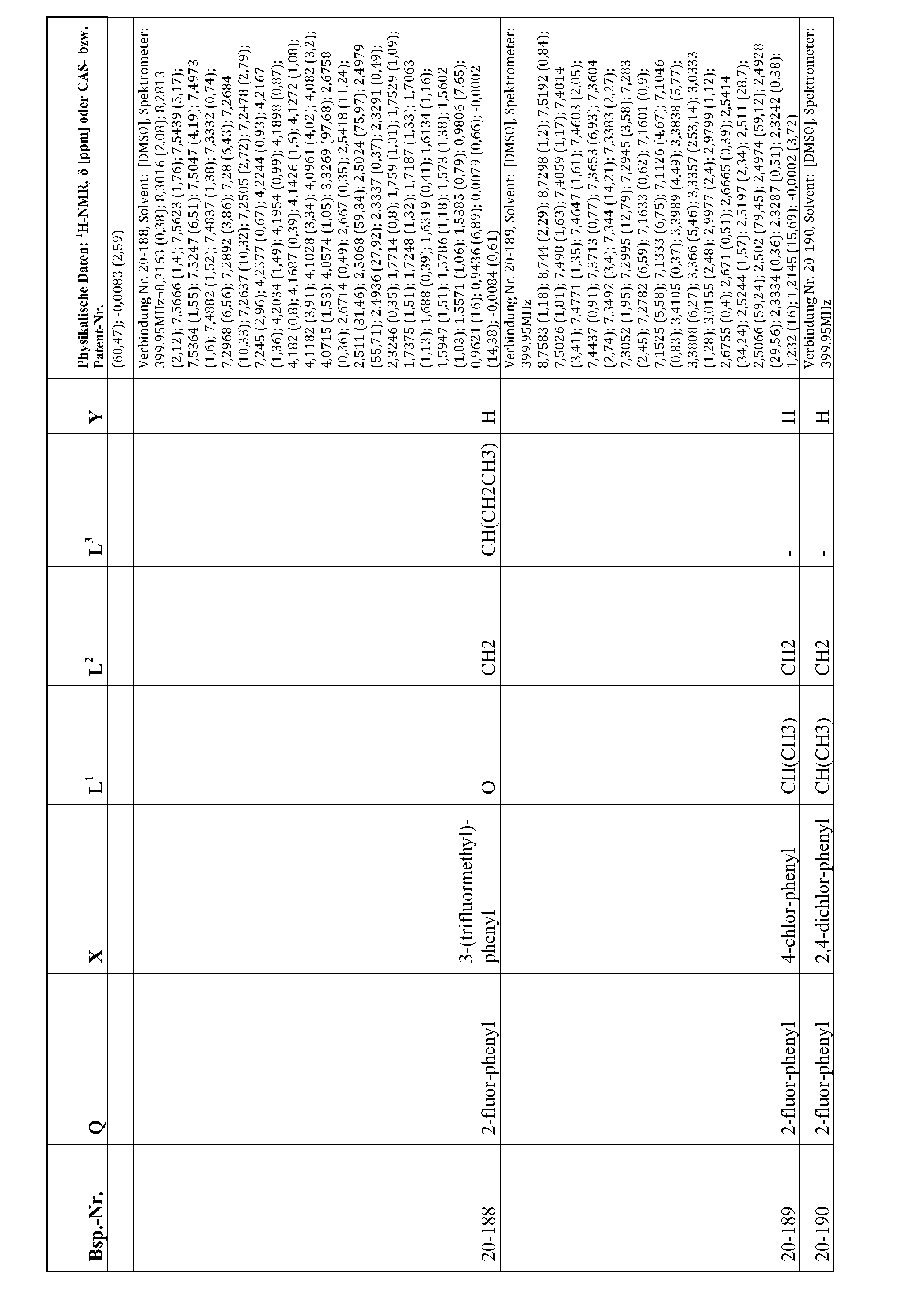

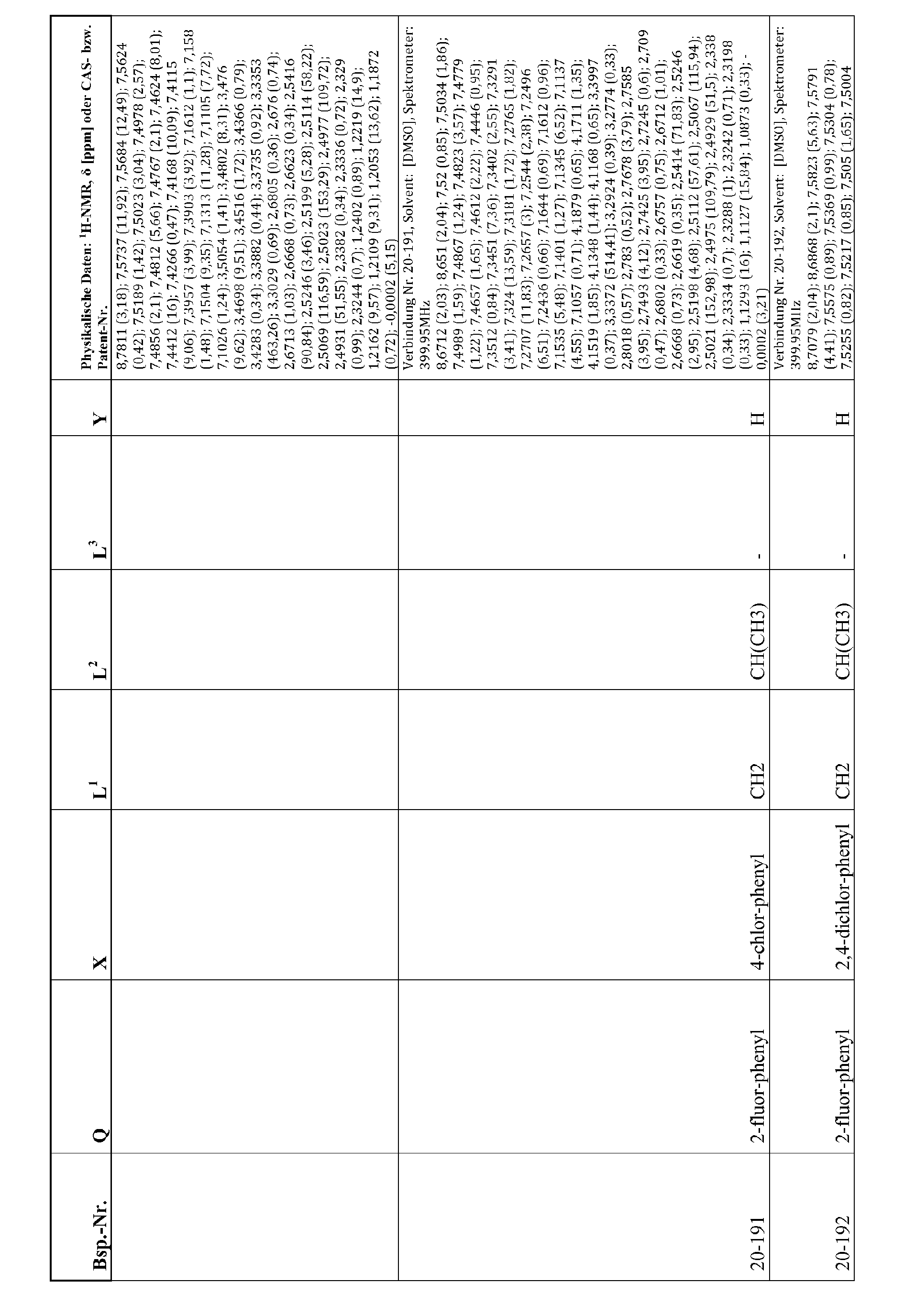

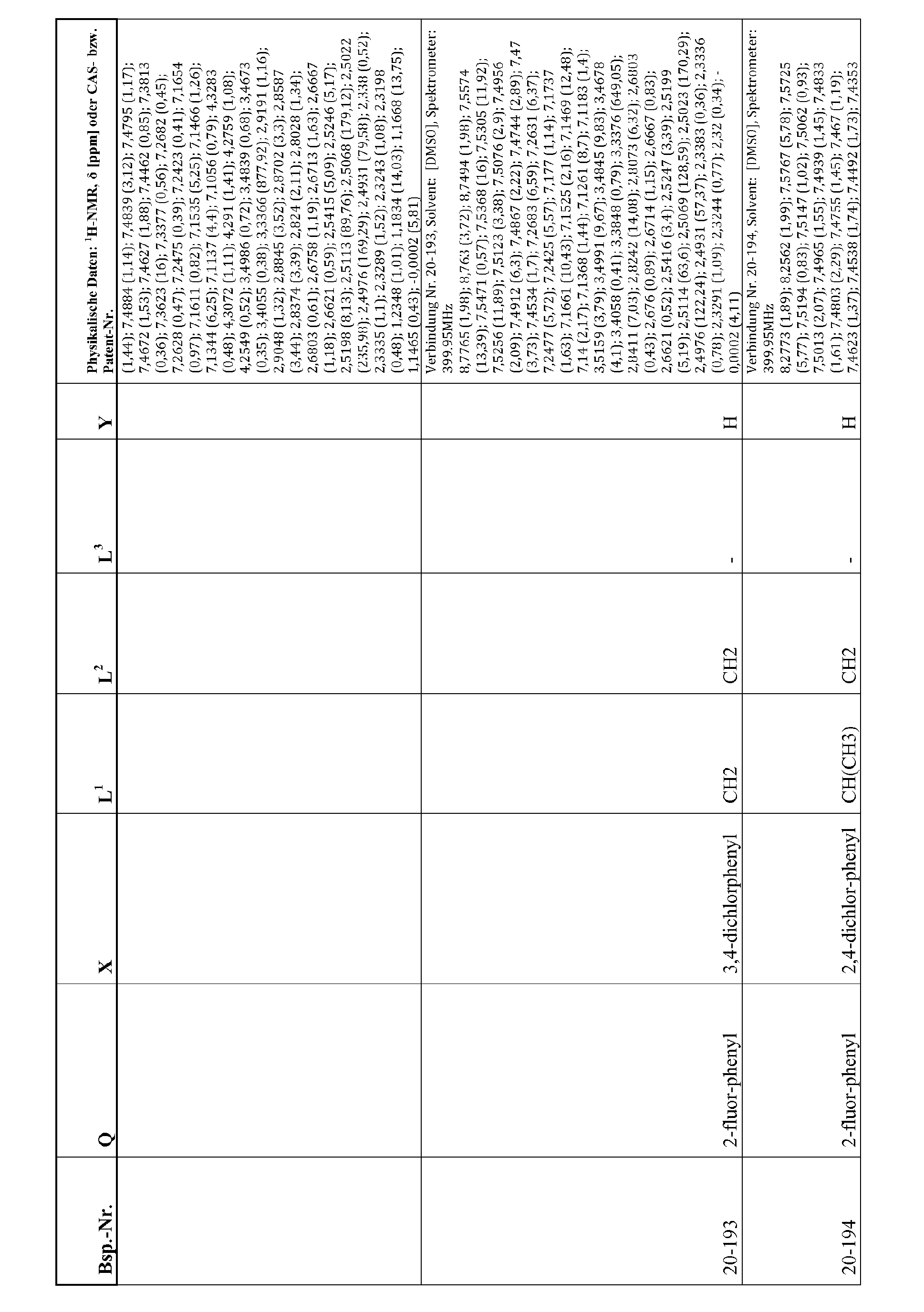

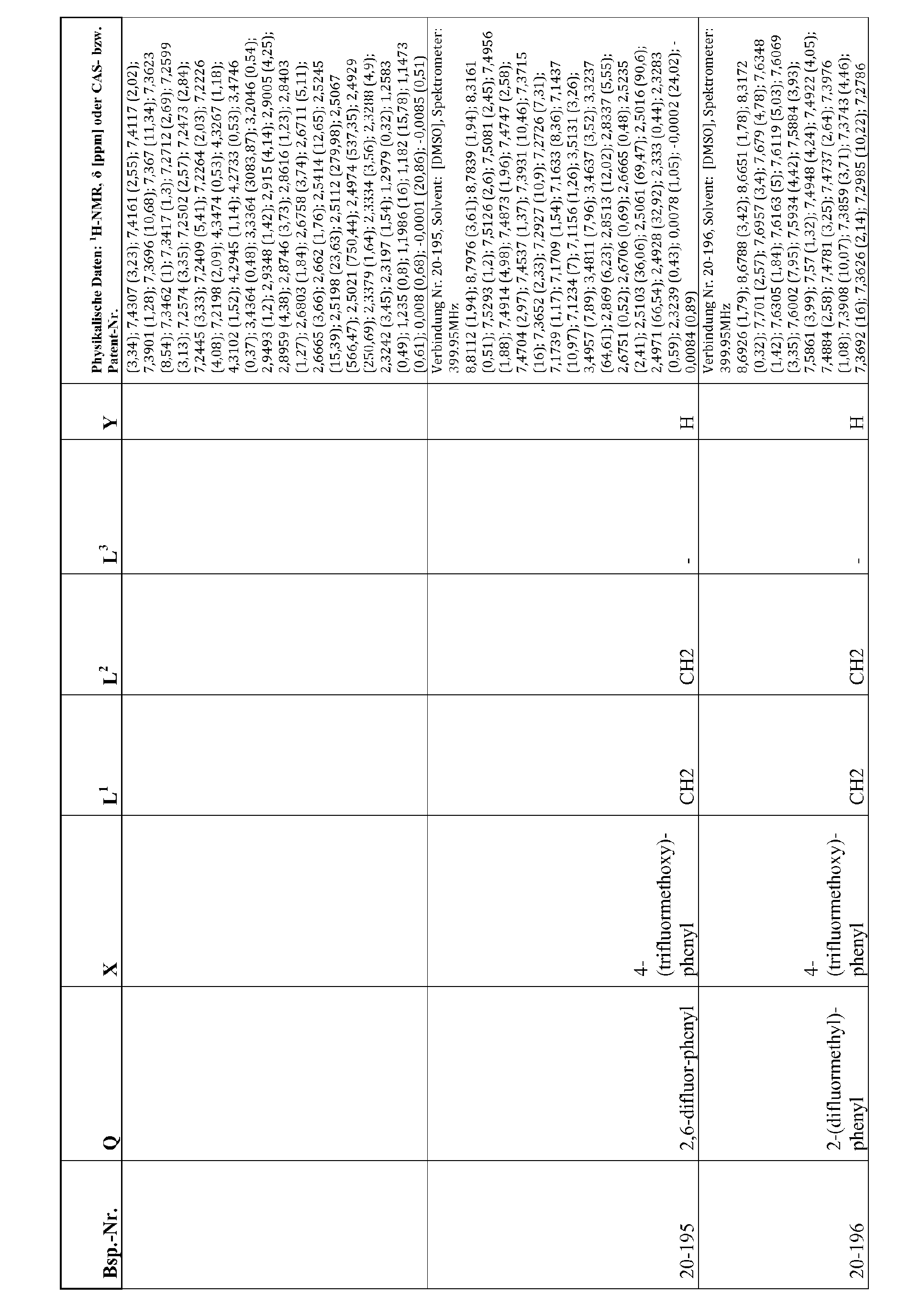

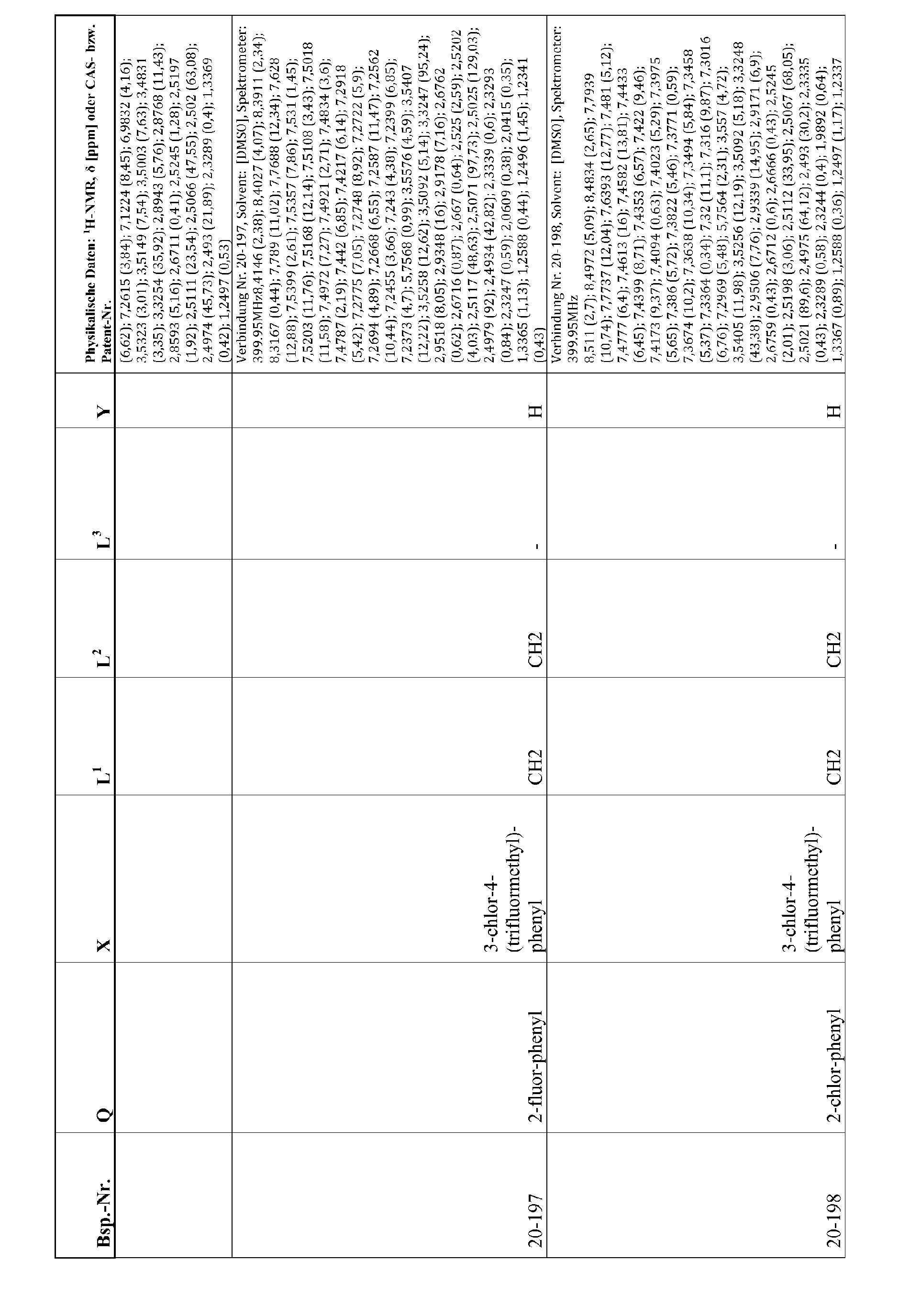

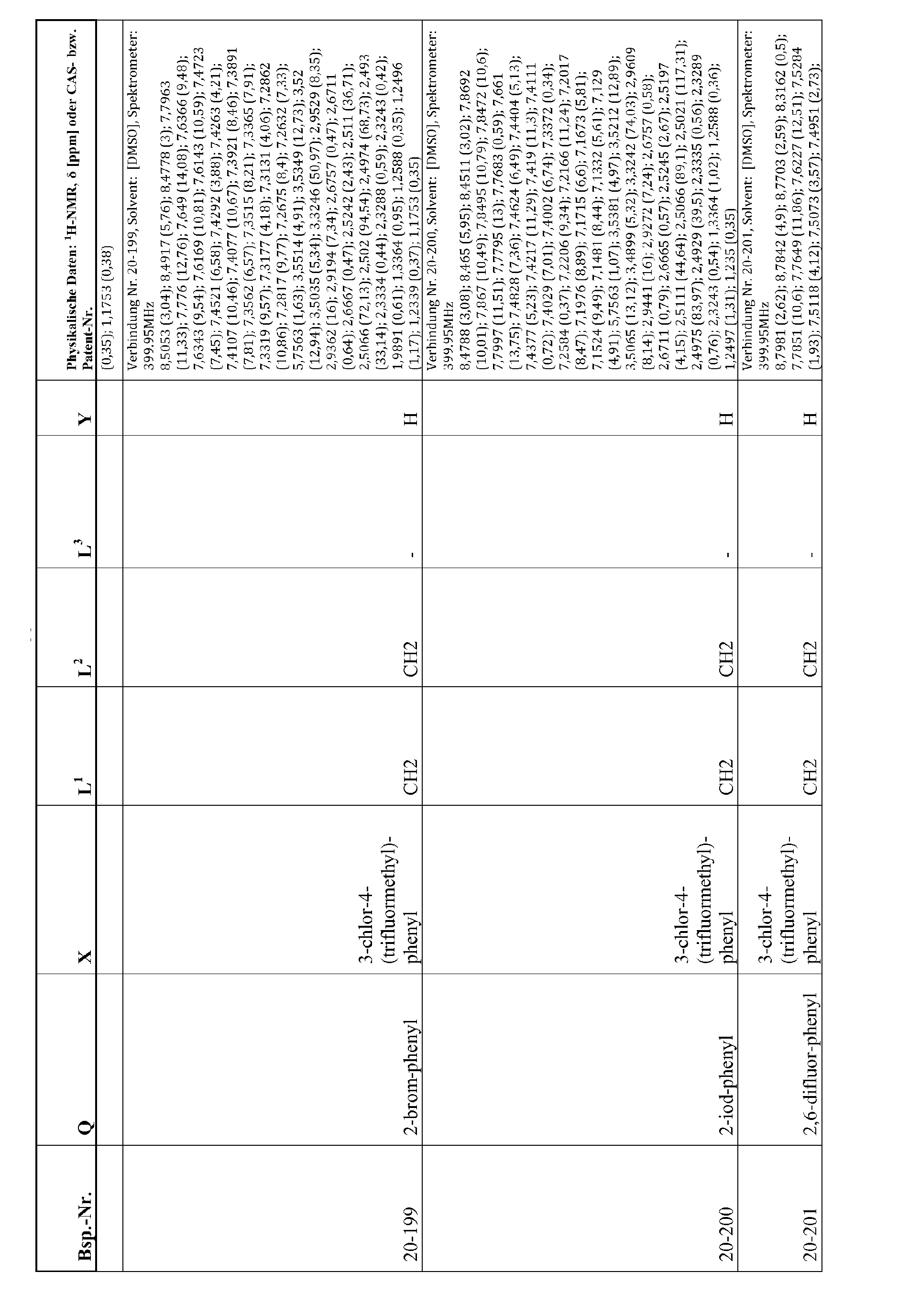

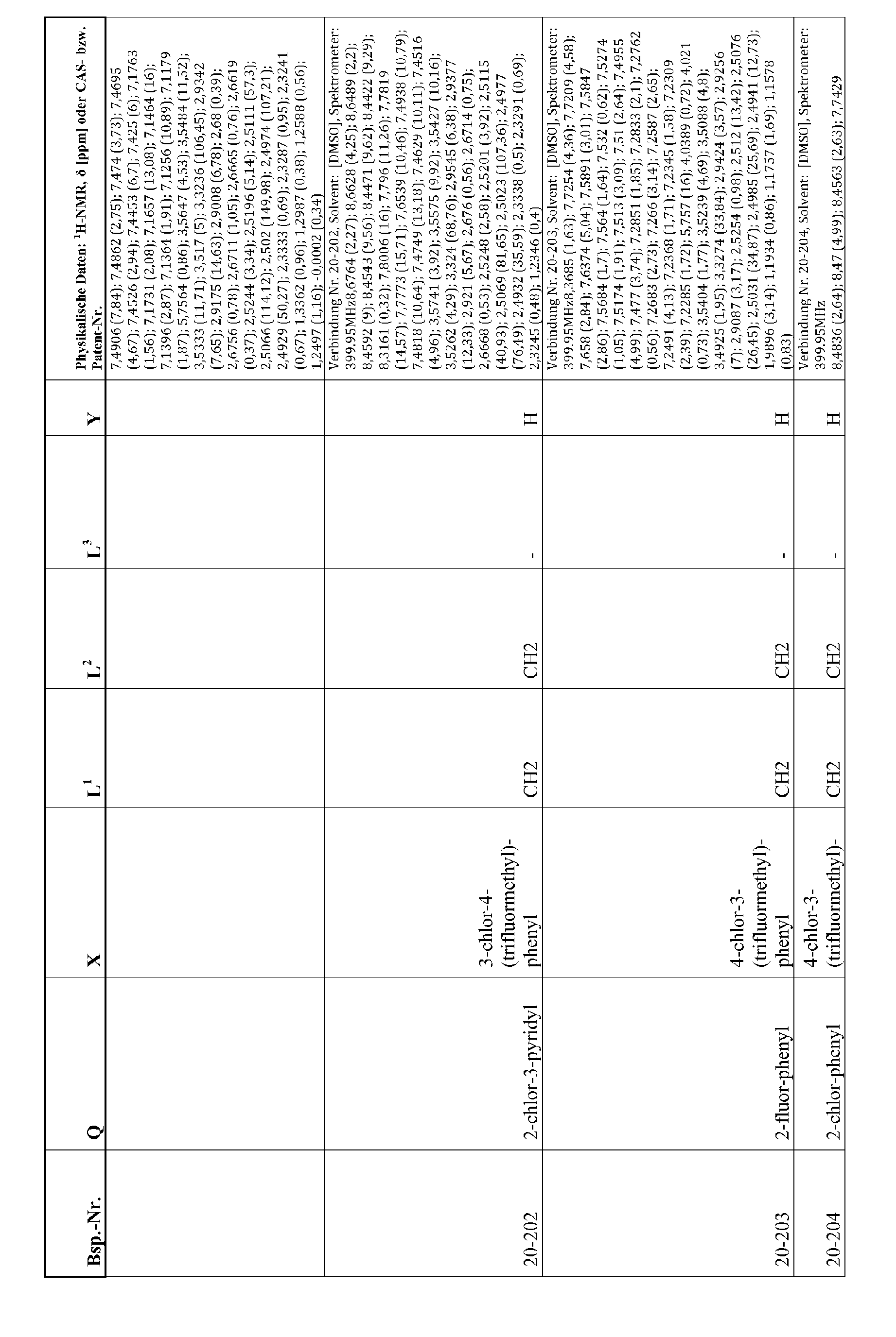

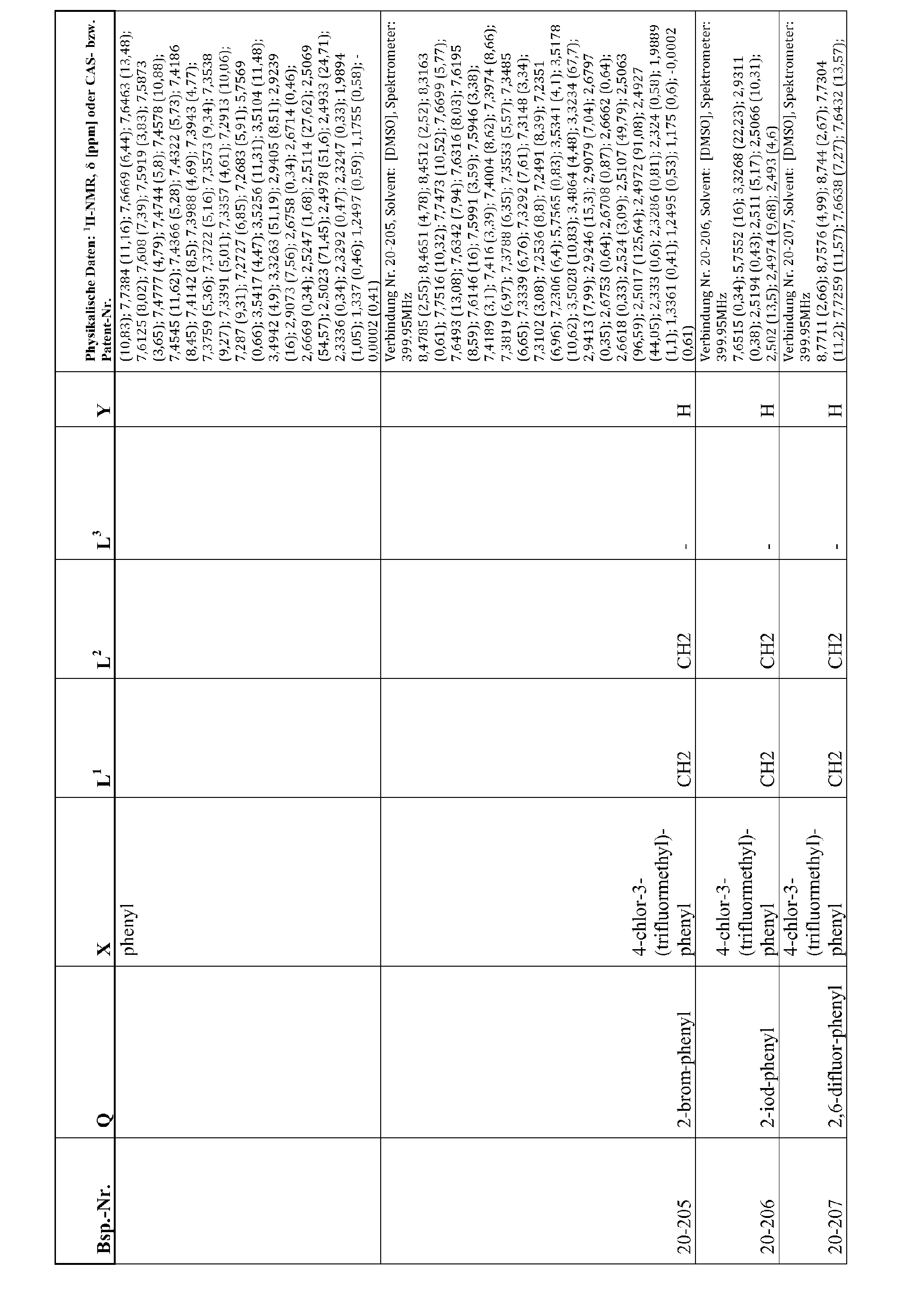

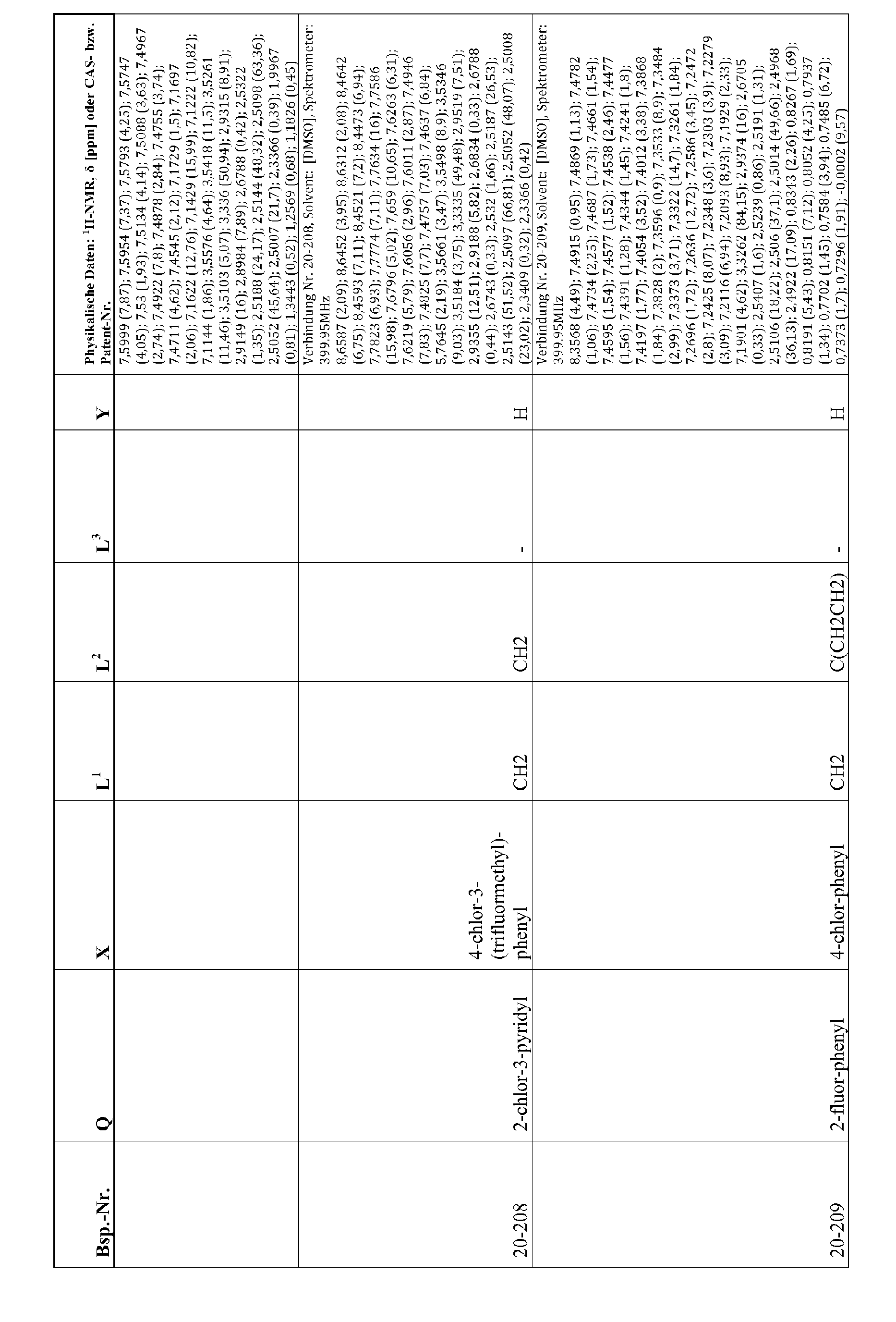

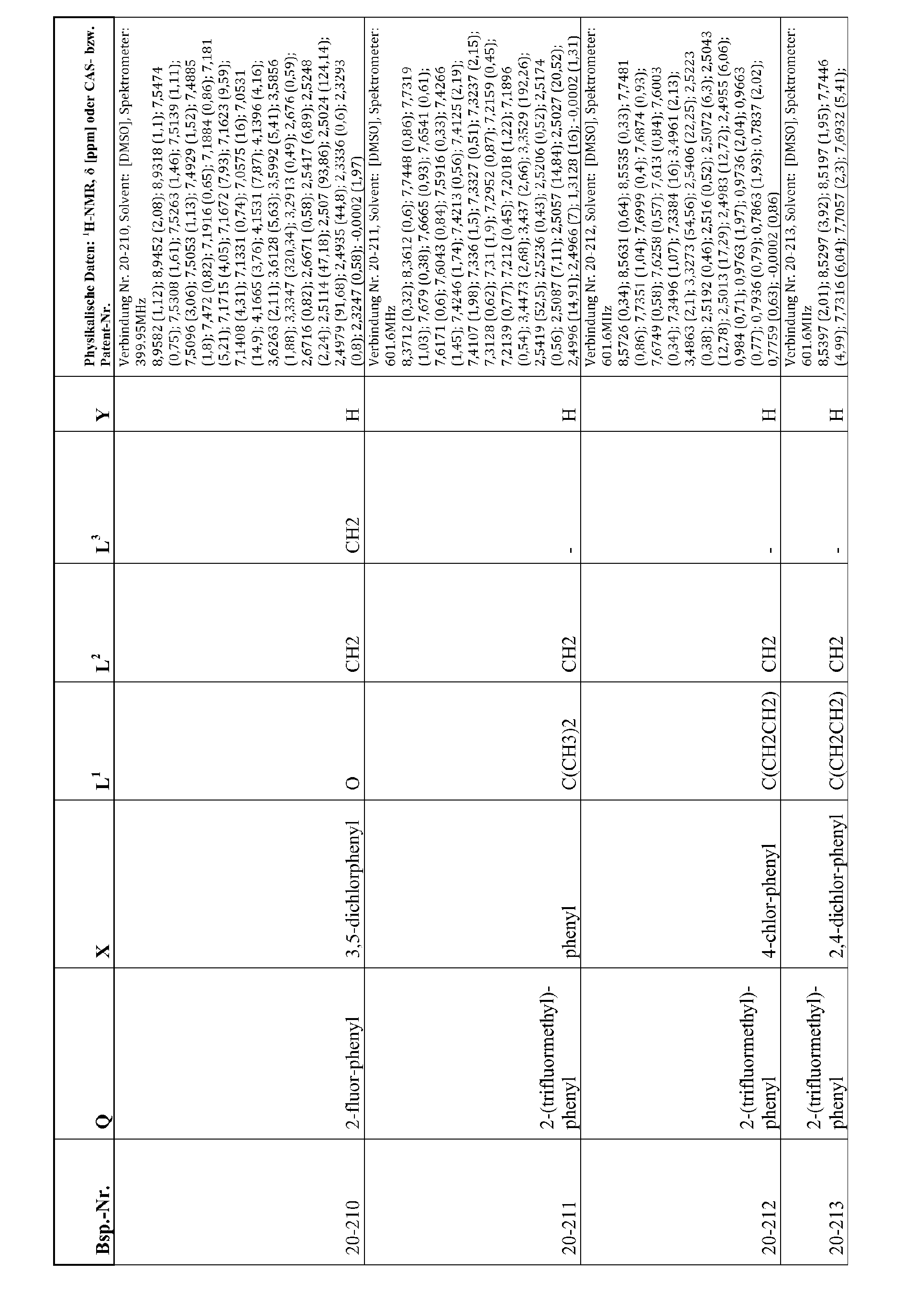

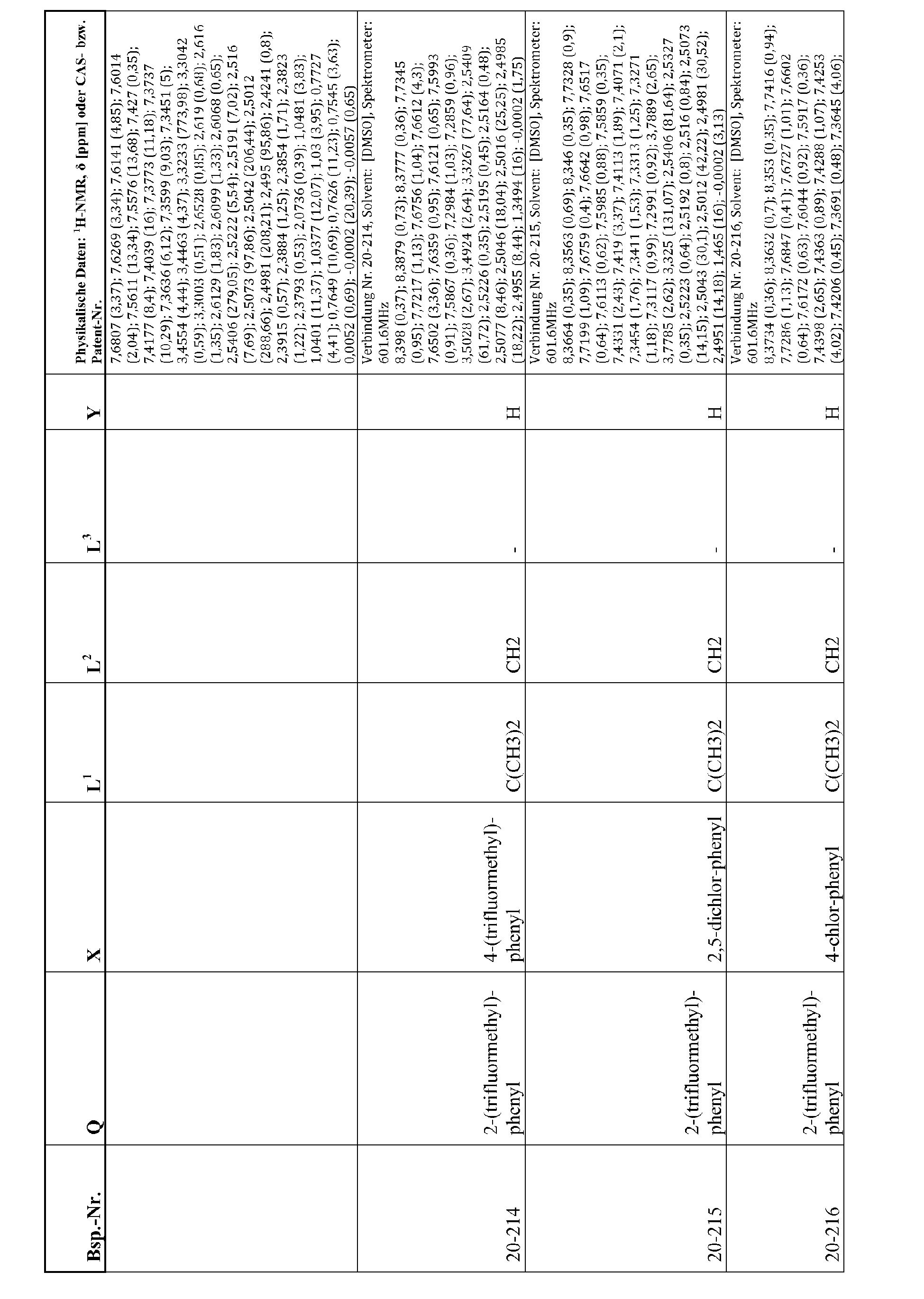

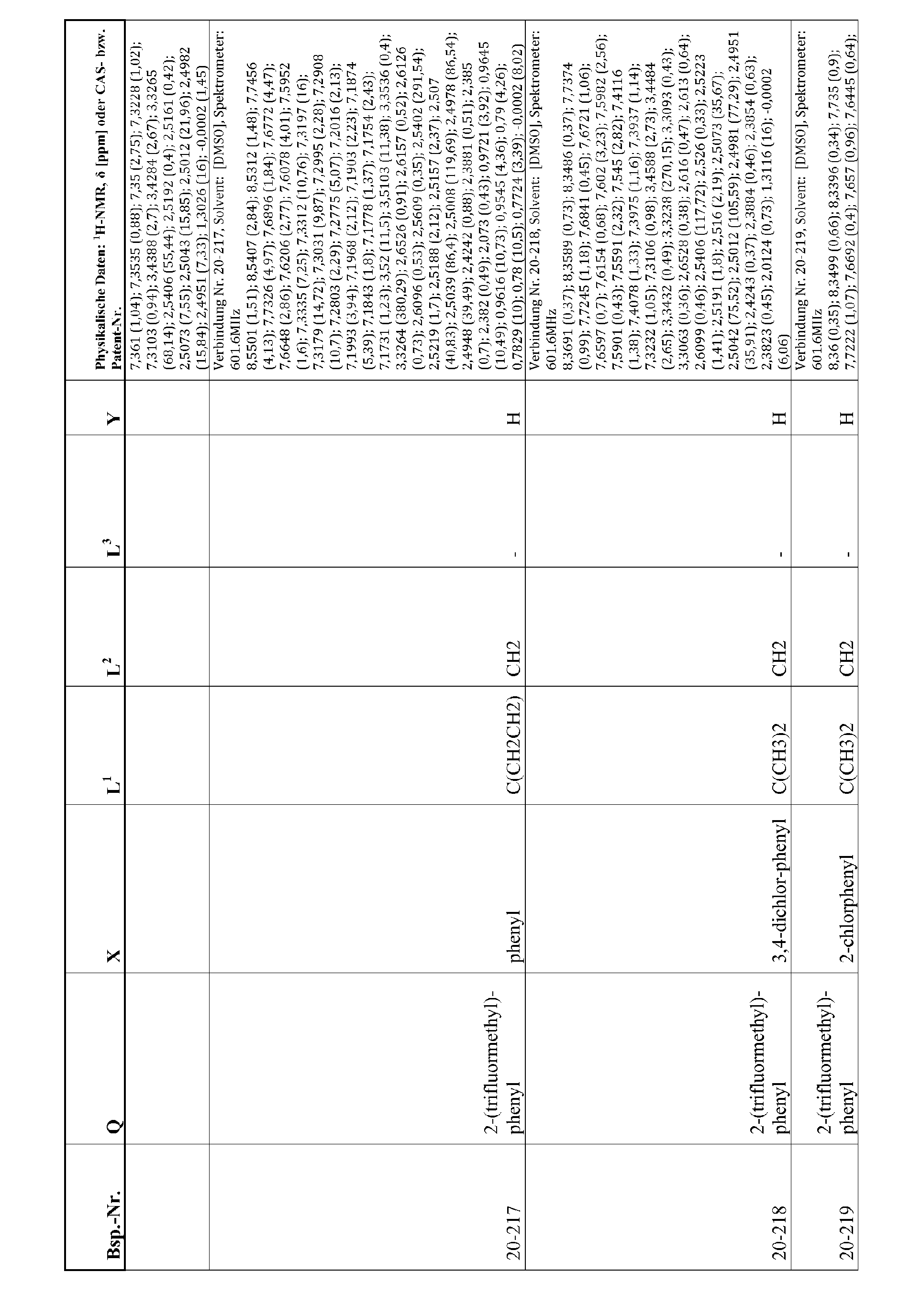

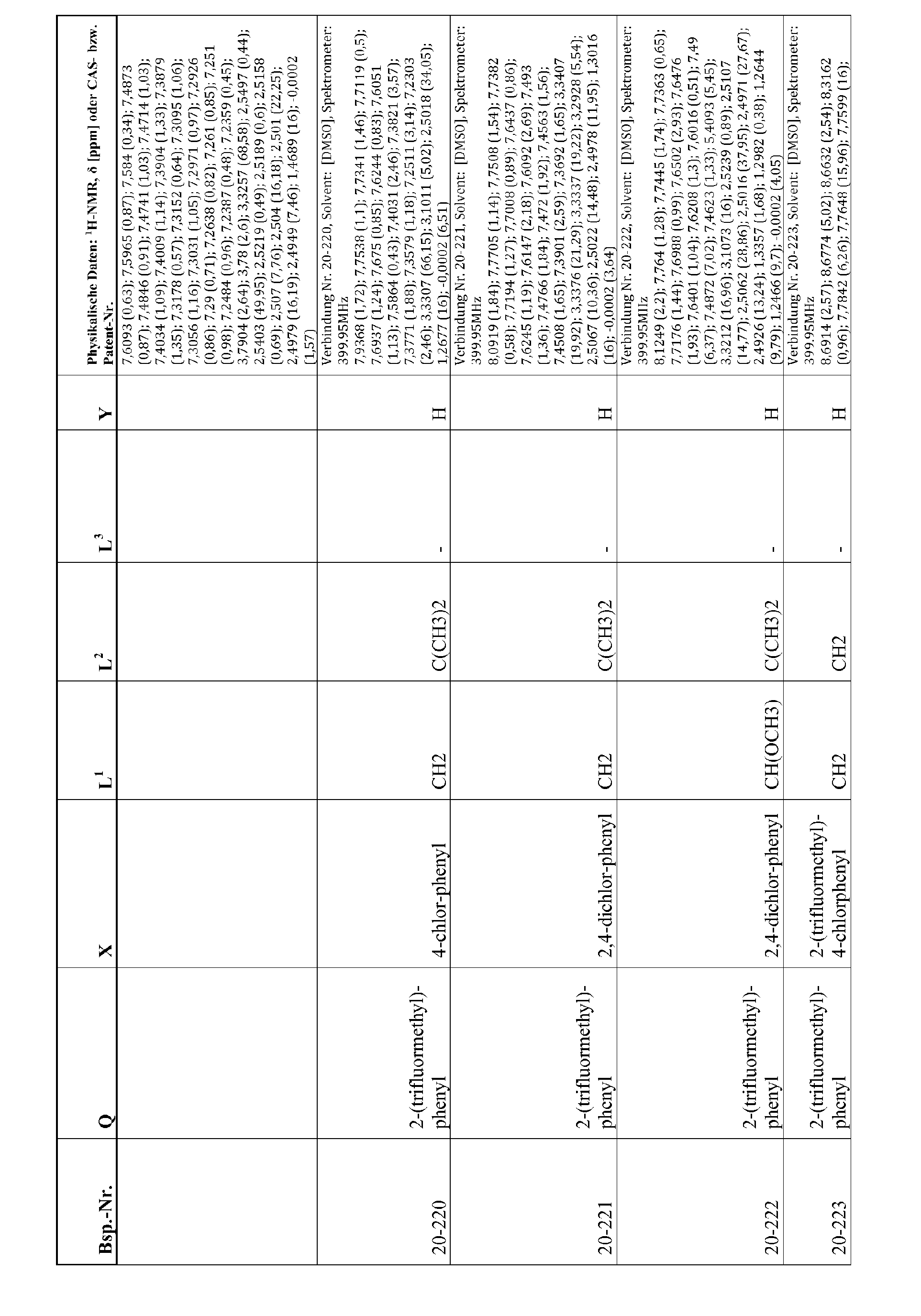

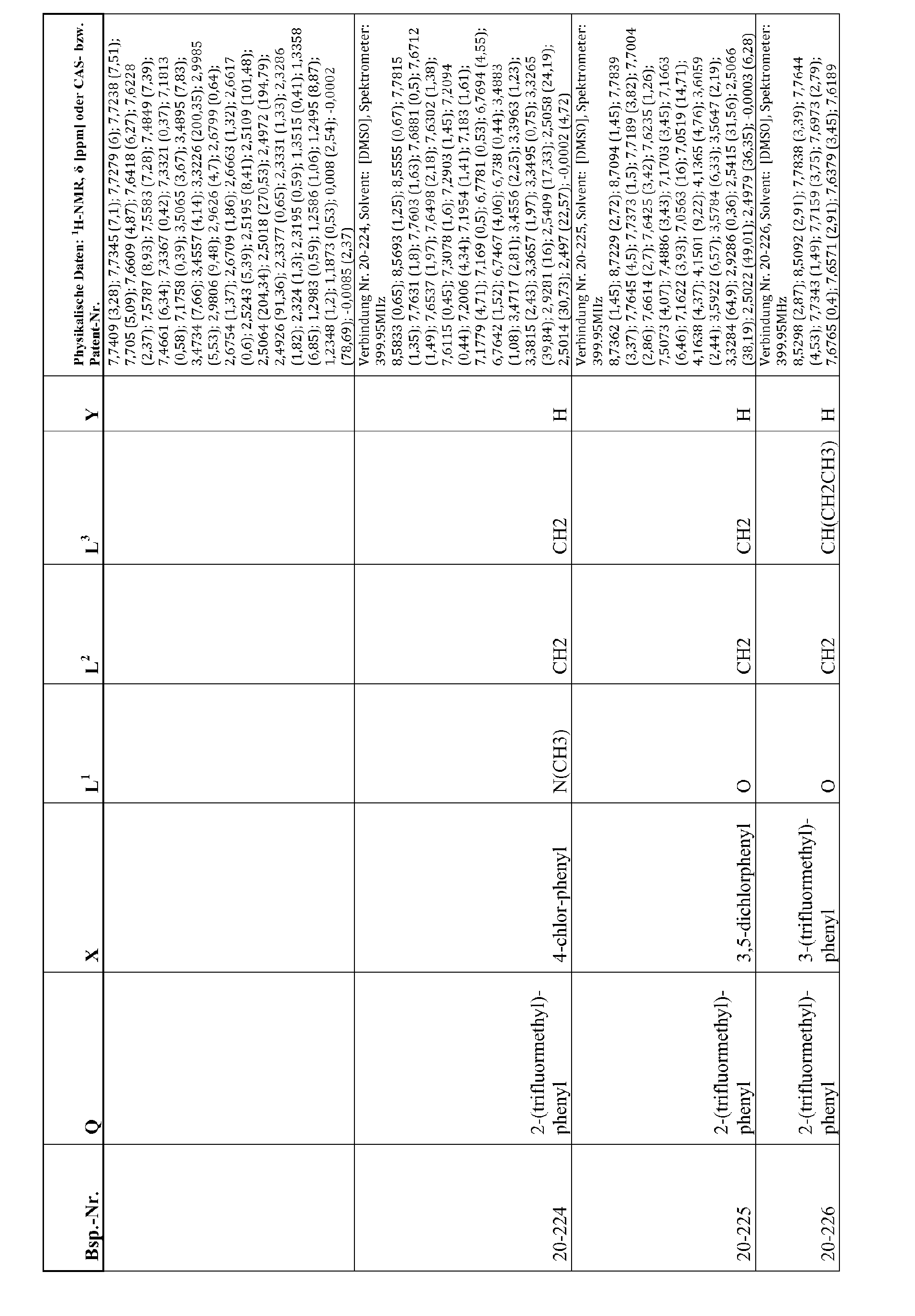

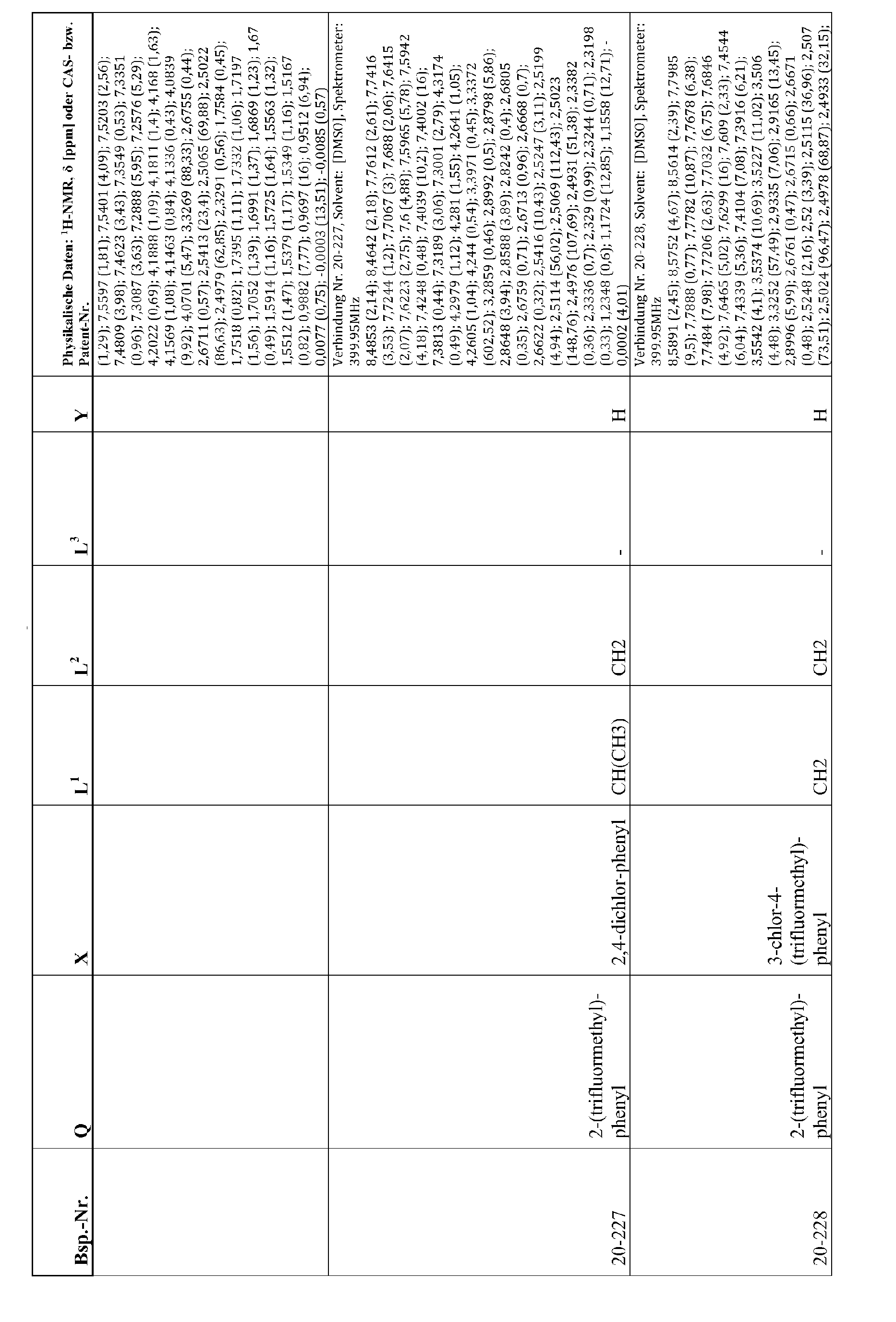

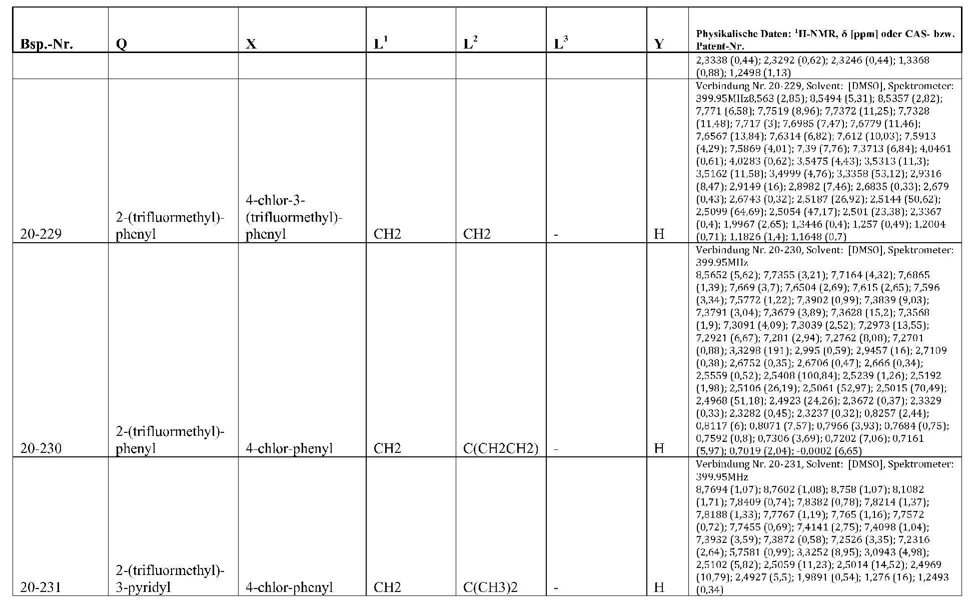

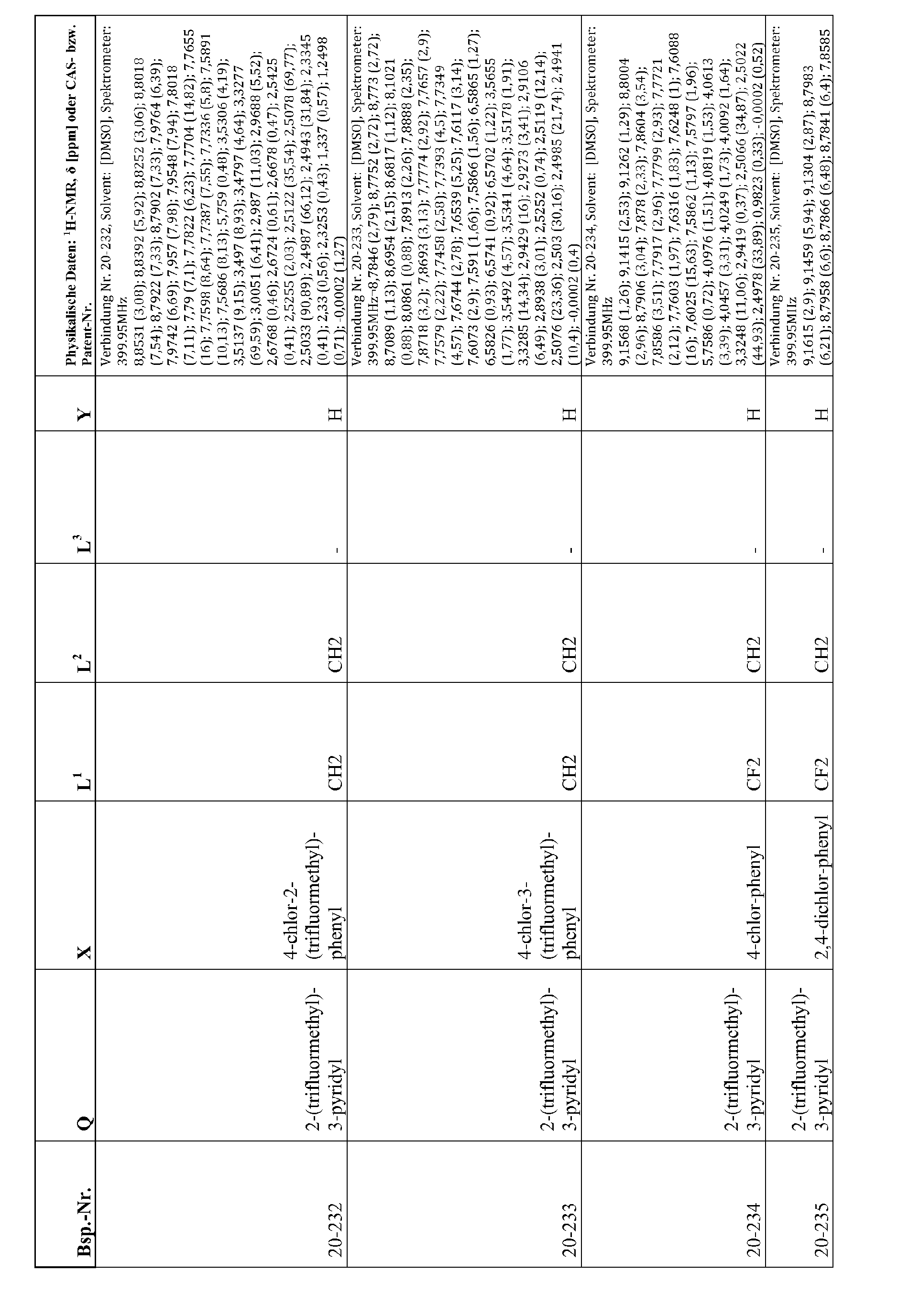

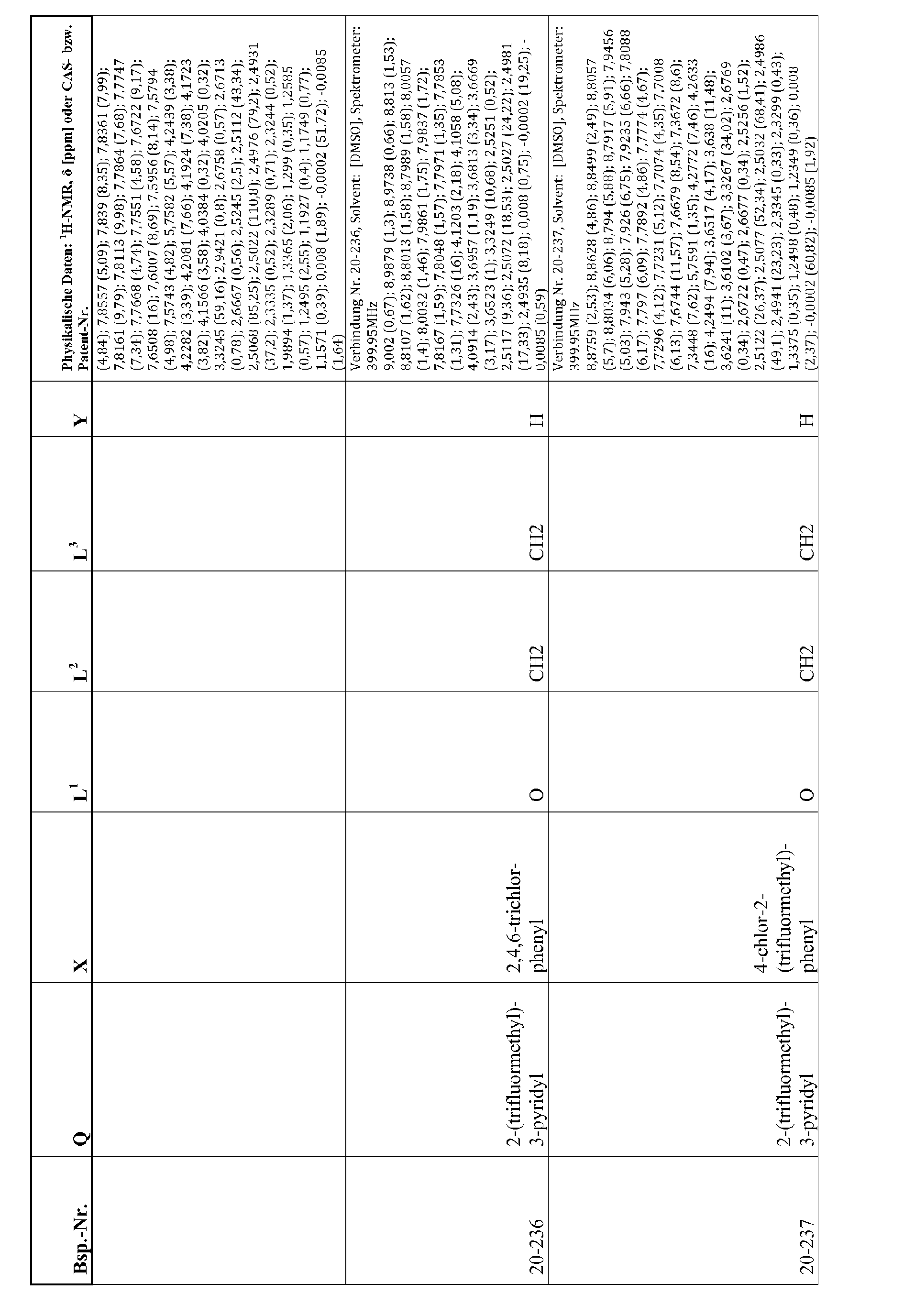

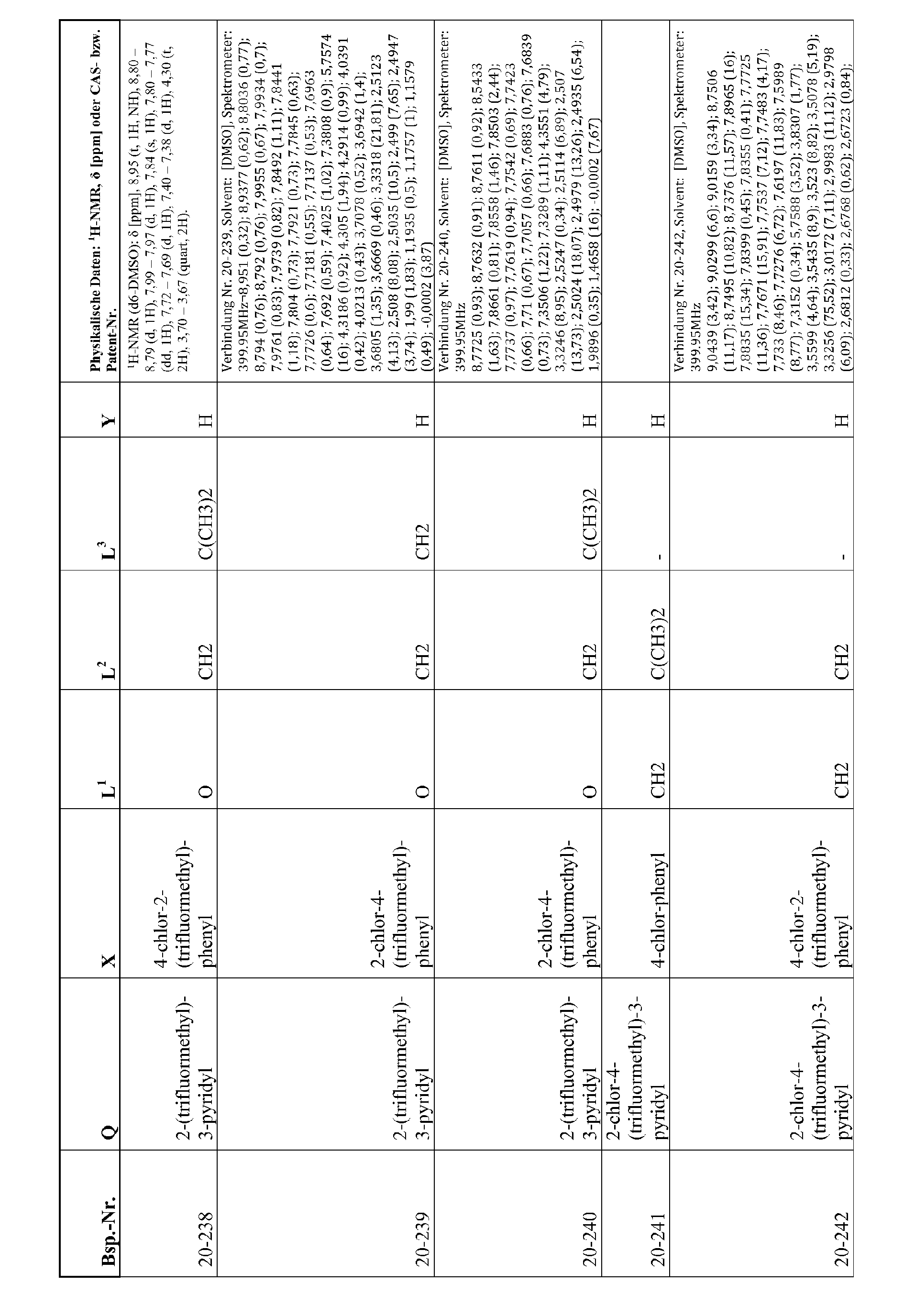

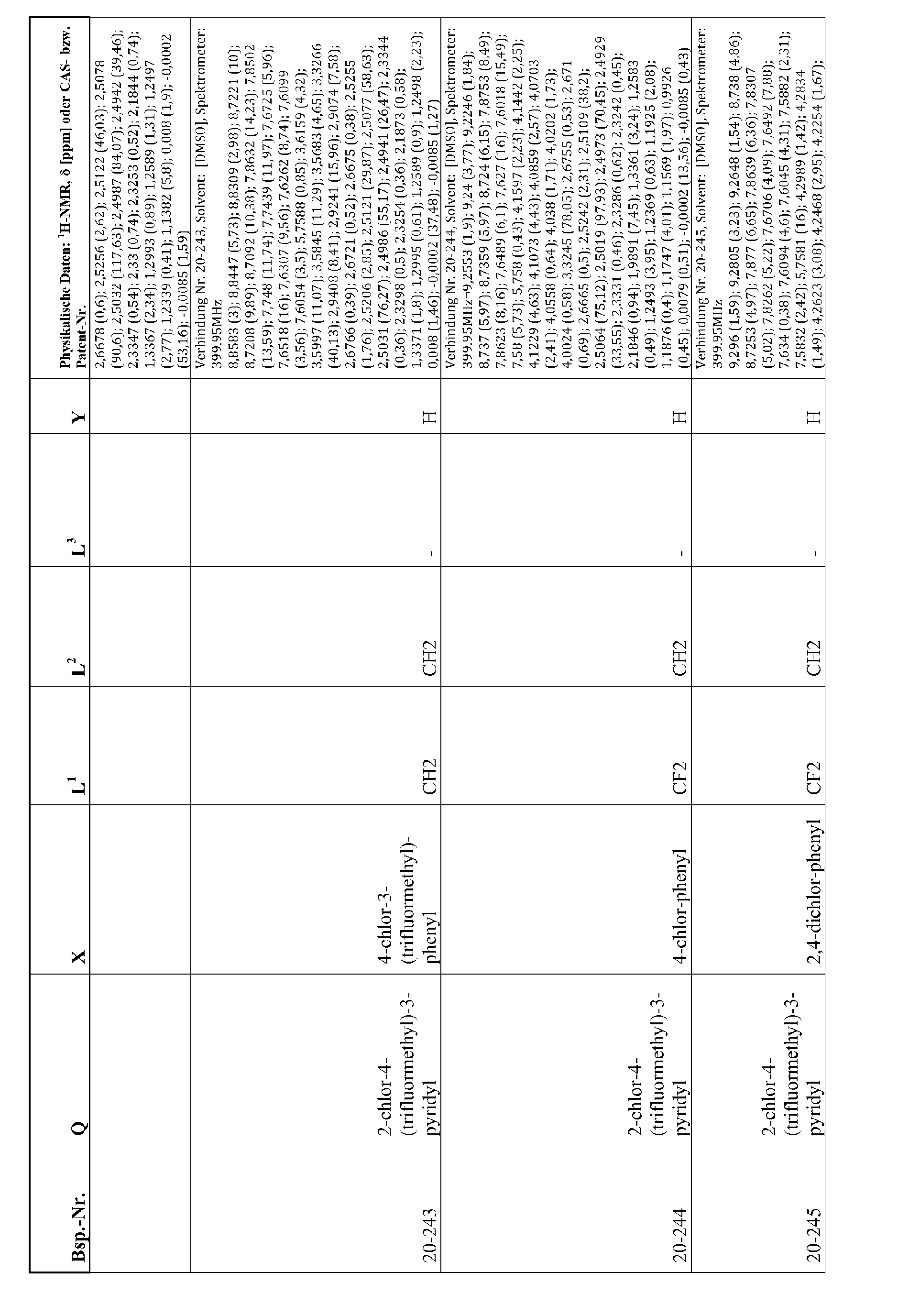

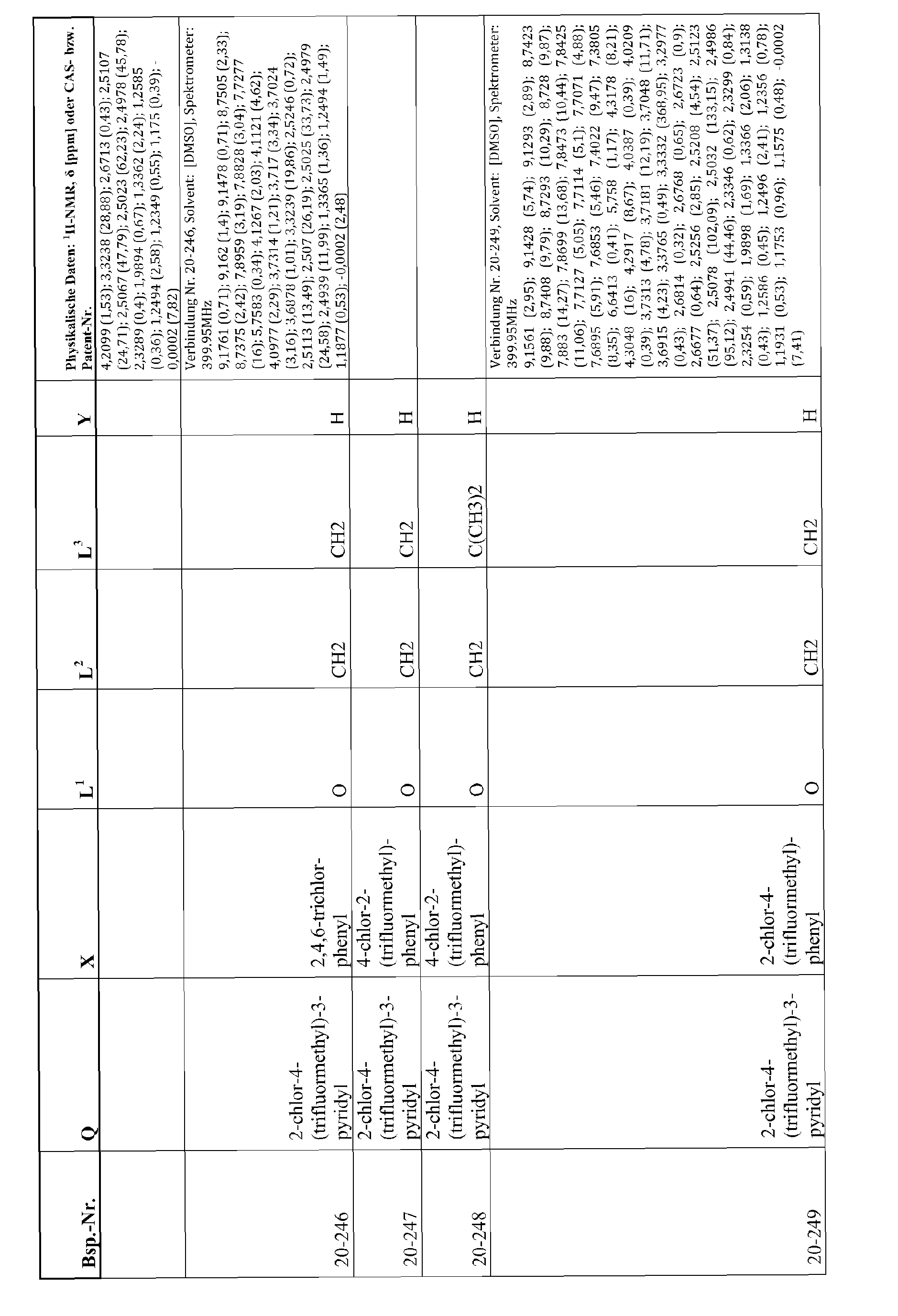

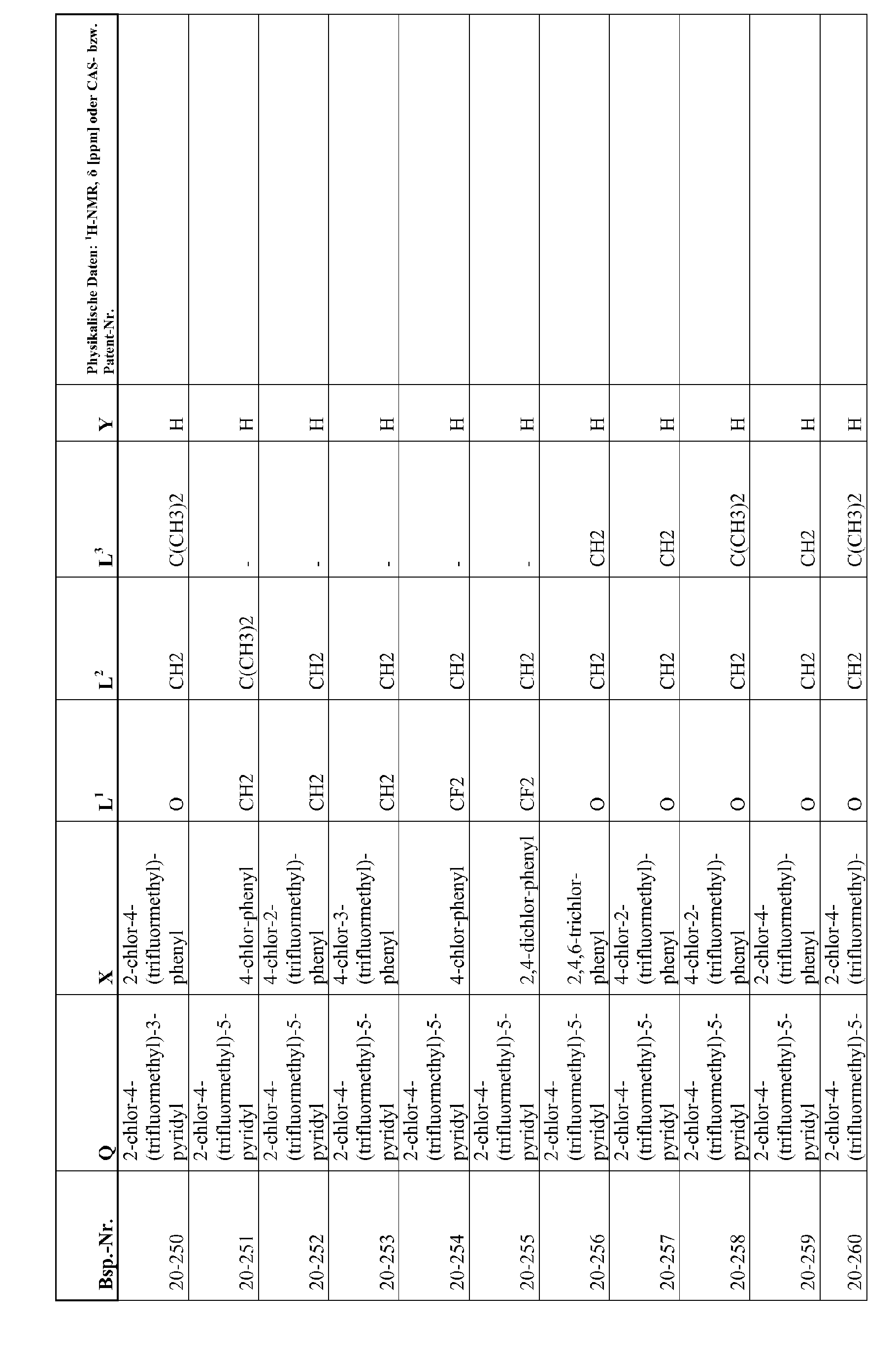

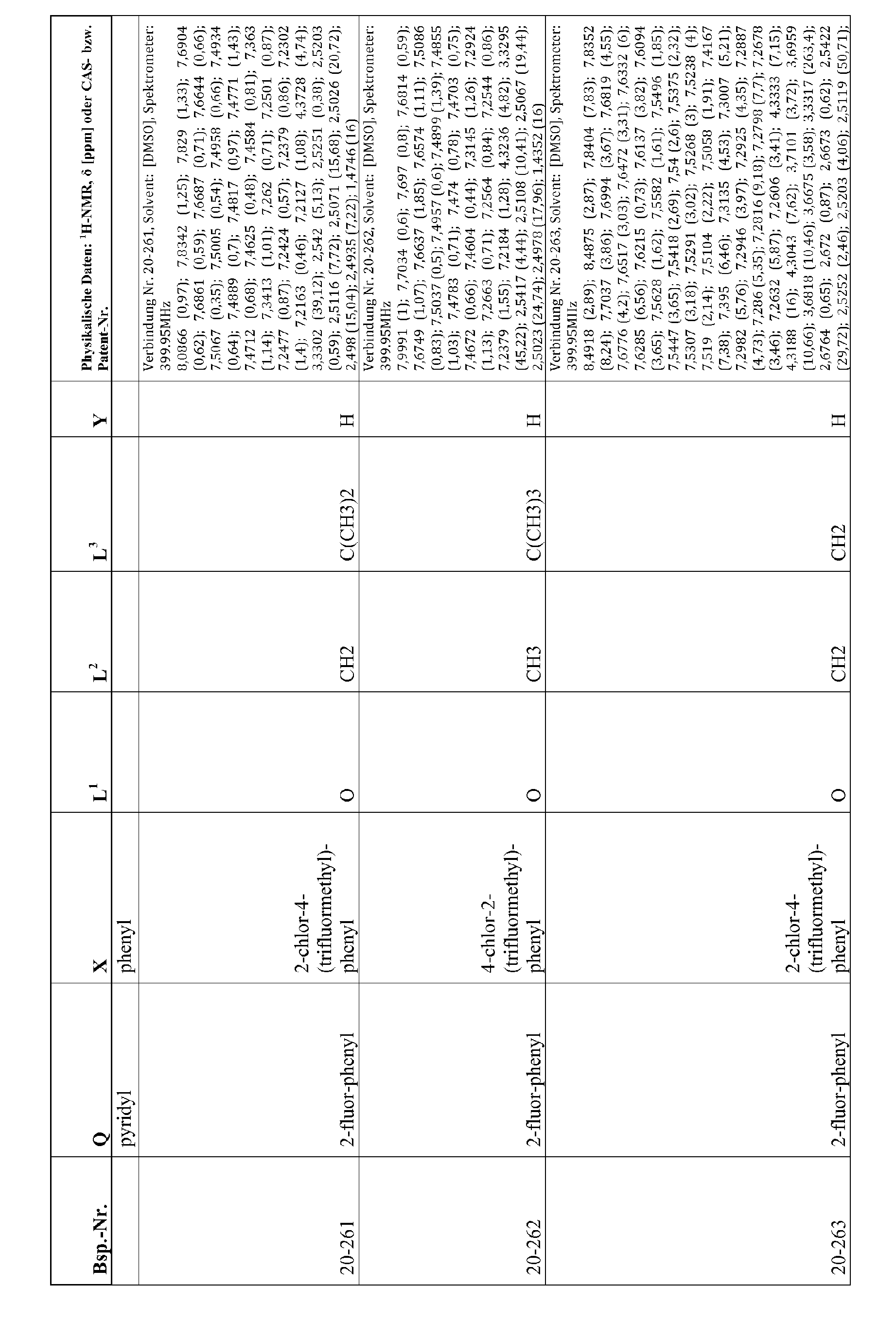

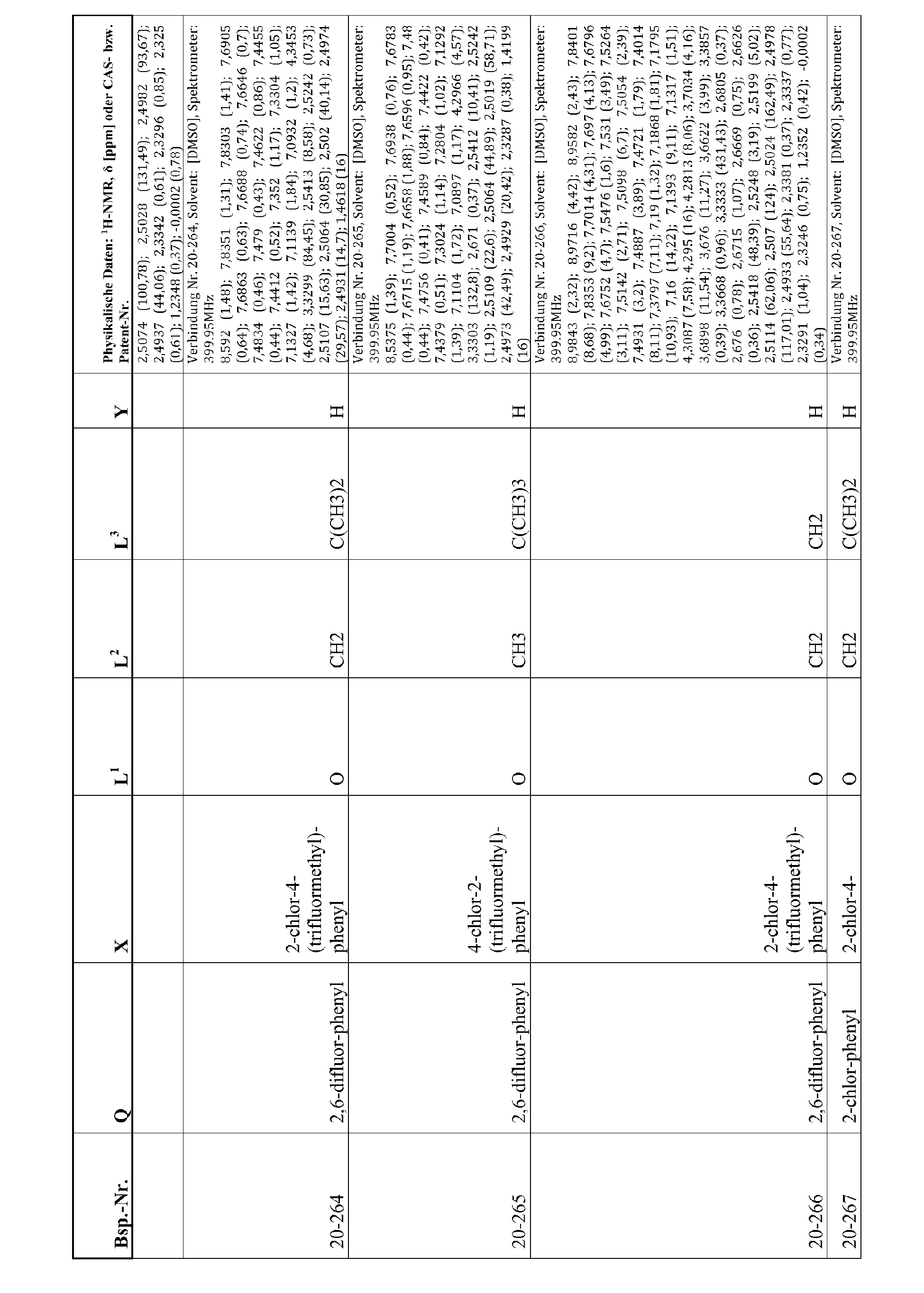

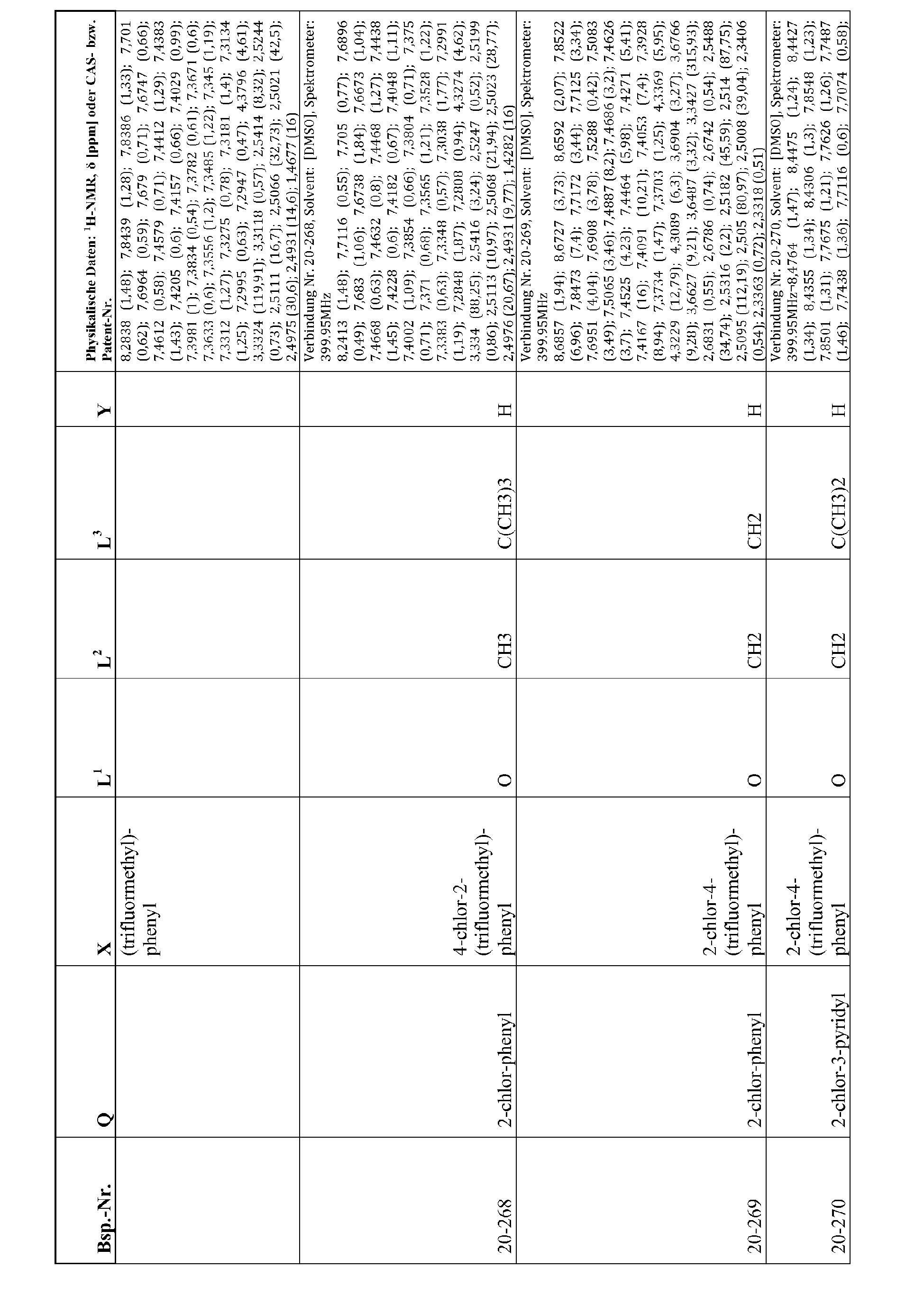

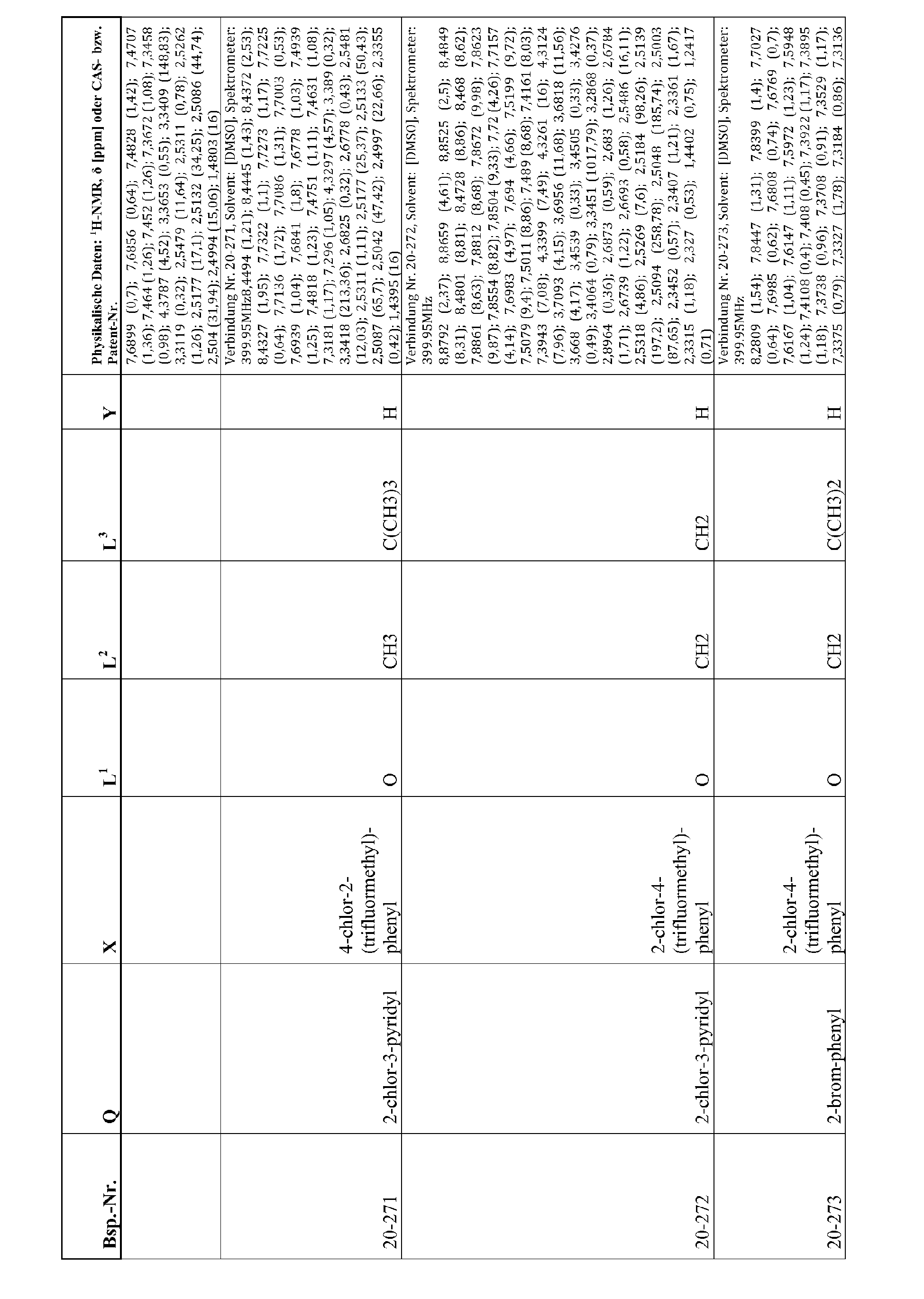

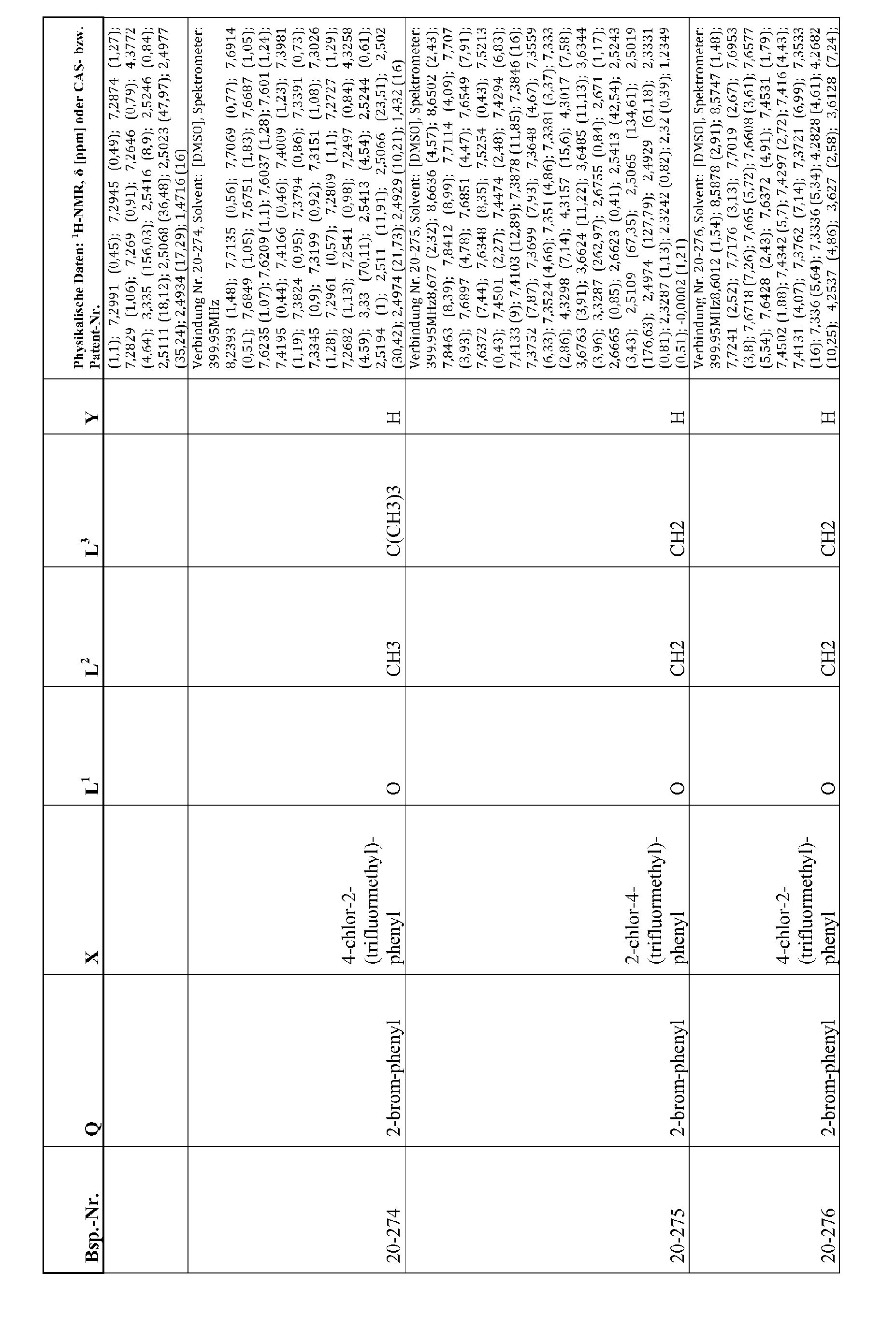

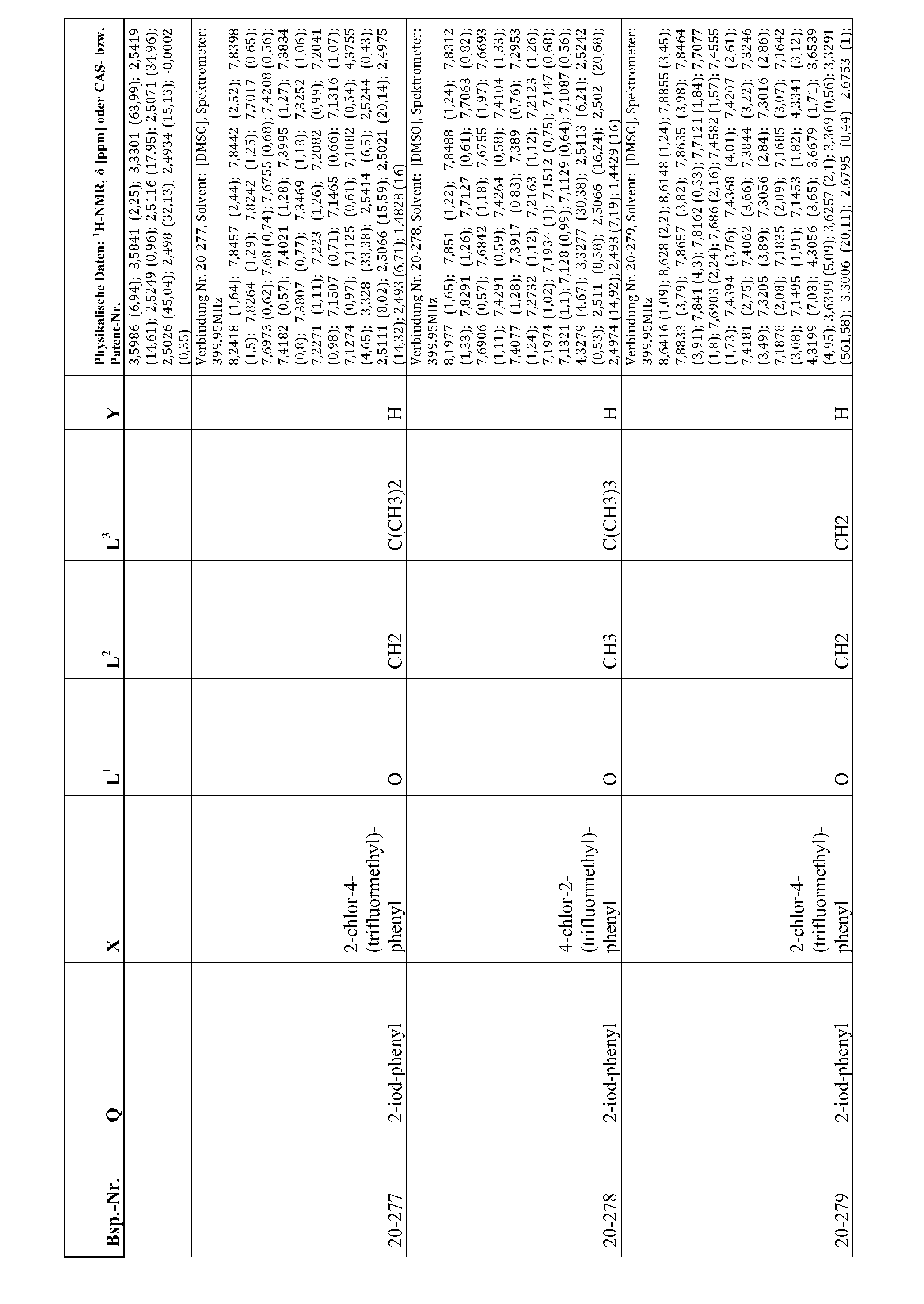

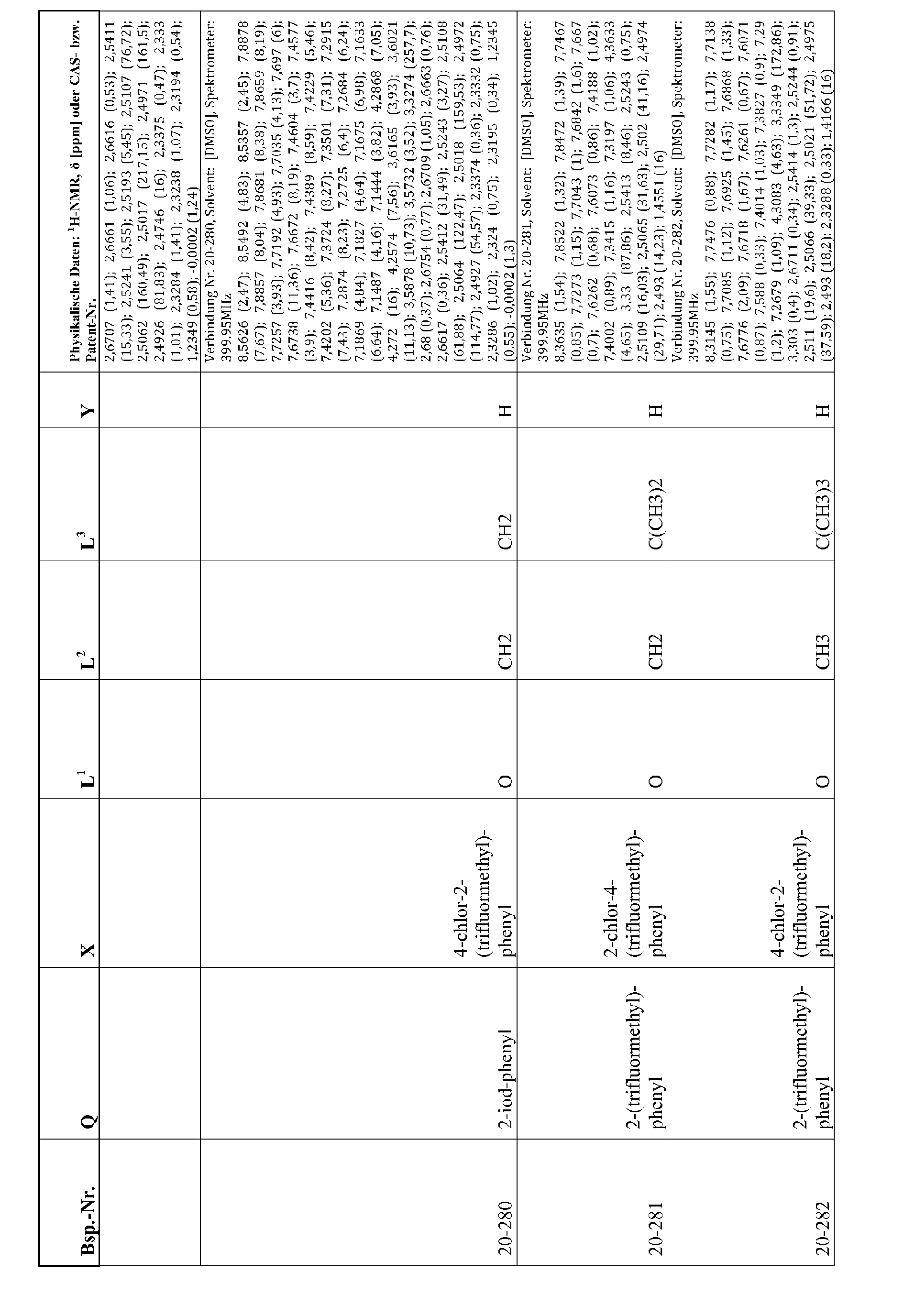

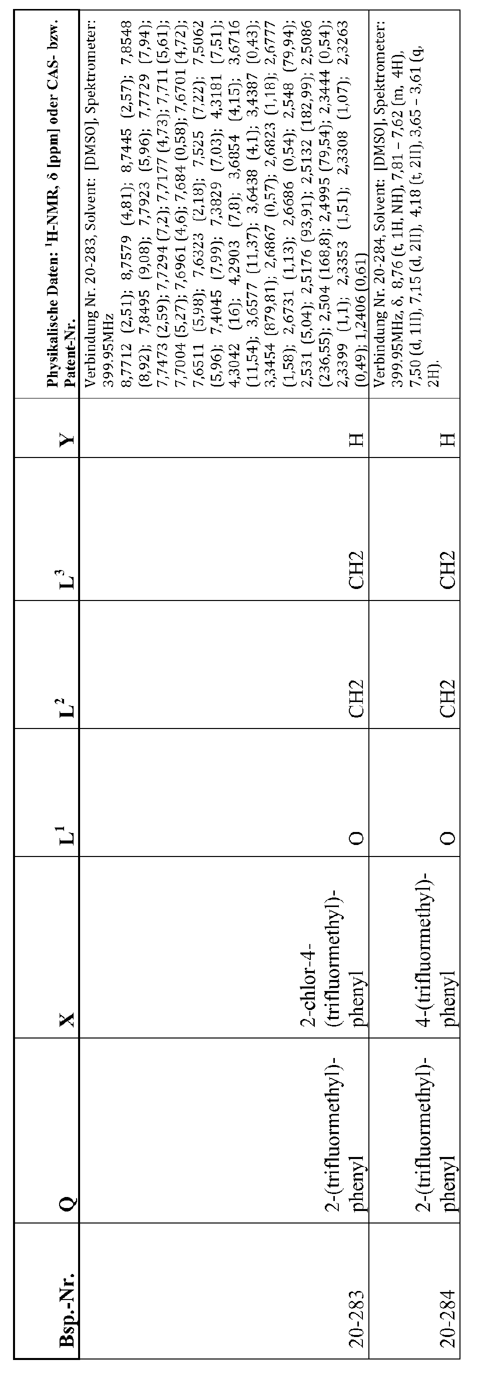

**Tabelle 21**

| | | | | | | |
|---|---|---|---|---|---|---|
| Verbindungen der Formel I-21 | | | | | | |
| 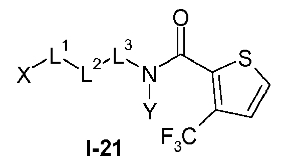 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 21-1 | 3-methyl-2-thienyl | CH2 | CH2 | - | H | |
| 21-2 | 2,4-dichlor-phenyl | CH2 | CH2 | CH2 | H | WO-A 2008/101976 |
| 21-3 | 4-chlor-phenyl | CH2 | CH2 | - | H | |
| 21-4 | 2,4-dichlor-phenyl | CH2 | CH2 | - | H | WO-A 2007/108483 |
| 21-5 | 4-chlor-phenyl | CH(OCH3) | CH(CH3) | - | H | |
| 21-6 | 2,4-dichlor-phenyl | CH(OCH3) | CH(CH3) | - | H | |
| 21-7 | 4-chlor-phenyl | CH(CH3) | CH2 | - | H | |
| 21-8 | 2,4-dichlor-phenyl | CH(CH3) | CH2 | - | H | |
| 21-9 | 4-chlor-phenyl | CH2 | CH(CH3) | - | H | |
| 21-10 | 2,4-dichlor-phenyl | CH2 | CH(CH3) | - | H | |
| 21-11 | 4-chlor-phenyl | C(CH3)2 | CH2 | - | H | |
| 21-12 | 2,4-dichlor-phenyl | C(CH3)2 | CH2 | - | H | |
| 21-13 | 2-chlor-phenyl | CH2 | CH2 | - | H | |
| 21-14 | 3,4-dichlor-phenyl | CH2 | CH2 | - | H | |
| 21-15 | 3,5-dichlor-phenyl | CH2 | CH2 | - | H | |
| 21-16 | 3-chlor-phenyl | CH2 | CH2 | - | H | |
| 21-17 | 2-fluor-phenyl | CH2 | CH2 | - | H | |
| 21-18 | 2,6-difluor-phenyl | CH2 | CH2 | - | H | |
| 21-19 | 2,6-dichlor-phenyl | CH2 | CH2 | - | H | |
| 21-20 | 3 -(trifluormethyl)-phenyl | CH2 | CH2 | - | H | |
| 21-21 | 4-(trifluormethyl)-phenyl | CH2 | CH2 | - | H | |
| 21-22 | 2-methyl-phenyl | CH2 | CH2 | - | H | |
| 21-23 | 2,4,6-trimethyl-phenyl | CH2 | CH2 | - | H | |
| 21-24 | 3,4-bismethoxy-phenyl | CH2 | CH2 | - | H | |
| 21-25 | phenyl | CH2 | CH2 | - | H | |
| 21-26 | 4-chlor-phenyl | C(CH2-CH2) | CH2 | - | H | |
| 21-27 | 2,4-dichlor-phenyl | C(CH2-CH2) | CH2 | - | H | |
| 21-28 | 4-chlor-phenyl | CH2 | C(CH2-CH2) | - | H | |
| 21-29 | 2,4-dichlor-phenyl | CH2 | C(CH2-CH2) | - | H | |
| 21-30 | 4-chlor-phenyl | O | CH2 | CH2 | H | |
| 21-31 | 2,4-dichlor-phenyl | O | CH2 | CH2 | H | |
| 21-32 | 4-chlor-phenyl | CH2 | CH2 | CH2 | H | |
| 21-33 | 4-chlor-phenyl | NCH3 | CH2 | CH2 | H | |
| 21-34 | 2,4-dichlor-phenyl | NCH3 | CH2 | CH2 | H | |
| 21-35 | 4-chlor-phenyl | CH(OCH3) | CH2 | - | H | |
| 21-36 | 2,4-dichlor-phenyl | CH(OCH3) | CH2 | - | H | |
| 21-37 | 2-thienyl | CH2 | CH2 | - | H | |
| 21-38 | 3-thienyl | CH2 | CH2 | - | H | |
| 21-39 | 2-furyl | CH2 | CH2 | - | H | |
| 21-40 | 3-furyl | CH2 | CH2 | - | H | |
| 21-41 | phenyl | CH2 | CH2 | CH(CH3) | H | |
| 21-42 | phenyl | CH2 | CH2 | CH2 | H | |
| 21-43 | 2-Cl-phenyl | CH2 | CH2 | CH(CH3) | H | |
| 21-44 | 4-t-butyl-phenyl | CH2 | CH2 | CH2 | H | |
| 21-45 | 4-methyl-phenyl | CH2 | CH2 | CH2 | H | |
| 21-46 | phenyl | CH2 | CH2 | CH(CH2CH3) | H | |
| 21-47 | 2-methoxy-phenyl | CH2 | CH2 | CH2 | H | |
| 21-48 | 2-methyl-phenyl | CH2 | CH2 | CH2 | H | |
| 21-49 | 3-methyl-phenyl | CH2 | CH2 | CH2 | H | |
| 21-50 | 3-chlor-phenyl | CH2 | CH2 | CH2 | H | |
| 21-51 | 2,6-difluor-phenyl | CH2 | CH2 | CH2 | H | |
| 21-52 | 4-chlor-phenyl | CH2 | CH2 | CH2 | H | |
| 21-53 | 2,6-dichlor-phenyl | CH2 | CH2 | CH(CH3) | H | |
| 21-54 | 3,5-dichlor-phenyl | CH2 | CH2 | CH(CH3) | H | |
| 21-55 | 2,6-dimethyl-phenyl | CH2 | CH2 | CH2 | H | |
| 21-56 | 2,5-dichlor-phenyl | CH2 | CH2 | CH2 | H | |
| 21-57 | 4-i-propoxy-phenyl | CH2 | CH2 | CH2 | H | |
| 21-58 | 3-trifluormethyl-phenyl | CH2 | CH2 | CH(CH3) | H | |
| 21-59 | 4-methyl-phenyl | CH2 | CH2 | CH(CH3) | H | |
| 21-60 | 2-methyl-phenyl | CH2 | CH2 | CH(CH3) | H | |
| 21-61 | 3,4-dichlor-phenyl | CH2 | CH2 | CH(CH3) | H | |
| 21-62 | 3,5-dichlor-phenyl | CH2 | CH2 | CH2 | H | |
| 21-63 | 2,6-dimethyl-phenyl | CH2 | CH2 | CH(CH3) | H | |
| 21-64 | 4-trifluormethyl-phenyl | CH2 | CH2 | CH(CH3) | H | |
| 21-65 | 2,5-dichlor-phenyl | CH2 | CH2 | CH(CH3) | H | |
| 21-66 | 4-phenoxy-phenyl | CH2 | CH2 | CH2 | H | |
| 21-67 | 3-chlor-phenyl | CH2 | CH2 | CH(CH3) | H | |
| 21-68 | 4-phenoxy-phenyl | CH2 | CH2 | CH(CH3) | H | |
| 21-69 | 2,4-dichlor-phenyl | CH2 | CH2 | CH(CH3) | H | |
| 21-70 | 2-difluormethoxy-phenyl | CH2 | CH2 | CH(CH3) | H | |
| 21-71 | 4-methoxy-phenyl | CH2 | CH2 | CH2 | H | |
| 21-72 | 4-chlor-phenyl | CH2 | CH2 | CH(CH3) | H | |
| 21-73 | 4-chlor-phenyl | CH2 | CH2 | CH(i-propyl) | H | |
| 21-74 | 4-fluor-phenyl | CH2 | CH2 | CH2 | H | |
| 21-75 | 4-chlor-phenyl | CH2 | CH2 | CH(n-propyl) | H | |
| 21-76 | 4-chlor-phenyl | CH2 | CH2 | CH(t-butyl) | H | |
| 21-77 | 2-chlor-phenyl | CH2 | CH2 | CH2 | H | |
| 21-78 | 4-chlor-phenyl | CH2 | CH(CH3) | - | cyclopropyl | |

**Tabelle 22**

| | | | | | | | |
|---|---|---|---|---|---|---|---|
| Verbindungen der Formel I-22 | | | | | | | |
| 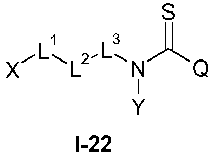 | | | | | | | |

| **Bsp.-Nr.** | **Q** | **X** | **L¹** | **L²** | **L³** | **Y** | **Physikalische Daten: ¹H-NMR, δ [ppm] oder Quelle** |
|---|---|---|---|---|---|---|---|
| 22-1 | 3-chlor-thienyl | 4-chlor-phenyl | CH(CH3) | CH2 | - | H | 1H-NMR (d6-DMSO): δ [ppm], 10,10 (s, 1H, NH), 7,76 (d, 1H), 7,38 - 7,32 (dd, 4H), 7,05 (d, 1H), 3,91 - 3,74 (2xm, 2H), 3,37 - 3,30 (m, 1H), 1,33 (d, 3H). |
| 22-2 | 3-chlor-thienyl | 2,4-dichlor-phenyl | CH(CH3) | CH3 | - | H | ¹H-NMR (d6-DMSO): δ [ppm], 10,15 (s, 1H, NH), 7,76 (d, 1H), 7,57 - 7,41 (m, 3H), 7,05 (d, 1H), 3,98 - 3,79 (m, 3H), 3,37 - 3,30 (m, 1H), 1,30 (d, 3H). |

**Tabelle 23**

| | | | | | | |
|---|---|---|---|---|---|---|
| Verbindungen der Formel I-23 | | | | | | |
| 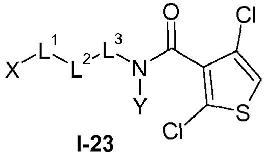 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 23-1 | 3-methyl-2-thienyl | CH2 | CH2 | - | H | |
| 23-2 | 2,4-dichlor-phenyl | CH2 | CH2 | CH2 | H | |
| | | | | | | Verbindung Nr. 23-3, Solvent: [DMSO], Spektrometer: 399.95MHz |
| 23-3 | 4-chlor-phenyl | CH2 | CH2 | - | H | 8,4094 (1,95); 8,3977 (3,36); 8,3841 (1,84); 7,3599 (7,78); 7,34 (12,6); 7,2746 (12,92); 7,262 (15,69); 7,2601 (16); 7,2543 (8,56); 3,441 (3); 3,4243 (6,9); 3,4086 (6,99); 3,3912 (3,64); 3,3363 (1234,04); 3,3339 (1028,02); 3,2344 (0,51); 2,8168 (5,5); 2,7988 (9,82); 2,7811 (4,71); 2,6717 (2,38); 2,5415 (19,39); 2,5397 (18,68); 2,5026 (365,92); 2,3295 (2,26); 2,0741 (0,43); 1,2344 (0,51); -0,0002 (16,17); - 0,0019 (15,45) |
| | | | | | | Verbindung Nr. 23-4, Solvent: [DMSO], Spektrometer: 399.95MHz |
| 23-4 | 2,4-dichlor-phenyl | CH2 | CH2 | - | H | 8,4444 (1,12); 8,4302 (2,16); 8,4161 (1,1); 8,316 (0,34); 7,587 (5,6); 7,3967 (0,43); 7,3722 (16); 7,3534 (0,33); 7,2635 (15,9); 3,4685 (1,84); 3,4514 (4,63); 3,4364 (4,68); 3,4193 (2,06); 3,3233 (165,14); 2,9383 (3,93); 2,9209 (7,68); 2,9035 (3,43); 2,6798 (0,41); 2,6753 (0,85); 2,6708 (1,17); 2,6663 (0,86); 2,6616 (0,41); 2,524 (3,72); 2,5107 (65,8); 2,5062 (132,4); 2,5017 (174,38); 2,4972 (125,44); 2,4927 (59,91); 2,3372 (0,38); 2,333 (0,81); 2,3285 (1,13); 2,3239 (0,82); 2,3195 (0,39); 1,3354 (0,4); 1,2493 (0,41); 0,008 (2,55); -0,0002 (72,77); -0,0084 (2,34) |
| 23-5 | 4-chlor-phenyl | CH(OCH3) | CH(CH3) | - | H | |
| 23-6 | 2,4-dichlor-phenyl | CH(OCH3) | CH(CH3) | - | H | |
| | | | | | | Verbindung Nr. 23-7, Solvent: [DMSO], Spektrometer: 399.95MHz |
| 23-7 | 4-chlor-phenyl | CH(CH3) | CH2 | - | H | 8,3694 (1,13); 8,3556 (2,13); 8,3417 (1,1); 7,3647 (6,44); 7,3599 (2,46); 7,3486 (3,12); 7,3435 (11,88); 7,3377 (1,75); 7,2964 (1,73); 7,2906 (10,87); 7,2858 (3,14); 7,2741 (2,26); 7,2694 (6,11); 7,2111 (16); 3,392 (0,68); 3,3769 (1,04); 3,3731 (1,13); 3,3588 (3,86); 3,3346 (379,03); 3,3077 (2,83); 3,2928 (0,87); 3,289 (0,84); 3,2749 (0,61); 3,0386 (1,11); 3,0207 (2,21); 3,0028 (2,12); 2,9848 (0,99); 2,7117 (0,72); 2,6759 (0,62); 2,6713 (0,82); 2,6668 (0,62); 2,5676 (0,49); 2,5416 (198,1); 2,5247 (2,6); 2,5198 (4,34); 2,5113 (48,81); 2,5068 (98,32); 2,5023 (129,34); 2,4977 (94,29); 2,4933 (46,11); 2,4671 (0,33); 2,3679 (0,73); 2,3336 (0,59); 2,329 (0,81); 2,3245 (0,59); 2,0745 (0,51); 1,2203 (13,97); 1,2028 (13,7); -0,0002 (8,06) |
| | | | | | | Verbindung Nr. 23-8, Solvent: [DMSO], Spektrometer: 399.95MHz |
| 23-8 | 2,4-dichlor-phenyl | CH(CH3) | CH2 | - | H | 20,0115 (0,42); 8,4185 (1,14); 8,4043 (2,34); 7,5676 (6,37); 7,5623 (6,93); 7,4672 (3,41); 7,446 (8,13); 7,4197 (4,9); 7,4144 (4,67); 7,3987 (2,12); 7,3933 (2,08); 7,2215 (16); 3,5268 (0,91); 3,5095 (2,03); 3,4925 (2,41); 3,4749 (2,34); 3,4598 (1,59); 3,4424 (2,42); 3,4267 (2,78); 3,4105 (1,85); 3,3964 (2,57); 3,3792 (3,85); 3,3646 (7,87); 3,3322 (2494,64); 3,2606 (1,24); 2,7111 (1,91); 2,6755 (4,55); 2,6709 (6,21); 2,6664 (4,54); 2,5412 (475,91); 2,5242 (23,84); 2,5109 (365,49); 2,5064 (724,17); 2,5019 (947); 2,4973 (685,77); 2,4929 (333,49); 2,3674 (1,83); 2,3331 (4,33); 2,3286 (5,98); 2,3241 (4,36); 2,2901 (0,62); 2,0742 (1,94); 1,2585 (0,47); 1,2351 (1); 1,2128 (13,29); 1,196 (13,12); 0,008 (1,33); -0,0001 (31,97); -0,0083 (0,96) |
| 23-9 | 4-chlor-phenyl | CH2 | CH(CH3) | - | H | |
| 23-10 | 2,4-dichlor-phenyl | CH2 | CH(CH3) | - | H | |
| | | | | | | Verbindung Nr. 23-12, Solvent: [DMSO], Spektrometer: 399.95MHz |
| 23-11 | 4-chlor-phenyl | C(CH3)2 | CH2 | - | H | 8,1803 (0,4); 8,1644 (0,81); 8,1492 (0,41); 7,5252 (2,25); 7,5195 (2,43); 7,4713 (1,46); 7,4496 (2,2); 7,3726 (1,46); 7,3667 (1,39); 7,351 (0,97); 7,3452 (0,92); 7,1644 (5,38); 3,7449 (2,75); 3,729 (2,72); 3,3766 (0,59); 3,3352 (262,2); 2,6755 (0,4); 2,6711 (0,56); 2,6669 (0,41); 2,5415 (52,18); 2,5244 (1,83); 2,511 (32,18); 2,5067 (65,15); 2,5021 (86,34); 2,4976 (63,79); 2,4932 (31,7); 2,3334 (0,38); 2,3289 (0,52); 2,3244 (0,38); 2,0742 (0,36); 1,4397 (16); -0,0002 (3,1) |
| 23-12 | 2,4-dichlor-phenyl | C(CH3)2 | CH2 | - | H | Verbindung Nr. 23-13, Solvent: [DMSO], Spektrometer: 399.95MHz |
| | | | | | | 15,3304 (0,91); 14,7843 (0,89); 8,4566 (1,23); 8,4447 (1,06); 7,8866 (0,91); 7,442 (2,27); 7,4235 (2,45); 7,4186 (2,68); 7,3595 (1,83); 7,3415 (2,82); 7,3077 (1,14); 7,2929 (2,85); 7,2887 (2,2); 7,2751 (14,86); 7,2598 (2,02); 7,2538 (1,87); 3,9437 (0,86); 3,7152 (1,16); 3,7111 (1,12); 3,6657 (1,33); 3,6279 (0,97); 3,6084 (1,08); 3,5888 (1,4); 3,5481 (1,58); 3,5292 (1,71); 3,4739 (3,46); 3,4572 (4,98); 3,4396 (5,96); 3,4243 (5,58); 3,3369 (4888,75); 3,2607 (2,51); 3,2253 (1,62); 3,2208 (1,54); 3,2035 (1,25); 3,1766 (0,88); 3,1355 (1,06); 3,0607 (1,03); 2,9574 (2,89); 2,9401 (4,58); 2,9213 (2,47); 2,7494 (1,02); 2,7109 (1,79); 2,6756 (11,72); 2,6711 (16); 2,6665 (11,63); 2,6487 (1,41); 2,6168 (1,28); 2,5979 (1,95); 2,5922 (1,93); 2,5414 (434,46); 2,5245 (53,58); 2,5111 (966,23); 2,5067 (1928,89); 2,5021 (2522,35); 2,4975 (1818,19); 2,493 (878,69); 2,4235 (1,24); 2,3848 (0,93); 2,3676 (1,3); 2,3334 (11,4); 2,3289 (15,19); 2,3244 (11,05); 2,2907 (1,19); 2,0739 (8,74); 1,4393 (0,91); 1,258 (1,52); 1,2445 (1,31); 1,2354 (2,36); 0,0079 (1,5); -0,0002 (37,15); - 3,2871 (0,87) |
| | | | | | | Verbindung Nr. 23-14, Solvent: [DMSO], Spektrometer: 399.95MHz |
| 23-13 | 2-chlor-phenyl | CH2 | CH2 | - | H | 8,4017 (1,01); 8,3886 (1,96); 8,3744 (1,08); 7,7614 (0,52); 7,5594 (5,74); 7,5492 (0,49); 7,5389 (6,81); 7,5281 (5,8); 7,5232 (5,72); 7,2553 (3,23); 7,2503 (3,09); 7,2382 (16); 7,2297 (2,73); 3,4901 (0,39); 3,4648 (2,16); 3,4478 (5,1); 3,4331 (5,43); 3,4161 (2,82); 3,3398 (844,78); 3,2759 (1,01); 3,2373 (0,37); 3,1759 (0,33); 2,8477 (0,4); 2,8323 (3,6); 2,8151 (7,12); 2,7978 (3,15); 2,7117 (1,08); 2,6761 (1,6); 2,6714 (2,17); 2,667 (1,53); 2,6622 (0,74); 2,5924 (0,4); 2,5873 (0,45); 2,5749 (0,5); 2,542 (235,81); 2,5245 (7,84); 2,5113 (134,34); 2,507 (263,42); 2,5025 (342,6); 2,498 (247,23); 2,4936 (119,52); 2,368 (1); 2,3335 (1,56); 2,3293 (2,16); 2,3248 (1,56); 2,0742 (0,7); 1,2346 (0,41); 0,0074 (0,4); -0,0002 (10,23); - 0,0087 (0,32) |
| 23-14 | 3,4-dichlor-phenyl | CH2 | CH2 | - | H | Verbindung Nr. 23-16, Solvent: [DMSO], Spektrometer: 399.95MHz8,4201 (1,05); 8,4067 (1,91); 8,3933 (1,06); 7,3474 (1,77); 7,3399 (0,51); 7,3274 (5,7); 7,3209 (5,13); 7,3166 (3,52); 7,3092 (4,57); 7,2827 (2,42); 7,2796 (3,41); 7,275 (2,72); 7,2628 (1,44); 7,2599 (1,79); 7,2548 (1,34); 7,2432 (16); 7,2187 (3,38); 7,2001 (2,39); 3,4617 (2,14); 3,4442 (4,72); 3,4297 (4,78); 3,4121 (2,62); 3,3445 (284,99); 2,8382 (3,58); 2,8205 (6,83); 2,8028 (3,26); 2,7121 (0,74); 2,6767 (0,55); 2,6721 (0,73); 2,6676 (0,55); 2,5699 (0,63); 2,5424 (172,33); 2,5118 (42); 2,5075 (83,31); 2,503 (108,79); 2,4985 (79,52); 2,4941 (39,13); 2,3684 (0,69); 2,3342 (0,48); 2,3297 (0,65); 2,3254 (0,48); -0,0002 (0,93) |
| 23-15 | 3,5-dichlor-phenyl | CH2 | CH2 | - | H | Verbindung Nr. 23-17, Solvent: [DMSO], Spektrometer: 399.95MHz8,4666 (0,86); 8,4532 (1,56); 8,439 (0,83); 7,3365 (0,95); 7,3324 (1,16); 7,3179 (1,94); 7,3133 (2,38); 7,3015 (0,9); 7,2975 (1,57); 7,2942 (1,53); 7,2882 (0,79); 7,2833 (1,44); 7,2767 (1,07); 7,2686 (1,59); 7,2625 (2,46); 7,2588 (16); 7,2492 (1,12); 7,2447 (0,82); 7,1788 (1,48); 7,1763 (1,88); 7,1555 (3,77); 7,15 (1,8); 7,1393 (3,41); 7,1363 (3,04); 7,1328 (1,61); 7,1296 (1,3); 7,1209 (1,43); 7,1179 (1,19); 3,452 (1,75); 3,4347 (3,58); 3,4197 (3,43); 3,4167 (3,32); 3,4014 (2,25); 3,3411 (246,18); 2,8659 (2,58); 2,8478 (4,55); 2,8296 (2,28); 2,6762 (0,46); 2,6718 (0,63); 2,6672 (0,48); 2,5421 (6,08); 2,525 (2,41); 2,5118 (38,02); 2,5073 (76,27); 2,5027 (100,15); 2,4981 (72,48); 2,4936 (35,32); 2,334 (0,46); 2,3295 (0,64); 2,3247 (0,45); -0,0002 (0,9) |
| 23-16 | 3-chlor-phenyl | CH2 | CH2 | - | H | Verbindung Nr. 23-18, Solvent: [DMSO], Spektrometer: 399.95MHz |
| | | | | | | 8,5227 (0,93); 8,5081 (1,73); 8,4939 (0,91); 7,3651 (0,56); 7,3482 (1,24); 7,3442 (1,11); 7,3273 (2,36); 7,3102 (1,17); 7,3065 (1,52); 7,2897 (0,7); 7,2308 (16); 7,1022 (0,48); 7,0901 (3,28); 7,0702 (5,22); 7,0591 (0,71); 7,0501 (2,73); 7,0374 (0,36); 3,4202 (1,99); 3,4034 (4,4); 3,3873 (4,77); 3,3698 (4,01); 3,342 (300,48); 3,2921 (0,49); 2,8849 (2,46); 2,8675 (4,61); 2,85 (2,18); 2,6763 (0,54); 2,6718 (0,75); 2,6674 (0,55); 2,542 (4,42); 2,525 (2,8); 2,5118 (45,29); 2,5073 (89,72); 2,5028 (117,31); 2,4982 (85,04); 2,4937 (41,62); 2,3338 (0,53); 2,3295 (0,73); 2,3248 (0,55); -0,0002 (0,86) |
| | | | | | | Verbindung Nr. 23-19, Solvent: [DMSO], Spektrometer: 399.95MHz |
| 23-17 | 2- fluor-phenyl | CH2 | CH2 | - | H | 19,1408 (0,92); 8,5291 (1,11); 7,4714 (4,01); 7,4516 (5,66); 7,3073 (1,97); 7,2832 (6,17); 7,2679 (0,98); 4,87 (1,07); 4,3055 (0,96); 4,1606 (1,02); 3,7688 (1,03); 3,7288 (0,93); 3,679 (0,98); 3,6295 (1,15); 3,6029 (1,58); 3,562 (1,5); 3,5478 (1,61); 3,5279 (2,65); 3,4948 (2,73); 3,4749 (2,51); 3,4383 (7); 3,426 (6,9); 3,3432 (6877,18); 3,2793 (8,44); 3,2304 (3,47); 3,1713 (1,12); 3,1518 (2,19); 3,1353 (3,17); 3,1176 (2,28); 3,0968 (1,28); 3,0709 (1,46); 3,0604 (0,99); 3,0447 (1,06); 3,0289 (0,95); 2,9409 (1,13); 2,9204 (1,01); 2,9094 (0,93); 2,676 (11,25); 2,6716 (16); 2,6673 (11,67); 2,5964 (1,82); 2,5418 (69,55); 2,5246 (57,73); 2,5113 (953,34); 2,507 (1877,51); 2,5026 (2445,77); 2,4981 (1776,72); 2,4938 (872,51); 2,4384 (2,36); 2,391 (1,34); 2,3673 (1,3); 2,3339 (10,71); 2,3293 (14,87); 2,3247 (10,66); 2,2914 (1,5); 2,0737 (7,17); 1,2978 (0,92); 1,258 (1,34); 1,2356 (1,54); 0,008 (2,92); -0,0002 (64,72); -3,221 (0,96); -3,6597 (0,89) |
| | | | | | | Verbindung Nr. 23-20, Solvent: [DMSO], Spektrometer: 399.95MHz |
| 23-18 | 2,6-difluor-phenyl | CH2 | CH2 | - | H | 8,4219 (1,19); 8,4085 (2,09); 8,3958 (1,11); 7,5895 (4,83); 7,5614 (8,2); 7,5514 (5,36); 7,5414 (1,47); 7,5337 (1,06); 7,5255 (0,45); 7,5157 (0,64); 7,2556 (0,57); 7,2173 (16); 3,4981 (2,2); 3,4806 (5,45); 3,4663 (5,53); 3,4491 (2,6); 3,4265 (0,35); 3,3483 (224,96); 3,345 (258,95); 3,2753 (0,34); 2,9348 (3,91); 2,9175 (7,81); 2,9 (3,5); 2,7123 (0,85); 2,6767 (0,51); 2,6723 (0,71); 2,6678 (0,51); 2,5427 (207,19); 2,5255 (2,54); 2,5207 (3,65); 2,5122 (42,66); 2,5078 (86,35); 2,5032 (113,99); 2,4987 (83,14); 2,4942 (40,86); 2,3686 (0,89); 2,3344 (0,51); 2,3298 (0,74); 2,3255 (0,55); 2,0742 (0,65); -0,0002 (5,44) |
| | | | | | | Verbindung Nr. 23-21, Solvent: [DMSO], Spektrometer: 399.95MHz |
| 23-19 | 2,6-dichlor-phenyl | CH2 | CH2 | - | H | 8,4426 (1,18); 8,4297 (2,14); 8,4162 (1,14); 7,6703 (5,64); 7,6501 (6,81); 7,4832 (6,41); 7,4632 (5,29); 7,2564 (16); 3,495 (2,04); 3,4777 (4,73); 3,4629 (4,72); 3,4454 (2,22); 3,3365 (221,13); 3,2922 (0,48); 2,9255 (3,31); 2,9078 (6,22); 2,8902 (2,9); 2,6764 (0,5); 2,6717 (0,67); 2,6672 (0,48); 2,542 (10,39); 2,5246 (2,82); 2,5113 (42,35); 2,5071 (81,57); 2,5026 (105,26); 2,4981 (76,57); 2,4939 (37,93); 2,3336 (0,5); 2,3297 (0,66); 2,3249 (0,5); 2,0743 (0,47); -0,0002 (5,32) |
| | | | | | | Verbindung Nr. 23-22, Solvent: [DMSO], Spektrometer: 399.95MHz |
| 23-20 | 3 -(trifluormethyl)-phenyl | CH2 | CH2 | - | H | 8,4812 (0,55); 8,4674 (1); 8,454 (0,55); 7,297 (8,12); 7,1679 (1,34); 7,1555 (1,83); 7,1449 (3,91); 7,1376 (1,55); 7,1323 (1,02); 7,1269 (3,06); 7,1239 (3,17); 7,1136 (2,28); 7,1088 (1,22); 7,1027 (1,44); 3,3909 (1,3); 3,3756 (2,45); 3,3723 (2,32); 3,3672 (2,13); 3,3393 (135,72); 2,8204 (2,14); 2,8049 (1,7); 2,8006 (2,45); 2,7821 (1,9); 2,7118 (0,37); 2,6716 (0,39); 2,5611 (0,45); 2,5419 (85,03); 2,5248 (1,52); 2,5197 (2,26); 2,5115 (23,4); 2,5071 (46,59); 2,5026 (61,15); 2,498 (44,67); 2,4936 (22,22); 2,3681 (0,39); 2,3338 (0,33); 2,3292 (0,5); 2,3244 (0,49); 2,3117 (16); -0,0002 (3,22) |
| 23-21 | 4-(trifluormethyl)-phenyl | CH2 | CH2 | - | H | Verbindung Nr. 23-23, Solvent: [DMSO], Spektrometer: 399.95MHz8,5693 (0,36); 8,5553 (0,7); 8,5404 (0,34); 7,347 (5,6); 6,8086 (3,84); 3,332 (151,28); 3,331 (153,07); 3,2272 (0,51); 3,2132 (0,83); 3,2085 (0,68); 3,1993 (0,82); 3,1923 (0,66); 3,1855 (0,88); 3,1717 (0,57); 2,7881 (1,05); 2,7749 (0,77); 2,7669 (1,01); 2,7613 (0,8); 2,7471 (0,84); 2,6753 (0,45); 2,6708 (0,63); 2,6664 (0,45); 2,5412 (3,48); 2,524 (2,48); 2,5108 (37); 2,5063 (73,94); 2,5018 (96,97); 2,4972 (69,95); 2,4926 (33,72); 2,3331 (0,46); 2,3285 (0,63); 2,3238 (0,46); 2,2873 (16); 2,1811 (6,78); -0,0002 (5,92) |
| | | | | | | Verbindung Nr. 23-25, Solvent: [DMSO], Spektrometer: 399.95MHz |
| 23-22 | 2-methyl-phenyl | CH2 | CH2 | - | H | 8,4296 (0,87); 8,4161 (1,52); 8,4022 (0,85); 7,32 (2,13); 7,3161 (0,95); 7,3017 (5,36); 7,2883 (1,9); 7,2836 (5,53); 7,2716 (16); 7,2511 (4,62); 7,2472 (7,01); 7,2419 (1,67); 7,2277 (4,16); 7,2114 (1,12); 7,2062 (2,81); 7,2005 (0,7); 7,1921 (0,62); 7,1884 (0,99); 7,1849 (0,5); 3,4471 (2); 3,4294 (3,2); 3,415 (3,12); 3,4108 (3,25); 3,3959 (2,24); 3,3378 (122,84); 3,3346 (136,02); 3,3327 (135,44); 2,8256 (3,4); 2,8065 (4,9); 2,7886 (3,03); 2,6756 (0,49); 2,6711 (0,66); 2,6665 (0,49); 2,5414 (12,22); 2,5242 (2,74); 2,511 (39,9); 2,5065 (79,55); 2,502 (104,35); 2,4974 (75,24); 2,4928 (36,63); 2,3334 (0,48); 2,3287 (0,66); 2,324 (0,47); 2,074 (0,46); - 0,0002 (6,11) |
| | | | | | | Verbindung Nr. 23-12, Solvent: [DMSO], Spektrometer: 399.95MHz |
| 23-23 | 2,4,6-trimethyl-phenyl | CH2 | CH2 | - | H | 8,1803 (0,4); 8,1644 (0,81); 8,1492 (0,41); 7,5252 (2,25); 7,5195 (2,43); 7,4713 (1,46); 7,4496 (2,2); 7,3726 (1,46); 7,3667 (1,39); 7,351 (0,97); 7,3452 (0,92); 7,1644 (5,38); 3,7449 (2,75); 3,729 (2,72); 3,3766 (0,59); 3,3352 (262,2); 2,6755 (0,4); 2,6711 (0,56); 2,6669 (0,41); 2,5415 (52,18); 2,5244 (1,83); 2,511 (32,18); 2,5067 (65,15); 2,5021 (86,34); 2,4976 (63,79); 2,4932 (31,7); 2,3334 (0,38); 2,3289 (0,52); 2,3244 (0,38); 2,0742 (0,36); 1,4397 (16); -0,0002 (3,1) |
| | | | | | | Verbindung Nr. 23-13, Solvent: [DMSO], Spektrometer: 399.95MHz |
| 23-24 | 3,4-bismethoxy-phenyl | CH2 | CH2 | - | H | 15,3304 (0,91); 14,7843 (0,89); 8,4566 (1,23); 8,4447 (1,06); 7,8866 (0,91); 7,442 (2,27); 7,4235 (2,45); 7,4186 (2,68); 7,3595 (1,83); 7,3415 (2,82); 7,3077 (1,14); 7,2929 (2,85); 7,2887 (2,2); 7,2751 (14,86); 7,2598 (2,02); 7,2538 (1,87); 3,9437 (0,86); 3,7152 (1,16); 3,7111 (1,12); 3,6657 (1,33); 3,6279 (0,97); 3,6084 (1,08); 3,5888 (1,4); 3,5481 (1,58); 3,5292 (1,71); 3,4739 (3,46); 3,4572 (4,98); 3,4396 (5,96); 3,4243 (5,58); 3,3369 (4888,75); 3,2607 (2,51); 3,2253 (1,62); 3,2208 (1,54); 3,2035 (1,25); 3,1766 (0,88); 3,1355 (1,06); 3,0607 (1,03); 2,9574 (2,89); 2,9401 (4,58); 2,9213 (2,47); 2,7494 (1,02); 2,7109 (1,79); 2,6756 (11,72); 2,6711 (16); 2,6665 (11,63); 2,6487 (1,41); 2,6168 (1,28); 2,5979 (1,95); 2,5922 (1,93); 2,5414 (434,46); 2,5245 (53,58); 2,5111 (966,23); 2,5067 (1928,89); 2,5021 (2522,35); 2,4975 (1818,19); 2,493 (878,69); 2,4235 (1,24); 2,3848 (0,93); 2,3676 (1,3); 2,3334 (11,4); 2,3289 (15,19); 2,3244 (11,05); 2,2907 (1,19); 2,0739 (8,74); 1,4393 (0,91); 1,258 (1,52); 1,2445 (1,31); 1,2354 (2,36); 0,0079 (1,5); -0,0002 (37,15); - 3,2871 (0,87) |
| | | | | | | Verbindung Nr. 23-14, Solvent: [DMSO], Spektrometer: 399.95MHz |
| 23-25 | phenyl | CH2 | CH2 | - | H | 8,4017 (1,01); 8,3886 (1,96); 8,3744 (1,08); 7,7614 (0,52); 7,5594 (5,74); 7,5492 (0,49); 7,5389 (6,81); 7,5281 (5,8); 7,5232 (5,72); 7,2553 (3,23); 7,2503 (3,09); 7,2382 (16); 7,2297 (2,73); 3,4901 (0,39); 3,4648 (2,16); 3,4478 (5,1); 3,4331 (5,43); 3,4161 (2,82); 3,3398 (844,78); 3,2759 (1,01); 3,2373 (0,37); 3,1759 (0,33); 2,8477 (0,4); 2,8323 (3,6); 2,8151 (7,12); 2,7978 (3,15); 2,7117 (1,08); 2,6761 (1,6); 2,6714 (2,17); 2,667 (1,53); 2,6622 (0,74); 2,5924 (0,4); 2,5873 (0,45); 2,5749 (0,5); 2,542 (235,81); 2,5245 (7,84); 2,5113 (134,34); 2,507 (263,42); 2,5025 (342,6); 2,498 (247,23); 2,4936 (119,52); 2,368 (1); 2,3335 (1,56); 2,3293 (2,16); 2,3248 (1,56); 2,0742 (0,7); 1,2346 (0,41); 0,0074 (0,4); -0,0002 (10,23); - 0,0087 (0,32) |
| 23-26 | 4-chlor-phenyl | C(CH2-CH2) | CH2 | - | H | |
| 23-27 | 2,4-dichlor-phenyl | C(CH2-CH2) | CH2 | - | H | |
| 23-28 | 4-chlor-phenyl | CH2 | C(CH2-CH2) | - | H | |
| 23-29 | 2,4-dichlor-phenyl | CH2 | C(CH2-CH2) | - | H | |
| 23-30 | 4-chlor-phenyl | O | CH2 | CH2 | H | |
| 23-31 | 2,4-dichlor-phenyl | O | CH2 | CH2 | H | |
| 23-32 | 4-chlor-phenyl | CH2 | CH2 | CH2 | H | |
| 23-33 | 4-chlor-phenyl | NCH3 | CH2 | CH2 | H | |
| 23-34 | 2,4-dichlor-phenyl | NCH3 | CH2 | CH2 | H | |
| 23-35 | 4-chlor-phenyl | CH(OCH3) | CH2 | - | H | |
| 23-36 | 2,4-dichlor-phenyl | CH(OCH3) | CH2 | - | H | |
| | | | | | | Verbindung Nr. 23-37, Solvent: [DMSO], Spektrometer: 399.95MHz |
| 23-37 | 2-thienyl | CH2 | CH2 | - | H | 8,4975 (1,11); 8,4841 (1,96); 8,472 (1,09); 7,3549 (3,89); 7,3518 (3,79); 7,3421 (4,28); 7,3392 (3,91); 7,3007 (16); 6,9718 (2,98); 6,9632 (4,29); 6,9591 (2,89); 6,9505 (4); 6,9227 (4,15); 6,9144 (3); 3,4638 (2,26); 3,446 (5); 3,4315 (5,19); 3,4138 (2,67); 3,3944 (0,51); 3,3382 (270,42); 3,0456 (4,16); 3,0277 (7,37); 3,0099 (3,45); 2,7121 (0,82); 2,6758 (0,55); 2,6714 (0,73); 2,6673 (0,53); 2,5416 (179,54); 2,5249 (3,15); 2,5111 (46,29); 2,507 (88,09); 2,5025 (112,01); 2,4981 (80,99); 2,3682 (0,83); 2,3338 (0,53); 2,3293 (0,73); 2,325 (0,52); 2,074 (0,47); -0,0002 (5,87) |
| | | | | | | |
| 23-38 | 3-thienyl | CH2 | CH2 | - | H | |
| 23-39 | 2-furyl | CH2 | CH2 | - | H | |
| 23-40 | 3-furyl | CH2 | CH2 | - | H | |
| 23-41 | phenyl | CH2 | CH2 | CH(CH3) | H | |
| 23-42 | phenyl | CH2 | CH2 | CH2 | H | |
| 23-43 | 2-Cl-phenyl | CH2 | CH2 | CH(CH3) | H | |
| 23-44 | 4-t-butyl-phenyl | CH2 | CH2 | CH2 | H | |
| 23-45 | 4-methyl-phenyl | CH2 | CH2 | CH2 | H | |
| 23-46 | phenyl | CH2 | CH2 | CH(CH2CH3) | H | |
| 23-47 | 2-methoxy-phenyl | CH2 | CH2 | CH2 | H | |
| 23-48 | 2-methyl-phenyl | CH2 | CH2 | CH2 | H | |
| 23-49 | 3-methyl-phenyl | CH2 | CH2 | CH2 | H | |
| 23-50 | 3-chlor-phenyl | CH2 | CH2 | CH2 | H | |
| 23-51 | 2,6-difluor-phenyl | CH2 | CH2 | CH2 | H | |
| 23-52 | 4-chlor-phenyl | CH2 | CH2 | CH2 | H | |
| 23-53 | 2,6-dichlor-phenyl | CH2 | CH2 | CH(CH3) | H | |
| 23-54 | 3,5-dichlor-phenyl | CH2 | CH2 | CH(CH3) | H | |
| 23-55 | 2,6-dimethyl-phenyl | CH2 | CH2 | CH2 | H | |
| 23-56 | 2,5-dichlor-phenyl | CH2 | CH2 | CH2 | H | |
| 23-57 | 4-i-propoxy-phenyl | CH2 | CH2 | CH2 | H | |
| 23-58 | 3-trifluormethyl-phenyl | CH2 | CH2 | CH(CH3) | H | |
| 23-59 | 4-methyl-phenyl | CH2 | CH2 | CH(CH3) | H | |
| 23-60 | 2-methyl-phenyl | CH2 | CH2 | CH(CH3) | H | |
| 23-61 | 3,4-dichlor-phenyl | CH2 | CH2 | CH(CH3) | H | |
| 23-62 | 3,5-dichlor-phenyl | CH2 | CH2 | CH2 | H | |
| 23-63 | 2,6-dimethyl-phenyl | CH2 | CH2 | CH(CH3) | H | |
| 23-64 | 4-trifluormethyl-phenyl | CH2 | CH2 | CH(CH3) | H | |
| 23-65 | 2,5-dichlor-phenyl | CH2 | CH2 | CH(CH3) | H | |
| 23-66 | 4-phenoxy-phenyl | CH2 | CH2 | CH2 | H | |
| 23-67 | 3-chlor-phenyl | CH2 | CH2 | CH(CH3) | H | |
| 23-68 | 4-phenoxy-phenyl | CH2 | CH2 | CH(CH3) | H | |
| 23-69 | 2,4-dichlor-phenyl | CH2 | CH2 | CH(CH3) | H | |
| 23-70 | 2-difluormethoxy-phenyl | CH2 | CH2 | CH(CH3) | H | |
| 23-71 | 4-methoxy-phenyl | CH2 | CH2 | CH2 | H | |
| 23-72 | 4-chlor-phenyl | CH2 | CH2 | CH(CH3) | H | |
| 23-73 | 4-chlor-phenyl | CH2 | CH2 | CH(i-propyl) | H | |
| 23-74 | 4-fluor-phenyl | CH2 | CH2 | CH2 | H | |
| 23-75 | 4-chlor-phenyl | CH2 | CH2 | CH(n-propyl) | H | |
| 23-76 | 4-chlor-phenyl | CH2 | CH2 | CH(t-butyl) | H | |
| 23-77 | 2-chlor-phenyl | CH2 | CH2 | CH2 | H | |
| 23-78 | 4-chlor-phenyl | CH(CF3) | CH2 | - | H | Verbindung Nr. 23-78, Solvent: [DMSO], Spektrometer: 399.95MHz8,5276 (1,42); 8,5134 (2,6); 8,4994 (1,44); 7,4844 (5,04); 7,4793 (2,2); 7,4681 (3,4); 7,4628 (14,97); 7,4396 (11,51); 7,418 (4,25); 7,3903 (0,52); 7,1454 (16); 4,0563 (1,04); 4,0384 (3,55); 4,0207 (3,69); 4,0141 (1,46); 4,0026 (1,91); 3,9903 (1,1); 3,9757 (1,03); 3,9666 (0,39); 3,8754 (1,03); 3,8618 (1,75); 3,8479 (1,12); 3,8413 (1,72); 3,8277 (2,63); 3,8139 (1,32); 3,7372 (1,46); 3,7211 (1,66); 3,7147 (1,44); 3,7029 (1,39); 3,6988 (1,57); 3,6872 (1,08); 3,6805 (0,98); 3,6645 (0,84); 3,3233 (20,51); 2,6714 (0,38); 2,5247 (1,29); 2,5111 (22,28); 2,5069 (43,47); 2,5024 (56,41); 2,4979 (41,12); 2,4936 (20,4); 2,3292 (0,35); 1,9894 (11,92); 1,3361 (0,65); 1,2491 (0,63); 1,1929 (3,24); 1,1751 (6,4); 1,1572 (3,15); 0,0079 (0,43); -0,0002 (11,12); -0,0085 (0,43) |

**Tabelle 24**

| | | | | | | |
|---|---|---|---|---|---|---|
| Verbindungen der Formel I-24 | | | | | | |
| 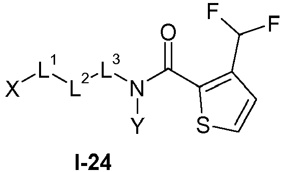 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| | | | | | | Verbindung Nr. 24-1, Solvent: [DMSO], Spektrometer: 399.95MHz |
| 24-1 | 4-chlor-phenyl | CH2 | CH2 | - | H | 8,6018 (1,23); 8,5881 (2,29); 8,5742 (1,19); 8,3155 (0,44); 7,802 (5,99); 7,789 (6,33); 7,4795 (2,37); 7,363 (1,02); 7,3567 (8,04); 7,3518 (3,4); 7,3466 (7,6); 7,3407 (8,93); 7,3354 (16); 7,2709 (1,7); 7,265 (10,87); 7,2603 (3,25); 7,2485 (2,58); 7,244 (6,87); 7,202 (2,52); 4,0379 (0,79); 4,0201 (0,82); 3,4588 (2,1); 3,4416 (4,42); 3,4268 (4,35); 3,4087 (2,3); 3,322 (68,28); 2,8414 (4,01); 2,8233 (7,02); 2,8055 (3,57); 2,6754 (0,49); 2,6708 (0,67); 2,6663 (0,49); 2,5241 (2,39); 2,5107 (38,3); 2,5063 (75,73); 2,5017 (98,94); 2,4972 (71,15); 2,4927 (34,13); 2,3328 (0,48); 2,3285 (0,66); 2,3239 (0,47); 1,9887 (3,59); 1,2494 (0,36); 1,1927 (0,97); 1,1749 (1,9); 1,1571 (0,94); 0,0079 (1,73); - 0,0002 (46,38); -0,0085 (1,59) |
| | | | | | | Verbindung Nr. 24-2, Solvent: [DMSO], Spektrometer: 399.95MHz |
| 24-2 | 2,4-dichlorphenyl | CH2 | CH2 | - | H | 8,6278 (1,72); 8,614 (3,26); 8,6001 (1,7); 7,8031 (6,94); 7,7901 (7,4); 7,5858 (6,65); 7,5818 (7,02); 7,4757 (2,83); 7,3787 (1,43); 7,3743 (1,11); 7,358 (9,35); 7,3502 (16); 7,3352 (11,46); 7,1982 (3); 6,5736 (1,09); 4,056 (0,95); 4,0383 (2,93); 4,0205 (2,96); 4,0027 (1); 3,4908 (2,48); 3,4738 (6,47); 3,4586 (6,59); 3,4419 (2,74); 3,3238 (55,1); 2,9641 (5,24); 2,9469 (10,17); 2,9296 (4,56); 2,6759 (0,35); 2,6714 (0,49); 2,6669 (0,37); 2,5068 (55,97); 2,5024 (72,04); 2,498 (53,39); 2,3334 (0,35); 2,3293 (0,47); 2,3246 (0,36); 1,9891 (12,66); 1,336 (1,08); 1,2494 (1,23); 1,2357 (0,52); 1,1932 (3,39); 1,1754 (6,64); 1,1576 (3,31); 0,007 (1,49); -0,0002 (29,67); -0,0083 (1,14) |
| | | | | | | Verbindung Nr. 24-3, Solvent: [DMSO], Spektrometer: 399.95MHz |
| 24-3 | 4-chlor-phenyl | CH(CH3) | CH2 | - | H | 8,5559 (1,46); 8,5421 (2,72); 8,5282 (1,43); 8,3164 (0,36); 7,7816 (6,22); 7,7687 (6,55); 7,4071 (2,5); 7,361 (7,75); 7,3564 (3,12); 7,3447 (4,12); 7,3398 (13,66); 7,3338 (2,76); 7,3284 (7,16); 7,3154 (6,56); 7,2825 (12,61); 7,2679 (7,05); 7,2613 (7,73); 7,1296 (2,65); 6,5742 (1,02); 4,0563 (0,82); 4,0385 (2,48); 4,0207 (2,49); 4,0029 (0,84); 3,4041 (0,39); 3,3888 (0,49); 3,3844 (0,57); 3,371 (2,43); 3,3568 (3,62); 3,3517 (3,53); 3,3445 (3,48); 3,3413 (3,79); 3,3368 (3,64); 3,3242 (30,15); 3,309 (0,73); 3,2946 (0,45); 3,0927 (1,33); 3,0748 (2,53); 3,0568 (2,41); 3,0387 (1,15); 2,6715 (0,35); 2,5112 (21,26); 2,5069 (40,11); 2,5024 (51,39); 2,4979 (37,55); 2,4937 (18,75); 2,329 (0,33); 1,9891 (10,58); 1,3365 (0,37); 1,2494 (0,52); 1,2165 (16); 1,199 (15,75); 1,1933 (4,88); 1,1752 (5,71); 1,1574 (2,85); 0,0077 (1,13); -0,0002 (23,1); -0,0083 (0,95) |
| | | | | | | Verbindung Nr. 24-4, Solvent: [DMSO], Spektrometer: 399.95MHz |
| 24-4 | 2,4-dichlorphenyl | CH(CH3) | CH2 | - | H | 8,7041 (2,05); 8,6909 (3,89); 8,6777 (2,01); 7,8323 (8,98); 7,8194 (9,47); 7,5715 (10,01); 7,5651 (10,66); 7,5505 (3,72); 7,4119 (7,89); 7,3842 (5,24); 7,3778 (5,14); 7,3672 (10,53); 7,3621 (7,84); 7,3549 (14,23); 7,2732 (3,88); 7,2404 (11,25); 7,2182 (8,36); 4,2266 (6,05); 4,212 (13,59); 4,1974 (6,57); 4,0559 (1,21); 4,0382 (3,71); 4,0203 (3,74); 4,0026 (1,25); 3,6341 (3,37); 3,6199 (9,51); 3,6057 (9,19); 3,5912 (3,13); 3,3231 (95,29); 2,6758 (0,64); 2,6712 (0,88); 2,6665 (0,65); 2,5243 (3,04); 2,511 (50,26); 2,5067 (98,59); 2,5022 (128,65); 2,4976 (93,43); 2,4932 (45,66); 2,3378 (0,32); 2,3333 (0,62); 2,3289 (0,86); 2,3244 (0,64); 1,989 (16); 1,3361 (0,39); 1,2495 (0,54); 1,1929 (4,3); 1,1751 (8,52); 1,1573 (4,17); 0,0079 (2,1); -0,0002 (54,16); -0,0085 (2,01) |
| | | | | | | Verbindung Nr. 24-5, Solvent: [DMSO], Spektrometer: 399.95MHz |
| 24-5 | 4-chlor-phenyl | CH2 | CH(CH3) | - | H | 8,4374 (2,62); 8,416 (2,65); 7,7917 (5,93); 7,7788 (6,24); 7,5632 (5,66); 7,5596 (5,45); 7,356 (0,94); 7,3351 (16); 7,3306 (8,14); 7,3206 (8,89); 7,3077 (6,33); 7,1817 (4,77); 7,0429 (2,38); 6,5736 (0,8); 4,3328 (0,51); 4,3112 (1,16); 4,2954 (1,47); 4,2803 (1,13); 4,2589 (0,54); 3,323 (43,65); 2,9677 (1,22); 2,954 (1,4); 2,9336 (3,47); 2,92 (3,2); 2,9025 (3,23); 2,8909 (0,69); 2,8803 (3,05); 2,8685 (1,27); 2,8463 (1,25); 2,6757 (0,32); 2,6714 (0,44); 2,6667 (0,34); 2,511 (26,45); 2,5068 (51,4); 2,5023 (66,93); 2,4978 (48,86); 2,4935 (24,19); 2,3333 (0,33); 2,3289 (0,44); 2,3246 (0,32); 1,989 (0,37); 1,2496 (0,43); 1,2182 (14,12); 1,2015 (13,92); 0,0079 (1,18); -0,0002 (28,35); -0,0085 (1,04) |
| | | | | | | Verbindung Nr. 24-6, Solvent: [DMSO], Spektrometer: 399.95MHz |
| 24-6 | 4-chlor-phenyl | C(CH3)2 | CH2 | - | H | 8,3673 (0,39); 8,3518 (0,77); 8,3362 (0,39); 7,7812 (1,95); 7,7682 (2,08); 7,4274 (2,01); 7,4223 (0,79); 7,411 (1,09); 7,4056 (4,35); 7,3995 (0,66); 7,372 (0,67); 7,3659 (4,47); 7,3605 (1,16); 7,3493 (0,84); 7,3441 (2,2); 7,317 (2,17); 7,3093 (0,93); 7,3042 (2,1); 7,1706 (1,64); 7,0318 (0,8); 6,5735 (0,4); 4,0558 (0,42); 4,038 (1,26); 4,0202 (1,28); 4,0024 (0,43); 3,4097 (2,7); 3,3939 (2,69); 3,3224 (17,03); 2,524 (0,59); 2,5107 (9,78); 2,5063 (19,27); 2,5018 (25,15); 2,4972 (18,14); 2,4928 (8,75); 1,9888 (5,57); 1,285 (16); 1,1928 (1,52); 1,175 (2,97); 1,1572 (1,55); 0,0079 (0,46); -0,0002 (11,85); -0,0085 (0,4) |
| | | | | | | Verbindung Nr. 24-7, Solvent: [DMSO], Spektrometer: 399.95MHz |
| 24-7 | 2,4-dichlorphenyl | C(CH3)2 | CH2 | - | H | 8,6818 (2,47); 8,6684 (4,54); 8,6551 (2,47); 7,8185 (12,63); 7,8055 (13,48); 7,5222 (5,31); 7,3835 (11,22); 7,3606 (14,19); 7,3533 (9,67); 7,3499 (12,63); 7,3478 (14,63); 7,3406 (9,98); 7,3376 (9,68); 7,2448 (5,58); 6,9705 (7,5); 6,962 (10,13); 6,9578 (7,23); 6,9493 (9,69); 6,9132 (8,67); 6,9111 (8,98); 6,9049 (6,64); 6,9028 (6,39); 3,4816 (4,69); 3,4639 (10,68); 3,4495 (10,85); 3,4317 (5,5); 3,323 (63,7); 3,0708 (8,93); 3,053 (16); 3,0351 (7,52); 2,6751 (0,55); 2,6707 (0,75); 2,6662 (0,55); 2,524 (2,42); 2,5106 (44,14); 2,5062 (87,93); 2,5016 (115,1); 2,4971 (82,78); 2,4926 (39,78); 2,3329 (0,56); 2,3284 (0,78); 2,3239 (0,56); 1,9886 (0,37); 1,336 (0,78); 1,2495 (0,91); 0,0079 (1,91); - 0,0002 (52,82); -0,0085 (1,77) |
| | | | | | | Verbindung Nr. 24-8, Solvent: [DMSO], Spektrometer: 399.95MHz |
| 24-8 | 3,4-dichlorphenyl | CH2 | CH2 | - | H | 8,5603 (1,57); 8,5466 (2,85); 8,5332 (1,49); 7,8117 (7,45); 7,7988 (7,82); 7,5533 (3,12); 7,4145 (6,59); 7,383 (0,33); 7,3533 (8,98); 7,3443 (10,3); 7,3402 (11,18); 7,3284 (4,49); 7,3234 (16); 7,3174 (2,28); 7,2755 (3,63); 7,2625 (13,91); 7,2414 (8,3); 4,038 (0,69); 4,0202 (0,69); 3,3222 (73,2); 3,2429 (2,71); 3,2257 (5,62); 3,2107 (5,55); 3,1934 (2,74); 2,6754 (0,68); 2,6709 (0,87); 2,6663 (0,65); 2,6258 (4,78); 2,6071 (7,35); 2,5876 (5,16); 2,524 (3,12); 2,5107 (48,34); 2,5063 (94,42); 2,5018 (122,69); 2,4973 (88,21); 2,4928 (42,39); 2,333 (0,58); 2,3286 (0,78); 2,3239 (0,57); 2,0667 (0,33); 1,9888 (2,99); 1,8358 (1,44); 1,8169 (3,97); 1,7987 (5,37); 1,7803 (3,69); 1,7621 (1,23); 1,4369 (0,46); 1,3357 (0,39); 1,2492 (0,46); 1,1927 (0,84); 1,175 (1,59); 1,1571 (0,8); 0,0079 (2,04); -0,0002 (52,95); - 0,0085 (1,83) |
| | | | | | | Verbindung Nr. 24-9, Solvent: [DMSO], Spektrometer: 399.95MHz |
| 24-9 | 2-thienyl | CH2 | CH2 | - | H | 8,3776 (0,43); 8,3623 (0,86); 8,3468 (0,43); 7,7684 (2); 7,7554 (2,12); 7,5356 (2,22); 7,5299 (2,35); 7,458 (1,47); 7,4363 (2,41); 7,3765 (1,58); 7,3707 (1,47); 7,3549 (0,98); 7,3491 (0,92); 7,3056 (2,23); 7,2926 (2,88); 7,1534 (1,71); 7,0145 (0,83); 6,5739 (0,4); 4,0563 (0,37); 4,0385 (1,12); 4,0207 (1,13); 4,0029 (0,38); 3,7595 (2,82); 3,7437 (2,79); 3,323 (9,07); 2,5246 (0,44); 2,5113 (7,45); 2,5069 (14,54); 2,5024 (18,86); 2,4979 (13,64); 2,4936 (6,65); 1,9892 (4,77); 1,5663 (0,69); 1,4346 (16); 1,1932 (1,33); 1,1754 (2,52); 1,1576 (1,26); 0,0078 (0,35); -0,0002 (8,32) |
| | | | | | | Verbindung Nr. 24-10, Solvent: [DMSO], Spektrometer: 399.95MHz |
| 24-10 | 2-chlor-phenyl | CH2 | CH2 | CH2 | H | 8,5971 (2,38); 8,5836 (4,51); 8,5697 (2,35); 7,8061 (10,93); 7,7931 (11,58); 7,7602 (0,42); 7,5523 (12,57); 7,5418 (0,63); 7,5318 (14,42); 7,5155 (11,52); 7,5106 (11,72); 7,4655 (4,46); 7,3466 (12,14); 7,3336 (12,09); 7,3268 (9,98); 7,2402 (6,79); 7,2352 (6,52); 7,2196 (5,99); 7,2146 (5,76); 7,1879 (4,76); 4,0564 (1,19); 4,0386 (3,65); 4,0208 (3,7); 4,003 (1,25); 3,4818 (3,86); 3,4647 (9,81); 3,4501 (10,06); 3,4331 (4,21); 3,3242 (46,13); 2,8593 (7,42); 2,8421 (14,89); 2,8247 (6,67); 2,6767 (0,51); 2,6718 (0,63); 2,6674 (0,46); 2,5415 (0,36); 2,5251 (1,98); 2,5117 (35,85); 2,5073 (69,98); 2,5028 (90,41); 2,4982 (64,7); 2,4938 (30,93); 2,334 (0,42); 2,3295 (0,57); 2,3251 (0,42); 1,9894 (16); 1,3366 (0,65); 1,2497 (0,78); 1,1932 (4,36); 1,1754 (8,58); 1,1576 (4,22); 0,0079 (1,68); -0,0002 (42,76); -0,0085 (1,37) |
| | | | | | | Verbindung Nr. 24-11, Solvent: [DMSO], Spektrometer: 399.95MHz |
| 24-11 | 2,4-dichlorphenyl | CH2 | CH2 | CH2 | H | 8,6095 (2,34); 8,5955 (4,48); 8,582 (2,35); 8,3162 (0,49); 7,8169 (11,27); 7,8039 (11,94); 7,5563 (4,65); 7,4997 (11,61); 7,4932 (12,31); 7,4605 (11,84); 7,4391 (15,84); 7,4175 (10,11); 7,3586 (12,53); 7,3456 (11,93); 7,3355 (0,47); 7,322 (8,17); 7,3154 (7,79); 7,3006 (6,11); 7,2941 (5,8); 7,2787 (4,99); 6,5741 (1,68); 4,0563 (1,21); 4,0385 (3,67); 4,0207 (3,69); 4,0029 (1,25); 3,3231 (73,71); 3,2869 (3,83); 3,2699 (9,1); 3,255 (9,1); 3,238 (3,93); 2,7451 (7,06); 2,7264 (9,23); 2,7065 (7,58); 2,6807 (0,4); 2,6763 (0,71); 2,6716 (0,94); 2,6671 (0,69); 2,5419 (0,61); 2,5249 (2,86); 2,5115 (51,05); 2,5071 (101,32); 2,5026 (132,53); 2,498 (95,7); 2,4936 (46,22); 2,3338 (0,63); 2,3293 (0,84); 2,3249 (0,62); 1,9893 (16); 1,8444 (2,01); 1,8263 (5,62); 1,8075 (7,18); 1,7888 (5,26); 1,7709 (1,74); 1,3363 (0,79); 1,2495 (0,93); 1,1932 (4,29); 1,1878 (0,55); 1,1754 (8,44); 1,1576 (4,18); 0,0079 (2,2); -0,0002 (60,79); -0,0085 (2,02) |
| | | | | | | Verbindung Nr. 24-12, Solvent: [DMSO], Spektrometer: 399.95MHz |
| 24-12 | 2,4-dichlorphenyl | O | CH2 | CH2 | H | 8,7049 (1,93); 8,6916 (3,77); 8,6782 (1,93); 8,3163 (0,55); 7,8328 (9,47); 7,8198 (10,06); 7,5718 (11,35); 7,5653 (12,08); 7,5587 (0,62); 7,551 (3,77); 7,4124 (8,13); 7,3844 (5,74); 7,378 (5,44); 7,3677 (10,83); 7,3623 (8,3); 7,3554 (15,58); 7,2738 (4); 7,2407 (11,98); 7,2184 (8,89); 4,2271 (6,07); 4,2124 (13,83); 4,1979 (6,63); 4,0561 (1,18); 4,0383 (3,64); 4,0205 (3,67); 4,0028 (1,23); 3,6346 (3,3); 3,6204 (9,39); 3,6062 (9,09); 3,5918 (3,08); 3,3238 (75,44); 2,676 (0,47); 2,6715 (0,66); 2,6669 (0,48); 2,5248 (2,05); 2,5199 (3,2); 2,5114 (38,35); 2,507 (76,68); 2,5024 (100,32); 2,4978 (71,87); 2,4933 (34,39); 2,3337 (0,49); 2,3292 (0,67); 2,3246 (0,49); 1,9893 (16); 1,3363 (0,46); 1,2495 (0,59); 1,193 (4,26); 1,1752 (8,46); 1,1574 (4,16); 0,0079 (1,73); -0,0002 (48,37); -0,0085 (1,56) |

**Tabelle 25**

| | | | | | | |
|---|---|---|---|---|---|---|
| Verbindungen der Formel I-25 | | | | | | |
| 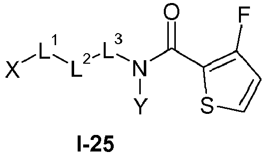 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| | | | | | | Verbindung Nr. 25-1, Solvent: [DMSO], Spektrometer: 399.95MHz |
| 25-1 | 4-chlor-phenyl | CH2 | CH2 | - | H | 7,9396 (1,73); 7,9311 (1,73); 7,7686 (4,15); 7,7583 (4,54); 7,7548 (4,19); 7,7446 (3,91); 7,365 (1,08); 7,3586 (9,94); 7,3537 (3,55); 7,3426 (4,21); 7,3375 (16); 7,3314 (2,15); 7,2699 (2,33); 7,2639 (13,49); 7,2589 (3,78); 7,2476 (3,25); 7,2428 (8,57); 7,2365 (0,98); 7,0835 (9,5); 7,0697 (9,18); 6,5721 (0,36); 5,7547 (1,61); 3,5072 (0,68); 3,476 (2,67); 3,4593 (5,26); 3,4407 (5,18); 3,425 (2,98); 3,41 (0,77); 3,3553 (461,2); 3,2932 (0,55); 2,8362 (5,17); 2,8174 (7,81); 2,7996 (4,57); 2,6778 (0,41); 2,6732 (0,57); 2,6687 (0,41); 2,5263 (1,92); 2,5216 (2,98); 2,5132 (32,03); 2,5087 (63,88); 2,5041 (83,46); 2,4995 (59,25); 2,4949 (27,68); 2,4805 (0,37); 2,3353 (0,41); 2,3308 (0,55); 2,3262 (0,38); 1,3357 (0,55); 1,2491 (0,75); 1,2344 (0,65); 0,0079 (2,41); -0,0002 (65,23); -0,0086 (1,89) |
| | | | | | | Verbindung Nr. 25-2, Solvent: [DMSO], Spektrometer: 399.95MHz |
| 25-2 | 2,4-dichlorphenyl | CH2 | CH2 | - | H | 8,0023 (2,51); 7,9943 (2,51); 7,7674 (5,03); 7,7572 (5,61); 7,7536 (5,21); 7,7434 (4,71); 7,5816 (8,7); 7,5771 (9,04); 7,3837 (2,16); 7,379 (1,59); 7,3631 (11,86); 7,3583 (13,1); 7,3537 (16); 7,3331 (2,5); 7,0815 (11,78); 7,0677 (11,39); 5,7565 (4,11); 3,5066 (3,17); 3,4897 (7,56); 3,4738 (7,79); 3,4568 (3,54); 3,3451 (371,93); 3,3111 (0,64); 3,298 (0,43); 2,9592 (7); 2,9415 (12,52); 2,9239 (6,12); 2,6778 (0,37); 2,6734 (0,5); 2,6689 (0,36); 2,5265 (1,86); 2,5132 (30,07); 2,5088 (58,71); 2,5043 (75,77); 2,4997 (53,82); 2,4952 (25,54); 2,3356 (0,35); 2,3311 (0,47); 2,3263 (0,33); 0,0079 (2,13); -0,0002 (51,9); -0,0085 (1,6) |
| | | | | | | Verbindung Nr. 25-3, Solvent: [DMSO], Spektrometer: 399.95MHz |
| 25-3 | 4-chlor-phenyl | CH2 | CH2 | CH2 | H | 7,9346 (2,57); 7,9275 (2,56); 7,7655 (6,73); 7,7552 (7,31); 7,7516 (6,79); 7,7414 (6,4); 7,2983 (5,13); 7,2944 (2,15); 7,2798 (13,24); 7,2661 (4,1); 7,2616 (12,84); 7,2272 (11,38); 7,2234 (16); 7,2182 (3,44); 7,2065 (8,26); 7,1937 (3,37); 7,1902 (4,43); 7,1867 (2,31); 7,177 (2,56); 7,1724 (6,78); 7,1671 (1,63); 7,1581 (1,5); 7,1545 (2,44); 7,1511 (1,21); 7,087 (14,65); 7,0798 (0,7); 7,0732 (14,16); 3,3639 (0,46); 3,334 (204,28); 3,3181 (0,97); 3,2764 (3,94); 3,2597 (7,93); 3,243 (7,99); 3,2262 (4,18); 2,6764 (0,38); 2,6719 (0,52); 2,6673 (0,38); 2,6238 (7,6); 2,605 (10,83); 2,5854 (8,23); 2,5253 (1,44); 2,5204 (2,26); 2,5119 (27,96); 2,5074 (56,26); 2,5028 (73,75); 2,4982 (52,42); 2,4936 (24,58); 2,3341 (0,34); 2,3296 (0,46); 2,3248 (0,34); 1,8486 (2,51); 1,8294 (5,98); 1,8112 (8,35); 1,7927 (5,49); 1,7743 (2,18); 1,2492 (0,35); 0,0079 (1,87); -0,0002 (54,32); -0,0086 (1,6) |
| | | | | | | Verbindung Nr. 25-4, Solvent: [DMSO], Spektrometer: 399.95MHz |
| 25-4 | 2,4-dichlorphenyl | CH2 | CH2 | CH2 | H | 7,9777 (2,85); 7,97 (2,81); 7,7691 (6,85); 7,7588 (7,37); 7,7553 (6,95); 7,745 (6,32); 7,7385 (0,53); 7,7343 (0,34); 7,7259 (0,51); 7,5633 (12,59); 7,5581 (12,78); 7,4329 (0,66); 7,4243 (7,37); 7,4036 (16); 7,3885 (0,4); 7,3741 (11,44); 7,3687 (10,37); 7,3595 (0,65); 7,3534 (5,03); 7,348 (4,95); 7,3385 (0,64); 7,3173 (0,85); 7,2599 (0,75); 7,2385 (0,4); 7,1372 (0,36); 7,0892 (15,24); 7,0823 (0,7); 7,0754 (14,69); 5,7565 (2,13); 3,4518 (0,35); 3,4312 (0,46); 3,422 (0,45); 3,4075 (0,72); 3,3843 (1,64); 3,3486 (493,97); 3,3207 (0,96); 3,3158 (1,08); 3,2995 (3,77); 3,2827 (8,56); 3,2668 (8,42); 3,2499 (3,93); 3,2301 (0,36); 2,742 (0,58); 2,7267 (7,48); 2,7082 (9,17); 2,6882 (7,67); 2,674 (0,81); 2,6694 (0,55); 2,6004 (0,42); 2,5804 (0,33); 2,5443 (0,47); 2,5272 (2,35); 2,5225 (3,46); 2,514 (37,08); 2,5095 (74,41); 2,5049 (97,4); 2,5002 (69,34); 2,4957 (32,5); 2,3362 (0,43); 2,3315 (0,6); 2,3271 (0,43); 1,8267 (2,3); 1,808 (5,65); 1,7897 (7,33); 1,7714 (5,07); 1,753 (1,8); 1,3364 (0,47); 1,2584 (0,33); 1,2492 (0,69); 0,1459 (0,33); 0,0079 (2,84); -0,0002 (79,17); -0,0086 (2,31) |

**Tabelle 26**

| | | | | | | |
|---|---|---|---|---|---|---|
| Verbindungen der Formel I-26 | | | | | | |
| 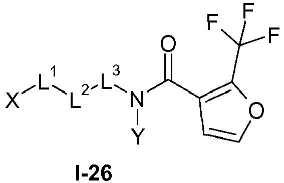 | | | | | | |

| **Bsp.-Nr.** | **X** | **L¹** | **L²** | **L³** | **Y** | **Physikalische Daten: ¹H-NMR, δ [ppm] oder CAS- bzw. Patent-Nr.** |
|---|---|---|---|---|---|---|
| 26-1 | 2,4-dichlor-phenyl | CH2 | CH2 | - | H | Verbindung Nr. 26-1, Solvent: [DMSO], Spektrometer: 399.95MHz |
| | | | | | | 8,6356 (1,81); 8,6217 (3,48); 8,6076 (1,81); 8,0013 (8,12); 7,9979 (8,15); 7,5901 (8,38); 7,5858 (8,97); 7,3883 (2,12); 7,3837 (1,45); 7,3676 (13,18); 7,3629 (15,31); 7,3594 (16); 7,3463 (0,38); 7,3393 (2,12); 6,9491 (6,58); 6,9467 (8,59); 6,9444 (8,51); 6,9422 (6,61); 3,4761 (3,16); 3,459 (7,5); 3,4439 (7,54); 3,4265 (3,54); 3,3616 (0,46); 3,334 (191,59); 3,3173 (1,15); 2,9412 (6,66); 2,9235 (12,39); 2,906 (5,9); 2,6728 (0,45); 2,5262 (1,26); 2,5215 (1,88); 2,5128 (25,55); 2,5083 (52,22); 2,5037 (68,89); 2,4991 (49,05); 2,4945 (23,19); 2,335 (0,33); 2,3304 (0,46); 2,3259 (0,32); 1,3365 (0,41); 1,2495 (0,54); 0,008 (0,8); -0,0002 (26,28); -0,0085 (0,81) |
| 26-2 | 4-chlor-phenyl | CH2 | CH2 | - | H | Verbindung Nr. 26-2, Solvent: [DMSO], Spektrometer: 399.95MHz |
| | | | | | | 8,6089 (1,36); 8,5953 (2,55); 8,5815 (1,37); 8,0014 (6,37); 7,998 (6,33); 7,3655 (1,01); 7,3591 (9,75); 7,3542 (3,36); 7,3431 (4,14); 7,338 (16); 7,3319 (2,07); 7,2734 (1,83); 7,2673 (13,57); 7,2623 (3,77); 7,251 (3,01); 7,2462 (8,58); 7,2399 (0,95); 6,9496 (5,06); 6,9473 (6,6); 6,945 (6,59); 6,9428 (5,15); 3,4476 (2,67); 3,4303 (5,32); 3,4155 (5,19); 3,3971 (2,96); 3,3249 (40,22); 2,8185 (5,02); 2,8002 (8,42); 2,7823 (4,48); 2,6715 (0,41); 2,5249 (1,11); 2,5202 (1,7); 2,5115 (22,62); 2,507 (45,83); 2,5024 (60,3); 2,4978 (42,9); 2,4932 (20,22); 2,3291 (0,38); 0,008 (0,66); -0,0002 (20,61); - 0,0085 (0,59) |
| 26-3 | 3,4-dichlor-phenyl | CH2 | CH2 | - | H | Verbindung Nr. 26-3, Solvent: [DMSO], Spektrometer: 399.95MHz |
| | | | | | | 8,5955 (2,06); 8,5818 (3,95); 8,5681 (2,09); 8,0021 (9,72); 7,9986 (9,6); 7,9976 (9,51); 7,5544 (13,94); 7,5442 (0,49); 7,5339 (16); 7,5233 (0,5); 7,5132 (11,75); 7,5082 (12,11); 7,2416 (6,86); 7,2365 (6,68); 7,221 (6,06); 7,2159 (5,93); 6,9274 (7,73); 6,925 (10,18); 6,9228 (10,16); 6,9205 (7,97); 5,7561 (2,11); 4,0387 (0,58); 4,0209 (0,58); 3,4721 (4,13); 3,4548 (10,04); 3,4401 (10,32); 3,4229 (4,64); 3,3479 (608,26); 3,3204 (1,46); 3,3026 (0,44); 3,2966 (0,34); 3,2852 (0,34); 2,834 (7,4); 2,8166 (14,78); 2,7992 (6,59); 2,6778 (0,54); 2,6732 (0,79); 2,6687 (0,58); 2,5434 (0,43); 2,5266 (2,49); 2,5218 (3,74); 2,5132 (41,84); 2,5087 (84,35); 2,5041 (110,41); 2,4995 (77,66); 2,4949 (35,75); 2,3355 (0,49); 2,3308 (0,68); 2,3262 (0,47); 1,9897 (2,48); 1,3361 (0,35); 1,2495 (0,47); 1,1932 (0,7); 1,1753 (1,37); 1,1576 (0,67); 0,008 (1,54); -0,0002 (43,91); -0,0086 (1,1) |
| 26-4 | 3,5-dichlor-phenyl | CH2 | CH2 | - | H | Verbindung Nr. 26-4, Solvent: [DMSO], Spektrometer: 399.95MHz |
| | | | | | | 8,5976 (1,31); 8,5838 (2,43); 8,5701 (1,28); 8,0077 (5,94); 8,0033 (6); 7,4448 (3,7); 7,44 (7,46); 7,4352 (4,21); 7,3247 (0,33); 7,3109 (16); 7,3062 (15,19); 6,9176 (4,53); 6,9154 (5,97); 6,9132 (6); 6,911 (4,73); 3,4838 (2,26); 3,4666 (6,04); 3,452 (6,26); 3,4351 (2,7); 3,3248 (23,55); 2,8469 (4,3); 2,8297 (8,79); 2,8125 (3,9); 2,6719 (0,37); 2,5252 (0,92); 2,5204 (1,42); 2,5119 (19,88); 2,5074 (40,56); 2,5028 (53,5); 2,4982 (38,14); 2,4936 (18,08); 2,3295 (0,34); 1,2495 (0,33); 0,008 (0,6); -0,0002 (19,31); -0,0085 (0,59) |
| 26-5 | 4-chlor-phenyl | CH2 | CH(CH3) | - | H | Verbindung Nr. 26-5, Solvent: [DMSO], Spektrometer: 399.95MHz |
| | | | | | | 8,4035 (2,3); 8,383 (2,29); 7,9889 (5,55); 7,9852 (5,61); 7,4624 (0,32); 7,3467 (0,88); 7,3405 (8,04); 7,3357 (2,76); 7,3244 (3,44); 7,3194 (13,07); 7,3134 (1,65); 7,2636 (0,54); 7,2573 (1,69); 7,2514 (11,57); 7,2465 (3,23); 7,2349 (2,61); 7,2303 (7,39); 6,9186 (5,51); 6,9165 (5,42); 4,1603 (0,65); 4,1435 (1,32); 4,1411 (1,35); 4,1242 (1,97); 4,1072 (1,44); 4,0882 (0,66); 3,3572 (252,39); 2,8167 (1,25); 2,7975 (1,19); 2,7831 (3,26); 2,7639 (3,28); 2,7494 (3,28); 2,7334 (3,32); 2,7158 (1,28); 2,6998 (1,15); 2,6739 (0,35); 2,5272 (0,99); 2,5224 (1,5); 2,514 (17,21); 2,5095 (34,45); 2,5049 (45,05); 2,5003 (32,11); 2,4958 (15,21); 1,9898 (0,39); 1,1307 (16); 1,114 (15,75); 1,1047 (1,19); 0,008 (0,56); -0,0002 (16,76); -0,0085 (0,5) |
| 26-6 | 4-chlor-phenyl | CH(CH3) | CH2 | - | H | Verbindung Nr. 26-6, Solvent: [DMSO], Spektrometer: 399.95MHz |
| | | | | | | 8,5641 (1,19); 8,5501 (2,3); 8,5357 (1,21); 7,9831 (5,35); 7,9793 (5,44); 7,3683 (0,77); 7,362 (7,64); 7,3572 (2,63); 7,3458 (3,37); 7,3408 (13,37); 7,3347 (1,79); 7,2877 (1,7); 7,2817 (12,3); 7,2766 (3,29); 7,2652 (2,54); 7,2604 (7,3); 7,2542 (0,82); 6,8923 (5,43); 6,8902 (5,39); 3,4008 (0,45); 3,39 (0,81); 3,3481 (342,01); 3,3232 (4,65); 3,3159 (3,57); 3,301 (0,89); 3,2867 (0,42); 3,2831 (0,5); 3,0364 (1,26); 3,0186 (2,51); 3,0008 (2,44); 2,983 (1,12); 2,6729 (0,43); 2,5261 (1,29); 2,5129 (25,33); 2,5084 (50,56); 2,5039 (66,04); 2,4993 (47,15); 2,4948 (22,49); 2,3306 (0,44); 1,2123 (16); 1,1948 (15,69); 0,0079 (0,74); -0,0002 (21,13); -0,0086 (0,65) |
| 26-7 | 2,4-dichlor-phenyl | CH2 | CH(CH3) | - | H | Verbindung Nr. 26-7, Solvent: [DMSO], Spektrometer: 399.95MHz |
| | | | | | | 8,4268 (2,05); 8,4054 (2,08); 7,9942 (4,7); 7,9907 (4,56); 7,9897 (4,56); 7,5685 (3,62); 7,5658 (6,64); 7,5628 (3,95); 7,3486 (16); 7,3455 (15,17); 6,9279 (3,66); 6,9256 (4,79); 6,9234 (4,82); 6,9211 (3,82); 5,7584 (2,25); 4,3007 (0,46); 4,28 (1,06); 4,2639 (1,41); 4,2481 (1,04); 4,2439 (0,82); 4,2271 (0,49); 3,3247 (22,34); 2,9385 (1,19); 2,9239 (1,34); 2,9043 (3,28); 2,8897 (3,03); 2,873 (3,14); 2,8521 (2,98); 2,8389 (1,19); 2,8179 (1,23); 2,5252 (0,91); 2,5204 (1,42); 2,5119 (17,27); 2,5074 (34,88); 2,5028 (45,79); 2,4982 (32,5); 2,4936 (15,27); 1,1769 (13,12); 1,1602 (12,99); 0,008 (0,5); -0,0002 (15,44); -0,0085 (0,44) |
| 26-8 | 2,4-dichlor-phenyl | CH(CH3) | CH2 | - | H | Verbindung Nr. 26-8, Solvent: [DMSO], Spektrometer: 399.95MHz |
| | | | | | | 8,5954 (1,56); 8,5809 (2,79); 8,5681 (1,33); 7,9821 (6,77); 7,9786 (6,71); 7,5619 (9,71); 7,5566 (10,19); 7,4648 (5,23); 7,4437 (12); 7,4164 (7,47); 7,411 (6,72); 7,3953 (3,13); 7,3899 (3,06); 6,903 (5,49); 6,9006 (7,06); 6,8984 (6,94); 6,8961 (5,32); 5,7569 (0,7); 4,0387 (0,47); 4,0209 (0,47); 3,5225 (1,18); 3,5056 (2,54); 3,4892 (3,95); 3,4812 (2,61); 3,4743 (2,68); 3,4651 (1,39); 3,4595 (3,14); 3,4445 (3,1); 3,4275 (1,6); 3,4021 (1,02); 3,3948 (1,27); 3,3789 (2,94); 3,364 (4,32); 3,3385 (331,19); 3,3212 (1,62); 3,3153 (0,82); 3,3007 (0,38); 2,6772 (0,36); 2,6727 (0,51); 2,6681 (0,35); 2,526 (1,63); 2,5213 (2,4); 2,5127 (28,94); 2,5082 (58,45); 2,5036 (76,65); 2,499 (54,08); 2,4944 (25,12); 2,3349 (0,36); 2,3304 (0,51); 2,3257 (0,34); 1,9896 (2,1); 1,4201 (0,47); 1,2493 (0,42); 1,2053 (15,95); 1,1967 (3,74); 1,1887 (16); 1,1753 (1,61); 1,1575 (0,67); 0,008 (0,93); -0,0002 (30,12); -0,0086 (0,87) |
| 26-9 | 2,6-difluor-phenyl | CH2 | CH2 | - | H | Verbindung Nr. 26-9, Solvent: [DMSO], Spektrometer: 399.95MHz |
| | | | | | | 8,7131 (2,65); 8,6985 (5,01); 8,6839 (2,65); 7,9991 (12,83); 7,9957 (12,64); 7,3654 (1,77); 7,3486 (3,86); 7,3444 (3,34); 7,3316 (2,64); 7,3276 (7,45); 7,3237 (2,99); 7,3104 (3,53); 7,3067 (4,8); 7,29 (2,26); 7,1033 (0,94); 7,0989 (1,33); 7,0869 (9,95); 7,0787 (1,84); 7,0669 (16); 7,0558 (1,94); 7,0468 (8,52); 7,0344 (1,18); 7,0311 (0,88); 6,9198 (10,15); 6,9174 (13,27); 6,9152 (13,21); 6,9129 (10,25); 3,4278 (4,8); 3,4111 (11,75); 3,3947 (11,69); 3,3777 (5,37); 3,3244 (43,4); 2,8847 (7,35); 2,867 (13,67); 2,8495 (6,6); 2,6765 (0,57); 2,6718 (0,76); 2,6672 (0,53); 2,5252 (2,1); 2,5205 (3,31); 2,5119 (43,26); 2,5074 (87,58); 2,5028 (114,99); 2,4981 (81,4); 2,4936 (38,07); 2,334 (0,54); 2,3295 (0,75); 2,325 (0,53); 1,3365 (0,65); 1,2498 (0,82); 0,008 (1,28); -0,0002 (40,16); -0,0085 (1,17) |
| 26-10 | 4-chlor-phenyl | CH2 | CH2 | CH2 | H | Verbindung Nr. 26-10, Solvent: [DMSO], Spektrometer: 399.95MHz |
| | | | | | | 8,56 (1,9); 8,5465 (3,39); 8,5334 (1,89); 8,0103 (9,73); 8,0068 (9,54); 8,0058 (9,4); 7,305 (5,4); 7,3011 (2,24); 7,2865 (13,19); 7,2728 (4,06); 7,2683 (12,59); 7,2269 (10,76); 7,2231 (16); 7,218 (3,55); 7,2061 (8,78); 7,197 (4,95); 7,1934 (2,47); 7,1837 (2,59); 7,179 (6,85); 7,1738 (1,68); 7,1645 (1,55); 7,161 (2,54); 7,1576 (1,31); 7,0007 (7,99); 6,9983 (10,42); 6,9961 (10,34); 6,9938 (8); 3,3236 (48,99); 3,2409 (4,43); 3,2233 (8,88); 3,2088 (8,84); 3,1912 (4,61); 2,6755 (0,59); 2,6709 (0,79); 2,6663 (0,58); 2,6306 (7,35); 2,6119 (10,08); 2,5921 (8,06); 2,5412 (0,37); 2,5244 (2,29); 2,5197 (3,47); 2,511 (42,44); 2,5065 (86); 2,5019 (112,99); 2,4973 (79,68); 2,4927 (36,96); 2,3333 (0,52); 2,3287 (0,74); 2,3241 (0,52); 1,8216 (2,43); 1,8026 (6); 1,7843 (8,01); 1,7661 (5,51); 1,7476 (2,14); 1,3361 (0,52); 1,2493 (0,69); 0,0079 (1,24); -0,0002 (38,22); -0,0086 (1,09) |
| 26-11 | 2,4-dichlor-phenyl | CH2 | CH2 | CH2 | H | Verbindung Nr. 26-11, Solvent: [DMSO], Spektrometer: 399.95MHz |
| | | | | | | 8,6033 (1,98); 8,5896 (3,67); 8,5759 (1,96); 8,0134 (9,34); 8,0098 (9,31); 7,5714 (10,58); 7,5663 (10,83); 7,4191 (5,24); 7,3984 (16); 7,3837 (11,47); 7,3786 (10,33); 7,3631 (3,36); 7,3579 (3,54); 7,3482 (0,4); 7,327 (0,57); 7,2584 (0,5); 7,2376 (0,41); 6,997 (7,66); 6,9948 (9,64); 6,9926 (9,29); 6,9905 (6,99); 3,3697 (0,48); 3,3575 (0,88); 3,3347 (213,35); 3,3149 (0,73); 3,2704 (3,55); 3,2532 (8,1); 3,2382 (8,13); 3,2211 (3,66); 2,7359 (6,63); 2,7171 (7,91); 2,6972 (6,97); 2,6779 (0,46); 2,6733 (0,58); 2,6687 (0,42); 2,5266 (1,71); 2,5218 (2,64); 2,5133 (31,11); 2,5088 (62,1); 2,5043 (80,8); 2,4996 (56,99); 2,4951 (26,48); 2,3354 (0,36); 2,331 (0,51); 2,3264 (0,36); 1,8044 (1,96); 1,786 (4,98); 1,7673 (6,47); 1,7488 (4,67); 1,7306 (1,64); 1,3368 (0,41); 1,2495 (0,52); 0,008 (0,87); -0,0002 (27,33); -0,0085 (0,8) |
| 26-12 | 2,6-difluor-phenyl | CH2 | CH2 | CH2 | H | Verbindung Nr. 26-12, Solvent: [DMSO], Spektrometer: 399.95MHz |
| | | | | | | 8,5982 (2,65); 8,5846 (4,8); 8,5711 (2,62); 8,0104 (12,76); 8,0069 (12,58); 7,3503 (1,8); 7,3334 (3,92); 7,3292 (3,29); 7,3164 (2,73); 7,3125 (7,46); 7,3085 (2,99); 7,2952 (3,48); 7,2916 (4,87); 7,2748 (2,28); 7,1031 (1,07); 7,0986 (1,37); 7,0862 (9,78); 7,0784 (1,87); 7,0659 (16); 7,054 (1,96); 7,0457 (8,06); 7,0326 (1,09); 7,0292 (0,81); 6,9798 (10,33); 6,9775 (13,21); 6,9752 (12,93); 6,9729 (9,92); 6,514 (0,37); 3,328 (26,54); 3,2621 (4,91); 3,2446 (9,9); 3,2293 (9,86); 3,2118 (5,04); 2,6849 (6,61); 2,6657 (10,52); 2,6462 (7,04); 2,527 (1,7); 2,5222 (2,59); 2,5137 (30,04); 2,5092 (59,96); 2,5046 (78,2); 2,5 (55,19); 2,4954 (25,66); 2,336 (0,36); 2,3314 (0,5); 2,3266 (0,35); 1,7839 (2,64); 1,7649 (6,6); 1,7464 (9,32); 1,7277 (6,07); 1,7091 (2,29); 1,3377 (0,59); 1,2503 (0,76); 0,008 (0,96); -0,0002 (28,5); -0,0086 (0,8) |

### Biologische Experimente

### Beispiel A

### Nippostrongylus brasiliensis-Test (NIPOBR)

### Testverfahren für in vitro Experimente mit Nippostrongylus brasiliensis

Adulte *Nippostrongylus brasiliensis* wurden aus dem Dünndarm weiblicher Wistar-Ratten isoliert, in 0.9% NaCl transferiert, das 20 µg/ml Sisomycin und 2 µg/ml Canesten enthielt. Die Inkubation der Würmer (beide Geschlechter) wurde in 1.0 ml Medium durchgeführt, das auch für die Bestimmung der Acetylcholinesterase-Aktivität verwendet wurde. Die Verbindungen wurden in DMSO gelöst und dem Inkubationsmedium zugefügt, sodass Endkonzentrationen von 100, 10 bzw. 1 µg/ml vorhanden waren. Die Kontrollen enthielten nur DMSO. Die Inkubation und die Enzymbestimmung wurden in der Arbeit von Rapson et al. (1987) Z. Parasitenkunde 73, 190-191 beschrieben.

Die Einteilung der Wirksamkeit erfolgte in den Kategorien 35 (0-35% Hemmung), 60 (>35-60%), 84 (>60-84%) und 100 (>84-100%).

| Beispiel-Nummer | Wirksamkeit bei 10 µg/ml |
|---|---|
| 1-2 | 100% |
| 1-7 | 100% |
| 1-14 | 100% |
| 1-20 | 100% |
| 1-22 | 100% |
| 2-3 | 100% |
| 2-7 | 100% |
| 2-8 | 100% |
| 2-9 | 100% |
| 2-11 | 100% |
| 2-12 | 100% |
| 2-16 | 100% |
| 2-19 | 100% |
| 2-20 | 100% |
| 2-23 | 100% |
| 3-4 | 100% |
| 3-9 | 100% |
| 3-11 | 100% |
| 3-14 | 100% |
| 3-15 | 100% |
| 3-19 | 100% |
| 3-20 | 100% |
| 3-21 | 100% |
| 4-3 | 100% |
| 4-5 | 100% |
| 6-7 | 100% |
| 13-4 | 100% |
| 14-4 | 100% |
| 20-22 | 100% |

### Beispiel B

### In-vivo Nematodentest

### Heligmosomoides polygyrus / Maus

Experimentell mit *Heligmosomoides polygyrus* infizierte Mäuse wurden nach Ablauf der Präpatenzzeit des Parasiten behandelt. Die Wirkstoffe wurden in einem Cremophor EL/Wassergemisch oral appliziert.

Der Wirkungsgrad wird dadurch bestimmt, dass bei der Sektion der Mäuse die Würmer im Darm ausgezählt werden. Die Wirksamkeit wird über die Wurmzahl in Relation zur Anzahl der Würmer im Darm unbehandelter Kontrolltiere bestimmt.

Geprüfte Wirkstoffe und wirksame Dosierungen (Dosis effectiva) sind aus der nachfolgenden Tabelle ersichtlich.

| Substanz | Wirksamkeit bei 100 mg/kg |
|---|---|
| 4-3 | 100% |
| 13-4 | 100% |
| 14-2 | 100% |
| 17-37 | 100% |

### Beispiel C

### In-vivo Nematodentest

### Haemonchus contortus / Schaf

Experimentell mit *Haemonchus contortus* infizierte Schafe wurden nach Ablauf der Präpatenzzeit des Parasiten behandelt. Die Wirkstoffe wurden als reiner Wirkstoff oral und/oder intramuskulär appliziert.

Der Wirkungsgrad wird dadurch bestimmt, dass bei der Sektion der Schafe die Würmer im Labmagen ausgezählt werden. Die Wirksamkeit wird über die Wurmzahl in Relation zur Anzahl der Würmer im Labmagen unbehandelter Kontrolltiere bestimmt.

Geprüfte Wirkstoffe und wirksame Dosierungen (Dosis effectiva) sind aus der nachfolgenden Tabelle ersichtlich.

| Substanz | Dosierung | Wirksamkeit |
|---|---|---|
| 13-4 | 20 mg/kg | 81% |
| 14-4 | 100 mg/kg | 100% |

### Beispiel D

### Haemonchus contortus-Test (HAEMCO)

### Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit "Ringerlösung" auf die gewünschte Konzentration. Gefäße mit der Wirkstoffzubereitung der gewünschten Konzentration werden mit ca. 40 *Haemonchus contortus* Larven besetzt.

Nach 5 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100 ppm: 20-26, 20-29

Bei diesem Test zeigen z.B. die folgenden Verbindungen der Herstellungsbeispiele eine Wirkung von 90% bei einer Aufwandmenge von 20 ppm: 13-4

Bei diesem Test zeigen z.B. die folgenden Verbindungen der Herstellungsbeispiele eine Wirkung von 80% bei einer Aufwandmenge von 20 ppm: 14-2, 14-4

### Cooperia curticei-Test (COOPCU)

### Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit "Ringerlösung" auf die gewünschte Konzentration. Gefäße mit der Wirkstoffzubereitung der gewünschten Konzentration werden mit ca. 40 *Cooperia curticei* Larven besetzt.

Nach 5 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100 ppm: 20-26

Bei diesem Test zeigen z.B. die folgenden Verbindungen der Herstellungsbeispiele eine Wirkung von 80% bei einer Aufwandmenge von 100 ppm: 17-37

Bei diesem Test zeigen z.B. die folgenden Verbindungen der Herstellungsbeispiele eine Wirkung von 90% bei einer Aufwandmenge von 20 ppm: 14-2, 14-4, 13-4

Bei diesem Test zeigen z.B. die folgenden Verbindungen der Herstellungsbeispiele eine Wirkung von 80% bei einer Aufwandmenge von 20 ppm: 20-29

## Patentansprüche

1. Verbindungen gemäß Formel (I) 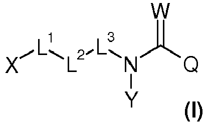 in welcher
X für ein ein- oder mehrfach durch M¹ substituiertes Phenyl, Thienyl oder Furanyl steht;
Q für ein ein- oder mehrfach durch M¹ substituiertes Pyridyl, Thienyl, Furanyl oder Isothiazolyl steht;
Y für Wasserstoff oder für gegebenenfalls ein- oder mehrfach durch M² substituiertes (C₁-C₁₀)-Alkyl, (C₂-C₁₀)-Alkenyl, (C₂-C₁₀)-Alkinyl, (C₁-C₁₀)-Halogenalkyl, (C₂-C₁₀)-Halogenalkenyl, (C₂-C₁₀)-Halogenalkinyl, (C₁-C₁₀)-Alkoxy, (C₂-C₁₀)-Alkenyloxy, (C₃-C₁₀)-Alkinyloxy, (C₃-C₁₄)-Cycloalkyl-(C₁-C₁₀)-alkyl oder für eine gegebenenfalls ein- oder mehrfach durch M² substituierte 3- bis 14-gliedrige cyclische Gruppe steht;
W für Sauerstoff oder Schwefel steht;
L¹ für -C(R¹¹,R¹²)-, Sauerstoff, Schwefel, oder -N(R¹)- steht;
L² für -C(R²¹,R²²)- steht;
L³ für -C(R³¹,R³²)- oder Direktbindung steht;
M¹ für Wasserstoff, Halogen, Cyano, Nitro, OH, (C₁-C₁₀)-Alkyl, (C₁-C₁₀)-Halogenalkyl, (C₁-C₁₀)-Alkoxy, (C₁-C₁₀)-Halogenalkoxy, (C₁-C₁₀)-Alkylthio, (C₁-C₁₀)-Halogenalkylthio, (C₁-C₁₀)-Alkylsulfonyl, (C₁-C₁₀)-Halogenalkylsulfonyl, (C₁-C₁₀)-Alkylsulfanyl, (C₁-C₁₀)-Halogenalkylsulfanyl, (3- bis 14-gliedrige cyclische Gruppe)-O- steht;
R¹ für Wasserstoff oder gegebenenfalls ein- oder mehrfach durch M² substituiertes (C₁-C₁₀)-Alkyl, (C₂-C₁₀)-Alkenyl, (C₂-C₁₀)-Alkinyl, (C₁-C₁₀)-Halogenalkyl, (C₂-C₁₀)-Halogenalkenyl, (C₂-C₁₀)-Halogenalkinyl, (C₃-C₁₀)-Cycloalkyl-(C₁-C₁₀)-alkyl oder für eine gegebenenfalls ein- oder mehrfach durch M² substituierte 3- bis 14-gliedrige cyclische Gruppe steht;
R¹¹, R¹² für jeweils unabhängig voneinander Wasserstoff, Halogen oder gegebenenfalls ein- oder mehrfach durch M² substituiertes (C₁-C₁₀)-Alkyl, (C₂-C₁₀)-Alkenyl, (C₂-C₁₀)-Alkinyl, (C₁-C₁₀)-Halogenalkyl, (C₂-C₁₀)-Halogenalkenyl, (C₂-C₁₀)-Halogenalkinyl, (C₁-C₁₀)-Alkoxy, (C₂-C₁₀)-Alkenyloxy, (C₃-C₁₀)-Alkinyloxy, (C₃-C₁₄)-Cycloalkyl-(C₁-C₁₀)-alkyl oder für eine gegebenenfalls ein- oder mehrfach durch M² substituierte 3- bis 14-gliedrige cyclische Gruppe steht;
R¹¹, R¹² zusammen für eine jeweils gegebenenfalls ein- oder mehrfach durch M² substituierte spiroverknüpfte 3- bis 14-gliedrige carbo- oder 3- bis 10-gliedrige heterocyclische Gruppe stehen;
R²¹, R²² für jeweils unabhängig voneinander Wasserstoff, Halogen oder gegebenenfalls ein- oder mehrfach durch M² substituiertes (C₁-C₁₀)-Alkyl, (C₂-C₁₀)-Alkenyl, (C₂-C₁₀)-Alkinyl, (C₁-C₁₀)-Halogenalkyl, (C₂-C₁₀)-Halogenalkenyl, (C₂-C₁₀)-Halogenalkinyl, (C₁-C₁₀)-Alkoxy, (C₁-C₁₀)-Halogenalkoxy, (C₂-C₁₀)-Alkenyloxy, (C₃-C₁₀)-Alkinyloxy, (C₃-C₁₄)-Cycloalkyl-(C₁-C₁₀)-alkyl oder für eine gegebenenfalls ein- oder mehrfach durch M² substituierte 3- bis 14-gliedrige cyclische Gruppe steht;
R²¹, R²² zusammen für eine jeweils gegebenenfalls ein- oder mehrfach durch M² substituierte spiroverknüpfte 3- bis 14-gliedrige carbo- oder 3- bis 10-gliedrige heterocyclische Gruppe stehen;
R³¹, R³² für jeweils unabhängig voneinander Wasserstoff oder gegebenenfalls ein- oder mehrfach durch M² substituiertes (C₁-C₁₀)-Alkyl, (C₂-C₁₀)-Alkenyl, (C₂-C₁₀)-Alkinyl, (C₁-C₁₀)-Halogenalkyl, (C₂-C₁₀)-Halogenalkenyl, (C₂-C₁₀)-Halogenalkinyl, (C₃-C₁₄)-Cycloalkyl-(C₁-C₁₀)-alkyl oder für eine gegebenenfalls ein- oder mehrfach durch M² substituierte 3- bis 14-gliedrige cyclische Gruppe steht;
R³¹, R³² zusammen für eine jeweils gegebenenfalls ein- oder mehrfach durch M² substituierte spiroverknüpfte 3- bis 14-gliedrige carbo- oder 3- bis 10-gliedrige heterocyclische Gruppe stehen;
M² für jeweils unabhängig voneinander für Halogen, Formyl, Cyano, Nitro, (C₁-C₁₀)-Alkyl, (C₁-C₁₀)-Halogenalkyl, (C₂-C₁₀)-Alkenyl, (C₂-C₁₀)-Halogenalkenyl, (C₂-C₁₀)-Alkinyl, (C₂-C₁₀)-Halogenalkinyl, (C₁-C₁₀)-Alkoxy, (C₁-C₁₀)-Halogenalkoxy, (C₂-C₁₀)-Alkenyloxy, (C₂-C₁₀)-Halogenalkenoxy, (C₃-C₁₀)-Alkinyloxy, (C₃-C₁₀)-Halogenalkinoxy, (C₁-C₁₀)-Alkylthio, (C₁-C₁₀)-Halogenalkylthio, (C₂-C₁₀)-Alkenylthio, (C₂-C₁₀)-Halogenalkenylthio, (C₃-C₁₀)-Alkinylthio, (C₃-C₁₀)-Halogenalkinylthio, (C₁-C₁₀)-Alkylsulfonyl, (C₁-C₁₀)-Halogenalkylsulfonyl, (C₂-C₁₀)-Alkenylsulfonyl, (C₂-C₁₀)-Halogenalkenylsulfonyl, (C₃-C₁₀)-Alkinylsulfonyl, (C₃-C₁₀)-Halogenalkinylsulfonyl, (C₁-C₁₀)-Alkylsulfanyl, (C₁-C₁₀)-Halogenalkylsulfanyl, (C₂-C₁₀)-Alkenylsulfanyl, (C₂-C₁₀)-Halogenalkenylsulfanyl, (C₃-C₁₀)-Alkinylsulfanyl, (C₃-C₁₀)-Halogenalkinylsulfanyl, (C₁-C₁₀)-Alkylcarbonyl, (C₁-C₁₀)-Halogenalkylcarbonyl, (C₂-C₁₀)-Alkenylcarbonyl, (C₂-C₁₀)-Halogenalkenylcarbonyl, (C₂-C₁₀)-Alkinylcarbonyl, (C₂-C₁₀)-Halogenalkinylcarbonyl, (C₁-C₁₀)-Alkoxycarbonyl, (C₁-C₁₀)-Halogenalkoxycarbonyl, (C₂-C₁₀)-Alkenoxycarbonyl, (C₂-C₁₀)-Halogenalkenoxycarbonyl, (C₃-C₁₀)-Alkinoxycarbonyl, (C₃-C₁₀)-Halogenalkinoxycarbonyl, (C₁-C₁₀)-Alkylcarbonyloxy, (C₁-C₁₀)-Halogenalkylcarbonyloxy, (C₂-C₁₀)-Alkenylcarbonyloxy, (C₂-C₁₀)-Halogenalkenylcarbonyloxy, (C₂-C₁₀)-Alkinylcarbonyloxy, (C₂-C₁₀)-Halogenalkinylcarbonyloxy, 3 bis 14-gliedrige cyclische Gruppe steht;
sowie deren Salze, N-Oxide und tautomere Formen;
zur Verwendung als Arzneimittel zur Bekämpfung von Endoparasiten bei Tieren oder Menschen .

2. Verbindungen gemäß Anspruch 1,
wobei
X für ein ein- bis mehrfach durch M¹ substituiertes Phenyl 2-Thienyl, 3-Thienyl, 2-Furanyl oder 3-Furanyl steht;
Q für ein- oder mehrfach durch M¹ substituiertes 2-Thienyl, 3- Thienyl, 2-Furanyl, 5-Isothiazolyl, 3-Pyridyl oder 2-Pyridyl steht;
Y für Wasserstoff oder für gegebenenfalls ein- oder mehrfach durch M² substituiertes (C₁-C₁₀)-Alkyl, (C₂-C₁₀)-Alkenyl, (C₂-C₁₀)-Alkinyl, (C₁-C₁₀)-Halogenalkyl, (C₂-C₁₀)-Halogenalkenyl, (C₂-C₁₀)-Halogenalkinyl, (C₁-C₁₀)-Alkoxy, (C₂-C₁₀)-Alkenyloxy, (C₃-C₁₀)-Alkinyloxy, (C₃-C₁₄)-Cycloalkyl-(C₁-C₁₀)-alkyl oder für eine gegebenenfalls ein- oder mehrfach durch M² substituierte 3- bis 14-gliedrige cyclische Gruppe steht;
L¹ für -C(R¹¹,R¹²)-, Sauerstoff oder -N(R¹)- steht;
M¹ für Wasserstoff, Halogen, Cyano, Nitro, OH, (C₁-C₆)-Alkyl, (C₁-C₆)-Halogenalkyl, (C₁-C₆)-Alkoxy, (C₁-C₆)-Halogenalkoxy, (C₁-C₆)-Alkylthio, (C₁-C₆)-Halogenalkylthio, (C₁-C₆)-Alkylsulfonyl, (C₁-C₆)-Halogenalkylsulfonyl, (C₁-C₆)-Alkylsulfanyl, (C₁-C₆)-Halogenalkylsulfanyl, (C₃-C₁₄)-Cycloalkyl-O-, (C₃-C₁₄)-Cycloalkenyl-O-, (C₆-C₁₄)-Aryl-O-, halogeniertes (C₃-C₁₄)-Cycloalkyl-O-, halogeniertes (C₃-C₁₄)-Cycloalkenyl-O-, halogeniertes (C₆-C₁₄)-Aryl)-O-steht;
R¹ für Wasserstoff oder gegebenenfalls ein- oder mehrfach durch M² substituiertes (C₁-C₄)-Alkyl, (C₂-C₄)-Alkenyl, (C₂-C₄)-Alkinyl, (C₁-C₄)-Halogenalkyl, (C₂-C₄)-Halogenalkenyl, (C₂-C₄)-Halogenalkinyl, (C₃-C₄)-Cycloalkyl-(C₁-C₁₀)-alkyl oder für eine gegebenenfalls ein- oder mehrfach durch M² substituierte (C₃-C₈)-Cycloalkyl oder halogeniertes (C₃-C₈)-Cycloalkyl steht;
R¹¹, R¹² jeweils unabhängig voneinander für Wasserstoff, Fluor oder gegebenenfalls ein- oder mehrfach durch M² substituiertes (C₁-C₆)-Alkyl, (C₁-C₆)-Halogenalkyl, (C₁-C₆)-Alkoxy, (C₃-C₆)-Cycloalkyl-(C₁-C₆)-alkyl, (C₃-C₈)-Cycloalkyl oder halogeniertes (C₃-C₈)-Cycloalkyl steht;
R¹¹, R¹² für C(R¹¹, R¹²) als spiro-C(CH₂-CH₂) steht;
R²¹, R²² jeweils unabhängig voneinander für Wasserstoff, Fluor oder gegebenenfalls ein- oder mehrfach durch M² substituiertes (C₁-C₄)-Alkyl, (C₂-C₄)-Alkenyl, (C₂-C₄)-Alkinyl, (C₁-C₄)-Halogenalkyl, (C₂-C₄)-Halogenalkenyl, (C₂-C₄)-Halogenalkinyl, (C₁-C₆)-Alkoxy, (C₁-C₄)-Halogenalkoxy, (C₂-C₄)-Alkenyloxy, (C₃-C₄)-Alkinyloxy, (C₃-C₄)-Cycloalkyl-(C₁-C₄)-alkyl, (C₃-C₈)-Cycloalkyl oder halogeniertes (C₃-C₈)-Cycloalkyl steht;
R²¹, R²² für C(R²¹, R²²) als spiro-C(CH₂-CH₂) steht;
R³¹, R³² jeweils unabhängig voneinander für Wasserstoff oder gegebenenfalls ein- oder mehrfach durch M² substituiertes (C₁-C₄)-Alkyl, (C₂-C₄)-Alkenyl, (C₂-C₄)-Alkinyl, (C₁-C₄)-Halogenalkyl, (C₂-C₄)-Halogenalkenyl, (C₂-C₄)-Halogenalkinyl, (C₁-C₆)-Alkoxy, (C₁-C₄)-Halogenalkoxy, (C₂-C₄)-Alkenyloxy, (C₃-C₄)-Alkinyloxy, (C₃-C₄)-Cycloalkyl-(C₁-C₄)-alkyl oder für eine gegebenenfalls ein- oder mehrfach durch M² substituierte (C₃-C₈)-Cycloalkyl oder halogeniertes (C₃-C₈)-Cycloalkyl steht;
R³¹, R³² für C(R³¹, R³²) als spiro-C(CH₂-CH₂) steht;
M² jeweils unabhängig voneinander für Halogen, Formyl, Cyano, Nitro, (C₁-C₄)-Alkyl, (C₁-C₄)-Halogenalkyl, (C₂-C₄)-Alkenyl, (C₂-C₄)-Halogenalkenyl, (C₂-C₄)-Alkinyl, (C₂-C₄)-Halogenalkinyl, (C₁-C₄)-Alkoxy, (C₁-C₄)-Halogenalkoxy, (C₂-C₄)-Alkenyloxy, (C₂-C₄)-Halogenalkenoxy, (C₃-C₄)-Alkinyloxy, (C₃-C₄)-Halogenalkinoxy, (C₁-C₄)-Alkylthio, (C₁-C₄)-Halogenalkylthio, (C₂-C₄)-Alkenylthio, (C₂-C₄)-Halogenalkenylthio, (C₃-C₄)-Alkinylthio, (C₃-C₄)-Halogenalkinylthio, (C₁-C₄)-Alkylsulfonyl, (C₁-C₄)-Halogenalkylsulfonyl, (C₂-C₄)-Alkenylsulfonyl, (C₂-C₄)-Halogenalkenylsulfonyl, (C₃-C₄)-Alkinylsulfonyl, (C₃-C₄)-Halogenalkinylsulfonyl, (C₁-C₄)-Alkylsulfanyl, (C₁-C₄)-Halogenalkylsulfanyl, (C₂-C₄)-Alkenylsulfanyl, (C₂-C₄)-Halogenalkenylsulfanyl, (C₃-C₄)-Alkinylsulfanyl, (C₃-C₄)-Halogenalkinylsulfanyl, (C₁-C₄)-Alkylcarbonyl, (C₁-C₄)-Halogenalkylcarbonyl, (C₂-C₄)-Al-kenylcarbonyl, (C₂-C₄)-Halogenalkenylcarbonyl, (C₂-C₄)-Alkinylcarbonyl, (C₂-C₄)-Halogenalkinylcarbonyl, (C₁-C₄-Alkoxycarbonyl, (C₁-C₄)-Halogenalkoxycarbonyl, (C₂-C₄)-Alkenoxycarbonyl, (C₂-C₄)-Halogenalkenoxycarbonyl, (C₃-C₄)-Alkinoxycarbonyl, (C₃-C₄)-Halogenalkinoxycarbonyl, (C₁-C₄)-Alkylcarbonyloxy, (C₁-C₄)-Halogenalkylcarbonyloxy, (C₂-C₄)-Alkenylcarbonyloxy, (C₂-C₄)-Halogenalkenylcarbonyloxy, (C₂-C₄)-Alkinylcarbonyloxy, (C₂-C₄)-Halogenalkinylcarbonyloxy oder (C₃-C₆)-Cycloalkyl steht;
sowie deren Salze, N-Oxide und tautomere Formen;
zur Verwendung als Arzneimittel zur Bekämpfung von Endoparasiten bei Tieren oder Menschen.

3. Verbindungen gemäß Anspruch 1 oder 2
wobei
X für ein ein- bis dreifach durch M¹ substituiertes Phenyl 2-Thienyl, 3-Thienyl, 2-Furanyl oder 3-Furanyl steht;
Q für ein- oder zweifach durch M¹ substituiertes 2-Thienyl, 3- Thienyl, 2-Furanyl, 5-Isothiazolyl, 3-Pyridyl oder 2-Pyridyl steht;
Y für Wasserstoff oder für gegebenenfalls ein- oder mehrfach durch M² substituiertes (C₁-C₄)-Alkyl, (C₂-C₄)-Alkenyl, (C₃-C₄)-Alkinyl, (C₁-C₄)-Halogenalkyl, (C₂-C₄)-Halogenalkenyl, (C₃-C₄)-Halogenalkinyl, (C₁-C₄)-Alkoxy, (C₂-C₄)-Alkenyloxy, (C₃-C₄)-Alkinyloxy, (C₃-C₆)-Cycloalkyl-(C₁-C₄)-alkyl oder für eine gegebenenfalls ein- oder mehrfach durch M² substituierte C₃- bis C₆-gliedrige carbocyclische Gruppe steht;
W für Sauerstoff steht;
M¹ für Wasserstoff, Halogen, Cyano, Nitro, OH, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Halogenalkylthio, (C₁-C₄)-Alkylsulfonyl, (C₁-C₄)-Halogenalkylsulfonyl, (C₁-C₄)-Alkylsulfanyl, (C₁-C₆)-Halogenalkylsulfanyl, (C₃-C₁₄)-Cycloalkyl-O-, (C₃-C₁₄)-Cycloalkenyl-O-, (C₆-C₁₄)-Aryl-O-, halogeniertes (C₃-C₁₄)-Cycloalkyl-O-, halogeniertes (C₃-C₁₄)-Cycloalkenyl-O-, halogeniertes (C₆-C₁₄)-Aryl)-O-steht;
R¹ für Wasserstoff oder Methyl, Ethyl, n-Propyl, iso-Propyl, Allyl, Propargyl, Cyclopropylmethyl oder Cyclopropyl steht;
R¹¹, R¹² jeweils unabhängig voneinander für Wasserstoff, Fluor oder (C₁-C₆)-Alkyl, (C₁-C₆)-Halogenalkyl, (C₁-C₆)-Alkoxy, (C₃-C₆)-Cycloalkyl-(C₁-C₆)-alkyl, (C₃-C₆)-Cycloalkyl steht;
R¹¹, R¹² für C(R¹¹, R¹²) als spiro-C(CH₂-CH₂) steht;
R²¹, R²² jeweils unabhängig voneinander für Wasserstoff, Fluor oder gegebenenfalls ein- oder mehrfach durch M² substituiertes (C₁-C₄)-Alkyl, (C₂-C₄)-Alkenyl, (C₂-C₄)-Alkinyl, (C₁-C₄)-Halogenalkyl, (C₁-C₆)-Alkoxy, (C₂-C₄)-Alkenyloxy, (C₃-C₄)-Alkinyloxy, (C₃-C₄)-Cycloalkyl-(C₁-C₄)-alkyl, (C₃-C₆)-Cycloalkyl steht;
R²¹, R²² für C(R²¹, R²²) als spiro-C(CH₂-CH₂) steht;
R³¹, R³² jeweils unabhängig voneinander für Wasserstoff, Methyl, Ethyl, n-Propyl, iso-Propyl, tert-Butyl, Allyl, Propargyl, Methoxy, Ethoxy, Allyloxy, Propargyloxy, Cyclopropylmethyl, Cyclopropyl steht;
R³¹, R³² für C(R³¹, R³²) als spiro-C(CH₂-CH₂) steht;
M² jeweils unabhängig voneinander für Chlor, Fluor, Formyl, Cyano, Nitro, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Halogenalkylthio, (C₁-C₄)-Alkylsulfonyl, (C₁-C₄)-Halogenalkylsulfonyl, (C₁-C₄)-Alkylsulfanyl, (C₁-C₄)-Halogenalkylsulfanyl, (C₁-C₄)-Alkylcarbonyl, (C₁-C₄)-Halogenalkylcarbonyl oder (C₃-C₆)-Cycloalkyl steht;
sowie deren Salze, N-Oxide und tautomere Formen;
zur Verwendung als Arzneimittel zur Bekämpfung von Endoparasiten bei Tieren oder Menschen.

4. Verbindungen gemäß einem der Ansprüche 1 bis 3
wobei
Q für 2-Thienyl, 3-Fluor-2-thienyl, 3-Chlor-2-thienyl, 3,4-Dichlor-2-thienyl, 3,4,5-Trichlor-2-thienyl, 3-Brom-2-thienyl, 3-Iod-2-thienyl, 3-Cyano-2-thienyl, 3-Methyl-2-thienyl, 3-(Trifluormethyl)-2-thienyl, 3-Methoxy-2-thienyl, 3-Ethoxy-2-thienyl, 3-Thienyl, 2-Fluor-3-thienyl, 2-Chlor-3-thienyl, 2-Brom-3-thienyl, 2-Iod-3-thienyl, 2-Cyano-3-thienyl, 2-Methyl-3-thienyl, 2-(Trifluormethyl)-3-thienyl, 2-Methoxy-3-thienyl, 2-Ethoxy-3-thienyl, 2-Furanyl, 3-Fluor-2-furanyl, 3-Chlor-2-furanyl, 3-Brom-2-furanyl, 3-Iod-2-furanyl, 3-Cyano-2-furanyl, 3-Methyl-2-furanyl, 3-(Trifluormethyl)-2-furanyl, 3-Methoxy-2-furanyl, 3-Ethoxy-2-furanyl, 3-Furanyl, 2-Chlor-3-furanyl, 2-Brom-3-furanyl, 2-Iod-3-furanyl, 2-Cyano-3-furanyl, 2-Methyl-3-furanyl, 2-(Trifluormethyl)-3-furanyl, 2-Methoxy-3-furanyl, 2-Ethoxy-3-furanyl, 4-Chlor-5-isothiazolyl, 3,4-Dichlor-5-isothiazolyl, 2-Chlor-3-pyridyl, 3-Chlor-2-pyridyl oder 2-(Trifluormethyl)-3-pyridyl steht;
Y für Wasserstoff, Methyl, Ethyl, n-Propyl, iso-Propyl, n-Butyl, sec-Butyl, iso-Butyl, tert.-Butyl, Cyanomethyl, 2,2-Difluor-ethyl, 2,2,2-Trifluor-ethyl, Allyl, Butenyl, Propargyl, Butinyl, 3,3-Dichlor-prop-2-enyl, Methoxy, Ethoxy, Cyclopropylmethyl, Cyclopropyl, Cyclobutyl, Cyclopentyl oder Cyclohexyl steht;
M¹ für für Wasserstoff, Halogen, Cyano, Nitro, OH, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Halogenalkylthio, (C₁-C₄)-Alkylsulfonyl, (C₁-C₄)-Halogenalkylsulfonyl, (C₁-C₄)-Alkylsulfanyl, (C₁-C₆)-Halogenalkylsulfanyl, (C₆-C₁₄)-Aryl-O-, halogeniertes (C₆-C₁₄)-Aryl)-O- steht;
R¹ für Wasserstoff oder (C₁-C₄)-Alkyl steht;
R¹¹, R¹² jeweils unabhängig voneinander für Wasserstoff, Fluor, Methyl, Ethyl, n-Propyl, iso-Propyl, Allyl, Propargyl, Methoxy, Ethoxy, Allyloxy, Propargyloxy, Cyclopropylmethyl, Cyclopropyl steht;
R²¹, R²² jeweils unabhängig voneinander für Wasserstoff, Fluor, Methyl, Ethyl, n-Propyl, iso-Propyl, Allyl, Propargyl, Methoxy, Ethoxy, Allyloxy, Propargyloxy, Cyclopropylmethyl, Cyclopropyl steht;
R³¹, R³² jeweils unabhängig voneinander für Wasserstoff oder (C₁-C₄)-Alkyl steht;
M² jeweils unabhängig voneinander für Fluor, Brom, Chlor, Iod, Cyano, Nitro, Methyl, Ethyl, iso-Propyl, tert.-Butyl, Trifluormethyl, Difluormethyl, Methoxy, Ethoxy, iso-Propoxy, Trifluormethoxy, Difluormethoxy, Methylthio, Trifluormethylthio, Difluormethylthio, 2,2,2-Trifluorethylthio, Methylsulfonyl, Ethylsulfonyl, Trifluormethylsulfonyl, 2,2,2-Trifluorethylsulfonyl, Methylsulfanyl, Ethylsulfanyl, Trifluormethylsulfanyl, 2,2,2-Trifluorethylsulfanyl, Cyclopropyl, Cyclobutyl oder Cyclopentyl steht.
sowie deren Salze, N-Oxide und tautomere Formen;
zur Verwendung als Arzneimittel zur Bekämpfung von Endoparasiten bei Tieren oder Menschen.

5. Verbindungen gemäß einem der Ansprüche 1 bis 4 zur Verwendung bei der Bekämpfung von Endoparasiten bei Tieren oder Menschen, **dadurch gekennzeichnet, dass** Verbindungen gemäß einem der Ansprüche 1 bis 4 sowie deren Salze, N-Oxide und tautomere Formen Tieren oder Menschen prophylaktisch oder therapeutisch verabreicht werden.

## Claims

1. Compounds of the formula (I) 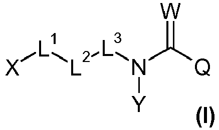 in which
X represents mono- or poly-M¹-substituted phenyl, thienyl or furanyl;
Q represents mono- or poly-M¹-substituted pyridyl, thienyl, furanyl or isothiazolyl;
Y represents hydrogen or represents optionally mono- or poly-M²-substituted (C₁-C₁₀) -alkyl, (C₂-C₁₀) -alkenyl, (C₂-C₁₀) -alkynyl, (C₁-C₁₀)-haloalkyl, (C₂-C₁₀) -haloalkenyl, (C₂-C₁₀)-haloalkynyl, (C₁-C₁₀) -alkoxy, (C₂-C₁₀)-alkenyloxy, (C₃-C₁₀) -alkynyloxy, (C₃-C₁₄) -cycloalkyl- (C₁-C₁₀)-alkyl or represents an optionally mono- or poly-M²-substituted 3- to 14-membered cyclic group;
W represents oxygen or sulfur;
L¹ represents -C(R¹¹, R¹²)-, oxygen, sulfur or -N(R¹)-;
L² represents -C(R²¹, R²²)-;
L³ represents -C(R³¹, R³²)- or a direct bond;
M¹ represents hydrogen, halogen, cyano, nitro, OH, (C₁-C₁₀) -alkyl, (C₁-C₁₀) -haloalkyl, (C₁-C₁₀)-alkoxy, (C₁-C₁₀) -haloalkoxy, (C₁-C₁₀) -alkylthio, (C₁-C₁₀)-haloalkylthio, (C₁-C₁₀)-alkylsulfonyl, (C₁-C₁₀)-haloalkylsulfonyl, (C₁-C₁₀)-alkylsulfanyl, (C₁-C₁₀)-haloalkylsulfanyl, (3- to 14-membered cyclic group)-O-;
R¹ represents hydrogen or optionally mono- or poly-M²-substituted (C₁-C₁₀)-alkyl, (C₂-C₁₀)-alkenyl, (C₂-C₁₀)-alkynyl, (C₁-C₁₀)-haloalkyl, (C₂-C₁₀)-haloalkenyl, (C₂-C₁₀)-haloalkynyl, (C₃-C₁₀)-cycloalkyl- (C₁-C₁₀)-alkyl or represents an optionally mono- or poly-M²-substituted 3- to 14-membered cyclic group;
R¹¹, R¹² each independently of one another represent hydrogen, halogen or optionally mono-or poly-M²-substituted (C₁-C₁₀)-alkyl, (C₂-C₁₀)-alkenyl, (C₂-C₁₀)-alkynyl, (C₁-C₁₀)-haloalkyl, (C₂-C₁₀)-haloalkenyl, (C₂-C₁₀)-haloalkynyl, (C₁-C₁₀)-alkoxy, (C₂-C₁₀)-alkenyloxy, (C₃-C₁₀)-alkynyloxy, (C₃-C₁₄)-cycloalkyl-(C₁-C₁₀)-alkyl or represent an optionally mono- or poly-M²-substituted 3- to 14-membered cyclic group;
R¹¹, R¹² together represent in each case an optionally mono- or poly-M²-substituted spiro-attached 3- to 14-membered carbo- or 3- to 10-membered heterocyclic group;
R²¹, R²² each independently of one another represent hydrogen, halogen or optionally mono- or poly-M²-substituted (C₁-C₁₀)-alkyl, (C₂-C₁₀)-alkenyl, (C₂-C₁₀)-alkynyl, (C₁-C₁₀)-haloalkyl, (C₂-C₁₀)-haloalkenyl, (C₂-C₁₀)-haloalkynyl, (C₁-C₁₀)-alkoxy, (C₁-C₁₀)-haloalkoxy, (C₂-C₁₀)-alkenyloxy, (C₃-C₁₀)-alkynyloxy, (C₃-C₁₄)-cycloalkyl-(C₁-C₁₀)-alkyl or represent an optionally mono- or poly-M²-substituted 3- to 14-membered cyclic group;
R²¹, R²² together represent in each case an optionally mono- or poly-M²-substituted spiro-attached 3- to 14-membered carbo- or 3- to 10-membered heterocyclic group;
R³¹, R³² each independently of one another represent hydrogen or optionally mono- or poly-M²-substituted (C₁-C₁₀)-alkyl, (C₂-C₁₀)-alkenyl, (C₂-C₁₀)-alkynyl, (C₁-C₁₀)-haloalkyl, (C₂-C₁₀)-haloalkenyl, (C₂-C₁₀)-haloalkynyl, (C₃-C₁₄)-cycloalkyl-(C₁-C₁₀)-alkyl or represent an optionally mono- or poly-M²-substituted 3- to 14-membered cyclic group;
R³¹, R³² together represent in each case an optionally mono- or poly-M²-substituted spiro-attached 3- to 14-membered carbo- or 3- to 10-membered heterocyclic group;
M² each independently of one another represent halogen, formyl, cyano, nitro, (C₁-C₁₀)-alkyl, (C₁-C₁₀)-haloalkyl, (C₂-C₁₀)-alkenyl, (C₂-C₁₀)-haloalkenyl, (C₂-C₁₀)-alkynyl, (C₂-C₁₀)-haloalkynyl, (C₁-C₁₀)-alkoxy, (C₁-C₁₀)-haloalkoxy, (C₂-C₁₀)-alkenyloxy, (C₂-C₁₀)-haloalkenyloxy, (C₃-C₁₀)-alkynyloxy, (C₃-C₁₀)-haloalkynyloxy, (C₁-C₁₀)-alkylthio, (C₁-C₁₀)-haloalkylthio, (C₂-C₁₀)-alkenylthio, (C₂-C₁₀)-haloalkenylthio, (C₃-C₁₀)-alkynylthio, (C₃-C₁₀)-haloalkynylthio, (C₁-C₁₀)-alkylsulfonyl, (C₁-C₁₀)-haloalkylsulfonyl, (C₂-C₁₀)-alkenylsulfonyl, (C₂-C₁₀)-haloalkenylsulfonyl, (C₃-C₁₀)-alkynylsulfonyl, (C₃-C₁₀)-haloalkynylsulfonyl, (C₁-C₁₀)-alkylsulfanyl, (C₁-C₁₀)-haloalkylsulfanyl, (C₂-C₁₀)-alkenylsulfanyl, (C₂-C₁₀)-haloalkenylsulfanyl, (C₃-C₁₀)-alkynylsulfanyl, (C₃-C₁₀)-haloalkynylsulfanyl, (C₁-C₁₀)-alkylcarbonyl, (C₁-C₁₀)-haloalkylcarbonyl, (C₂-C₁₀)-alkenylcarbonyl, (C₂-C₁₀)-haloalkenylcarbonyl, (C₂-C₁₀)-alkynylcarbonyl, (C₂-C₁₀)-haloalkynylcarbonyl, (C₁-C₁₀)-alkoxycarbonyl, (C₁-C₁₀)-haloalkoxycarbonyl, (C₂-C₁₀)-alkenyloxycarbonyl, (C₂-C₁₀)-haloalkenyloxycarbonyl, (C₃-C₁₀)-alkynyloxycarbonyl, (C₃-C₁₀)-haloalkynyloxycarbonyl, (C₁-C₁₀)-alkylcarbonyloxy, (C₁-C₁₀)-haloalkylcarbonyloxy, (C₂-C₁₀)-alkenylcarbonyloxy, (C₂-C₁₀)-haloalkenylcarbonyloxy, (C₂-C₁₀)-alkynylcarbonyloxy, (C₂-C₁₀)-haloalkynylcarbonyloxy, a 3- to 14-membered cyclic group;
and salts, N-oxides and tautomeric forms thereof;
for use as medicaments for controlling endoparasites in animals or humans.

2. Compounds according to Claim 1
where
X represents mono- to poly-M¹-substituted phenyl, 2-thienyl, 3-thienyl, 2-furanyl or 3-furanyl;
Q represents mono- or poly-M¹-substituted 2-thienyl, 3-thienyl, 2-furanyl, 5-isothiazolyl, 3-pyridyl or 2-pyridyl;
Y represents hydrogen or represents optionally mono- or poly-M²-substituted (C₁-C₁₀) -alkyl, (C₂-C₁₀)-alkenyl, (C₂-C₁₀)-alkynyl, (C₁-C₁₀)-haloalkyl, (C₂-C₁₀)-haloalkenyl, (C₂-C₁₀)-haloalkynyl, (C₁-C₁₀)-alkoxy, (C₂-C₁₀)-alkenyloxy, (C₃-C₁₀)-alkynyloxy, (C₃-C₁₄)-cycloalkyl-(C₁-C₁₀)-alkyl or represents an optionally mono- or poly-M²-substituted 3- to 14-membered cyclic group;
L¹ represents -C(R¹¹, R¹²)-, oxygen or -N(R¹)-;
M¹ represents hydrogen, halogen, cyano, nitro, OH, (C₁-C₆)-alkyl, (C₁-C₆)-haloalkyl, (C₁-C₆)-alkoxy, (C₁-C₆)-haloalkoxy, (C₁-C₆)-alkylthio, (C₁-C₆)-haloalkylthio, (C₁-C₆)-alkylsulfonyl, (C₁-C₆)-haloalkylsulfonyl, (C₁-C₆)-alkylsulfanyl, (C₁-C₆)-haloalkylsulfanyl, (C₃-C₁₄)-cycloalkyl-O-, (C₃-C₁₄)-cycloalkenyl-O-, (C₆-C₁₄)-aryl-O-, halogenated (C₃-C₁₄)-cycloalkyl-O-, halogenated (C₃-C₁₄)-cycloalkenyl-O-, halogenated (C₆-C₁₄)-aryl-O-;
R¹ represents hydrogen or optionally mono- or poly-M²-substituted (C₁-C₄)-alkyl, (C₂-C₄)-alkenyl, (C₂-C₄)-alkynyl, (C₁-C₄)-haloalkyl, (C₂-C₄)-haloalkenyl, (C₂-C₄)-haloalkynyl, (C₃-C₄)-cycloalkyl-(C₁-C₁₀)-alkyl or represents optionally mono- or poly-M²-substituted (C₃-C₈)-cycloalkyl or halogenated (C₃-C₈)-cycloalkyl;
R¹¹, R¹² each independently of one another represent hydrogen, fluorine or optionally mono- or poly-M²-substituted (C₁-C₆)-alkyl, (C₁-C₆)-haloalkyl, (C₁-C₆)-alkoxy, (C₃-C₆)-cycloalkyl-(C₁-C₆)-alkyl, (C₃-C₈)-cycloalkyl or halogenated (C₃-C₈)-cycloalkyl;
R¹¹, R¹² represent C(R¹¹, R¹²) as spiro-C (CH₂-CH₂);
R²¹, R²² each independently of one another represent hydrogen, fluorine or optionally mono- or poly-M²-substituted (C₁-C₄)-alkyl, (C₂-C₄)-alkenyl, (C₂-C₄)-alkynyl, (C₁-C₄)-haloalkyl, (C₂-C₄)-haloalkenyl, (C₂-C₄)-haloalkynyl, (C₁-C₆)-alkoxy, (C₁-C₄)-haloalkoxy, (C₂-C₄)-alkenyloxy, (C₃-C₄)-alkynyloxy, (C₃-C₄)-cycloalkyl-(C₁-C₄)-alkyl, (C₃-C₈)-cycloalkyl or halogenated (C₃-C₈)-cycloalkyl;
R²¹, R²² represent C(R²¹, R²²) as spiro-C(CH₂-CH₂);
R³¹, R³² each independently of one another represent hydrogen or optionally mono- or poly-M²-substituted (C₁-C₄)-alkyl, (C₂-C₄)-alkenyl, (C₂-C₄)-alkynyl, (C₁-C₄)-haloalkyl, (C₂-C₄)-haloalkenyl, (C₂-C₄)-haloalkynyl, (C₁-C₆)-alkoxy, (C₁-C₄)-haloalkoxy, (C₂-C₄)-alkenyloxy, (C₃-C₄)-alkynyloxy, (C₃-C₄)-cycloalkyl-(C₁-C₄)-alkyl or represent an optionally mono- or poly-M²-substituted (C₃-C₈)-cycloalkyl or halogenated (C₃-C₈)-cycloalkyl;
R³¹, R³² represent C(R³¹, R³²) as spiro-C(CH₂-CH₂);
M² each independently of one another represent halogen, formyl, cyano, nitro, (C₁-C₄) -alkyl, (C₁-C₄)-haloalkyl, (C₂-C₄)-alkenyl, (C₂-C₄)-haloalkenyl, (C₂-C₄)-alkynyl, (C₂-C₄)-haloalkynyl, (C₁-C₄)-alkoxy, (C₁-C₄)-haloalkoxy, (C₂-C₄)-alkenyloxy, (C₂-C₄)-haloalkenyloxy, (C₃-C₄)-alkynyloxy, (C₃-C₄)-haloalkynyloxy, (C₁-C₄)-alkylthio, (C₁-C₄)-haloalkylthio, (C₂-C₄)-alkenylthio, (C₂-C₄)-haloalkenylthio, (C₃-C₄)-alkynylthio, (C₃-C₄)-haloalkynylthio, (C₁-C₄)-alkylsulfonyl, (C₁-C₄)-haloalkylsulfonyl, (C₂-C₄)-alkenylsulfonyl, (C₂-C₄)-haloalkenylsulfonyl, (C₃-C₄)-alkynylsulfonyl, (C₃-C₄)-haloalkynylsulfonyl, (C₁-C₄)-alkylsulfanyl, (C₁-C₄)-haloalkylsulfanyl, (C₂-C₄)-alkenylsulfanyl, (C₂-C₄)-haloalkenylsulfanyl, (C₃-C₄)-alkynylsulfanyl, (C₃-C₄)-haloalkynylsulfanyl, (C₁-C₄)-alkylcarbonyl, (C₁-C₄)-haloalkylcarbonyl, (C₂-C₄)-alkenylcarbonyl, (C₂-C₄)-haloalkenylcarbonyl, (C₂-C₄)-alkynylcarbonyl, (C₂-C₄)-haloalkynylcarbonyl, (C₁-C₄)-alkoxycarbonyl, (C₁-C₄)-haloalkoxycarbonyl, (C₂-C₄)-alkenyloxycarbonyl, (C₂-C₄)-haloalkenyloxycarbonyl, (C₃-C₄)-alkynyloxycarbonyl, (C₃-C₄)-haloalkynyloxycarbonyl, (C₁-C₄)-alkylcarbonyloxy, (C₁-C₄)-haloalkylcarbonyloxy, (C₂-C₄)-alkenylcarbonyloxy, (C₂-C₄)-haloalkenylcarbonyloxy, (C₂-C₄)-alkynylcarbonyloxy, (C₂-C₄)-haloalkynylcarbonyloxy or (C₃-C₆)-cycloalkyl;
and salts, N-oxides and tautomeric forms thereof;
for use as medicaments for controlling endoparasites in animals or humans.

3. Compounds according to Claim 1 or 2
where
X represents mono- to tri-M¹-substituted phenyl, 2-thienyl, 3-thienyl, 2-furanyl or 3-furanyl;
Q represents mono- or di-M¹-substituted 2-thienyl, 3-thienyl, 2-furanyl, 5-isothiazolyl, 3-pyridyl or 2-pyridyl;
Y represents hydrogen or represents optionally mono- or poly-M²-substituted (C₁-C₄)-alkyl, (C₂-C₄)-alkenyl, (C₃-C₄)-alkynyl, (C₁-C₄)-haloalkyl, (C₂-C₄) -haloalkenyl, (C₃-C₄)-haloalkynyl, (C₁-C₄)-alkoxy, (C₂-C₄)-alkenyloxy, (C₃-C₄)-alkynyloxy, (C₃-C₆)-cycloalkyl-(C₁-C₄)-alkyl or represents an optionally mono- or poly-M²-substituted C₃- to C₆-membered carbocyclic group;
W represents oxygen;
M¹ represents hydrogen, halogen, cyano, nitro, OH, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-haloalkylthio, (C₁-C₄)-alkylsulfonyl, (C₁-C₄)-haloalkylsulfonyl, (C₁-C₄)-alkylsulfanyl, (C₁-C₆)-haloalkylsulfanyl, (C₃-C₁₄)-cycloalkyl-O-, (C₃-C₁₄)-cycloalkenyl-O-, (C₆-C₁₄)-aryl-O-, halogenated (C₃-C₁₄)-cycloalkyl-O-, halogenated (C₃-C₁₄)-cycloalkenyl-O-, halogenated (C₆-C₁₄)-aryl)-O-;
R¹ represents hydrogen or methyl, ethyl, n-propyl, isopropyl, allyl, propargyl, cyclopropylmethyl or cyclopropyl;
R¹¹, R¹² each independently of one another represent hydrogen, fluorine or (C₁-C₆)-alkyl, (C₁-C₆)-haloalkyl, (C₁-C₆)-alkoxy, (C₃-C₆)-cycloalkyl-(C₁-C₆)-alkyl, (C₃-C₆)-cycloalkyl;
R¹¹, R¹² represent C(R¹¹, R¹²) as spiro-C (CH₂-CH₂);
R²¹, R²² each independently of one another represent hydrogen, fluorine or optionally mono- or poly-M²-substituted (C₁-C₄)-alkyl, (C₂-C₄)-alkenyl, (C₂-C₄)-alkynyl, (C₁-C₄)-haloalkyl, (C₁-C₆)-alkoxy, (C₂-C₄)-alkenyloxy, (C₃-C₄)-alkynyloxy, (C₃-C₄)-cycloalkyl- (C₁-C₄)-alkyl, (C₃-C₆)-cycloalkyl;
R²¹, R²² represent C(R²¹, R²²) as spiro-C (CH₂-CH₂) ;
R³¹, R³² each independently of one another represent hydrogen, methyl, ethyl, n-propyl, isopropyl, tert-butyl, allyl, propargyl, methoxy, ethoxy, allyloxy, propargyloxy, cyclopropylmethyl, cyclopropyl;
R³¹, R³² represent C(R³¹, R³²) as spiro-C (CH₂-CH₂) ;
M² each independently of one another represent chlorine, fluorine, formyl, cyano, nitro, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-haloalkylthio, (C₁-C₄)-alkylsulfonyl, (C₁-C₄)-haloalkylsulfonyl, (C₁-C₄)-alkylsulfanyl, (C₁-C₄)-haloalkylsulfanyl, (C₁-C₄)-alkylcarbonyl, (C₁-C₄)-haloalkylcarbonyl or (C₃-C₆)-cycloalkyl;
and salts, N-oxides and tautomeric forms thereof;
for use as medicaments for controlling endoparasites in animals or humans.

4. Compounds according to any of Claims 1 to 3
where
Q represents 2-thienyl, 3-fluoro-2-thienyl, 3-chloro-2-thienyl, 3,4-dichloro-2-thienyl, 3,4,5-trichloro-2-thienyl, 3-bromo-2-thienyl, 3-iodo-2-thienyl, 3-cyano-2-thienyl, 3-methyl-2-thienyl, 3-(trifluoromethyl)-2-thienyl, 3-methoxy-2-thienyl, 3-ethoxy-2-thienyl, 3-thienyl, 2-fluoro-3-thienyl, 2-chloro-3-thienyl, 2-bromo-3-thienyl, 2-iodo-3-thienyl, 2-cyano-3-thienyl, 2-methyl-3-thienyl, 2-(trifluoromethyl)-3-thienyl, 2-methoxy-3-thienyl, 2-ethoxy-3-thienyl, 2-furanyl, 3-fluoro-2-furanyl, 3-chloro-2-furanyl, 3-bromo-2-furanyl, 3-iodo-2-furanyl, 3-cyano-2-furanyl, 3-methyl-2-furanyl, 3-(trifluoromethyl)-2-furanyl, 3-methoxy-2-furanyl, 3-ethoxy-2-furanyl, 3-furanyl, 2-chloro-3-furanyl, 2-bromo-3-furanyl, 2-iodo-3-furanyl, 2-cyano-3-furanyl, 2-methyl-3-furanyl, 2-(trifluoromethyl)-3-furanyl, 2-methoxy-3-furanyl, 2-ethoxy-3-furanyl, 4-chloro-5-isothiazolyl, 3,4-dichloro-5-isothiazolyl, 2-chloro-3-pyridyl, 3-chloro-2-pyridyl or 2-(trifluoromethyl)-3-pyridyl;
Y represents hydrogen, methyl, ethyl, n-propyl, isopropyl, n-butyl, sec-butyl, isobutyl, tert-butyl, cyanomethyl, 2,2-difluoroethyl, 2,2,2-trifluoroethyl, allyl, butenyl, propargyl, butynyl, 3,3-dichloroprop-2-enyl, methoxy, ethoxy, cyclopropylmethyl, cyclopropyl, cyclobutyl, cyclopentyl or cyclohexyl;
M¹ represents hydrogen, halogen, cyano, nitro, OH, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-haloalkylthio, (C₁-C₄)-alkylsulfonyl, (C₁-C₄)-haloalkylsulfonyl, (C₁-C₄)-alkylsulfanyl, (C₁-C₆)-haloalkylsulfanyl, (C₆-C₁₄)-aryl-O-, halogenated (C₆-C₁₄)-aryl)-O-;
R¹ represents hydrogen or (C₁-C₄)-alkyl;
R¹¹, R¹² each independently of one another represent hydrogen, fluorine, methyl, ethyl, n-propyl, isopropyl, allyl, propargyl, methoxy, ethoxy, allyloxy, propargyloxy, cyclopropylmethyl, cyclopropyl;
R²¹, R²² each independently of one another represent hydrogen, fluorine, methyl, ethyl, n-propyl, isopropyl, allyl, propargyl, methoxy, ethoxy, allyloxy, propargyloxy, cyclopropylmethyl, cyclopropyl;
R³¹, R³² each independently of one another represent hydrogen or (C₁-C₄)-alkyl;
M² each independently of one another represent fluorine, bromine, chlorine, iodine, cyano, nitro, methyl, ethyl, isopropyl, tert-butyl, trifluoromethyl, difluoromethyl, methoxy, ethoxy, isopropoxy, trifluoromethoxy, difluoromethoxy, methylthio, trifluoromethylthio, difluoromethylthio, 2,2,2-trifluoroethylthio, methylsulfonyl, ethylsulfonyl, trifluoromethylsulfonyl, 2,2,2-trifluoroethylsulfonyl, methylsulfanyl, ethylsulfanyl, trifluoromethylsulfanyl, 2,2,2-trifluoroethylsulfanyl, cyclopropyl, cyclobutyl or cyclopentyl.
and salts, N-oxides and tautomeric forms thereof;
for use as medicaments for controlling endoparasites in animals or humans.

5. Compounds according to any of Claims 1 to 4 for use in the control of endoparasites in animals or humans, **characterized in that** compounds according to any of Claims 1 to 4 and their salts, N-oxides or tautomeric forms are administered prophylactically or therapeutically to animals or humans.

## Revendications

1. Composés selon la formule (I) : 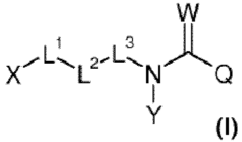 dans lesquels
X représente un phényle, thiényle ou furanyle substitué une ou plusieurs fois par M¹ ;
Q représente un pyridyle, thiényle, furanyle ou isothiazolyle substitué une ou plusieurs fois par M¹ ;
Y représente l'hydrogène ou un alkyle en (C₁-C₁₀), alcényle en (C₂-C₁₀), alcynyle en (C₂-C₁₀), halogénoalkyle en (C₁-C₁₀), halogénoalcényle en (C₂-C₁₀), halogénoalcynyle en (C₂-C₁₀), alcoxy en (C₁-C₁₀), alcényloxy en (C₂-C₁₀), alcynyloxy en (C₃-C₁₀), cycloalkyle en (C₃-C₁₄) -alkyle en (C₁-C₁₀) éventuellement substitué une ou plusieurs fois par M², ou un groupe cyclique de 3 à 14 chaînons éventuellement substitué une ou plusieurs fois par M² ;
W représente l'oxygène ou le soufre ;
L¹ représente -C(R¹¹, R¹²)-, l'oxygène, le soufre ou - N(R¹)- ;
L² représente -C(R²¹, R²²)- ;
L³ représente -C(R³¹, R³²) - ou une liaison directe ;
M¹ représente l'hydrogène, un halogène, cyano, nitro, OH, alkyle en (C₁-C₁₀), halogénoalkyle en (C₁-C₁₀), alcoxy en (C₁-C₁₀), halogénoalcoxy en (C₁-C₁₀), alkylthio en (C₁-C₁₀), halogénoalkylthio en (C₁-C₁₀), alkylsulfonyle en (C₁-C₁₀), halogénoalkylsulfonyle en (C₁-C₁₀), alkylsulfanyle en (C₁-C₁₀), halogénoalkylsulfanyle en (C₁-C₁₀), (groupe cyclique de 3 à 14 chaînons)-O- ;
R¹ représente l'hydrogène ou un alkyle en (C₁-C₁₀), alcényle en (C₂-C₁₀), alcynyle en (C₂-C₁₀), halogénoalkyle en (C₁-C₁₀), halogénoalcényle en (C₂-C₁₀), halogénoalcynyle en (C₂-C₁₀), cycloalkyle en (C₃-C₁₀)-alkyle en (C₁-C₁₀) éventuellement substitué une ou plusieurs fois par M², ou un groupe cyclique de 3 à 14 chaînons éventuellement substitué une ou plusieurs fois par M² ;
R¹¹, R¹² représentent chacun indépendamment l'un de l'autre l'hydrogène, un halogène ou un alkyle en (C₁-C₁₀), alcényle en (C₂-C₁₀), alcynyle en (C₂-C₁₀), halogénoalkyle en (C₁-C₁₀), halogénoalcényle en (C₂-C₁₀), halogénoalcynyle en (C₂-C₁₀), alcoxy en (C₁-C₁₀), alcényloxy en (C₂-C₁₀), alcynyloxy en (C₃-C₁₀), cycloalkyle en (C₃-C₁₄)-alkyle en (C₁-C₁₀) éventuellement substitué une ou plusieurs fois par M², ou un groupe cyclique de 3 à 14 chaînons éventuellement substitué une ou plusieurs fois par M² ;
R¹¹, R¹² représentent ensemble un groupe carbocyclique de 3 à 14 chaînons ou hétérocyclique de 3 à 10 chaînons à liaison spiro, à chaque fois éventuellement substitué une ou plusieurs fois par M² ;
R²¹, R²² représentent chacun indépendamment l'un de l'autre l'hydrogène, un halogène, ou un alkyle en (C₁-C₁₀), alcényle en (C₂-C₁₀), alcynyle en (C₂-C₁₀), halogénoalkyle en (C₁-C₁₀), halogénoalcényle en (C₂-C₁₀), halogénoalcynyle en (C₂-C₁₀), alcoxy en (C₁-C₁₀), halogénoalcoxy en (C₁-C₁₀), alcényloxy en (C₂-C₁₀), alcynyloxy en (C₃-C₁₀), cycloalkyle en (C₃-C₁₄) -alkyle en (C₁-C₁₀) éventuellement substitué une ou plusieurs fois par M², ou un groupe cyclique de 3 à 14 chaînons éventuellement substitué une ou plusieurs fois par M² ;
R²¹, R²² représentent ensemble un groupe carbocyclique de 3 à 14 chaînons ou hétérocyclique de 3 à 10 chaînons à liaison spiro, à chaque fois éventuellement substitué une ou plusieurs fois par M² ;
R³¹, R³² représentent chacun indépendamment l'un de l'autre l'hydrogène, ou un alkyle en (C₁-C₁₀), alcényle en (C₂-C₁₀), alcynyle en (C₂-C₁₀), halogénoalkyle en (C₁-C₁₀), halogénoalcényle en (C₂-C₁₀), halogénoalcynyle en (C₂-C₁₀), cycloalkyle en (C₃-C₁₄)-alkyle en (C₁-C₁₀) éventuellement substitué une ou plusieurs fois par M², ou un groupe cyclique de 3 à 14 chaînons éventuellement substitué une ou plusieurs fois par M² ;
R³¹, R³² représentent ensemble un groupe carbocyclique de 3 à 14 chaînons ou hétérocyclique de 3 à 10 chaînons à liaison spiro, à chaque fois éventuellement substitué une ou plusieurs fois par M² ;
les M² représentent chacun indépendamment les uns des autres halogène, formyle, cyano, nitro, alkyle en (C₁-C₁₀), halogénoalkyle en (C₁-C₁₀), alcényle en (C₂-C₁₀), halogénoalcényle en (C₂-C₁₀), alcynyle en (C₂-C₁₀), halogénoalcynyle en (C₂-C₁₀), alcoxy en (C₁-C₁₀), halogénoalcoxy en (C₁-C₁₀), alcényloxy en (C₂-C₁₀), halogénoalcénoxy en (C₂-C₁₀), alcynyloxy en (C₃-C₁₀), halogénoalcynoxy en (C₃-C₁₀), alkylthio en (C₁-C₁₀), halogénoalkylthio en (C₁-C₁₀), alcénylthio en (C₂-C₁₀), halogénoalcénylthio en (C₂-C₁₀), alcynylthio en (C₃-C₁₀), halogénoalcynylthio en (C₃-C₁₀), alkylsulfonyle en (C₁-C₁₀), halogénoalkylsulfonyle en (C₁-C₁₀), alcénylsulfonyle en (C₂-C₁₀), halogénoalcénylsulfonyle en (C₂-C₁₀), alcynylsulfonyle en (C₃-C₁₀), halogénoalcynylsulfonyle en (C₃-C₁₀), alkylsulfanyle en (C₁-C₁₀), halogénoalkylsulfanyle en (C₁-C₁₀), alcénylsulfanyle en (C₂-C₁₀), halogénoalcénylsulfanyle en (C₂-C₁₀), alcynylsulfanyle en (C₃-C₁₀), halogénoalcynylsulfanyle en (C₃-C₁₀), alkylcarbonyle en (C₁-C₁₀), halogénoalkylcarbonyle en (C₁-C₁₀), alcénylcarbonyle en (C₂-C₁₀), halogénoalcénylcarbonyle en (C₂-C₁₀), alcynylcarbonyle en (C₂-C₁₀), halogénoalcynylcarbonyle en (C₂-C₁₀), alcoxycarbonyle en (C₁-C₁₀), halogénoalcoxycarbonyle en (C₁-C₁₀), alcénoxycarbonyle en (C₂-C₁₀), halogénoalcénoxycarbonyle en (C₂-C₁₀), alcynoxycarbonyle en (C₃-C₁₀), halogénoalcynoxycarbonyle en (C₃-C₁₀), alkylcarbonyloxy en (C₁-C₁₀), halogénoalkylcarbonyloxy en (C₁-C₁₀), alcénylcarbonyloxy en (C₂-C₁₀), halogénoalcénylcarbonyloxy en (C₂-C₁₀), alcynylcarbonyloxy en (C₂-C₁₀), halogénoalcynylcarbonyloxy en (C₂-C₁₀), groupe cyclique de 3 à 14 chaînons ;
ainsi que leurs sels, N-oxydes et formes tautomères ;
destinés à une utilisation en tant que médicaments pour lutter contre des endoparasites chez des animaux ou des hommes.

2. Composés selon la revendication 1,
dans lesquels
X représente un phényle, 2-thiényle, 3-thiényle, 2-furanyle ou 3-furanyle substitué une ou plusieurs fois par M¹ ;
Q représente un 2-thiényle, 3-thiényle, 2-furanyle, 5-isothiazolyle, 3-pyridyle ou 2-pyridyle substitué une ou plusieurs fois par M¹ ;
Y représente l'hydrogène ou un alkyle en (C₁-C₁₀), alcényle en (C₂-C₁₀), alcynyle en (C₂-C₁₀), halogénoalkyle en (C₁-C₁₀), halogénoalcényle en (C₂-C₁₀), halogénoalcynyle en (C₂-C₁₀), alcoxy en (C₁-C₁₀), alcényloxy en (C₂-C₁₀), alcynyloxy en (C₃-C₁₀), cycloalkyle en (C₃-C₁₄) -alkyle en (C₁-C₁₀) éventuellement substitué une ou plusieurs fois par M², ou un groupe cyclique de 3 à 14 chaînons éventuellement substitué une ou plusieurs fois par M² ;
L¹ représente -C(R¹¹, R¹²)-, l'oxygène ou -N(R¹)- ;
M¹ représente l'hydrogène, un halogène, cyano, nitro, OH, alkyle en (C₁-C₆), halogénoalkyle en (C₁-C₆), alcoxy en (C₁-C₆), halogénoalcoxy en (C₁-C₆), alkylthio en (C₁-C₆), halogénoalkylthio en (C₁-C₆), alkylsulfonyle en (C₁-C₆), halogénoalkylsulfonyle en (C₁-C₆), alkylsulfanyle en (C₁-C₆), halogénoalkylsulfanyle en (C₁-C₆), cycloalkyle en (C₃-C₁₄)-O-, cycloalcényle en (C₃-C₁₄)-O-, aryle en (C₆-C₁₄) -O-, cycloalkyle en (C₃-C₁₄)-O-halogéné, cycloalcényle en (C₃-C₁₄)-O- halogéné, aryle en (C₆-C₁₄) -O- halogéné ;
R¹ représente l'hydrogène ou un alkyle en (C₁-C₄), alcényle en (C₂-C₄), alcynyle en (C₂-C₄), halogénoalkyle en (C₁-C₄), halogénoalcényle en (C₂-C₄), halogénoalcynyle en (C₂-C₄), cycloalkyle en (C₃-C₄) -alkyle en (C₁-C₁₀) éventuellement substitué une ou plusieurs fois par M², ou un cycloalkyle en (C₃-C₈) ou cycloalkyle en (C₃-C₈) halogéné éventuellement substitué une ou plusieurs fois par M² ;
R¹¹, R¹² représentent chacun indépendamment l'un de l'autre l'hydrogène, le fluor ou un alkyle en (C₁-C₆), halogénoalkyle en (C₁-C₆), alcoxy en (C₁-C₆), cycloalkyle en (C₃-C₆)-alkyle en (C₁-C₆), cycloalkyle en (C₃-C₈) ou cycloalkyle en (C₃-C₈) halogéné, éventuellement substitué une ou plusieurs fois par M² ;
R¹¹, R¹² représentent C(R¹¹, R¹²) sous la forme de spiro-C(CH₂-CH₂) ;
R²¹, R²² représentent chacun indépendamment l'un de l'autre l'hydrogène, le fluor, ou un alkyle en (C₁-C₄), alcényle en (C₂-C₄), alcynyle en (C₂-C₄), halogénoalkyle en (C₁-C₄), halogénoalcényle en (C₂-C₄), halogénoalcynyle en (C₂-C₄), alcoxy en (C₁-C₆), halogénoalcoxy en (C₁-C₄), alcényloxy en (C₂-C₄), alcynyloxy en (C₃-C₄), cycloalkyle en (C₃-C₄)-alkyle en (C₁-C₄), cycloalkyle en (C₃-C₈) ou cycloalkyle en (C₃-C₈) halogéné, éventuellement substitué une ou plusieurs fois par M² ;
R²¹, R²² représentent C(R²¹, R²²) sous la forme de spiro-C(CH₂-CH₂) ;
R³¹, R³² représentent chacun indépendamment l'un de l'autre l'hydrogène, ou un alkyle en (C₁-C₄), alcényle en (C₂-C₄), alcynyle en (C₂-C₄), halogénoalkyle en (C₁-C₄), halogénoalcényle en (C₂-C₄), halogénoalcynyle en (C₂-C₄), alcoxy en (C₁-C₆), halogénoalcoxy en (C₁-C₄), alcényloxy en (C₂-C₄), alcynyloxy en (C₃-C₄), cycloalkyle en (C₃-C₄)-alkyle en (C₁-C₄) éventuellement substitué une ou plusieurs fois par M², ou un cycloalkyle en (C₃-C₈) ou cycloalkyle en (C₃-C₈) halogéné éventuellement substitué une ou plusieurs fois par M² ;
R³¹, R³² représentent C(R³¹, R³²) sous la forme de spiro-C(CH₂-CH₂) ;
les M² représentent chacun indépendamment les uns des autres un halogène, formyle, cyano, nitro, alkyle en (C₁-C₄), halogénoalkyle en (C₁-C₄), alcényle en (C₂-C₄), halogénoalcényle en (C₂-C₄), alcynyle en (C₂-C₄), halogénoalcynyle en (C₂-C₄), alcoxy en (C₁-C₄), halogénoalcoxy en (C₁-C₄), alcényloxy en (C₂-C₄), halogénoalcénoxy en (C₂-C₄), alcynyloxy en (C₃-C₄), halogénoalcynoxy en (C₃-C₄), alkylthio en (C₁-C₄), halogénoalkylthio en (C₁-C₄), alcénylthio en (C₂-C₄), halogénoalcénylthio en (C₂-C₄), alcynylthio en (C₃-C₄), halogénoalcynylthio en (C₃-C₄), alkylsulfonyle en (C₁-C₄), halogénoalkylsulfonyle en (C₁-C₄), alcénylsulfonyle en (C₂-C₄), halogénoalcénylsulfonyle en (C₂-C₄), alcynylsulfonyle en (C₃-C₄), halogénoalcynylsulfonyle en (C₃-C₄), alkylsulfanyle en (C₁-C₄), halogénoalkylsulfanyle en (C₁-C₄), alcénylsulfanyle en (C₂-C₄), halogénoalcénylsulfanyle en (C₂-C₄), alcynylsulfanyle en (C₃-C₄), halogénoalcynylsulfanyle en (C₃-C₄), alkylcarbonyle en (C₁-C₄), halogénoalkylcarbonyle en (C₁-C₄), alcénylcarbonyle en (C₂-C₄), halogénoalcénylcarbonyle en (C₂-C₄), alcynylcarbonyle en (C₂-C₄), halogénoalcynylcarbonyle en (C₂-C₄), alcoxycarbonyle en (C₁-C₄), halogénoalcoxycarbonyle en (C₁-C₄), alcénoxycarbonyle en (C₂-C₄), halogénoalcénoxycarbonyle en (C₂-C₄), alcynoxycarbonyle en (C₃-C₄), halogénoalcynoxycarbonyle en (C₃-C₄), alkylcarbonyloxy en (C₁-C₄), halogénoalkylcarbonyloxy en (C₁-C₄), alcénylcarbonyloxy en (C₂-C₄), halogénoalcénylcarbonyloxy en (C₂-C₄), alcynylcarbonyloxy en (C₂-C₄), halogénoalcynylcarbonyloxy en (C₂-C₄) ou cycloalkyle en (C₃-C₆) ;
ainsi que leurs sels, N-oxydes et formes tautomères ;
destinés à une utilisation en tant que médicaments pour lutter contre des endoparasites chez des animaux ou des hommes.

3. Composés selon la revendication 1 ou 2,
dans lesquels
X représente un phényle, 2-thiényle, 3-thiényle, 2-furanyle ou 3-furanyle substitué une à trois fois par M¹;
Q représente un 2-thiényle, 3-thiényle, 2-furanyle, 5-isothiazolyle, 3-pyridyle ou 2-pyridyle substitué une ou deux fois par M¹ ;
Y représente l'hydrogène ou un alkyle en (C₁-C₄), alcényle en (C₂-C₄), alcynyle en (C₃-C₄), halogénoalkyle en (C₁-C₄), halogénoalcényle en (C₂-C₄), halogénoalcynyle en (C₃-C₄), alcoxy en (C₁-C₄), alcényloxy en (C₂-C₄), alcynyloxy en (C₃-C₄), cycloalkyle en (C₃-C₆) -alkyle en (C₁-C₄) éventuellement substitué une ou plusieurs fois par M², ou un groupe carbocyclique de C₃ à C₆ chaînons éventuellement substitué une ou plusieurs fois par M² ;
W représente l'oxygène ;
M¹ représente l'hydrogène, un halogène, cyano, nitro, OH, alkyle en (C₁-C₄), halogénoalkyle en (C₁-C₄), alcoxy en (C₁-C₄), halogénoalcoxy en (C₁-C₄), alkylthio en (C₁-C₄), halogénoalkylthio en (C₁-C₄), alkylsulfonyle en (C₁-C₄), halogénoalkylsulfonyle en (C₁-C₄), alkylsulfanyle en (C₁-C₄), halogénoalkylsulfanyle en (C₁-C₆), cycloalkyle en (C₃-C₁₄)-O-, cycloalcényle en (C₃-C₁₄)-O-, aryle en (C₆-C₁₄)-O-, cycloalkyle en (C₃-C₁₄)-O-halogéné, cycloalcényle en (C₃-C₁₄)-O- halogéné, aryle en (C₆-C₁₄)-O- halogéné ;
R¹ représente l'hydrogène ou méthyle, éthyle, n-propyle, iso-propyle, allyle, propargyle, cyclopropylméthyle ou cyclopropyle ;
R¹¹, R¹² représentent chacun indépendamment l'un de l'autre l'hydrogène, le fluor ou un alkyle en (C₁-C₆), halogénoalkyle en (C₁-C₆), alcoxy en (C₁-C₆), cycloalkyle en (C₃-C₆) -alkyle en (C₁-C₆), cycloalkyle en (C₃-C₆) ;
R¹¹, R¹² représentent C(R¹¹, R¹²) sous la forme de spiro-C(CH₂-CH₂) ;
R²¹, R²² représentent chacun indépendamment l'un de l'autre l'hydrogène, le fluor, ou un alkyle en (C₁-C₄), alcényle en (C₂-C₄), alcynyle en (C₂-C₄), halogénoalkyle en (C₁-C₄), alcoxy en (C₁-C₆), alcényloxy en (C₂-C₄), alcynyloxy en (C₃-C₄), cycloalkyle en (C₃-C₄) -alkyle en (C₁-C₄), cycloalkyle en (C₃-C₆), éventuellement substitué une ou plusieurs fois par M² ;
R²¹, R²² représentent C(R²¹, R²²) sous la forme de spiro-C(CH₂-CH₂) ;
R³¹, R³² représentent chacun indépendamment l'un de l'autre l'hydrogène, méthyle, éthyle, n-propyle, iso-propyle, tert-butyle, allyle, propargyle, méthoxy, éthoxy, allyloxy, propargyloxy, cyclopropylméthyle, cyclopropyle ;
R³¹, R³² représentent C(R³¹, R³²) sous la forme de spiro-C(CH₂-CH₂) ;
les M² représentent chacun indépendamment les uns des autres chlore, fluor, formyle, cyano, nitro, alkyle en (C₁-C₄), halogénoalkyle en (C₁-C₄), alcoxy en (C₁-C₄), halogénoalcoxy en (C₁-C₄), alkylthio en (C₁-C₄), halogénoalkylthio en (C₁-C₄), alkylsulfonyle en (C₁-C₄), halogénoalkylsulfonyle en (C₁-C₄), alkylsulfanyle en (C₁-C₄), halogénoalkylsulfanyle en (C₁-C₄), alkylcarbonyle en (C₁-C₄), halogénoalkylcarbonyle en (C₁-C₄) ou cycloalkyle en (C₃-C₆) ;
ainsi que leurs sels, N-oxydes et formes tautomères ;
destinés à une utilisation en tant que médicaments pour lutter contre des endoparasites chez des animaux ou des hommes.

4. Composés selon l'une quelconque des revendications 1 à 3,
dans lesquels
Q représente 2-thiényle, 3-fluoro-2-thiényle, 3-chloro-2-thiényle, 3,4-dichloro-2-thiényle, 3,4,5-trichloro-2-thiényle, 3-bromo-2-thiényle, 3-iodo-2-thiényle, 3-cyano-2-thiényle, 3-méthyl-2-thiényle, 3-(trifluorométhyl)-2-thiényle, 3-méthoxy-2-thiényle, 3-éthoxy-2-thiényle, 3-thiényle, 2-fluoro-3-thiényle, 2-chloro-3-thiényle, 2-bromo-3-thiényle, 2-iodo-3-thiényle, 2-cyano-3-thiényle, 2-méthyl-3-thiényle, 2-(trifluorométhyl)-3-thiényle, 2-méthoxy-3-thiényle, 2-éthoxy-3-thiényle, 2-furanyle, 3-fluoro-2-furanyle, 3-chloro-2-furanyle, 3-bromo-2-furanyle, 3-iodo-2-furanyle, 3-cyano-2-furanyle, 3-méthyl-2-furanyle, 3-(trifluorométhyl)-2-furanyle, 3-méthoxy-2-furanyle, 3-éthoxy-2-furanyle, 3-furanyle, 2-chloro-3-furanyle, 2-bromo-3-furanyle, 2-iodo-3-furanyle, 2-cyano-3-furanyle, 2-méthyl-3-furanyle, 2-(trifluorométhyl)-3-furanyle, 2-méthoxy-3-furanyle, 2-éthoxy-3-furanyle, 4-chloro-5-isothiazolyle, 3,4-dichloro-5-isothiazolyle, 2-chloro-3-pyridyle, 3-chloro-2-pyridyle ou 2-(trifluorométhyl)-3-pyridyle ;
Y représente hydrogène, méthyle, éthyle, n-propyle, iso-propyle, n-butyle, sec-butyle, iso-butyle, tert.-butyle, cyanométhyle, 2,2-difluoro-éthyle, 2,2,2-trifluoro-éthyle, allyle, butényle, propargyle, butynyle, 3,3-dichloro-prop-2-ényle, méthoxy, éthoxy, cyclopropylméthyle, cyclopropyle, cyclobutyle, cyclopentyle ou cyclohexyle ;
M¹ représente l'hydrogène, un halogène, cyano, nitro, OH, alkyle en (C₁-C₄), halogénoalkyle en (C₁-C₄), alcoxy en (C₁-C₄), halogénoalcoxy en (C₁-C₄), alkylthio en (C₁-C₄), halogénoalkylthio en (C₁-C₄), alkylsulfonyle en (C₁-C₄), halogénoalkylsulfonyle en (C₁-C₄), alkylsulfanyle en (C₁-C₄), halogénoalkylsulfanyle en (C₁-C₆), aryle en (C₆-C₁₄)-O-, aryle en (C₆-C₁₄)-O- halogéné ;
R¹ représente l'hydrogène ou un alkyle en (C₁-C₄) ;
R¹¹, R¹² représentent chacun indépendamment l'un de l'autre l'hydrogène, le fluor, méthyle, éthyle, n-propyle, iso-propyle, allyle, propargyle, méthoxy, éthoxy, allyloxy, propargyloxy, cyclopropylméthyle, cyclopropyle ;
R²¹, R²² représentent chacun indépendamment l'un de l'autre l'hydrogène, le fluor, méthyle, éthyle, n-propyle, iso-propyle, allyle, propargyle, méthoxy, éthoxy, allyloxy, propargyloxy, cyclopropylméthyle, cyclopropyle ;
R³¹, R³² représentent chacun indépendamment l'un de l'autre l'hydrogène ou un alkyle en (C₁-C₄) ;
les M² représentent chacun indépendamment les uns des autres fluor, brome, chlore, iode, cyano, nitro, méthyle, éthyle, iso-propyle, tert.-butyle, trifluorométhyle, difluorométhyle, méthoxy, éthoxy, iso-propoxy, trifluorométhoxy, difluorométhoxy, méthylthio, trifluorométhylthio, difluorométhylthio, 2,2,2-trifluoroéthylthio, méthylsulfonyle, éthylsulfonyle, trifluorométhylsulfonyle, 2,2,2-trifluoroéthylsulfonyle, méthylsulfanyle, éthylsulfanyle, trifluorométhylsulfanyle, 2,2,2-trifluoroéthylsulfanyle, cyclopropyle, cyclobutyle ou cyclopentyle ;
ainsi que leurs sels, N-oxydes et formes tautomères ;
destinés à une utilisation en tant que médicaments pour lutter contre des endoparasites chez des animaux ou des hommes.

5. Composés selon l'une quelconque des revendications 1 à 4, destinés à une utilisation dans la lutte contre des endoparasites chez des animaux ou des hommes, **caractérisés en ce que** des composés selon l'une quelconque des revendications 1 à 4, ainsi que leurs sels, N-oxydes et formes tautomères, sont administrés à titre prophylactique ou thérapeutique à des animaux ou des hommes.
